# EUROPEAN PATENT SPECIFICATION

(11) **EP 2 155 907 B1**
(45) Date of publication and mention of the grant of the patent: **27.05.2015**
(21) Application number: 08751458.4
(22) Date of filing: 30.04.2008
(51) Int. Cl.: C12Q 1/68

(54) **GENETIC VARIANTS USEFUL FOR RISK ASSESSMENT OF CORONARY ARTERY DISEASE AND MYOCARDIAL INFARCTION**
FÜR DIE RISIKOBEURTEILUNG VON KORONARER HERZKRANKHEIT UND MYOKARDINFARKT GEEIGNETE GENETISCHE VARIANTEN
VARIANTES GÉNÉTIQUES D'ÉVALUATION DE LA PRÉDISPOSITION AUX MALADIE DES ARTÈRES CORONAIRES ET À L'INFARCTUS DU MYOCARDE

(30) Priority: 30.04.2007 IS 8639
(43) Date of publication of application: 24.02.2010
(73) Proprietor: Decode Genetics EHF, 101 Reykjavik (IS)
(72) Inventor: HELGADOTTIR, Anna, IS-112 Reykjavik (IS); THORLEIFSSON, Gudmar, 111 Reykjavik (IS); MANOLESCU, Andrei, 105 Reykjavik (IS)
(74) Representative: Arnason Faktor
(86) International application number: PCT/IS2008/000011
(87) International publication number: WO 2008/132763

(56) References cited:
- WO-A-2004/035741
- WO-A-2004/058052
- WO-A-2004/081187
- WO-A-2005/110039
- WO-A-2006/099365
- WO-A-2007/006858
- US-A1- 2005 272 051
- DATABASE NCBI [Online] 9 November 2006 (2006-11-09), XP002490055 retrieved from HTTP://WWW.NCBI.NLM.NIH.GOV/SNP/SNP_REF.CG I?RS=11751605 Database accession no. rs11751605
- KATHARINA NEUREUTHER: "Association of Single Nucleotide Polymorphisms in the LPA gene region with serum Lp(a) levels and myocardial infarction. Dissertation zur Erlandung des Doktorgrades der Naturwissenschaften der naturwissenschaftlichen Fakultät III - Biologie und vorklinische Medizin - der Universität Regensburg." [Online] 6 February 2008 (2008-02-06), , UNIVERSITY REGENSBURG , XP002490054 Retrieved from the Internet: URL:http://www.opus-bayern.de/uni-regensbu rg/volltexte/2008/1002/pdf/Katharina_Neure uther.pdf> the whole document
- JAMES M. KELLEY ET AL: "An African Ancestry-Specific Allele of CTLA4 Confers Protection against Rheumatoid Arthritis in African Americans", PLOS GENETICS, vol. 5, no. 3, 20 March 2009 (2009-03-20), page e1000424, XP055045421, DOI: 10.1371/journal.pgen.1000424
- JAMES KELLEY ET AL: 'Retraction: An African Ancestry-Specific Allele of CTLA4 Confers Protection against Rheumatoid Arthritis in African Americans' PLOS GENETICS 01 December 2009, pages 1 - 14, XP055045473 DOI: 10.1371/annotation/80bd7285-9d2d-403a-8e6f- 9c375bf977ca

## Description

### BACKGROUND OF THE INVENTION

### Epidemiology and etiology

Cardiovascular diseases continue to be the principal cause of death in developed countries (Bonow, RO et al., Circulation 106:1602-05 (2002)). The costs of these diseases are high both in terms of morbidity and mortality, as well as in terms of the financial burden on health care systems. The major complications of coronary artery disease, i.e. Myocardial Infarction (MI) and Acute Coronary Syndrome (ACS), are the leading causes of hospital admissions in industrialized countries. Of all deaths caused by cardiovascular diseases, more than half are attributed to heart attack (myocardial infarction (MI)). The cost of the disease is high both in terms of morbidity and mortality, as well as in terms of the financial burden on the health care systems. According to the NHLBI's ARIC study (Thom, T et al., Circulation 113:e85-151 (2006)), the annual rate of first heart attack (MI or coronary heart disease (CHD) death) per 1000 individuals for ages 65-74 are 19.2 and 6.8, in European American men and women, respectively. For the same age group, the rates are 21.6 and 8.6, in African American men and women respectively. In the older age groups these numbers are much higher with the lifetime risk after the age of 40 of developing heart attack being 49% for men and 32% for women.

Myocardial infarction generally occurs when there is an abrupt decrease in coronary blood flow following a thrombotic occlusion of a coronary artery previously damaged by atherosclerosis (i.e. in subjects with coronary artery disease). In most cases, infarction occurs when an atherosclerotic plaque fissures, ruptures or ulcerates and when conditions favor thrombogenesis. In rare cases, infarction may be due to coronary artery occlusion caused by coronary emboli, congenital abnormalities, coronary spasm, and a wide variety of systemic, particularly inflammatory diseases. Medical risk factors for MI include cigarette smoking, diabetes, hypertension and serum total cholesterol levels > 200 mg/dL, elevated serum LDL cholesterol, and low serum HDL cholesterol. Event rates in individuals without a prior history of cardiovascular disease are about 1%. In individuals who have had a first MI or ACS, the risk of a repeat MI within the next year is 10-14%, despite maximal medical management including angioplasty and stent placement.

Atherosclerosis can affect vascular beds in many large and medium arteries. Myocardial infarction and unstable angina (acute coronary syndrome (ACS)) stem from coronary artery atherosclerosis (coronary artery disease), while ischemic stroke most frequently is a consequence of carotid or cerebral artery atherosclerosis. Limb ischemia caused by peripheral arterial occlusive disease (PAOD) may occur as a consequence of iliac, femoral and popliteal artery atherosclerosis. The atherosclerotic diseases remain common despite the wide-spread use of medications that inhibit thrombosis (aspirin) or treat medical risk factors such as elevated cholesterol levels in blood (statins), diabetes, or hypertension (diuretics and antihypertensives).

Atherosclerotic disease is initiated by the accumulation of lipids within the artery wall, and in particular, the accumulation of low-density lipoprotein (LDL) cholesterol. The trapped LDL becomes oxidized and internalized by macrophages. This causes the formation of atherosclerotic lesions containing accumulations of cholesterol-engorged macrophages, referred to as "foam cells As disease progresses, smooth muscle cells proliferate and grow into the artery wall forming a "fibrous cap" of extracellular matrix enclosing a lipid-rich, necrotic core. Present in the arterial walls of most people throughout their lifetimes, fibrous atherosclerotic plaques are relatively stable. Such fibrous lesions cause extensive remodeling of the arterial wall, outwardly displacing the external, elastic membrane, without reduction in luminal diameter or serious impact on delivery of oxygen to the heart. Accordingly, patients can develop large, fibrous atherosclerotic lesions without luminal narrowing until late in the disease process. However, the coronary arterial lumen can become gradually narrowed over time and in some cases compromise blood flow to the heart, especially under high demand states such as exercise. This can result in reversible ischemia causing chest pain relieved by rest called stable angina.

In contrast to the relative stability of fibrous atherosclerotic lesions, the culprit lesions associated with myocardial infarction and unstable angina (each of which are part of the acute coronary syndrome) are characterized by a thin fibrous cap, a large lipid core, and infiltration of inflammatory cells such as T-lymphocytes and monocyte/macrophages. Non-invasive imaging techniques have shown that most MI's occur at sites with low- or intermediate-grade stenoses, indicating that coronary artery occlusion is due most frequently to rupture of culprit lesions with consequent formation of a thrombus or blood clot and not solely due to luminal narrowing by stenosis. Plaque rupture may be due to erosion or uneven thinning of the fibrous cap, usually at the margins of the lesion where macrophages enter, accumulate, and become activated by a local inflammatory process. Thinning of the fibrous cap may result from degradation of the extracellular matrix by proteases released from activated macrophages. These changes producing plaque instability and risk of MI may be augmented by production of tissue-factor procoagulant and other factors increasing the likelihood of thrombosis.

In acute coronary syndrome, the culprit lesion showing rupture or erosion with local thrombosis typically is treated by angioplasty or by balloon dilation and placement of a stent to maintain luminal patency. Patients experiencing ACS are at high risk for a second coronary event due to the multi-vessel nature of coronary artery disease with event rates approaching 10-14% within 12 months after the first incident.

The emerging view of MI is as an inflammatory disease of the arterial vessel wall on preexisting chronic atherosclerotic lesions, sometimes triggering rupture of culprit lesions and leading to local thrombosis and subsequent myocardial infarction. The process that triggers and sustains arterial wall inflammation leading to plaque instability is unknown, however, it results in the release into the circulation of tumor necrosis factor alpha and interleukin-6. These and other cytokines or biological mediators released from the damaged vessel wall stimulate an inflammatory response in the liver causing elevation in several non-specific general inflammatory markers including C-reactive protein. Although not specific to atherosclerosis, elevated C-reactive protein (CRP) and serum amyloid A appear to predict risk for MI, perhaps as surrogates for vessel wall inflammation. Many general inflammatory markers predict risk of coronary heart disease, although these markers are not specific to atherosclerosis. For example, Stein (Stein, S., Am J Cardiol, 87 (suppl):21A-26A (2001)) discusses the use of any one of the following serum inflammatory markers as surrogates for predicting risk of coronary heart disease including C-reactive protein (CRP), serum amyloid A, fibrinogen, Interleukin-6, tissue necrosis factor-alpha, soluble vascular cell adhesion molecules (sVCAM), soluble intervascular adhesion molecules (sICAM), E-selectin, matrix metalloprotease type-1, matrix metalloprotease type-2, matrix metalloprotease type-3, and matrix metalloprotease type-9. Elevation in one more of these serum inflammatory markers is not specific to coronary heart disease but also occurs with age or in association with cerebrovascular disease, peripheral vascular disease, non-insulin dependent diabetes, osteoarthritis, bacterial infection, and sepsis. Elevated CRP or other serum inflammatory markers is also prognostic for increased risk of a second myocardial infarct in patients with a previous myocardial infarct (Retterstol, L. et al., Atheroscler., 160: 433-440 (2002)).

Although classical risk factors such as smoking, hyperlipidemia, hypertension, and diabetes are associated with many cases of coronary heart disease (CHD) and MI, many patients do not have involvement of these risk factors. In fact, many patients who exhibit one or more of these risk factors do not develop MI. Family history has long been recognized as one of the major risk factors. Although some of the familial clustering of MI reflects the genetic contribution to the other conventional risk factors, a large number of studies have suggested that there are significant genetic susceptibility factors, beyond those of the known risk factors (Friedlander Y, et al., Br. Heart J. 1985; 53:382-7, Shea S. et al., J. Am. Coll. Cardiol. 1984; 4:793-801, and Hopkins P.N., et al., Am. J. Cardiol. 1988; 62:703-7). Major genetic susceptibility factors have only been identified for the rare Mendelian forms of hyperlipidemia such as a familial hypercholesterolemia.

### Genetic risk factors

Genetic risk of disease is conferred by subtle differences in genes among individuals in a population. Genes differ between individuals most frequently due to single nucleotide polymorphisms (SNP), although other variations are also important. SNPs are located on average every 1000 base pairs in the human genome. Accordingly, a typical human gene containing 250,000 base pairs may contain 250 different SNPs. Only a minor number of SNPs are located in exons and alter the amino acid sequence of the protein encoded by the gene. Most SNPs have no effect on gene function, while others may alter transcription, splicing, translation, or stability of the mRNA encoded by the gene. Additional genetic polymorphisms in the human genome are caused by insertions, deletions, translocations, or inversions of either short or long stretches of DNA. Genetic polymorphisms conferring disease risk may therefore directly alter the amino acid sequence of proteins, may increase the amount of protein produced from the gene, or may decrease the amount of protein produced by the gene.

As genetic polymorphisms conferring risk of disease are uncovered, genetic testing for such risk factors is becoming important for clinical medicine. Examples are apolipoprotein E testing to identify genetic carriers of the ApoE4 polymorphism in dementia patients for the differential diagnosis of Alzheimer's disease, and of Factor V Leiden testing for predisposition to deep venous thrombosis. More importantly, in the treatment of cancer, diagnosis of genetic variants in tumor cells is used for the selection of the most appropriate treatment regime for the individual patient. In breast cancer, genetic variation in estrogen receptor expression or heregulin type 2 (Her2) receptor tyrosine kinase expression determine if anti-estrogenic drugs (tamoxifen) or anti-Her2 antibody (Herceptin) will be incorporated into the treatment plan. In chronic myeloid leukemia (CML) diagnosis of the Philadelphia chromosome genetic translocation fusing the genes encoding the Bcr and Abl receptor tyrosine kinases indicates that Gleevec (STI571), a specific inhibitor of the Bcr-Abl kinase should be used for treatment of the cancer. For CML patients with such a genetic alteration, inhibition of the Bcr-Abl kinase leads to rapid elimination of the tumor cells and remission from leukemia.

The familial clustering of CAD and MI has long been recognized and family history is considered one of the major risk factors for MI. Evidence for a genetic contribution to MI has been provided by both twin and family studies. These studies have estimated the heritability of MI to be 0.26 in males and 0.60 in females (Jorde, L. et al. Medical Genetics 3rd ed. St. Louis: Mosby (2003)) and that the concordance rates for monzygotic vs. dizygotic twins is 0.39 vs. 0.26 for males and 0.44 vs. 0.14 for females. A longitudinal study of 21,004 Swedish twins showed that the relative hazard of death from CAD when one twin died of an early-onset CAD (defined as before age of 55 for males and 65 for females), compared to the hazard ratio when one's twin did not die of early-onset CAD, was 8.1 and 3.8 for male monozygotic and dizygotic twins, respectively (Marenberg, ME, N Engl J Med 330:1041-46 (1994)). The corresponding relative hazard for female twins was 15 for monozygotic twins and 2.6 for dizygotic twins.

Family studies have consistently demonstrated that having a first degree relative with premature CAD death increases the risk of developing CAD (Schildkraut, JM et al Am J Cardiol 64:555-59 (1989); Slack, J & Evans, KA, J Med Genet 3:239-57 (1966)), although the reported increase in risk has varied between studies. The mechanisms/processes by which the family history contributes to MI are currently not well defined. Some of the familial clustering of MI may reflect the familial aggregation to the other conventional risk factors. However, a large number of studies, including the Reykjavik Cohort Study, have suggested that there are significant genetic susceptibility factors, beyond those of the known risk factors (Friedlander, Y., Br Heart J 53:382-87 (1985); Shea, S., J Am Coll Cardiol 4:793-801 (1984); Andresdottir, MB, Eur Heart J 23:1655-63 (2002)). The Reykjavik Cohort Study in Iceland examined the relationship between the history of MI in first-degree relatives and the risk of developing CHD (MI or coronary revascularization) in a large prospective study. Compared with subjects without a family history, the hazard ratio of CHD was 1.75 (95% CI: 1.59-1.92) for men and 1.83 (1.60-2.11) for women, with one or more first-degree relatives with MI, and after adjusting for the conventional risk factors, it was 1.66 (1.51-1.82) and 1.64 (1.43-1.89) for men and women, respectively (Andresdottir, MB, Eur Heart J 23:1655-63 (2002)).

Case-control association studies have suggested the involvement of specific genetic susceptibility variants for MI. The majority of those studies have focused on variants in selected candidate genes (the candidate gene approach), genes that might be involved in the pathogenesis of the disease, or in the development of the intermediate phenotype (e.g. known risk factors). In most cases SNPs that change amino acids or are in promoter regions of genes, have been tested for association. Two studies have tested variants within multiple candidate genes for association with MI. In one, association of C1019T polymorphism in the connexin 37 gene to MI in men and 4G-668-5G polymorphism in the plasminogen-activator-inhibitor type 1 gene and the 5A-1171/6A polymorphism in the stromelysin-1 gene in women was reported (Yamada, Y. et al N Engl J Med 347:1916-23 (2002)). Another study examining 72 SNPs in 62 candidate genes in individuals with premature MI and controls from the US, showed that three variants in genes encoding thrombosponding-4, thrombospondin-2, and thrombospondin-1, associated with premature MI (Topol, EJ, et al Circulation 104:2641-44 (2001). A more recent study tested 11,053 SNPs in 6,891 genes for association to MI in a group of European-Americans (Shiffman, d., et al Am J Hum Genet 77:596-605 (2005)). The SNPs tested were primarly selected as variants likely to affect protein function or expression. MI associated variants in four novel genes (*KIAA0992, ROS1,TAS2R50,* and *OR13G1*) were identified through this effort. Importantly, variants identified as associated to MI in the above studies remain to be validated in replication studies. Their involvement in the etiology of MI therefore remains uncertain.

Recently, genetic suscepbitiliby variants for CAD and MI were identified on chromosome 9, within a region that contains the *CDKN2A* and *CDKN2B* genes (Helgadottir, A. et al, Science 316:1491-93 (2007); McPherson, R. et al, Science 316:1488-91 (2007); The Wellcome Trust Case Control Consortium, Nature 447:661-78 (2007); Samani, NJ et al, N Engl J Med 357:443-53 (2007)). Follow-up investigations have shown that the variants, which are all located within a region of high linkage disequilibrium (LD), also contribute to risk of intracranial aneurysm and abdominal aortic aneurysm (Helgadottir, A. et al, Nature Genet 40:217-24 (2008)), showing that these variants not only contribute to MI and CAD, but also to cardiovascular disease in general.

Cardiovascular disease remains the most important cause or morbidity and mortality and there is a large unmet medical need for its prevention. Identifying the key pathways involved in MI and CAD may facilitate development of new therapeutics and diagnostics. Identification of genetic risk factors for MI and CAD may offer clinical practitioners valuable diagnostic and prognostic tools. Genetic variants that are found to be an important key to the presentation of clinical symptoms in MI may also facilitate drug development, through the identification of novel drug targets.

There is thus an unmet need for identification of additional genetic variants that confer susceptibility of CAD and MI. The present invention provides such variants.

### SUMMARY OF THE INVENTION

The present invention relates to methods of risk assessment, diagnosis and/or prognosis of coronary artery disease (CAD) and myocardial infarction (MI). Certain genetic variants have been found to be associated with risk of CAD and/or MI. The invention thus relates to methods of assessing increased susceptibility to CAD and/or MI, as well as methods of assessing a decreased susceptibility to CAD and/or MI, or determine a protection against CAD and/or MI.

In one aspect, the invention relates to a method for determining a susceptibility to myocardial infarction or coronary artery disease in a human individual from a Caucasian population, comprising determining the presence or absence of at least one allele of at least one polymorphic marker in a nucleic acid sample obtained from the individual wherein the at least one polymorphic marker is selected from the markers rs11751605, and markers in linkage disequilibrium therewith selected from the group consisting of the markers set forth in table 5, and wherein the presence of the at least one allele is indicative of a susceptibility to myocardial infarction or coronary artery disease in individuals from a Caucasian population.

The genotype dataset comprises in one embodiment information about marker identity, and the allelic status of the individual, i.e. information about the identity of the two alleles carried by the individual for the marker. The genotype dataset may comprise allelic information about one or more marker, including two or more markers, three or more markers, five or more markers, one hundred or more markers, etc. In some embodiments, the genotype dataset comprises genotype information from a whole-genome assessment of the individual including hundreds of thousands of markers, or even one million or more markers.

In one embodiment, the method of determining a susceptibility, or diagnosing a susceptibility of, coronary artery disease or myocardial infarction, further comprises assessing the frequency of at least one haplotype in the individual. In one such embodiment, the at least one haplotype is selected from haplotypes that are in linkage disequilibrium with rsll751605 (SEQ ID NO:1).

In certain embodiments of the invention, determination of the presence of at least one at-risk allele of at least one polymorphic marker in a nucleic acid sample from the individual is indicative of an increased susceptibility to coronary artery disease or myocardial infarction. In one embodiment, the increased susceptibility is characterized by a relative risk (RR) or odds ratio (OR) of at least 1.15. In another embodiment, the increased susceptibility is characterized by a relative risk (RR) or odds ratio (OR) of at least 1.20.

In some embodiments, the presence of allele C in rs11751605 is indicative of increased susceptibility of coronary artery disease or myocardial infarction in the individual.

In some other embodiments, the presence of at least one protective allele in a nucleic acid sample from the individual is indicative of a decreased susceptibility of coronary artery disease or myocardial infarction. In another embodiment, the absence of at least one at-risk allele in a nucleic acid sample from the individual is indicative of a decreased susceptibility of coronary artery disease or myocardial infarction.

Certain uses of the invention further comprise a step of screening the nucleic acid for the presence of at least one at-risk genetic variant for a coronary artery disease or myocardial infarction not associated with, i.e. not in linkage disequilibrium with, any one of the markers set forth in Table 4. Such additional genetic variants can in specific embodiments include any variant that has been identified as a susceptibility or risk variant for coronary artery disease or myocardial infarction.

Also disclosed is a method of identification of a marker for use in assessing susceptibility to coronary artery disease or myocardial infarction in human individuals in a Caucasian population, the method comprising:
identifying at least one polymorphic marker in linkage disequilibrium with rs11751605 selected from the group consisting of the markers set forth in table 5;
determining the genotype status of a sample of individuals diagnosed with, or having a susceptibility to, coronary artery disease or myocardial infarction; and
determining the genotype status of a sample of control individuals;
wherein a significant difference in frequency of at least one allele in at least one polymorphism in individuals diagnosed with, or having a susceptibility to, coronary artery disease or myocardial infarction, as compared with the frequency of the at least one allele in the control sample is indicative of the at least one polymorphism being useful for assessing susceptibility to coronary artery disease or myocardial infarction.

In one embodiment, "significant" is determined by statistical means, e.g. the difference is statistically significant. In one such embodiment, statistical significance is characterized by a P-value of less than 0.05. In other embodiments, the statistical significance is characterized a P-value of less than 0.01, less than 0.001, less than 0.0001, less than 0.00001, less than 0.000001, less than 0.0000001, less than 0.0000000001, or less than 0.00000001.

In one embodiment, an increase in frequency of the at least one allele in the at least one polymorphism in individuals diagnosed with, or having a susceptibility to, coronary artery disease or myocardial infarction, as compared with the frequency of the at least one allele in the control sample, is indicative of the at least one polymorphism being useful for assessing increased susceptibility to coronary artery disease or myocardial infarction. In another embodiment, a decrease in frequency of the at least one allele in the at least one polymorphism in individuals diagnosed with, or having a susceptibility to, a coronary artery disease or myocardial infarction, as compared with the frequency of the at least one allele in the control sample is indicative of the at least one polymorphism being useful for assessing decreased susceptibility to, or protection against, the coronary artery disease or myocardial infarction.

An application of the invention relates to a method of genotyping a nucleic acid sample obtained from a human individual, comprising determining the presence or absence of at least one allele of at least one polymorphic marker in the sample, wherein the at least one marker is selected from rs11751605, and markers in linkage disequilibrium therewith, and wherein determination of the presence or absence of the at least one allele of the at least one polymorphic marker is predictive of a susceptibility of coronary artery disease or myocardial infarction.

In one example, genotyping comprises amplifying a segment of a nucleic acid that comprises the at least one polymorphic marker by Polymerase Chain Reaction (PCR), using a nucleotide primer pair flanking the at least one polymorphic marker. In another embodiment, genotyping is performed using a process selected from allele-specific probe hybridization, allele-specific primer extension, allele-specific amplification, nucleic acid sequencing, 5'-exonuclease digestion, molecular beacon assay, oligonucleotide ligation assay, size analysis, and single-stranded conformation analysis. In one particular embodiment, the process comprises allele-specific probe hybridization. In another embodiment, the process comprises DNA sequencing. In a preferred embodiment, the method comprises:
1) contacting copies of the nucleic acid with a detection oligonucleotide probe and an enhancer oligonucleotide probe under conditions for specific hybridization of the oligonucleotide probe with the nucleic acid;
   wherein
   a) the detection oligonucleotide probe is from 5-100 nucleotides in length and specifically hybridizes to a first segment of the nucleic acid whose nucleotide sequence is given by any one of SEQ ID NO:1-172 that comprises at least one polymorphic site;
   b) the detection oligonucleotide probe comprises a detectable label at its 3' terminus and a quenching moiety at its 5' terminus;
   c) the enhancer oligonucleotide is from 5-100 nucleotides in length and is complementary to a second segment of the nucleotide sequence that is 5' relative to the oligonucleotide probe, such that the enhancer oligonucleotide is located 3' relative to the detection oligonucleotide probe when both oligonucleotides are hybridized to the nucleic acid; and
   d) a single base gap exists between the first segment and the second segment, such that when the oligonucleotide probe and the enhancer oligonucleotide probe are both hybridized to the nucleic acid, a single base gap exists between the oligonucleotides;
2) treating the nucleic acid with an endonuclease that will cleave the detectable label from the 3' terminus of the detection probe to release free detectable label when the detection probe is hybridized to the nucleic acid; and
3) measuring free detectable label, wherein the presence of the free detectable label indicates that the detection probe specifically hybridizes to the first segment of the nucleic acid, and indicates the sequence of the polymorphic site as the complement of the detection probe.

In a particular embodiment, the copies of the nucleic acid are provided by amplification by Polymerase Chain Reaction (PCR). In another embodiment, the susceptibility determined is increased susceptibility. In another embodiment, the susceptibility determined is decreased susceptibility.

Another utility of the invention relates to a method of assessing an individual for probability of response to a therapeutic agent for preventing and/or ameliorating symptoms associated with coronary artery disease or myocardial infarction, comprising: determining the presence or absence of at least one allele of at least one polymorphic marker in a nucleic acid sample obtained from the individual, wherein the at least one polymorphic marker is selected from the markers set forth in Table 4, and markers in linkage disequilibrium therewith, wherein determination of the presence of the at least one allele of the at least one marker is indicative of a probability of a positive response to coronary artery disease or myocardial infarction therapeutic agent. In one embodiment, the at least one polymorphic marker is selected from marker rs11751605, and markers in linkage disequilibrium therewith. In one embodiment, the therapeutic agent is selected from beta blockers, anticoagulation agents, including heparin and/or low molecular weigth heparin, antiplatelet agents, such as clopidogrel, aspirin, beta blockers, including metoprolol and carvedilol, ACE inhibitors, Statins, Aldosterone antagonists, including eplerenone, leukotriene synthesis inhibitors, the agents set forth in Agent Table I, Agent Table II, (R)-(+)-alpha-cyclopentyl-4-(2-quinolinylmethoxy)-Benzeneacetic acid, atreleuton, and 4-{(S)-2-[4-(4-Chloro-phenoxy)-phenoxymethyl]-pyrrolidin-1-yl}-butyramide, also known as DG-051. Other embodiments may include any one or a combination of the therapeutic agents described herein to be useful for therapeutic intervention of coronary artery disease or myocardial infarction.

Yet another utility of the invention relates to a method of predicting prognosis of an individual diagnosed with coronary artery disease or myocardial infarction, the method comprising determining the presence or absence of at least one allele of at least one polymorphic marker in a nucleic acid sample obtained from the individual, wherein the at least one polymorphic marker is selected from the group consisting of the markers set forth in Table 3, and markers in linkage disequilibrium therewith, wherein determination of the presence of the at least one allele is indicative of a worse prognosis of coronary artery disease or myocardial infarction in the individual. The prognosis may in certain embodiment relate to susceptibility of recurrent MI events, recurrent stroke events, or susceptibility to other complications relating to coronary artery disease or myocardial infarction.

A further utility of the invention relates to a method of monitoring progress of a treatment of an individual undergoing treatment for coronary artery disease or myocardial infarction, the method comprising determining the presence or absence of at least one allele of at least one polymorphic marker in a nucleic acid sample obtained from the individual, wherein the at least one polymorphic marker is selected from the group consisting of the markers set forth in Table 3, and markers in linkage disequilibrium therewith, wherein determination of the presence of the at least one allele is indicative of the treatment outcome of the individual. The treatment may in certain embodiments be surgical treatment. In other embodiments, the treatment is by administration of a therapeutic agent, optionally including lifestyle changes or alterations in environmental exposure to risk factors for coronary artery disease or myocardial infarction, as described further herein.

In one embodiment, the method further comprises assessing at least one biomarker in a sample from the individual. The biomarker is in certain embodiments a cardiac marker or an inflammatory marker. In one embodiment, the at least one biomarker is selected from creatin kinase, troponin, glycogen phosphorylase, C-reactive protein (CRP), serum amyloid A, fibrinogen, interleukin-6, tissue necrosis factor-alpha, soluble vascular cell adhesion molecules (sVCAM), soluble intervascular adhesion molecules (sICAM), E-selectin, matrix metalloprotease type-1, matrix metalloprotease type-2, matrix metalloprotease type-3, matrix metalloprotease type-9, serum sCD40L, leukotrienes, leukotriene metabolites, interleukin-6, tissue necrosis factor-alpha, myeloperoxidase (MPO), and N-tyrosine. In one embodiment, the leukotriene is selected from LTB4, LTC4, LTD4 and LTE4. In another embodiment, the method further comprises analyzing non-genetic information to make risk assessment, diagnosis, or prognosis of the individual. The non-genetic information is in one embodiment selected from age, gender, ethnicity, socioeconomic status, previous disease diagnosis, medical history of subject, family history of coronary artery disease or myocardial infarction, biochemical measurements, and clinical measurements. In a particular preferred embodiment, a further step comprising calculating overall risk is employed.

Another utility of the invention relates to analyzing a sample comprising genomic DNA from a human individual or a genotype dataset derived from a human individual for the presence or absence of at least one at-risk allele of at least one at-risk variant for coronary artery disease or myocardial infarction not in linkage disequilibrium with any one of the markers set forth. Thus, the variants described herein to be associated with coronary artery disease or myocardial infarction may be combined with other genetic variants for coronary artery disease or myocardial infarction, that are not genetically related (i.e., not in linkage disequilibrium with) the markers described herein. Such analysis may be undertaken in combination with any of the methods described herein. Furthermore any two markers herein, or any other combination of markers and/or haplotypes described herein to be associated with coronary artery disease or myocardial infarction may be combined to assess an increased susceptibility to coronary artery disease or myocardial infarction.

In some embodiments of the methods of the invention, non-genetic information is analyzed, to make risk assessment, diagnosis, or prognosis of the individual. The non-genetic information is in certain embodiments selected from age, gender, ethnicity, socioeconomic status, previous disease diagnosis, medical history of subject, family history of coronary artery disease or myocardial infarction, biochemical measurements, and clinical measurements. Combined genetic factors and/or combinations of genetic and non-genetic factors may be analyzed by known methods, to generate a combined risk.

Also disclosed is a kit for assessing susceptibility to a coronary artery disease or myocardial infarction in a human individual, the kit comprising reagents for selectively detecting the presence or absence of at least one allele of at least one polymorphic marker in the genome of the individual, wherein the polymorphic marker is selected from the markers set forth herein, and wherein the presence of the at least one allele is indicative of a susceptibility to a coronary artery disease or myocardial infarctions.

In one example, the reagents comprise at least one contiguous oligonucleotide that hybridizes to a fragment of the genome of the individual comprising the at least one polymorphic marker, a buffer and a detectable label. In one embodiment, the reagents comprise at least one pair of oligonucleotides that hybridize to opposite strands of a genomic nucleic acid segment obtained from the subject, wherein each oligonucleotide primer pair is designed to selectively amplify a fragment of the genome of the individual that includes one polymorphic marker, and wherein the fragment is at least 30 base pairs in size. In a particular embodiment the at least one oligonucleotide is completely complementary to the genome of the individual. In another embodiment, the at least one oligonucleotide can comprise at least one mismatch to the genome of the individual. In one embodiment, the oligonucleotide is about 18 to about 50 nucleotides in length. In another embodiment, the oligonucleotide is 20-30 nucleotides in length.

In one example, the kit comprises:
a detection oligonucleotide probe that is from 5-100 nucleotides in length; an enhancer oligonucleotide probe that is from 5-100 nucleotides in length; and an endonuclease enzyme;
wherein the detection oligonucleotide probe specifically hybridizes to a first segment of the nucleic acid whose nucleotide sequence is given by any one of SEQ ID NO:1-172 that comprises at least one polymorphic site; and wherein the detection oligonucleotide probe comprises a detectable label at its 3' terminus and a quenching moiety at its 5' terminus; wherein the enhancer oligonucleotide is from 5-100 nucleotides in length and is complementary to a second segment of the nucleotide sequence that is 5' relative to the oligonucleotide probe, such that the enhancer oligonucleotide is located 3' relative to the detection oligonucleotide probe when both oligonucleotides are hybridized to the nucleic acid; wherein a single base gap exists between the first segment and the second segment, such that when the oligonucleotide probe and the enhancer oligonucleotide probe are both hybridized to the nucleic acid, a single base gap exists between the oligonucleotides; and wherein treating the nucleic acid with the endonuclease will cleave the detectable label from the 3' terminus of the detection probe to release free detectable label when the detection probe is hybridized to the nucleic acid.

Also disclosed is the use of an oligonucleotide probe in the manufacture of a diagnostic reagent for diagnosing and/or assessing susceptibility to coronary artery disease or myocardial infarction in a human individual, wherein the probe hybridizes to a segment of a nucleic acid whose nucleotide sequence is given by any one of SEQ ID NO:1-172 that comprises at least one polymorphic site, wherein the fragment is 15-500 nucleotides in length. In one embodiment, the polymorphic site is selected from the polymorphic markers rs11751605, and markers in linkage disequilibrium therewith.

Another aspect relates to an apparatus for determining a genetic indicator for coronary artery disease or myocardial infarction in a human individual, comprising: a computer readable memory; and a routine stored on the computer readable memory; wherein the routine is adapted to be executed on a processor to analyze marker and/or haplotype information for at least one human individual with respect to at least one polymorphic marker selected from rs11751605, and markers in linkage disequilibrium therewith selected from the group consisting of the markers set forth in table 5, and generate an output based on the marker or haplotype information, wherein the output comprises a risk measure of the at least one marker or haplotype as a genetic indicator of coronary artery disease or myocardial infarction for the human individual.

In one embodiment, the routine further comprises an indicator of the frequency of at least one allele of at least one polymorphic marker or at least one haplotype in a plurality of individuals diagnosed with coronary artery disease or myocardial infarction, and an indicator of the frequency of at the least one allele of at least one polymorphic marker or at least one haplotype in a plurality of reference individuals, and wherein a risk measure is based on a comparison of the at least one marker and/or haplotype status for the human individual to the indicator of the frequency of the at least one marker and/or haplotype information for the plurality of individuals diagnosed with coronary artery disease or myocardial infarction.

The present invention, as described herein, may be reduced to practice using any one, or a combination of, the polymorphic markers described herein as being useful for the determination of a susceptibility to coronary artery disease or myocardial infarction. This includes markers that are shown herein to be associated with coronary artery disease or myocardial infarction, but also includes markers that are in linkage disequilibrium with such variants. In one embodiment, the at least one marker is rs11751605 (SEQ ID NO:1). In these embodiments, the at least one marker may optionally be selected from markers in linkage disequilibrium with rs11751605 selected from the markers in Table 5.

The present invention relates to method, or apparatus useful for detecting or determining a susceptibility to myocardial infarction or coronary artery disease. It is however contemplated that the variants described herein to be correlated with risk of developing myocardial infarction or coronary artery disease may also be risk variants for other cardiovascular diseases. This includes Stroke, Peripheral Artery Disease, Restenosis, Intracranial Aneurysm and Aorta Abdominal Aneurysm. The Restenosis phenotype may for example be Coronary In-stent Restenosis. The In-stent Restenosis may further be either Restenosis following Bare Metal Stent (BMS) placement, or Restenosis following placement of a Drug Eluting Stent (DES).

In certain embodiments of the methods, and apparatus of the invention, linkage disequilibrium is characterized by specific cutoff values for a quantitative measure of linkage disequilibrium. In one such embodiment, linkage disequilibrium is characterized by specific numerical values, or ranges of numerical values, for r². In another such embodiment, linkage disequilibrium is characterized by specific numerical values, or ranges of numerical values, for |D'|. In yet another embodiment, linkage disequilibrium is characterized by specific numerical values for r² and |D'|, or ranges thereof. In one preferred embodiment, linkage disequilibrium is characterized by values for r² of greater than 0.1. In another preferred embodiment, linkage disequilibrium is characterized by values for r² of greater than 0.2. Other cutoff values for r² are also possible, including, but not limited to, 0.3, 0.4, 0.5, 0.6, 0.7, 0.8, 0.9, 0.95, 0.96, 0.97, 0.98, 0.99. In another preferred embodiment, linkage disequilibrium is characterized by values for |D'| of greater than 0.5. In another preferred embodiment, linkage disequilibrium is characterized by values for |D'| of greater than 0.8. Other cutoff values for |D'| are also possible, including, but not limited to, 0.2, 0.3, 0.4, 0.6, 0.7, 0.8, 0.9, 0.95, 0.96, 0.97, 0.98 and 0.99. In certain embodiments, linkage disequilibrium is characterized by numeric cutoff values for either |D'| and r². In one such embodiment linkage disequilibrium is characterized by numerical values for either |D'| of greater than 0.8 and r² of greater than 0.2, or both.

In certain other embodiments of the method, or apparatus of the invention, the individual is of a specific human ancestry, Caucasian ancestry. The ancestry is in certain embodiment self-reported by the individual who undergoes genetic analysis or genotyping. In other embodiments, the ancestry is determined by genetic determination comprising detecting at least one allele of at least one polymorphic marker in a nucleic acid sample from the individual, wherein the presence or absence of the allele is indicative of the ancestry of the individual.

In other particular embodiments of the method, and apparatus of the invention, the presence of at least one at-risk variant, i.e. an at-risk allele in at least one polymorphic marker or an at-risk haplotype, is indicative of an early onset of coronary artery disease or myocardial infarction. Early onset is in some embodiments categorized as onset before age 75. In other embodiments, early onset is categorized as onset before age 70, before age 65, before age 60, before age 55, before age 50, before age 45, or before age 40. Other values for categorization of age at onset are also contemplated, including, but not limited to, all integer values of age, and such age categories are also within scope of the invention. In certain embodiments, the age at onset is below 50 for males and/or below 60 for females.

### DETAILED DESCRIPTION OF THE INVENTION

### Definitions

Unless otherwise indicated, nucleic acid sequences are written left to right in a 5' to 3' orientation. Numeric ranges recited within the specification are inclusive of the numbers defining the range and include each integer or any non-integer fraction within the defined range. Unless defined otherwise, all technical and scientific terms used herein have the same meaning as commonly understood by the ordinary person skilled in the art to which the invention pertains.

The following terms shall, In the present context, have the meaning as indicated:
A "polymorphic marker", sometime referred to as a "marker", as described herein, refers to a genomic polymorphic site. Each polymorphic marker has at least two sequence variations characteristic of particular alleles at the polymorphic site. Thus, genetic association to a polymorphic marker implies that there is association to at least one specific allele of that particular polymorphic marker. The marker can comprise any allele of any variant type found in the genome, including SNPs, mlcrosatellites, insertions, deletions, duplications and translocations.
An "allele" refers to the nucleotide sequence of a given locus (position) on a chromosome. A polymorphic marker allele thus refers to the composition (i.e., sequence) of the marker on a chromosome. Genomic DNA from an individual contains two alleles (*e.g.,* allele-specific sequences) for any given polymorphic marker, representative of each copy of the marker on each chromosome. Sequence codes for nucleotides used herein are: A = 1, C = 2, G = 3, T = 4. For microsatellite alleles, the CEPH sample (Centre d'Etudes du Polymorphisme Humain, genomics repository, CEPH sample 1347-02) is used as a reference, the shorter allele of each microsatellite in this sample is set as 0 and all other alleles in other samples are numbered in relation to this reference. Thus, e.g., allele 1 is 1 bp longer than the shorter allele in the CEPH sample, allele 2 is 2 bp longer than the shorter allele in the CEPH sample, allele 3 is 3 bp longer than the lower allele in the CEPH sample, etc., and allele -1 is 1 bp shorter than the shorter allele in the CEPH sample, allele -2 is 2 bp shorter than the shorter allele in the CEPH sample, etc.

Sequence conucleotide ambiguity as described herein is as proposed by IUPAC-IUB. These codes are compatible with the codes used by the EMBL, GenBank, and PIR databases.

| **IUB code** | **Meaning** |
|---|---|
| A | Adenosine |
| C | Cytidine |
| G | Guanine |
| T | Thymidine |
| R | G or A |
| Y | T or C |
| K | G orT |
| M | A or C |
| S | G or C |
| W | A or T |
| B | C G or T |
| D | A G or T |
| H | A C or T |
| V | A C or G |
| N | A C G or T (Any base) |

A nucleotide position at which more than one sequence is possible in a population (either a natural population or a synthetic population, *e.g.,* a library of synthetic molecules) is referred to herein as a "polymorphic site".

A "Single Nucleotide Polymorphism" or "SNP" is a DNA sequence variation occurring when a single nucleotide at a specific location in the genome differs between members of a species or between paired chromosomes in an individual. Most SNP polymorphisms have two alleles. Each individual is in this instance either homozygous for one allele of the polymorphism (i.e. both chromosomal copies of the individual have the same nucleotide at the SNP location), or the individual is heterozygous (i.e. the two sister chromosomes of the individual contain different nucleotides). The SNP nomenclature as reported herein refers to the official Reference SNP (rs) ID identification tag as assigned to each unique SNP by the National Center for Biotechnological Information (NCBI).

A "variant", as described herein, refers to a segment of DNA that differs from the reference DNA. A "marker" or a "polymorphic marker", as defined herein, is a variant. Alleles that differ from the reference are referred to as "variant" alleles.

A "microsatellite" is a polymorphic marker that has multiple small repeats of bases that are 2-8 nucleotides in length (such as CA repeats) at a particular site, in which the number of repeat lengths varies in the general population. An "indel" is a common form of polymorphism comprising a small insertion or deletion that is typically only a few nucleotides long.

A "haplotype," as described herein, refers to a segment of genomic DNA that is characterized by a specific combination of alleles arranged along the segment. For diploid organisms such as humans, a haplotype comprises one member of the pair of alleles for each polymorphic marker or locus. In a certain embodiment, the haplotype can comprise two or more alleles, three or more alleles, four or more alleles, or five or more alleles.

The term "susceptibility", as described herein, refers to the proneness of an individual towards the development of a certain state (*e.g.,* a certain trait, phenotype or disease), or towards being less able to resist a particular state than the average individual. The term encompasses both increased susceptibility and decreased susceptibility. Thus, particular polymorphic markers and/or haplotypes of the invention may be characteristic of increased susceptibility (i.e., increased risk) of coronary artery disease and myocardial infarction, as characterized by a relative risk (RR) or odds ratio (OR) of greater than one for the particular allele or haplotype. Alternatively, the markers and/or haplotypes of the invention are characteristic of decreased susceptibility (i.e., decreased risk) of coronary artery disease and myocardial infarction, as characterized by a relative risk of less than one.

The term "and/or" shall in the present context be understood to indicate that either or both of the items connected by it are involved. In other words, the term herein shall be taken to mean "one or the other or both".

The term "look-up table", as described herein, is a table that correlates one form of data to another form, or one or more forms of data to a predicted outcome to which the data is relevant, such as phenotype or trait. For example, a look-up table can comprise a correlation between allelic data for at least one polymorphic marker and a particular trait or phenotype, such as a particular disease diagnosis, that an individual who comprises the particular allelic data is likely to display, or is more likely to display than individuals who do not comprise the particular allelic data. Look-up tables can be multidimensional, *i.e.* they can contain information about multiple alleles for single markers simultaneously, or the can contain information about multiple markers, and they may also comprise other factors, such as particulars about diseases diagnoses, racial information, biomarkers, biochemical measurements, therapeutic methods or drugs, etc.

A "computer-readable medium", is an information storage medium that can be accessed by a computer using a commercially available or custom-made interface. Exemplary compute-readable media include memory (*e.g.,* RAM, ROM, flash memory, etc.), optical storage media (*e.g.,* CD-ROM), magnetic storage media (*e.g.,* computer hard drives, floppy disks, etc.), punch cards, or other commercially available media. Information may be transferred between a system of interest and a medium, between computers, or between computers and the computer-readable medium for storage or acess of stored information. Such transmission can be electrical, or by other available methods, such as IR links, wireless connections, etc.

A "nucleic acid sample", as described herein, refers to a sample obtained from an individuals that contains nucleic acid. In certain embodiments, i.e. the detection of specific polymorphic markers and/or haplotypes, the nucleic acid sample comprises genomic DNA. Such a nucleic acid sample can be obtained from any source that contains genomic DNA, including as a blood sample, sample of amniotic fluid, sample of cerebrospinal fluid, or tissue sample from skin, muscle, buccal or conjunctival mucosa, placenta, gastrointestinal tract or other organs.

The term "CAD and/or MI therapeutic agent", as described herein, refers to an agent that can be used to ameliorate or prevent symptoms associated with coronary artery disease or myocardial infarction. Such agents can for example be statins, beta blockers, calcium channel blockers, cardiac glycosides, antihypertensive agents, diuretics, agents acting on the renin-angiotensin system, and aspirin, or other therapeutic agents as described herein.

The term "CAD and/or MI - associated nucleic acid", as described herein, refers to a nucleic acid that has been found to be associated to coronary artery disease and myocardial infarction. This includes, but is not limited to, the markers and haplotypes described herein and markers and haplotypes in linkage disequilibrium (LD) therewith.

The term "early onset", as described herein, refers to onset of a disease that is lower than is typically observed. Fore example, in certain embodiments as applied to the MI phenotype, "early onset" can be defined as a MI event before the age of 50 for males and before the age of 60 for females. However, the term can be defined using other age cutoffs, as is deemed appopriate by the skilled person, and should in the present context be taken to mean age of onset of the disease that is lower than one would typically observe, and consequently considered early.

### Association of genetic variants to coronary artery disease and myocardial infarction

Through a genome-wide association search for variants contributing to increased risk of deloping coronary artery disease (CAD) and myocardial infarction (MI), a number of SNP variants have been found to be associated with MI and CAD. These variants, (see Table 1), and variants in linkage disequilibrium with the identified variants, can be used to assess risk of developing CAD or MI in an individual, by determining the genotype of the individual for the individual variants. Haplotypes comprising the variants of the present invention, or haplotypes in LD with the variants of the invention, can also be used to assess risk of developing CAD or MI.

The genome-wide scan was expanded to a larger discovery cohort and the analysis repeated (Table 3). Most of the SNPs showing association in the discovery sample were investigated in six replication cohorts, from Durham (NC), Atlanta (GA), Philadelphia (PA), and Johns Hopkins (MD) in the United States, from Italy and from the Wellcome Trust Case Control Consortium (WTCCC). As shown in Table 4, several variants show evidence for association in the replication samples, in particular markers rs11751605, rs6076623, rs1412444, rs2163612 and rs1029396. These markers are therefore particularly useful in the methods, uses, apparatus and kits described further herein. The association of marker rs11751605 is particularly significant; the overall P-value of association for this marker to CAD is 1.16 x 10⁻⁸, which is genome-wide significant after correcting for the number of tests performed (Bonferroni correction gives 0.05/300,000 ~1.7 x 10⁻⁷).

Markers in linkage disequilibrium with any one of rs11751605, rs6076623, rs1412444, rs2163612 and rs1029396 are also useful in the methods, uses, kits and apparatus of the invention. Markers in LD with rs11751605 are particularly useful. Surrogate markers for rs11751605, based on r² values in the Hapmap Caucasian CEU sample of greater than 0.2).

The marker rs11751605 is located on Chromosome 6, near the 5' end of the Lipoprotein(a) gene (also called Lp(a)). The *Lp(a)* gene encodes a lipoprotein in a subclass consisting of an LDL-like particle and the specific apolipoprotein(a) [apo(a)], which is covalently bound to the apoB of the LDL-like particle.

Apo(a) proteins vary in size due to a size polymorphism [KIV-2 VNTR], which is caused by a variable number of so called kringle IV repeats in the LPA gene. These variable apo(a) sizes are known as "apo(a) isoforms". There is a general inverse correlation between the size of the apo(a)isoform and the Lp(a) plasma concentration

Lp(a) concentrations are highly heritable and vary over one thousandfold between individuals, from < 0.2 to > 200 mg/dL. Lp(a) plasma concentrations is mainly controlled by the apolipoprotein(a) gene (LPA) located on chromosome 6q26-27. However, the apo(a) size polymorphism does not explain all the interindividual variation in Lp(a) levels in plasma. For alleles with identical number of K IV repeats, the Lp(a) level can vary by a factor of 200. Additional polymorphisms (independent from the K IV size polymorphism) in the gene have been shown to affect the Lp(a) concentration (Human Molecular Genetics, 2001, 10: 815-824). Therefore, the genetic determination of Lp(a) levels is highly complex and currently not fully understood.

Lipoprotein structure is similar to plasminogen and tPA (tissue plasminogen activator) and it has been shown to affect (reduce) fibrinolysis and (increase) thrombogenesis. In addition, the LDL cholesterol content of Lp-a contributes to atherosclerosis.

High Lp(a) in blood has been shown to be a risk factor for coronary heart disease (CHD), cerebrovascular disease (CVD), atherosclerosis, thrombosis, and stroke. However, few reported studies have investigated the association between genetic variants that affect Lp(a) concentration and coronary artery disease. The frequency of small apo(a) isoforms has been shown to be higher in patients with myocardial infarction compared with healthy controls (Atherosierosis 144:323-333 (1999)) and another study has shown the isoforms to be higher in those with advanced atherosclerosis of the carotid arteries compared to those without advanced atherosclerosis (Circulation 100:1154-1160 (1999)).

A recent study reported that a single nucleotide polymorphism (rs3798220) in the coding sequence of the LPA gene associated with severe coronary artery disease and Lp(a) levels (Arterioscler Thromb Vasc Biol 27:2030-2036 (2007)). The rs11751605 marker is however not correlated with rs3798220. In a sample of over 5000 Icelanders genotyped for both markers, the at-risk allele of rs3798220 does not lie on the haplotype background of the risk allele rs11751605 (r²=0.002). In other words, the at-risk allele of rs3798220 and the at-risk allele of rs11751605 reside on different chromosomal backgrounds, and they are thus not inherited together. As a consequence, these signals are unrelated. The allelic frequency of rs11751605 allele C (the at-risk allele) is 11%, the frequency of rs3798220 allele C (the reported at-risk allele) is 1.5%, whereas their joint frequency is zero.

Thus, the association observed for rs11751605 is clearly not due to, or correlated to, rs3798220. Moreover, the rs3798220 C allele found to associate with Lp(a) levels is very rare (1.5%), whereas the at-risk C allele of rs11751605 is relatively common (11%), thus explaining a higher proportion of the risk conferred by the variant for MI and CAD susceptibility. It is at present not known if the rs11751605 marker correlates with Lp(a) levels in plasma.

It is important to note that correlation of a certain disease (MI or CAD, for example) to certain biological risk factors (biomarkers or related traits) does not, in general translate into association of the disease to markers in the gene encoding the biomarker. Moreover, genetic risk for a confounding trait or disease, or a known risk factor for a disease, does not necessarily translate into shared genetic effects. Many such examples are known from diseases such as type 2 diabetes, LDL cholesterol and obesity, which all have observable effects on the risk of CAD. For example, the strongest genetic risk factor for type II diabetes (in the TCF7L2 gene) does not show any association with MI. The markers rs693 and rs562338, located within the ApoB locus, have been shown to strongly correlate with LDL cholesterol levels (Nature Genet. 40:161-9(2008)), yet these markers do not show any association with CAD. In addition, genetic markers within the FTO gene that have been shown to associate with obesity are not associated with CAD.

Thus, even in light of the prior knowledge of the correlation of Lp(a) levels with risk of CAD and MI, and the identification of genetic variants within the *Lp(a)* gene with Lp(a) levels, it would by no means have been obvious that genetic variants within the gene confer risk of MI or CAD. If this were the case, such a genetic relationship would presumably have been established much earlier. The present finding of an association between rs11751605 and risk of CAD and MI is thus very surprising.

### Assessment for markers and haplotypes

The genomic sequence within populations is not identical when individuals are compared. Rather, the genome exhibits sequence variability between individuals at many locations in the genome. Such variations in sequence are commonly referred to as polymorphisms, and there are many such sites within each genome. For example, the human genome exhibits sequence variations which occur on average every 500 base pairs. The most common sequence variant consists of base variations at a single base position in the genome, and such sequence variants, or polymorphisms, are commonly called Single Nucleotide Polymorphisms ("SNPs"). These SNPs are believed to have occurred in a single mutational event, and therefore there are usually two possible alleles possible at each SNPsite; the original allele and the mutated allele. Due to natural genetic drift and possibly also selective pressure, the original mutation has resulted in a polymorphism characterized by a particular frequency of its alleles in any given population. Many other types of sequence variants are found in the human genome, including microsatellites, insertions, deletions, inversions and copy number variations. A polymorphic microsatellite has multiple small repeats of bases (such as CA repeats, TG on the complimentary strand) at a particular site in which the number of repeat lengths varies in the general population. In general terms, each version of the sequence with respect to the polymorphic site represents a specific allele of the polymorphic site. These sequence variants can all be referred to as polymorphisms, occurring at specific polymorphic sites characteristic of the sequence variant in question. In general terms, polymorphisms can comprise any number of specific alleles. Thus in one embodiment of the invention, the polymorphism is characterized by the presence of two or more alleles in any given population. In another embodiment, the polymorphism is characterized by the presence of three or more alleles. In other embodiments, the polymorphism is characterized by four or more alleles, five or more alleles, six or more alleles, seven or more alleles, nine or more alleles, or ten or more alleles. All such polymorphisms can be utilized in the methods and kits of the present invention, and are thus within the scope of the invention.

In some instances, reference is made to different alleles at a polymorphic site without choosing a reference allele. Alternatively, a reference sequence can be referred to for a particular polymorphic site. The reference allele is sometimes referred to as the "wild-type" allele and it usually is chosen as either the first sequenced allele or as the allele from a "non-affected" individual (*e.g.,* an individual that does not display a trait or disease phenotype). Alleles for SNP markers as referred to herein refer to the bases A, C, G or T as they occur at the polymorphic site in the SNP assay employed. The allele codes for SNPs used herein are as follows: 1= A, 2=C, 3=G, 4=T. The person skilled in the art will however realise that by assaying or reading the opposite DNA strand, the complementary allele can in each case be measured. Thus, for a polymorphic site (polymorphic marker) containing an A/G polymorphism, the assay employed may either measure the percentage or ratio of the two bases possible, i.e. A and G. Alternatively, by designing an assay that determines the opposite strand on the DNA template, the percentage or ratio of the complementary bases T/C can be measured. Quantitatively (for example, in terms of relative risk), identical results would be obtained from measurement of either DNA strand (+ strand or - strand).

Typically, a reference sequence is referred to for a particular sequence. Alleles that differ from the reference are referred to as "variant" alleles. A variant sequence, as used herein, refers to a sequence that differs from the reference sequence but is otherwise substantially similar. Alleles at the polymorphic genetic markers that make up the haplotypes described herein are variants. Additional variants can include changes that affect a polypeptide. Sequence differences, when compared to a reference nucleotide sequence, can include the insertion or deletion of a single nucleotide, or of more than one nucleotide, resulting in a frame shift; the change of at least one nucleotide, resulting in a change in the encoded amino acid; the change of at least one nucleotide, resulting in the generation of a premature stop codon; the deletion of several nucleotides, resulting in a deletion of one or more amino acids encoded by the nucleotides; the insertion of one or several nucleotides, such as by unequal recombination or gene conversion, resulting in an interruption of the coding sequence of a reading frame; duplication of all or a part of a sequence; transposition; or a rearrangement of a nucleotide sequence, as described in detail herein. Such sequence changes alter the polypeptide encoded by the nucleic acid. For example, if the change in the nucleic acid sequence causes a frame shift, the frame shift can result in a change in the encoded amino acids, and/or can result in the generation of a premature stop codon, causing generation of a truncated polypeptide. Alternatively, a polymorphism associated with coronary artery disease and myocardial infarction or a susceptibility to coronary artery disease and myocardial infarction can be a synonymous change in one or more nucleotides (*i.e.,* a change that does not result in a change in the amino acid sequence). Such a polymorphism can, for example, alter splice sites, affect the stability or transport of mRNA, or otherwise affect the transcription or translation of an encoded polypeptide. It can also alter DNA to increase the possibility that structural changes, such as amplifications or deletions, occur at the somatic level. The polypeptide encoded by the reference nucleotide sequence is the "reference" polypeptide with a particular reference amino acid sequence, and polypeptides encoded by variant alleles are referred to as "variant" polypeptides with variant amino acid sequences.

A haplotype refers to a segment of DNA that is characterized by a specific combination of alleles arranged along the segment. For diploid organisms such as humans, a haplotype comprises one member of the pair of alleles for each polymorphic marker or locus. In a certain embodiment, the haplotype can comprise two or more alleles, three or more alleles, four or more alleles, or five or more alleles, each allele corresponding to a specific polymorphic marker along the segment. Haplotypes can comprise a combination of various polymorphic markers, *e.g.,* SNPs and microsatellites, having particular alleles at the polymorphic sites. The haplotypes thus comprise a combination of alleles at various genetic markers.

Detecting specific polymorphic markers and/or haplotypes can be accomplished by methods known in the art for detecting sequences at polymorphic sites. For example, standard techniques for genotyping for the presence of SNPs and/or microsatellite markers can be used, such as fluorescence-based techniques (*e.g.,* Chen, X. et al., Genome Res. 9(5): 492-98 (1999); Kutyavin et al., Nucleic Acid Res. 34:e128 (2006)), utilizing PCR, LCR, Nested PCR and other techniques for nucleic acid amplification. Specific methodologies available for SNP genotyping include, but are not limited to, TaqMan genotyping assays and SNPlex platforms (Applied Biosystems), mass spectrometry (e.g., MassARRAY system from Sequenom), minisequencing methods, real-time PCR, Bio-Plex system (BioRad), CEQ and SNPstream systems (Beckman), Molecular Inversion Probe array technology (e.g., Affymetrix GeneChip), and BeadArray Technologies (e.g., Illumina GoldenGate and Infinium assays). By these or other methods available to the person skilled in the art, one or more alleles at polymorphic markers, including microsatellites, SNPs or other types of polymorphic markers, can be identified.

In certain methods described herein, an individual who is at an increased susceptibility (i.e., at risk) for Coronary Artery Aisease and myocardial infarction is an individual in whom at least one specific allele at one or more polymorphic marker or haplotype conferring increased susceptibility for Coronary Artery Disease and myocardial infarction is identified (i.e., at-risk marker alleles or haplotypes). In one aspect, the at-risk marker or haplotype is one that confers a significant increased risk (or susceptibility) of coronary artery disease and myocardial infarction. In one embodiment, significance associated with a marker or haplotype is measured by a relative risk (RR). In another embodiment, significance associated with a marker or haplotye is measured by an odds ratio (OR). In a further embodiment, the significance is measured by a percentage. In one embodiment, a significant increased risk is measured as a risk (relative risk and/or odds ratio) of at least 1.2, including but not limited to: at least 1.2, at least 1.3, at least 1.4, at least 1.5, at least 1.6, at least 1.7, 1.8, at least 1.9, at least 2.0, at least 2.5, at least 3.0, at least 4.0, and at least 5.0. In a particular embodiment, a risk (relative risk and/or odds ratio) of at least 1.2 is significant. In another particular embodiment, a risk of at least 1.3 is significant. In yet another embodiment, a risk of at least 1.4 is significant. In a further embodiment, a relative risk of at least 1.5 is significant. In another further embodiment, a significant increase in risk is at least 1.7 is significant. However, other cutoffs are also contemplated, *e.g.,* at least 1.15, 1.25, 1.35, and so on, and such cutoffs are also within scope of the present invention. In other embodiments, a significant increase in risk is at least about 20%, including but not limited to about 25%, 30%, 35%, 40%, 45%, 50%, 55%, 60%, 65%, 70%, 75%, 80%, 85%, 90%, 95%, 100%, 150%, 200%, 300%, and 500%. In one particular embodiment, a significant increase in risk is at least 20%. In other embodiments, a significant increase in risk is at least 30%, at least 40%, at least 50%, at least 60%, at least 70%, at least 80%, at least 90% and at least 100%. Other cutoffs or ranges as deemed suitable by the person skilled in the art to characterize the invention are however also contemplated, and those are also within scope of the present invention. In certain embodiments, a significant increase in risk is characterized by a p-value, such as a p-value of less than 0.05, less than 0.01, less than 0.001, less than 0.0001, less than 0.00001, less than 0.000001, less than 0.0000001, less than 0.00000001, or less than 0.000000001.

An at-risk polymorphic marker or haplotype of the present invention is one where at least one allele of at least one marker or haplotype is more frequently present in an individual at risk for the disease or trait (affected), compared to the frequency of its presence in a comparison group (control), and wherein the presence of the marker or haplotype is indicative of susceptibility to coronary artery disease and/or myocardial infarction. The control group may in one embodiment be a population sample, i.e. a random sample from the general population. In another embodiment, the control group is represented by a group of individuals who are disease-free. Such disease-free control may in one embodiment be characterized by the absence of one or more specific disease-associated symptoms. In another embodiment, the disease-free control group is characterized by the absence of one or more disease-specific risk factors. Such risk factors are in one embodiment at least one environmental risk factor. Representative environmental factors are natural products, minerals or other chemicals which are known to affect, or contemplated to affect, the risk of developing the specific disease or trait. Other environmental risk factors are risk factors related to lifestyle, including but not limited to food and drink habits, geographical location of main habitat, and occupational risk factors. In another embodiment, the risk factors comprise at least one additional genetic risk factor.

As an example of a simple test for correlation would be a Fisher-exact test on a two by two table. Given a cohort of chromosomes, the two by two table is constructed out of the number of chromosomes that include both of the markers or haplotypes, one of the markers or haplotypes but not the other and neither of the markers or haplotypes. Other statistical tests of association known to the skilled person are also contemplated and are also within scope of the invention.

In other embodiments of the invention, an individual who is at a decreased susceptibility (i.e., at a decreased risk) for coronary artery disease and/or myocardial infarction is an individual in whom at least one specific allele at one or more polymorphic marker or haplotype conferring decreased susceptibility for coronary artery disease and myocardial infarction is identified. The marker alleles and/or haplotypes conferring decreased risk are also said to be protective. In one aspect, the protective marker or haplotype is one that confers a significant decreased risk (or susceptibility) of coronary artery disease and myocardial infarction. In one embodiment, significant decreased risk is measured as a relative risk (or odds ratio) of less than 0.9, including but not limited to less than 0.9, less than 0.8, less than 0.7, less than 0.6, less than 0.5, less than 0.4, less than 0.3, less than 0.2 and less than 0.1. In one particular embodiment, significant decreased risk is less than 0.7. In another embodiment, significant decreased risk is less than 0.5. In yet another embodiment, significant decreased risk is less than 0.3. In another embodiment, the decrease in risk (or susceptibility) is at least 20%, including but not limited to at least 25%, at least 30%, at least 35%, at least 40%, at least 45%, at least 50%, at least 55%, at least 60%, at least 65%, at least 70%, at least 75%, at least 80%, at least 85%, at least 90%, at least 95% and at least 98%. In one particular embodiment, a significant decrease in risk is at least about 30%. In another embodiment, a significant decrease in risk is at least about 50%. In another embodiment, the decrease in risk is at least about 70%. Other cutoffs or ranges as deemed suitable by the person skilled in the art to characterize the invention are however also contemplated, and those are also within scope of the present invention.

The person skilled in the art will appreciate that for markers with two alleles present in the population being studied (such as SNPs), and wherein one allele is found in increased frequency in a group of individuals with a trait or disease in the population, compared with controls, the other allele of the marker will be found in decreased frequency in the group of individuals with the trait or disease, compared with controls. In such a case, one allele of the marker (the one found in increased frequency in individuals with the trait or disease) will be the at-risk allele, while the other allele will be a protective allele.

A genetic variant associated with a disease or a trait (*e.g.* CAD or MI) can be used alone to predict the risk of the disease for a given genotype. For a biallelic marker, such as a SNP, there are 3 possible genotypes: homozygote for the at risk variant, heterozygote, and non carrier of the at risk variant. Risk associated with variants at multiple loci can be used to estimate overall risk. For multiple SNP variants, there are *k* possible genotypes *k* = 3*ⁿ* × 2*^{p}*; where *n* is the number autosomal loci and *p* the number of gonosomal (sex chromosomal) loci. Overall risk assessment calculations usually assume that the relative risks of different genetic variants multiply, *i.e.* the overall risk (*e.g.,* RR or OR) associated with a particular genotype combination is the product of the risk values for the genotype at each locus. If the risk presented is the relative risk for a person, or a specific genotype for a person, compared to a reference population with matched gender and ethnicity, then the combined risk - is the product of the locus specific risk values - and which also corresponds to an overall risk estimate compared with the population. If the risk for a person is based on a comparison to non-carriers of the at risk allele, then the combined risk corresponds to an estimate that compares the person with a given combination of genotypes at all loci to a group of individuals who do not carry risk variants at any of those loci. The group of non-carriers of any at risk variant has the lowest estimated risk and has a combined risk, compared with itself (*i.e.,* non-carriers) of 1.0, but has an overall risk, compare with the population, of less than 1.0. It should be noted that the group of non-carriers can potentially be very small, especially for large number of loci, and in that case, its relevance is correspondingly small.

The multiplicative model is a parsimonious model that usually fits the data of complex traits reasonably well. Deviations from multiplicity have been rarely described in the context of common variants for common diseases, and if reported are usually only suggestive since very large sample sizes are usually required to be able to demonstrate statistical interactions between loci.

By way of an example, let us consider a total of eight variants that have been described to associate with prostate cancer (Gudmundsson, J., et al., Nat Genet 39:631-7 (2007), Gudmundsson, J., et al., Nat Genet 39:977-83 (2007); Yeager, M., et al, Nat Genet 39:645-49 (2007), Amundadottir, L., el al., Nat Genet 38:652-8 (2006); Haiman, C.A., et al., Nat Genet 39:638-44 (2007)). Seven of these loci are on autosomes, and the remaining locus is on chromosome X. The total number of theoretical genotypic combinations is then 3⁷ × 2¹ = 4374. Some of those genotypic classes are very rare, but are still possible, and should be considered for overall risk assessment. It is likely that the multiplicative model applied in the case of multiple genetic variant will also be valid in conjugation with non-genetic risk variants assuming that the genetic variant does not clearly correlate with the "environmental" factor. In other words, genetic and non-genetic at-risk variants can be assessed under the multiplicative model to estimate combined risk, assuming that the non-genetic and genetic risk factors do not interact.

Using the same quantitative approach, the combined or overall risk associated with a plurality of variants associated with CAD and MI may be assessed. In certain such embodiments, the markers described herein are assessed together with at least one marker in linkage disequilibrium with the CDKN2A and/or CDKN2A genes, such as rs10811650, rs10116277, rs1333040, rs10738607, rs4977574, rs6475608, D9S1870, rs2383207, rs1333045, rs1333046, rs10757278 or rs1333048. In a preferred embodiment, the markers described herein are assessed in combination with rs10757278.

### Linkage Disequilibrium

The natural phenomenon of recombination, which occurs on average once for each chromosomal pair during each meiotic event, represents one way in which nature provides variations in sequence (and biological function by consequence). It has been discovered that recombination does not occur randomly in the genome; rather, there are large variations in the frequency of recombination rates, resulting in small regions of high recombination frequency (also called recombination hotspots) and larger regions of low recombination frequency, which are commonly referred to as Linkage Disequilibrium (LD) blocks (Myers, S. et al., Biochem Soc Trans 34:526-530 (2006); Jeffreys, A.J., et al., Nature Genet 29:217-222 (2001); May, C.A., et al., Nature Genet 31:272-275(2002)).

Linkage Disequilibrium (LD) refers to a non-random assortment of two genetic elements. For example, if a particular genetic element (*e.g.,* "alleles" of a polymorphic marker) occurs in a population at a frequency of 0.50 (50%) and another occurs at a frequency of 0.50 (50%), then the predicted occurrance of a person's having both elements is 0.25 (25%), assuming a random distribution of the elements. However, if it is discovered that the two elements occur together at a frequency higher than 0.25, then the elements are said to be in linkage disequilibrium since they tend to be inherited together at a higher rate than what their independent allele frequencies would predict. Roughly speaking, LD is generally correlated with the frequency of recombination events between the two elements. Allele frequencies can be determined in a population by genotyping individuals in a population and determining the occurence of each allele in the population. For populations of diploids, *e.g.,* human populations, individuals will typically have two alleles for each genetic element (*e.g.,* a marker or gene).

Many different measures have been proposed for assessing the strength of linkage disequilibrium (LD). Most capture the strength of association between pairs of biallelic sites. Two important pairwise measures of LD are r² (sometimes denoted Δ²) and |D'|. Both measures range from 0 (no disequilibrium) to 1 ('complete' disequilibrium), but their interpretation is slightly different. |D'| is defined in such a way that it is equal to 1 if just two or three of the possible haplotypes are present, and it is <1 if all four possible haplotypes are present. So, a value of |D'| that is <1 indicates that historical recombination may have occurred between two sites (recurrent mutation can also cause |D'| to be <1, but for single nucleotide polymorphisms (SNPs) this is usually regarded as being less likely than recombination). The measure r² represents the statistical correlation between two sites, and takes the value of 1 if only two haplotypes are present.

The r² measure is arguably the most relevant measure for association mapping, because there is a simple inverse relationship between r² and the sample size required to detect association between susceptibility loci and SNPs. These measures are defined for pairs of sites, but for some applications a determination of how strong LD is across an entire region that contains many polymorphic sites might be desirable (*e.g.,* testing whether the strength of LD differs significantly among loci or across populations, or whether there is more or less LD in a region than predicted under a particular model). Measuring LD across a region is not straightforward, but one approach is to use the measure r, which was developed in population genetics. Roughly speaking, r measures how much recombination would be required under a particular population model to generate the LD that is seen in the data. This type of method can potentially also provide a statistically rigorous approach to the problem of determining whether LD data provide evidence for the presence of recombination hotspots. For the methods described herein, a significant r² value can be at least 0.1 such as at least 0.1, 0.15, 0.2, 0.25, 0.3, 0.35, 0.4, 0.45, 0.5, 0.55, 0.6, 0.65, 0.7, 0.75, 0.8, 0.85, 0.9, 0.91, 0.92, 0.93, 0.94, 0.95, 0.96, 0.97, 0.98, or at lesat 0.99. In one preferred embodiment, the significant r² value can be at least 0.2. Alternatively, linkage disequilibrium as described herein, refers to linkage disequilibrium characterized by values of |D'| of at least 0.2, such as 0.3, 0.4, 0.5, 0.6, 0.7, 0.8, 0.85, 0.9, 0.95, 0.96, 0.97, 0.98, or at least 0.99. Thus, linkage disequilibrium represents a correlation between alleles of distinct markers. It is measured by correlation coefficient or |D'| (r² up to 1.0 and |D'| up to 1.0). In certain embodiments, linkage disequilibrium is defined in terms of values for both the r² and |D'| measures. In one such embodiment, a significant linkage disequilibrium is defined as r² > 0.1 and |D'| >0.8. In another embodiment, a significant linkage disequilibrium is defined as r² > 0.2 and |D'| >0.9. Other combinations and permutations of values of r² and |D'| for determining linkage disequilibrium are also contemplated, and are also within the scope of the invention. Linkage disequilibrium can be determined in a single human population, as defined herein, or it can be determined in a collection of samples comprising individuals from more than one human population. In one embodiment of the invention, LD is determined in a sample from one or more of the HapMap populations (caucasian, african, japanese, chinese), as defined (http://www.hapmap.org). In one such embodiment, LD is determined in the Caucasian CEU population of the HapMap samples. In another embodiment, LD is determined in the African YRI population. In yet another embodiment, LD is determined in samples from the Icelandic population.

If all polymorphisms in the genome were independent at the population level (i.e., no LD), then every single one of them would need to be investigated in association studies, to assess all the different polymorphic states. However, due to linkage disequilibrium between polymorphisms, tightly linked polymorphisms are strongly correlated, which reduces the number of polymorphisms that need to be investigated in an association study to observe a significant association. Another consequence of LD is that many polymorphisms may give an association signal due to the fact that these polymorphisms are strongly correlated.

Genomic LD maps have been generated across the genome, and such LD maps have been proposed to serve as framework for mapping disease-genes (Risch, N. & Merkiangas, K, Science 273:1516-1517 (1996); Maniatis, N., et al., Proc Natl Acad Sci USA 99:2228-2233 (2002); Reich, DE et al, Nature 411:199-204 (2001)).

It is now established that many portions of the human genome can be broken into series of discrete haplotype blocks containing a few common haplotypes; for these blocks, linkage disequilibrium data provides little evidence indicating recombination (see, *e.g.,* Wall., J.D. and Pritchard, J.K., Nature Reviews Genetics 4:587-597 (2003); Daly, M. et al., Nature Genet. 29:229-232 (2001); Gabriel, S.B. et al., Science 296:2225-2229 (2002); Patil, N. et al., Science 294:1719-1723 (2001); Dawson, E. et al., Nature 418:544-548 (2002); Phillips, M.S. et al., Nature Genet. 33:382-387 (2003)).

There are two main methods for defining these haplotype blocks: blocks can be defined as regions of DNA that have limited haplotype diversity (see, *e.g.,* Daly, M. et al., Nature Genet. 29:229-232 (2001); Patil, N. et al., Science 294:1719-1723 (2001); Dawson, E. et al., Nature 418:544-548 (2002); Zhang, K. et al., Proc. Natl. Acad. Sci. USA 99:7335-7339 (2002)), or as regions between transition zones having extensive historical recombination, identified using linkage disequilibrium (see, *e.g.,* Gabriel, S.B. et al., Science 296:2225-2229 (2002); Phillips, M.S. et al., Nature Genet. 33:382-387 (2003); Wang, N. et al., Am. J. Hum. Genet. 71:1227-1234 (2002); Stumpf, M.P., and Goldstein, D.B., Curr. Biol. 13:1-8 (2003)). More recently, a fine-scale map of recombination rates and corresponding hotspots across the human genome has been generated (Myers, S., et al., Science 310:321-32324 (2005); Myers, S. et al., Biochem Soc Trans 34:526530 (2006)). The map reveals the enormous variation in recombination across the genome, with recombination rates as high as 10-60 cM/Mb in hotspots, while closer to 0 in intervening regions, which thus represent regions of limited haplotype diversity and high LD. The map can therefore be used to define haplotype blocks/LD blocks as regions flanked by recombination hotspots. As used herein, the terms "haplotype block" or "LD block" includes blocks defined by any of the above described characteristics, or other alternative methods used by the person skilled in the art to define such regions.

Haplotype blocks (LD blocks) can be used to map associations between phenotype and haplotype status, using single markers or haplotypes comprising a plurality of markers. The main haplotypes can be identified in each haplotype block, and then a set of "tagging" SNPs or markers (the smallest set of SNPs or markers needed to distinguish among the haplotypes) can then be identified. These tagging SNPs or markers can then be used in assessment of samples from groups of individuals, in order to identify association between phenotype and haplotype. If desired, neighboring haplotype blocks can be assessed concurrently, as there may also exist linkage disequilibrium among the haplotype blocks.

It has thus become apparent that for any given observed association to a polymorphic marker in the genome, it is likely that additional markers in the genome also show association. This is a natural consequence of the uneven distribution of LD across the genome, as observed by the large variation in recombination rates. The markers used to detect association thus in a sense represent "tags" for a genomic region (i.e., a haplotype block or LD block) that is associating with a given disease or trait, and as such are useful for use in the methods and kits of the present invention. One or more causative (functional) variants or mutations may reside within the region found to be associating to the disease or trait. Such variants may confer a higher relative risk (RR) or odds ratio (OR) than observed for the tagging markers used to detect the association. The present invention thus refers to the markers used for detecting association to the disease, as described herein, as well as markers in linkage disequilibrium with the markers. Thus, in certain embodiments of the invention, markers that are in LD with the markers and/or haplotypes of the invention, as described herein, may be used as surrogate markers. The surrogate markers have in one embodiment relative risk (RR) and/or odds ratio (OR) values smaller than for the markers or haplotypes initially found to be associating with the disease, as described herein. In other embodiments, the surrogate markers have RR or OR values greater than those initially determined for the markers initially found to be associating with the disease, as described herein. An example of such an embodiment would be a rare, or relatively rare (such as < 10% allelic population frequency) variant in LD with a more common variant (> 10% population frequency) initially found to be associating with the disease, such as the variants described herein. Identifying and using such markers for detecting the association discovered by the inventors as described herein can be performed by routine methods well known to the person skilled in the art, and are therefore within the scope of the present invention.

### Determination of haplotype frequency

The frequencies of haplotypes in patient and control groups can be estimated using an expectation-maximization algorithm (Dempster A. et al., J. R. Stat. Soc. B, 39:1-38 (1977)). An implementation of this algorithm that can handle missing genotypes and uncertainty with the phase can be used. Under the null hypothesis, the patients and the controls are assumed to have identical frequencies. Using a likelihood approach, an alternative hypothesis is tested, where a candidate at-risk-haplotype, which can include the markers described herein, is allowed to have a higher frequency in patients than controls, while the ratios of the frequencies of other haplotypes are assumed to be the same in both groups. Likelihoods are maximized separately under both hypotheses and a corresponding 1-df likelihood ratio statistic is used to evaluate the statistical significance.

To look for at-risk and protective markers and haplotypes within a linkage region, for example, association of all possible combinations of genotyped markers is studied, provided those markers span a practical region. The combined patient and control groups can be randomly divided into two sets, equal in size to the original group of patients and controls. The marker and haplotype analysis is then repeated and the most significant p-value registered is determined. This randomization scheme can be repeated, for example, over 100 times to construct an empirical distribution of p-values. In a preferred embodiment, a p-value of <0.05 is indicative of an significant marker and/or haplotype association.

### Haplotype Analysis

One general approach to haplotype analysis involves using likelihood-based inference applied to NEsted MOdels (Gretarsdottir S., et al., Nat. Genet. 35:131-38 (2003)). The method is implemented in the program NEMO, which allows for many polymorphic markers, SNPs and microsatellites. The method and software are specifically designed for case-control studies where the purpose is to identify haplotype groups that confer different risks. It is also a tool for studying LD structures. In NEMO, maximum likelihood estimates, likelihood ratios and p-values are calculated directly, with the aid of the EM algorithm, for the observed data treating it as a missing-data problem.

Even though likelihood ratio tests based on likelihoods computed directly for the observed data, which have captured the information loss due to uncertainty in phase and missing genotypes, can be relied on to give valid p-values, it would still be of interest to know how much information had been lost due to the information being incomplete. The information measure for haplotype analysis is described in Nicolae and Kong (Technical Report 537, Department of Statistics, University of Statistics, University of Chicago; Biometrics, 60(2):368-75 (2004)) as a natural extension of information measures defined for linkage analysis, and is implemented in NEMO.

For single marker association to a disease, the Fisher exact test can be used to calculate two-sided p-values for each individual allele. Usually, all p-values are presented unadjusted for multiple comparisons unless specifically indicated. The presented frequencies (for microsatellites, SNPs and haplotypes) are allelic frequencies as opposed to carrier frequencies. To minimize any bias due the relatedness of the patients who were recruited as families for the linkage analysis, first and second-degree relatives can be eliminated from the patient list. Furthermore, the test can be repeated for association correcting for any remaining relatedness among the patients, by extending a variance adjustment procedure described in Risch, N. & Teng, J. (Genome Res., 8:1273-1288 (1998)), DNA pooling (*ibid*) for sibships so that it can be applied to general familial relationships, and present both adjusted and unadjusted p-values for comparison. The differences are in general very small as expected. To assess the significance of single-marker association corrected for multiple testing we can carry out a randomization test using the same genotype data. Cohorts of patients and controls can be randomized and the association analysis redone multiple times (*e.g.,* up to 500,000 times) and the p-value is the fraction of replications that produced a p-value for some marker allele that is lower than or equal to the p-value we observed using the original patient and control cohorts. Alternatively, a test of association is done in one or several additional case-control cohorts in replication analyses. For tests of single markers, a replication sample analysis only needs to fulfill nominal statistical significance of P<0.05 (two-sided testing; P<0.1 may in fact be more appropriate since a single predefined effect of a single marker is being tested). If many markers are tested in a replication effort, a correction for the number of markers tested in the replication analysis is performed. Alternatively, the results of replication in several case-control cohorts can be combined to provide an overall assessment of the underlying effect, and this may be the most appropriate way of testing the validity of the initially discovered association signal. Deficiencies in individual case-control comparisons, for example due to recruitment bias or population can be neutralized by combined analysis of several case-control studies. The most common method for performing such analysis is the Mantel Haenszel model, which allows different population frequencies for alleles, haplotypes and genotypes but assums a common relative risk (Mantel, N. & Haenszel, W., J Natl Cancer Inst 22:719 (1959)).

For both single-marker and haplotype analyses, relative risk (RR) and the population attributable risk (PAR) can be calculated assuming a multiplicative model (haplotype relative risk model) (Terwilliger, J.D. & Ott, J., Hum. Hered. 42:337-46 (1992) and Falk, C.T. & Rubinstein, P, Ann. Hum. Genet. 51 (Pt 3):227-33 (1987)), i.e., that the risks of the two alleles/haplotypes a person carries multiply. For example, if RR is the risk of A relative to a, then the risk of a person homozygote AA will be RR times that of a heterozygote Aa and RR² times that of a homozygote aa. The multiplicative model has a nice property that simplifies analysis and computations - haplotypes are independent, *i.e.,* in Hardy-Weinberg equilibrium, within the affected population as well as within the control population. As a consequence, haplotype counts of the affecteds and controls each have multinomial distributions, but with different haplotype frequencies under the alternative hypothesis. Specifically, for two haplotypes, *hᵢ* and *hⱼ,* risk(*hᵢ*)/risk(*hⱼ*) = (*fᵢ*/*pᵢ*)/(*fⱼ*/*pⱼ*), where *f* and *p* denote, respectively, frequencies in the affected population and in the control population. While there is some power loss if the true model is not multiplicative, the loss tends to be mild except for extreme cases. Most importantly, p-values are always valid since they are computed with respect to null hypothesis.

### Linkage Disequilibrium Using NEMO

LD between pairs of markers can be calculated using the standard definition of D' and r² (Lewontin, R., Genetics 49:49-67 (1964); Hill, W.G. & Robertson, A. Theor. Appl. Genet. 22:226-231 (1968)). Using NEMO, frequencies of the two marker allele combinations are estimated by maximum likelihood and deviation from linkage equilibrium is evaluated by a likelihood ratio test. The definitions of D' and r² are extended to include microsatellites by averaging over the values for all possible allele combination of the two markers weighted by the marginal allele probabilities. When plotting all marker combination to elucidate the LD structure in a particular region, we plot D' in the upper left corner and the p-value in the lower right corner. In the LD plots the markers can be plotted equidistant rather than according to their physical location, if desired.

### Risk assessment and Diagnostics

Within any given population, there is an absolute risk of developing a disease or trait, defined as the chance of a person developing the specific disease or trait over a specified time-period. For example, a woman's lifetime absolute risk of breast cancer is one in nine. That is to say, one woman in every nine will develop breast cancer at some point in their lives. Risk is typically measured by looking at very large numbers of people, rather than at a particular individual. Risk is often presented in terms of Absolute Risk (AR) and Relative Risk (RR). Relative Risk is used to compare risks associating with two variants or the risks of two different groups of people. For example, it can be used to compare a group of people with a certain genotype with another group having a different genotype. For a disease, a relative risk of 2 means that one group has twice the chance of developing a disease as the other group. The Risk presented is usually the relative risk for a person, or a specific genotype of a person, compared to the population with matched gender and ethnicity. Risks of two individuals of the same gender and ethnicity could be compared in a simple manner. For example, if, compared to the population, the first individual has relative risk 1.5 and the second has relative risk 0.5, then the risk of the first individual compared to the second individual is 1.5/0.5 = 3.

In certain embodiments, marker and haplotype analysis involves defining candidate susceptibility region or locus based on "haplotype blocks" (also called "LD blocks"). It is now established that many portions of the human genome can be broken into series of discrete haplotype blocks containing a few common haplotypes; for these blocks, linkage disequilibrium data provides little evidence indicating recombination (see, *e.g.,* Wall., J.D. and Pritchard, J.K., Nature Reviews Genetics 4:587-597 (2003); Daly, M. et al., Nature Genet. 29:229-232 (2001); Gabriel, S.B. et al., Science 296:2225-2229 (2002); Patil, N. et al., Science 294:1719-1723 (2001); Dawson, E. et al., Nature 418:544-548 (2002); Phillips, M.S. et al., Nature Genet. 33:382-387 (2003)).

There are two main methods for defining these haplotype blocks: blocks can be defined as regions of DNA that have limited haplotype diversity (see, *e.g.,* Daly, M. et al., Nature Genet. 29:229-232 (2001); Patil, N. et al., Science 294:1719-1723 (2001); Dawson, E. et al., Nature 418:544-548 (2002); Zhang, K. et al., Proc. Natl. Acad. Sci. USA 99:7335-7339 (2002)), or as regions between transition zones having extensive historical recombination, identified using linkage disequilibrium (see, *e.g.,* Gabriel, S.B. et al., Science 296:2225-2229 (2002); Phillips, M.S. et al., Nature Genet. 33:382-387 (2003); Wang, N. et al., Am. J. Hum. Genet. 71:1227-1234 (2002); Stumpf, M.P., and Goldstein, D.B., Curr. Biol. 13:1-8 (2003)). As used herein, the terms "haplotype block" or "LD block" includes blocks defined by either characteristic.

Representative methods for identification of haplotype blocks are set forth, for example, in U.S. Published Patent Application Nos. 20030099964, 20030170665, 20040023237 and 20040146870. Haplotype blocks can be used readily to map associations between phenotype and haplotype status. The main haplotypes can be identified in each haplotype block, and then a set of "tagging" SNPs or markers (the smallest set of SNPs or markers needed to distinguish among the haplotypes) can then be identified. These tagging SNPs or markers can then be used in assessment of samples from groups of individuals, in order to identify association between phenotype and haplotype. If desired, neighboring haplotype blocks can be assessed concurrently, as there may also exist linkage disequilibrium among the haplotype blocks.

As described herein, certain markers and haplotypes comprising such markers are found to be useful for risk assessment of cardiovascular disease, including coronary artery disease and myocardial infarction. Risk assessment can involve the use of the markers and variants for diagnosing a susceptibility to cardiovascular disease, including coronary artery disease and myocardial infarction. Particular alleles of polymorphic markers and haplotypes are found more frequently in individuals with cardiovascular disease, including coronary artery disease and myocardial infarction than in individuals without diagnosis of cardiovascular disease, including coronary artery disease and myocardial infarction. Therefore, these marker alleles and haplotypes have predictive value for detecting cardiovascular disease, including coronary artery disease and myocardial infarction, or a susceptibility to coronary artery disease and myocardial infarction, in an individual. Tagging markers within haplotype blocks or LD blocks comprising at-risk markers, such as the markers of the present invention, can be used as surrogates for other markers within the haplotype block or LD block. Markers with values of *r*² equal to 1 are perfect surrogates for the at-risk variants, i.e. genotypes for one marker perfectly predicts genotypes for the other. Markers with smaller values of *r*² than 1 can also be surrogates for the at-risk variant, or alternatively represent variants with relative risk values as high or possibly even higher than the at-risk variant. The at-risk variant identified may therefore not be the functional variant itself, but is in this instance in linkage disequilibrium with the true functional variant. The present invention encompasses the assessment of such surrogate markers for the markers as disclosed herein. Such markers are annotated, mapped and listed in public databases, as well known to the skilled person, or can alternatively be readily identified by sequencing the region or a part of the region identified by the markers of the present invention in a group of individuals, and identify polymorphisms in the resulting group of sequences. As a consequence, the person skilled in the art can readily and without undue experimentation genotype surrogate markers in linkage disequilibrium with the markers and/or haplotypes as described herein. The tagging or surrogate markers in LD with the at-risk variants detected also have predictive value for detecting association to CAD and/or MI, or a susceptibility to CAD and/or MI, in an individual. These tagging or surrogate markers that are in LD with the markers of the present invention can also include other markers that distinguish among haplotypes in associated genomic regions, as these similarly have predictive value for detecting susceptibility to CAD and MI.

The present invention can in certain embodiments be practiced by assessing a sample comprising genomic DNA from an individual for the presence of variants described herein to be associated with the cardiovascular diseases CAD and MI. Such assessment includes steps of detecting the presence or absence of at least one allele of at least one polymorphic marker, using methods well known to the skilled person and further described herein, and based on the outcome of such assessment, determine whether the individual from whom the sample is derived is at increased or decreased risk (increased or decreased susceptibility) of CAD and/or MI. Alternatively, the invention can be practiced utilizing a dataset comprising information about the genotype status of at least one polymorphic marker described herein to be associated with CAD and/or MI (or markers in linkage disequilibrium with at least one marker shown herein to be associated with CAD and/or MI). In other words, a dataset containing information about such genetic status, for example in the form of genotype counts at a certain polymorphic marker, or a plurality of markers (*e.g.,* an indication of the presence or absence of certain at-risk alleles), or actual genotypes for one or more markers, can be queried for the presence or absence of certain at-risk alleles at certain polymorphic markers shown by the present inventors to be associated with CAD and/or MI. A positive result for a variant (*e.g.,* marker allele) associated with CAD and/or MI, as shown herein, is indicative of the individual from which the dataset is derived is at increased susceptibility (increased risk) of CAD and/or MI.

In certain embodiments of the invention, a polymorphic marker is correlated to CAD and/or MI by referencing genotype data for the polymorphic marker to a look-up table that comprises correlations between at least one allele of the polymorphism and CAD and/or MI. In some embodiments, the table comprises a correlation for one polymorhpism. In other embodiments, the table comprises a correlation for a plurality of polymorhpisms. In both scenarios, by referencing to a look-up table that gives an indication of a correlation between a marker and CAD and/or MI, a risk for CAD and/or MI, or a susceptibility to CAD and/or MI, can be identified in the individual from whom the sample is derived. In some embodiments, the correlation is reported as a statistical measure. The statistical measure may be reported as a risk measure, such as a relative risk (RR), an absolute risk (AR) or an odds ratio (OR).

The markers and haplotypes of the invention, *e.g.,* the markers presented in Table 4, may be useful for risk assessment and diagnostic purposes for coronary artery disease and myocardial infarction, either alone or in combination. Thus, even in the cases where the increase in risk by individual markers is relatively modest, i.e. on the order of 10-30%, the association may have significant implications. Thus, relatively common variants may have significant contribution to the overall risk (Population Attributable Risk is high), or combination of markers can be used to define groups of individual who, based on the combined risk of the markers, is at significant combined risk of developing CAD and/or MI. Thus, in one embodiment of the invention, a plurality of variants (markers and/or haplotypes) is used for overall risk assessment. In such embodiments, the genotype status of a plurality of markers and/or haplotypes is determined in an individual, and the status of the individual compared with the population frequency of the associated variants, or the frequency of the variants in clinically healthy subjects, such as age-matched and sex-matched subjects. Methods known in the art, such as multivariate analyses or joint risk analyses, may subsequently be used to determine the overall risk conferred based on the genotype status at the multiple loci. Assessment of risk based on such analysis may subsequently be used in the methods and kits of the invention, as described herein.

As described in the above, the haplotype block structure of the human genome has the effect that a large number of variants (markers and/or haplotypes) in linkage disequilibrium with the variant originally associated with a disease or trait may be used as surrogate markers for assessing association to the disease or trait. The number of such surrogate markers will depend on factors such as the historical recombination rate in the region, the mutational frequency in the region (i.e., the number of polymorphic sites or markers in the region), and the extent of LD (size of the LD block) in the region. These markers are usually located within the physical boundaries of the LD block or haplotype block in question as defined using the methods described herein, or by other methods known to the person skilled in the art. However, sometimes marker and haplotype association is found to extend beyond the physical boundaries of the haplotype block as defined. Such markers and/or haplotypes may in those cases be also used as surrogate markers and/or haplotypes for the markers and/or haplotypes physically residing within the haplotype block as defined. As a consequence, markers and haplotypes in LD with the markers and haplotypes of the present invention are also within the scope of the invention, even if they are physically located beyond the boundaries of the haplotype block as defined. This includes markers that are described herein (*e.g.,*Table 5).

For the SNP markers described herein, the opposite allele to the allele found to be in excess in patients (at-risk allele) is found in decreased frequency in coronary artery disease and myocardial infarction. These markers and haplotypes in LD and/or comprising such markers, are thus protective for coronary artery disease and myocardial infarction, i.e. they confer a decreased risk or susceptibility of individuals carrying these markers and/or haplotypes developing coronary artery disease and myocardial infarction.

Certain variants of the present invention, including certain haplotypes comprise, in some cases, a combination of various genetic markers, *e.g.,* SNPs and microsatellites. Detecting haplotypes can be accomplished by methods known in the art and/or described herein for detecting sequences at polymorphic sites. Furthermore, correlation between certain haplotypes or sets of markers and disease phenotype can be verified using standard techniques. A representative example of a simple test for correlation would be a Fisher-exact test on a two by two table.

In specific embodiments, a marker allele or haplotype associated with coronary artery disease and myocardial infarction (e.g., marker alleles as listed in Table 4) is one in which the marker allele or haplotype is more frequently present in an individual at risk for coronary artery disease and/or myocardial infarction (affected), compared to the frequency of its presence in a healthy individual (control), wherein the presence of the marker allele or haplotype is indicative of coronary artery disease and/or myocardial infarction or a susceptibility to coronary artery disease and/or myocardial infarction. In other embodiments, at-risk markers in linkage disequilibrium with one or more markers found to be associated with coronary artery disease and/or myocardial infarction (e.g., marker alleles as listed in Table 4) are tagging markers that are more frequently present in an individual at risk for coronary artery disease and/or myocardial infarction (affected), compared to the frequency of their presence in a healthy individuals (controls), wherein the presence of the tagging markers is indicative of increased susceptibility to coronary artery disease and/or myocardial infarction. In a further embodiment, at-risk markers alleles (i.e. conferring increased susceptibility) in linkage disequilibrium with one or more markers found to be associated with coronary artery disease and/or myocardial infarction (e.g., marker alleles as listed in Table 4), are markers comprising one or more allele that is more frequently present in an individual at risk for coronary artery disease and/or myocardial infarction, compared to the frequency of their presence in a healthy individual (control), wherein the presence of the markers is indicative of increased susceptibility to coronary artery disease and/or myocardial infarction.

### Utility of Genetic Testing

The person skilled in the art will appreciate and understand that the variants described herein in general do not, by themselves, provide an absolute identification of individuals who will develop coronary artery disease or myocardial infarction. The variants described herein do however indicate increased and/or decreased likelihood that individuals carrying the at-risk or protective variants of the invention will develop CAD or MI, or develop symptoms associated with these diseases. This information Is however extremely valuable in itself, as outlined in more detail in the below, as it can be used to, for example, initiate preventive measures at an early stage, perform regular physical and/or mental exams to monitor the progress and/or appearance of symptoms, or to schedule exams at a regular interval to identify early symptoms, so as to be able to apply treatment at an early stage.

The knowledge about a genetic variant that confers a risk of developing cardiovascular disease, including coronary artery disease and myocardial infarction, offers the opportunity to apply a genetic test to distinguish between individuals with increased risk of developing the disease (i.e. carriers of the at-risk variant) and those with decreased risk of developing the disease (i.e. carriers of the protective variant). The core values of genetic testing, for individuals belonging to both of the above mentioned groups, are the possibilities of being able to diagnose disease, or a predisposition to disease, at an early stage and provide information to the clinician about prognosis/aggressiveness of disease in order to be able to apply the most appropriate treatment.

Individuals with a family history of CAD and/or MI and carriers of at-risk variants may benefit from genetic testing since the knowledge of the presence of a genetic risk factor, or evidence for increased risk of being a carrier of one or more risk factors, may provide increased incentive for implementing a healthier lifestyle (*e.g.,* lose weight, increase exercise, give um smoking, reduce stress, etc.), by avoiding or minimizing known environmental risk factors for these cardiovascular diseases. Genetic testing of patients may furthermore give valuable information about the primary cause of the disease and can aid the clinician in selecting the best treatment options and medication for each individual.

The present invention can be thus be used for risk assessment for coronary artery disease and/or myocardial infarction, including diagnosing whether an individual is at risk for developing myocardial infarction and/or coronary artery disease. The polymorphic markers of the present invention can be used alone or in combination, as well as in combination with other factors, including known biomarkers, for risk assessment of an individual for coronary artery disease and/or myocardial infarction. Many factors known to affect the predisposition of indiviudal towards developing risk of developing cardiovascular disease, including coronary artery disease and/or myocardial infarction, are known to the person skilled in the art and can be utilized in such assessment. These include, but are not limited to, age, gender, smoking status, physical activiy, waist-to-hip circumference ratio, family history of Cardiovascular Disease (*e.g.,* MI and/or CAD), previously diagnosed cardiovascular disease, obesity, diagnosis of Diabetes mellitus, stress, depression, elevated heart rate, hypertriglyceridemia, low HDL cholesterol, hypertension, elevated blood pressure, cholesterol levels, HDL cholesterol, LDL cholesterol, triglycerides, apolipoprotein AI and B levels, fibrinogen, ferritin, C-reactive protein and leukotriene levels. Methods known in the art can be used for such assessment, including multivariate analyses or logistic regression, as described further herein.

### METHODS

Methods for risk management and risk assessment of CAD and MI are described herein and are encompassed by the invention. Also disclosed methods of assessing an individual for probability of response to a therapeutic agent for a CAD and/or MI, methods for predicting the effectiveness of a therapeutic agent for CAD and/or MI, nucleic acids, polypeptides and antibodies and computer-implemented functions useful in such methods. Kits for assaying a sample from a subject to detect susceptibility to cardiovascular disease are also disclosed.

### Diagnostic and screening methods

In certain embodiments, the present invention pertains to method of diagnosing, or aiding in the diagnosis of coronary artery disease (CAD) and/or myocardial infarction (MI) or a susceptibility to CAD and/or MI, by detecting particular alleles at genetic markers that appear more frequently in individuals diagnosed with CAD and MI or individuals who are susceptible to CAD and MI. In a particular embodiment, the invention is a method of diagnosing a susceptibility to CAD and/or MI by detecting at least one allele of at least one polymorphic marker (e.g., the markers described herein). The present invention describes methods whereby detection of particular alleles of particular markers or haplotypes is indicative of a susceptibility to CAD and MI. Such prognostic or predictive assays can also be used to determine prophylactic treatment of a subject prior to the onset of symptoms of cardiovascular disease, including coronary artery disease and myocardial infarction. The present invention pertains in some embodiments to methods of clinical applications of diagnosis, *e.g.,* diagnosis performed by a medical professional. In other embodiments, the invention pertains to methods of diagnosis or determination of a susceptibility performed by a layman. The layman can be the customer of a genotyping service. The layman may also be a genotype service provider, who performs genotype analysis on a DNA sample from an individual, in order to provide service related to genetic risk factors for particular traits or diseases, based on the genotype status of the individual (*i.e.,* the customer). A third party who provides service to individuals can be one who provides a guidance or teaching as to how to relate or interpret genotype information to calculate or estimate genetic risk. In one embodiment, the third party may receive a sample containing genomic DNA from a customer, send the sample, or DNA isolated from the sample, to a genotype service provider, and present the customer with the genotype data and/or an interpretation of the genotype data, such as by calculating disease risk for specific markers. The customer may access his/her genotype information, including results of any risk calculations, via a secure we interface. Alternatively, the customer may receive disease risk information by other means, such as by regular mail. Recent technological advances in genotyping technologies, including high-throughput genotyping of SNP markers, such as Molecular Inversion Probe array technology (*e.g.,* Affymetrix GeneChip), and BeadArray Technologies (*e.g.,* Illumina GoldenGate and Infinium assays) have made it possible for individuals to have their own genome assessed for up to one million SNPs simultaneously, at relatively little cost. The resulting genotype information, made available to the individual can be compared to information from the public literature about disease or trait risk associated with various SNPs. The diagnostic application of disease-associated alleles as described herein, can thus for example be performed by the individual, through analysis of his/her genotype data, by a health professional based on results of a clinical test, or by a third party. In other words, the diagnosis or determination of a susceptibility of genetic risk can be made by health professionals, genetic counselors, third parties providing such service or by the layman, based on information about the genotype status of an individual and knowledge about the risk conferred by particular genetic risk factors (*e.g.,* particular SNPs). In the present context, the term "diagnosing", "diagnose a susceptibility" and "determine a susceptibility" is meant to refer to any available diagnostic method, including those mentioned above.

In certain embodiments, a sample containing genomic DNA from an individual is collected. Such sample can for example be a buccal swab, a saliva sample, a blood sample, or other suitable samples containing genomic DNA, as described further herein. The genomic DNA is then analyzed using any common technique available to the skilled person, such as high-throughput array technologies. Results from such genotyping are stored in a convenient data storage unit, such as a data carrier, including computer databases, data storage disks, or by other convenient data storage means. In certain embodiments, the computer database is an object database, a relational database or a post-relational database. The genotype data is subsequently analyzed for the presence of certain variants known to be susceptibility variants for a particular human conditions, such as the genetic variants described herein. Genotype data can be retrieved from the data storage unit using any convenient data query method. Calculating risk conferred by a particular genotype for the individual is based on comparing the genotype to previously determined risk (expressed as a relative risk (RR) or and odds ratio (OR), for example) for the genotype, for example for an heterozygous carrier of an at-risk variant for a particular disease or trait (such as CAD and/or MI). Risk presented can be the relative risk for a person, or a specific genotype of a person, compared to the population with matched gender and ethnicity. The average population risk can be expressed as a weighted average of the risks of different genotypes, using results from a reference population, and the appropriate calculations to calculate the risk of a genotype group relative to the population can then be performed. Alternatively, the risk for an individual is based on a comparison of particular genotypes, for example heterozygous carriers of an at-risk allele of a marker compared with non-carriers of the at-risk allele. Using the population average may in certain embodiments be more convenient, since it provides a measure which is easy to interpret for the user, i.e. a measure that gives the risk for the individual, based on his/her genotype, compared with the average in the population.

Overall risk for multiple risk variants can be performed using standard methodology. For example, assuming a multiplicative model, *i.e*. assuming that the risk of individual risk variants multiply to establish the overall effect, allows for a straight-forward calculation of the overall risk for multiple markers.

In addition, in certain other embodiments, the present invention pertains to method of diagnosing, or aiding in the diagnosis of, a decreased susceptibility to cardiovascular disease, in particular CAD and/or MI, by detecting particular genetic marker alleles or haplotypes that appear less frequently in individuals diagnosed with CAD and/or MI than in individual not diagnosed with these diseases, or in the general population.

As described and exemplified herein, particular marker alleles or haplotypes are associated with risk of coronary artery disease and/or myocardial infarction. In one embodiment, the marker allele or haplotype is one that confers a significant risk or susceptibility to coronary artery disease and/or myocardial infarction. In another embodiment, the invention relates to a method of diagnosing a susceptibility to CAD and/or MI in a human individual, the method comprising determining the presence or absence of at least one allele of at least one polymorphic marker in a nucleic acid sample obtained from the individual, wherein the at least one polymorphic marker is selected from the group consisting of rs11751605, and markers in linkage disequilibrium therewith listed in Table 5. In another embodiment, the invention pertains to methods of diagnosing a susceptibility to CAD and/or MI in a human individual, by screening for at least one marker allele or haplotype. In another embodiment, the marker allele or haplotype is more frequently present in a subject having, or who is susceptible to, CAD and/or MI (affected), as compared to the frequency of its presence in a healthy subject (control, such as population controls). In certain embodiments, the significance of association of the at least one marker allele or haplotype is characterized by a p value < 0.05. In other embodiments, the significance of association is characterized by smaller p-values, such as < 0.01, <0.001, <0.0001, <0.00001, <0.000001, <0.0000001, <0.00000001 or <0.000000001.

In these embodiments, the presence of the at least one marker allele or haplotype is indicative of a susceptibility to coronary artery disease and/or myocardial infarction. These diagnostic methods involve detecting the presence or absence of at least one marker allele or haplotype that is associated with coronary artery disease and/or myocardial infarction. The haplotypes described herein include combinations of alleles at various genetic markers (e.g., SNPs, microsatellites). The detection of the particular genetic marker alleles that make up the particular haplotypes can be performed by a variety of methods described herein and/or known in the art. For example, genetic markers can be detected at the nucleic acid level (e.g., by direct nucleotide sequencing or by other means known to the skilled in the art) or at the amino acid level if the genetic marker affects the coding sequence of a protein encoded by a coronary artery disease and/or myocardial infarction -associated nucleic acid (e.g., by protein sequencing or by immunoassays using antibodies that recognize such a protein). The marker alleles or haplotypes of the present invention correspond to fragments of a genomic DNA sequence associated with coronary artery disease and/or myocardial infarction. Such fragments encompass the DNA sequence of the polymorphic marker or haplotype in question, but may also include DNA segments in strong LD (linkage disequilibrium) with the marker or haplotype.

In one embodiment, diagnosis of a susceptibility to CAD and/or MI can be accomplished using hybridization methods, including, but not limited to, hybridization to a microarray, Southern analysis, Northern analysis, and/or *in situ* hybridizations (see Current Protocols in Molecular Biology, Ausubel, F. et al., eds., John Wiley & Sons, including all supplements). The presence of a specific marker allele can be indicated by sequence-specific hybridization of a nucleic acid probe specific for the particular allele. The presence of more than one specific marker allele or a specific haplotype can be indicated by using several sequence-specific nucleic acid probes, each being specific for a particular allele. In one embodiment, a haplotype can be indicated by a single nucleic acid probe that is specific for the specific haplotype (i.e., hybridizes specifically to a DNA strand comprising the specific marker alleles characteristic of the haplotype). A sequence-specific probe can be directed to hybridize to genomic DNA, RNA, or cDNA. A "nucleic acid probe", as used herein, can be a DNA probe or an RNA probe that hybridizes to a complementary sequence. One of skill in the art would know how to design such a probe so that sequence specific hybridization will occur only if a particular allele is present in a genomic sequence from a test sample. Other methods of using hybridization probes in allele-specific detection methods include methods of single base extension of nucleotide probes flanking a single base polymorphic sites. Such methods are well known to the skilled artisan.

To determine a susceptibility or diagnose a susceptibility, a hybridization sample is formed by contacting the test sample containing an nucleic acid associated with CAD and/or MI, such as a genomic DNA sample, with at least one nucleic acid probe. A non-limiting example of a probe for detecting mRNA or genomic DNA is a labeled nucleic acid probe that is capable of hybridizing to mRNA or genomic DNA sequences described herein. The nucleic acid probe can be, for example, a full-length nucleic acid molecule, or a portion thereof, such as an oligonucleotide of at least 15, 30, 50, 100, 250 or 500 nucleotides in length that is sufficient to specifically hybridize under stringent conditions to appropriate mRNA or genomic DNA. For example, the nucleic acid probe can comprise all or a portion of the nucleotide sequence of any one of SEQ ID NO:1-172, as set forth herein, or the probe can be the complementary sequence of such a sequence. Other suitable probes for use in the diagnostic assays of the invention are described herein. Hybridization can be performed by methods well known to the person skilled in the art (see, *e.g.,* Current Protocols in Molecular Biology, Ausubel, F. et al., eds., John Wiley & Sons, including all supplements). In one embodiment, hybridization refers to specific hybridization, i.e., hybridization with no mismatches (exact hybridization). In one embodiment, the hybridization conditions for specific hybridization are high stringency.

Specific hybridization, if present, is detected using standard methods. If specific hybridization occurs between the nucleic acid probe and the nucleic acid in the test sample, then the sample contains the allele that is complementary to the nucleotide that is present in the nucleic acid probe. The process can be repeated for any markers of the present invention, or markers that make up a haplotype of the present invention, or multiple probes can be used concurrently to detect more than one marker alleles at a time. It is also possible to design a single probe containing more than one marker alleles of a particular haplotype (e.g., a probe containing alleles complementary to 2, 3, 4, 5 or all of the markers that make up a particular haplotype). Detection of the particular markers of the haplotype in the sample is indicative that the source of the sample has the particular haplotype (e.g., a haplotype) and therefore is susceptible to CAD and/or MI.

In one preferred embodiment, a method utilizing a detection oligonucleotide probe comprising a fluorescent moiety or group at its 3' terminus and a quencher at its 5' terminus, and an enhancer oligonucleotide, is employed, as described by Kutyavin *et al.* (*Nucleic Acid Res.* **34:**e128 (2006)). The fluorescent moiety can be Gig Harbor Green or Yakima Yellow, or other suitable fluorescent moieties. The detection probe is designed to hybridize to a short nucleotide sequence that includes the SNP polymorphism to be detected. Preferably, the SNP is anywhere from the terminal residue to -6 residues from the 3' end of the detection probe. The enhancer is a short oligonucleotide probe which hybridizes to the DNA template 3' relative to the detection probe. The probes are designed such that a single nucleotide gap exists between the detection probe and the enhancer nucleotide probe when both are bound to the template. The gap creates a synthetic abasic site that is recognized by an endonuclease, such as Endonuclease IV. The enzyme cleaves the dye off the fully complementary detection probe, but cannot cleave a detection probe containing a mismatch. Thus, by measuring the fluorescence of the released fluorescent moiety, assessment of the presence of a particular allele defined by nucleotide sequence of the detection probe can be performed.

The detection probe can be of any suitable size, although preferably the probe is relatively short. In one embodiment, the probe is from 5-100 nucleotides in length. In another embodiment, the probe is from 10-50 nucleotides in length, and in another embodiment, the probe is from 12-30 nucleotides in length. Other lengths of the probe are possible and within scope of the skill of the average person skilled in the art.

In a preferred embodiment, the DNA template containing the SNP polymorphism is amplified by Polymerase Chain Reaction (PCR) prior to detection. In such an embodiment, the amplified DNA serves as the template for the detection probe and the enhancer probe.

Certain embodiments of the detection probe, the enhancer probe, and/or the primers used for amplification of the template by PCR include the use of modified bases, including modified A and modified G. The use of modified bases can be useful for adjusting the melting temperature of the nucleotide molecule (probe and/or primer) to the template DNA, for example for increasing the melting temperature in regions containing a low percentage of G or C bases, in which modified A with the capability of forming three hydrogen bonds to its complementary T can be used, or for decreasing the melting temperature in regions containing a high percentage of G or C bases, for example by using modified G bases that form only two hydrogen bonds to their complementary C base in a double stranded DNA molecule. In a preferred embodiment, modified bases are used in the design of the detection nucleotide probe. Any modified base known to the skilled person can be selected in these methods, and the selection of suitable bases is well within the scope of the skilled person based on the teachings herein and known bases available from commercial sources as known to the skilled person.

In another hybridization method, Northern analysis (see Current Protocols in Molecular Biology, Ausubel, F. et al., eds., John Wiley & Sons, *supra*) is used to identify the presence of a polymorphism associated with CAD and/or MI. For Northern analysis, a test sample of RNA is obtained from the subject by appropriate means. As described herein, specific hybridization of a nucleic acid probe to RNA from the subject is indicative of a particular allele complementary to the probe. For representative examples of use of nucleic acid probes, see, for example, U.S. Patent Nos. 5,288,611 and 4,851,330.

Additionally, or alternatively, a peptide nucleic acid (PNA) probe can be used in addition to, or instead of, a nucleic acid probe in the hybridization methods described herein. A PNA is a DNA mimic having a peptide-like, inorganic backbone, such as N-(2-aminoethyl)glycine units, with an organic base (A, G, C, T or U) attached to the glycine nitrogen via a methylene carbonyl linker (see, for example, Nielsen, P., et al., Bioconjug. Chem. 5:3-7 (1994)). The PNA probe can be designed to specifically hybridize to a molecule in a sample suspected of containing one or more of the marker alleles or haplotypes that are associated with CAD and/or MI. Hybridization of the PNA probe is thus diagnostic for a susceptibility of CAD and/or MI.

In one embodiment of the invention, a test sample containing genomic DNA obtained from the subject is collected and the polymerase chain reaction (PCR) is used to amplify a fragment comprising one ore more markers or haplotypes of the present invention. As described herein, identification of a particular marker allele or haplotype associated with CAD and/MI, can be accomplished using a variety of methods (e.g., sequence analysis, analysis by restriction digestion, specific hybridization, single stranded conformation polymorphism assays (SSCP), electrophoretic analysis, etc.). In another embodiment, diagnosis is accomplished by expression analysis using quantitative PCR (kinetic thermal cycling). This technique can, for example, utilize commercially available technologies, such as TaqMan^{®} (Applied Biosystems, Foster City, CA) . The technique can assess the presence of an alteration in the expression or composition of a polypeptide or splicing variant(s) that is encoded by a nucleic acid associated with CAD and/or MI, as described herein. Further, the expression of the variant(s) can be quantified as physically or functionally different.

In another embodiment of the method of the invention, analysis by restriction digestion can be used to detect a particular allele if the allele results in the creation or elimination of a restriction site relative to a reference sequence. Restriction fragment length polymorphism (RFLP) analysis can be conducted, e.g., as described in Current Protocols in Molecular Biology, *supra.* The digestion pattern of the relevant DNA fragment indicates the presence or absence of the particular allele in the sample.

Sequence analysis can also be used to detect specific alleles or haplotypes associated with CAD and/or MI (e.g. the polymorphic markers of Table 4 and markers in linkage disequilibrium therewith). Therefore, in one embodiment, determination of the presence or absence of a particular marker alleles or haplotypes comprises sequence analysis of a test sample of DNA or RNA obtained from a subject or individual. PCR or other appropriate methods can be used to amplify a portion of a nucleic acid template, and the presence of a specific allele can then be detected directly by sequencing the polymorphic site (or multiple polymorphic sites in a haplotype) of the genomic DNA in the sample.

In another embodiment, arrays of oligonucleotide probes that are complementary to target nucleic acid sequence segments from a subject, can be used to identify polymorphisms in a nucleic acid associated with CAD and/or MI. For example, an oligonucleotide array can be used. Oligonucleotide arrays typically comprise a plurality of different oligonucleotide probes that are coupled to a surface of a substrate in different known locations. These arrays can generally be produced using mechanical synthesis methods or light directed synthesis methods that incorporate a combination of photolithographic methods and solid phase oligonucleotide synthesis methods, or by other methods known to the person skilled in the art (see, *e.g.,* Fodor, S. et al., Science, 251:767-773 (1991); Pirrung et al., U.S. Patent No. 5,143,854 (see also published PCT Application No. WO 90/15070); and Fodor. S. et al., published PCT Application No. WO 92/10092 and U.S. Patent No. 5,424,186). Techniques for the synthesis of these arrays using mechanical synthesis methods are described in, e.g., U.S. Patent No. 5,384,261. In another example, linear arrays can be utilized. Additional descriptions of use of oligonucleotide arrays for detection of polymorphisms can be found, for example, in U.S. Patent Nos. 5,858,659 and 5,837,832.

Other methods of nucleic acid analysis that are available to those skilled in the art can be used to detect a particular allele at a polymorphic site associated with CAD and/or MI (e.g. the polymorphic markers of Table 4 and markers in linkage disequilibrium therewith). Representative methods include, for example, direct manual sequencing (Church and Gilbert, Proc. Natl. Acad. Sci. USA, 81: 1991-1995 (1988); Sanger, F., et al., Proc. Natl. Acad. Sci. USA, 74:5463-5467 (1977); Beavis, et al., U.S. Patent No. 5,288,644); automated fluorescent sequencing; single-stranded conformation polymorphism assays (SSCP); clamped denaturing gel electrophoresis (CDGE); denaturing gradient gel electrophoresis (DGGE) (Sheffield, V., et al., Proc. Natl. Acad. Sci. USA, 86:232-236 (1989)), mobility shift analysis (Orita, M., etal., Proc. Natl. Acad. Sci. USA, 86:2766-2770 (1989)), restriction enzyme analysis (Flavell, R., et al., Cell, 15:25-41 (1978); Geever, R., et al., Proc. Natl. Acad. Sci. USA, 78:5081-5085 (1981)); heteroduplex analysis; chemical mismatch cleavage (CMC) (Cotton, R., et al., Proc. Natl. Acad. Sci. USA, 85:4397-4401 (1985)); RNase protection assays (Myers, R., et al., Science, 230:1242-1246 (1985); use of polypeptides that recognize nucleotide mismatches, such as *E. coli* mutS protein; and allele-specific PCR.

In another embodiment of the invention, diagnosis of CAD and/or MI or a susceptibility to CAD and/or MI can be made by examining expression and/or composition of a polypeptide encoded by a nucleic acid associated with these diseases, in those instances where the genetic marker(s) or haplotype(s) of the present invention result in a change in the composition or expression of the polypeptide. Thus, diagnosis or determination of a susceptibility can be made by examining expression and/or composition of one of these polypeptides, or another polypeptide encoded by a nucleic acid associated with CAD and/or MI, in those instances where the genetic marker or haplotype of the present invention results in a change in the composition or expression of the polypeptide. The haplotypes and markers of the present invention that show association to CAD and MI may play a role through their effect on one or more of these nearby genes (*e.g.,* the *Lp(a)* gene). Possible mechanisms affecting these genes include, e.g., effects on transcription, effects on RNA splicing, alterations in relative amounts of alternative splice forms of mRNA, effects on RNA stability, effects on transport from the nucleus to cytoplasm, and effects on the efficiency and accuracy of translation.

Thus, in another embodiment, the variants (markers or haplotypes) of the invention showing association to CAD and/or MI affect the expression of a nearby gene, such as the *Lp(a)* gene. It is well known that regulatory element affecting gene expression may be located far away, even as far as tenths or hundreds of kilobases away, from the promoter region of a gene. By assaying for the presence or absence of at least one allele of at least one polymorphic marker of the present invention, it is thus possible to assess the expression level of such nearby genes. It is thus contemplated that the detection of the markers or haplotypes of the present invention can be used for assessing expression of genes associated with (in LD with) the associated markers.

A variety of methods can be used for detecting protein expression levels, including enzyme linked immunosorbent assays (ELISA), Western blots, immunoprecipitations and immunofluorescence. A test sample from a subject is assessed for the presence of an alteration in the expression and/or an alteration in composition of the polypeptide encoded by a nucleic acid associated with CAD and/or MI. An alteration in expression of a polypeptide encoded by a nucleic acid associated with CAD and/or MI can be, for example, an alteration in the quantitative polypeptide expression (i.e., the amount of polypeptide produced). An alteration in the composition of a polypeptide encoded by a nucleic acid associated with CAD and/or MI is an alteration in the qualitative polypeptide expression (e.g., expression of a mutant polypeptide or of a different splicing variant). In one embodiment, diagnosis of a susceptibility to CAD and/or MI is made by detecting a particular splicing variant encoded by a nucleic acid associated with these diseases, or a particular pattern of splicing variants.

Both such alterations (quantitative and qualitative) can also be present. An "alteration" in the polypeptide expression or composition, as used herein, refers to an alteration in expression or composition in a test sample, as compared to the expression or composition of the polypeptide in a control sample. A control sample Is a sample that corresponds to the test sample (e.g., is from the same type of cells), and is from a subject who is not affected by, and/or who does not have a susceptibility to, CAD and/or MI. In one embodiment, the control sample is from a subject that does not possess a marker allele or haplotype associated with CAD and/or MI, as described herein. Similarly, the presence of one or more different splicing variants in the test sample, or the presence of significantly different amounts of different splicing variants in the test sample, as compared with the control sample, can be indicative of a susceptibility to CAD and/or MI. An alteration in the expression or composition of the polypeptide in the test sample, as compared with the control sample, can be indicative of a specific allele in the instance where the allele alters a splice site relative to the reference in the control sample. Various means of examining expression or composition of a polypeptide encoded by a nucleic acid are known to the person skilled in the art and can be used, including spectroscopy, colorimetry, electrophoresis, isoelectric focusing, and immunoassays (e.g., David et al., U.S. Pat. No. 4,376,110) such as immunoblotting (see, e.g., Current Protocols in Molecular Biology, particularly chapter 10, *supra*).

For example, in one embodiment, an antibody (e.g., an antibody with a detectable label) that is capable of binding to a polypeptide encoded by a nucleic acid associated with CAD and/or MI can be used. Antibodies can be polyclonal or monoclonal. An intact antibody, or a fragment thereof (e.g., Fv, Fab, Fab', F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a labeled secondary antibody (e.g., a fluorescently-labeled secondary antibody) and end-labeling of a DNA probe with biotin such that it can be detected with fluorescently-labeled streptavidin.

In one embodiment of this method, the level or amount of polypeptide encoded by a nucleic acid associated with CAD and/or MI in a test sample is compared with the level or amount of the polypeptide in a control sample. A level or amount of the polypeptide in the test sample that is higher or lower than the level or amount of the polypeptide in the control sample, such that the difference is statistically significant, is indicative of an alteration in the expression of the polypeptide encoded by the nucleic acid, and is diagnostic for a particular allele or haplotype responsible for causing the difference in expression. Alternatively, the composition of the polypeptide in a test sample is compared with the composition of the polypeptide in a control sample. In another embodiment, both the level or amount and the composition of the polypeptide can be assessed in the test sample and in the control sample.

In another embodiment, the diagnosis of a susceptibility to CAD and/or MI is made by detecting at least one marker or haplotypes of the present invention (e.g., associated alleles of the markers listed in Table 4, and markers in linkage disequilibrium therewith), in combination with an additional protein-based, RNA-based or DNA-based assay.

### Kits

Kits useful in the methods of the invention comprise components useful in any of the methods described herein, including for example, primers for nucleic acid amplification, hybridization probes, restriction enzymes (e.g., for RFLP analysis), allele-specific oligonucleotides, antibodies that bind to an altered polypeptide encoded by a nucleic acid of the invention as described herein (e.g., a genomic segment comprising at least one polymorphic marker and/or haplotype of the present invention) or to a non-altered (native) polypeptide encoded by a nucleic acid of the invention as described herein, means for amplification of a nucleic acid templates, means for analyzing the nucleic acid sequence of a nucleic acids, means for analyzing the amino acid sequence of a polypeptide encoded by a nucleic acid associated with CAD and/or MI, etc. The kits can for example include necessary buffers, nucleic acid primers for amplifying nucleic acids of the invention (e.g., a nucleic acid segment comprising one or more of the polymorphic markers as described herein), and reagents for allele-specific detection of the fragments amplified using such primers and necessary enzymes (e.g., DNA polymerase). Additionally, kits can provide reagents for assays to be used in combination with the methods of the present invention, e.g., reagents for use with other diagnostic assays for CAD and/or MI.

A useful kit with the invention is a kit for assaying a sample from a subject to detect a susceptibility to coronary artery disease and/or myocardial infarction in a subject, wherein the kit comprises reagents necessary for selectively detecting at least one allele of at least one polymorphism of the present invention in the genome of the individual. In a particular embodiment, the reagents comprise at least one contiguous oligonucleotide that hybridizes to a fragment of the genome of the individual comprising at least one polymorphism of the present invention. In another embodiment, the reagents comprise at least one pair of oligonucleotides that hybridize to opposite strands of a genomic segment obtained from a subject, wherein each oligonucleotide primer pair is designed to selectively amplify a fragment of the genome of the individual that includes one polymorphism, wherein the polymorphism is selected from the group consisting of the polymorphisms as defined in and polymorphic markers in linkage disequilibrium therewith. In yet another embodiment the fragment is at least 20 base pairs in size. Such oligonucleotides or nucleic acids (e.g., oligonucleotide primers) can be designed using portions of the nucleic acids flanking polymorphisms (e.g., SNPs or microsatellites) that are indicative of coronary artery disease and myocardial infarction. In another embodiment, the kit comprises one or more labeled nucleic acids capable of detecting one or more specific polymorphic markers or haplotypes associated with coronary artery disease and/or myocardial infarction, and reagents for detection of the label. Suitable labels include, e.g., a radioisotope, a fluorescent label, an enzyme label, an enzyme co-factor label, a magnetic label, a spin label, an epitope label.

In particular embodiments, the polymorphic marker or haplotype to be detected by the reagents of the kit comprises one or more markers, two or more markers, three or more markers, four or more markers or five or more markers. In another embodiment, the marker or haplotype to be detected is marker rs11751605. In such embodiments, the presence of the marker or haplotype is indicative of a susceptibility (increased susceptibility or decreased susceptibility) to cardiovascular disease, including coronary artery disease and myocardial infarction.

In one preferred embodiment, the kit for detecting the markers of the invention comprises a detection oligonucleotide probe, that hybridizes to a segment of template DNA containing a SNP polymorphisms to be detected, an enhancer oligonucleotide probe and an endonuclease. As explained in the above, the detection oligonucleotide probe comprises a fluorescent moiety or group at its 3' terminus and a quencher at its 5' terminus, and an enhancer oligonucleotide, is employed, as described by Kutyavin et al. (Nucleic Acid Res. 34:e128 (2006)). The fluorescent moiety can be Gig Harbor Green or Yakima Yellow, or other suitable fluorescent moieties. The detection probe is designed to hybridize to a short nucleotide sequence that includes the SNP polymorphism to be detected. Preferably, the SNP is anywhere from the terminal residue to -6 residues from the 3' end of the detection probe. The enhancer is a short oligonucleotide probe which hybridizes to the DNA template 3' relative to the detection probe. The probes are designed such that a single nucleotide gap exists between the detection probe and the enhancer nucleotide probe when both are bound to the template. The gap creates a synthetic abasic site that is recognized by an endonuclease, such as Endonuclease IV. The enzyme cleaves the dye off the fully complementary detection probe, but cannot cleave a detection probe containing a mismatch. Thus, by measuring the fluorescence of the released fluorescent moiety, assessment of the presence of a particular allele defined by nucleotide sequence of the detection probe can be performed.

The detection probe can be of any suitable size, although preferably the probe is relatively short. In one embodiment, the probe is from 5-100 nucleotides in length. In another embodiment, the probe is from 10-50 nucleotides in length, and in another embodiment, the probe is from 12-30 nucleotides in length. Other lengths of the probe are possible and within scope of the skill of the average person skilled in the art.

In a preferred embodiment, the DNA template containing the SNP polymorphism is amplified by Polymerase Chain Reaction (PCR) prior to detection, and primers for such amplification are included in the reagent kit. In such an embodiment, the amplified DNA serves as the template for the detection probe and the enhancer probe.

In one embodiment, the DNA template is amplified by means of Whole Genome Amplification (WGA) methods, prior to assessment for the presence of specific polymorphic markers as described herein. Standard methods well known to the skilled person for performing WGA may be utilized, and are within scope of the invention. In one such embodiment, reagents for performing WGA are included in the reagent kit.

Certain embodiments of the detection probe, the enhancer probe, and/or the primers used for amplification of the template by PCR include the use of modified bases, including modified A and modified G. The use of modified bases can be useful for adjusting the melting temperature of the nucleotide molecule (probe and/or primer) to the template DNA, for example for increasing the melting temperature in regions containing a low percentage of G or C bases, in which modified A with the capability of forming three hydrogen bonds to its complementary T can be used, or for decreasing the melting temperature in regions containing a high percentage of G or C bases, for example by using modified G bases that form only two hydrogen bonds to their complementary C base in a double stranded DNA molecule. In a preferred embodiment, modified bases are used in the design of the detection nucleotide probe. Any modified base known to the skilled person can be selected in these methods, and the selection of suitable bases is well within the scope of the skilled person based on the teachings herein and known bases available from commercial sources as known to the skilled person.

In one of such embodiments, the presence of the marker or haplotype is indicative of a susceptibility (increased susceptibility or decreased susceptibility) to CAD and/or MI. In another embodiment, the presence of the marker or haplotype is indicative of response to a therapeutic agent for a cardiovascular disease, such as CAD and MI. In another embodiment, the presence of the marker or haplotype is indicative of prognosis of MI and/or CAD. In yet another embodiment, the presence of the marker or haplotype is indicative of progress of treatment of CAD and/or MI. Such treatment may include intervention by surgery, medication or by other means (e.g., lifestyle changes).

A useful kit is a pharmaceutical pack (kit), the pack comprising a therapeutic agent and a set of instructions for administration of the therapeutic agent to humans diagnostically tested for one or more variants of the present invention, as disclosed herein. The therapeutic agent can be a small molecule drug, an antibody, a peptide, an antisense or RNAi molecule, or other therapeutic molecules. In one embodiment, an individual identified as a carrier of at least one variant of the present invention is instructed to take a prescribed dose of the therapeutic agent. In one such embodiment, an individual identified as a homozygous carrier of at least one variant of the present invention is instructed to take a prescribed dose of the therapeutic agent. In another embodiment, an individual identified as a non-carrier of at least one variant of the present invention is instructed to take a prescribed dose of the therapeutic agent.

In certain embodiments, the kit further comprises a set of instructions for using the reagents comprising the kit.

### Therapeutic agents

Variants of the present invention can be used to identify novel therapeutic targets for coronary artery disease or myocardial infarction, or other cardiovascular disease. For example, genes containing, or in linkage disequilibrium with, variants (markers and/or haplotypes) associated with cardiovascular disease, or their products (e.g., Lipoprotein(a) gene and its gene product), as well as genes or their products that are directly or indirectly regulated by or interact with these genes or their products, can be targeted for the development of therapeutic agents to treat cardiovascular disease, or prevent or delay onset of symptoms associated with cardiovascular disease. Therapeutic agents may comprise one or more of, for example, small non-protein and non-nucleic acid molecules, proteins, peptides, protein fragments, nucleic acids (DNA, RNA), PNA (peptide nucleic acids), or their derivatives or mimetics which can modulate the function and/or levels of the target genes or their gene products.

The nucleic acids and/or variants, or nucleic acids comprising their complementary sequence, may be used as antisense constructs to control gene expression in cells, tissues or organs. The methodology associated with antisense techniques is well known to the skilled artisan, and is described and reviewed in AntisenseDrug Technology: Principles, Strategies, and Applications, Crooke, ed., Marcel Dekker Inc., New York (2001). In general, antisense nucleic acid molecules are designed to be complementary to a region of mRNA expressed by a gene, so that the antisense molecule hybridizes to the mRNA, thus blocking translation of the mRNA into protein. Several classes of antisense oligonucleotide are known to those skilled in the art, including cleavers and blockers. The former bind to target RNA sites, activate intracellular nucleases (e.g., RnaseH or Rnase L), that cleave the target RNA. Blockers bind to target RNA, inhibit protein translation by steric hindrance of the ribosomes. Examples of blockers include nucleic acids, morpholino compounds, locked nucleic acids and methylphosphonates (Thompson, Drug Discovery Today, 7:912-917 (2002)). Antisense oligonucleotides are useful directly as therapeutic agents, and are also useful for determining and validating gene function, for example by gene knock-out or gene knock-down experiments. Antisense technology is further described in Lavery et al., Curr. Opin. Drug Discov. Devel. 6:561-569 (2003), Stephens et al., Curr. Opin. Mol. Ther. 5:118-122 (2003), Kurreck, Eur. J. Biochem. 270:1628-44 (2003), Dias et al., Mol. Cancer Ter. 1:347-55 (2002), Chen, Methods Mol. Med. 75:621-636 (2003), Wang et al., Curr. Cancer Drug Targets 1:177-96 (2001), and Bennett, Antisense Nucleic Acid Drug.Dev. 12:215-24 (2002)

The variants described herein can be used for the selection and design of antisense reagents that are specific for particular variants. Using information about the variants described herein, antisense oligonucleotides or other antisense molecules that specifically target mRNA molecules that contain one or more variants of the invention can be designed. In this manner, expression of mRNA molecules that contain one or more variant of the present invention (markers and/or haplotypes) can be inhibited or blocked. In one embodiment, the antisense molecules are designed to specifically bind a particular allelic form (i.e., one or several variants (alleles and/or haplotypes)) of the target nucleic acid, thereby inhibiting translation of a product originating from this specific allele or haplotype, but which do not bind other or alternate variants at the specific polymorphic sites of the target nucleic acid molecule.

As antisense molecules can be used to inactivate mRNA so as to inhibit gene expression, and thus protein expression, the molecules can be used to treat a disease or disorder, such as a cardiovascular disease. The methodology can involve cleavage by means of ribozymes containing nucleotide sequences complementary to one or more regions in the mRNA that attenuate the ability of the mRNA to be translated. Such mRNA regions include, for example, protein-coding regions, in particular protein-coding regions corresponding to catalytic activity, substrate and/or ligand binding sites, or other functional domains of a protein.

The phenomenon of RNA interference (RNAi) has been actively studied for the last decade, since its original discovery in C. *elegans* (Fire et al., Nature 391:806-11 (1998)), and in recent years its potential use in treatment of human disease has been actively pursued (reviewed in Kim & Rossi, Nature Rev. Genet. 8:173-204 (2007)). RNA interference (RNAi), also called gene silencing, is based on using double-stranded RNA molecules (dsRNA) to turn off specific genes. In the cell, cytoplasmic double-stranded RNA molecules (dsRNA) are processed by cellular complexes into small interfering RNA (siRNA). The siRNA guide the targeting of a protein-RNA complex to specific sites on a target mRNA, leading to cleavage of the mRNA (Thompson, Drug Discovery Today, 7:912-917 (2002)). The siRNA molecules are typically about 20, 21, 22 or 23 nucleotides in length. Thus, one aspect of the invention relates to isolated nucleic acid molecules, and the use of those molecules for RNA interference, i.e. as small interfering RNA molecules (siRNA). In one embodiment, the isolated nucleic acid molecules are 18-26 nucleotides in length, preferably 19-25 nucleotides in length, more preferably 20-24 nucleotides in length, and more preferably 21, 22 or 23 nucleotides in length.

Another pathway for RNAi-mediated gene silencing originates in endogenously encoded primary microRNA (pri-miRNA) transcripts, which are processed in the cell to generate precursor miRNA (pre-miRNA). These miRNA molecules are exported from the nucleus to the cytoplasm, where they undergo processing to generate mature miRNA molecules (miRNA), which direct translational inhibition by recognizing target sites in the 3' untranslated regions of mRNAs, and subsequent mRNA degradation by processing P-bodies (reviewed in Kim & Rossi, Nature Rev. Genet. 8:173-204 (2007)).

Clinical applications of RNAi include the incorporation of synthetic siRNA duplexes, which preferably are approximately 20-23 nucleotides in size, and preferably have 3' overlaps of 2 nucleotides. Knockdown of gene expression is established by sequence-specific design for the target mRNA. Several commercial sites for optimal design and synthesis of such molecules are known to those skilled in the art.

Other applications provide longer siRNA molecules (typically 25-30 nucleotides in length, preferably about 27 nucleotides), as well as small hairpin RNAs (shRNAs; typically about 29 nucleotides In length). The latter are naturally expressed, as described in Amarzguioui et al. (FEBS Lett. 579:5974-81 (2005)). Chemically synthetic siRNAs and shRNAs are substrates for *in vivo* processing, and in some cases provide more potent gene-silencing than shorter designs (Kim et al., Nature Biotechnol. 23:222-226 (2005); Siolas et al., Nature Biotechnol. 23:227-231 (2005)). In general siRNAs provide for transient silencing of gene expression, because their intracellular concentration is diluted by subsequent cell divisions. By contrast, expressed shRNAs mediate long-term, stable knockdown of target transcripts, for as long as transcription of the shRNA takes place (Marques et al., Nature Biotechnol. 23:559-565 (2006); Brummelkamp et al., Science 296: 550-553 (2002)).

Since RNAi molecules, including siRNA, miRNA and shRNA, act in a sequence-dependent manner, the variants of the present invention (e.g., the markers and haplotypes set forth in Tables 3, 4 and 5) can be used to design RNAi reagents that recognize specific nucleic acid molecules comprising specific alleles and/or haplotypes (*e.g.,* the alleles and/or haplotypes of the present invention), while not recognizing nucleic acid molecules comprising other alleles or haplotypes. These RNAi reagents can thus recognize and destroy the target nucleic acid molecules. As with antisense reagents, RNAi reagents can be useful as therapeutic agents (i.e., for turning off disease-associated genes or disease-associated gene variants), but may also be useful for characterizing and validating gene function (*e.g.,* by gene knock-out or gene knock-down experiments).

Delivery of RNAi may be performed by a range of methodologies known to those skilled in the art. Methods utilizing non-viral delivery include cholesterol, stable nucleic acid-lipid particle (SNALP), heavy-chain antibody fragment (Fab), aptamers and nanoparticles. Viral delivery methods include use of lentivirus, adenovirus and adeno-associated virus. The siRNA molecules are in some embodiments chemically modified to increase their stability. This can include modifications at the 2' position of the ribose, including 2'-O-methylpurines and 2'-fluoropyrimidines, which provide resistance to Rnase activity. Other chemical modifications are possible and known to those skilled in the art.

The following references provide a further summary of RNAi, and possibilities for targeting specific genes using RNAi: Kim & Rossi, Nat. Rev. Genet. 8:173-184 (2007), Chen & Rajewsky, Nat. Rev. Genet. 8: 93-103 (2007), Reynolds, et al., Nat. Biotechnol. 22:326-330 (2004), Chi et al., Proc. Natl. Acad. Sci. USA 100:6343-6346 (2003), Vickers et al., J. Biol. Chem. 278:7108-7118 (2003), Agami, Curr. Opin. Chem. Biol. 6:829-834 (2002), Lavery, et al., Curr. Opin. Drug Discov, Devel. 6:561-569 (2003), Shi, Trends Genet. 19:9-12 (2003), Shuey et al., Drug Discov. Today 7:1040-46 (2002), McManus et al., Nat. Rev. Genet. 3:737-747 (2002), Xia et al., Nat. Biotechnol. 20:1006-10 (2002), Plasterk et al., curr. Opin. Genet. Dev. 10:562-7 (2000), Bosher et al., Nat. Cell Biol. 2: E31-6 (2000), and Hunter, Curr. Biol. 9:R440-442 (1999).

A genetic defect leading to increased predisposition or risk for development of a cardiovascular disease, or a defect causing the disease, may be corrected permanently by administering to a subject carrying the defect a nucleic acid fragment that incorporates a repair sequence that supplies the normal/wild-type nucleotide(s) at the site of the genetic defect. Such site-specific repair sequence may concompass an RNA/DNA oligonucleotide that operates to promote endogenous repair of a subject's genomic DNA. The administration of the repair sequence may be performed by an appropriate vehicle, such as a complex with polyethelenimine, encapsulated in anionic liposomes, a viral vector such as an adenovirus vector, or other pharmaceutical compositions suitable for promoting intracellular uptake of the adminstered nucleic acid. The genetic defect may then be overcome, since the chimeric oligonucleotides induce the incorporation of the normal sequence into the genome of the subject, leading to expression of the normal/wild-type gene product. The replacement is propagated, thus rendering a permanent repair and alleviation of the symptoms associated with the disease or condition.

Also disclosed methods for identifying compounds or agents that can be used to treat cardiovascular diseases, such as coronary artery disease and myocardial infarction. Thus, the variants of the invention are useful as targets for the identification and/or development of therapeutic agents. In certain embodiments, such methods include assaying the ability of an agent or compound to modulate the activity and/or expression of a nucleic acid that includes at least one of the variants (markers and/or haplotypes) of the present invention, or the encoded product of the nucleic acid (*e.g,* the *Lp(a)* gene). This in turn can be used to identify agents or compounds that inhibit or alter the undesired activity or expression of the encoded nucleic acid product. Assays for performing such experiments can be performed in cell-based systems or in cell-free systems, as known to the skilled person. Cell-based systems include cells naturally expressing the nucleic acid molecules of interest, or recombinant cells that have been genetically modified so as to express a certain desired nucleic acid molecule.

Variant gene expression in a patient can be assessed by expression of a variant-containing nucleic acid sequence (for example, a gene containing at least one variant of the present invention, which can be transcribed into RNA containing the at least one variant, and in turn translated into protein), or by altered expression of a normal/wild-type nucleic acid sequence due to variants affecting the level or pattern of expression of the normal transcripts, for example variants in the regulatory or control region of the gene. Assays for gene expression include direct nucleic acid assays (mRNA), assays for expressed protein levels, or assays of collateral compounds involved in a pathway, for example a signal pathway. Furthermore, the expression of genes that are up- or down-regulated in response to the signal pathway can also be assayed. One embodiment includes operably linking a reporter gene, such as luciferase, to the regulatory region of the gene(s) of interest.

Modulators of gene expression can in one embodiment be identified when a cell is contacted with a candidate compound or agent, and the expression of mRNA is determined. The expression level of mRNA in the presence of the candidate compound or agent is compared to the expression level in the absence of the compound or agent. Based on this comparison, candidate compounds or agents for treating cardiovascular disease can be identified as those modulating the gene expression of the variant gene. When expression of mRNA or the encoded protein is statistically significantly greater in the presence of the candidate compound or agent than in its absence, then the candidate compound or agent is identified as a stimulator or up-regulator of expression of the nucleic acid. When nucleic acid expression or protein level is statistically significantly less in the presence of the candidate compound or agent than in its absence, then the candidate compound is identified as an inhibitor or down-regulator of the nucleic acid expression.

### Methods of assessing probability of response to therapeutic agents and methods, methods of monitoring treatment progress and methods for treating cardiovascular disease

As is known in the art, individuals can have differential responses to a particular therapy (e.g., a therapeutic agent or therapeutic method). Pharmacogenomics addresses the issue of how genetic variations (*e.g.,* the variants (markers and/or haplotypes) of the present invention) affect drug response, due to altered drug disposition and/or abnormal or altered action of the drug. Thus, the basis of the differential response may be genetically determined in part. Clinical outcomes due to genetic variations affecting drug response may result in toxicity of the drug in certain individuals (*e*.*g*., carriers or non-carriers of the genetic variants of the present invention), or therapeutic failure of the drug. Therefore, the variants of the present invention may determine the manner in which a therapeutic agent and/or method acts on the body, or the way in which the body metabolizes the therapeutic agent.

Accordingly, in one embodiment, the presence of a particular allele of a polymorphic marker, or the presence of a haplotype as described herein is indicative of a different response rate to a particular treatment modality for coronary artery disease or myocardial infarction. This means that a patient diagnosed with coronary artery disease or myocardial infarction, or at risk for coronary artery disease or myocardial infarction, and carrying a certain allele at a polymorphic or haplotype of the present invention (*e.g.,* the at-risk alleles and/or haplotypes of the invention) would respond better to, or worse to, a specific therapeutic, drug and/or other therapy used to treating these diseases. Therefore, the presence or absence of the marker allele or haplotype could aid in deciding what treatment is appropriate for the patient. For example, for a newly diagnosed patient, the presence of a marker or haplotype of the present invention may be assessed (*e.g.,* through testing DNA derived from a blood sample or other sample containing genomic DNA, as described herein). If the patient is positive for a marker allele or haplotype at (that is, at least one specific allele of the marker, or haplotype, is present), then the physician recommends one particular therapy (*e.g.,* one particular therapeutic agent or a combination of therapeutic agents), while if the patient is negative for the at least one allele of a marker, or a haplotype, then a different course of therapy may be recommended (which may include recommending that no immediate therapy, other than serial monitoring for progression of the disease, be performed). Thus, the patient's carrier status could be used to help determine whether a particular treatment modality should be administered. The value lies within the possibilities of being able to diagnose the disease at an early stage and provide information to the clinician about prognosis/aggressiveness of the disease in order to be able to apply the most appropriate treatment.

As one example, the application of a genetic test can identify subjects who are at high risk of developing restenosis after coronary stent procedure. While it is know that some treatment methods for coronary artery disease, such as introducing drug-eluting stents and brachytherapy, are associated with decreased risk of in-stent restenosis, the use of these methods are restricted because of number of reasons, including economical reasons. Identification of individuals within the group of those undergoing coronary stent procedure who are carriers of particular genetic risk variants will allow targeting of those individuals that would benefit most from therapy associated with decreased risk of in-stent restenosis.

Also disclosed methods of monitoring effectiveness of a treatment for coronary artery disease and/or myocardial infarction. This can be done based on the genotype and/or haplotype status of the markers and haplotypes of the present invention, or by monitoring expression of genes that are associated with the variants (markers and haplotypes) of the present invention (*e.g*., the *Lp(a)* gene). The risk gene mRNA or the encoded polypeptide can be measured in a tissue sample (*e.g.,* a peripheral blood sample, or a biopsy sample). Expression levels and/or mRNA levels can thus be determined before and during treatment to monitor its effectiveness. Alternatively, or concomitantly, the genotype and/or haplotype status of at least one risk variant for coronary artery disease or myocardial infarction as presented herein is determined before and during treatement to monitor its effectivenes.

The treatment modules of coronary artery disease and/or myocardial infarction to which the invention pertains includes, but is not limited to, methods of treatment for coronary artery disease or myocardial infarction or a susceptibility to coronary artery disease or myocardial infarction; methods of phophylaxis therapy *for coronary* artery disease or myocardial infarction; methods of treatment for acute coronary syndrome (*e.g.,* unstable angina, non-ST-elevation myocardial infarction (NSTEMI) or ST-elevation myocardial infarction (STEMI)); methods for reducing risk of coronary artery disease and/or myocardial infarction; methods for decreasing risk of a second myocardial infarction; methods of treatment for atherosclerosis, such as for patients requiring treatment (*e.g.,* angioplasty, stents, revascularization procedure) to restore blood flow in arteries *(e.g.,* coronary, carotid, and/or femoral arteries); and/or methods for decreasing leukotriene synthesis (*e.g.,* for treatment of coronary artery disease or myocardial infarction).

Treatment of coronary artery disease and myocardial infarction (MI) may be categorized as (i) preventive treatment and (ii) disease management. The *main* goal of the latter is to minimize damage to the heart and prevent further complications. The first line of disease management typically includes one or more of adminstration of oxygen, aspirin, glyceryl nitrate (nitroglycerin) and analgesia, such as morphine or related drugs. Once diagnosis of MI is made, additional therapy may include beta blockers, anticoagulation agents, including heparin and/or low molecular weigth heparin, and possibly also antiplatelet agents, such as clopidogrel. Secondary prevention, i.e. the management of risk of a recurrent MI, typically includes one or more of the following: Antiplatelet drug therapy, including aspirin and/or clopidogrel, beta blocker therapy, including metoprolol and carvedilol, ACE inhibitor therapy, Statin therapy, Aldosterone antagonist therapy, including eplerenone. Further, non-therapeutic administration of food supplements such as omega-3 fatty acids may be benefitional.

New preventive therapy for coronary artery disease and/or myocardial infarction, includes agents that act on the formation and/or rupture of plaques, and also includes phosphodiesterase inhibitors. Such therapeutic agents are useful in the methods of the invention, as described herein. This includes, but is not limited to, agents that target the leukotriene synthesis pathway. The leukotriene synthesis inhibitor can be any agent that inhibits or antagonizes a member of the leukotriene synthesis pathway (*e.g.,* FLAP, 5-LO, LTC4S, LTA4H, and LTB4DH). For example, the leukotriene synthesis inhibitor can be an agent that inhibits or antagonizes FLAP polypeptide activity (*e.g.,* a FLAP inhibitor) and/or FLAP nucleic acid expression (*e.g.,* a FLAP nucleic acid antagonist). In another embodiment, the leukotriene synthesis inhibitor is an agent that inhibits or antagonizes polypeptide activity and/or nucleic acid expression of another member of the leukotriene biosynthetic pathway *(e.g.,* LTC4S, LTA4H) or that increases breakdown of leukotrienes *(e.g.,* LTB4DH). In preferred embodiments, the agent alters activity and/or nucleic acid expression of FLAP, LTA4H or of 5-LO. Preferred agents include those set forth in the Agent Table I herein. In another embodiment, preferred agents can be: 1-((4-chlorophenyl)methyl)-3-((1,1-dimethylethyl)thio)-alpha,alpha-dimethyl-5-(2-quinolinylmethoxy)- 1H-Indole-2-propanoic acid otherwise known as MK-0591, (R)-(+)-alpha-cyclopentyl-4-(2-quinolinylmethoxy)-Benzeneacetic acid, otherwise known as BAY-x-1005, 3-(3-(1,1-dimethylethylthio-5-(quinoline-2-ylmethoxy)-1-(4-chloromethylphenyl)indole-2-yl)-2,2-dimethylpropionaldehyde oxime-0-2-acetic acid otherwise known as A-81834; or can be zileuton, atreleuton, 6-((3-fluoro-5-(tetrahydro-4-methoxy-2H-pyran-4yl)phenoxy)methyl)-1-methyl-2(1H)-quinlolinone otherwise known as ZD-2138, 1-((4-chlorophenyl)methyl)-3-((1,1dimethylethyl)thio)-alpha,alpha-dimethyl-5-(2-quinolinylmethoxy)-1H-Indole-2-propanoic acid otherwise known as MK-886, 4-(3-(4-(2-Methyl-imidazol-1-yl)-phenylsulfanyl)-phenyl)-tetrahydro-pyran-4-carboxylic acid amide otherwise known as CJ-13610: Additional agents include those described in Penning et al., Med Chem. 2002 45(16):3482-90, Penning, Curr Pharm Des. 2001, 7(3):163-79 and Penning et al., J Med Chem. 2000 43(4):721-35.In another embodiment, the agent alters metabolism or activity of a leukotriene (*e.g.,* LTA4, LTB4, LTC4, LTD4, LTE4, Cys LT1, Cys LT2), such as leukotriene antagonists or antibodies to leukotrienes, as well as agents which alter activity of a leukotriene receptor (*e.g.,* BLT1, BLT2, CysLTR1, and CysLTR2).

In other preferred embodiments, the agent alters activity and/or nucleic acid expression of LTA4H. Preferred agents include those set forth in the Agent Table II; but also include the following agents: 1-[2-[4-(phenylmethyl)phenoxy]ethyl]-2-methyl-4-tetrazolylpieridine; 1-[2-[4-(4-oxazolyl)phenoxy)phenoxy]ethyl]pyrrolidine; 3-[methyl[3-[4-(2-thienylmethyl)phenoxy]propyl]amino]propionic acid; methyl 3-[methyl[3-[4-(2-thienylmethyl)phenoxy]propyl]amino]propionate; 3-[methyl[3-[4-(3-thienylmethyl)phenoxy]propyl]amino]propionic acid; methyl-3-[methyl[3-4-(3-theinylmethyl)phenoxy]propyl]amino]propionate; 3-[methyl[3-[4-(4-fluorophenoxy)phenoxy]propyl]amino]propionic acid; 3-[methyl[3-[4-(4-biphenyloxy)phenoxy]propyl]amino]propionic acid; N-[3-[[3-[4-(phenylmethyl)phenoxy] propyl]methylamino]propionyl]benzenesulfonamide; 1-[2-[4-(phenylmethyl)phenoxy]ethyl]-2-methyl-4-(1H-tetrazol-5-yl)piperidine; 1-[2-[4-(phenylmethyl)phenoxy]ethyl]-4-(1H-tetrazol-5-yl)piperidine. In another embodiment, preferred agents can be: ethyl-1-[2-[4-(phenylmethyl)phenoxy]ethyl]-4-piperidine-carboxylate, otherwise known as SC-56938; [4-[5-(3-Phenyl-propyl)thiophen-2-yl]butoxy]acetic acid, otherwise known as RP64966; (R)-S-[[4-(dimethylamino)phenyl]methyl]-N-(3-mercapto-2methyl-1-oxopropyl-L-cycteine, otherwise known as SA6541. In one preferred embodiment, the therapeutic agent is 4-{(S)-2-[4-(4-Chloro-phenoxy)-phenoxymethyl]-pyrrolidin-1-yl}-butyramide, also known as DG-051.

The agents for treating or preventing coronary artery disease and/or myocardial infarction can be adminstered alone, or in combination with a statin. Statins include, but are not limited to, the agents rovuvastatin, fluvastatin, atorvastatin, lovastatin (also known as mevolin), simvastatin, pravastatin, pitavastatin, mevastatin, crevastatin, ML-236A, ML-236B, MBV-530A and MB-530B.

All agents listed in the above and in Agent Table I and Agent Table II also include their optically pure enantiomers, salts, chemical derivatives, and analogues.

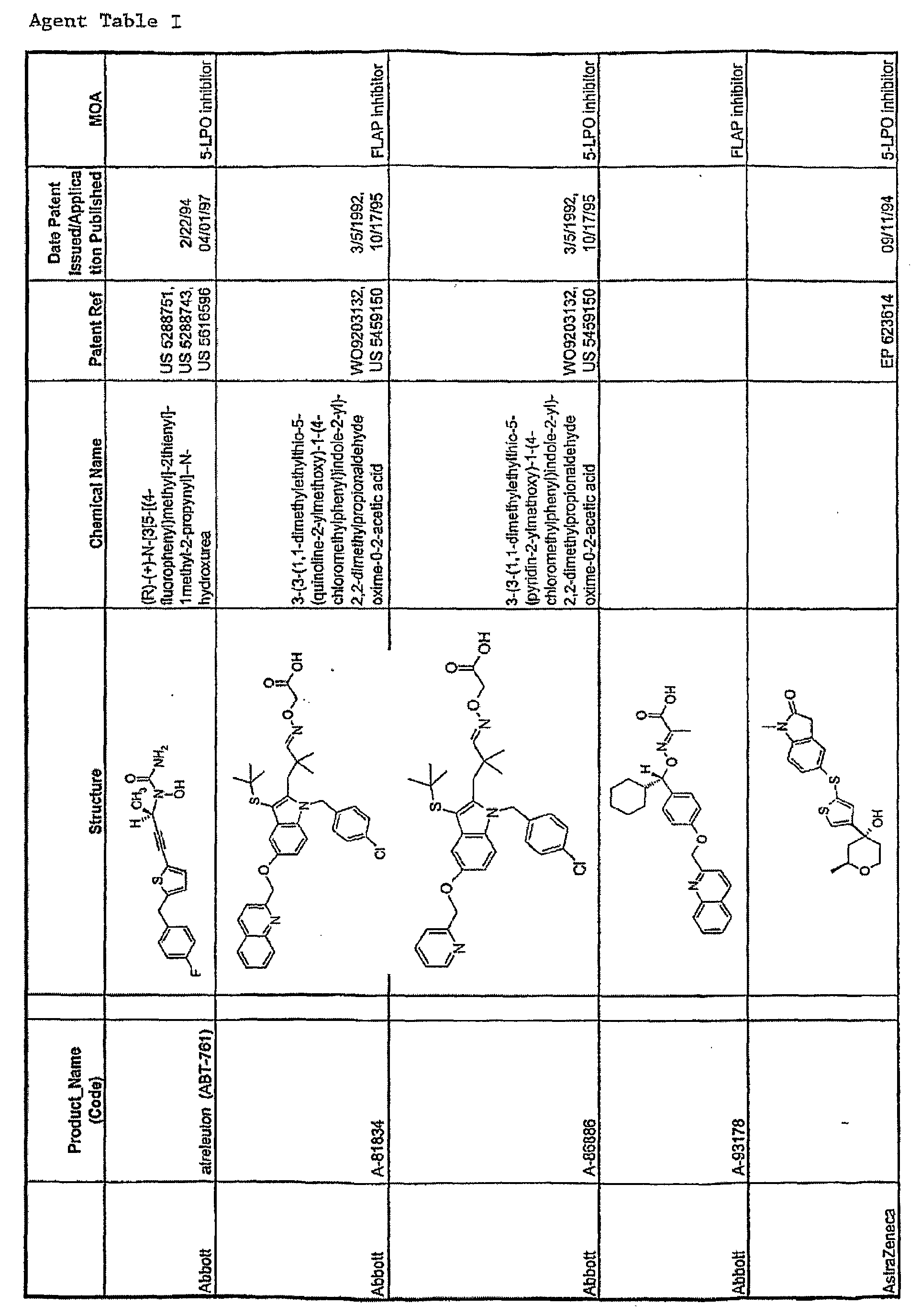

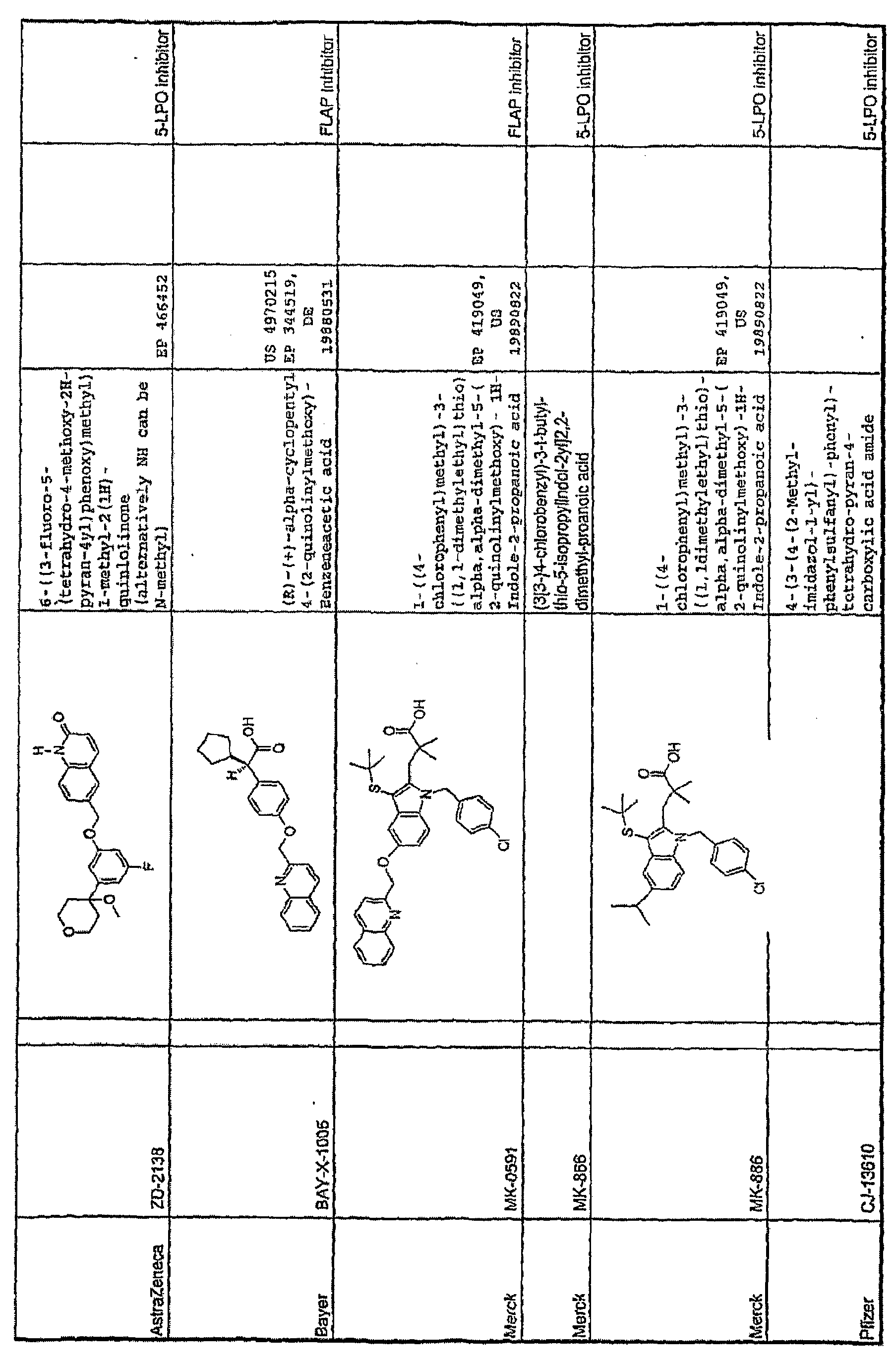

**Agent Table II**

| **Target** | **Compound ID** | **Chemical Name** | **Patent / Reference** |
|---|---|---|---|
| LTA4H Inhibitor | SC-57461A | 3-[methyl[3-[4-(phenylmethyl)phenoxy]-propyl]amino]propionic acid | Penning, T.D. et.al. Bioorg Med. Chem. Letters (2003), 13, 1137-1139. |
| | | | ibid, (2002), 12, 3383-3386 |
| LTA4H Inhibitor | SC-56938 | Ethyl-1-[2-[4-(phenylmethyl)phenoxy]et hyl]-4-piperidine-carboxylate | Penning, T.D. et.al. Bioorg Med. Chem. Letters (2003), 13, 1137-1139. |
| | | | ibid, (2002), 12, 3383-3386. US 6,506,87 6A1 |
| LTA4H Inhibitor | RP 64966 | [4-[5-(3-Phenyl-propyl)thiophen-2-yl]butoxy]acetic acid | WO9627585 |
| LTA4H Inhibitor | SA 6541 | (R)-S-[[4-(dimethylamino)phenyl]m ethyl]-N-(3-mercapto-2methyl-1-oxopropyl-L-cycteine | WO9809943 |
| LTA4H Inhibitor | SA-9499/ SA-6541 | (R)-3-(4-Dimethylamino-benzylsulfanyl)-2-((R)-3-mercapto-2-methyl-propionylamino)-propionic acid | |
| LTB4 Receptor Antagonist | Amelubant/ BIIL-284 | Carbamic acid,((4-((3-((4-(1-(4-hydroxyphenyl)-1-methylethyl)phenoxy)met hyl)phenyl)me thoxy)phenyl)iminomethyl )- ethyl ester | US 6,576,669 |
| LTB4 Receptor Antagonist | BIRZ-227 | 5-Chloro-2-[3-(4-methoxy-phenyl)-2-pyridin-2-yl-pyrrolidin-1-yl]-benzooxazole | Journal of Organic Chemistry 1998,63:2(326-330). |
| LTB4 Receptor Antagonist | CP 195543 | 2-[(3S,4R)-3,4-dihydro-4-hydroxy-3-(phenylmethyl)-2H-1-benzopyran-7-yl]-4-(trifluoromethyl) benzoic acid | Process: WO 98/11085 1998, priority US 60/26372 1996; J. |
| | | | Pharamacology and Expert. Therapy, 1998, 285: 946-54 |
| LTB4 Receptor Antagonist | Ebselen | 2-Phenyl-benzo[d]isoselenazol-3-one | Journal of Cerebral Blood Flow and Metabolism 1995, July 2-6 (S162); Drugs of the Future 1995, 20: 10 (1057) |
| LTB4 Receptor Antagonist | LTB 019; CGS-25019C | 4-[5-(4-Carbamimidoyl-phenoxy)-pentyloxy]-N,N-diisopropyl-3-methoxy-benzamide maleate | ACS Meeting 1994,207th:San Diego (MEDI 003); International Congress of the Inflammation Research Association 1994, 7th:White Haven (Abs W23) |
| LTB4 Receptor Antagonist | LY 210073 | 5-(2-Carboxy-ethyl)-6-[6-(4-methoxy-phenyl)-hex-5-enyloxy]-9-oxo-9H-xanthene-2-carboxylic acid | J Med Chem 1993 36 (12) 1726-1734 |
| LTB4 Receptor Antagonist | LY 213024 | 5-(3-carboxybenzoyl)-2-(decyloxy)benzenepropan oic acid | J Med Chem 1993 36 (12) 1726-1734 |
| LTB4 Receptor Antagonist | LY 255283 | 1-[5-ethyl-2-hydroxy-4-[[6-methyl-6-(1H-tetrazol-5-yl)heptyl]oxy]ph enyl]ethanone | EP 276064 B 1990, priority US 2479 1987 |
| LTB4 Receptor Antagonist | LY 264086 | 7-carboxy-3-(decyloxy)-9-oxo-9H-xanthene-4-propanoic acid | US 4996230 1991, priority US 481413 1990 |
| LTB4 Receptor Antagonist | LY 292728 | 7-carboxy-3-[3-[(5-ethyl-4'-fluoro-2-hydroxy[1,1'-biphenyl]-4-yl)ox y]propoxy]-9-oxo-9H-xanthene-4-propanoic acid disodium salt | EP 743064 A 1996, priority US 443179 1995 |
| LTB4 Receptor Antagonist | LY-293111 (VML-295) | Benzoic acid, 2-(3-(3-((5-ethyl-4'-fluoro-2-hydroxy(1,1'-biphenyl)-4-yl)oxy)propoxy)-2-propylphenoxy)- | Proceedings of the American Society for Clinical Oncology 2002, 21:1 (Abs 343) [LY-293111 for Cancer] SCRIP World Pharmaceutical News 1997,2272 (13) [for VML-295] |
| LTB4 Receptor Antagonist | ONO 4057; LB 457 | (E)-2-(4-carboxybutoxy)-6-[[6-(4-methoxyphenyl)-5-hexenyl]oxy]benzeneprop anoic acid | EP 405116 A 1991 |
| LTB4 Receptor Antagonist | PF 10042 | 1-[5-hydroxy-5-[8-(1-hydroxy-2-phenylethyl)-2-dibenzofuranyl]-1-oxo pentyl]pyrrolidine | EP 422329 B 1995, priority US 409630 1989 |
| LTB4 Receptor Antagonist | RG-14893 | 8-Benzyloxy-4-[(methyl-phenethyl-carbamoyl)-methyl]-naphthalene-2-carboxylic acid | SCRIP World Pharmaceutical News 1996,2168 (20) |
| LTB4 Receptor Antagonist | SB-201993 | 3-{6-(2-Carboxy-vinyl)-5-[8-(4-methoxy-phenyl)-octyloxy]-pyridin-2-ylmethylsulfanylmethyl}-benzoic acid | WO-09500487 |
| LTB4 Receptor Antagonist | SC-52798 | 7-[3-(2-Cyclopropylmethyl-3-methoxy-4-thiazol-4-yl-phenoxy)-propoxy]-8-propyl-chroman-2-carboxylic acid | Bioorganic and Medicinal Chemistry Letters 1994,4:6 (811-816); Journal of Medicinal Chemistry 1995, 38:6 (858-868) |
| LTB4 Receptor Antagonist | SC-53228 | 3-{7-[3-(2-Cyclopropylmethyl-3-methoxy-4-methylcarbamoyl-phenoxy)-propoxy]-8-propyl-chroman-2-yl}-propionic acid | International Congress of the Inflammation Research Association 1994,7th:White Haven (Abs W5) |
| LTB4 Receptor Antagonist | WAY 121006 | 3-fluoro-4'-(2-quinolinylmethoxy)-[1,1'-biphenyll-4-acetic acid | Drugs under Experimental and Clinical research 1991,17:8 (381-387) |
| LTB4 Receptor Antagonist | ZD-2138 | 3-Amino-3-(4-methoxy-tetrahydro-pyran-4-yl)-acrylic acid 1-methyl-2-oxo-1,2-dihydro-quinolin-6-ylmethyl ester | International Symposium on Medicinal Chemistry 1994,13th:Paris (P 197) |

Alternatively, biological networks or metabolic pathways related to the markers and haplotypes of the present invention can be monitored by determining mRNA and/or polypeptide levels. This can be done for example, by monitoring expression levels or polypeptides for several genes belonging to the network and/or pathway, in samples taken before and during treatment. Alternatively, metabolites belonging to the biological network or metabolic pathway can be determined before and during treatment. Effectiveness of the treatment is determined by comparing observed changes in expression levels/metabolite levels during treatment to corresponding data from healthy subjects.

In a further aspect, the markers can be used to increase power and effectiveness of clinical trials. Thus, individuals who are carriers of the at-risk variants of the present invention may be more likely to respond to a particular treatment modality. In one embodiment, individuals who carry at-risk variants for gene(s) in a pathway and/or metabolic network for which a particular treatment (*e.g.,* small molecule drug, *e.g.* the small molecule drugs as listed in the above, *e.g.,* the drugs listed in Agent Table I and Agent Table II) is targeting, are more likely to be responders to the treatment. In another embodiment, individuals who carry at-risk variants for a gene, which expression and/or function is altered by the at-risk variant, are more likely to be responders to a treatment modality targeting that gene, its expression or its gene product.

In a further aspect, the markers and haplotypes can be used for targeting the selection of pharmaceutical agents for specific individuals. Personalized selection of treatment modalities, lifestyle changes (*e.g.,* change in diet, exercise, weight loss program, smoking abstinence, less stressful lifestyle, etc.) or combination of the two, can be realized by the utilization of the at-risk variants of the present invention. Thus, the knowledge of an individual's status for particular markers of the present invention, can be useful for selection of treatment options that target genes or gene products affected by the at-risk variants of the invention. Certain combinations of variants may be suitable for one selection of treatment options, while other gene variant combinations may target other treatment options. Such combination of variant may include one variant, two variants, three variants, or four or more variants, as needed to determine with clinically reliable accuracy the selection of treatment module.

### Computer-implemented aspects

The present invention also relates to computer-implemented applications of the polymorphic markers and haplotypes described herein to be associated with coronary artery disease and myocardial infarction. Such applications can be useful for storing, manipulating or otherwise analyzing genotype data that is useful in the methods of the invention. One example pertains to storing genotype information derived from an individual on readable media, so as to be able to provide the genotype information to a third party (*e.g.,* the individual), or for deriving information from the genotype data, *e.g.,* by comparing the genotype data to information about genetic risk factors contributing to increased susceptibility to coronary artery disease and/or myocardial infarction, and reporting results based on such comparison.

Computer-readable media are useful in such aspects. In general terms, such medium has capabilities of storing (i) identifier information for at least one polymorphic marker or a haplotype; (ii) an indicator of the frequency of at least one allele of said at least one marker, or the frequency of a haplotype, in individuals with coronary artery disease and/or myocardial infarction; and an indicator of the frequency of at least one allele of said at least one marker, or the frequency of a haplotype, in a reference population. The reference population can be a disease-free population of individuals. Alternatively, the reference population is a random sample from the general population, and is thus representative of the population at large. The frequency indicator may be a calculated frequency, a count of alleles and/or haplotype copies, or normalized or otherwise manipulated values of the actual frequencies that are suitable for the particular medium.

Additional information about the individual can be stored on the medium, such as ancestry information, information about sex, physical attributes or characteristics (including height and weight), biochemical measurements (such as blood pressure, blood lipid levels, lipid levels, such as cholesterol levels), biomarkers relevant for cardiovascular disease, as described further herein, or other useful information that is desirable to store or manipulate in the context of the genotype status of a particular individual.

The invention furthermore relates to an apparatus that is suitable for determination or manipulation of genetic data useful for determining a susceptibility to coronary artery disease and/or myocardial infarction in a human individual. Such an apparatus can include a computer-readable memory, a routine for manipulating data stored on the computer-readable memory, and a routine for generating an output that includes a measure of the genetic data. Such measure can include values such as allelic or haplotype frequencies, genotype counts, sex, age, phenotype information, values for odds ratio (OR) or relative risk (RR), population attributable risk (PAR), or other useful information that is either a direct statistic of the original genotype data or based on calculations based on the genetic data.

The markers and haplotypes shown herein to be associated with increased susceptibility (e.g., increased risk) of cardiovascular disease, are in certain embodiments useful for interpretation and/or analysis of genotype data. Thus in certain embodiments, an identification of an at-risk allele for coronary artery disease and/or myocardial infarction, as shown herein, or an allele at a polymorphic marker in LD with any one of the markers shown herein to be associated with coronary artery disease and/or myocardial infarction, is indicative of the individual from whom the genotype data originates is at increased risk of coronary artery disease and/or myocardial infarction. In one such embodiment, genotype data is generated for at least one polymorphic marker shown herein to be associated with coronary artery disease and/or myocardial infarction, or a marker in linkage disequilibrium therewith. The genotype data is subsequently made available to a third party, such as the individual from whom the data originates, for example via a user interface accessible over the internet, together with an interpretation of the genotype data, e.g., in the form of a risk measure (such as an absolute risk (AR), risk ratio (RR) or odds ratio (OR)) for the cardiovascular disease. In another embodiment, at-risk markers identified in a genotype dataset derived from an individual are assessed and results from the assessment of the risk conferred by the presence of such at-risk variants in the dataset are made available to the individual, for example via a secure web interface, or by other communication means. The results of such risk assessment can be reported in numeric form (e.g., by risk values, such as absolute risk, relative risk, and/or an odds ratio, or by a percentage increase in risk compared with a reference), by graphical means, or by other means suitable to illustrate the risk to the individual from whom the genotype data is derived. In particular embodiments, the results of risk assessment is made available to a third party, *e.g.,* a physician, other healthcare worker or genetic counselor.

### Markers useful in various aspects of the invention

The above-described applications can all be practiced with the markers and haplotypes of the invention that have in more detail been described with respect to methods of assessing susceptibility to cardiovascular disease and described in detail herein. Thus, these applications can in general be reduced to practice using any of the markers listed in Table 5In some preferred embodiments, the marker is selected from rs11751605 (SEQ ID NO:1), and markers in linkage disequilibrium therewith. In other preferred embodiments, the marker is selected from rs6076623 (SEQ ID NO:2), and markers in linkage disequilibrium therewith. In a particularly preferred embodiment, the marker is rs11751605 (SEQ ID NO:1).

In some embodiments, the presence of allele C in rs11751605 is indicative of increased susceptibility of coronary artery disease or myocardial infarction in the individual who is undergoing testing, through analysis of genotype information (genotype dataset) derived from the individual, or through analysis of a sample comprising genomic DNA from the individual.

### Nucleic acids and polypeptides

The nucleic acids and polypeptides described herein can be used in methods and kits of the present invention. An "isolated" nucleic acid molecule, as used herein, is one that is separated from nucleic acids that normally flank the gene or nucleotide sequence (as in genomic sequences) and/or has been completely or partially purified from other transcribed sequences (e.g., as in an RNA library). For example, an isolated nucleic acid of the invention can be substantially isolated with respect to the complex cellular milieu in which it naturally occurs, or culture medium when produced by recombinant techniques, or chemical precursors or other chemicals when chemically synthesized. In some instances, the isolated material will form part of a composition (for example, a crude extract containing other substances), buffer system or reagent mix. In other circumstances, the material can be purified to essential homogeneity, for example as determined by polyacrylamide gel electrophoresis (PAGE) or column chromatography (e.g., HPLC). An isolated nucleic acid molecule of the invention can comprise at least about 50%, at least about 80% or at least about 90% (on a molar basis) of all macromolecular species present. With regard to genomic DNA, the term "isolated" also can refer to nucleic acid molecules that are separated from the chromosome with which the genomic DNA is naturally associated. For example, the isolated nucleic acid molecule can contain less than about 250 kb, 200 kb, 150 kb, 100 kb, 75 kb, 50 kb, 25 kb, 10 kb, 5 kb, 4 kb, 3 kb, 2 kb, 1 kb, 0.5 kb or 0.1 kb of the nucleotides that flank the nucleic acid molecule in the genomic DNA of the cell from which the nucleic acid molecule is derived.

The nucleic acid molecule can be fused to other coding or regulatory sequences and still be considered isolated. Thus, recombinant DNA contained in a vector is included in the definition of "isolated" as used herein. Also, isolated nucleic acid molecules include recombinant DNA molecules in heterologous host cells or heterologous organisms, as well as partially or substantially purified DNA molecules in solution. "Isolated" nucleic acid molecules also encompass *in vivo* and *in vitro* RNA transcripts of the DNA molecules of the present invention. An isolated nucleic acid molecule or nucleotide sequence can include a nucleic acid molecule or nucleotide sequence that is synthesized chemically or by recombinant means. Such isolated nucleotide sequences are useful, for example, in the manufacture of the encoded polypeptide, as probes for isolating homologous sequences (*e.g.,* from other mammalian species), for gene mapping (*e.g.,* by *in situ* hybridization with chromosomes), or for detecting expression of the gene in tissue (*e.g.,* human tissue), such as by Northern blot analysis or other hybridization techniques.

Also disclosed nucleic acid molecules that hybridize under high stringency hybridization conditions, such as for selective hybridization, to a nucleotide sequence described herein (*e.g.,* nucleic acid molecules that specifically hybridize to a nucleotide sequence containing a polymorphic site associated with a haplotype described herein). In one embodiment, the invention uses variants that hybridize under high stringency hybridization and wash conditions (*e.g.,* for selective hybridization) to a nucleotide sequence that comprises the nucleotide sequence according to any one of SEQ ID NO:1-172 or a fragment thereof (or a nucleotide sequence comprising the complement of the nucleotide sequence of any one of SEQ ID NO:1-172), wherein the nucleotide sequence comprises at least one at-risk allele of at least one polymorphic marker, or at least one haplotype, as described herein.

The percent identity of two nucleotide or amino acid sequences can be determined by aligning the sequences for optimal comparison purposes (e.g., gaps can be introduced in the sequence of a first sequence). The nucleotides or amino acids at corresponding positions are then compared, and the percent identity between the two sequences is a function of the number of identical positions shared by the sequences (i.e., % identity = # of identical positions/total # of positions x 100). In certain embodiments, the length of a sequence aligned for comparison purposes is at least 30%, at least 40%, at least 50%, at least 60%, at least 70%, at least 80%, at least 90%, or at least 95%, of the length of the reference sequence. The actual comparison of the two sequences can be accomplished by well-known methods, for example, using a mathematical algorithm. A non-limiting example of such a mathematical algorithm is described in Karlin, S. and Altschul, S., Proc. Natl. Acad. Sci. USA, 90:5873-5877 (1993). Such an algorithm is incorporated into the NBLAST and XBLAST programs (version 2.0), as described in Altschul, S. et al., Nucleic Acids Res., 25:3389-3402 (1997). When utilizing BLAST and Gapped BLAST programs, the default parameters of the respective programs (e.g., NBLAST) can be used. See the website on the world wide web at ncbi.nlm.nih.gov. In one embodiment, parameters for sequence comparison can be set at score=100, wordlength=12, or can be varied (e.g., W=5 or W=20). Another example of an algorithm is BLAT (Kent, W.J. Genome Res. 12:656-64 (2002)).

Also disclosed isolated nucleic acid molecules that contain a fragment or portion that hybridizes under highly stringent conditions to a nucleic acid that comprises, or consists of, the nucleotide sequence of any one of SEQ ID NO:1-172, or a nucleotide sequence comprising, or consisting of, the complement of the nucleotide sequence of any one of SEQ ID NO:1-172, wherein the nucleotide sequence comprises at least one polymorphic allele contained in the markers and haplotypes described herein. The nucleic acid fragments are at least about 15, at least about 18, 20, 23 or 25 nucleotides, and can be 30, 40, 50, 100, 200, 500, 1000, 10,000 or more nucleotides in length.

The nucleic acid fragments are used as probes or primers in assays such as those described herein. "Probes" or "primers" are oligonucleotides that hybridize in a base-specific manner to a complementary strand of a nucleic acid molecule. In addition to DNA and RNA, such probes and primers include polypeptide nucleic acids (PNA), as described in Nielsen, P. et al., Science 254:1497-1500 (1991). A probe or primer comprises a region of nucleotide sequence that hybridizes to at least about 15, typically about 20-25, and in certain embodiments about 40, 50 or 75, consecutive nucleotides of a nucleic acid molecule comprising a contiguous nucleotide sequence from any one of SEQ ID NO:1-172 and comprising at least one allele of at least one polymorphic marker or at least one haplotype described herein, or the complement thereof. In particular embodiments, a probe or primer can comprise 100 or fewer nucleotides; for example, in certain embodiments from 6 to 50 nucleotides, or, for example, from 12 to 30 nucleotides. In other embodiments, the probe or primer is at least 70% identical, at least 80% identical, at least 85% identical, at least 90% identical, or at least 95% identical, to the contiguous nucleotide sequence or to the complement of the contiguous nucleotide sequence. In another embodiment, the probe or primer is capable of selectively hybridizing to the contiguous nucleotide sequence or to the complement of the contiguous nucleotide sequence. Often, the probe or primer further comprises a label, *e.g.,* a radioisotope, a fluorescent label, an enzyme label, an enzyme cofactor label, a magnetic label, a spin label, an epitope label.

The nucleic acid molecules, such as those described above, can be identified and isolated using standard molecular biology techniques and the sequence information provided by the nucleotide sequence of any one of SEQ ID NO:1-172. See generally PCR Technology: Principles and Applications for DNA Amplification (ed. H.A. Erlich, Freeman Press, NY, NY, 1992); PCR Protocols: A Guide to Methods and Applications (Eds. Innis, et al., Academic Press, San Diego, CA, 1990); Mattila, P. et al., Nucleic Acids Res., 19:4967-4973 (1991); Eckert, K. and Kunkel, T., PCR Methods and Applications, 1:17-24 (1991); PCR (eds. McPherson et al., IRL Press, Oxford); and U.S. Patent 4,683,202.

### Antibodies

Polyclonal antibodies and/or monoclonal antibodies that specifically bind one form of the gene product but not to the other form of the gene product (such as Lipoprotein(a), including kringle IV variants of Lipoprotein(a)) are also provided. Antibodies are also provided which bind a portion of either the variant or the reference gene product that contains the polymorphic site or sites. The term "antibody" as used herein refers to immunoglobulin molecules and immunologically active portions of immunoglobulin molecules, *i.e.,* molecules that contain antigen-binding sites that specifically bind an antigen. A molecule that specifically binds to a polypeptide of the invention is a molecule that binds to that polypeptide or a fragment thereof, but does not substantially bind other molecules in a sample, *e.g.,* a biological sample, which naturally contains the polypeptide. Examples of immunologically active portions of immunoglobulin molecules include F(ab) and F(ab')₂ fragments which can be generated by treating the antibody with an enzyme such as pepsin. The invention provides polyclonal and monoclonal antibodies that bind to a polypeptide of the invention. The term "monoclonal antibody" or "monoclonal antibody composition", as used herein, refers to a population of antibody molecules that contain only one species of an antigen binding site capable of immunoreacting with a particular epitope of a polypeptide of the invention. A monoclonal antibody composition thus typically displays a single binding affinity for a particular polypeptide of the invention with which it immunoreacts.

Polyclonal antibodies can be prepared as described above by immunizing a suitable subject with a desired immunogen, *e.g.,* polypeptide of the invention or afragment thereof. The antibody titer in the immunized subject can be monitored over time by standard techniques, such as with an enzyme linked immunosorbent assay (ELISA) using immobilized polypeptide. If desired, the antibody molecules directed against the polypeptide can be isolated from the mammal (*e.g.,* from the blood) and further purified by well-known techniques, such as protein A chromatography to obtain the IgG fraction. At an appropriate time after immunization, *e.g.,* when the antibody titers are highest, antibody-producing cells can be obtained from the subject and used to prepare monoclonal antibodies by standard techniques, such as the hybridoma technique originally described by Kohler and Milstein, Nature 256:495-497 (1975), the human B cell hybridoma technique (Kozbor et al., Immunol. Today 4: 72 (1983)), the EBV-hybridoma technique (Cole et al., Monoclonal Antibodies and Cancer Therapy, Alan R. Liss,1985, Inc., pp. 77-96) or trioma techniques. The technology for producing hybridomas is well known (see generally Current Protocols in Immunology (1994) Coligan et al., (eds.) John Wiley & Sons, Inc., New York, NY). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds a polypeptide of the invention.

Any of the many well known protocols used for fusing lymphocytes and immortalized cell lines can be applied for the purpose of generating a monoclonal antibody to a polypeptide of the invention (see, *e.g., Current Protocols in Immunology, supra;* Galfre et al., Nature 266:55052 (1977); R.H. Kenneth, in Monoclona! Antibodies: A New Dimension In Biological Analyses, Plenum Publishing Corp., New York, New York (1980); and Lerner, Yale J. Biol. Med. 54:387-402 (1981)). Moreover, the ordinarily skilled worker will appreciate that there are many variations of such methods that also would be useful.

Alternative to preparing monoclonal antibody-secreting hybridomas, a monoclonal antibody to a polypeptide of the invention can be identified and isolated by screening a recombinant combinatorial immunoglobulin library (*e.g.,* an antibody phage display library) with the polypeptide to thereby isolate immunoglobulin library members that bind the polypeptide. Kits for generating and screening phage display libraries are commercially available *(e.g.,* the Pharmacia *Recombinant Phage Antibody System,* Catalog No. 27-9400-01; and the Stratagene SurfZAP™ Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., Bio/Technology 9: 1370-1372 (1991); Hay et al., Hum. Antibod. Hybridomas 3:81-85 (1992); Huse et al., Science 246: 1275-1281 (1989); and Griffiths et al., EMBO J. 12:725-734 (1993).

Additionally, recombinant antibodies, such as chimeric and humanized monoclonal antibodies, comprising both human and non-human portions, which can be made using standard recombinant DNA techniques, are disclosed. Such chimeric and humanized monoclonal antibodies can be produced by recombinant DNA techniques known in the art.

In general, antibodies (*e.g.,* a monoclonal antibody) can be used to isolate a polypeptide of the invention by standard techniques, such as affinity chromatography or immunoprecipitation. A polypeptide-specific antibody can facilitate the purification of natural polypeptide from cells and of recombinantly produced polypeptide expressed in host cells. Moreover, an antibody specific for a polypeptide of the invention can be used to detect the polypeptide (*e.g.,* in a cellular lysate, cell supernatant, or tissue sample) in order to evaluate the abundance and pattern of expression of the polypeptide. Antibodies can be used diagnostically to monitor protein levels in tissue as part of a clinical testing procedure, *e.g.,* to, for example, determine the efficacy of a given treatment regimen. The antibody can be coupled to a detectable substance to facilitate its detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Antibodies may also be useful in pharmacogenomic analysis. In such embodiments, antibodies against variant proteins encoded by nucleic acids (for example, variant forms of Lipoprotein(a), such as kringle IV repeat polymorphisms), such as variant proteins that are encoded by nucleic acids that contain at least one polymorpic marker of the invention, can be used to identify individuals that require modified treatment modalities.

Antibodies can furthermore be useful for assessing expression of variant proteins in disease states, such as in active stages of a cardiovascular disease, or in an individual with a predisposition to a disease related to the function of the protein, in particular a cardiovascular disease. Examples are provided by biomarker (*e.g.,* cardiac markers), as described further herein.. Antibodies specific for a variant protein of the present invention that is encoded by a nucleic acid that comprises at least one polymorphic marker or haplotype as described herein (e.g., CDKN2A and/or CDKN2B )can be used to screen for the presence of the variant protein, for example to screen for a predisposition to cardiovascular disease as indicated by the presence of the variant protein.

Antibodies can be used in other methods. Thus, antibodies are useful as diagnostic tools for evaluating proteins, such as variant proteins of the invention (*e.g.,* variants of Lipoprotein(a), such as kringle IV repeat variants), in conjunction with analysis by electrophoretic mobility, isoelectric point, tryptic or other protease digest, or for use in other physical assays known to those skilled In the art. Antibodies may also be used in tissue typing. In one such embodiment, a specific variant protein has been correlated with expression in a specific tissue type, and antibodies specific for the variant protein can then be used to identify the specific tissue type.

Subcellular localization of proteins, including variant proteins, can also be determined using antibodies, and can be applied to assess aberrant subcellular localization of the protein in cells in various tissues. Such use can be applied in genetic testing, but also in monitoring a particular treatment modality. In the case where treatment is aimed at correcting the expression level or presence of the variant protein or aberrant tissue distribution or developmental expression of the variant protein, antibodies specific for the variant protein or fragments thereof can be used to monitor therapeutic efficacy.

Antibodies are further useful for inhibiting variant protein function (*e.g.,* Lipoprotein(a)), for example by blocking the binding of a variant protein to a binding molecule or partner. Such uses can also be applied in a therapeutic context in which treatment involves inhibiting a variant protein's function. An antibody can be for example be used to block or competitively inhibit binding, thereby modulating (i.e., agonizing or antagonizing) the activity of the protein. Antibodies can be prepared against specific protein fragments containing sites required for specific function or against an intact protein that is associated with a cell or cell membrane. For administration *in vivo,* an antibody may be linked with an additional therapeutic payload, such as radionuclide, an enzyme, an immunogenic epitope, or a cytotoxic agent, including bacterial toxins (diphtheria or plant toxins, such as ricin). The *in vivo* half-life of an antibody or a fragment thereof may be increased by pegylation through conjugation to polyethylene glycol.

Also disclosed kits for using antibodies in the methods described herein. This includes, but is not limited to, kits for detecting the presence of a variant protein in a test sample. One preferred embodiment comprises antibodies such as a labelled or labelable antibody and a compound or agent for detecting variant proteins in a biological sample, means for determining the amount or the presence and/or absence of variant protein in the sample, and means for comparing the amount of variant protein in the sample with a standard, as well as instructions for use of the kit.

The present invention will now be exemplified by the following non-limiting examples.

### EXEMPLIFICATION

### Example 1.

The following contains description of the identifiction of susceptibility factors found to be associated with coronary artery disease and myocardial infarction through single-point analysis of SNP markers and microsatellite markers.

### Methods

The study was approved by the Data Protection Commission of Iceland and the National Bioethics Committee.

### Icelandic coronary artery disease cohort

Over the last eight years individuals who have suffered an MI we have been recruited through cardiovascular disease (CVD) genetic programs at deCODE. Currently blood samples have been collected from 2525 MI patients. The individuals who had suffered an MI were identified from a registry of over 10,000 individuals who: a) had an MI before the age of 75 in Iceland in the years 1981 to 2002 and satisfy the MONICA criteria (J Clin Epidemiol 41, 105-14 (1988)); b) participated in a large prospective epidemiology study (*1*) done by the Icelandic Heart Association (IHA) over the past 30 years and had MI prior to 1981; c) had MI discharge diagnosis from the major hospitals in Reykjavik in the years 2003 and 2004. MI diagnoses of all individuals in the registry follow strict diagnostic rules based on signs, symptoms, electrocardiograms, cardiac enzymes and necropsy findings (*2*). The patients were contacted through collaborating physicians in the CVD genetic programs at deCODE. Most of the participants in the study visited the IHA and had their blood drawn, although participants who lived outside the Reykjavik area visited their local health care center.

Additional subjects with coronary artery disease, but are without known history of myocardial infarction, are identified from a list of those who have undergone coronary stent procedure in the major hospitals in Reykjavik in the years 1993 and 2003.

The controls used for the study were recruited as a part of various genetic programs at deCODE. The medical history for the controls were unknown unless if the control subjects also had participated in any of the CVD genetic programs (i.e. MI, stroke, peripheral vascular disease, type II diabetes, obesity, familial combined hyperlipidemia, coronary restenosis, and hypertension genetic programs). Individuals with known MI, stroke, peripheral vascular or coronary artery disease were excluded as controls.

### Subjects from the United States

### Cohort from Philadelphia

The study participants from Philadelphia were enrolled at the University of Pennsylvania Medical Center through the PENN CATH study program which studies the association of biochemical and genetic factors to coronary artery disease (CAD) in subjects undergoing cardiac catheterization. A total of 3850 subjects have participated. For the purpose of the current study we selected from the PENN CATH study individuals diagnosed with one of the following coronary artery disease: MI based on criteria for acute MI in terms of elevations of cardiac enzymes and electrocardiographic changes, or a self-reported history of MI, history of coronary artery bypass surgery (CABG) or percutaneous, transluminal coronary angioplasty (PTCA). To use as controls we selected individuals who were without significant luminal stenosis on coronary angiography (luminal stenosis less than 50%). Ethnicity information was self-reported.

The University of Pennsylvania Institutional Review Board approved the study and all subjects provided written informed consent.

### Cohort from Durham, North Carolina

The study participants were enrolled at Duke University Medical Center (Durham, NC) through the CATHGEN biorepository, recruited sequentially through the cardiac catheterization laboratories from 2001-2005. Biological samples and extensive clinical, angiographic, and longitudinal follow-up data were collected on all subjects consenting to participation. For purposes of this study, cases of MI were defined as those having a history of MI (by self-report and corroborated by review of medical records), or having suffered an MI during the study follow-up period. Controls from this cohort, defined as those with no history of MI prior or subsequent to the index cardiac catheterization, as well as no history of percutaneous or surgical coronary revascularization procedure; no subsequent percutaneous or surgical coronary revascularization procedures; ejection fraction on left ventriculogram greater than 40%; and no or minimal CAD on coronary angiography (defined as a CAD index less than or equal to 23 and no coronary vessel with clinically significant CAD (stenosis greater than 50%)). This study was approved by the Duke University Medical Center Institutional Review Board on Human Subjects and all subjects gave written informed consent.

### Cohort from Atlanta

The study participants were enrolled at the Emory University Hospital, the Emory Clinic and Grady Memorial Hospitals through its Emory Genebank study and Clinical Registry in Neurology (CRIN). The Emory Genebank studies the association of biochemical and genetic factors with CAD in subjects undergoing cardiac catheterization. For the purpose of the current study those subjects who had a self-reported history of MI, CABG, or PTCA, were selected and used as a patient group. Control subjects were selected from a group of individuals with non-vascular neurological diseases (mainly Parkinson's and Alzheimer's diseases) recruited from CRIN, their spouses, unrelated friends and community volunteers. These subjects were matched for age, and ethnicity to the patient population. Controls were excluded if they had a known history of MI or coronary artery disease. All subjects provided written informed consent. Information on ethnicity was self-reported.

### Genotyping

A genome-wide scan of 1700 Icelandic individuals diagnosed with myocardial infarction (MI), 2034 patients with coronary artery disease and 10635 population controls was performed using Infinium HumanHap300 SNP chips from Illumina for assaying approximately 317,000 single nucleotide polymorphisms (SNPs) on a single chip (Illumina, San Diego, CA, USA). SNP genotyping for replication in other case-control cohorts was carried using the Centaurus platform (Nanogen).

### Statistical Methods for Association Analysis.

To test individual markers for association to disease phenotypes such as coronary artery disease or myocardial infarction, we use a likelihood ratio test to calculate a two-sided P-value for each allele of the markes. We calculate relative risk (RR) and population attributable risk (PAR) assuming a multiplicative model (C. T. Falk, P. Rubinstein, Ann Hum Genet 51 (Pt 3), 227 (1987); J. D. Terwilliger, J. Ott, Hum Hered 42, 337 (1992)). To elucidate the linkage disequilibrium between markers in the region we used the CEPH Caucasian HapMap data. We calculated LD between pairs of SNPs using the standard definition of D' (R. C. Lewontin, Genetics 50, 757 (1964)) and for the correlation coefficient *r*² (W. G. Hill, A. Robertson, Genetics 60, 615 (Nov, 1968). For the Icelandic cohort, to take into account that some of the individuals are related to each other, we obtained the null statistic of the test statistic either by simulating genotypes through the Icelandic genealogy or from the test statistic for all the 300,000 tested for association in the initial genome-wide association scan (citation). Model-free estimates of the genotype relative risk are generated as follows: RR of genotype G₁ compared to genotype G₀ was estimated by [n(G₁)/n(G₀)]/ [m(G₁)/m(G₀)] where n and m denote genotype counts in patients and controls respectively. Results from different cohorts were combined using a Mantel-Hanezel model (citation) where cohorts are allowed to have different population frequencies for the alleles/genotypes but assume to have common relative risks.

We use multiple regression to test for association between markers and quantitative traits, such as ago of onset of MI in the cases, where the number of copies of the at-risk variant carried by an individual is taken as explanatory variable and the quantitative trait as the response variable. The association is adjusted for age and gender, where appropriate, by including corresponding terms in the regression analysis as explanatory variables.

### Results

### Genome-wide association study

We successfully genotyped 1700 Icelandic myocardial infarction patients, 2034 coronary artery disease Patients and 10635 population control individuals without known history of coronary artery disease (Cohort A) using the Illumina 330K chip. We performed a genome-wide scan for association to MI, testing individually each of the 309,091 SNPs that was successfully genotyped. Results of this association analysis is presented in Table 1, wherein markers showing significant association to MI are indicated (p-value less than 0.01). As can be seen in the table, a large number of markers show significant association to MI, including markers rs2227165 on chromosome X, rs12304836 on chromosome 12 and rs11225090 on chromosome 11.

The markers found to be associating with MI as presented in Table 1 may be useful for risk assessment and diagnostic purposes for MI, either alone or in combination. Even in the cases where the increase in risk by individual markers is relatively modest, i.e. on the order of 15-30%, the association may have significant implications. Thus, relatively common variants may have significant contribution to the overall risk (Population Attributable Risk is high), or combination of markers can be used to define groups of individual who, based on the combined risk of the markers, is at signficant combined risk of developing MI.

**Table 1. Association results for SNPs showing association to Myoardial Infarction (MI) and coronary artery disease (CAD). The columns indicate (1) chromosome, (2) position in NCBI Build 34, (3) rs name of the SNP in question, (4), the associating allele, (5) frequency of the associating allele in controls, (6) the number of controls, (7), the p-value for association to MI, (8) the Relative Risk of association to MI, (9) the frequency of the associating allele in MI patients, (10) the number of MI patients, (11) the p-value of association to CAD, (12) the Relative Risk of association to CAD, (13) the frequency of the associating allele in CAD patients, (14) the number of CAD patients. The table is sorted on p-values for association to MI. 3**

| **chr** | **position b34** | **rs-name** | **allele** | **f(con)** | **N(con)** | **p(MI)** | **RR(MI)** | **f(MI)** | **N(MI)** | **p(CAD)** | **rr(CAD)** | **f(CAD)** | **N(CAD)** |
|---|---|---|---|---|---|---|---|---|---|---|---|---|---|
| CX | 91765264 | rs2227165 | C | 0.897 | 8178 | 2.08E-07 | 1.61 | 0.934 | 1068 | 5.16E-05 | 1.39 | 0.924 | 1276 |
| C12 | 124441592 | rs12304836 | C | 0.141 | 10541 | 7.66E-06 | 1.27 | 0.173 | 1689 | 6.26E-06 | 1.25 | 0.171 | 2020 |
| C11 | 101370325 | rs11225090 | T | 0.969 | 10513 | 8.71E-06 | 1.85 | 0.983 | 1646 | 2.95E-05 | 1.68 | 0.981 | 1979 |
| C05 | 158504417 | rs7442701 | T | 0.174 | 10629 | 1.05E-05 | 1.25 | 0.208 | 1699 | 5.90E-05 | 1.21 | 0.202 | 2033 |
| C12 | 116419694 | rs816204 | A | 0.851 | 10607 | 1.10E-05 | 1.29 | 0.881 | 1693 | 4.70E-05 | 1.25 | 0.877 | 2026 |
| C20 | 4131671 | rs6076623 | T | 0.364 | 10632 | 1.20E-05 | 1.19 | 0.406 | 1700 | 2.40E-06 | 1.19 | 0.406 | 2034 |
| C17 | 17995010 | rs4925125 | T | 0.347 | 10430 | 1.55E-05 | 1.19 | 0.388 | 1680 | 3.40E-04 | 1.15 | 0.379 | 2009 |
| C10 | 90667504 | rs1412444 | A | 0.356 | 10626 | 1.60E-05 | 1.19 | 0.397 | 1700 | 2.38E-04 | 1.15 | 0.389 | 2034 |
| C17 | 18235585 | rs854787 | C | 0.398 | 10568 | 1.72E-05 | 1.19 | 0.44 | 1689 | 2.63E-05 | 1.17 | 0.436 | 2023 |
| C10 | 90675056 | rs2243547 | G | 0.339 | 10200 | 1.82E-05 | 1.2 | 0.38 | 1642 | 3.87E-04 | 1.15 | 0.37 | 1970 |
| C08 | 506479 | rs722782 | C | 0.888 | 10607 | 2.44E-05 | 1.33 | 0.914 | 1668 | 1.72E-04 | 1.26 | 0.909 | 2002 |
| C11 | 82905504 | rs2163612 | G | 0.476 | 10625 | 3.12E-05 | 1.18 | 0.517 | 1698 | 3.92E-05 | 1.16 | 0.513 | 2032 |
| C01 | 62766259 | rs12134779 | T | 0.29 | 10634 | 3.21E-05 | 1.19 | 0.327 | 1699 | 1.81E-04 | 1.16 | 0.321 | 2033 |
| CX | 66228891 | rs7050085 | G | 0.909 | 8267 | 5.71E-05 | 1.45 | 0.936 | 1103 | 1.15E-04 | 1.39 | 0.933 | 1316 |
| C07 | 116846839 | rs12534186 | T | 0.079 | 10631 | 5.99E-05 | 1.31 | 0.102 | 1699 | 2.22E-04 | 1.26 | 0.098 | 2033 |
| C05 | 158510030 | rs7447732 | C | 0.316 | 10635 | 6.14E-05 | 1.18 | 0.353 | 1700 | 3.34E-04 | 1.15 | 0.347 | 2034 |
| C07 | 116800818 | rs2299445 | C | 0.079 | 10630 | 6.16E-05 | 1.31 | 0.101 | 1699 | 2.21E-04 | 1.27 | 0.098 | 2033 |
| C07 | 116800818 | rs2299445 | C | 0.079 | 10630 | 6.16E-05 | 1.31 | 0.101 | 1699 | 2.21E-04 | 1.27 | 0.098 | 2033 |
| C19 | 4229502 | rs4807567 | T | 0.419 | 10586 | 6.30E-05 | 1.17 | 0.458 | 1699 | 1.99E-04 | 1.15 | 0.453 | 2033 |
| C13 | 26936609 | rs4769613 | T | 0.467 | 10633 | 6.56E-05 | 1.17 | 0.506 | 1699 | 9.01E-06 | 1.18 | 0.507 | 2033 |
| C05 | 158671846 | rs4921437 | T | 0.212 | 10594 | 6.57E-05 | 1.21 | 0.245 | 1691 | 6.09E-04 | 1.16 | 0.238 | 2023 |
| C10 | 15439877 | rs12269612 | T | 0.398 | 10634 | 6.66E-05 | 1.17 | 0.437 | 1700 | 8.04E-04 | 1.13 | 0.428 | 2034 |
| C12 | 87995622 | rs1568383 | T | 0.097 | 10635 | 7.05E-05 | 1.28 | 0.121 | 1700 | 1.11E-04 | 1.25 | 0.119 | 2034 |
| C21 | 24038081 | rs2828495 | A | 0.211 | 10635 | 7.27E-05 | 1.2 | 0.244 | 1700 | 4.74E-05 | 1.19 | 0.242 | 2034 |
| C10 | 45239070 | rs2279434 | T | 0.065 | 10634 | 7.50E-05 | 1.34 | 0.085 | 1700 | 5.02E-04 | 1.27 | 0.081 | 2033 |
| C02 | 212588265 | rs12329252 | T | 0.676 | 10635 | 7.87E-05 | 1.18 | 0.712 | 1700 | 6.42E-04 | 1.14 | 0.705 | 2034 |
| C03 | 62420156 | rs9874646 | T | 0.709 | 10634 | 8.11E-05 | 1.19 | 0.744 | 1700 | 3.83E-04 | 1.16 | 0.738 | 2034 |
| C04 | 21568604 | rs4282162 | T | 0.528 | 10633 | 8.28E-05 | 1.17 | 0.567 | 1700 | 1.66E-04 | 1.15 | 0.563 | 2034 |
| C03 | 62402714 | rs1534725 | T | 0.782 | 10627 | 8.33E-05 | 1.21 | 0.814 | 1699 | 1.33E-04 | 1.19 | 0.811 | 2033 |
| C03 | 62402714 | rs1534725 | T | 0.782 | 10627 | 8.33E-05 | 1.21 | 0.814 | 1699 | 1.33E-04 | 1.19 | 0.811 | 2033 |
| C09 | 87419341 | rs3211683 | A | 0.088 | 10635 | 9.41E-05 | 1.29 | 0.111 | 1700 | 2.86E-04 | 1.25 | 0.108 | 2034 |
| C11 | 42877662 | rs1532096 | T | 0.861 | 10635 | 9.63E-05 | 1.26 | 0.887 | 1700 | 9.38E-05 | 1.24 | 0.885 | 2034 |
| C22 | 42582255 | rs738407 | A | 0.635 | 10634 | 9.70E-05 | 1.18 | 0.672 | 1700 | 5.67E-04 | 1.14 | 0.665 | 2034 |
| C17 | 17947855 | rs3183702 | T | 0.349 | 10630 | 9.76E-05 | 1.17 | 0.386 | 1699 | 1.01E-03 | 1.13 | 0.378 | 2033 |
| C02 | 39191408 | rs963731 | C | 0.951 | 10365 | 1.01E-04 | 1.49 | 0.966 | 1651 | 1.21E-04 | 1.44 | 0.965 | 1978 |
| C09 | 87414699 | rs3211650 | T | 0.089 | 10634 | 1.02E-04 | 1.29 | 0.111 | 1700 | 3.09E-04 | 1.25 | 0.108 | 2034 |
| C06 | 71671372 | rs7753765 | A | 0.144 | 10633 | 1.03E-04 | 1.23 | 0.172 | 1700 | 2.32E-04 | 1.2 | 0.169 | 2034 |
| C01 | 30378256 | rs593993 | C | 0.834 | 10626 | 1.09E-04 | 1.24 | 0.862 | 1697 | 7.25E-05 | 1.23 | 0.86 | 2031 |
| C11 | 17756131 | rs7946133 | T | 0.915 | 10540 | 1.12E-04 | 1.35 | 0.936 | 1641 | 1.43E-04 | 1.31 | 0.934 | 1968 |
| C02 | 212605929 | rs17406681 | T | 0.275 | 10635 | 1.13E-04 | 1.18 | 0.31 | 1700 | 3.42E-04 | 1.15 | 0.305 | 2034 |
| C02 | 212605929 | rs17406681 | T | 0.275 | 10635 | 1.13E-04 | 1.18 | 0.31 | 1700 | 3.42E-04 | 1.15 | 0.305 | 2034 |
| C03 | 14276852 | rs6772823 | G | 0.276 | 10580 | 1.18E-04 | 1.18 | 0.31 | 1675 | 1.98E-04 | 1.16 | 0.307 | 2008 |
| C17 | 17934326 | rs9902941 | C | 0.349 | 10634 | 1.25E-04 | 1.17 | 0.385 | 1700 | 1.18E-03 | 1.13 | 0.377 | 2034 |
| C02 | 123847245 | rs1395930 | T | 0.494 | 10633 | 1.25E-04 | 1.16 | 0.532 | 1698 | 3.18E-05 | 1.16 | 0.532 | 2032 |
| C05 | 158647626 | rs254850 | T | 0.215 | 10635 | 1.29E-04 | 1.19 | 0.247 | 1700 | 1.30E-03 | 1.15 | 0.24 | 2034 |
| C03 | 170405740 | rs756644 | A | 0.431 | 10633 | 1.30E-04 | 1.16 | 0.468 | 1700 | 1.56E-03 | 1.12 | 0.459 | 2034 |
| C12 | 47223943 | rs12581026 | C | 0.046 | 10634 | 1.33E-04 | 1.39 | 0.063 | 1700 | 3.67E-03 | 1.27 | 0.058 | 2034 |
| C17 | 17849989 | rs4925108 | C | 0.392 | 10623 | 1.35E-04 | 1.16 | 0.429 | 1700 | 4.28E-04 | 1.14 | 0.424 | 2034 |
| C17 | 17849989 | rs4925108 | C | 0.392 | 10623 | 1.35E-04 | 1.16 | 0.429 | 1700 | 4.28E-04 | 1.14 | 0.424 | 2034 |
| C12 | 95967869 | rs11108788 | G | 0.032 | 10618 | 1.38E-04 | 1.47 | 0.046 | 1698 | 3.43E-04 | 1.4 | 0.044 | 2032 |
| C01 | 53740224 | rs2294512 | G | 0.682 | 10635 | 1.41E-04 | 1.18 | 0.716 | 1700 | 1.47E-03 | 1.13 | 0.708 | 2034 |
| C05 | 102457857 | rs9327877 | T | 0.19 | 10634 | 1.45E-04 | 1.2 | 0.22 | 1700 | 2.47E-04 | 1.18 | 0.217 | 2034 |
| C04 | 21549446 | rs10516402 | C | 0.504 | 10633 | 1.46E-04 | 1.16 | 0.541 | 1700 | 2.09E-04 | 1.15 | 0.538 | 2034 |
| C04 | 21549446 | rs10516402 | C | 0.504 | 10633 | 1.46E-04 | 1.16 | 0.541 | 1700 | 2.09E-04 | 1.15 | 0.538 | 2034 |
| C20 | 15053882 | rs411944 | G | 0.645 | 10632 | 1.47E-04 | 1.17 | 0.68 | 1700 | 1.22E-03 | 1.13 | 0.673 | 2033 |
| C13 | 42581715 | rs9525923 | A | 0.208 | 10635 | 1.56E-04 | 1.19 | 0.239 | 1700 | 5.62E-04 | 1.16 | 0.234 | 2034 |
| C02 | 176947426 | rs921083 | G | 0.776 | 10633 | 1.56E-04 | 1.2 | 0.806 | 1700 | 2.93E-04 | 1.18 | 0.803 | 2034 |
| C05 | 97515007 | rs950195 | G | 0.63 | 10631 | 1.65E-04 | 1.17 | 0.665 | 1700 | 5.67E-05 | 1.17 | 0.665 | 2034 |
| C05 | 6897773 | rs870347 | T | 0.924 | 10635 | 1.73E-04 | 1.35 | 0.942 | 1700 | 1.14E-03 | 1.27 | 0.939 | 2034 |
| C14 | 67308303 | rs8003722 | A | 0.102 | 10634 | 1.73E-04 | 1.26 | 0.126 | 1700 | 2.73E-04 | 1.23 | 0.123 | 2034 |
| C05 | 50867460 | rs6449708 | T | 0.452 | 10611 | 1.73E-04 | 1.16 | 0.489 | 1693 | 1.17E-03 | 1.13 | 0.482 | 2027 |
| C05 | 50867460 | rs6449708 | T | 0.452 | 10611 | 1.73E-04 | 1.16 | 0.489 | 1693 | 1.17E-03 | 1.13 | 0.482 | 2027 |
| C06 | 160872518 | rs11751605 | C | 0.11 | 10632 | 1.75E-04 | 1.25 | 0.134 | 1700 | 3.87E-04 | 1.22 | 0.131 | 2033 |
| C02 | 123861662 | rs2661006 | T | 0.493 | 10623 | 1.76E-04 | 1.16 | 0.529 | 1696 | 5.58E-05 | 1.16 | 0.529 | 2030 |
| C13 | 45452894 | rs7322927 | A | 0.125 | 10635 | 1.78E-04 | 1.24 | 0.151 | 1700 | 3.29E-03 | 1.17 | 0.144 | 2034 |
| C05 | 56215087 | rs832540 | C | 0.415 | 10626 | 1.81E-04 | 1.16 | 0.451 | 1700 | 3.23E-04 | 1.14 | 0.447 | 2034 |
| C04 | 166320428 | rs11723570 | C | 0.511 | 10635 | 1.92E-04 | 1.16 | 0.547 | 1700 | 1.49E-04 | 1.15 | 0.545 | 2034 |
| C19 | 17027981 | rs2278997 | T | 0.229 | 10635 | 1.93E-04 | 1.19 | 0.261 | 1700 | 8.49E-04 | 1.15 | 0.255 | 2034 |
| C19 | 17027981 | rs2278997 | T | 0.229 | 10635 | 1.93E-04 | 1.19 | 0.261 | 1700 | 8.49E-04 | 1.15 | 0.255 | 2034 |
| C16 | 73532441 | rs12927636 | G | 0.105 | 10635 | 1.94E-04 | 1.25 | 0.129 | 1700 | 7.63E-04 | 1.21 | 0.125 | 2034 |
| C11 | 1371919 | rs7944761 | T | 0.526 | 10631 | 2.04E-04 | 1.16 | 0.563 | 1700 | 8.52E-04 | 1.13 | 0.557 | 2034 |
| C07 | 116887417 | rs975722 | G | 0.389 | 10635 | 2.05E-04 | 1.16 | 0.425 | 1700 | 3.18E-04 | 1.14 | 0.421 | 2034 |
| C07 | 116887417 | rs975722 | G | 0.389 | 10635 | 2.05E-04 | 1.16 | 0.425 | 1700 | 3.18E-04 | 1.14 | 0.421 | 2034 |
| C17 | 18035257 | rs6502622 | G | 0.359 | 10635 | 2.07E-04 | 1.16 | 0.395 | 1700 | 2.81E-03 | 1.12 | 0.386 | 2034 |
| C02 | 76284921 | rs443713 | G | 0.394 | 10635 | 2.09E-04 | 1.16 | 0.43 | 1700 | 1.77E-04 | 1.15 | 0.428 | 2034 |
| C09 | 16319537 | rs1888955 | A | 0.742 | 10632 | 2.19E-04 | 1.19 | 0.774 | 1700 | 8.61E-04 | 1.15 | 0.768 | 2034 |
| C09 | 16324761 | rs12683609 | A | 0.571 | 10635 | 2.20E-04 | 1.16 | 0.607 | 1699 | 4.83E-04 | 1.14 | 0.602 | 2033 |
| C05 | 167501721 | rs7736530 | G | 0.798 | 10634 | 2.20E-04 | 1.21 | 0.826 | 1700 | 2.77E-04 | 1.19 | 0.824 | 2034 |
| C15 | 24283869 | rs4572353 | T | 0.492 | 10632 | 2.25E-04 | 1.16 | 0.528 | 1700 | 4.64E-04 | 1.14 | 0.524 | 2034 |
| C07 | 116792948 | rs213977 | G | 0.409 | 10633 | 2.28E-04 | 1.16 | 0.445 | 1700 | 3.73E-04 | 1.14 | 0.441 | 2034 |
| C02 | 76346853 | rs1595968 | T | 0.394 | 10622 | 2.29E-04 | 1.16 | 0.43 | 1695 | 1.92E-04 | 1.15 | 0.428 | 2029 |
| C01 | 20544933 | rs12724604 | A | 0.036 | 10634 | 2.35E-04 | 1.42 | 0.051 | 1700 | 5.31E-05 | 1.43 | 0.051 | 2034 |
| C04 | 21483069 | rs1827591 | G | 0.535 | 10635 | 2.41E-04 | 1.16 | 0.571 | 1700 | 2.98E-04 | 1.14 | 0.568 | 2034 |
| C01 | 148957002 | rs12066445 | A | 0.299 | 10635 | 2.47E-04 | 1.17 | 0.332 | 1700 | 1.08E-03 | 1.14 | 0.326 | 2034 |
| C17 | 9590955 | rs3891720 | C | 0.869 | 10613 | 2.47E-04 | 1.25 | 0.893 | 1698 | 4.95E-04 | 1.22 | 0.89 | 2032 |
| C17 | 17943470 | rs4925119 | A | 0.347 | 10615 | 2.54E-04 | 1.16 | 0.381 | 1696 | 2.24E-03 | 1.12 | 0.373 | 2029 |
| C08 | 144065203 | rs7844961 | T | 0.09 | 10635 | 2.61E-04 | 1.27 | 0.112 | 1700 | 1.82E-03 | 1.21 | 0.107 | 2034 |
| C01 | 57254228 | rs2991515 | T | 0.479 | 10635 | 2.67E-04 | 1.15 | 0.515 | 1700 | 2.39E-05 | 1.17 | 0.518 | 2034 |
| C20 | 4150948 | rs1741315 | A | 0.483 | 10633 | 2.68E-04 | 1.15 | 0.519 | 1700 | 5.86E-04 | 1.13 | 0.514 | 2034 |
| C20 | 4150948 | rs1741315 | A | 0.483 | 10633 | 2.68E-04 | 1.15 | 0.519 | 1700 | 5.86E-04 | 1.13 | 0.514 | 2034 |
| C09 | 132592188 | rs4917341 | C | 0.481 | 10587 | 2.69E-04 | 1.15 | 0.516 | 1697 | 1.12E-03 | 1.13 | 0.51 | 2029 |
| C08 | 28215758 | rs4732849 | T | 0.273 | 10635 | 2.70E-04 | 1.17 | 0.306 | 1700 | 1.05E-02 | 1.11 | 0.294 | 2034 |
| C20 | 47199260 | rs981303 | A | 0.929 | 10623 | 2.73E-04 | 1.35 | 0.946 | 1696 | 1.53E-05 | 1.4 | 0.948 | 2029 |
| C10 | 84830992 | rs7921473 | G | 0.815 | 10635 | 2.77E-04 | 1.21 | 0.842 | 1700 | 3.79E-05 | 1.22 | 0.844 | 2034 |
| C15 | 43779321 | rs11630033 | C | 0.9 | 10606 | 2.77E-04 | 1.29 | 0.92 | 1694 | 3.81E-04 | 1.26 | 0.918 | 2028 |
| C20 | 13686900 | rs2026058 | T | 0.764 | 10634 | 2.77E-04 | 1.19 | 0.794 | 1700 | 3.63E-03 | 1.14 | 0.786 | 2034 |
| C21 | 16738666 | rs1349227 | T | 0.238 | 10635 | 2.77E-04 | 1.18 | 0.269 | 1700 | 4.65E-04 | 1.16 | 0.266 | 2034 |
| C07 | 116579323 | rs1029396 | G | 0.069 | 10634 | 2.78E-04 | 1.3 | 0.088 | 1700 | 2.26E-04 | 1.28 | 0.087 | 2034 |
| C11 | 7588325 | rs12292613 | T | 0.892 | 10509 | 2.88E-04 | 1.28 | 0.913 | 1670 | 3.35E-03 | 1.2 | 0.908 | 2003 |
| C08 | 70812569 | rs3829049 | G | 0.708 | 10635 | 2.91E-04 | 1.17 | 0.74 | 1700 | 5.19E-04 | 1.15 | 0.737 | 2034 |
| C03 | 25122677 | rs6763490 | C | 0.6 | 10635 | 2.94E-04 | 1.16 | 0.635 | 1700 | 7.39E-04 | 1.14 | 0.63 | 2034 |
| C17 | 17911836 | rs4925114 | A | 0.359 | 10615 | 2.97E-04 | 1.16 | 0.393 | 1697 | 1.89E-03 | 1.12 | 0.386 | 2031 |
| C01 | 19443039 | rs7535952 | A | 0.717 | 10510 | 2.99E-04 | 1.18 | 0.749 | 1660 | 3.29E-03 | 1.13 | 0.741 | 1990 |
| C07 | 46273364 | rs10243723 | G | 0.97 | 10628 | 3.04E-04 | 1.61 | 0.981 | 1699 | 1.27E-03 | 1.47 | 0.979 | 2033 |
| C08 | 18243855 | rs7006687 | T | 0.575 | 10632 | 3.05E-04 | 1.16 | 0.61 | 1698 | 7.18E-05 | 1.16 | 0.61 | 2032 |
| C16 | 61289938 | rsl2924635 | G | 0.389 | 10380 | 3.06E-04 | 1.16 | 0.425 | 1624 | 3.29E-03 | 1.12 | 0.416 | 1953 |
| C04 | 10479642 | rs4697653 | G | 0.526 | 10604 | 3.08E-04 | 1.15 | 0.562 | 1699 | 8.82E-04 | 1.13 | 0.556 | 2032 |
| C12 | 112489807 | rs249047 | A | 0.614 | 10634 | 3.11E-04 | 1.16 | 0.648 | 1700 | 3.87E-03 | 1.12 | 0.639 | 2034 |
| C07 | 67324200 | rs12540706 | G | 0.311 | 10634 | 3.15E-04 | 1.16 | 0.344 | 1700 | 2.70E-03 | 1.12 | 0.337 | 2034 |
| C10 | 26191683 | rs7086207 | G | 0.404 | 10625 | 3.17E-04 | 1.15 | 0.439 | 1699 | 5.10E-04 | 1.14 | 0.435 | 2033 |
| C05 | 40281304 | rs13165359 | G | 0.877 | 10634 | 3.19 E-04 | 1.26 | 0.899 | 1700 | 2.77E-03 | 1.19 | 0.894 | 2034 |
| C05 | 50777478 | rs10038238 | A | 0.567 | 10634 | 3.20E-04 | 1.15 | 0.602 | 1699 | 1.41E-03 | 1.13 | 0.596 | 2033 |
| C01 | 65319795 | rs2025805 | C | 0.565 | 10634 | 3.25E-04 | 1.15 | 0.6 | 1700 | 3.31E-04 | 1.14 | 0.597 | 2034 |
| C17 | 18204411 | rs854813 | C | 0.387 | 10632 | 3.25E-04 | 1.15 | 0.422 | 1700 | 6.90E-04 | 1.13 | 0.417 | 2034 |
| C04 | 21576609 | rs4377576 | C | 0.54 | 10634 | 3.25E-04 | 1.15 | 0.575 | 1700 | 5.32E-04 | 1.14 | 0.572 | 2034 |
| C04 | 21576609 | rs4377576 | C | 0.54 | 10634 | 3.25E-04 | 1.15 | 0.575 | 1700 | 5.32E-04 | 1.14 | 0.572 | 2034 |
| C12 | 65789635 | rs1465026 | G | 0.832 | 10632 | 3.27E-04 | 1.22 | 0.858 | 1700 | 8.82E-05 | 1.22 | 0.858 | 2034 |
| C18 | 26049280 | rs4271662 | C | 0.58 | 10594 | 3.34E-04 | 1.16 | 0.615 | 1695 | 9.97E-04 | 1.13 | 0.61 | 2026 |
| C01 | 150696580 | rs4845552 | G | 0.125 | 10588 | 3.40E-04 | 1.23 | 0.149 | 1698 | 2.40E-04 | 1.21 | 0.148 | 2030 |
| C15 | 24926452 | rs2110209 | A | 0.047 | 10635 | 3.42E-04 | 1.36 | 0.062 | 1700 | 9.53E-04 | 1.3 | 0.06 | 2034 |
| C03 | 170400106 | rs1430435 | G | 0.503 | 10634 | 3.44E-04 | 1.15 | 0.538 | 1700 | 2.59E-03 | 1.12 | 0.53 | 2034 |
| C06 | 72208904 | rs182106 | T | 0.523 | 10635 | 3.50E-04 | 1.15 | 0.558 | 1700 | 8.17E-04 | 1.13 | 0.554 | 2034 |
| C03 | 145877192 | rs10935515 | C | 0.583 | 10623 | 3.51E-04 | 1.15 | 0.618 | 1699 | 6.10E-04 | 1.14 | 0.614 | 2033 |
| C06 | 72149930 | rs9360453 | G | 0.711 | 10616 | 3.57E-04 | 1.17 | 0.742 | 1697 | 4.11E-04 | 1.16 | 0.74 | 2030 |
| C05 | 115427457 | rs17138668 | C | 0.947 | 10634 | 3.60E-04 | 1.41 | 0.962 | 1700 | 1.06E-03 | 1.34 | 0.96 | 2034 |
| CX | 91547326 | rs6615536 | G | 0.987 | 8272 | 3.62E-04 | 2.85 | 0.995 | 1103 | 1.19E-04 | 2.84 | 0.995 | 1316 |
| C03 | 194770024 | rs883279 | C | 0.905 | 10632 | 3.63E-04 | 1.29 | 0.925 | 1699 | 1.46E-04 | 1.29 | 0.925 | 2033 |
| C07 | 116680904 | rs2283054 | A | 0.069 | 10634 | 3.73E-04 | 1.3 | 0.087 | 1699 | 3.39E-04 | 1.27 | 0.086 | 2033 |
| C06 | 136340016 | rs9376173 | A | 0.629 | 10613 | 3.74E-04 | 1.16 | 0.663 | 1692 | 1.42E-03 | 1.13 | 0.657 | 2026 |
| C20 | 4152600 | rs1741318 | A | 0.447 | 10633 | 3.75E-04 | 1.15 | 0.482 | 1700 | 1.07E-03 | 1.13 | 0.477 | 2034 |
| C20 | 4152600 | rs1741318 | A | 0.447 | 10633 | 3.75E-04 | 1.15 | 0.482 | 1700 | 1.07E-03 | 1.13 | 0.477 | 2034 |
| C12 | 47206273 | rs12425518 | A | 0.043 | 10611 | 3.78E-04 | 1.37 | 0.058 | 1697 | 9.36E-03 | 1.25 | 0.053 | 2029 |
| C10 | 8196155 | rs541045 | C | 0.431 | 10501 | 3.79E-04 | 1.15 | 0.466 | 1659 | 1.79E-03 | 1.12 | 0.459 | 1992 |
| C01 | 20789662 | rs12741305 | C | 0.036 | 10635 | 3.85E-04 | 1.4 | 0.05 | 1698 | 1.09E-04 | 1.41 | 0.05 | 2031 |
| C07 | 116688223 | rs2237721 | C | 0.069 | 10634 | 3.85E-04 | 1.29 | 0.087 | 1700 | 3.50E-04 | 1.27 | 0.086 | 2034 |
| C08 | 5498753 | rs10503316 | C | 0.776 | 10635 | 3.87E-04 | 1.19 | 0.804 | 1700 | 2.83E-03 | 1.14 | 0.798 | 2034 |
| C05 | 119516035 | rs7721883 | T | 0.461 | 10597 | 3.89E-04 | 1.15 | 0.496 | 1698 | 7.26E-03 | 1.1 | 0.486 | 2032 |
| C02 | 135122883 | rs2139311 | C | 0.594 | 10622 | 3.90E-04 | 1.15 | 0.628 | 1699 | 3.72E-04 | 1.14 | 0.626 | 2033 |
| C01 | 113676295 | rs2476601 | A | 0.13 | 10633 | 3.93E-04 | 1.22 | 0.154 | 1700 | 2.39E-04 | 1.21 | 0.153 | 2034 |
| C18 | 53271813 | rs634285 | G | 0.913 | 10634 | 3.98E-04 | 1.3 | 0.931 | 1700 | 3.28E-03 | 1.22 | 0.927 | 2034 |
| C14 | 99695081 | rs17776453 | G | 0.951 | 10633 | 4.00E-04 | 1.43 | 0.966 | 1699 | 2.30E-04 | 1.41 | 0.965 | 2033 |
| C05 | 56242295 | rs252890 | A | 0.425 | 10632 | 4.02E-04 | 1.15 | 0.459 | 1699 | 5.18E-04 | 1.14 | 0.456 | 2033 |
| C01 | 40523163 | rs7520394 | T | 0.651 | 10619 | 4.13E-04 | 1.16 | 0.684 | 1698 | 6.36E-03 | 1.11 | 0.674 | 2032 |
| C22 | 44896799 | rs739164 | A | 0.894 | 10523 | 4.21E-04 | 1.28 | 0.915 | 1645 | 5.43E-04 | 1.25 | 0.913 | 1978 |
| C10 | 90674890 | rs2243548 | A | 0.509 | 10625 | 4.25E-04 | 1.15 | 0.544 | 1698 | 5.06E-03 | 1.11 | 0.534 | 2031 |
| C10 | 90674890 | rs2243548 | A | 0.509 | 10625 | 4.25E-04 | 1.15 | 0.544 | 1698 | 5.06E-03 | 1.11 | 0.534 | 2031 |
| CX | 108301033 | rs3788769 | C | 0.445 | 8210 | 4.25E-04 | 1.19 | 0.487 | 1095 | 1.26E-03 | 1.16 | 0.481 | 1307 |
| C22 | 34873996 | rs7364143 | T | 0.28 | 10592 | 4.25E-04 | 1.16 | 0.311 | 1696 | 6.90E-04 | 1.15 | 0.308 | 2030 |
| C03 | 191533619 | rs9864293 | C | 0.385 | 10632 | 4.43E-04 | 1.15 | 0.419 | 1700 | 9.88E-04 | 1.13 | 0.415 | 2033 |
| C07 | 102617539 | rs2270019 | G | 0.165 | 10614 | 4.44E-04 | 1.2 | 0.192 | 1697 | 2.59E-04 | 1.19 | 0.191 | 2031 |
| C20 | 15229479 | rs6034134 | G | 0.594 | 10634 | 4.48E-04 | 1.15 | 0.628 | 1700 | 1.86E-03 | 1.12 | 0.622 | 2034 |
| C05 | 153269681 | rs1461236 | T | 0.855 | 10614 | 4.49E-04 | 1.23 | 0.879 | 1689 | 4.26E-03 | 1.17 | 0.873 | 2023 |
| C11 | 92904904 | rs2658801 | A | 0.269 | 10634 | 4.49E-04 | 1.16 | 0.3 | 1700 | 1.76E-04 | 1.16 | 0.299 | 2034 |
| C17 | 10435400 | rs9903582 | C | 0.745 | 10634 | 4.53E-04 | 1.18 | 0.775 | 1700 | 1.92E-03 | 1.14 | 0.769 | 2034 |
| C15 | 84295353 | rs4887480 | G | 0.769 | 10629 | 4.55E-04 | 1.18 | 0.798 | 1700 | 3.82E-03 | 1.14 | 0.791 | 2034 |
| C17 | 17860913 | rs752579 | G | 0.388 | 10632 | 4.57E-04 | 1.15 | 0.421 | 1700 | 1.55E-03 | 1.12 | 0.416 | 2034 |
| C17 | 17860913 | rs752579 | G | 0.388 | 10632 | 4.57E-04 | 1.15 | 0.421 | 1700 | 1.55E-03 | 1.12 | 0.416 | 2034 |
| C14 | 92765567 | rs10498639 | A | 0.423 | 10633 | 4.57E-04 | 1.15 | 0.457 | 1700 | 1.35E-03 | 1.12 | 0.452 | 2034 |
| C20 | 284362 | rs723477 | T | 0.74 | 10613 | 4.57E-04 | 1.18 | 0.77 | 1694 | 2.16E-03 | 1.14 | 0.764 | 2027 |
| C05 | 10009076 | rs16883905 | T | 0.111 | 10635 | 4.59E-04 | 1.23 | 0.134 | 1700 | 7.81E-04 | 1.21 | 0.131 | 2034 |
| C06 | 106193116 | rs845851 | A | 0.766 | 10631 | 4.60E-04 | 1.18 | 0.794 | 1700 | 2.14E-03 | 1.14 | 0.789 | 2034 |
| C08 | 143178480 | rs9644545 | T | 0.476 | 10632 | 4.61E-04 | 1.15 | 0.511 | 1700 | 4.98E-03 | 1.11 | 0.502 | 2034 |
| C12 | 9180220 | rs2160424 | G | 0.557 | 10608 | 4.62E-04 | 1.15 | 0.591 | 1690 | 4.58E-03 | 1.11 | 0.583 | 2022 |
| C12 | 19360345 | rs10743315 | G | 0.878 | 10613 | 4.84E-04 | 1.25 | 0.9 | 1694 | 1.58E-03 | 1.2 | 0.896 | 2028 |
| C05 | 56298431 | rs2408654 | C | 0.273 | 10635 | 4.84E-04 | 1.16 | 0.304 | 1700 | 4.74E-04 | 1.15 | 0.302 | 2034 |
| C05 | 167473287 | rs7705176 | G | 0.781 | 10635 | 4.87E-04 | 1.19 | 0.809 | 1700 | 1.68E-03 | 1.15 | 0.805 | 2034 |
| C03 | 62396924 | rs1997366 | C | 0.792 | 10623 | 4.88E-04 | 1.19 | 0.82 | 1700 | 9.05E-04 | 1.17 | 0.817 | 2033 |
| C03 | 62396924 | rs1997366 | C | 0.792 | 10623 | 4.88E-04 | 1.19 | 0.82 | 1700 | 9.05E-04 | 1.17 | 0.817 | 2033 |
| C04 | 21494114 | rs6448072 | C | 0.655 | 10634 | 4.89E-04 | 1.16 | 0.688 | 1700 | 1.95E-04 | 1.16 | 0.687 | 2034 |
| C20 | 49593462 | rs913673 | C | 0.539 | 10624 | 4.90E-04 | 1.15 | 0.574 | 1700 | 1.89 E-03 | 1.12 | 0.568 | 2034 |
| C10 | 119318254 | rs1557227 | C | 0.855 | 10635 | 4.91E-04 | 1.23 | 0.879 | 1700 | 4.19E-04 | 1.21 | 0.877 | 2034 |
| C05 | 158541049 | rs270661 | A | 0.205 | 10634 | 4.93 E-04 | 1.18 | 0.233 | 1699 | 3.06E-03 | 1.14 | 0.227 | 2033 |
| C05 | 158541049 | rs270661 | A | 0.205 | 10634 | 4.93 E-04 | 1.18 | 0.233 | 1699 | 3.06E-03 | 1.14 | 0.227 | 2033 |
| C06 | 72209498 | rs199623 | A | 0.523 | 10635 | 4.98E-04 | 1.15 | 0.557 | 1700 | 1.17E-03 | 1.13 | 0.552 | 2034 |
| C09 | 134953713 | rs2385891 | A | 0.544 | 10612 | 4.98E-04 | 1.15 | 0.578 | 1694 | 2.79E-03 | 1.12 | 0.571 | 2028 |
| C09 | 16321304 | rs9407756 | G | 0.67 | 10634 | 5.13E-04 | 1.16 | 0.702 | 1700 | 3.35E-03 | 1.12 | 0.695 | 2034 |
| C08 | 1315247 | rs7386449 | A | 0.68 | 10634 | 5.29E-04 | 1.16 | 0.711 | 1700 | 7.10E-04 | 1.14 | 0.708 | 2034 |
| C08 | 1315247 | rs7386449 | A | 0.68 | 10634 | 5.29E-04 | 1.16 | 0.711 | 1700 | 7.10E-04 | 1.14 | 0.708 | 2034 |
| C05 | 178690232 | rs3756566 | G | 0.401 | 10632 | 5.32E-04 | 1.15 | 0.434 | 1700 | 3.57E-04 | 1.14 | 0.433 | 2034 |
| C10 | 84919047 | rs11198993 | A | 0.688 | 10564 | 5.37E-04 | 1.16 | 0.719 | 1692 | 1.05E-05 | 1.19 | 0.725 | 2024 |
| C12 | 65801361 | rs1526839 | G | 0.831 | 10632 | 5.39E-04 | 1.21 | 0.856 | 1700 | 1.32E-04 | 1.21 | 0.856 | 2034 |
| C18 | 58503179 | rs12458349 | G | 0.061 | 10603 | 5.50E-04 | 1.3 | 0.078 | 1700 | 1.46E-04 | 1.31 | 0.078 | 2032 |
| C06 | 106204603 | rs4946680 | T | 0.632 | 10635 | 5.50E-04 | 1.15 | 0.665 | 1700 | 1.76E-03 | 1.13 | 0.66 | 2034 |
| C03 | 124473843 | rs9847700 | G | 0.852 | 10529 | 5.53E-04 | 1.23 | 0.876 | 1654 | 9.60E-04 | 1.2 | 0.873 | 1986 |
| C18 | 56405620 | rs603119 | T | 0.386 | 10606 | 5.54E-04 | 1.15 | 0.419 | 1699 | 9.25E-05 | 1.16 | 0.421 | 2032 |
| C06 | 113938753 | rs4945955 | T | 0.092 | 10614 | 5.55E-04 | 1.25 | 0.112 | 1697 | 5.18E-04 | 1.23 | 0.111 | 2031 |
| C05 | 26932085 | rs1382932 | C | 0.68 | 10635 | 5.60E-04 | 1.16 | 0.711 | 1700 | 1.13E-03 | 1.14 | 0.707 | 2034 |
| C05 | 76583322 | rs974280 | G | 0.357 | 10635 | 5.63E-04 | 1.15 | 0.39 | 1700 | 1.34E-03 | 1.13 | 0.385 | 2034 |
| C16 | 19044749 | rs7500550 | G | 0.774 | 10635 | 5.63E-04 | 1.18 | 0.802 | 1699 | 1.97E-03 | 1.15 | 0.798 | 2033 |
| C05 | 56284090 | rs1445998 | A | 0.4 | 10635 | 5.67E-04 | 1.15 | 0.433 | 1700 | 5.78E-04 | 1.14 | 0.43 | 2034 |
| C14 | 48189298 | rs4898630 | A | 0.876 | 10633 | 5.71E-04 | 1.24 | 0.898 | 1700 | 9.58E-04 | 1.21 | 0.895 | 2034 |
| C19 | 34828875 | rs4805463 | G | 0.906 | 10634 | 5.72E-04 | 1.28 | 0.925 | 1700 | 1.08E-03 | 1.24 | 0.923 | 2034 |
| C07 | 28352068 | rs1976489 | G | 0.249 | 10635 | 5.77E-04 | 1.17 | 0.279 | 1700 | 2.15E-03 | 1.14 | 0.274 | 2034 |
| C12 | 19263624 | rs9805106 | G | 0.817 | 10618 | 5.79E-04 | 1.2 | 0.843 | 1695 | 7.98E-04 | 1.18 | 0.84 | 2029 |
| C17 | 17915667 | rs11868035 | A | 0.296 | 10623 | 5.79E-04 | 1.16 | 0.327 | 1699 | 5.61E-03 | 1.12 | 0.319 | 2033 |
| C17 | 17915667 | rs11868035 | A | 0.296 | 10623 | 5.79E-04 | 1.16 | 0.327 | 1699 | 5.61E-03 | 1.12 | 0.319 | 2033 |
| C01 | 209613176 | rs792446 | G | 0.611 | 10634 | 5.80E-04 | 1.15 | 0.644 | 1700 | 1.30E-02 | 1.1 | 0.633 | 2033 |
| C13 | 109862872 | rs1163852 | C | 0.714 | 10635 | 5.83E-04 | 1.17 | 0.744 | 1700 | 2.89E-04 | 1.16 | 0.743 | 2034 |
| C03 | 22619885 | rs1870480 | G | 0.38 | 10635 | 5.85E-04 | 1.15 | 0.413 | 1700 | 3.12E-04 | 1.14 | 0.412 | 2034 |
| C15 | 97226804 | rs2684790 | T | 0.146 | 10634 | 5.87E-04 | 1.2 | 0.17 | 1700 | 7.79E-04 | 1.18 | 0.168 | 2034 |
| C14 | 44542892 | rs1742655 | C | 0.884 | 10635 | 5.91E-04 | 1.25 | 0.905 | 1700 | 3.42E-03 | 1.19 | 0.901 | 2034 |
| C12 | 114213023 | rs1920947 | C | 0.812 | 10635 | 5.92E-04 | 1.2 | 0.838 | 1700 | 7.20E-03 | 1.14 | 0.831 | 2034 |
| C01 | 30387821 | rs12029866 | G | 0.771 | 10634 | 5.93E-04 | 1.18 | 0.799 | 1700 | 1.33E-04 | 1.19 | 0.8 | 2034 |
| C11 | 91095458 | rs2568712 | T | 0.385 | 10599 | 5.98E-04 | 1.15 | 0.418 | 1699 | 1.68E-03 | 1.12 | 0.413 | 2031 |
| C01 | 168129978 | rs11582943 | A | 0.94 | 10619 | 5.99E-04 | 1.36 | 0.955 | 1693 | 6.87E-04 | 1.33 | 0.954 | 2027 |
| C01 | 168129978 | rs11582943 | A | 0.94 | 10619 | 5.99E-04 | 1.36 | 0.955 | 1693 | 6.87E-04 | 1.33 | 0.954 | 2027 |
| C02 | 49219942 | rs3788982 | G | 0.871 | 10635 | 6.02E-04 | 1.24 | 0.893 | 1700 | 8.11E-05 | 1.25 | 0.894 | 2034 |
| C10 | 131670000 | rs639079 | G | 0.187 | 10389 | 6.03E-04 | 1.18 | 0.214 | 1664 | 7.05E-04 | 1.17 | 0.212 | 1992 |
| C01 | 76636957 | rs1566250 | G | 0.702 | 10634 | 6.13E-04 | 1.16 | 0.732 | 1700 | 1.98E-04 | 1.16 | 0.733 | 2034 |
| C12 | 87964674 | rs1401875 | T | 0.057 | 10626 | 6.13E-04 | 1.31 | 0.073 | 1697 | 1.83E-03 | 1.26 | 0.07 | 2031 |
| C12 | 87964674 | rs1401875 | T | 0.057 | 10626 | 6.13E-04 | 1.31 | 0.073 | 1697 | 1.83E-03 | 1.26 | 0.07 | 2031 |
| C05 | 158753477 | rs953861 | G | 0.16 | 10632 | 6.14E-04 | 1.19 | 0.185 | 1700 | 1.51E-02 | 1.13 | 0.176 | 2034 |
| C01 | 82599958 | rs12032817 | G | 0.371 | 10635 | 6.15E-04 | 1.15 | 0.404 | 1700 | 6.29E-03 | 1.11 | 0.396 | 2034 |
| C03 | 78544480 | rs2055707 | A | 0.418 | 10603 | 6.20E-04 | 1.15 | 0.451 | 1699 | 2.42E-03 | 1.12 | 0.445 | 2033 |
| C19 | 4231186 | rs10419363 | T | 0.299 | 10610 | 6.22E-04 | 1.16 | 0.331 | 1695 | 8.39E-04 | 1.14 | 0.328 | 2028 |
| C02 | 235330231 | rs12617590 | C | 0.199 | 10624 | 6.29E-04 | 1.18 | 0.227 | 1700 | 2.26E-03 | 1.15 | 0.222 | 2034 |
| C04 | 87398087 | rs2589506 | G | 0.451 | 10632 | 6.31E-04 | 1.14 | 0.485 | 1700 | 1.15E-04 | 1.15 | 0.486 | 2034 |
| C10 | 87792858 | rs3814614 | T | 0.501 | 10628 | 6.35E-04 | 1.14 | 0.535 | 1700 | 1.05E-03 | 1.13 | 0.531 | 2034 |
| C04 | 97290133 | rs10516987 | G | 0.797 | 10598 | 6.35E-04 | 1.19 | 0.824 | 1691 | 1.55E-03 | 1.16 | 0.82 | 2023 |
| C13 | 101108783 | rs1375719 | C | 0.499 | 10634 | 6.36E-04 | 1.14 | 0.532 | 1700 | 1.03E-02 | 1.1 | 0.522 | 2034 |
| C17 | 18231090 | rs854793 | A | 0.202 | 10631 | 6.36E-04 | 1.18 | 0.229 | 1700 | 1.59E-03 | 1.15 | 0.225 | 2034 |
| C15 | 76722261 | rs11072793 | G | 0.227 | 10613 | 6.38E-04 | 1.17 | 0.255 | 1695 | 1.52E-03 | 1.14 | 0.251 | 2029 |
| C09 | 8437732 | rs2890795 | T | 0.577 | 10630 | 6.38E-04 | 1.15 | 0.61 | 1700 | 1.20E-03 | 1.13 | 0.606 | 2034 |
| C07 | 67296404 | rs4527771 | A | 0.406 | 10572 | 6.43 E-04 | 1.15 | 0.439 | 1694 | 8.45E-03 | 1.1 | 0.43 | 2027 |
| C13 | 66843127 | rs17070671 | T | 0.833 | 10633 | 6.44E-04 | 1.21 | 0.857 | 1700 | 9.00E-03 | 1.14 | 0.85 | 2034 |
| C16 | 73542120 | rs1038864 | C | 0.183 | 10634 | 6.45E-04 | 1.18 | 0.209 | 1700 | 4.45E-03 | 1.14 | 0.203 | 2034 |
| C06 | 25536933 | rs722086 | T | 0.856 | 10166 | 6.47E-04 | 1.22 | 0.879 | 1687 | 7.47E-03 | 1.16 | 0.873 | 2015 |
| C08 | 70811871 | rs4738034 | C | 0.701 | 10635 | 6.49E-04 | 1.16 | 0.731 | 1700 | 9.61E-04 | 1.14 | 0.728 | 2034 |
| C18 | 55095560 | rs657773 | C | 0.551 | 10634 | 6.57E-04 | 1.15 | 0.585 | 1700 | 1.04E-03 | 1.13 | 0.581 | 2034 |
| C02 | 134844429 | rs2321299 | C | 0.568 | 10633 | 6.58E-04 | 1.15 | 0.601 | 1700 | 3.50E-03 | 1.11 | 0.595 | 2033 |
| C06 | 149543409 | rs6570956 | T | 0.734 | 10635 | 6.61E-04 | 1.17 | 0.764 | 1700 | 6.82E-03 | 1.12 | 0.756 | 2034 |
| C12 | 116667030 | rs4767629 | T | 0.139 | 10634 | 6.61E-04 | 1.2 | 0.163 | 1700 | 5.33E-03 | 1.15 | 0.157 | 2034 |
| C01 | 80891035 | rs1030414 | G | 0.901 | 10635 | 6.73E-04 | 1.27 | 0.92 | 1700 | 3.45E-04 | 1.26 | 0.92 | 2033 |
| C06 | 69176005 | rs1565487 | A | 0.856 | 10630 | 6.74E-04 | 1.22 | 0.879 | 1700 | 2.07E-04 | 1.22 | 0.879 | 2034 |
| C10 | 123913719 | rs2239586 | T | 0.118 | 10630 | 6.75E-04 | 1.22 | 0.14 | 1700 | 2.95E-03 | 1.18 | 0.136 | 2034 |
| C11 | 122290366 | rs4936757 | C | 0.565 | 10633 | 6.76E-04 | 1.15 | 0.598 | 1700 | 3.91 E-03 | 1.11 | 0.591 | 2034 |
| C12 | 22090132 | rs2955503 | A | 0.654 | 10629 | 6.82E-04 | 1.15 | 0.686 | 1700 | 2.41E-04 | 1.15 | 0.686 | 2033 |
| C17 | 52089014 | rs11656018 | C | 0.703 | 10600 | 6.84E-04 | 1.16 | 0.733 | 1699 | 8.06E-04 | 1.15 | 0.731 | 2032 |
| C06 | 159976575 | rs2758317 | C | 0.427 | 10572 | 6.86E-04 | 1.14 | 0.46 | 1690 | 6.67E-03 | 1.11 | 0.452 | 2019 |
| C18 | 43266738 | rs17734724 | G | 0.271 | 10635 | 6.87E-04 | 1.16 | 0.301 | 1700 | 3.27E-03 | 1.13 | 0.295 | 2034 |
| C02 | 19979982 | rs12467812 | G | 0.331 | 10634 | 6.88E-04 | 1.15 | 0.363 | 1700 | 2.30E-04 | 1.15 | 0.363 | 2034 |
| C15 | 51085506 | rs1156234 | G | 0.791 | 10633 | 6.91E-04 | 1.18 | 0.818 | 1700 | 5.59E-04 | 1.17 | 0.816 | 2034 |
| C10 | 123156230 | rs2936875 | T | 0.782 | 10629 | 6.92E-04 | 1.18 | 0.809 | 1699 | 4.42E-03 | 1.14 | 0.803 | 2033 |
| C19 | 58638423 | rs11668495 | C | 0.944 | 10470 | 7.08E-04 | 1.38 | 0.959 | 1668 | 1.43E-03 | 1.32 | 0.957 | 1995 |
| C07 | 136198861 | rs11761344 | T | 0.116 | 10635 | 7.12E-04 | 1.22 | 0.138 | 1699 | 8.84E-04 | 1.2 | 0.136 | 2033 |
| C10 | 84739213 | rs4617529 | G | 0.852 | 10635 | 7.15E-04 | 1.22 | 0.875 | 1700 | 6.68E-04 | 1.2 | 0.873 | 2034 |
| C08 | 139196618 | rs4909761 | T | 0.262 | 10634 | 7.20E-04 | 1.16 | 0.291 | 1700 | 2.65E-03 | 1.13 | 0.286 | 2034 |
| C05 | 94602573 | rs6556861 | G | 0.567 | 10634 | 7.24E-04 | 1.14 | 0.6 | 1700 | 2.99E-03 | 1.12 | 0.594 | 2034 |
| C09 | 132606652 | rs1930788 | C | 0.247 | 10635 | 7.27E-04 | 1.16 | 0.276 | 1700 | 4.10E-03 | 1.13 | 0.27 | 2034 |
| C05 | 157926810 | rs2964513 | A | 0.689 | 10559 | 7.29E-04 | 1.16 | 0.72 | 1698 | 3.01E-03 | 1.13 | 0.714 | 2030 |
| C08 | 5508780 | rs6991017 | T | 0.794 | 10607 | 7.30E-04 | 1.19 | 0.82 | 1698 | 5.36E-03 | 1.14 | 0.814 | 2032 |
| C02 | 135133800 | rs12473666 | T | 0.137 | 10629 | 7.31E-04 | 1.2 | 0.16 | 1700 | 2.94E-03 | 1.17 | 0.156 | 2034 |
| C17 | 18149041 | rs2955355 | C | 0.287 | 10635 | 7.35E-04 | 1.16 | 0.317 | 1700 | 3.19E-03 | 1.12 | 0.311 | 2034 |
| C11 | 44349237 | rs120838271 | C | 0.21 | 10635 | 7.42E-04 | 1.17 | 0.238 | 1700 | 3.32E-03 | 1.14 | 0.233 | 2034 |
| C01 | 238617451 | rs1341449 | A | 0.545 | 10559 | 7.44E-04 | 1.14 | 0.579 | 1681 | 1.15E-03 | 1.13 | 0.575 | 2014 |
| C15 | 34765965 | rs1901725 | T | 0.125 | 10635 | 7.47E-04 | 1.21 | 0.148 | 1700 | 3.63E-03 | 1.17 | 0.143 | 2034 |
| C10 | 87786571 | rs4933391 | A | 0.386 | 10634 | 7.50E-04 | 1.14 | 0.418 | 1700 | 1.30E-03 | 1.13 | 0.414 | 2034 |
| C01 | 80911800 | rs7531507 | G | 0.921 | 10634 | 7.52E-04 | 1.3 | 0.938 | 1700 | 3.18E-04 | 1.3 | 0.938 | 2034 |
| C14 | 57289229 | rs2069334 | A | 0.184 | 10635 | 7.54E-04 | 1.18 | 0.211 | 1700 | 6.14E-03 | 1.13 | 0.204 | 2034 |
| C02 | 17299060 | rs2342551 | T | 0.713 | 10635 | 7.54E-04 | 1.16 | 0.743 | 1700 | 1.23E-03 | 1.14 | 0.74 | 2034 |
| C02 | 128492107 | rs2244871 | C | 0.41 | 10632 | 7.62E-04 | 1.14 | 0.442 | 1700 | 7.38E-04 | 1.13 | 0.44 | 2034 |
| C16 | 75061517 | rs1862737 | C | 0.481 | 10635 | 7.67E-04 | 1.14 | 0.514 | 1700 | 1.75E-03 | 1.12 | 0.51 | 2034 |
| C04 | 129868334 | rs7690289 | T | 0.63 | 10596 | 7.70E-04 | 1.15 | 0.661 | 1697 | 7.24E-04 | 1.14 | 0.659 | 2031 |
| C04 | 129868334 | rs7690289 | T | 0.63 | 10596 | 7.70E-04 | 1.15 | 0.661 | 1697 | 7.24E-04 | 1.14 | 0.659 | 2031 |
| C18 | 73086657 | rs2156643 | G | 0.41 | 10631 | 7.70E-04 | 1.14 | 0.443 | 1700 | 1.49E-03 | 1.12 | 0.439 | 2034 |
| C11 | 7440298 | rs11041390 | A | 0.729 | 10635 | 7.70E-04 | 1.16 | 0.758 | 1700 | 8.75E-03 | 1.12 | 0.75 | 2034 |
| C15 | 34765482 | rs7183966 | G | 0.125 | 10632 | 7.75E-04 | 1.21 | 0.148 | 1698 | 3.73E-03 | 1.17 | 0.143 | 2032 |
| C09 | 28114180 | rs1452336 | A | 0.495 | 10608 | 7.76E-04 | 1.14 | 0.529 | 1695 | 1.89E-04 | 1.15 | 0.53 | 2029 |
| C05 | 102480237 | rs34374 | A | 0.671 | 10635 | 7.80E-04 | 1.15 | 0.702 | 1699 | 5.86E-03 | 1.11 | 0.695 | 2033 |
| C05 | 82104191 | rs6452479 | C | 0.934 | 10595 | 7.88E-04 | 1.33 | 0.95 | 1695 | 6.46E-04 | 1.31 | 0.949 | 2028 |
| C18 | 73085584 | rs2850889 | C | 0.41 | 10634 | 7.91E-04 | 1.14 | 0.443 | 1700 | 1.53E-03 | 1.12 | 0.439 | 2034 |
| C11 | 20014579 | rs920675 | A | 0.358 | 10635 | 7.95E-04 | 1.15 | 0.39 | 1700 | 5.37E-04 | 1.14 | 0.389 | 2034 |
| C03 | 39292564 | rs2853705 | T | 0.209 | 10634 | 7.97E-04 | 1.17 | 0.236 | 1700 | 2.79E-03 | 1.14 | 0.231 | 2034 |
| C18 | 73086818 | rs2850892 | C | 0.41 | 10631 | 7.98E-04 | 1.14 | 0.443 | 1700 | 1.55E-03 | 1.12 | 0.439 | 2034 |
| C04 | 178149338 | *rs309770* | G | 0.889 | 10635 | 8.01E-04 | 1.25 | 0.909 | 1700 | 5.03E-04 | 1.24 | 0.908 | 2034 |
| C11 | 7444767 | rs7113222 | G | 0.739 | 10632 | 8.03E-04 | 1.17 | 0.768 | 1700 | 1.29E-02 | 1.11 | 0.759 | 2034 |
| C03 | 6735499 | rs7638636 | A | 0.494 | 10231 | 8.03E-04 | 1.14 | 0.528 | 1648 | 5.59E-04 | 1.14 | 0.526 | 1972 |
| C02 | 42489923 | rs10202624 | A | 0.094 | 10634 | 8.15E-04 | 1.24 | 0.114 | 1700 | 1.79E-04 | 1.25 | 0.114 | 2034 |
| C06 | 20707867 | rs2294809 | G | 0.799 | 10626 | 8.19E-04 | 1.19 | 0.825 | 1699 | 3.18E-03 | 1.15 | 0.82 | 2033 |
| C02 | 118487620 | rs2245113 | A | 0.174 | 10632 | 8.20E-04 | 1.18 | 0.199 | 1700 | 1.79E-02 | 1.12 | 0.191 | 2034 |
| C19 | 17024661 | rs2305758 | G | 0.684 | 10633 | 8.21E-04 | 1.16 | 0.714 | 1700 | 4.38E-04 | 1.15 | 0.714 | 2034 |
| C19 | 17024661 | rs2305758 | G | 0.684 | 10633 | 8.21E-04 | 1.16 | 0.714 | 1700 | 4.38E-04 | 1.15 | 0.714 | 2034 |
| C01 | 63908878 | rs1747924 | T | 0.808 | 10622 | 8.23E-04 | 1.19 | 0.834 | 1700 | 9.80E-03 | 1.13 | 0.827 | 2034 |
| C22 | 34883676 | rs5995259 | A | 0.337 | 10635 | 8.26E-04 | 1.15 | 0.369 | 1700 | 2.83E-03 | 1.12 | 0.363 | 2034 |
| C02 | 45292999 | rs908571 | C | 0.291 | 10635 | 8.27E-04 | 1.15 | 0.321 | 1699 | 1.62E-03 | 1.13 | 0.318 | 2033 |
| C20 | 33195666 | rs734484 | C | 0.709 | 10635 | 8.29E-04 | 1.16 | 0.738 | 1699 | 2.20E-03 | 1.13 | 0.734 | 2033 |
| C16 | 2009168 | rs8057913 | C | 0.264 | 10634 | 8.39E-04 | 1.16 | 0.293 | 1700 | 1.13E-03 | 1.14 | 0.29 | 2034 |
| C03 | 99699602 | rs3796139 | G | 0.145 | 10476 | 8.40E-04 | 1.2 | 0.169 | 1685 | 4.25E-03 | 1.16 | 0.164 | 2011 |
| C02 | 135194839 | rs1257208 | A | 0.138 | 10633 | 8.41E-04 | 1.2 | 0.161 | 1700 | 1.98E-03 | 1.17 | 0.158 | 2034 |
| C20 | 5928108 | rs236110 | G | 0.87 | 10635 | 8.43E-04 | 1.23 | 0.892 | 1698 | 9.17E-04 | 1.21 | 0.89 | 2031 |
| C05 | 3644654 | rs710998 | T | 0.899 | 10630 | 8.55E-04 | 1.26 | 0.918 | 1699 | 1.20E-03 | 1.23 | 0.917 | 2033 |
| C02 | 49398605 | rs6743414 | G | 0.847 | 10634 | 8.62E-04 | 1.21 | 0.87 | 1700 | 1.51E-04 | 1.22 | 0.871 | 2034 |
| C13 | 38516680 | rs9315759 | C | 0.13 | 10635 | 8.63E-04 | 1.21 | 0.152 | 1700 | 3.14E-03 | 1.17 | 0.148 | 2034 |
| C01 | 184657396 | rs4651384 | G | 0.687 | 10634 | 8.72E-04 | 1.15 | 0.717 | 1700 | 4.41E-03 | 1.12 | 0.711 | 2034 |
| C19 | 17031885 | rs12459084 | T | 0.351 | 10633 | 8.76E-04 | 1.15 | 0.382 | 1700 | 3.24E-03 | 1.12 | 0.376 | 2034 |
| C17 | 48847103 | rs4794128 | A | 0.272 | 10634 | 8.77E-04 | 1.16 | 0.302 | 1700 | 1.05E-03 | 1.14 | 0.299 | 2034 |
| C08 | 32544721 | rs12547858 | T | 0.195 | 10624 | 8.80E-04 | 1.17 | 0.221 | 1700 | 7.59E-04 | 1.16 | 0.22 | 2034 |
| C09 | 16318137 | rs4961695 | C | 0.803 | 10614 | 8.81E-04 | 1.19 | 0.829 | 1697 | 1.23E-03 | 1.17 | 0.826 | 2029 |
| C02 | 170296713 | rs2268365 | G | 0.125 | 10635 | 8.83E-04 | 1.21 | 0.147 | 1698 | 2.14E-03 | 1.18 | 0.144 | 2032 |
| C02 | 170296713 | rs2268365 | G | 0.125 | 10635 | 8.83E-04 | 1.21 | 0.147 | 1698 | 2.14E-03 | 1.18 | 0.144 | 2032 |
| C07 | 17752626 | rs4470902 | C | 0.417 | 10634 | 8.84E-04 | 1.14 | 0.45 | 1700 | 8.58E-04 | 1.13 | 0.447 | 2034 |
| C14 | 57303694 | rs17095237 | G | 0.16 | 10634 | 8.85E-04 | 1.19 | 0.185 | 1700 | 6.54E-03 | 1.14 | 0.179 | 2034 |
| C05 | 38969280 | rs357291 | C | 0.395 | 10579 | 8.89E-04 | 1.14 | 0.428 | 1688 | 6.20E-03 | 1.11 | 0.42 | 2020 |
| C06 | 30109076 | rs166327 | A | 0.601 | 10634 | 8.91E-04 | 1.14 | 0.633 | 1700 | 4.98E-03 | 1.11 | 0.626 | 2034 |
| C03 | 125374402 | rs6438839 | A | 0.077 | 10632 | 8.92E-04 | 1.26 | 0.095 | 1700 | 4.40E-04 | 1.25 | 0.095 | 2034 |
| C05 | 153231957 | rs1870739 | C | 0.855 | 10629 | 8.92E-04 | 1.21 | 0.877 | 1697 | 6.74E-03 | 1.16 | 0.872 | 2031 |
| C01 | 40507124 | rs661221 | G | 0.803 | 10614 | 8.92E-04 | 1.19 | 0.828 | 1697 | 8.83E-03 | 1.13 | 0.821 | 2030 |
| C05 | 50944695 | rs4865673 | G | 0.643 | 10635 | 8.94E-04 | 1.15 | 0.674 | 1700 | 1.99E-03 | 1.13 | 0.67 | 2034 |
| C03 | 186444329 | rs6803944 | C | 0.841 | 10635 | 8.95E-04 | 1.21 | 0.865 | 1700 | 7.54E-04 | 1.19 | 0.863 | 2034 |
| C05 | 102439618 | rs26434 | T | 0.704 | 10617 | 8.95E-04 | 1.16 | 0.734 | 1697 | 5.12E-03 | 1.12 | 0.727 | 2031 |
| Cl0 | 29327574 | rs788053 | T | 0.747 | 10635 | 9.01E-04 | 1.17 | 0.775 | 1700 | 1.80E-03 | 1.14 | 0.771 | 2034 |
| C12 | 101671864 | rs12311520 | C | 0.867 | 10624 | 9.12E-04 | 1.22 | 0.889 | 1700 | 5.86E-03 | 1.17 | 0.884 | 2033 |
| C01 | 29565842 | rs1932397 | G | 0.821 | 10635 | 9.14E-04 | 1.19 | 0.845 | 1700 | 2.07E-03 | 1.16 | 0.842 | 2034 |
| C04 | 10653042 | rs959233 | G | 0.445 | 10622 | 9.20E-04 | 1.14 | 0.477 | 1700 | 1.23E-03 | 1.13 | 0.474 | 2033 |
| C08 | 144018676 | rs5297 | C | 0.09 | 10632 | 9.22E-04 | 1.24 | 0.109 | 1700 | 4.01E-03 | 1.19 | 0.105 | 2034 |
| C08 | 143110958 | rs747551 | T | 0.436 | 10633 | 9.27E-04 | 1.14 | 0.469 | 1700 | 1.39E-03 | 1.12 | 0.465 | 2034 |
| C18 | 55790697 | rs8082768 | T | 0.18 | 10621 | 9.28E-04 | 1.18 | 0.206 | 1700 | 1.97E-04 | 1.19 | 0.207 | 2034 |
| C12 | 111537264 | rs1015249 | C | 0.689 | 10635 | 9.36E-04 | 1.15 | 0.719 | 1700 | 1.59E-03 | 1.13 | 0.715 | 2034 |
| C08 | 59026980 | rs7010934 | C | 0.842 | 10632 | 9.45E-04 | 1.2 | 0.865 | 1700 | 2.40E-03 | 1.17 | 0.862 | 2034 |
| C01 | 78361370 | rs1412414 | G | 0.621 | 10634 | 9.53E-04 | 1.15 | 0.652 | 1699 | 5.85E-03 | 1.11 | 0.645 | 2033 |
| C06 | 165543591 | rs1744503 | T | 0.478 | 10631 | 9.57E-04 | 1.14 | 0.511 | 1700 | 2.74E-04 | 1.14 | 0.512 | 2033 |
| C01 | 76521831 | rs10518531 | C | 0.842 | 10561 | 9.57E-04 | 1.21 | 0.865 | 1668 | 3.57E-04 | 1.21 | 0.865 | 1998 |
| C12 | 119365986 | rs719450 | C | 0.918 | 10635 | 9.57E-04 | 1.29 | 0.935 | 1700 | 2.69E-04 | 1.3 | 0.935 | 2034 |
| C12 | 119365986 | rs719450 | C | 0.918 | 10635 | 9.57E-04 | 1.29 | 0.935 | 1700 | 2.69E-04 | 1.3 | 0.935 | 2034 |
| C03 | 175246538 | rs1421420 | A | 0.725 | 10633 | 9.62E-04 | 1.16 | 0.754 | 1700 | 2.21E-02 | 1.1 | 0.743 | 2034 |
| C06 | 29542163 | rs2107191 | G | 0.439 | 10634 | 9.63E-04 | 1.14 | 0.471 | 1700 | 4.51E-03 | 1.11 | 0.465 | 2034 |
| C06 | 29542163 | rs2107191 | G | 0.439 | 10634 | 9.63E-04 | 1.14 | 0.471 | 1700 | 4.51E-03 | 1.11 | 0.465 | 2034 |
| C05 | 76806011 | rs3816609 | A | 0.356 | 10632 | 9.64E-04 | 1.14 | 0.387 | 1700 | 2.31 E-03 | 1.12 | 0.382 | 2034 |
| C18 | 56570151 | rs2332026 | A | 0.499 | 10634 | 9.72E-04 | 1.14 | 0.532 | 1700 | 1.79E-03 | 1.12 | 0.528 | 2034 |
| C17 | 68940662 | rs312750 | C | 0.547 | 10633 | 9.72E-04 | 1.14 | 0.579 | 1700 | 7.18E-03 | 1.1 | 0.572 | 2034 |
| C03 | 72792674 | rs2208 | A | 0.265 | 10634 | 9.76E-04 | 1.16 | 0.294 | 1700 | 4.77E-03 | 1.12 | 0.288 | 2034 |
| C03 | 72733647 | rs7632726 | T | 0.307 | 10635 | 9.77E-04 | 1.15 | 0.337 | 1700 | 3.48E-03 | 1.12 | 0.332 | 2034 |
| C03 | 145911459 | rs4401402 | T | 0.583 | 10633 | 9.79E-04 | 1.14 | 0.615 | 1700 | 1.80E-03 | 1.12 | 0.611 | 2034 |
| C04 | 30752184 | rs4132132 | G | 0.439 | 10618 | 9.80E-04 | 1.14 | 0.471 | 1696 | 7.47E-04 | 1.13 | 0.47 | 2030 |
| C01 | 88967295 | rs10801705 | A | 0.456 | 10635 | 9.80E-04 | 1.14 | 0.489 | 1699 | 8.76E-04 | 1.13 | 0.487 | 2033 |
| C10 | 123151213 | rs2935689 | C | 0.837 | 10635 | 9.81E-04 | 1.2 | 0.86 | 1700 | 6.25E-03 | 1.15 | 0.855 | 2034 |
| C18 | 39875310 | rs1015735 | C | 0.262 | 10605 | 9.82E-04 | 1.16 | 0.291 | 1695 | 2.57E-03 | 1.13 | 0.286 | 2029 |
| C04 | 82259686 | rs10026644 | G | 0.926 | 10619 | 9.83E-04 | 1.3 | 0.942 | 1697 | 4.26E-03 | 1.23 | 0.939 | 2028 |
| C20 | 4111436 | rs1741285 | G | 0.56 | 10634 | 9.88E-04 | 1.14 | 0.592 | 1698 | 1.72E-03 | 1.12 | 0.588 | 2032 |
| C20 | 4111436 | rs1741285 | G | 0.56 | 10634 | 9.88E-04 | 1.14 | 0.592 | 1698 | 1.72E-03 | 1.12 | 0.588 | 2032 |
| C14 | 47963078 | rs8007161 | T | 0.884 | 10602 | 9.90E-04 | 1.24 | 0.904 | 1698 | 1.63E-03 | 1.21 | 0.901 | 2030 |
| C14 | 57288897 | rs392030 | G | 0.635 | 10635 | 9.98E-04 | 1.15 | 0.666 | 1700 | 2.27E-03 | 1.12 | 0.661 | 2034 |
| C01 | 19432522 | rs7523192 | T | 0.809 | 10634 | 1.01E-03 | 1.19 | 0.834 | 1700 | 2.17 E-02 | 1.12 | 0.826 | 2034 |
| C08 | 144284828 | rs4977114 | C | 0.026 | 10635 | 1.01E-03 | 1.44 | 0.037 | 1700 | 7.98E-04 | 1.42 | 0.036 | 2034 |
| CX | 4923358 | rs1919026 | C | 0.189 | 8271 | 1.02E-03 | 1.22 | 0.221 | 1103 | 4.66E-03 | 1.17 | 0.214 | 1316 |
| C01 | 18207864 | rs2946534 | T | 0.572 | 10631 | 1.03E-03 | 1.14 | 0.604 | 1700 | 7.54E-04 | 1.13 | 0.602 | 2034 |
| C09 | 28116482 | rs1452337 | G | 0.798 | 10568 | 1.03E-03 | 1.18 | 0.824 | 1687 | 1.01E-03 | 1.17 | 0.822 | 2021 |
| C19 | 17039119 | rs7254154 | C | 0.351 | 10617 | 1.03E-03 | 1.14 | 0.383 | 1695 | 3.90E-03 | 1.12 | 0.377 | 2029 |
| C19 | 17039119 | rs7254154 | C | 0.351 | 10617 | 1.03E-03 | 1.14 | 0.383 | 1695 | 3.90E-03 | 1.12 | 0.377 | 2029 |
| C08 | 73727022 | rs1837544 | G | 0.24 | 10634 | 1.03E-03 | 1.16 | 0.268 | 1700 | 2.02E-03 | 1.14 | 0.264 | 2034 |
| C02 | 183500703 | rs1946815 | G | 0.216 | 10634 | 1.03E-03 | 1.17 | 0.243 | 1700 | 2.25E-03 | 1.14 | 0.239 | 2034 |
| C07 | 102702234. | rs17152161 | C | 0.159 | 10633 | 1.03E-03 | 1.19 | 0.183 | 1700 | 5.79E-03 | 1.14 | 0.177 | 2034 |
| C02 | 216388367 | rs13417729 | C | 0.713 | 10635 | 1.04E-03 | 1.16 | 0.742 | 1700 | 1.20E-03 | 1.14 | 0.74 | 2034 |
| C20 | 13933789 | rs6079235 | C | 0.735 | 10635 | 1.04E-03 | 1.16 | 0.763 | 1700 | 1.05E-02 | 1.11 | 0.755 | 2034 |
| C04 | 8425936 | rs3756193 | G | 0.46 | 10623 | 1.05E-03 | 1.14 | 0.492 | 1699 | 2.26E-03 | 1.12 | 0.488 | 2033 |
| C05 | 76635083 | rs4704400 | T | 0.496 | 10628 | 1.06E-03 | 1.14 | 0.528 | 1699 | 3.23E-03 | 1.11 | 0.523 | 2033 |
| C17 | 17897321 | rs11649804 | A | 0.292 | 10506 | 1.06E-03 | 1.15 | 0.322 | 1650 | 7.96E-03 | 1.11 | 0.315 | 1979 |
| C17 | 17897321 | rs11649804 | A | 0.292 | 10506 | 1.06E-03 | 1.15 | 0.322 | 1650 | 7.96E-03 | 1.11 | 0.315 | 1979 |
| C14 | 64588026 | rs17754686 | A | 0.306 | 10606 | 1.06E-03 | 1.15 | 0.336 | 1698 | 1.49E-03 | 1.13 | 0.333 | 2032 |
| C16 | 2013121 | rs8045288 | T | 0.266 | 10618 | 1.06E-03 | 1.15 | 0.295 | 1700 | 1.23E-03 | 1.14 | 0.293 | 2034 |
| C04 | 139047812 | rs10028163 | G | 0.202 | 10634 | 1.06E-03 | 1.17 | 0.229 | 1700 | 4.06E-04 | 1.17 | 0.229 | 2034 |
| C14 | 98131730 | rs7158073 | T | 0.429 | 10592 | 1.06E-03 | 1.14 | 0.461 | 1696 | 2.61E-03 | 1.12 | 0.457 | 2029 |
| C07 | 42943442 | rs3757564 | C | 0.069 | 10624 | 1.07E-03 | 1.27 | 0.085 | 1696 | 1.02E-03 | 1.25 | 0.084 | 2029 |
| C11 | 110045689 | rs11604632 | A | 0.173 | 10632 | 1.07E-03 | 1.18 | 0.198 | 1700 | 4.90E-04 | 1.18 | 0.197 | 2032 |
| C17 | 17862368 | rs4925109 | A | 0.317 | 10634 | 1.07E-03 | 1.15 | 0.348 | 1700 | 3.75E-03 | 1.12 | 0.342 | 2034 |
| C17 | 17862368 | rs4925109 | A | 0.317 | 10634 | 1.07E-03 | 1.15 | 0.348 | 1700 | 3.75E-03 | 1.12 | 0.342 | 2034 |
| C12 | 96888528 | rs7135604 | A | 0.652 | 10635 | 1.07E-03 | 1.15 | 0.682 | 1699 | 2.11E-02 | 1.09 | 0.672 | 2033 |
| C11 | 97095503 | rs1039953 | C | 0.68 | 10635 | 1.08E-03 | 1.15 | 0.71 | 1700 | 1.67E-03 | 1.13 | 0.706 | 2034 |
| C04 | 163985671 | rs9991428 | G | 0.96 | 10613 | 1.08E-03 | 1.44 | 0.972 | 1699 | 3.03E-03 | 1.35 | 0.97 | 2033 |
| C01 | 148995481 | rs9826 | G | 0.326 | 10553 | 1.09E-03 | 1.15 | 0.357 | 1687 | 5.76E-03 | 1.11 | 0.35 | 2016 |
| C15 | 90440532 | rs6496899 | C | 0.763 | 10468 | 1.09E-03 | 1.17 | 0.79 | 1684 | 1.08E-03 | 1.16 | 0.788 | 2015 |
| C17 | 70257535 | rs9911654 | T | 0.894 | 10635 | 1.09E-03 | 1.25 | 0.913 | 1700 | 2.81E-02 | 1.14 | 0.906 | 2034 |
| C06 | 104259812 | rs9373729 | G | 0.355 | 10623 | 1.09E-03 | 1.14 | 0.386 | 1698 | 2.92E-03 | 1.12 | 0.381 | 2032 |
| C05 | 102509625 | rs34813 | G | 0.709 | 10635 | 1.11E-03 | 1.16 | 0.738 | 1700 | 8.32E-03 | 1.11 | 0.731 | 2034 |
| C11 | 134184293 | rs1289450 | A | 0.196 | 10629 | 1.11E-03 | 1.17 | 0.222 | 1699 | 5.54E-04 | 1.17 | 0.221 | 2033 |
| C12 | 69318052 | rs2584019 | T | 0.89 | 10635 | 1.11E-03 | 1.24 | 0.909 | 1700 | 2.22E-04 | 1.26 | 0.91 | 2034 |
| C12 | 69318052 | rs2584019 | T | 0.89 | 10635 | 1.11E-03 | 1.24 | 0.909 | 1700 | 2.22E-04 | 1.26 | 0.91 | 2034 |
| C08 | 79248604 | rs2369121 | T | 0.72 | 10633 | 1.11E-03 | 1.16 | 0.749 | 1700 | 8.70E-04 | 1.15 | 0.747 | 2034 |
| C07 | 23296885 | rs1542608 | G | 0.192 | 10634 | 1.11E-03 | 1.17 | 0.218 | 1700 | 1.53E-03 | 1.15 | 0.215 | 2034 |
| C10 | 15456765 | rs1925819 | T | 0.558 | 10635 | 1.11E-03 | 1.14 | 0.59 | 1700 | 1.40E-02 | 1.09 | 0.58 | 2034 |
| C18 | 3294629 | rs1791098 | C | 0.761 | 10634 | 1.11E-03 | 1.17 | 0.788 | 1700 | 1.78E-03 | 1.15 | 0.785 | 2034 |
| C07 | 150001841 | rs11762978 | A | 0.654 | 10634 | 1.11E-03 | 1.15 | 0.684 | 1700 | 8.03E-03 | 1.11 | 0.677 | 2034 |
| C02 | 209032070 | rs6435401 | A | 0.334 | 10625 | 1.13E-03 | 1.14 | 0.365 | 1699 | 3.65E-03 | 1.12 | 0.359 | 2033 |
| C02 | 85444821 | rs11675205 | A | 0.254 | 10635 | 1.13E-03 | 1.16 | 0.283 | 1700 | 1.59E-03 | 1.14 | 0.28 | 2034 |
| C05 | 158546004 | rs270654 | G | 0.133 | 10635 | 1.13E-03 | 1.2 | 0.156 | 1700 | 3.21E-03 | 1.17 | 0.152 | 2034 |
| C05 | 158546004 | rs270654 | G | 0.133 | 10635 | 1.13E-03 | 1.2 | 0.156 | 1700 | 3.21E-03 | 1.17 | 0.152 | 2034 |
| CX | 40429441 | rs3013140 | G | 0.931 | 8271 | 1.14E-03 | 1.4 | 0.95 | 1103 | 8.63E-04 | 1.38 | 0.949 | 1316 |
| C10 | 78560604 | rs697171 | G | 0.91 | 10620 | 1.14E-03 | 1.27 | 0.928 | 1697 | 1.04E-02 | 1.19 | 0.923 | 2030 |
| C04 | 134880774 | rs4864193 | C | 0.959 | 10626 | 1.14E-03 | 1.43 | 0.971 | 1700 | 4.87E-04 | 1.42 | 0.971 | 2034 |
| C12 | 119366443 | rs3742049 | C | 0.918 | 10622 | 1.14E-03 | 1.28 | 0.935 | 1699 | 3.44E-04 | 1.29 | 0.936 | 2033 |
| C22 | 32544493 | rs2267296 | G | 0.444 | 10634 | 1.15E-03 | 1.14 | 0.476 | 1700 | 2.30E-03 | 1.12 | 0.472 | 2034 |
| C12 | 9208670 | rs3782677 | C | 0.548 | 10635 | 1.15E-03 | 1.14 | 0.579 | 1700 | 8.35E-03 | 1.1 | 0.572 | 2034 |
| C18 | 39882041 | rs4340393 | T | 0.261 | 10635 | 1.15E-03 | 1.15 | 0.289 | 1700 | 2.80E-03 | 1.13 | 0.285 | 2034 |
| C06 | 162239920 | rs875785 | C | 0.916 | 10613 | 1.15E-03 | 1.28 | 0.933 | 1697 | 3.18E-04 | 1.29 | 0.934 | 2031 |
| C05 | 102428250 | rs3776859 | T | 0.709 | 10634 | 1.15E-03 | 1.15 | 0.738 | 1700 | 8.34E-03 | 1.11 | 0.731 | 2034 |
| C17 | 72288299 | rs191227 | A | 0.572 | 10632 | 1.15E-03 | 1.14 | 0.604 | 1700 | 6.91E-04 | 1.13 | 0.603 | 2033 |
| C07 | 38886642 | rs11763728 | T | 0.332 | 10635 | 1.15E-03 | 1.14 | 0.363 | 1700 | 1.57E-03 | 1.13 | 0.36 | 2034 |
| C05 | 157527972 | rs716376 | G | 0.182 | 10635 | 1.16E-03 | 1.17 | 0.208 | 1700 | 4.51E-03 | 1.14 | 0.203 | 2034 |
| C06 | 156331097 | rs4512236 | T | 0.418 | 10634 | 1.17E-03 | 1.14 | 0.45 | 1700 | 9.53E-04 | 1.13 | 0.448 | 2034 |
| C12 | 111845540 | rs1293758 | A | 0.407 | 10633 | 1.17E-03 | 1.14 | 0.439 | 1700 | 6.83E-04 | 1.13 | 0.438 | 2034 |
| C08 | 32600898 | rs10503927 | T | 0.471 | 10635 | 1.17E-03 | 1.14 | 0.503 | 1700 | 2.49E-03 | 1.12 | 0.498 | 2034 |
| C03 | 175270719 | rs2111956 | G | 0.758 | 10633 | 1.17E-03 | 1.17 | 0.785 | 1700 | 3.73E-02 | 1.09 | 0.774 | 2033 |
| C02 | 229956337 | rs9288639 | C | 0.076 | 10621 | 1.18E-03 | 1.26 | 0.093 | 1697 | 1.45E-02 | 1.18 | 0.088 | 2030 |
| C01 | 148893831 | rs2495392 | G | 0.274 | 10629 | 1.18E-03 | 1.15 | 0.303 | 1700 | 7.91E-03 | 1.11 | 0.296 | 2034 |
| C12 | 9208040 | rs3213831 | T | 0.547 | 10635 | 1.18E-03 | 1.14 | 0.579 | 1700 | 8.97E-03 | 1.1 | 0.571 | 2034 |
| C14 | 63868848 | rs1953416 | T | 0.482 | 10627 | 1.18E-03 | 1.14 | 0.514 | 1698 | 4.79E-03 | 1.11 | 0.508 | 2032 |
| C17 | 37372424 | rs7210156 | G | 0.93 | 10631 | 1.18E-03 | 1.31 | 0.946 | 1699 | 3.16E-03 | 1.25 | 0.943 | 2033 |
| C16 | 63942198 | rs7192142 | T | 0.811 | 10602 | 1.18E-03 | 1.18 | 0.835 | 1693 | 2.07E-03 | 1.16 | 0.833 | 2025 |
| C10 | 15465576 | rs1013406 | C | 0.561 | 10623 | 1.18E-03 | 1.14 | 0.593 | 1697 | 1.35E-02 | 1.1 | 0.583 | 2030 |
| C12 | 65812323 | rs11835794 | T | 0.852 | 10635 | 1.18E-03 | 1.21 | 0.874 | 1700 | 1.80E-04 | 1.22 | 0.876 | 2034 |
| C05 | 57500192 | rs2112905 | A | 0.402 | 10635 | 1.19E-03 | 1.14 | 0.433 | 1700 | 4.16E-03 | 1.11 | 0.427 | 2034 |
| C04 | 33363983 | rs10517277 | C | 0.102 | 10629 | 1.19E-03 | 1.22 | 0.122 | 1700 | 3.92E-03 | 1.18 | 0.118 | 2034 |
| C07 | 37924826 | rs1357648 | T | 0.318 | 10602 | 1.20E-03 | 1.14 | 0.348 | 1691 | 2.54E-03 | 1.12 | 0.344 | 2024 |
| C05 | 158795428 | rs10045431 | A | 0.262 | 10582 | 1.20E-03 | 1.15 | 0.291 | 1675 | 6.80E-03 | 1.12 | 0.285 | 2007 |
| C22 | 32522356 | rs8135273 | C | 0.156 | 10593 | 1.20E-03 | 1.19 | 0.18 | 1689 | 9.52E-03 | 1.14 | 0.174 | 2020 |
| C04 | 162467610 | rs12503470 | T | 0.378 | 10632 | 1.20E-03 | 1.14 | 0.409 | 1700 | 4.24E-03 | 1.11 | 0.403 | 2034 |
| C04 | 162156054 | rs17359034 | T | 0.386 | 10551 | 1.20E-03 | 1.14 | 0.417 | 1700 | 1.11E-02 | 1.1 | 0.409 | 2034 |
| C14 | 51013885 | rs1458114 | T | 0.708 | 10457 | 1.21E-03 | 1.16 | 0.737 | 1653 | 4.43E-03 | 1.12 | 0.732 | 1982 |
| C02 | 127990589 | rs3943703 | C | 0.211 | 10632 | 1.21E-03 | 1.16 | 0.237 | 1700 | 5.13E-04 | 1.16 | 0.237 | 2034 |
| C07 | 85202542 | rs10276025 | T | 0.372 | 10635 | 1.22E-03 | 1.14 | 0.403 | 1700 | 1.23E-03 | 1.13 | 0.401 | 2034 |
| C18 | 58474237 | rs7241455 | T | 0.061 | 10635 | 1.22E-03 | 1.28 | 0.077 | 1700 | 3.10E-04 | 1.29 | 0.078 | 2034 |
| C15 | 90437337 | rs3743369 | T | 0.61 | 10633 | 1.22E-03 | 1.14 | 0.641 | 1700 | 8.00E-04 | 1.13 | 0.639 | 2034 |
| C03 | 72701976 | rs2046867 | T | 0.276 | 10630 | 1.23E-03 | 1.15 | 0.305 | 1700 | 7.59E-03 | 1.11 | 0.298 | 2034 |
| C05 | 52576529 | rs38055 | T | 0.383 | 10632 | 1.23E-03 | 1.14 | 0.414 | 1700 | 3.56E-05 | 1.17 | 0.42 | 2034 |
| C08 | 70844508 | rs7015359 | G | 0.186 | 10635 | 1.23E-03 | 1.17 | 0.211 | 1700 | 1.45E-03 | 1.16 | 0.209 | 2034 |
| C17 | 17015262 | rs4401079 | T | 0.21 | 10418 | 1.23E-03 | 1.17 | 0.236 | 1666 | 3.95E-04 | 1.17 | 0.237 | 1992 |
| C10 | 23293068 | rs10828393 | C | 0.891 | 10615 | 1.23E-03 | 1.24 | 0.91 | 1698 | 3.82E-03 | 1.19 | 0.907 | 2032 |
| C14 | 57437666 | rs11158238 | T | 0.141 | 10634 | 1.24E-03 | 1.19 | 0.163 | 1700 | 3.43E-03 | 1.16 | 0.16 | 2034 |
| C08 | 97736809 | rs1075479 | A | 0.79 | 10601 | 1.24E-03 | 1.17 | 0.815 | 1696 | 7.77E-03 | 1.13 | 0.81 | 2029 |
| C07 | 21550808 | rs4722054 | G | 0.087 | 10596 | 1.25E-03 | 1.24 | 0.105 | 1682 | 1.77E-03 | 1.21 | 0.103 | 2014 |
| C22 | 34890870 | rs763086 | A | 0.299 | 10624 | 1.26E-03 | 1.15 | 0.329 | 1699 | 2.88E-03 | 1.12 | 0.324 | 2031 |
| C19 | 4187996 | rs4740 | A | 0.269 | 10634 | 1.26E-03 | 1.15 | 0.298 | 1700 | 5.17E-03 | 1.12 | 0.292 | 2034 |
| C12 | 75974496 | rs7135414 | A | 0.914 | 10635 | 1.26E-03 | 1.27 | 0.931 | 1700 | 5.96E-03 | 1.21 | 0.928 | 2034 |
| C12 | 75974496 | rs7135414 | A | 0.914 | 10635 | 1.26E-03 | 1.27 | 0.931 | 1700 | 5.96E-03 | 1.21 | 0.928 | 2034 |
| C05 | 5893171 | rs864267 | A | 0.452 | 10633 | 1.26E-03 | 1.14 | 0.483 | 1700 | 1.57E-03 | 1.12 | 0.48 | 2034 |
| C12 | 121504211 | rs10847227 | T | 0.273 | 10616 | 1.26E-03 | 1.15 | 0.301 | 1700 | 1.46E-04 | 1.16 | 0.304 | 2034 |
| C08 | 97440350 | rs1992087 | G | 0.254 | 10635 | 1.27E-03 | 1.15 | 0.282 | 1700 | 2.92E-03 | 1.13 | 0.278 | 2034 |
| C13 | 53306486 | rs1993776 | C | 0.869 | 10635 | 1.28E-03 | 1.22 | 0.889 | 1700 | 2.60E-03 | 1.18 | 0.887 | 2034 |
| C19 | 17080105 | rs7246865 | A | 0.205 | 10635 | 1.28E-03 | 1.17 | 0.231 | 1700 | 3.11E-03 | 1.14 | 0.227 | 2034 |
| C19 | 17080105 | rs7246865 | A | 0.205 | 10635 | 1.28E-03 | 1.17 | 0.231 | 1700 | 3.11E-03 | 1.14 | 0.227 | 2034 |
| C04 | 178163770 | rs309800 | C | 0.798 | 10632 | 1.28E-03 | 1.18 | 0.823 | 1699 | 1.30E-04 | 1.2 | 0.826 | 2033 |
| C05 | 57486156 | rs2962014 | G | 0.501 | 10528 | 1.28E-03 | 1.14 | 0.533 | 1682 | 4.01E-03 | 1.11 | 0.528 | 2015 |
| C19 | 14236895 | rs7248277 | C | 0.563 | 10624 | 1.29E-03 | 1.14 | 0.594 | 1699 | 5.08E-04 | 1.14 | 0.594 | 2033 |
| C01 | 30315299 | rs407838 | C | 0.843 | 10633 | 1.29E-03 | 1.2 | 0.865 | 1700 | 7.85E-04 | 1.19 | 0.865 | 2034 |
| C01 | 184669059 | rs2250227 | C | 0.337 | 10635 | 1.29E-03 | 1.14 | 0.367 | 1700 | 3.60E-03 | 1.12 | 0.362 | 2034 |
| C04 | 17186841 | rs2697684 | G | 0.771 | 10635 | 1.29E-03 | 1.17 | 0.798 | 1700 | 1.46E-03 | 1.15 | 0.795 | 2034 |
| C10 | 85007873 | rs1199316 | C | 0.713 | 10568 | 1.29E-03 | 1.15 | 0.742 | 1687 | 1.02E-04 | 1.17 | 0.745 | 2019 |
| CX | 84984650 | rs2213468 | G | 0.659 | 8219 | 1.30E-03 | 1.18 | 0.696 | 1094 | 2.51E-03 | 1.16 | 0.691 | 1307 |
| CX | 66115625 | rs2031751 | C | 0.941 | 8270 | 1.30E-03 | 1.44 | 0.959 | 1102 | 8.21E-03 | 1.31 | 0.955 | 1315 |
| C02 | 212342079 | rs4442936 | G | 0.282 | 10600 | 1.32E-03 | 1.15 | 0.311 | 1683 | 9.92E-04 | 1.14 | 0.309 | 2016 |
| C01 | 162568432 | rs2651860 | T | 0.786 | 10635 | 1.32E-03 | 1.17 | 0.811 | 1700 | 4.88E-03 | 1.14 | 0.806 | 2033 |
| C02 | 135143228 | rs10496721 | T | 0.17 | 10631 | 1.32E-03 | 1.18 | 0.195 | 1699 | 6.12E-03 | 1.14 | 0.189 | 2033 |
| C04 | 129841129 | rs6534683 | T | 0.646 | 10635 | 1.32E-03 | 1.14 | 0.676 | 1700 | 8.61E-04 | 1.14 | 0.675 | 2034 |
| C05 | 108055795 | rs4957775 | G | 0.832 | 10635 | 1.33E-03 | 1.19 | 0.855 | 1700 | 1.22E-03 | 1.18 | 0.853 | 2034 |
| C13 | 21281716 | rs1887263 | A | 0.204 | 10630 | 1.33E-03 | 1.16 | 0.229 | 1700 | 1.56E-03 | 1.15 | 0.227 | 2034 |
| C12 | 120804218 | rs1169081 | C | 0.692 | 10626 | 1.33E-03 | 1.15 | 0.721 | 1700 | 2.42E-02 | 1.09 | 0.711 | 2034 |
| C11 | 68636018 | rs7111999 | G | 0.47 | 10494 | 1.33E-03 | 1.14 | 0.502 | 1666 | 1.94E-02 | 1.09 | 0.492 | 1994 |
| C13 | 36900184 | rs9566343 | T | 0.814 | 10634 | 1.34E-03 | 1.18 | 0.838 | 1700 | 9.66E-04 | 1.17 | 0.837 | 2034 |
| C12 | 22807863 | rs16925384 | A | 0.194 | 10621 | 1.34E-03 . | 1.17 | 0.219 | 1699 | 1.79E-03 . | 1.15 | 0.216 | 2033 |
| C01 | 149009396 | rs949969 | T | 0.195 | 10635 | 1.34E-03 | 1.17 | 0.221 | 1700 | 2.26E-03 | 1.15 | 0.218 | 2034 |
| C14 | 64466293 | rs894921 | G | 0.377 | 10633 | 1.35E-03 | 1.14 | 0.408 | 1700 | 1.12E-02 | 1.1 | 0.4 | 2034 |
| C10 | 15419285 | rs11596590 | T | 0.559 | 10634 | 1.35E-03 | 1.14 | 0.59 | 1699 | 1.36E-02 | 1.1 | 0.581 | 2033 |
| C11 | 90747051 | rs1436622 | A | 0.686 | 10633 | 1.35E-03 | 1.15 | 0.715 | 1700 | 5.93E-04 | 1.15 | 0.715 | 2034 |
| C08 | 129199390 | rs10505507 | A | 0.924 | 10634 | 1.36E-03 | 1.29 | 0.94 | 1700 | 5.90E-03 | 1.22 | 0.937 | 2034 |
| C18 | 45713028 | rs1790778 | G | 0.512 | 10632 | 1.36E-03 | 1.13 | 0.544 | 1699 | 1.91E-03 | 1.12 | 0.54 | 2033 |
| C07 | 85200311 | rs2373018 | G | 0.372 | 10635. | 1.36E-03 | 1.14 | 0.403 | 1700 | 1.35E-03 | 1.13 | 0.401 | 2034 |
| C14 | 57397846 | rs10483707 | A | 0.139 | 10635 | 1.36E-03 | 1.19 | 0.161 | 1700 | 5.05E-03 | 1.15 | 0.157 | 2034 |
| C20 | 5929566 | rs236113 | T | 0.883 | 10635 | 1.37E-03 | 1.23 | 0.902 | 1700 | 3.33E-03 | 1.19 | 0.899 | 2034 |
| C07 | 11501113 | rs1526543 | T | 0.829 | 10634 | 1.37E-03 | 1.19 | 0.852 | 1700 | 3.94E-03 | 1.15 | 0.849 | 2034 |
| C05 | 5904611 | rs2617549 | A | 0.453 | 10629 | 1.38E-03 | 1.13 | 0.485 | 1700 | 1.57E-03 | 1.12 | 0.482 | 2034 |
| C02 | 36022756 | rs6747236 | T | 0.716 | 10628 | 1.38E-03 | 1.15 | 0.744 | 1700 | 4.89E-04 | 1.15 | 0.744 | 2034 |
| C10 | 8304796 | rs7092384 | C | 0.86 | 10629 | 1.39E-03 | 1.21 | 0.881 | 1700 | 1.31E-03 | 1.19 | 0.88 | 2034 |
| C02 | 42505686 | rs6724757 | A | 0.09 | 10631 | 1.40E-03 | 1.23 | 0.109 | 1699 | 3.19E-04 | 1.24 | 0.109 | 2033 |
| C05 | 66155122 | rs2441109 | G | 0.162 | 10633 | 1.40E-03 | 1.18 | 0.186 | 1700 | 1.58E-02 | 1.12 | 0.179 | 2034 |
| C10 | 131748550 | rs1484652 | T | 0.905 | 10635 | 1.40E-03 | 1.26 | 0.923 | 1700 | 8.26E-03 | 1.19 | 0.919 | 2034 |
| C16 | 2010569 | rs2286472 | C | 0.269 | 10627 | 1.40E-03 | 1.15 | 0.297 | 1690 | 2.09E-03 | 1.13 | 0.294 | 2024 |
| C05 | 39122462 | rs6880882 | A | 0.352 | 10599 | 1.41E-03 | 1.14 | 0.382 | 1697 | 3.56E-03 | 1.12 | 0.377 | 2028 |
| C16 | 88204775 | rs4075964 | A | 0.284 | 10632 | 1.41E-03 | 1.15 | 0.312 | 1699 | 1.71E-03 | 1.13 | 0.31 | 2033 |
| C09 | 28113402 | rs1452335 | G | 0.715 | 10614 | 1.41E-03 | 1.15 | 0.744 | 1694 | 6.47E-04 | 1.15 | 0.743 | 2027 |
| C14 | 31305463 | rs1950216 | T | 0.798 | 10634 | 1.41E-03 | 1.18 | 0.823 | 1700 | 3.06E-03 | 1.15 | 0.82 | 2034 |
| C13 | 45766462 | rs9526329 | G | 0.921 | 10632 | 1.41E-03 | 1.28 | 0.938 | 1700 | 3.25E-03 | 1.23 | 0.935 | 2034 |
| C11 | 14150002 | rs10832223 | T | 0.837 | 10626 | 1.41E-03 | 1.19 | 0.86 | 1700 | 2.90E-03 | 1.16 | 0.857 | 2034 |
| C02 | 77672532 | rs2861078 | T | 0.57 | 10635 | 1.42E-03 | 1.14 | 0.601 | 1700 | 1.64E-03 | 1.12 | 0.599 | 2034 |
| C01 | 62762582 | rs11208061 | A | 0.345 | 10547 | 1.42E-03 | 1.14 | 0.375 | 1697 | 3.56E-03 | 1.12 | 0.37 | 2029 |
| C20 | 49529811 | rs4811008 | G | 0.461 | 10463 | 1.42E-03 | 1.14 | 0.493 | 1668 | 3.89E-03 | 1.11 | 0.488 | 1996 |
| C14 | 102711567 | rs8016595 | C | 0.754 | 10630 | 1.43E-03 | 1.16 | 0.781 | 1700 | 4.46E-03 | 1.13 | 0.776 | 2034 |
| C01 | 243972662 | rs12068067 | G | 0.776 | 10589 | 1.43E-03 | 1.17 | 0.801 | 1695 | 9.78E-04 | 1.16 | 0.8 | 2029 |
| C10 | 84836912 | rs10787854 | A | 0.83 | 10634 | 1.43E-03 | 1.19 | 0.853 | 1700 | 2.82E-04 | 1.2 | 0.854 | 2034 |
| CX | 55725022 | rs5914778 | G | 0.802 | 8269 | 1.44E-03 | 1.22 | 0.832 | 1103 | 6.95E-04 | 1.22 | 0.831 | 1316 |
| C05 | 102584028 | rs183752 | T | 0.716 | 10621 | 1.44E-03 | 1.15 | 0.744 | 1699 | 1.26E-02 | 1.11 | 0.736 | 2033 |
| C14 | 31304803 | rs991387 | G | 0.799 | 10634 | 1.44E-03 | 1.18 | 0.823 | 1700 | 3.12E-03 | 1.15 | 0.82 | 2034 |
| C11 | 68632276 | rs1005858 | G | 0.47 | 10634 | 1.45E-03 | 1.13 | 0.502 | 1700 | 2.30E-02 | 1.09 | 0.491 | 2034 |
| C03 | 195828700 | rs4497978 | A | 0.667 | 10576 | 1.45E-03 | 1.15 | 0.696 | 1682 | 2.58E-03 | 1.13 | 0.693 | 2012 |
| C05 | 40656007 | rs13175020 | T | 0.816 | 10632 | 1.46E-03 | 1.18 | 0.84 | 1699 | 4.64E-04 | 1.19 | 0.841 | 2033 |
| C01 | 62822706 | rs6696619 | C | 0.662 | 10624 | 1.46E-03 | 1.14 | 0.692 | 1700 | 6.63E-04 | 1.14 | 0.691 | 2034 |
| C13 | 104637486 | rs354438 | T | 0.351 | 10634 | 1.46E-03 | 1.14 | 0.381 | 1700 | 5.60E-04 | 1.14 | 0.381 | 2034 |
| C14 | 64464393 | rs1147437 | G | 0.38 | 10634 | 1.46E-03 | 1.14 | 0.411 | 1700 | 8.57E-03 | 1.1 | 0.403 | 2034 |
| C17 | 295873 | rs4130140 | A | 0.175 | 10635 | 1.46E-03 | 1.17 | 0.199 | 1700 | 4.28E-03 | 1.14 | 0.195 | 2034 |
| C07 | 20937657 | rs2110007 | C | 0.943 | 10606 | 1.47E-03 | 1.34 | 0.956 | 1699 | . 1.90E-03 | 1.3 | 0.955 | 2030 |
| C21 | 26986173 | rs2251540 | G | 0.642 | 10633 | 1.47E-03 | 1.14 | 0.672 | 1700 | 7.17E-04 | 1.14 | 0.671 | 2034 |
| C10 | 23311212 | rs993352 | G | 0.883 | 10593 | 1.47E-03 | 1.23 | 0.903 | 1689 | 4.02E-03 | 1.19 | 0.9 | 2021 |
| C04 | 139831081 | rs7669668 | T | 0.119 | 10635 | 1.48E-03 | 1.2 | 0.139 | 1700 | 1.69E-02 | 1.14 | 0.133 | 2034 |
| C02 | 172634850 | rs1399959 | G | 0.669 | 10611 | 1.48E-03 | 1.14 | 0.698 | 1699 | 1.32E-02 | 1.1 | 0.69 | 2032 |
| C06 | 127842427 | rs11154416 | G | 0.76 | 10566 | 1.49E-03 | 1.16 | 0.787 | 1684 | 5.06E-03 | 1.13 | 0.782 | 2016 |
| C12 | 22797592 | rs2171392 | A | 0.194 | 10635 | 1.49E-03 | 1.17 | 0.219 | 1700 | 2.03E-03 | 1.15 | 0.217 | 2034 |
| C07 | 17747986 | rs11766489 | T | 0.423 | 10635 | 1.49E-03 | 1.13 | 0.454 | 1700 | 1.74E-03 | 1.12 | 0.451 | 2034 |
| C20 | 4116799 | rs6084644 | G | 0.436 | 10635 | 1.49E-03 | 1.13 | 0.467 | 1700 | 2.33E-03 | 1.12 | 0.464 | 2034 |
| C20 | 4116799 | rs6084644 | G | 0.436 | 10635 | 1.49E-03 | 1.13 | 0.467 | 1700 | 2.33E-03 | 1.12 | 0.464 | 2034 |
| C18 | 70239058 | rs4892210 | A | 0.192 | 10634 | 1.49E-03 | 1.17 | 0.217 | 1700 | 8.76E-04 | 1.16 | 0.216 | 2034 |
| C01 | 153057498 | rs822519 | A | 0.932 | 10635 | 1.50E-03 | 1.3 | 0.947 | 1700 | 8.24E-04 | 1.3 | 0.946 | 2034 |
| C10 | 5181871 | rs4880711 | C | 0.35 | 10503 | 1.51E-03 | 1.14 | 0.38 | 1693 | 2.56E-02 | 1.09 | 0.37 | 2017 |
| C14 | 101394200 | rs10129841 | A | 0.577 | 10634 | 1.51E-03 | 1.14 | 0.608 | 1700 | 4.86E-03 | 1.11 | 0.603 | 2034 |
| C07 | 38982261 | rs4478468 | G | 0.31 | 10635 | 1.52E-03 | 1.14 | 0.339 | 1700 | 1.46E-03 | 1.13 | 0.337 | 2034 |
| C08 | 39425796 | rs7830101 | T | 0.274 | 10556 | 1.53E-03 | 1.15 | 0.302 | 1686 | 2.51E-03 | 1.13 | 0.299 | 2019 |
| C16 | 19860174 | rs1634675 | G | 0.787 | 10633 | 1.53E-03 | 1.17 | 0.812 | 1700 | 6.40E-03 | 1.13 | 0.807 | 2034 |
| C08 | 75342454 | rs6996971 | C | 0.476 | 10603 | 1.53E-03 | 1.13 | 0.507 | 1698 | 2.99E-04 | 1.14 | 0.509 | 2032 |
| C05 | 102596616 | rs26821 | A | 0.709 | 10628 | 1.53E-03 | 1.15 | 0.737 | 1697 | 1.27E-02 | 1.11 | 0.73 | 2031 |
| C19 | 330003 | rs10414677 | T | 0.276 | 10341 | 1.53E-03 | 1.15 | 0.305 | 1639 | 1.23E-02 | 1.11 | 0.297 | 1956 |
| C14 | 64458802 | rs1147443 | T | 0.254 | 10635 | 1.54E-03 | 1.15 | 0.281 | 1700 | 2.45E-03 | 1.13 | 0.278 | 2034 |
| C11 | 108465132 | rs1509729 | G | 0.688 | 10634 | 1.55E-03 | 1.15 | 0.717 | 1700 | 9.48E-03 | 1.11 | 0.71 | 2034 |
| C05 | 176640493 | rs7724098 | G | 0.289 | 10627 | 1.55E-03 | 1.14 | 0.317 | 1697 | 2.16E-03 | 1.13 | 0.314 | 2031 |
| C03 | 54346463 | rs1530733 | A | 0.156 | 10635 | 1.55E-03 | 1.18 | 0.179 | 1700 | 3.48E-03 | 1.15 | 0.176 | 2034 |
| C16 | 88263882 | rs904800 | G | 0.38 | 10635 | 1.55E-03 | 1.14 | 0.411 | 1700 | 5.04E-03 | 1.11 | 0.405 | 2034 |
| C22 | 34142107 | rs2071744 | C | 0.78 | 10607 | 1.55E-03 | 1.17 | 0.806 | 1700 | 5.19E-03 | 1.13 | 0.801 | 2033 |
| C07 | 136071365 | rs324594 | C | 0.27 | 10632 | 1.55E-03 | 1.15 | 0.298 | 1700 | 7.92E-04 | 1.14 | 0.297 | 2034 |
| C07 | 102722901 | rs10282605 | C | 0.187 | 10633 | 1.55E-03 | 1.17 | 0.211 | 1700 | 9.25E-03 | 1.13 | 0.206 | 2034 |
| C04 | 37546025 | rs3849018 | T | 0.279 | 10635 | 1.55E-03 | 1.15 | 0.307 | 1700 | 2.28E-03 | 1.13 | 0.304 | 2033 |
| C05 | 97972689 | rs168654 | T | 0.636 | 10630 | 1.56E-03 | 1.14 | 0.665 | 1699 | 5.48E-03 | 1.11 | 0.66 | 2033 |
| C06 | 28156603 | rs203877 | T | 0.729 | 10461 | 1.56E-03 | 1.16 | 0.757 | 1666 | 1.78E-02 | 1.1 | 0.748 | 1992 |
| C05 | 76862558 | rs251470 | T | 0.439 | 10634 | 1.56E-03 | 1.13 | 0.47 | 1700 | 6.26E-03 | 1.11 | 0.464 | 2034 |
| C09 | 122155539 | rs3780680 | T | 0.452 | 10610 | 1.56E-03 | 1.13 | 0.483 | 1698 | 2.92E-02 | 1.08 | 0.472 | 2031 |
| C04 | 10657250 | rs10011464 | T | 0.462 | 10581 | 1.56E-03 | 1.13 | 0.493 | 1685 | 2.56E-03 | 1.12 | 0.49 | 2016 |
| C08 | 143147459 | rs11167132 | T | 0.218 | 10568 | 1.57E-03 | 1.16 | 0.244 | 1693 | 3.04E-03 | 1.14 | 0.241 | 2027 |
| CX | 66121372 | rs1576059 | T | 0.941 | 8273 | 1.57E-03 | 1.43 | 0.958 | 1103 | 9.27E-03 | 1.31 | 0.954 | 1316 |
| C16 | 82550760 | rs10514560 | C | 0.732 | 10632 | 1.57E-03 | 1.15 | 0.759 | 1700 | 2.17E-03 | 1.14 | 0.756 | 2034 |
| C20 | 57601797 | rs486921 | C | 0.853 | 10634 | 1.58E-03 | 1.2 | 0.875 | 1699 | 7.96E-04 | 1.2 | 0.875 | 2033 |
| C17 | 14301595 | rs2856177 | A | 0.622 | 10630 | 1.59E-03 | 1.14 | 0.652 | 1700 | 6.13E-03 | 1.11 | 0.647 | 2034 |
| C01 | 63947687 | rs2806535 | A | 0.487 | 10630 | 1.59E-03 | 1.13 | 0.519 | 1700 | 2.11E-03 | 1.12 | 0.515 | 2034 |
| C22 | 18426996 | rs3804044 | T | 0.858 | 10612 | 1.59E-03 | 1.2 | 0.879 | 1700 | 4.62E-03 | 1.17 | 0.876 | 2034 |
| C19 | 50054507 | rs377702 | T | 0.384 | 10629 | 1.61E-03 | 1.14 | 0.415 | 1700 | 1.75E-03 | 1.12 | 0.412 | 2034 |
| C18 | 55144365 | rs4806 | C | 0.284 | 10592 | 1.61E-03 | 1.15 | 0.312 | 1686 | 4.58E-03 | 1.12 | 0.307 | 2020 |
| C08 | 75320456 | rs4410896 | A | 0.479 | 10635 | 1.61E-03 | 1.13 | 0.51 | 1700 | 4.67E-04 | 1.14 | 0.511 | 2034 |
| C13 | 43040379 | rs963569 | A | 0.748 | 10635 | 1.61E-03 | 1.16 | 0.775 | 1700 | 4.72E-02 | 1.09 | 0.764 | 2034 |
| CX | 69317680 | rs2341629 | C | 0.467 | 8261 | 1.62E-03 | 1.16 | 0.505 | 1103 | 1.66E-03 | 1.15 | 0.502 | 1316 |
| C11 | 17901963 | rs2237921 | C | 0.156 | 10634 | 1.62E-03 | 1.18 | 0.179 | 1700 | 6.00E-03 | 1.14 | 0.174 | 2034 |
| C10 | 84972068 | rs11199158 | G | 0.608 | 10635 | 1.62E-03 | 1.14 | 0.638 | 1700 | 2.26E-05 | 1.17 | 0.645 | 2034 |
| C11 | 1390257 | rs4963076 | T | 0.482 | 10635 | 1.62E-03 | 1.13 | 0.513 | 1700 | 3.68E-03 | 1.11 | 0.508 | 2034 |
| C11 | 1390257 | rs4963076 | T | 0.482 | 10635 | 1.62E-03 | 1.13 | 0.513 | 1700 | 3.68E-03 | 1.11 | 0.508 | 2034 |
| C15 | 52585989 | rs2553223 | G | 0.346 | 10626 | 1.62E-03 | 1.14 | 0.376 | 1700 | 6.64E-04 | 1.14 | 0.376 | 2034 |
| C08 | 32791061 | rs16880128 | G | 0.446 | 10541 | 1.63E-03 | 1.13 | 0.477 | 1693 | 9.35E-03 | 1.1 | 0.47 | 2026 |
| C08 | 96746644 | rs3134517 | G | 0.64 | 10632 | 1.63E-03 | 1.14 | 0.67 | 1700 | 7.83E-03 | 1.11 | 0.663 | 2033 |
| C01 | 55121739 | rs4927219 | G | 0.601 | 10635 | 1.63E-03 | 1.14 | 0.631 | 1700 | 3.78E-04 | 1.14 | 0.632 | 2034 |
| C14 | 19276803 | rs10872856 | C | 0.912 | 10635 | 1.63E-03 | 1.26 | 0.929 | 1700 | 1.56E-02 | 1.18 | 0.924 | 2034 |
| C01 | 148792338 | rs1970568 | T | 0.813 | 10634 | 1.64E-03 | 1.18 | 0.837 | 1700 | 2.08E-03 | 1.16 | 0.835 | 2034 |
| C12 | 20945636 | rs3764006 | C | 0.104 | 10626 | 1.64E-03 | 1.21 | 0.123 | 1700 | 8.33 E-04 | 1.21 | 0.123 | 2034 |
| C18 | 45732885 | rs1557359 | G | 0.407 | 10635 | 1.64E-03 | 1.13 | 0.437 | 1700 | 1.64E-03 | 1.12 | 0.435 | 2034 |
| C17 | 14260485 | rs2323095 | A | 0.663 | 10635 | 1.64E-03 | 1.14 | 0.692 | 1700 | 3.76E-03 | 1.12 | 0.688 | 2034 |
| C08 | 96745945 | rs3102526 | G | 0.64 | 10635 | 1.65E-03 | 1.14 | 0.669 | 1700 | 7.97E-03 | 1.11 | 0.663 | 2034 |
| C04 | 96606254 | rs6850673 | C | 0.141 | 10631 | 1.65E-03 | 1.19 | 0.163 | 1700 | 2.15E-03 | 1.17 | 0.161 | 2034 |
| C22 | 18449129 | rs2073778 | C | 0.858 | 10635 | 1.65E-03 | 1.2 | 0.879 | 1700 | 5.41E-03 | 1.16 | 0.875 | 2034 |
| C07 | 116742325 | rs2027945 | A | 0.19 | 10631 | 1.66E-03 | 1.17 | 0.215 | 1700 | 1.06E-03 | 1.16 | 0.214 | 2034 |
| C07 | 144298585 | rs7791708 | A | 0.677 | 10614 | 1.66E-03 | 1.14 | 0.706 | 1693 | 4.26E-03 | 1.12 | 0.701 | 2027 |
| C05 | 3977821 | rs11956711 | C | 0.066 | 10608 | 1.66E-03 | 1.26 | 0.082 | 1698 | 4.36E-03 | 1.22 | 0.08 | 2031 |
| C05 | 39071087 | rs9292729 | C | 0.338 | 10625 | 1.66E-03 | 1.14 | 0.368 | 1700 | 6.86E-03 | 1.11 | 0.362 | 2034 |
| C01 | 29291511 | rs10799128 | T | 0.196 | 10601 | 1.66E-03 | 1.16 | 0.221 | 1686 | 3.50E-03 | 1.14 | 0.217 | 2020 |
| C05 | 3595286 | rs1697997 | A | 0.914 | 10635 | 1.66E-03 | 1.26 | 0.931 | 1700 | 5.14E-03 | 1.21 | 0.928 | 2034 |
| C02 | 215347262 | rs10932529 | A | 0.247 | 10635 | 1.67E-03 | 1.15 | 0.274 | 1700 | 1.88E-03 | 1.14 | 0.272 | 2034 |
| CX | 6130802 | rs7892324 | C | 0.165 | 8255 | 1.67E-03 | 1.22 | 0.194 | 1093 | 3.38E-03 | 1.19 | 0.19 | 1306 |
| C12 | 62930978 | rs10878126 | G | 0.493 | 10634 | 1.67E-03 | 1.13 | 0.524 | 1700 | 6.47E-04 | 1.13 | 0.525 | 2034 |
| C04 | 44935711 | rs6447404 | G | 0.617 | 10633 | 1.67E-03 | 1.14 | 0.647 | 1700 | 1.07E-02 | 1.1 | 0.639 | 2034 |
| C03 | 145977000 | rs10935523 | A | 0.741 | 10632 | 1.68E-03 | 1.16 | 0.767 | 1700 | 7.37E-03 | 1.12 | 0.762 | 2034 |
| C16 | 79586916 | rs7188879 | T | 0.964 | 10629 | 1.69E-03 | 1.45 | 0.975 | 1699 | 7.87E-03 | 1.33 | 0.972 | 2033 |
| C05 | 159076573 | rs889053 | T | 0.162 | 10634 | 1.69E-03 | 1.18 | 0.185 | 1700 | 3.69E-03 | 1.15 | 0.182 | 2034 |
| C11 | 91175735 | rs808003 | T | 0.554 | 10634 | 1.69E-03 | 1.13 | 0.585 | 1700 | 2.62E-02 | 1.09 | 0.574 | 2034 |
| C17 | 14258654 | rs12449610 | T | 0.669 | 10635 | 1.69E-03 | 1.14 | 0.697 | 1700 | 3.74E-03 | 1.12 | 0.693 | 2034 |
| C04 | 3811449 | rs6822427 | A | 0.487 | 10635 | 1.70E-03 | 1.13 | 0.518 | 1700 | 2.14E-03 | 1.12 | 0.515 | 2034 |
| C01 | 218934430 | rs1519463 | G | 0.5 | 10635 | 1.70E-03 | 1.13 | 0.531 | 1700 | 4.65E-04 | 1.14 | 0.532 | 2034 |
| C02 | 183549717 | rs4666836 | T | 0.22 | 10633 | 1.70E-03 | 1.16 | 0.246 | 1699 | 3.72E-03 | 1.13 | 0.242 | 2033 |
| C03 | 62438401 | rs1452075 | T | 0.734 | 10549 | 1.70E-03 | 1.15 | 0.761 | 1692 | 5.65E-03 | 1.12 | 0.756 | 2019 |
| C03 | 62438401 | rs1452075 | T | 0.734 | 10549 | 1.70E-03 | 1.15 | 0.761 | 1692 | 5.65E-03 | 1.12 | 0.756 | 2019 |
| C10 | 15387774 | rs2138930 | T | 0.11 | 10620 | 1.71E-03 | 1.21 | 0.129 | 1700 | 8.36E-03 | 1.16 | 0.125 | 2033 |
| C12 | 6177827 | rs797773 | T | 0.394 | 10607 | 1.71E-03 | 1.13 | 0.425 | 1698 | 2.20E-03 | 1.12 | 0.422 | 2031 |
| C12 | 52152886 | rs784563 | G | 0.441 | 10631 | 1.71E-03 | 1.13 | 0.472 | 1700 | 1.70E-03 | 1.12 | 0.47 | 2034 |
| C01 | 113143551 | rs773588 | C | 0.276 | 10632 | 1.71E-03 | 1.15 | 0.304 | 1700 | 2.18E-04 | 1.16 | 0.307 | 2034 |
| C05 | 66177928 | rs39700 | G | 0.374 | 10631 | 1.72E-03 | 1.13 | 0.404 | 1699 | 1.04E-02 | 1.1 | 0.396 | 2033 |
| C11 | 132266819 | rs1395504 | A | 0.493 | 10634 | 1.72E-03 | 1.13 | 0.524 | 1700 | 3.66E-03 | 1.11 | 0.519 | 2034 |
| C18 | 70236272 | rs1365254 | T | 0.192 | 10634 | 1.73E-03 | 1.16 | 0.217 | 1700 | 1.01E-03 | 1.16 | 0.216 | 2034 |
| C05 | 38927129 | rs525735 | C | 0.774 | 10632 | 1.74E-03 | 1.16 | 0.799 | 1699 | 1.06E-02 | 1.12 | 0.793 | 2033 |
| C08 | 75357793 | rs10283076 | G | 0.477 | 10627 | 1.74E-03 | 1.13 | 0.507 | 1698 | 3.56E-04 | 1.14 | 0.509 | 2032 |
| C10 | 108671040 | rs1337430 | A | 0.788 | 10215 | 1.74E-03 | 1.17 | 0.813 | 1685 | 9.30E-03 | 1.13 | 0.807 | 2014 |
| C18 | 55191365 | rs9319948 | A | 0.283 | 10629 | 1.75E-03 | 1.14 | 0.311 | 1700 | 4.57E-03 | 1.12 | 0.306 | 2034 |
| C15 | 30230982 | rs4779984 | G | 0.1 | 10616 | 1.75E-03 | 1.22 | 0.119 | 1698 | 9.51E-04 | 1.21 | 0.119 | 2032 |
| C10 | 33672138 | rs2804453 | G | 0.111 | 10621 | 1.76E-03 | 1.21 | 0.131 | 1697 | 8.26E-03 | 1.16 | 0.126 | 2030 |
| C21 | 40710416 | rs1882757 | A | 0.506 | 10635 | 1.76E-03 | 1.13 | 0.536 | 1700 | 2.56E-03 | 1.12 | 0.533 | 2034 |
| C06 | 39827103 | rs9471170 | G | 0.885 | 10635 | 1.77E-03 | 1.22 | 0.904 | 1700 | 1.10E-02 | 1.16 | 0.899 | 2034 |
| C09 | 28154645 | rs2044961 | G | 0.571 | 10633 | 1.77E-03 | 1.13 | 0.601 | 1700 | 7.85E-04 | 1.13 | 0.601 | 2034 |
| C10 | 94841520 | rs10509657 | C | 0.099 | 10631 | 1.78E-03 | 1.22 | 0.118 | 1700 | 3.06E-03 | 1.19 | 0.115 | 2034 |
| C12 | 101677726 | rs2247919 | T | 0.738 | 10635 | 1.79E-03 | 1.15 | 0.764 | 1700 | 6.44E-03 | 1.12 | 0.759 | 2034 |
| C02 | 11287711 | rs7608117 | T | 0.486 | 10629 | 1.79E-03 | 1.13 | 0.517 | 1698 | 4.79E-03 | 1.11 | 0.512 | 2031 |
| C01 | 31037636 | rs12126748 | A | 0.345 | 10489 | 1.79E-03 | 1.14 | 0.375 | 1665 | 5.15E-03 | 1.11 | 0.37 | 1995 |
| C03 | 63602103 | rs7653410 | C | 0.319 | 10620 | 1.79E-03 | 1.14 | 0.348 | 1700 | 1.04E-02 | 1.1 | 0.341 | 2034 |
| C17 | 70788248 | rs2041088 | A | 0.719 | 10633 | 1.79E-03 | 1.15 | 0.747 | 1700 | 3.91E-03 | 1.13 | 0.743 | 2034 |
| C06 | 36751662 | rs1864750 | G | 0.248 | 10635 | 1.80E-03 | 1.15 | 0.275 | 1700 | 2.56E-04 | 1.16 | 0.277 | 2034 |
| C11 | 12971706 | rs3935878 | T | 0.738 | 10634 | 1.80E-03 | 1.15 | 0.764 | 1700 | 5.90E-03 | 1.12 | 0.76 | 2034 |
| C12 | 67493429 | rs1470383 | C | 0.143 | 10634 | 1.81E-03 | 1.18 | 0.165 | 1700 | 3.15E-03 | 1.16 | 0.162 | 2034 |
| C05 | 56037706 | rs7720499 | T | 0.112 | 10635 | 1.81E-03 | 1.2 | 0.132 | 1700 | 3.98E-03 | 1.17 | 0.129 | 2033 |
| C02 | 172714469 | rs951917 | T | 0.387 | 10634 | 1.83E-03 | 1.13 | 0.417 | 1700 | 1.00E-02 | 1.1 | 0.41 | 2034 |
| C03 | 6764265 | rs12491369 | G | 0.571 | 10634 | 1.83E-03 | 1.13 | 0.601 | 1700 | 9.89E-04 | 1.13 | 0.601 | 2034 |
| C06 | 79771759 | rs9352688 | A | 0.623 | 10384 | 1.83E-03 | 1.14 | 0.653 | 1662 | 2.17E-03 | 1.12 | 0.65 | 1991 |
| C19 | 446722 | rs758503 | T | 0.294 | 10524 | 1.83E-03 | 1.14 | 0.323 | 1686 | 3.19E-03 | 1.12 | 0.319 | 2018 |
| C03 | 74187210 | rs4234186 | A | 0.943 | 10617 | 1.83E-03 | 1.33 | 0.957 | 1697 | 8.06E-03 | 1.25 | 0.954 | 2031 |
| C07 | 16590961 | rs706076 | G | 0.146 | 10619 | 1.83E-03 | 1.18 | 0.169 | 1700 | 1.53E-02 | 1.13 | 0.162 | 2034 |
| C02 | 76317619 | rs7563058 | C | 0.504 | 10635 | 1.84E-03 | 1.13 | 0.535 | 1700 | 2.64E-03 | 1.12 | 0.531 | 2034 |
| C04 | 175513600 | rs4695894 | G | 0.5 | 10632 | 1.84E-03 | 1.13 | 0.53 | 1700 | 2.89E-03 | 1.11 | 0.527 | 2034 |
| C17 | 18352177 | rs2746025 | G | 0.641 | 10635 | 1.84E-03 | 1.14 | 0.671 | 1700 | 2.07E-03 | 1.13 | 0.668 | 2034 |
| C07 | 28337194 | rs10486588 | G | 0.231 | 10616 | 1.85E-03 | 1.15 | 0.258 | 1694 | 3.86E-03 | 1.13 | 0.254 | 2028 |
| C06 | 39793734 | rs9296304 | A | 0.498 | 10635 | 1.85E-03 | 1.13 | 0.529 | 1700 | 9.98E-03 | 1.1 | 0.521 | 2034 |
| C13 | 20599475 | rs4770252 | A | 0.802 | 10626 | 1.86E-03 | 1.17 | 0.826 | 1699 | 4.75E-04 | 1.18 | 0.827 | 2032 |
| C04 | 178964553 | rs1983130 | G | 0.414 | 10625 | 1.86E-03 | 1.13 | 0.444 | 1699 | 3.12E-03 | 1.12 | 0.441 | 2033 |
| C02 | 39306628 | rs2168043 | C | 0.868 | 10635 | 1.86E-03 | 1.21 | 0.888 | 1700 | 2.99E-03 | 1.18 | 0.886 | 2034 |
| C06 | 105791384 | rs1149305 | T | 0.059 | 10544 | 1.87E-03 | 1.28 | 0.074 | 1695 | 1.49E-03 | 1.26 | 0.074 | 2027 |
| C22 | 18432012 | rs5992507 | A | 0.858 | 10633 | 1.87E-03 | 1.2 | 0.879 | 1700 | 5.40E-03 | 1.16 | 0.876 | 2034 |
| C13 | 53208642 | rs2050972 | C | 0.878 | 10635 | 1.87E-03 | 1.22 | 0.897 | 1700 | 6.10E-03 | 1.17 | 0.894 | 2034 |
| C03 | 66633156 | rs1563545 | G | 0.523 | 10562 | 1.87E-03 | 1.13 | 0.553 | 1681 | 2.31E-03 | 1.12 | 0.55 | 2014 |
| C03 | 7494813 | rs752299 | T | 0.685 | 10633 | 1.87E-03 | 1.14 | 0.714 | 1700 | 3.80E-03 | 1.12 | 0.71 | 2034 |
| C01 | 173267354 | rs12033847 | T | 0.462 | 10416 | 1.88E-03 | 1.13 | 0.493 | 1668 | 2.13E-03 | 1.12 | 0.49 | 1988 |
| C07 | 147146014 | rs10271435 | A | 0.06 | 10631 | 1.88E-03 | 1.27 | 0.076 | 1700 | 2.74E-04 | 1.3 | 0.077 | 2034 |
| C03 | 29295798 | rs13079598 | A | 0.905 | 10635 | 1.88E-03 | 1.25 | 0.923 | 1700 | 5.92E-03 | 1.2 | 0.92 | 2034 |
| CX | 143133206 | rs1094472 | G | 0.787 | 8262 | 1.89E-03 | 1.21 | 0.817 | 1098 | 3.45E-03 | 1.18 | 0.814 | 1311 |
| C07 | 116708662 | rs2237724 | A | 0.19 | 10635 | 1.90E-03 | 1.16 | 0.215 | 1700 | 1.21E-03 | 1.16 | 0.214 | 2034 |
| C07 | 116694598 | rs10487368 | T | 0.19 | 10635 | 1.90E-03 | 1.16 | 0.215 | 1700 | 1.21E-03 | 1.16 | 0.214 | 2034 |
| C07 | 116702050 | rs2237723 | T | 0.193 | 10630 | 1.90E-03 | 1.16 | 0.217 | 1700 | 1.05E-03 | 1.16 | 0.217 | 2034 |
| C02 | 173071729 | rs6718013 | G | 0.538 | 10634 | 1.91E-03 | 1.13 | 0.568 | 1700 | 1.45E-02 | 1.09 | 0.56 | 2034 |
| C03 | 7486375 | rs779710 | A | 0.589 | 10634 | 1.91E-03 | 1.13 | 0.619 | 1700 | 1.47E-03 | 1.13 | 0.617 | 2034 |
| C07 | 38853360 | rs1525798 | G | 0.303 | 10635 | 1.91E-03 | 1.14 | 0.331 | 1700 | 1.23E-03 | 1.13 | 0.33 | 2034 |
| C18 | 53741253 | rs4940582 | G | 0.849 | 10631 | 1.91E-03 | 1.19 | 0.871 | 1699 | 1.77E-03 | 1.18 | 0.869 | 2033 |
| C04 | 161577318 | rs17326199 | G | 0.211 | 10634 | 1.91E-03 | 1.16 | 0.237 | 1699 | 8.27E-03 | 1.12 | 0.231 | 2033 |
| C13 | 20622774 | rs9285207 | C | 0.195 | 10608 | 1.91E-03 | 1.16 | 0.22 | 1693 | 1.89E-03 | 1.15 | 0.218 | 2026 |
| C08 | 75316641 | rs10504576 | A | 0.478 | 10551 | 1.92E-03 | 1.13 | 0.509 | 1677 | 5.94E-04 | 1.13 | 0.51 | 2010 |
| C14 | 92766673 | rs2281519 | T | 0.257 | 10633 | 1.92E-03 | 1.15 | 0.284 | 1700 | 5.98E-03 | 1.12 | 0.279 | 2033 |
| C15 | 37096582 | rs1841441 | A | 0.466 | 10634 | 1.92E-03 | 1.13 | 0.496 | 1700 | 1.15E-03 | 1.13 | 0.496 | 2034 |
| C10 | 84843813 | rs2349157 | G | 0.855 | 10635 | 1.93E-03 | 1.2 | 0.876 | 1700 | 9.76E-04 | 1.19 | 0.876 | 2034 |
| C05 | 167524725 | rs2617972 | C | 0.808 | 10632 | 1.93E-03 | 1.17 | 0.832 | 1699 | 9.94E-03 | 1.13 | 0.827 | 2033 |
| C12 | 96022770 | rs989264 | A | 0.041 | 10635 | 1.93E-03 | 1.33 | 0.054 | 1700 | 1.86E-03 | 1.31 | 0.053 | 2034 |
| C06 | 10958298 | rs9357002 | T | 0.631 | 10453 | 1.93E-03 | 1.14 | 0.66 | 1678 | 4.72E-03 | 1.11 | 0.655 | 2007 |
| C04 | 147453178 | rs723794 | T | 0.764 | 10635 | 1.94E-03 | 1.16 | 0.79 | 1700 | 5.53E-03 | 1.13 | 0.785 | 2034 |
| C11 | 127732102 | rs7933433 | G | 0.648 | 10633 | 1.95E-03 | 1.14 | 0.677 | 1699 | 1.69E-03 | 1.13 | 0.675 | 2033 |
| C03 | 5243162 | rs341973 | A | 0.51 | 10634 | 1.95E-03 | 1.13 | 0.541 | 1700 | 1.92E-02 | 1.09 | 0.531 | 2034 |
| C22 | 18482283 | rs2238798 | G | 0.858 | 10633 | 1.95E-03 | 1.2 | 0.879 | 1700 | 6.39E-03 | 1.16 | 0.875 | 2034 |
| Cl0 | 65928341 | rs990828 | G | 0.416 | 10631 | 1.96E-03 | 1.13 | 0.446 | 1700 | 1.23E-02 | 1.1 | 0.438 | 2034 |
| C08 | 143121983 | rs7834060 | G | 0.434 | 10635 | 1.96E-03 | 1.13 | 0.464 | 1700 | 2.51E-03 | 1.12 | 0.461 | 2034 |
| C01 | 184791657 | rs1339083 | A | 0.34 | 10635 | 1.96E-03 | 1.14 | 0.369 | 1700 | 1.56E-03 | 1.13 | 0.367 | 2034 |
| C11 | 92444704 | rs9971402 | C | 0.872 | 10633 | 1.96E-03 | 1.21 | 0.892 | 1700 | 5.09E-04 | 1.22 | 0.893 | 2034 |
| C04 | 162770424 | rs958247 | A | 0.287 | 10626 | 1.96E-03 | 1.14 | 0.315 | 1700 | 1.64E-03 | 1.13 | 0.314 | 2034 |
| C07 | 150069821 | rs2373885 | A | 0.75 | 10631 | 1.96E-03 | 1.16 | 0.776 | 1700 | 8.98E-04 | 1.15 | 0.776 | 2034 |
| C08 | 139195557 | rs4909759 | T | 0.261 | 10633 | 1.96E-03 | 1.15 | 0.288 | 1700 | 7.46E-03 | 1.12 | 0.282 | 2034 |
| C02 | 209737500 | rs9288396 | T | 0.204 | 10635 | 1.97E-03 | 1.16 | 0.229 | 1700 | 1.09E-03 | 1.15 | 0.229 | 2034 |
| C01 | 40520068 | rs7524868 | G | 0.306 | 10611 | 1.97E-03 | 1.14 | 0.334 | 1691 | 3.27E-02 | 1.09 | 0.324 | 2024 |
| C06 | 79657373 | rs10943606 | G | 0.746 | 10632 | 1.97E-03 | 1.15 | 0.772 | 1700 | 1.90E-03 | 1.14 | 0.771 | 2033 |
| C16 | 2014037 | rs3785284 | C | 0.265 | 10571 | 1.98E-03 | 1.15 | 0.292 | 1698 | 2.17E-03 | 1.13 | 0.29 | 2032 |
| C02 | 215385419 | rs2216544 | A | 0.253 | 10635 | 1.98E-03 | 1.15 | 0.28 | 1700 | 2.46E-03 | 1.13 | 0.278 | 2034 |
| C17 | 53871788 | rs9895713 | G | 0.176 | 10633 | 1.98E-03 | 1.17 | 0.2 | 1700 | 2.03E-03 | 1.15 | 0.198 | 2034 |
| C03 | 2631107 | rs17565216 | C | 0.363 | 10631 | 1.98E-03 | 1.13 | 0.392 | 1699 | 9.20E-04 | 1.13 | 0.392 | 2033 |
| C07 | 116815796 | rs213987 | T | 0.327 | 10632 | 1.99E-03 | 1.14 | 0.356 | 1700 | 3.83E-03 | 1.12 | 0.352 | 2034 |
| C07 | 116815796 | rs213987 | T | 0.327 | 10632 | 1.99E-03 | 1.14 | 0.356 | 1700 | 3.83E-03 | 1.12 | 0.352 | 2034 |
| C03 | 175238582 | rs1549100 | T | 0.73 | 10574 | 1.99E-03 | 1.15 | 0.757 | 1693 | 3.92E-02 | 1.09 | 0.747 | 2026 |
| C06 | 69212746 | rs9454539 | A | 0.877 | 10630 | 1.99E-03 | 1.21 | 0.896 | 1699 | 9.23E-04 | 1.21 | 0.896 | 2033 |
| C01 | 65243954 | rs3818513 | C | 0.457 | 10635 | 1.99E-03 | 1.13 | 0.488 | 1700 | 5.23E-02 | 1.07 | 0.475 | 2034 |
| C12 | 123074369 | rs4765477 | T | 0.044 | 10632 | 1.99E-03 | 1.32 | 0.057 | 1700 | 3.78E-03 | 1.27 | 0.056 | 2034 |
| C02 | 118543519 | rs1477451 | T | 0.124 | 10635 | 2.00E-03 | 1.19 | 0.145 | 1700 | 2.98E-02 | 1.12 | 0.137 | 2034 |
| C08 | 143128135 | rs3934930 | T | 0.433 | 10635 | 2.00E-03 | 1.13 | 0.464 | 1700 | 2.52E-03 | 1.12 | 0.461 | 2034 |
| C07 | 81287212 | rs6467882 | C | 0.165 | 10631 | 2.00E-03 | 1.17 | 0.188 | 1700 | 1.69E-03 | 1.16 | 0.187 | 2034 |
| C01 | 155206460 | rs4971177 | T | 0.913 | 10634 | 2.00E-03 | 1.26 | 0.93 | 1700 | 8.85E-04 | 1.26 | 0.93 | 2034 |
| C06 | 119414590 | rs1360782 | A | 0.348 | 10634 | 2.01E-03 | 1.13 | 0.378 | 1700 | 1.44E-03 | 1.13 | 0.376 | 2034 |
| C07 | 50023386 | rs12669163 | C | 0.297 | 10525 | 2.01E-03 | 1.14 | 0.325 | 1675 | 5.51E-03 | 1.12 | 0.321 | 2004 |
| C07 | 16810246 | rs1029577 | T | 0.319 | 10625 | 2.02E-03 | 1.14 | 0.347 | 1697 | 2.55E-03 | 1.12 | 0.345 2031 | |
| C04 | 82277070 | rs10026120 | T | 0.96 | 10616 | 2.03E-03 | 1.4 | 0.971 | 1699 | 7.73E-03 | 1.31 | 0.969 | 2029 |
| C05 | 133913277 | rs1476097 | T | 0.656 | 10629 | 2.03E-03 | 1.14 | 0.685 | 1699 | 4.64E-04 | 1.15 | 0.686 | 2032 |
| C07 | 13679025 | rs3735344 | G | 0.421 | 10583 | 2.04E-03 | 1.13 | 0.451 | 1687 | 7.75E-03 | 1.1 | 0.445 | 2021 |
| C17 | 7878493 | rs4791759 | A | 0.038 | 10621 | 2.05E-03 | 1.34 | 0.051 | 1699 | 5.53E-03 | 1.28 | 0.048 | 2031 |
| C05 | 14319911 | rs30616 | T | 0.852 | 10634 | 2.05E-03 | 1.19 | 0.873 | 1700 | 4.29E-03 | 1.16 | 0.87 | 2034 |
| C06 | 143011798 | rs478884 | A | 0.879 | 10635 | 2.05E-03 | 1.22 | 0.898 | 1700 | 7.23E-04 | 1.22 | 0.898 | 2034 |
| C14 | 47996364 | rs12436454 | T | 0.713 | 10592 | 2.05E-03 | 1.15 | 0.74 | 1697 | 4.47E-03 | 1.12 | 0.736 | 2030 |
| C09 | 23762277 | rs7038525 | T | 0.152 | 10629 | 2.05E-03 | 1.18 | 0.174 | 1700 | 4.27E-04 | 1.19 | 0.176 | 2034 |
| C06 | 69230698 | rs10806602 | T | 0.877 | 10614 | 2.06E-03 | 1.21 | 0.897 | 1696 | 1.03E-03 | 1.21 | 0.897 | 2030 |
| C11 | 17918391 | rs3993323 | A | 0.14 | 10630 | 2.06E-03 | 1.18 | 0.162 | 1697 | 5.18E-03 | 1.15 | 0.159 | 2031 |
| C18 | 35157614 | rs12964741 | A | 0.667 | 10489 | 2.06E-03 | 1.14 | 0.695 . | 1671 | 3.40E-04 | 1.15 | 0.698 | 2002 |
| C08 | 73289641 | rs10504537 | A | 0.879 | 10635 | 2.08E-03 | 1.22 | 0.899 | 1700 | 4.08E-04 | 1.23 | 0.9 | 2034 |
| C06 | 72275474 | rs12207816 | A | 0.914 | 10635 | 2.08E-03 | 1.26 | 0.93 | 1700 | 7.95E-04 | 1.26 | 0.93 | 2034 |
| C19 | 4202069 | rs1045750 | C | 0.265 | 10634 | 2.08E-03 | 1.14 | 0.292 | 1699 | 6.95E-03 | 1.12 | 0.287 | 2033 |
| C02 | 203864322 | rs6704822 | G | 0.912 | 10558 | 2.08E-03 | 1.26 | 0.929 | 1689 | 3.09E-03 | 1.22 | 0.927 | 2019 |
| C01 | 173423432 | rs2175177 | A | 0.465 | 10625 | 2.09E-03 | 1.13 | 0.495 | 1698 | 1.84E-03 | 1.12 | 0.493 | 2031 |
| CX | 115198464 | rs2526763 | G | 0.365 | 8266 | 2.09E-03 | 1.17 | 0.401 | 1102 | 7.05E-03 | 1.13 | 0.394 | 1315 |
| C11 | 134180799 | rs1289455 | T | 0.147 | 10628 | 2.09E-03 | 1.18 | 0.169 | 1698 | 2.49E-03 | 1.16 | 0.167 | 2032 |
| C01 | 238623817 | rs3014559 | A | 0.546 | 10632 | 2.10E-03 | 1.13 | 0.576 | 1700 | 2.98E-03 | 1.12 | 0.573 | 2034 |
| C02 | 76254681 | rs317285 | A | 0.504 | 10634 | 2.10E-03 | 1.13 | 0.534 | 1700 | 2.98E-03 | 1.11 | 0.531 | 2034 |
| C08 | 37465414 | rs7017907 | C | 0.083 | 10598 | 2. low-03 | 1.23 | 0.101 | 1691 | 6.65E-03 | 1.19 | 0.098 | 2024 |
| C16 | 53597062 | rs3751812 | T | 0.404 | 10627 | 2.10E-03 | 1.13 | 0.433 | 1699 | 3.87E-04 | 1.14 | 0.436 | 2033 |
| C05 | 167526920 | rs1077046 | C | 0.553 | 10633 | 2.10E-03 | 1.13 | 0.583 | 1700 | 1.46E-02 | 1.09 | 0.575 | 2034 |
| C10 | 131748188 | rs11597160 | A | 0.906 | 10635 | 2.11E-03 | 1.25 | 0.923 | 1700 | 1.05E-02 | 1.18 | 0.919 | 2034 |
| C18 | 13442635 | rs12964045 | C | 0.13 | 10634 | 2.11E-03 | 1.19 | 0.151 | 1700 | 8.59E-03 | 1.15 | 0.147 | 2034 |
| C05 | 9694366 | rs3213627 | T | 0.167 | 10630 | 2.13E-03 | 1.17 | 0.19 | 1700 | 4.17E-03 | 1.15 | 0.187 | 2034 |
| C21 | 24693776 | rs2829040 | A | 0.449 | 10633 | 2.13E-03 | 1.13 | 0.479 | 1700 | 1.51E-03 | 1.12 | 0.478 | 2034 |
| C07 | 85269850 | rs1012995 | C | 0.219 | 10600 | 2.13E-03 | 1.15 | 0.244 | 1685 | 1.45E-03 | 1.15 | 0.243 | 2019 |
| C07 | 18443601 | rs2269752 | C | 0.93 | 10630 | 2.13E-03 | 1.29 | 0.944 | 1700 | 8.19E-03 | 1.22 | 0.941 | 2034 |
| C01 | 85995056 | rs1360903 | G | 0.511 | 10626 | 2.14E-03 | 1.13 | 0.541 | 1700 | 5.66E-03 | 1.11 | 0.536 | 2033 |
| C16 | 62628004 | rs2962080 | G | 0.609 | 10631 | 2.15E-03 | 1.13 | 0.638 | 1699 | 2.28E-03 | 1.12 | 0.636 | 2033 |
| C12 | 20081535 | rs2417821 | T | 0.749 | 10635 | 2.15E-03 | 1.15 | 0.775 | 1700 | 1.80E-04 | 1.18 | 0.778 | 2034 |
| C04 | 10634963 | rs2170752 | T | 0.576 | 10634 | 2.16E-03 | 1.13 | 0.606 | 1700 | 2.79E-02 | 1.09 | 0.596 | 2034 |
| C09 | 110310944 | rs4979085 | T | 0.297 | 10629 | 2.16E-03 | 1.14 | 0.324 | 1700 | 2.97E-03 | 1.12 | 0.322 | 2034 |
| C01 | 216145811 | rs1118175 | G | 0.076 | 10635 | 2.16E-03 | 1.24 | 0.092 | 1700 | 2.03E-03 | 1.22 | 0.091 | 2034 |
| C06 | 57080845 | rs7756421 | A | 0.84 | 10188 | 2.16E-03 | 1.19 | 0.862 | 1690 | 4.45E-03 | 1.16 | 0.859 | 2019 |
| C06 | 29594220 | rs3094564 | G | 0.386 | 10635 | 2.17E-03 | 1.13 | 0.415 | 1700 | 4.26E-03 | 1.11 | 0.411 | 2034 |
| C18 | 53282820 | rs580647 | A | 0.886 | 10622 | 2.17E-03 | 1.22 | 0.904 | 1698 | 6.59E. | 1.18 | 0.901 | 2031 |
| C14 | 63874253 | rs7145500 | A | 0.661 | 10634 | 2.17E-03 | 1.14 | 0.69 | 1700 | 1.37E-03 | 1.13 | 0.689 | 2034 |
| C02 | 218495194 | rs1478581 | G | 0.819 | 10632 | 2.18E-03 | 1.18 | 0.841 | 1700 | 3.13E-03 | 1.15 | 0.839 | 2032 |
| C13 | 102932541 | rs1347329 | C | 0.592 | 10616 | 2.18E-03 | 1.13 | 0.621 | 1700 | 1.53E-02 | 1.09 | 0.614 | 2033 |
| C05 | 39159439 | rs444556 | C | 0.301 | 10632 | 2.18E-03 | 1.14 | 0.329 | 1700 | 3.08E-03 | 1.12 | 0.326 | 2034 |
| C09 | 14600866 | rs1343705 | G | 0.471 | 10631 | 2.18E-03 | 1.13 | 0.501 | 1699 | 3.38E-03 | 1.11 | 0.498 | 2033 |
| C16 | 19038467 | rs8053779 | G | 0.734 | 10634 | 2.19E-03 | 1.15 | 0.76 | 1700 | 1.04E-02 | 1.11 | 0.754 | 2034 |
| C21 | 26972748 | rs2830357 | G | 0.568 | 10634 | 2.19E-03 | 1.13 | 0.598 | 1700 | 1.78E-03 | 1.12 | 0.597 | 2034 |
| C07 | 77798006 | rs2192658 | T | 0.076 | 10630 | 2.19E-03 | 1.24 | 0.092 | 1699 | 1.17E-02 | 1.18 | 0.088 | 2032 |
| C17 | 62590633 | rs1985961 | C | 0.412 | 10633 | 2.20E-03 | 1.13 | 0.442 | 1700 | 2.23E-03 | 1.12 | 0.44 | 2034 |
| C10 | 96398926 | rs10509680 | T | 0.05 | 10601 | 2.20E-03 | 1.29 | 0.064 | 1695 | 9.38E-03 | 1.23 | 0.061 | 2027 |
| C11 | 98634196 | rs1461684 | T | 0.849 | 10635 | 2.20E-03 | 1.19 | 0.87 | 1700 | 8.48E-03 | 1.15 | 0.866 | 2034 |
| C06 | 106231995 | rs4440505 | C | 0.781 | 10635 | 2.20E-03 | 1.16 | 0.806 | 1700 | 5.16E-03 | 1.13 | 0.802 | 2034 |
| C03 | 14269176 | rs2128163 | G | 0.533 | 10617 | 2.21E-03 | 1.13 | 0.563 | 1700 | 2.43E-03 | 1.12 | 0.56 | 2034 |
| C07 | 8516487 | rs12234501 | T | 0.133 | 10635 | 2.22E-03 | 1.19 | 0.154 | 1700 | 6.54E-04 | 1.19 | 0.155 | 2034 |
| C10 | 84892441 | rs10886432 | G | 0.638 | 10634 | 2.22E-03 | 1.14 | 0.666 | 1700 | 1.76E-04 | 1.16 | 0.67 | 2034 |
| C08 | 41405289 | rs7819949 | T | 0.271 | 10634 | 2.23E-03 | 1.14 | 0.299 | 1700 | 4.46E-02 | 1.08 | 0.288 | 2034 |
| C21 | 42743171 | rs3788014 | C | 0.925 | 10631 | 2.23E-03 | 1.28 | 0.94 | 1700 | 2.98E-03 | 1.24 | 0.939 | 2033 |
| C16 | 75032079 | rs4243110 | T | 0.458 | 10628 | 2.23E-03 | 1.13 | 0.489 | 1699 | 4.24E-03 | 1.11 | 0.485 | 2033 |
| C15 | 52604593 | rs2414325 | T | 0.489 | 10634 | 2.23E-03 | 1.13 | 0.519 | 1700 | 1.35E-03 | 1.12 | 0.518 | 2034 |
| C07 | 86657613 | rs31662 | G | 0.862 | 10635 | 2.24E-03 | 1.2 | 0.882 | 1700 . | 4.06E-03 | 1.17 | 0.88 | 2034 |
| C01 | 148824678 | rs6702842 | G | 0.269 | 10611 | 2.25E-03 | 1.14 | 0.296 | 1694 | 1.24E-02 | 1.11 | 0.289 | 2028 |
| C06 | 28141066 | rs149951 | T | 0.726 | 10635 | 2.25E-03 | 1.15 | 0.753 | 1700 | 2.41E-02 | 1.1 | 0.744 | 2034 |
| C06 | 28335583 | rs1635 | G | 0.942 | 10565 | 2.25E-03 | 1.32 | 0.955 | 1689 | 8.32E-03 | 1.24 | 0.953 | 2019 |
| C16 | 53594877 | rs8050136 | A | 0.404 | 10634 | 2.26E-03 | 1.13 | 0.433 | 1699 | 4.22E-04 | 1.14 | 0.436 | 2033 |
| C19 | 48943265 | rs346535 | T | 0.234 | 10635 | 2.26E-03 | 1.15 | 0.26 | 1700 | 4.17E-03 | 1.13 | 0.257 | 2034 |
| C13 | 101270990 | rs2038709 | C | 0.081 | 10629 | 2.26E-03 | 1.23 | 0.098 | 1700 | 1.17E-02 | 1.18 | 0.094 | 2034 |
| C01 | 19425721 | rs3748744 | G | 0.899 | 10634 | 2.26E-03 | 1.23 | 0.916 | 1700 | 2.68E-02 | 1.15 | 0.911 | 2034 |
| C04 | 107037574 | rs1982346 | C | 0.062 | 10480 | 2.27E-03 | 1.27 | 0.077 | 1682 | 1.13E-03 | 1.26 | 0.077 | 2011 |
| C15 | 34830800 | rs194620 | T | 0.232 | 10633 | 2.27E-03 | 1.15 | 0.257 | 1700 | 9.03E-03 | 1.12 | 0.252 | 2034 |
| C10 | 5948421 | rs7912210 | C | 0.963 | 10616 | 2.28E-03 | 1.42 | 0.973 | 1679 | 5.95E-04 | 1.45 | 0.974 | 2012 |
| C21 | 14334270 | rs2822391 | A | 0.548 | 10634 | 2.28E-03 | 1.13 | 0.578 | 1700 | 8.05E-04 | 1.13 | 0.578 | 2034 |
| C05 | 15952862 | rs7715223 | C | 0.819 | 10597 | 2.28E-03 | 1.18 | 0.842 | 1692 | 1.73E-02 | 1.12 | 0.835 | 2026 |
| C02 | 75385815 | rs4853116 | A | 0.624 | 10364 | 2.29E-03 | 1.13 | 0.653 | 1695 | 2.15E-03 | 1.12 | 0.651 | 2025 |
| C05 | 56050092 | rs4700485 | A | 0.322 | 10635 | 2.29E-03 | 1.14 | 0.351 | 1700 | 1.00E-02 | 1.1 | 0.344 | 2034 |
| C01 | 184820295 | rs2383658 | G | 0.388 | 10633 | 2.29E-03 | 1.13 | 0.417 | 1700 | 1.58E-03 | 1.12 | 0.416 | 2034 |
| C03 | 82844262 | rs13075335 | T | 0.9 | 10058 | 2.29E-03 | 1.24 | 0.918 | 1678 | 9.58E-03 | 1.18 | 0.914 | 2004 |
| C04 | 82322766 | rs6830629 | A | 0.975 | 10625 | 2.30E-03 | 1.56 | 0.984 | 1699 | 8.03E-03 | 1.41 | 0.983 | 2032 |
| C14 | 63868436 | rs2411822 | C | 0.485 | 10630 | 2.30E-03 | 1.13 | 0.515 | 1700 | 9.77E-03 | 1.1 | 0.509 | 2034 |
| C05 | 55844670 | rs30365 | C | 0.898 | 10635 | 2.30E-03 | 1.23 | 0.915 | 1700 | 5.89E-03 | 1.19 | 0.912 | 2034 |
| C06 | 29516393 | rs2074464 | T | 0.445 | 10625 | 2.31E-03 | 1.13 | 0.475 | 1699 | 9.91E-03 | 1.1 | 0.469 | 2033 |
| C06 | 29588861 | rs3094572 | A | 0.386 | 10635 | 2.31E-03 | 1.13 | 0.416 | 1700 | 4.61E-03 | 1.11 | 0.412 | 2034 |
| C01 | 218026319 | rs2642438 | G | 0.71 | 10581 | 2.31E-03 | 1.14 | 0.737 | 1693 | 1.21E-02 | 1.11 | 0.731 | 2025 |
| C14 | 101389489 | rs4708 | A | 0.546 | 10627 | 2.31E-03 | 1.13 | 0.576 | 1698 | 4.92E-03 | 1.11 | 0.572 | 2030 |
| C02 | 76278450 | rs404892 | C | 0.505 | 10634 | 2.31E-03 | 1.13 | 0.535 | 1700 | 3.19E-03 | 1.11 | 0.532 | 2034 |
| C11 | 1341537 | rs7122936 | A | 0.572 | 10556 | 2.31E-03 | 1.13 | 0.602 | 1700 | 4.84E-03 | 1.11 | 0.598 | 2034 |
| C11 | 1341537 | rs7122936 | A | 0.572 | 10556 | 2.31 E-03 | 1.13 | 0.602 | 1700 | 4.84E-03 | 1.11 | 0.598 | 2034 |
| C05 | 56080018 | rs2657615 | C | 0.115 | 10635 | 2.32E-03 | 1.2 | 0.135 | 1700 | 5.17E-03 | 1.17 | 0.132 | 2034 |
| C14 | 64537666 | rs6573648 | G | 0.274 | 10634 | 2.33E-03 | 1.14 | 0.301 | 1700 | 2.25E-03 | 1.13 | 0.299 | 2034 |
| C17 | 70794100 | rs2159010 | A | 0.727 | 10583 | 2.33E-03 | 1.15 | 0.753 | 1700 | 4.40E-03 | 1.13 | 0.75 | 2032 |
| C07 | 46518795 | rs956010 | A | 0.88 | 10635 | 2.33E-03 | 1.21 | 0.899 | 1700 | 2.09E-03 | 1.2 | 0.898 | 2034 |
| C15 | 92040939 | rs10152962 | G | 0.154 | 10635 | 2.33E-03 | 1.17 | 0.176 | 1700 | 7.80E-04 | 1.18 | 0.177 | 2034 |
| C20 | 49591938 | rs913670 | C | 0.631 | 10595 | 2.33E-03 | 1.13 | 0.66 | 1700 | 3.99E-03 | 1.12 | 0.656 | 2033 |
| C20 | 57938553 | rs6128397 | G | 0.689 | 10633 | 2.34E-03 | 1.14 | 0.716 | 1700 | 7.46E-03 | 1.11 | 0.711 | 2034 |
| C21 | 21803122 | rs2017705 | C | 0.073 | 10598 | 2.34E-03 | 1.24 | 0.089 | 1696 | 1.14E-02 | 1.19 | 0.086 | 2029 |
| C05 | 3996863 | rs634344 | G | 0.067 | 10633 | 2.34E-03 | 1.25 | 0.082 | 1700 | 6.05E-03 | 1.21 | 0.079 . | 2033 |
| C06 | 27418512 | rs9461366 | G | 0.614 | 10635 | 2.34E-03 | 1.13 | 0.643 | 1700 | 6.05E-03 | 1.11 | 0.638 | 2034 |
| C22 | 19648862 | rs178260 | T | 0.14 | 10632 | 2.34E-03 | 1.18 | 0.161 | 1700 | 6.21E-03 | 1.15 | 0.158 | 2034 |
| C09 | 112545345 | rs2236388 | G | 0.899 | 10634 | 2.35E-03 | 1.23 | 0.917 | 1700 | 7.73E-04 | 1.24 | 0.917 | 2034 |
| C07 | 19730452 | rs10486378 | G | 0.277 | 10449 | 2.35E-03 | 1.14 | 0.304 | 1673 | 1.11E-02 | 1.11 | 0.298 | 2002 |
| C05 | 6325742 | rs1457122 | T | 0.333 | 10633 | 2.35E-03 | 1.13 | 0.361 | 1700 | 2.33E-03 | 1.12 | 0.359 | 2034 |
| C08 | 92473760 | rs2607102 | T | 0.927 | 10586 | 2.35E-03 | 1.28 | 0.942 | 1690 | 5.93E-03 | 1.23 | 0.939 | 2020 |
| C06 | 165937562 | rs9348022 | T | 0.657 | 10631 | 2.37E-03 | 1.14 | 0.685 | 1699 | 5.08E-03 | 1.12 | 0.681 | 2033 |
| C10 | 78039047 | rs1865017 | C | 0.734 | 10633 | 2.37E-03 | 1.15 | 0.76 | 1700 | 5.34E-03 | 1.12 | 0.756 | 2034 |
| C13 | 45593565 | rs9534568 | A | 0.76 | 10269 | 2.37E-03 | 1.15 | 0.785 | 1695 | 2.46E-03 | 1.14 | 0.783 | 2024 |
| C08 | 32774151 | rs9656744 | T | 0.511 | 10632 | 2.38E-03 | 1.13 | 0.541 | 1699 | 3.71E-03 | 1.11 | 0.537 | 2030 |
| C09 | 68331465 | rs943535 | A | 0.917 | 10584 | 2.39E-03 | 1.26 | 0.933 | 1690 | 9.80E-04 | 1.26 | 0.933 | 2020 |
| C10 | 87769103 | rs10749535 | T | 0.472 | 10635 | 2.40E-03 | 1.13 | 0.502 | 1700 | 2.92E-03 | 1.11 | 0.499 | 2034 |
| C11 | 98579265 | rs7120353 | T | 0.899 | 10634 | 2.40E-03 | 1.23 | 0.916 | 1700 | 2.08E-03 | 1.22 | 0.915 | 2034 |
| C05 | 31377276 | rs6875512 | A | 0.742 | 10632 | 2.40E-03 | 1.15 | 0.768 | 1700 | 7.97E-03 | 1.12 | 0.763 | 2034 |
| C02 | 198322740 | rs6434921 | A | 0.392 | 10635 | 2.40E-03 | 1.13 | 0.421 | 1700 | 1.59E-02 | 1.09 | 0.414 | 2034 |
| C06 | 5382731 | rs2113691 | C | 0.488 | 10479 | 2.40E-03 | 1.13 | 0.518 | 1644 | 3.11E-03 | 1.12 | 0.515 | 1974 |
| C10 | 50087691 | rs3793786 | C | 0.24 | 10635 | 2.40E-03 | 1.15 | 0.266 | 1700 | 4.64E-03 | 1.13 | 0.262 | 2034 |
| C08 | 23073423 | rs11775256 | T | 0.27 | 10634 | 2.41E-03 | 1.14 | 0.297 | 1700 | 9.12E-03 | 1.11 | 0.292 | 2034 |
| C12 | 116686949 | rs2592289 | A | 0.156 | 10632 | 2.41E-03 | 1.17 | 0.179 | 1700 | 2.57E-03 | 1.16 | 0.177 | 2034 |
| C07 | 157067324 | rs11769878 | C | 0.742 | 10433 | 2.41E-03 | 1.15 | 0.768 | 1690 | 2.84E-03 | 1.14 | 0.766 | 2022 |
| C11 | 56669965 | rs717117 | G | 0.075 | 10632 | 2.41E-03 | 1.24 | 0.091 | 1700 | 7.09E-03 | 1.19 | 0.088 | 2034 |
| C21 | 14824849 | rs2822753 | G | 0.357 | 10631 | 2.41E-03 | 1.13 | 0.386 | 1700 | 4.57E-03 | 1.11 | 0.382 | 2034 |
| C16 | 53995959 | rs1033043 | T | 0.574 | 10635 | 2.41E-03 | 1.13 | 0.604 | 1700 | 1.67E-02 | 1.09 | 0.596 | 2034 |
| C09 | 33091838 | rs10758186 | G | 0.381 | 10633 | 2.41E-03 | 1.13 | 0.41 | 1700 | 5.48E-03 | 1.11 | 0.406 | 2034 |
| C08 | 125394399 | rs4620270 | T | 0.13 | 10627 | 2.42E-03 | 1.19 | 0.15 | 1699 | 8.40E-04 | 1.19 | 0.151 | 2033 |
| C01 | 69518464 | rs10489554 | T | 0.77 | 10633 | 2.42E-03 | 1.16 | 0.795 | 1700 | 4.45E-03 | 1.13 | 0.792 | 2034 |
| C01 | 153811448 | rs12567958 | C | 0.266 | 10615 | 2.42E-03 | 1.14 | 0.293 | 1700 | 7.12E-03 | 1.12 | 0.288 | 2034 |
| C04 | 17086471 | rs7442366 | T | 0.821 | 10635 | 2.42E-03 | 1.17 | 0.844 | 1700 | 4.54E-04 | 1.19 | 0.845 | 2034 |
| C07 | 99312573 | rs999885 | T | 0.498 | 10467 | 2.43E-03 | 1.13 | 0.528 | 1678 | 4.30E-03 | 1.11 | 0.524 | 2012 |
| C03 | 61747598 | rs603052 | T | 0.778 | 10629 | 2.43E-03 | 1.16 | 0.802 | 1700 | 4.06E-03 | 1.14 | 0.799 | 2034 |
| C07 | 72804700 | rs7787362 | C | 0.557 | 10628 | 2.43E-03 | 1.13 | 0.587 | 1700 | 1.23E-02 | 1.1 | 0.58 | 2034 |
| C06 | 79723924 | rs1338023 | T | 0.623 | 10465 | 2.43E-03 | 1.13 | 0.652 | 1647 | 3.54E-03 | 1.12 | 0.649 | 1975 |
| C05 | 667701 | rs1697952 | C | 0.368 | 10612 | 2.43E-03 | 1.13 | 0.397 | 1697 | 4.73E-03 | 1.11 | 0.393 | 2030 |
| C17 | 40648038 | rs1122326 | C | 0.241 | 10633 | 2.44E-03 | 1.15 | 0.267 | 1700 | 3.93E-03 | 1.13 | 0.264 | 2034 |
| C10 | 10412081 | rs10508403 | T | 0.091 | 10635 | 2.44E-03 | 1.22 | 0.109 | 1700 | 2.93E-02 | 1.14 | 0.103 | 2034 |
| C02 | 171512196 | rs1837241 | A | 0.697 | 10634 | 2.44E-03 | 1.14 | 0.724 | 1700 | 1.28E-03 | 1.14 | 0.724 | 2034 |
| C06 | 17345030 | rs6940891 | C | 0.169 | 10635 | 2.45E-03 | 1.17 | 0.192 | 1700 | 2.87E-03 | 1.15 | 0.19 | 2034 |
| C06 | 42287224 | rs713383 | G | 0.684 | 10635 | 2.45E-03 | 1.14 | 0.712 | 1700 | 4.07E-03 | 1.12 | 0.708 | 2034 |
| C12 | 121500993 | rs7979863 | A | 0.375 | 10630 | 2.46E-03 | 1.13 | 0.404 | 1700 | 2.05E-03 | 1.12 | 0.402 | 2034 |
| C06 | 37577310 | rs9380681 | C | 0.308 | 10635 | 2.46E-03 | 1.14 | 0.335 | 1700 | 2.37E-03 | 1.13 | 0.333 | 2034 |
| C17 | 77817713 | rs17674253 | A | 0.579 | 10632 | 2.46E-03 | 1.13 | 0.608 | 1698 | 7.85E-03 | 1.1 | 0.603 | 2032 |
| C01 | 113140463 | rs773586 | A | 0.483 | 10633 | 2.46E-03 | 1.13 | 0.513 | 1700 | 3.45E-04 | 1.14 | 0.515 | 2034 |
| C06 | 92200200 | rs6454860 | G | 0.325 | 10314 | 2.46E-03 | 1.14 | 0.353 | 1639 | 3.16E-03 | 1.12 | 0.35 | 1969 |
| C10 | 61460650 | rs4948410 | A | 0.784 | 10631 | 2.47E-03 | 1.16 | 0.808 | 1700 | 4.52E-03 | 1.14 | 0.805 | 2034 |
| C06 | 157865565 | rs16900254 | A | 0.408 | 10635 | 2.47E-03 | 1.13 | 0.437 | 1700 | 1.34E-02 | 1.1 | 0.43 | 2034 |
| C15 | 93466050 | rs176656 | A | 0.09 | 10635 | 2.48E-03 | 1.22 | 0.108 | 1700 | 6.94E-04 | 1.23 | 0.109 | 2034 |
| C11 | 102978426 | rs719527 | C | 0.233 | 10612 | 2.49E-03 | 1.15 | 0.258 | 1700 | 5.40E-03 | 1.13 | 0.254 | 2034 |
| C20 | 57627978 | rs6128327 | A | 0.64 | 10481 | 2.50E-03 | 1.13 | 0.668 | 1683 | 1.61E-03 | 1.13 | 0.667 | 2015 |
| C07 | 21751434 | rs1174943 | G | 0.155 | 10631 | 2.51E-03 | 1.17 | 0.176 | 1700 | 1.20E-02 | 1.13 | 0.171 | 2034 |
| C07 | 8617064 | rs917038 | C | 0.784 | 10591 | 2.51E-03 | 1.16 | 0.808 | 1689 | 2.80E-02 | 1.1 | 0.8 | 2023 |
| C20 | 22311552 | rs199812 | C | 0.886 | 10635 | 2.51E-03 | 1.22 | 0.904 | 1700 | 9.30E-04 | 1.22 | 0.904 | 2034 |
| C04 | 147576738 | rs2241521 | A | 0.234 | 10634 | 2.51E-03 | 1.15 | 0.26 | 1700 | 1.05E-02 | 1.11 | 0.254 | 2034 |
| C11 | 20014361 | rs920671 | A | 0.182 | 10634 | 2.51E-03 | 1.16 | 0.205 | 1700 | 2.83E-03 | 1.15 | 0.203 | 2034 |
| C11 | 82788837 | rs4623925 | A | 0.267 | 10633 | 2.51E-03 | 1.14 | 0.294 | 1700 | 2.34E-02 | 1.1 | 0.286 | 2034 |
| C21 | 24056599 | rs2828515 | G | 0.122 | 10633 | 2.52E-03 | 1.19 | 0.142 | 1700 | 8.56E-04 | 1.2 | 0.143 | 2034 |
| C02 | 44054859 | r6709904 | A | 0.907 | 10635 | 2.52E-03 | 1.24 | 0.924 | 1700 | 1.38E-03 | 1.24 | 0.924 | 2034 |
| C17 | 21252111 | rs9911448 | C | 0.962 | 10635 | 2.52E-03 | 1.41 | 0.972 | 1700 | 5.90E-03 | 1.33 | 0.971 | 2034 |
| C18 | 55909735 | rs9948303 | G | 0.099 | 10635 | 2.52E-03 | 1.21 | 0.117 | 1700 | 1.15E-03 | 1.21 | 0.117 | 2034 |
| C01 | 20385176 | rs2072671 | C | 0.314 | 10635 | 2.53E-03 | 1.13 | 0.341 | 1700 | 8.33E-04 | 1.14 | 0.342 | 2034 |
| C01 | 113074488 | rs6688859 | T | 0.68 | 10635 | 2.53E-03 | 1.14 | 0.708 | 1700 | 2.92E-04 | 1.15 | 0.711 | 2034 |
| C15 | 95693327 | rs7177625 | G | 0.683 | 10633 | 2.53E-03 | 1.14 | 0.71 | 1700 | 9.07E-03 | 1.11 | 0.705 | 2033 |
| C12 | 20118148 | rs11045018 | G | 0.749 | 10626 | 2.53E-03 | 1.15 | 0.774 | 1699 | 5.07E-04 | 1.16 | 0.776 | 2033 |
| C16 | 82538999 | rs1870843 | T | 0.599 | 10633 | 2.53E-03 | 1.13 | 0.628 . | 1700 | 2.99E-03 | 1.12 | 0.625 | 2034 |
| C01 | 78369472 | rs1543675 | C | 0.376 | 10633 | 2.53E-03 | 1.13 | 0.405 | 1700 | 1.08E-02 | 1.1 | 0.399 | 2034 |
| C01 | 107498557 | rs2335793 | C | 0.887 | 10628 | 2.53E-03 | 1.22 | 0.905 | 1698 | 8.13E-04 | 1.23 | 0.906 | 2031 |
| C03 | 6741178 | rs978658 | G | 0.611 | 10635 | 2.54E-03 | 1.13 | 0.639 | 1700 | 1.13E-03 | 1.13 | 0.639 | 2034 |
| C05 | 76843306 | rs690280 | T | 0.422 | 10580 | 2.54E-03 | 1.13 | 0.451 | 1694 | 8.75E-03 | 1.1 | 0.446 | 2028 |
| C10 | 30821283 | rs7086224 | A | 0.418 | 10633 | 2.54E-03 | 1.13 | 0.448 | 1700 | 4.77E-03 | 1.11 | 0.444 | 2034 |
| C07 | 86648971 | rs2373593 | A | 0.862 | 10635 | 2.55E-03 | 1.2 | 0.882 | 1700 | 4.51E-03 | 1.17 | 0.88 | 2034 |
| C19 | 53768499 | rs3760802 | A | 0.499 | 10633 | 2.55E-03 | 1.13 | 0.528 | 1699 | 3.62E-03 | 1.11 | 0.525 | 2033 |
| C17 | 40663717 | rs12603327 | C | 0.241 | 10632 | 2.55E-03 | 1.15 | 0.267 | 1700 | 4.05E-03 | 1.13 | 0.264 | 2034 |
| C10 | 101886011 | rs1539089 | C | 0.222 | 10635 | 2.55E-03 | 1.15 | 0.247 | 1700 | 1.13E-02 | 1.12 | 0.241 | 2034 |
| C19 | 8795907 | rs10406508 | T | 0.764 | 10635 | 2.56E-03 | 1.15 | 0.789 | 1700 | 2.84E-02 | 1.1 | 0.78 | 2034 |
| C05 | 50786999 | rs1423611 | C | 0.445 | 10631 | 2.56E-03 | 1.13 | 0.474 | 1700 | 5.58E-03 | 1.11 | 0.47 | 2034 |
| C05 | 50786999 | rs1423611 | C | 0.445 | 10631 | 2.56E-03 | 1.13 | 0.474 | 1700 | 5.58E-03 | 1.11 | 0.47 | 2034 |
| C06 | 29574824 | rs1345227 | G | 0.379 | 10630 | 2.57E-03 | 1.13 | 0.408 | 1700 | 4.62E-03 | 1.11 | 0.404 | 2034 |
| C02 | 172857641 | rs6758704 | T | 0.709 | 10633 | 2.57E-03 | 1.14 | 0.736 | 1700 | 4.05E-03 | 1.12 | 0.733 | 2034 |
| C05 | 9699750 | rs2106687 | G | 0.125 | 10631 | 2.58E-03 | 1.19 | 0.146 | 1700 | 6.01E-03 | 1.16 | 0.142 | 2033 |
| C22 | 19683076 | rs430305 | T | 0.828 | 10635 | 2.58E-03 | 1.18 | 0.85 | 1700 | 2.96E-03 | 1.16 | 0.848 | 2034 |
| C19 | 22695812 | rs4933027 | T | 0.085 | 10634 | 2.58E-03 | 1.22 | 0.102 | 1700 | 9.19E-03 | 1.18 | 0.099 | 2034 |
| C11 | 83108627 | rs7952084 | A | 0.357 | 10635 | 2.58E-03 | 1.13 | 0.386 | 1700 | 5.81E-03 | 1.11 | 0.382 | 2034 |
| C07 | 27992355 | rs10225945 | A | 0.846 | 10535 | 2.59E-03 | 1.19 | 0.867 | 1697 | 4.58E-03 | 1.16 | 0.865 | 2030 |
| C04 | 10063925 | rs714086 | G | 0.759 | 10631 | 2.59E-03 | 1.15 | 0.784 | 1700 | 1.36E-02 | 1.11 | 0.778 | 2033 |
| C08 | 138245904 | rs4451334 | A | 0.062 | 10632 | 2.59E-03 | 1.26 | 0.077 | 1698 | 7.62E-03 | 1.21 | 0.074 | 2032 |
| C05 | 115396824 | rs9326981 | C | 0.949 | 10634 | 2.59E-03 | 1.34 | 0.961 | 1700 | 3.01E-03 | 1.3 | 0.96 | 2034 |
| C10 | 101903540 | rs11190578 | T | 0.222 | 10631 | 2.59E-03 | 1.15 | 0.247 | 1700 | 1.15E-02 | 1.12 | 0.241 | 2034 |
| C10 | 102146771 | rs4630219 | C | 0.099 | 10572 | 2.60E-03 | 1.21 | 0.117 | 1696 | 2.90E-03 | 1.19 | 0.115 | 2027 |
| C17 | 40667190 | rs7214921 | C | 0.241 | 10634 | 2.60E-03 | 1.15 | 0.267 | 1700 | 4.13E-03 | 1.13 | 0.264 | 2034 |
| C14 | 64300426 | rs12433694 | C | 0.479 | 10635 | 2.61E-03 | 1.13 | 0.509 | 1700 | 1.43E-02 | 1.09 | 0.501 | 2034 |
| C22 | 18282773 | rs1978058 | T | 0.376 | 10630 | 2.61E-03 | 1.13 | 0.404 | 1700 | 1.08E-02 | 1.1 | 0.398 | 2033 |
| C04 | 131671262 | rs1027884 | T | 0.497 | 10634 | 2.61E-03 | 1.13 | 0.527 | 1700 | 2.40E-03 | 1.12 | 0.525 | 2034 |
| C08 | 28224848 | rs904053 | G | 0.225 | 10634 | 2.62E-03 | 1.15 | 0.25 | 1700 | 3.98E-02 | 1.09 | 0.24 | 2034 |
| C08 | 28224848 | rs904053 | G | 0.225 | 10634 | 2.61E-03 | 1.15 | 0.25 | 1700 | 3.98E-02 | 1.09 | 0.24 | 2034 |
| C16 | 54000987 | rs2665282 | A | 0.574 | 10607 | 2.61E-03 | 1.13 | 0.603 | 1696 | 1.79E-02 | 1.09 | 0.596 | 2030 |
| C05 | 136028429 | rs4141140 | G | 0.7 | 10632 | 2.62E-03 | 1.14 | 0.727 | 1700 | 1.58E-02 | 1.1 | 0.721 | 2034 |
| C07 | 49507399 | rs13240380 | T | 0.434 | 10632 | 2.62E-03 | 1.13 | 0.463 | 1700 | 1.78E-03 | 1.12 | 0.462 | 2033 |
| C09 | 11743695 | rs 10960174 | G | 0.613 | 10629 | 2.62E-03 | 1.13 | 0.641 | 1698 | 1.74E-03 | 1.13 | 0.64 | 2031 |
| C15 | 43706836 | rs7172161 | G | 0.513 | 10634 | 2.62E-03 | 1.13 | 0.543 | 1700 | 2.59E-03 | 1.12 | 0.541 | 2034 |
| C04 | 162740637 | rs9942159 | T | 0.377 | 10550 | 2.63E-03 | 1.13 | 0.406 | 1691 | 2.09E-03 | 1.12 | 0.404 | 2024 |
| C05 | 158736740 | rs1433048 | G | 0.192 | 10317 | 2.63E-03 | 1.16 | 0.216 | 1652 | 1.82E-02 | 1.11 | 0.21 | 1981 |
| C10 | 84886235 | rs4411234 | C | 0.638 | 10631 | 2.63E-03 | 1.13 | 0.666 | 1700 | 2.14E-04 | 1.15 | 0.67 | 2034 |
| C12 | 100468481 | rs864302 | T | 0.483 | 10630 | 2.63E-03 | 1.13 | 0.512 | 1700 | 7.50E-03 | 1.1 | 0.507 | 2034 |
| C05 | 76657043 | rs251421 | C | 0.26 | 10618 | 2.64E-03 | 1.14 | 0.286 | 1690 | 5.58E-03 | 1.12 | 0.283 | 2023 |
| C07 | 46531242 | rs6956126 | G | 0.885 | 10632 | 2.64E-03 | 1.22 | 0.904 | 1700 | 2.41E-03 | 1.2 | 0.903 | 2034 |
| C09 | 129104520 | rs7026302 | G | 0.908 | 10633 | 2.64E-03 | 1.24 | 0.924 | 1700 | 2.87E-02 | 1.15 | 0.919 | 2034 |
| C11 | 121657794 | rs531743 | A | 0.954 | 10633 | 2.64E-03 | 1.36 | 0.966 | 1700 | 2.00E-03 | 1.34 | 0.965 | 2034 |
| C11 | 33462777 | rs10836066 | T | 0.301 | 10634 | 2.65E-03 | 1.14 | 0.328 | 1700 | 3.03E-02 | 1.09 | 0.319 | 2034 |
| C05 | 122595829 | rs9327287 | G | 0.262 | 10632 | 2.65E-03 | 1.14 | 0.288 | 1700 | 7.19E-03 | 1.12 | 0.284 | 2034 |
| C04 | 181363537 | rs17090640 | G | 0.952 | 10635 | 2.65E-03 | 1.35 | 0.964 | 1700 | 1.64E-02 | 1.24 | 0.961 | 2034 |
| C04 | 119551429 | rs6820367 | A | 0.35 | 10634 | 2.65E-03 | 1.13 | 0.379 | 1700 | 4.66E-03 | 1.11 | 0.375 | 2034 |
| C19 | 62070079 | rs8113561 | G | 0.954 | 10625 | 2.65E-03 | 1.36 | 0.966 | 1700 | 3.66E-02 | 1.21 | 0.962 | 2034 |
| C08 | 97162902 | rs2440213 | A | 0.358 | 10632 | 2.65E-03 | 1.13 | 0.387 | 1700 | 5.98E-03 | 1.11 | 0.383 | 2033 |
| C10 | 93013669 | rs1028935 | A | 0.185 | 10633 | 2.65E-03 | 1.16 | 0.208 | 1700 | 1.86E-03 | 1.15 | 0.207 | 2034 |
| C06 | 24562175 | rs9393558 | T | 0.347 | 10635 | 2.65E-03 | 1.13 | 0.375 | 1700 | 2.11E-02 | 1.09 | 0.367 | 2034 |
| C09 | 103242888 | rs7854755 | A | 0.198 | 10589 | 2.65E-03 | 1.16 | 0.222 | 1694 | 7.15E-03 | 1.13 | 0.218 | 2027 |
| C09 | 79420643 | rs10867740 | C | 0.236 | 10625 | 2.65E-03 | 1.15 | 0.262 | 1699 | 2.00E-03 | 1.14 | 0.261 | 2033 |
| C20 | 53952228 | rs6068930 | G | 0.039 | 10620 | 2.66E-03 | 1.32 | 0.051 | 1700 | 9.79E-03 | 1.26 | 0.049 | 2034 |
| C14 | 68370111 | rs1291302 | C | 0.259 | 10527 | 2.66E-03 | 1.14 | 0.285 | 1675 | 3.98E-03 | 1.13 | 0.282 | 2008 |
| C01 | 93943749 | rs4147861 | G | 0.923 | 10526 | 2.66E-03 | 1.27 | 0.939 | 1668 | 2.12E-03 | 1.25 | 0.938 | 2000 |
| C14 | 98178185 | rs2273840 | A | 0.931 | 10569 | 2.67E-03 | 1.28 | 0.945 | 1677 | 1.07E-03 | 1.29 | 0.945 | 2009 |
| C14 | 98178185 | rs2273840 | A | 0.931 | 10569 | 2.67E-03 | 1.28 | 0.945 | 1677 | 1.07E-03 | 1.29 | 0.945 | 2009 |
| CX | 138713457 | rs1948488 | A | 0.223 | 8219 | 2.68E-03 | 1.19 | 0.254 | 1094 | 4.52E-03 | 1.16 | 0.25 | 1307 |
| C12 | 20196656 | rs7299937 | T | 0.059 | 10635 | 2.68E-03 | 1.26 | 0.074 | 1700 | 7.12E-03 | 1.22 | 0.071 | 2034 |
| C04 | 87630493 | r510516770 | T | 0.055 | 10635 | 2.69E-03 | 1.27 | 0.069 | 1700 | 4.44E-03 | 1.24 | 0.067 | 2034 |
| C02 | 172822314 | rs4668412 | A | 0.709 | 10634 | 2.69E-03 | 1.14 | 0.736 | 1700 | 4.24E-03 | 1.12 | 0.733 | 2034 |
| C06 | 36770559 | rs236444 | T | 0.253 | 10634 | 2.70E-03 | 1.14 | 0.279 | 1700 | 3.68E-04 | 1.16 | 0.281 | 2034 |
| C11 | 103253198 | rs10502020 | G | 0.446 | 10634 | 2.71E-03 | 1.13 | 0.476 | 1700 | 8.02E-03 | 1.1 | 0.47 | 2034 |
| C04 | 131653392 | rs4383635 | A | 0.491 | 10630 | 2.71E-03 | 1.13 | 0.521 | 1699 | 2.25E-03 | 1.12 | 0.519 | 2033 |
| C04 | 87557966 | rs2199309 | C | 0.229 | 10234 | 2.71E-03 | 1.15 | 0.255 | 1653 | 8.94E-04 | 1.15 | 0.256 | 1977 |
| C09 | 23737791 | rs1329044 | C | 0.153 | 10631 | 2.71E-03 | 1.17 | 0.175 | 1700 | 6.15E-04 | 1.18 | 0.176 | 2034 |
| C12 | 127797687 | rs470477 | T | 0.311 | 10632 | 2.71E-03 | 1.13 | 0.338 | 1700 | 5.14E-03 | 1.12 | 0.334 | 2033 |
| C13 | 105492839 | rs1927771 | A | 0.411 | 10633 | 2.72E-03 | 1.13 | 0.44 | 1700 | 4.66E-03 | 1.11 | 0.436 | 2034 |
| CX | 4916022 | rs6638525 | C | 0.107 | 8269 | 2.73E-03 | 1.25 | 0.13 | 1103 | 1.91E-03 | 1.24 | 0.129 | 1316 |
| C09 | 134921961 | rs2297538 | G | 0.73 | 10630 | 2.75E-03 | 1.15 | 0.756 | 1700 | 5.61E-03 | 1.12 | 0.752 | 2034 |
| C11 | 68645465 | rs2924536 | A | 0.447 | 10631 | 2.75E-03 | 1.13 | 0.476 | 1700 | 1.83E-02 | 1.09 | 0.469 | 2034 |
| C02 | 173022859 | rs10165126 | C | 0.705 | 10630 | 2.75E-03 | 1.14 | 0.732 | 1699 | 2.98E-03 | 1.13 | 0.729 | 2033 |
| C11 | 10906227 | rs10500734 | A | 0.767 | 10617 | 2.75E-03 | 1.15 | 0.791 | 1692 | 2.25E-02 | 1.11 | 0.784 | 2025 |
| C02 | 12183852 | rs10929777 | C | 0.3 | 10634 | 2.75E-03 | 1.14 | 0.327 | 1700 | 7.76E-03 | 1.11 | 0.323 | 2034 |
| C01 | 106549733 | rs10494048 | T | 0.387 | 10589 | 2.75E-03 | 1.13 | 0.416 | 1699 | 2.94E-02 | 1.08 | 0.406 | 2030 |
| C10 | 69228609 | rs17456902 | G | 0.872 | 10635 | 2.76E-03 | 1.2 | 0.891 | 1700 | 3.98E-03 | 1.18 | 0.889 | 2034 |
| C04 | 90099908 | rs2924936 | G | 0.869 | 10635 | 2.76E-03 | 1.2 | 0.888 | 1700 | 3.77E-04 | 1.22 | 0.89 | 2034 |
| C06 | 37561316 | rs9296225 | C | 0.472 | 10635 | 2.76E-03 | 1.13 | 0.502 | 1700 | 3.40E-03 | 1.11 | 0.499 | 2034 |
| C22 | 17337894 | rs2019061 | T | 0.685 | 10621 | 2.77E-03 | 1.14 | 0.712 | 1694 | 6.23E-03 | 1.12 | 0.708 | 2028 |
| C02 | 16792100 | rs11897395 | C | 0.889 | 10590 | 2.77E-03 | 1.22 | 0.907 | 1686 | 1.35E-02 | 1.16 | 0.903 | 2020 |
| C02 | 43918060 | rs1991739 | A | 0.619 | 10632 | 2.77E-03 | 1.13 | 0.647 | 1700 | 1.72E-02 | 1.09 | 0.64 | 2034 |
| C07 | 102594288 | rs7804867 | A | 0.205 | 10634 | 2.78E-03 | 1.15 | 0.229 | 1700 | 1.92E-03 | 1.15 | 0.228 | 2034 |
| C06 | 10170597 | rs6459613 | A | 0.545 | 10601 | 2.78E-03 | 1.13 | 0.574 | 1698 | 2.65E-02 | 1.09 | 0.565 | 2030 |
| C05 | 50904720 | rs7708362 | T | 0.345 | 10619 | 2.78E-03 | 1.13 | 0.373 | 1687 | 5.55E-03 | 1.11 | 0.369 | 2021 |
| C05 | 94682297 | rs464906 | C | 0.614 | 10630 | 2.78E-03 | 1.13 | 0.642 | 1700 | 9.79E-03 | 1.1 | 0.637 | 2034 |
| C10 | 8311160 | rs1419294 | T | 0.711 | 10635 | 2.78E-03 | 1.14 | 0.738 | 1700 | 1.09E-03 | 1.14 | 0.738 | 2034 |
| C10 | 2108862 | rs12776254 | G | 0.167 | 10634 | 2.78E-03 | 1.17 | 0.19 | 1700 | 1.95E-03 | 1.16 | 0.189 | 2034 |
| C20 | 59771295 | rs6128843 | T | 0.182 | 10634 | 2.79E-03 | 1.16 | 0.206 | 1700 | 1.16E-03 | 1.16 | 0.206 | 2034 |
| C02 | 242432072 | rs12052982 | A | 0.958 | 10630 | 2.79E-03 | 1.38 | 0.969 | 1698 | 7.24E-03 | 1.3 | 0.967 | 2032 |
| C10 | 102016372 | rs9420802 | G | 0.209 | 10632 | 2.80E-03 | 1.15 | 0.233 | 1698 | 1.15E-02 | 1.12 | 0.228 | 2032 |
| C07 | 149980541 | rs10277774 | C | 0.383 | 10632 | 2.80E-03 | 1.13 | 0.412 | 1699 | 1.84E-03 | 1.12 | 0.411 | 2033 |
| C12 | 21065184 | rs1910186 | C | 0.109 | 10630 | 2.80E-03 | 1.2 | 0.128 | 1700 | 1.84E-03 | 1.19 | 0.128 | 2034 |
| C12 | 21065184 | rs1910186 | C | 0.109 | 10630 | 2.80E-03 | 1.2 | 0.128 | 1700 | 1.84E-03 | 1.19 | 0.128 | 2034 |
| C17 | 73745676 | rs1000299 | A | 0.055 | 10635 | 2.81E-03 | 1.27 | 0.069 | 1700 | 2.50E-02 | 1.19 | 0.064 | 2034 |
| C06 | 162143621 | rs7739802 | T | 0.491 | 10594 | 2.81E-03 | 1.12 | 0.521 | 1699 | 4.70E-04 | 1.14 | 0.523 | 2030 |
| C11 | 102072274 | rs1276252 | G | 0.252 | 10634 | 2.81E-03 | 1.14 | 0.277 | 1700 | 1.08E-02 | 1.11 | 0.272 | 2034 |
| C12 | 123335210 | rs3759107 | T | 0.127 | 10635 | 2.81E-03 | 1.19 | 0.147 | 1700 | 3.67E-03 | 1.17 | 0.145 | 2034 |
| C11 | 42941748 | rs7939657 | T | 0.558 | 10632 | 2.81E-03 | 1.13 | 0.587 | 1700 | 3.56E-03 | 1.11 | 0.585 | 2034 |
| C14 | 63855666 | rs7147624 | G | 0.798 | 10635 | 2.81E-03 | 1.16 | 0.821 | 1700 | 3.48E-03 | 1.15 | 0.819 | 2034 |
| C07 | 21959042 | rs17146355 | A | 0.05 | 10619 | 2.82E-03 | 1.29 | 0.063 | 1697 | 1.93E-03 | 1.28 | 0.063 | 2031 |
| C01 | 7589173 | rs10462018 | C | 0.816 | 10632 | 2.82E-03 | 1.17 | 0.839 | 1700 | 1.67E-02 | 1.12 | 0.833 | 2034 |
| C15 | 87930503 | rs12595195 | C | 0.275 | 10632 | 2.82E-03 | 1.14 | 0.301 | 1700 | 5.15E-03 | 1.12 | 0.298 | 2034 |
| C04 | 90107239 | rs13130 | T | 0.87 | 10623 | 2.82E-03 | 1.2 | 0.889 | 1698 | 3.57E-04 | 1.22 | 0.891 | 2032 |
| C03 | 99664143 | rs1531377 | T | 0.173 | 10628 | 2.83E-03 | 1.16 | 0.196 | 1699 | 1.02E-02 | 1.13 | 0.191 | 2033 |
| C04 | 30136639 | rs2060515 | T | 0.111 | 10569 | 2.83E-03 | 1.2 | 0.13 | 1689 | 4.85E-03 | 1.17 | 0.127 | 2020 |
| C18 | 58447316 | rs2332575 | A | 0.053 | 10634 | 2.84E-03 | 1.28 | 0.066 | 1699 | 2.85E-03 | 1.26 | 0.065 | 2033 |
| C06 | 12338790 | rs1028716 | T | 0.06 | 10632 | 2.84E-03 | 1.26 | 0.074 | 1700 | 4.77E-03 | 1.23 | 0.072 | 2034 |
| C05 | 55657524 | rs32496 | C | 0.573 | 10633 | 2.85E-03 | 1.13 | 0.601 | 1700 | 4.48E-03 | 1.11 | 0.598 | 2034 |
| C08 | 5835084 | rs890014 | C | 0.757 | 10635 | 2.86E-03 | 1.15 | 0.781 | 1700 | 4.61E-03 | 1.13 | 0.779 | 2034 |
| C21 | 14821123 | rs2822744 | A | 0.157 | 10633 | 2.86E-03 | 1.17 | 0.179 | 1700 | 4.96E-03 | 1.15 | 0.176 | 2034 |
| C06 | 19958854 | rs6905466 | T | 0.327 | 10635 | 2.86E-03 | 1.13 | 0.355 | 1700 | 1.18E-03 | 1.13 | 0.355 | 2034 |
| C15 | 77441636 | rs4255747 | G | 0.668 | 10635 | 2.86E-03 | 1.13 | 0.695 | 1700 | 1.01E-02 | 1.11 | 0.69 | 2034 |
| C15 | 53381369 | rs8041044 | C | 0.914 | 10627 | 2.86E-03 | 1.25 | 0.93 | 1698 | 1.09E-02 | 1.19 | 0.927 | 2032 |
| C02 | 24312396 | rs10200481 | A | 0.106 | 10635 | 2.86E-03 | 1.2 | 0.125 | 1700 | 1.22E-02 | 1.15 | 0.121 | 2033 |
| C10 | 16380276 | rs2184383 | G | 0.805 | 10628 | 2.87E-03 | 1.16 | 0.828 | 1700 | 4.18E-03 | 1.14 | 0.825 | 2034 |
| C09 | 8506194 | rs10119236 | A | 0.294 | 10632 | 2.87E-03 | 1.14 | 0.321 | 1700 | 2.66E-02 | 1.09 | 0.312 | 2034 |
| C02 | 78341614 | rs2861264 | T | 0.105 | 10635 | 2.87E-03 | 1.2 | 0.123 | 1700 | 3.16E-03 | 1.18 | 0.122 | 2034 |
| C18 | 58394216 | rs3744927 | G | 0.773 | 10634 | 2.88E-03 | 1.15 | 0.797 | 1700 | 1.35E-02 | 1.12 | 0.792 | 2034 |
| C02 | 77756026 | rs6547152 | C | 0.787 | 10620 | 2.89E-03 | 1.16 | 0.811 | 1698 | 1.38E-03 | 1.16 | 0.811 | 2032 |
| C12 | 52445647 | rs6580960 | A | 0.541 | 10635 | 2.89E-03 | 1.13 | 0.57 | 1700 | 3.40E-03 | 1.11 | 0.567 | 2034 |
| C07 | 27780647 | rs740127 | G | 0.595 | 10635 | 2.89E-03 | 1.13 | 0.624 | 1700 | 1.23E-02 | 1.1 | 0.618 | 2034 |
| C07 | 50816853 | rs17134122 | A | 0.966 | 10634 | 2.89E-03 | 1.43 | 0.976 | 1700 | 1.10E-02 | 1.32 | 0.974 | 2034 |
| C22 | 34868465 | rs132717 | C | 0.62 | 10359 | 2.90E-03 | 1.13 | 0.648 | 1664 | 2.36E-02 | 1.09 | 0.64 | 1990 |
| C18 | 55211067 | rs8088514 | C | 0.461 | 10602 | 2.90E-03 | 1.12 | 0.49 | 1693 | 1.65E-02 | 1.09 | 0.482 | 2027 |
| C10 | 119681455 | rs7922488 | G | 0.533 | 10616 | 2.90E-03 | 1.13 | 0.562 | 1692 | 4.75E-03 | 1.11 | 0.558 | 2026 |
| C05 | 158745072 | rs7709212 | T | 0.678 | 10635 | 2.90E-03 | 1.14 | 0.705 | 1700 | 1.55E-02 | 1.1 | 0.699 | 2034 |
| C05 | 23761851 | rs1743379 | T | 0.507 | 10633 | 2.91E-03 | 1.12 | 0.536 | 1700 | 4.46E-04 | 1.14 | 0.539 | 2034 |
| C06 | 130039640 | rs376632 | G | 0.89 | 10633 | 2.91E-03 | 1.22 | 0.908 | 1699 | 2.60E-03 | 1.2 | 0.907 | 2033 |
| C10 | 8277669 | rs4747760 | G | 0.875 | 10631 | 2.91E-03 | 1.2 | 0.894 | 1700 | 3.50E-03 | 1.18 | 0.892 | 2034 |
| C03 | 145966921 | rs13061557 | T | 0.699 | 10630 | 2.91E-03 | 1.14 | 0.726 | 1700 | 1.57E-02 | 1.1 | 0.719 | 2034 |
| C06 . | 122997014 | rs9388117 | A | 0.671 | 10616 | 2.92E-03 | 1.13 | 0.698 | 1699 | 1.83E-02 | 1.1 | 0.691 | 2033 |
| C10 | 132443494 | rs893504 | C | 0.071 | 10635 | 2.92E-03 | 1.24 | 0.087 | 1700 | 2.64E-03 | 1.22 | 0.086 | 2034 |
| C04 | 40312795 | rs4447895 | C | 0.888 | 10634 | 2.92E-03 | 1.22 | 0.906 | 1700 | 1.56E-03 | 1.21 | 0.906 | 2034 |
| C06 | 162147237 | rs3016536 | G | 0.517 | 10635 | 2.93E-03 | 1.12 | 0.546 | 1700 | 1.72E-03 | 1.12 | 0.545 | 2034 |
| C15 | 58046702 | rs4775234 | C | 0.259 | 10530 | 2.93E-03 | 1.14 | 0.285 | 1681 | 3.63E-02 | 1.09 | 0.276 | 2009 |
| C10 | 107117270 | rs11192557 | T | 0.876 | 10623 | 2.93E-03 | 1.2 | 0.895 | 1698 | 2.56E-03 | 1.19 | 0.893 | 2032 |
| C07 | 1681599 | rs10267593 | G | 0.835 | 10635 | 2.93E-03 | 1.18 | 0.856 | 1700 | 2.02E-04 | 1.21 | 0.859 | 2034 |
| C14 | 98123983 | rs2895811 | C | 0.448 | 10607 | 3.04E-03 | 1.12 | 0.478 | 1694 | 3.73E-03 | 1.11 | 0.475 | 2027 |
| C17 | 18260758 | rs854771 | C | 0.554 | 10635 | 3.18E-03 | 1.12 | 0.583 | 1700 | 1.54E-03 | 1.12 | 0.583 | 2034 |
| C04 | 129890119 | rs4549394 | T | 0.097 | 10631 | 3.31E-03 | 1.21 | 0.115 | 1700 | 2.25E-03 | 1.2 | 0.114 | 2034 |
| C02 | 176994186 | rs12621733 | G | 0.899 | 10634 | 3.47E-03 | 1.22 | 0.916 | 1700 | 9.37E-04 | 1.24 | 0.917 | 2034 |
| C01 | 113692475 | rs1217407 | A | 0.249 | 10631 | 3.56E-03 | 1.14 | 0.274 | 1700 | 1.20E-03 | 1.14 | 0.275 | 2034 |
| C02 | 36037410 | rs10203034 | A | 0.68 | 10634 | 3.64E-03 | 1.13 | 0.706 | 1700 | 2.27E-03 | 1.13 | 0.706 | 2034 |
| C01 | 113628929 | rs2797409 | C | 0.249 | 10634 | 3.79E-03 | 1.14 | 0.274 | 1700 | 1.29E-03 | 1.14 | 0.275 | 2034 |
| C12 | 69325443 | rs7978668 | T | 0.918 | 10635 | 3.84E-03 | 1.25 | 0.933 | 1700 . | 1.61E-03 | 1.25 | 0.933 | 2034 |
| C12 | 69326403 | rs767760 | A | 0.914 | 10635 | 3.96E-03 | 1.24 | 0.93 | 1700 | 1.10E-03 | 1.25 | 0.93 | 2034 |
| C12 | 69368222 | rs949664 | A | 0.863 | 10632 | 3.98E-03 | 1.19 | 0.882 | 1700 | 1.46E-03 | 1.19 | 0.882 | 2034 |
| C12 | 69340682 | rs7309888 | C | 0.794 | 10631 | 4.11E-03 | 1.15 | 0.816 | 1700 | 1.40E-03 | 1.16 | 0.817 | 2034 |
| C14 | 98160936 | rs12435918 | G | 0.333 | 10635 | 4.43E-03 | 1.12 | 0.36 | 1700 | 2.64E-03 | 1.12 | 0.359 | 2034 |
| C07 | 11669876 | rs2355129 | A | 0.362 | 10635 | 4.59E-03 | 1.12 | 0.389 | 1699 | . 1.13E-03 | 1.13 | 0.391 | 2033 |
| C08 | 129057022 | rs2648896 | C | 0.149 | 10633 | 5.75E-03 | 1.16 | 0.169 | 1700 | 2.49E-03 | 1.16 | 0.169 | 2034 |
| C12 | 69347673 | rs7976576 | T | 0.794 | 10635 | 6.31E-03 | 1.15 | 0.816 | 1700 | 2.22E-03 | 1.15 | 0.816 | 2034 |
| C07 | 11658292 | rs10215881 | C | 0.494 | 10630 | 8.18E-03 | 1.11 | 0.52 | 1699 | 1.88E-03 | 1.12 | 0.523 | 2033 |
| C12 | 124462726 | rs7310042 | G | 0.193 | 10634 | 8.35E-03 | 1.14 | 0.214 | 1700 | 2.55E-03 | 1.15 | 0.215 | 2034 |
| C07 | 11646149 | rs6460851 | G | 0.288 | 10624 | 8.43E-03 | 1.12 | 0.312 | 1698 | 1.88E-03 | 1.13 | 0.314 | 2031 |
| C02 | 128444077 | rs4662743 | T | 0.515 | 10622 | 1.30E-02 | 1.1 | 0.539 | 1700 | 2.49E-03 | 1.12 | 0.543 | 2034 |

**Table 2. Association results for the Icelandic discovery cohorts, and the replication cohorts. The columns in the table indicate (1) the cohort, (2) rs name of the marker in question, (3) the chromosome, (4) position of the marker in NCBI Build 34, (5) p-value of the association, (6) the Relative Risk of the association, (7) the number of MI patients, (8) the frequency of the associating allele in patients, (9) the number of controls, (10) the frequency of the associating allele in controls, (11) the associating allele.**

| **Cohort** | **rs-name** | **chr** | **position** | **p-value** | **RR** | **Naff** | **f(aff)** | **Ncon** | **f(con)** | **allele** |
|---|---|---|---|---|---|---|---|---|---|---|
| | | | | | | | | | | |
| **SG01S1480** | | **C01** | **57254228** | | | | | | | |
| Iceland discovery group | rs2991515 | | | 0.001045 | 1.14 | 1642 | 0.514 | 6868 | 0.480 | T |
| Iceland replication group | rs2991515 | | | 0.054380 | 1.13 | 560 | 0.507 | 3393 | 0.476 | T |
| Iceland All | rs2991515 | | | 0.000108 | 1.14 | 2202 | 0.512 | 10261 | 0.479 | T |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.054379 | 1.13 | 544 | | | | T |
| All groups | | | | 0.000108 | 1.14 | 2016 | | | | T |
| | | | | | | | | | | |
| **SG01S1340** | | **C01** | **113676295** | | | | | | | |
| Iceland discovery group | rs2476601 | | | 0.000452 | 1.23 | 1632 | 0.156 | 6858 | 0.130 | A |
| Iceland replication group | rs2476601 | | | 0.001253 | 1.34 | 562 | 0.167 | 3393 | 0.130 | A |
| Iceland All | rs2476601 | | | 0.000003 | 1.26 | 2194 | 0.159 | 10251 | 0.130 | A |
| Philadelphia | | | | NA | | | | | | |
| Atlanta | | | | NA | | | | | | |
| Durham | rs2476601 | | | 0.407813 | 1.12 | 932 | 0.095 | 544 | 0.086 | A |
| All US groups | | | | 0.407813 | 1.1164 | 932 | | | | A |
| All replication groups | | | | 0.001741 | 1.2678 | 1478 | | | | A |
| All groups | | | | 0.000003 | 1.2404 | 2941 | | | | A |
| | | | | | | | | | | |
| **SG02S870** | | **C02** | **36022756** | | | | | | | |
| Iceland discovery group | rs6747236 | | | 0.004309 | 1.14 | 1634 | 0.743 | 6863 | 0.717 | T |
| Iceland replication group | rs6747236 | | | 0.101400 | 1.13 | 544 | 0.741 | 3391 | 0.717 | T |
| Iceland All | rs6747236 | | | 0.000905 | 1.14 | 2178 | 0.742 | 10254 | 0.717 | T |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | 0.101403 | 0.101403 | 1.13 | 528 | | | | T |
| All groups | | | | 0.000905 | 1.14 | 1994 | | | | T |
| | | | | | | | | | | |
| **SG02S804** | | **C02** | **176947426** | | | | | | | |
| Iceland discovery group | rs921083 | | | 0.000163 | 1.21 | 1642 | 0.806 | 6867 | 0.774 | G |
| Iceland replication group | rs921083 | | | 0.054180 | 1.17 | 549 | 0.805 | 3392 | 0.779 | G |
| Iceland All | rs921083 | | | 0.000025 | 1.20 | 2191 | 0.806 | 10259 | 0.776 | G |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.054178 | 1.17 | 533 | | | | G |
| All groups | | | | 0.000025 | 1.20 | 2006 | | | | G |
| | | | | | | | | | | |
| **SG02S832** | | **C02** | **212588265** | | | | | | | |
| Iceland discovery group | rs12329252 | | | 0.000680 | 1.16 | 1641 | 0.710 | 6868 | 0.677 | T |
| Iceland replication qroup | rs12329252 | | | 0.327900 | 1.07 | 554 | 0.692 | 3393 | 0.677 | T |
| Iceland All | rs12329252 | | | 0.000500 | 1.14 | 2195 | 0.705 | 10261 | 0.677 | T |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.327938 | 1.07 | 538 | | | | T |
| All groups | | | | 0.000500 | 1.14 | 2010 | | | | T |
| | | | | | | | | | | |
| **SG03S670** | | **C03** | **62420156** | | | | | | | |
| Iceland discovery group | rs9874646 | | | 0.000330 | 1.18 | 1642 | 0.746 | 6867 | 0.713 | T |
| Iceland replication group | rs9874646 | | | 0.173800 | 1.10 | 555 | 0.721 | 3393 | 0.700 | T |
| Iceland All | rs9874646 | | | 0.000078 | 1.17 | 2197 | 0.739 | 10260 | 0.709 | T |
| Philadelphia | | | | NA | | | | | | |
| Atlanta | | | | NA | | | | | | |
| Durham | | | | NA | | | | | | |
| All US qroups | | | | NA | NA | NA | | | | |
| All replication groups | | | | 0.173753 | 1.10 | 539 | | | | T |
| All groups | | | | 0.000078 | 1.17 | 2012 | | | | T |
| | | | | | | | | | | |
| **SG03S671** | | **C03** | **191533619** | | | | | | | |
| Iceland discovery group | rs9864293 | | | 0.007956 | 1.12 | 1624 | 0.418 | 6866 | 0.391 | C |
| Iceland replication group | rs9864293 | | | 0.143800 | 1.22 | 119 | 0.424 | 3392 | 0.377 | C |
| Iceland All | rs9864293 | | | 0.000591 | 1.14 | 1743 | 0.419 | 10258 | 0.386 | C |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.143805 | 1.22 | 115 | | | | C |
| All groups | | | | 0.000591 | 1.14 | 1596 | | | | C |
| | | | | | | | | | | |
| **SG04S816** | | **C04** | **21568604** | | | | | | | |
| Iceland discovery group | rs4282162 | | | 0.000562 | 1.15 | 1641 | 0.565 | 6867 | 0.529 | T |
| Iceland replication group | rs4282162 | | | 0.221000 | 1.08 | 559 | 0.547 | 3393 | 0.527 | T |
| Iceland All | rs4282162 | | | 0.000244 | 1.14 | 2200 | 0.560 | 10260 | 0.528 | T |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.221045 | 1.08 | 543 | | | | T |
| All groups | | | | 0.000244 | 1.14 | 2014 | | | | T |
| | | | | | | | | | | |
| **SG04S820** | | **C04** | **45948792** | | | | | | | |
| Iceland discovery group | rs7661204 | | 0.034390 | 0.034390 | 1.09 | 1641 | 0.576 | 6868 | 0.555 | C |
| Iceland replication group | rs7661204 | | | 0.026880 | 1.16 | 562 | 0.583 | 3393 | 0.547 | C |

| **Cohort** | **rs-name** | **chr** | **position** | **p-value** | **RR** | **Naff** | **f(aff)** | **Ncon** | **f(con** | **allele** |
|---|---|---|---|---|---|---|---|---|---|---|
| Iceland All | rs7661204 | | | 0.002633 | 1.11 | 2203 | 0.578 | 10261 | 0.552 | C |
| Philadelphia | | | | NA | | | | | | |
| Atlanta | | | | NA | | | | | | |
| Durham | | | | NA | | | | | | |
| All US groups | | | | NA | NA | NA | | | | |
| All replication qroups | | | | 0.026881 | 1.16 | 546 | | | | C |
| All qroups | | | | 0.002633 | 1.11 | 2017 | | | | C |
| | | | | | | | | | | |
| **SG05S3061** | | **C05** | **118135750** | | | | | | | |
| Iceland discovery group | rs728676 | | | 0.003297 | 1.13 | 1641 | 0.470 | 6868 | 0.440 | T |
| Iceland replication group | rs728676 | | | 0.011820 | 1.18 | 561 | 0.482 | 3392 | 0.441 | T |
| Iceland All | rs728676 | | | 0.000145 | 1.14 | 2202 | 0.473 | 10260 | 0.440 | T |
| Philadelphia | | | | NA | | | | | | |
| Atlanta | rs728676 | | | 0.101514 | 1.14 | 464 | 0.498 | 1012 | 0.465 | T |
| Durham | rs728676 | | | 0.596462 | 0.97 | 1168 | 0.484 | 718 | 0.493 | T |
| All US groups | | | | 0.513855 | 1.03 | 1632 | | | | T |
| All replication groups | | | | 0.038993 | 1.09 | 2177 | | | | T |
| All groups | | | | 0.000449 | 1.11 | 3648 | | | | T |
| | | | | | | | | | | |
| **SG05S3058** | | **C05** | **158504417** | | | | | | | |
| Iceland discovery group | rs7442701 | | | 0.000114 | 1.22 | 1625 | 0.208 | 6865 | 0.176 | T |
| Iceland replication group | rs7442701 | | | 0.913600 | 1.02 | 120 | 0.175 | 3390 | 0.172 | T |
| Iceland All | rs7442701 | | | 0.000045 | 1.22 | 1745 | 0.205 | 10255 | 0.175 | T |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.913822 | 1.02 | 116 | | | | T |
| All groups | | | | 0.000045 | 1.22 | 1598 | | | | T |
| | | | | | | | | | | |
| **SG06S1298** | | **C06** | **29516393** | | | | | | | |
| Iceland discovery group | rs2074464 | | | 0.002492 | 1.13 | 1633 | 0.475 | 6862 | 0.444 | T |
| Iceland replication group | rs2074464 | | | 0.191700 | 1.09 | 545 | 0.466 | 3390 | 0.445 | T |

| **Cohort** | **rs-name** | **chr** | **position** | **p-value** | **RR** | **Naff** | **f(aff)** | **Ncon** | **f(con)** | **allele** |
|---|---|---|---|---|---|---|---|---|---|---|
| Iceland All | rs2074464 | | | 0.000945 | 1.12 | 2178 | 0.473 | 10252 | 0.444 | T |
| Philadelphia | | | | NA | | | | | | |
| Atlanta | rs2074464 | | | 0.076812 | 1.18 | 365 | 0.484 | 576 | 0.442 | T |
| Durham | rs2074464 | | | 0.290169 | 1.07 | 1200 | 0.469 | 728 | 0.451 | T |
| All US groups | | | | 0.059737 | 1.11 | 1565 | | | | T |
| All replication groups | | | | 0.022395 | 1.10 | 2094 | | | | T |
| All groups | | | | 0.000145 | 1.12 | 3559 | | | | T |
| | | | | | | | | | | |
| **SG07S302** | | **C07** | **116579323** | | | | | | | |
| Iceland discovery group | rs1029396 | | | 0.001065 | 1.28 | 1634 | 0.089 | 6867 | 0.071 | G |
| Iceland replication group | rs1029396 | | 0.719800 | 0.719800 | 0.95 | 548 | 0.063 | 3393 | 0.066 | G |
| Iceland All | rs1029396 | | | 0.003796 | 1.21 | 2182 | 0.082 | 10260 | 0.069 | G |
| Philadelphia | | | | NA | | | | | | |
| Atlanta | rs10Z9396 | | | 0.391029 | 1.15 | 363 | 0.094 | 572 | 0.082 | G |
| Durham | rs1029396 | | | 0.053907 | 1.25 | 1212 | 0.101 | 735 | 0.083 | G |
| All US groups | | | | 0.038030 | 1.22 | 1575 | | | | G |
| All replication qroups | | | | 0.138284 | 1.12 | 2107 | | | | G |
| All qroups | | | | 0.000370 | 1.21 | 3573 | | | | G |
| | | | | | | | | | | |
| **SG08S1314** | | **C08** | **18899082** | | | | | | | |
| Iceland discovery qroup | rs334198 | | | 0.020830 | 1.19 | 1630 | 0.090 | 6865 | 0.076 | A |
| Iceland replication group | rs334198 | | | 0.059380 | 1.25 | 549 | 0.089 | 3393 | 0.073 | A |
| Iceland All | rs334198 | | | 0.002535 | 1.21 | 2179 | 0.089 | 10258 | 0.075 | A |
| Philadelphia | rs334198 | | | 0.370833 | 0.87 | 543 | 0.088 | 475 | 0.100 | A |
| Atlanta | rs334198 | | | 0.778416 | 1.05 | 367 | 0.086 | 542 | 0.082 | A |
| Durham | | | | NA | | | | | | |
| All US groups | | | | 0.627319 | 0.95 | 910 | | | | A |
| All replication groups | | | | 0.348780 | 1.08 | 1443 | | | | A |
| All groups | | | | 0.016141 | 1.14 | 2905 | | | | A |
| | | | | | | | | | | |
| **SG08S1315** | | **C08** | **28215758** | | | | | | | |
| Iceland discovery group | rs4732849 | | | 0.000349 | 1.18 | 1641 | 0.306 | 6868 | 0.273 | T |
| Iceland replication group | r4732849 | | 0.077900 | 0.077900 | 1.14 | 550 | 0.298 | 3393 | 0.272 | T |
| Iceland All | rs4732849 | | | 0.000056 | 1.17 | 2191 | 0.304 | 10261 | 0.273 | T |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.077897 | 1.14 | 534 | | | | T |
| All groups | | | | 0.000056 | 1.17 | 2006 | | | | T |
| | | | | | | | | | | |
| **SG08S1325** | | **C08** | **129065286** | | | | | | | |
| Iceland discovery group | rs2302793 | | | 0.002452 | 1.14 | 1614 | 0.462 | 6846 | 0.431 | G |
| Iceland replication group | rs2302793 | | | 0.601500 | 1.09 | 87 | 0.454 | 3370 | 0.434 | G |
| Iceland All | rs2302793 | | | 0.001920 | 1.13 | 1701 | 0.461 | 10216 | 0.432 | G |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.601476 | 1.09 | 84 | | | | G |
| All groups | | | | 0.001920 | 1.13 | 1557 | | | | G |
| | | | | | | | | | | |
| **SG08S1326** | | **C08** | **129067370** | | | | | | | |
| Iceland discovery group | rs3931282 | | | 0.001650 | 1.14 | 1634 | 0.461 | 6868 | 0.428 | C |
| Iceland replication group | rs3931282 | | | 0.050490 | 1.14 | 547 | 0.463 | 3393 | 0.431 | C |
| Iceland All | rs3931282 | | | 0.000216 | 1.14 | 2181 | 0.461 | 10261 | 0.429 | C |
| Philadelphia | | | | NA | | | | | | |
| Atlanta | rs3931282 | | | 0.205449 | 0.89 | 362 | 0.438 | 587 | 0.468 | C |
| Durham | rs3931282 | | | 0.208270 | 0.92 | 1196 | 0.455 | 732 | 0.476 | C |
| All US groups | | | | 0.078904 | 0.91 | 1558 | | | | C |
| All replication groups | | | | 0.904130 | 0.99 | 2089 | | | | C |
| All groups | | | | 0.030734 | 1.07 | 3555 | | | | C |
| | | | | | | | | | | |
| **SG10S822** | | **C10** | **45239070** | | | | | | | |
| Iceland discovery group | rs2279434 | | | 0.000031 | 1.38 | 1634 | 0.088 | 6868 | 0.065 | T |
| Iceland replication group | rs2279434 | | | 0.509900 | 1.09 | 544 | 0.071 | 3392 | 0.065 | T |
| Iceland All | rs2279434 | | | 0.000053 | 1.31 | 2178 | 0.083 | 10260 | 0.065 | T |
| Philadelphia | rs2279434 | | | | 0.81 | 532 | 0.081 | 472 | 0.097 | T |
| Atlanta | rs2279434 | | | | 0.83 | 362 | 0.070 | 513 | 0.084 | T |
| Durham | | | | NA | | | | | | |
| All US groups | | | | 0.096180 | 0.82 | 894 | | | | T |
| All replication groups | | | | 0.422694 | 0.93 | 1422 | | | | T |
| All groups | | | | 0.006627 | 1.17 | 2888 | | | | T |
| | | | | | | | | | | |
| **SG10S314** | | **C10** | **90667504** | | | | | | | |
| Iceland discovery group | rs1412444 | | | 0.000082 | 1.18 | 1644 | 0.396 | 6861 | 0.357 | A |
| Iceland replication group | rs1412444 | | | 0.312500 | 1.07 | 582 | 0.374 | 3389 | 0.358 | A |
| Iceland All | rs1412444 | | | 0.000076 | 1.15 | 2226 | 0.390 | 10250 | 0.357 | A |
| Philadelphia | | | | NA | | | | | | |
| Atlanta | rs1412444 | | | 0.314043 | 1.11 | 367 | 0.356 | 581 | 0.333 | A |
| Durham | rs1412444 | | | 0.913736 | 1.02 | 257 | 0.368 | 121 | 0.364 | A |
| All US groups | | | | 0.360084 | 1.08 | 624 | | | | A |
| All replication groups | | | | 0.173888 | 1.07 | 1189 | | | | A |
| All groups | | | | 0.000063 | 1.14 | 2662 | | | | A |
| | | | | | | | | | | |
| **SG12S844** | | **C12** | **21092930** | | | | | | | |
| Iceland discovery group | rs11045689 | | | 0.011540 | 1.12 | 1630 | 0.321 | 6854 | 0.297 | G |
| Iceland replication group | rs11045689 | | | 0.133900 | 1.11 | 541 | 0.318 | 3386 | 0.295 | G |
| Iceland All | rs11045689 | | | 0.002878 | 1.12 | 2171 | 0.320 | 10240 | 0.296 | G |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.133868 | 1.11 | 526 | | | | G |
| All groups | | | | 0.002878 | 1.12 | 1988 | | | | G |
| | | | | | | | | | | |
| **SG14S801** | | **C14** | **67308303** | | | | | | | |
| Iceland discovery group | rs8003722 | | | 0.000343 | 1.26 | 1642 | 0.127 | 6867 | 0.104 | A |
| Iceland replication group | rs8003722 | | | 0.007542 | 1.32 | 561 | 0.125 | 3393 | 0.098 | A |
| Iceland All | rs8003722 | | | 0.000006 | 1.28 | 2203 | 0.126 | 10260 | 0.102 | A |
| Philadelphia | | | | NA | | | | | | |
| Atlanta | rs8003722 | | | 0.734831 | 1.04 | 472 | 0.151 | 1029 | 0.147 | A |
| Durham | rs8003722 | | | 0.431112 | 0.93 | 1202 | 0.135 | 735 | 0.144 | A |
| All US groups | | | | 0.708199 | 0.97 | 1674 | | | | |
| All replication groups | | | | 0.225792 | 1.08 | 2219 | | | | A |
| All groups | | | | 0.000670 | 1.16 | 3691 | | | | A |
| | | | | | | | | | | |
| **SG14S796** | | **C14** | **98131730** | | | | | | | |
| Iceland discovery group | rs7158073 | | | 0.000697 | 1.15 | 1630 | 0.461 | 6835 | 0.426 | T |
| Iceland replication group | rs7158073 | | | 0.017800 | 1.17 | 546 | 0.477 | 3384 | 0.438 | T |
| *Iceland All* | rs7158073 | | | 0.000055 | 1.15 | 2176 | 0.465 | 10219 | . 0.430 | T |
| Philadelphia | | | | NA | | | | | | |
| Atlanta | rs7158073 | | | 0.647425 | 1.05 | 362 | 0.419 | 575 | 0.408 | T |
| Durham | rs7158073 | | | 0.059831 | 1.14 | 1174 | 0.460 | 719 | 0.429 | T |
| All US groups | | | | 0.071211 | 1.10 | 1536 | | | | T |
| All replication groups | | | | 0.003703 | 1.13 | 2066 | | | | T |
| All groups | | | | 0.000012 | 1.14 | 3528 | | | | T |
| | | | | | | | | | | |
| **SG14S794_SG14S795*** | | **C14** | **52400957** | | | | | | | |
| Iceland discovery group | rs4444235 | rs1957860 | | 0.000323 | 0.84 | 1642 | 0.290 | 6868 | 0.326 | C |
| Iceland replication group | rs4444235 | rs1957860 | | 0.015250 | 0.83 | 561 | 0.279 | 3393 | 0.317 | C |
| Iceland All | rs4444235 | rs1957860 | | 0.000020 | 0.84 | 2203 | 0.287 | 10261 | 0.323 | C |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | rs4444235 | rs1957860 | | 0.015245 | 0.83 | 545 | | | | C |
| All groups | rs4444235 | rs1957860 | | 0.000020 | 0.84 | 2017 | | | | C |
| | | | | | | | | | | |
| **SG15S142** | | **C15** | **24926452** | | | | | | | |
| Iceland discovery qroup | rs2110209 | | | 0.001794 | 1.32 | 1634 | 0.063 | 6868 | 0.049 | A |
| Iceland replication group | rs2110209 | | | 0.036840 | 1.36 | 545 | 0.059 | 3393 | 0.044 | A |
| Iceland All | rs2110209 | | | 0.000106 | 1.34 | 2179 | 0.062 | 10261 | 0.047 | A |
| Philadelphia | | | | NA | | | | | | |
| Atlanta | | | | NA | | | | | | |
| Durham | | | | NA | | | | | | |
| All US groups | | | | NA | NA | NA | | | | |
| All replication groups | | | | 0.036835 | 1.36 | 529 | | | | A |
| All groups | | | | 0.000105 | 1.34 | 1995 | | | | A |
| | | | | | | | | | | |
| **SG17S742** | | **C17** | **17934326** | | | | | | | |
| Iceland discovery group | rs9902941 | | | 0.000041 | 1.19 | 1642 | 0.388 | 6867 | 0.348 | C |
| Iceland replication group | rs9902941 | | | 0.961500 | 1.00 | 565 | 0.351 | 3393 | 0.351 | C |
| Iceland All | rs9902941 | | | 0.000278 | 1.14 | 2207 | 0.379 | 10260 | 0.349 | C |
| Philadelphia | rs9902941 | | | 0.170324 | 1.13 | 542 | 0.423 | 474 | 0.393 | C |
| Atlanta | rs9902941 | | | 0.246078 | 1.09 | 591 | 0.372 | 1210 | 0.352 | C |
| Durham | rs9902941 | | | 0.163124 | 1.10 | 1165 | 0.378 | 736 | 0.355 | C |
| All US groups | | | | 0.024495 | 1.10 | 2298 | | | | C |
| All replication groups | | | | 0.055496 | 1.07 | 2847 | | | | C |
| All groups | | | | 0.000023 | 1.13 | 4319 | | | | C |
| | | | | | | | | | | |
| **SG17S743** | | **C17** | **17943470** | | | | | | | |
| Iceland discovery group | rs4925119 | | | 0.000093 | 1.18 | 1630 | 0.384 | 6857 | 0.345 | A |
| Iceland replication group | rs4925119 | | | 0.975100 | 1.00 | 541 | 0.349 | 3384 | 0.349 | A |
| Iceland All | rs4925119 | | | 0.000529 | 1.13 | 2171 | 0.375 | 10241 | 0.346 | A |
| Philadelphia | rs4925119 | | | 0.312163 | 1.10 | 537 | 0.419 | 470 | 0.397 | A |
| Atlanta | rs4925119 | | | 0.155471 | 1.11 | 646 | 0.372 | 1207 | 0.349 | A |
| Durham | rs4925119 | | | 0.139467 | 1.11 | 1189 | 0.373 | 729 | 0.350 | A |
| All US groups | | | | 0.022416 | 1.10 | 2372 | | | | A |
| All replication groups | | | | 0.051193 | 1.07 | 2898 | | | | A |
| All groups | | | | 0.000037 | 1.12 | 4360 | | | | A |
| | | | | | | | | | | |
| **SG17S744** | | **C17** | **17947855** | | | | | | | |
| Iceland discovery group | rs3183702 | | | 0.000036 | 1.19 | 1633 | 0.389 | 6866 | 0.348 | T |
| Iceland replication group | rs3183702 | | | 0.970600 | 1.00 | 546 | 0.352 | 3391 | 0.351 | T |
| Iceland All | rs3183702 | | | 0.000241 | 1.14 | 2179 | 0.380 | 10257 | 0.349 | T |
| Philadelphia | rs3183702 | | | 0.132368 | 1.15 | 531 | 0.427 | 469 | 0.393 | T |
| Atlanta | rs3183702 | | | 0.018991 | 1.26 | 362 | 0.391 | 539 | 0.337 | T |
| Durham | rs3183702 | | | 0.469038 | 1.06 | 939 | 0.368 | 572 | 0.355 | T |
| All US groups | | | | 0.011987 | 1.14 | 1832 | | | | T |
| All replication groups | | | | 0.041110 | 1.09 | 2362 | | | | T |
| All groups | | | | 0.000009 | 1.14 | 3827 | | | | T |
| | | | | | | | | | | |
| **SG17S746** | | **C17** | **18035257** | | | | | | | |
| Iceland discovery group | rs6502622 | | | 0.000128 | 1.18 | 1641 | 0.397 | 6868 | 0.359 | G |
| Iceland replication group | rs6502622 | | | 0.699700 | 1.03 | 560 | 0.366 | 3393 | 0.360 | G |
| Iceland All | rs6502622 | | | 0.000347 | 1.14 | 2201 | 0.389 | 10261 | 0.359 | G |
| Philadelphia | rs6502622 | | | 0.277357 | 1.10 | 540 | 0.433 | 475 | 0.409 | G |
| Atlanta | rs6502622 | | | 0.041642 | 1.16 | 593 | 0.401 | 1227 | 0.366 | G |
| Durham | rs6502622 | | | 0.180470 | 1.10 | 1165 | 0.399 | 734 | 0.377 | G |
| All US groups | | | | 0.009300 | 1.12 | 2298 | | | | G |
| All replication groups | | | | 0.016554 | 1.09 | 2842 | | | | G |
| All groups | | | | 0.000010 | 1.13 | 4313 | | | | G |
| | | | | | | | | | | |
| **SG17S750** | | **C17** | **18149041** | | | | | | | |
| Iceland discovery group | rs2955355 | | | 0.000393 | 1.17 | 1634 | 0.319 | 6868 | 0.285 | C |
| Iceland replication group | rs2955355 | | | 0.393900 | 1.06 | 547 | 0.303 | 3393 | 0.290 | C |
| Iceland All | rs2955355 | | | 0.000440 | 1.14 | 2181 | 0.315 | 10261 | 0.287 | C |
| Philadelphia | rs2955355 | | | 0.630066 | 1.05 | 544 | 0.362 | 476 | 0.352 | C |
| Atlanta | rs2955355 | | | 0.002362 | 1.37 | 360 | 0.360 | 534 | 0.291 | C |
| Durham | | | | NA | | | | | | |
| All US groups | | | | 0.016778 | 1.18 | 904 | | | | C |
| All replication groups | | | | 0.020472 | 1.12 | 1435 | | | | C |
| All groups | | | | 0.000023 | 1.15 | 2901 | | | | C |
| | | | | | | | | | | |
| **SG17S747** | | **C17** | **18204411** | | | | | | | |
| Iceland discovery group | rs854813 | | | 0.000154 | 1.17 | 1634 | 0.425 | 6865 | 0.387 | C |
| Iceland replication group | rs854813 | | | 0.964800 | 1.00 | 543 | 0.386 | 3393 | 0.387 | C |
| Iceland All | rs854813 | | | 0.000785 | 1.13 | 2177 | 0.415 | 10258 | 0.387 | C |
| Philadelphia | rs854813 | | | 0.077440 | 1.17 | 520 | 0.468 | 462 | 0.429 | C |
| Atlanta | rs854813 | | | 0.009373 | 1.29 | 364 | 0.449 | 539 | 0.388 | C |
| Durham | | | | NA | | | | | | |
| All US groups | | | | 0.002182 | 1.23 | 884 | | | | C |
| All replication groups | | | | 0.030822 | 1.11 | 1411 | | | | C |
| All groups | | | | 0.000011 | 1.15 | 2877 | | | | C |
| | | | | | | | | | | |
| **SG17S749** | | **C17** | **18231090** | | | | | | | |
| Iceland discovery group | rs854793 | | | 0.000495 | 1.19 | 1634 | 0.231 | 6865 | 0.201 | A |
| Iceland replication qroup | rs854793 | | | 0.576100 | 0.95 | 491 | 0.198 | 3392 | 0.205 | A |
| Iceland All | rs854793 | | | 0.004364 | 1.13 | 2125 | 0.223 | 10257 | 0.203 | A |
| Philadelphia | rs854793 | | | 0.456629 | 1.08 | 505 | 0.260 | 454 | 0.246 | A |
| Atlanta | rs854793 | | | 0.040285 | 1.26 | 369 | 0.255 | 539 | 0.213 | A |
| Durham | | | | NA | | | | | | |
| All US groups | | | | 0.052002 | 1.16 | 874 | | | | A |
| All replication groups | | | | 0.283691 | 1.06 | 1351 | | | | A |
| All groups | | | | 0.000591 | 1.14 | 2820 | | | | A |
| | | | | | | | | | | |
| **SG17S748** | | **C17** | **18235585** | | | | | | | |
| Iceland discovery group | rs854787 | | | 0.000005 | 1.21 | 1635 | 0.444 | 6816 | 0.398 | C |
| Iceland replication group | rs854787 | | | 0.580200 | 0.96 | 565 | 0.390 | 3379 | 0.399 | C |
| Iceland all | rs854787 | | | 0.000171 | 1.14 | 2200 | 0.430 | 10195 | 0.398 | C |
| Philadelphia | rs854787 | | | 0.241799 | 1.11 | 542 | 0.458 | 475 | 0.433 | C |
| Atlanta | rs854787 | | | 0.001746 | 1.36 | 360 | 0.475 | 507 | 0.399 | C |
| Durham | | | | NA | | | | | | |
| All US groups | | | | 0.002955 | 1.22 | 902 | | | | C |
| All replication groups | | | | 0.085873 | 1.08 | 1451 | | | | C |
| All groups | | | | 0.000002 | 1.16 | 2916 | | | | C |

| | | | | | | | | | | |
|---|---|---|---|---|---|---|---|---|---|---|
| *2-marker haplotype consisting of markers SG14S794 and SG14S795 | | | | | | | | | | |

### Example 2.

Further genome-wide genotyping of Icelandic patients diagnosed with coronary artery disease and/or myocardial infarction was performed. Recruitment and phenotyping was described above under Example, and all methods involved were as described in the above.

Descriptions of the additional replication cohorts are as follows:
*Cohort from Italy:* The subjects from Verona were enrolled into the Verona Heart Project, which is an ongoing study aimed at identifying new risk factors for coronary artery disease (CAD) and myocardial infarction (MI) in a population of subjects with angiographic documentation of their coronary vessels. In brief, the CAD group had angiographically documented severe coronary atherosclerosis, the majority of them being candidates for coronary artery bypass grafting or percutaneous coronary intervention. Control subjects were selected such that they had normal coronary arteries, being submitted to coronary angiography for reasons other than CAD. Controls with history or clinical evidence of atherosclerosis in vascular districts beyond the coronary bed were excluded. Information on MI diagnoses was gathered through medical records showing diagnostic electrocardiogram and enzyme changes, and/or the typical sequelae of MI on ventricular angiography. The study was approved by local Ethical Committee. Informed consent was obtained from all the patients after a full explanation of the study.
*Cohort from Johns Hopkins:* The study group from Baltimore is comprised of The Johns Hopkins University Sibling and Family Heart Study, G-Cal, and GeneSTAR studies. The subjects consisted of families identified from 1983-2006 from probands with premature coronary disease event (before 60 years of age). Probands with documented CAD were identified at the time of hospitalization in any of the Baltimore area hospitals. Their apparently healthy siblings without CAD were recruited as well as adult offspring and spouses. Of probands, generally 24% had an MI as their index event, 65% had a PCI and/or CABG, 8.4% had angina with angiography documented CAD and were treated medically, 1.7% had sudden cardiac death, and 1.7% had angiographic documented coronary stenosis with an abnormal stress test and no symptoms.
*Cohort from WTCCC:* This study cohort has been described previously (Nature 447:661-78 (2007)). Summary statistics from a genome-wide association study performed on these samples is publicly available at http://www.wtccc.org.uk/info/summary_stats.shtml.

Reanalysis of the markers presented in Table 1 was performed using the expanded dataset of 3,047 individuals diagnosed with coronary artery disease, 2,395 diagnosed with myocardial infarction, and 28,321 population controls. The results are presented in Table 3.

The top 100 markers in the scan were explored further by replication analysis in six cohorts from Durham (North Carolina, US), Philadelphia (Pennsylvania, US), Atlanta (Georgia, US), John Hopkins University (Maryland, US), Italy, and in a dataset from the Wellcom Trust Case Control Consortium (WTCCC; England). The replication cohorts combined comprise 6,108 cases and 19,396 controls. The results of the replication analysis is shown in Table 4A - 4H, both separately for each cohort and a combined analysis for all the replication cohorts, as well as a combined for all the cohorts, including the Icelandic discovery cohort. Many of the variants discovered in the Icelandic discovery cohort show overall significant association in the combined replication groups. The effects are modest, which explains why some cohorts do not indicate significant association, while other cohorts do so.

In the combined analysis of all the groups (9,155 cases; 47,717 controls; Table 4H), marker rs11751605 stands out as the most significant, with a combined P-value of 1.16 x 10⁻⁸. This is a highly significant number, and in fact represents statistical significance at the genome-wide level, after applying the conservative Bonferroni correction for the number of statistical tests performed (approx. 300,000 in the combined analysis). It should be noted that the number of tests performed in the replication effort is much smaller; consequently, the correction to be applied to correct for the number of tests is smaller, or 100 (giving P-values of 0.05/100, or about 0.0005 as significant after correction). Several markers meet this threshold of significance.

**Table 3. Association results for SNPs showing association to myoardial infarction (MI) and coronary artery disease (CAD). The results are based on a genomic scan using 2,395 MI samples, 3,047 CAD samples, and 28,321 controls. The columns indicate (1) chromosome, (2) position in NCBI Build 36, (3) the associating allele (4), rs name of the SNP, (5) frequency of the associating allele in controls, (6) the number of controls, (7), the p-value for association to MI, (8) the Relative Risk of association to MI, (9) the frequency of the associating allele in MI patients, (10) the number of MI patients, (11) the p-value of association to CAD, (12) the Relative Risk of association to CAD, (13) the frequency of the associating allele in CAD patients, (14) the number of CAD patients.**

| **Chr** | **SNP** | **Pos in Build 36** | **Allele** | **f(con)** | **N(con)** | **P (MI)** | **RR(MI)** | **f(MI)** | **N(MI)** | **P (CAD)** | **RR (CAD)** | **f (CAD)** | **N (CAD)** |
|---|---|---|---|---|---|---|---|---|---|---|---|---|---|
| C01 | rs10462018 | 7802214 | C | 0.821 | 28185 | 1.40E-02 | 1.11 | 0.836 | 2390 | 1.20E-01 | 1.06 | 0.830 | 3040 |
| C01 | rs2946534 | 18647370 | T | 0.571 | 28302 | 3.40E-04 | 1.13 | 0.599 | 2394 | 4.50E-04 | 1.11 | 0.596 | 3046 |
| C01 | rs3748744 | 19828833 | G | 0.898 | 28280 | 6.30E-02 | 1.11 | 0.907 | 2394 | 3.30E-02 | 1.11 | 0.908 | 3046 |
| C01 | rs7523192 | 19835634 | T | 0.813 | 28296 | 7.10E-02 | 1.08 | 0.824 | 2395 | 1.50E-01 | 1.06 | 0.821 | 3047 |
| C01 | rs7535952 | 19846151 | A | 0.722 | 28166 | 6.60E-02 | 1.07 | 0.735 | 2355 | 6.40E-02 | 1.06 | 0.734 | 3003 |
| C01 | rs2072671 | 20788288 | C | 0.313 | 28310 | 0.0011 | 1.12 | 0.338 | 2395 | 1.30E-03 | 1.11 | 0.335 | 3047 |
| C01 | rs12724604 | 20948045 | A | 0.037 | 28248 | 9.80E-03 | 1.23 | 0.045 | 2386 | 2.10E-02 | 1.19 | 0.043 | 3037 |
| C01 | rs12741305 | 21192774 | C | 0.038 | 28320 | 0.0087 | 1.23 | 0.046 | 2393 | 2.30E-02 | 1.18 | 0.044 | 3044 |
| C01 | rs10799128 | 29476586 | T | 0.200 | 28211 | 0.062 | 1.08 | 0.212 | 2378 | 1.40E-01 | 1.06 | 0.208 | 3029 |
| C01 | rs1932397 | 29835784 | G | 0.822 | 28320 | 1.80E-03 | 1.15 | 0.841 | 2395 | 2.80E-03 | 1.13 | 0.839 | 3047 |
| C01 | rs407838 | 30585414 | C | 0.846 | 28311 | 0.0039 | 1.14 | 0.863 | 2395 | 4.70E-03 | 1.13 | 0.861 | 3047 |
| C01 | rs593993 | 30648371 | C | 0.838 | 28311 | 0.00099 | 1.16 | 0.858 | 2392 | 9.30E-04 | 1.15 | 0.856 | 3044 |
| C01 | rs12029866 | 30657936 | G | 0.775 | 28318 | 0.0043 | 1.12 | 0.794 | 2395 | 4.40E-03 | 1.11 | 0.792 | 3047 |
| C01 | rs12126748 | 31307750 | A | 0.346 | 27660 | 0.0018 | 1.11 | 0.371 | 2341 | 9.90E-03 | 1.08 | 0.365 | 2979 |
| C01 | r661221 | 40866417 | G | 0.805 | 28299 | 1.80E-02 | 1.10 | 0.820 | 2392 | 2.40E-01 | 1.05 | 0.811 | 3044 |
| C01 | rs7524868 | 40879361 | G | 0.306 | 28288 | 1.80E-02 | 1.09 | 0.324 | 2385 | 1.20E-01 | 1.05 | 0.316 | 3036 |
| C01 | rs7520394 | 40882456 | T | 0.649 | 28231 | 0.0014 | 1.12 | 0.674 | 2390 | 7.50E-02 | 1.06 | 0.662 | 3041 |
| C01 | rs2294512 | 54142894 | G | 0.688 | 28321 | 0.064 | 1.07 | 0.702 | 2395 | 7.00E-02 | 1.06 | 0.701 | 3047 |
| C01 | rs4927219 | 55524409 | G | 0.603 | 28319 | 4.50E-03 | 1.10 | 0.625 | 2395 | 3.80E-03 | 1.09 | 0.624 | 3047 |
| C01 | rs2991515 | 57656898 | T | 0.483 | 28319 | 0.0033 | 1.10 | 0.507 | 2395 | 7.70E-04 | 1.10 | 0.508 | 3047 |
| C01 | rs11208061 | 63165253 | A | 0.346 | 27897 | 0.0014 | 1.12 | 0.371 | 2375 | 6.10E-03 | 1.09 | 0.365 | 3012 |
| C01 | rs12134779 | 63168930 | T | 0.292 | 28320 | 0.0000094 | 1.17 | 0.325 | 2394 | 2.80E-04 | 1.12 | 0.316 | 3046 |
| C01 | rs6696619 | 63225377 | C | 0.673 | 28302 | 6.70E-02 | 1.07 | 0.687 | 2395 | 3.20E-01 | 1.03 | 0.680 | 3047 |
| C01 | rs1747924 | 64311549 | T | 0.810 | 28282 | 0.019 | 1.10 | 0.825 | 2395 | 0.23 | 1.05 | 0.817 | 3044 |
| C01 | rs2806535 | 64350358 | A | 0.483 | 28297 | 0.013 | 1.09 | 0.504 | 2394 | 0.0066 | 1.08 | 0.503 | 3043 |
| C01 | rs3818513 | 65646625 | C | 0.462 | 28320 | 0.049 | 1.07 | 0.477 | 2395 | 0.38 | 1.03 | 0.468 | 3047 |
| C01 | rs2025805 | 65722466 | C | 0.567 | 28315 | 0.023 | 1.08 | 0.585 | 2395 | 0.015 | 1.08 | 0.584 | 3047 |
| C01 | rs10489554 | 69921136 | T | 0.776 | 28311 | 0.0089 | 1.11 | 0.793 | 2395 | 0.038 | 1.08 | 0.788 | 3046 |
| C01 | rs10518531 | 76871826 | C | 0.845 | 28121 | 0.032 | 1.10 | 0.858 | 2359 | 0.06 | 1.08 | 0.855 | 3004 |
| C01 | rs1566250 | 76986952 | G | 0.703 | 28309 | 0.00084 | 1.13 | 0.728 | 2395 | 0.0011 | 1.11 | 0.725 | 3047 |
| C01 | rs1412414 | 78711365 | G | 0.620 | 28290 | 2.40E-03 | 1.11 | 0.644 | 2394 | 0.00059 | 1.11 | 0.645 | 3044 |
| C01 | rs1543675 | 78719467 | C | 0.380 | 28318 | 0.0013 | 1.11 | 0.405 | 2395 | 0.0036 | 1.09 | 0.401 | 3046 |
| C01 | rs1030414 | 81241030 | G | 0.902 | 28318 | 0.039 | 1.12 | 0.912 | 2395 | 0.046 | 1.11 | 0.911 | 3046 |
| C01 | rs7531507 | 81261795 | G | 0.922 | 28296 | 0.039 | 1.14 | 0.930 | 2394 | 0.021 | 1.14 | 0.931 | 3045 |
| C01 | rs12032817 | 82949953 | G | 0.375 | 28320 | 4.30E-03 | 1.10 | 0.398 | 2395 | 0.0046 | 1.09 | 0.396 | 3047 |
| C01 | rs1360903 | 86345051 | G | 0.508 | 28234 | 0.0055 | 1.09 | 0.531 | 2389 | 0.006 | 1.09 | 0.529 | 3040 |
| C01 | rs10801705 | 89272970 | A | 0.462 | 28318 | 0.0074 | 1.09 | 0.483 | 2394 | 0.018 | 1.07 | 0.479 | 3046 |
| C01 | rs4147861 | 94244536 | G | 0.922 | 28179 | 0.0019 | 1.22 | 0.935 | 2359 | 0.0012 | 1.21 | 0.934 | 3009 |
| C01 | rs10494048 | 106996582 | T | 0.381 | 28261 | 0.00089 | 1.12 | 0.407 | 2394 | 0.014 | 1.08 | 0.399 | 3044 |
| C01 | rs2335793 | 107945406 | C | 0.890 | 28309 | 0.006 | 1.16 | 0.904 | 2393 | 0.014 | 1.13 | 0.901 | 3045 |
| C01 | rs6688859 | 113577284 | T | 0.683 | 28320 | 0.19 | 1.05 | 0.693 | 2395 | 0.11 | 1.05 | 0.694 | 3047 |
| C01 | rs773586 | 113643259 | A | 0.488 | 28315 | 0.015 | 1.08 | 0.508 | 2395 | 0.0096 | 1.08 | 0.507 | 3047 |
| C01 | rs773588 | 113646347 | C | 0.282 | 28300 | 0.11 | 1.06 | 0.293 | 2394 | 0.023 | 1.08 | 0.297 | 3046 |
| C01 | rs2797409 | 114131725 | C | 0.248 | 28318 | 0.0024 | 1.12 | 0.269 | 2395 | 0.0036 | 1.10 | 0.266 | 3047 |
| C01 | rs2476601 | 114179091 | A | 0.129 | 28313 | 0.000031 | 1.21 | 0.152 | 2395 | 0.000097 | 1.18 | 0.148 | 3047 |
| C01 | rs1217407 | 114195271 | A | 0.247 | 28266 | 2.10E-03 | 1.12 | 0.269 | 2394 | 0.0031 | 1.10 | 0.266 | 3045 |
| C01 | rs1970568 | 149842380 | T | 0.815 | 28313 | 0.001 | 1.15 | 0.835 | 2395 | 0.021 | 1.09 | 0.828 | 3047 |
| C01 | rs6702842 | 149874720 | G | 0.272 | 28099 | 0.0072 | 1.10 | 0.292 | 2379 | 0.072 | 1.06 | 0.284 | 3029 |
| C01 | rs2495392 | 149943873 | G | 0.278 | 28312 | 0.0038 | 1.11 | 0.299 | 2395 | 0.052 | 1.07 | 0.291 | 3046 |
| C01 | rs12066445 | 150007044 | A | 0.302 | 28320 | 5.90E-04 | 1.13 | 0.327 | 2395 | 0.0094 | 1.09 | 0.319 | 3047 |
| C01 | rs9826 | 150045523 | G | 0.329 | 28106 | 0.0021 | 1.11 | 0.353 | 2377 | 0.03 | 1.07 | 0.344 | 3022 |
| C01 | rs949969 | 150059438 | T | 0.199 | 28317 | 0.016 | 1.10 | 0.214 | 2395 | 0.039 | 1.08 | 0.211 | 3047 |
| C01 | rs4845552 | 151746622 | G | 0.128 | 28269 | 0.0025 | 1.15 | 0.145 | 2393 | 0.00041 | 1.16 | 0.146 | 3044 |
| C01 | rs822519 | 154107330 | A | 0.935 | 28320 | 0.024 | 1.17 | 0.944 | 2395 | 0.025 | 1.15 | 0.943 | 3047 |
| C01 | rs12567958 | 154861280 | C | 0.265 | 28272 | 0.024 | 1.09 | 0.282 | 2392 | 0.075 | 1.06 | 0.277 | 3043 |
| C01 | rs4971177 | 156256292 | T | 0.914 | 28315 | 5.20E-04 | 1.24 | 0.929 | 2395 | 0.0015 | 1.19 | 0.927 | 3047 |
| C01 | rs2651860 | 163647679 | T | 0.785 | 28321 | 0.011 | 1.11 | 0.801 | 2395 | 0.012 | 1.10 | 0.800 | 3046 |
| C01 | rs11582943 | 169209225 | A | 0.941 | 28257 | 0.0019 | 1.26 | 0.953 | 2388 | 0.00098 | 1.25 | 0.952 | 3039 |
| C01 | rs12033847 | 174294601 | T | 0.460 | 27452 | 0.011 | 1.09 | 0.481 | 2360 | 0.0029 | 1.09 | 0.482 | 2999 |
| C01 | rs2175177 | 174450679 | A | 0.461 | 28011 | 6.20E-03 | 1.09 | 0.483 | 2386 | 0.0016 | 1.10 | 0.484 | 3030 |
| C01 | rs4651384 | 185685027 | G | 0.687 | 28319 | 0.0025 | 1.11 | 0.709 | 2395 | 0.028 | 1.07 | 0.702 | 3047 |
| C01 | rs2250227 | 185696690 | C | 0.337 | 28310 | 0.0058 | 1.10 | 0.358 | 2395 | 0.021 | 1.07 | 0.353 | 3047 |
| C01 | rs1339083 | 185819288 | A | 0.345 | 28315 | 0.01 | 1.09 | 0.365 | 2395 | 0.0018 | 1.10 | 0.367 | 3047 |
| C01 | rs2383658 | 185847926 | G | 0.394 | 28312 | 0.022 | 1.08 | 0.412 | 2395 | 0.0057 | 1.09 | 0.414 | 3047 |
| C01 | rs792446 | 210623301 | G | 0.617 | 28317 | 0.016 | 1.09 | 0.636 | 2395 | 0.052 | 1.06 | 0.631 | 3046 |
| C01 | rs11118175 | 217155936 | G | 0.077 | 28315 | 0.026 | 1.14 | 0.087 | 2395 | 0.0077 | 1.15 | 0.087 | 3047 |
| C01 | rs2642438 | 219036651 | G | 0.712 | 11355 | 0.0079 | 1.12 | 0.735 | 1767 | 0.083 | 1.07 | 0.726 | 2084 |
| C01 | rs1519463 | 219944762 | G | 0.508 | 28316 | 0.0096 | 1.09 | 0.529 | 2395 | 0.0035 | 1.09 | 0.530 | 3047 |
| C01 | rs1341449 | 239642925 | A | 0.548 | 28236 | 0.0079 | 1.09 | 0.570 | 2373 | 0.0083 | 1.08 | 0.568 | 3024 |
| C01 | rs3014559 | 239649291 | A | 0.550 | 28231 | 0.022 | 1.08 | 0.569 | 2393 | 0.015 | 1.08 | 0.568 | 3044 |
| C01 | rs12068067 | 245092561 | G | 0.774 | 28183 | 0.0059 | 1.12 | 0.793 | 2390 | 0.0064 | 1.10 | 0.791 | 3041 |
| C02 | rs7608117 | 11183423 | T | 0.487 | 28310 | 0.0037 | 1.10 | 0.511 | 2393 | 0.028 | 1.07 | 0.503 | 3044 |
| C02 | rs10929777 | 12079564 | C | 0.305 | 28316 | 1.60E-01 | 1.05 | 0.315 | 2395 | 0.22 | 1.04 | 0.313 | 3047 |
| C02 | rs11897395 | 16671068 | C | 0.890 | 28200 | 0.0085 | 1.15 | 0.903 | 2379 | 0.028 | 1.11 | 0.900 | 3028 |
| C02 | rs2342551 | 17178028 | T | 0.713 | 28317 | 0.0023 | 1.12 | 0.735 | 2395 | 0.012 | 1.09 | 0.729 | 3047 |
| C02 | rs12467812 | 19858951 | G | 0.330 | 28257 | 0.035 | 1.08 | 0.346 | 2395 | 0.018 | 1.08 | 0.346 | 3047 |
| C02 | rs10200481 | 24191365 | A | 0.110 | 28314 | 0.018 | 1.13 | 0.122 | 2395 | 0.079 | 1.09 | 0.118 | 3047 |
| C02 | rs6747236 | 35901725 | T | 0.717 | 28311 | 0.0021 | 1.12 | 0.739 | 2395 | 0.0098 | 1.09 | 0.734 | 3047 |
| C02 | rs10203034 | 35916379 | A | 0.681 | 28318 | 0.0032 | 1.11 | 0.703 | 2395 | 0.018 | 1.08 | 0.697 | 3047 |
| C02 | rs963731 | 39070377 | C | 0.954 | 27813 | 0.0019 | 1.30 | 0.964 | 2347 | 0.001 | 1.28 | 0.963 | 2991 |
| C02 | rs2168043 | 39185597 | C | 0.867 | 28278 | 0.0077 | 1.14 | 0.881 | 2395 | 0.0042 | 1.14 | 0.881 | 3047 |
| C02 | rs10202624 | 42368892 | A | 0.095 | 28319 | 0.028 | 1.13 | 0.106 | 2395 | 0.04 | 1.11 | 0.104 | 3047 |
| C02 | rs6724757 | 42384655 | A | 0.092 | 28315 | 0.042 | 1.12 | 0.101 | 2395 | 0.057 | 1.10 | 0.100 | 3047 |
| C02 | rs1991739 | 43797029 | A | 0.618 | 28313 | 0.035 | 1.07 | 0.635 | 2395 | 0.081 | 1.06 | 0.631 | 3047 |
| C02 | rs6709904 | 43933828 | A | 0.909 | 28315 | 0.058 | 1.12 | 0.918 | 2395 | 0.014 | 1.14 | 0.919 | 3046 |
| C02 | rs908571 | 45171969 | C | 0.289 | 28285 | 0.00081 | 1.13 | 0.314 | 2392 | 0.0028 | 1.10 | 0.310 | 3043 |
| C02 | rs3788982 | 49098912 | G | 0.871 | 28320 | 0.0034 | 1.16 | 0.887 | 2395 | 0.0029 | 1.15 | 0.885 | 3047 |
| C02 | rs6743414 | 49277575 | G | 0.847 | 28314 | 0.0011 | 1.16 | 0.866 | 2395 | 0.0011 | 1.15 | 0.864 | 3047 |
| C02 | rs4853116 | 75264786 | A | 0.632 | 28051 | 0.066 | 1.06 | 0.646 | 2390 | 0.015 | 1.08 | 0.649 | 3041 |
| C02 | rs317285 | 76133652 | A | 0.499 | 28318 | 0.0026 | 1.10 | 0.524 | 2395 | 0.006 | 1.09 | 0.520 | 3047 |
| C02 | rs404892 | 76157421 | C | 0.500 | 28297 | 0.002 | 1.11 | 0.525 | 2395 | 0.0052 | 1.09 | 0.521 | 3046 |
| C02 | rs443713 | 76163892 | G | 0.392 | 28316 | 0.0011 | 1.11 | 0.418 | 2395 | 0.0011 | 1.10 | 0.415 | 3047 |
| C02 | rs7563058 | 76196590 | C | 0.499 | 28313 | 0.0018 | 1.11 | 0.524 | 2395 | 0.0045 | 1.09 | 0.520 | 3047 |
| C02 | rs1595968 | 76225824 | T | 0.393 | 11401 | 0.00037 | 1.15 | 0.427 | 1770 | 0.0009 | 1.13 | 0.423 | 2089 |
| C02 | r2861078 | 77551503 | T | 0.579 | 28249 | 0.28 | 1.04 | 0.588 | 2392 | 0.18 | 1.04 | 0.589 | 3042 |
| C02 | rs6547152 | 77634997 | C | 0.790 | 28305 | 0.12 | 1.07 | 0.801 | 2393 | 0.1 | 1.06 | 0.800 | 3044 |
| C02 | rs2861264 | 78220585 | T | 0.107 | 28303 | 0.0057 | 1.15 | 0.121 | 2394 | 0.024 | 1.11 | 0.117 | 3046 |
| C02 | rs11675205 | 85323792 | A | 0.255 | 28302 | 0.0005 | 1.14 | 0.280 | 2394 | 0.0011 | 1.11 | 0.276 | 3046 |
| C02 | rs2245113 | 118108945 | A | 0.179 | 28279 | 0.017 | 1.10 | 0.194 | 2392 | 0.08 | 1.07 | 0.189 | 3043 |
| C02 | rs1477451 | 118164844 | T | 0.126 | 28320 | 0.0012 | 1.17 | 0.144 | 2395 | 0.0099 | 1.12 | 0.139 | 3047 |
| C02 | rs1395930 | 123468570 | T | 0.499 | 28303 | 3.20E-03 | 1.10 | 0.523 | 2393 | 0.00012 | 1.12 | 0.528 | 3045 |
| C02 | rs2661006 | 123482987 | T | 0.497 | 28301 | 0.0035 | 1.10 | 0.521 | 2391 | 0.00013 | 1.12 | 0.525 | 3042 |
| C02 | rs3943703 | 127613790 | C | 0.214 | 28280 | 0.02 | 1.09 | 0.229 | 2395 | 0.06 | 1.07 | 0.225 | 3046 |
| C02 | rs4662743 | 128067278 | T | 0.515 | 28231 | 0.042 | 1.07 | 0.531 | 2392 | 0.016 | 1.07 | 0.533 | 3042 |
| C02 | rs2244871 | 128115310 | C | 0.413 | 28251 | 0.013 | 1.09 | 0.433 | 2394 | 0.015 | 1.08 | 0.430 | 3046 |
| C02 | rs2321299 | 134350130 | C | 0.571 | 28315 | 0.034 | 1.07 | 0.588 | 2395 | 0.21 | 1.04 | 0.580 | 3046 |
| C02 | rs2139311 | 134628584 | C | 0.601 | 28304 | 0.0044 | 1.10 | 0.624 | 2394 | 0.0022 | 1.10 | 0.623 | 3045 |
| C02 | rs12473666 | 134639501 | T | 0.140 | 28308 | 0.03 | 1.10 | 0.153 | 2394 | 0.11 | 1.07 | 0.149 | 3046 |
| C02 | rs10496721 | 134648929 | T | 0.175 | 28306 | 0.094 | 1.07 | 0.185 | 2394 | 0.39 | 1.03 | 0.179 | 3046 |
| C02 | rs1257208 | 134700540 | A | 0.140 | 28317 | 0.017 | 1.12 | 0.154 | 2395 | 0.0067 | 1.12 | 0.154 | 3047 |
| C02 | rs2268365 | 169802415 | G | 0.127 | 28319 | 0.038 | 1.10 | 0.138 | 2393 | 0.045 | 1.09 | 0.136 | 3045 |
| C02 | rs1837241 | 171017898 | A | 0.696 | 28315 | 0.00086 | 1.13 | 0.720 | 2395 | 0.0024 | 1.10 | 0.716 | 3047 |
| C02 | rs1399959 | 172140552 | G | 0.668 | 28291 | 0.0069 | 1.10 | 0.688 | 2393 | 0.026 | 1.07 | 0.683 | 3044 |
| C02 | rs951917 | 172220171 | T | 0.383 | 28319 | 0.015 | 1.08 | 0.403 | 2395 | 0.3 | 1.03 | 0.391 | 3047 |
| C02 | rs4668412 | 172328016 | A | 0.709 | 28319 | 0.0031 | 1.11 | 0.730 | 2395 | 0.018 | 1.08 | 0.724 | 3047 |
| C02 | rs6758704 | 172363343 | T | 0.709 | 28303 | 3.10E-03 | 1.11 | 0.730 | 2395 | 0.02 | 1.08 | 0.724 | 3047 |
| C02 | rs10165126 | 172528561 | C | 0.702 | 28297 | 0.00068 | 1.13 | 0.727 | 2394 | 0.003 | 1.10 | 0.721 | 3046 |
| C02 | rs6718013 | 172577431 | G | 0.536 | 28318 | 0.0027 | 1.10 | 0.560 | 2395 | 0.073 | 1.05 | 0.549 | 3047 |
| C02 | rs921083 | 176453128 | G | 0.781 | 28310 | 0.0092 | 1.11 | 0.798 | 2394 | 0.04 | 1.08 | 0.794 | 3044 |
| C02 | rs12621733 | 176499888 | G | 0.901 | 28320 | 0.05 | 1.12 | 0.911 | 2395 | 0.056 | 1.10 | 0.910 | 3047 |
| C02 | rs1946815 | 183006405 | G | 0.219 | 28310 | 0.019 | 1.09 | 0.235 | 2395 | 0.061 | 1.07 | 0.231 | 3047 |
| C02 | rs4666836 | 183055419 | T | 0.223 | 28298 | 0.075 | 1.07 | 0.236 | 2394 | 0.15 | 1.05 | 0.232 | 3046 |
| C02 | rs6434921 | 197828442 | A | 0.397 | 28313 | 0.01 | 1.09 | 0.418 | 2395 | 0.03 | 1.07 | 0.413 | 3045 |
| C02 | rs6704822 | 203370024 | G | 0.915 | 28086 | 0.021 | 1.15 | 0.926 | 2380 | 0.13 | 1.09 | 0.922 | 3030 |
| C02 | rs6435401 | 208537772 | A | 0.339 | 28305 | 0.015 | 1.09 | 0.358 | 2394 | 0.025 | 1.07 | 0.355 | 3044 |
| C02 | rs9288396 | 209243202 | T | 0.205 | 28308 | 0.0077 | 1.11 | 0.223 | 2395 | 0.0013 | 1.12 | 0.225 | 3047 |
| C02 | rs4442936 | 211847782 | G | 0.280 | 28075 | 0.00038 | 1.14 | 0.307 | 2367 | 0.00015 | 1.13 | 0.306 | 3016 |
| C02 | rs12329252 | 212093968 | T | 0.681 | 28321 | 0.0047 | 1.11 | 0.703 | 2395 | 0.0059 | 1.09 | 0.700 | 3047 |
| C02 | rs17406681 | 212111632 | T | 0.277 | 28319 | 0.0075 | 1.10 | 0.297 | 2394 | 0.022 | 1.08 | 0.292 | 3046 |
| C02 | rs10932529 | 214852965 | A | 0.250 | 28318 | 2.80E-02 | 1.09 | 0.266 | 2395 | 0.027 | 1.08 | 0.264 | 3047 |
| C02 | rs2216544 | 214891122 | A | 0.257 | 28310 | 0.031 | 1.08 | 0.273 | 2395 | 0.027 | 1.08 | 0.272 | 3047 |
| C02 | rs13417729 | 215894070 | C | 0.715 | 11415 | 0.0067 | 1.13 | 0.739 | 1775 | 0.011 | 1.11 | 0.736 | 2094 |
| C02 | rs1478581 | 218000897 | G | 0.821 | 28316 | 0.0043 | 1.13 | 0.839 | 2395 | 0.0014 | 1.13 | 0.839 | 3045 |
| C02 | rs9288639 | 229462042 | C | 0.080 | 28295 | 0.021 | 1.14 | 0.091 | 2392 | 0.059 | 1.11 | 0.088 | 3042 |
| C02 | rs12617590 | 234708220 | C | 0.201 | 28301 | 0.0073 | 1.11 | 0.219 | 2395 | 0.015 | 1.09 | 0.215 | 3047 |
| C02 | rs12052982 | 241760005 | A | 0.956 | 28309 | 0.00035 | 1.36 | 0.967 | 2393 | 0.00018 | 1.34 | 0.967 | 3045 |
| C03 | rs17565216 | 2631107 | C | 0.366 | 28306 | 0.0031 | 1.10 | 0.390 | 2392 | 0.0033 | 1.09 | 0.387 | 3044 |
| C03 | rs341973 | 5243162 | A | 0.508 | 28284 | 0.021 | 1.08 | 0.527 | 2393 | 0.051 | 1.06 | 0.523 | 3044 |
| C03 | rs7638636 | 6735499 | A | 0.511 | 27822 | 0.031 | 1.07 | 0.529 | 2376 | 0.11 | 1.05 | 0.523 | 3013 |
| C03 | rs978658 | 6741178 | G | 0.615 | 28313 | 0.13 | 1.05 | 0.627 | 2394 | 0.26 | 1.04 | 0.623 | 3046 |
| C03 | rs12491369 | 6764265 | G | 0.577 | 28320 | 0.31 | 1.03 | 0.585 | 2395 | 0.63 | 1.01 | 0.581 | 3047 |
| C03 | rs779710 | 7486375 | A | 0.596 | 28311 | 0.088 | 1.06 | 0.610 | 2395 | 0.15 | 1.04 | 0.607 | 3047 |
| C03 | rs752299 | 7494813 | T | 0.685 | 11413 | 1.50E-03 | 1.15 | 0.714 | 1775 | 0.0056 | 1.12 | 0.708 | 2094 |
| C03 | rs2128163 | 14269176 | G | 0.539 | 28300 | 0.046 | 1.07 | 0.556 | 2395 | 0.025 | 1.07 | 0.556 | 3047 |
| C03 | rs6772823 | 14276852 | G | 0.280 | 27955 | 0.0033 | 1.11 | 0.302 | 2370 | 0.00061 | 1.12 | 0.303 | 3014 |
| C03 | rs1870480 | 22619924 | G | 0.388 | 28314 | 0.14 | 1.05 | 0.400 | 2395 | 0.089 | 1.05 | 0.400 | 3047 |
| C03 | rs6763490 | 25122716 | C | 0.600 | 28310 | 0.00071 | 1.12 | 0.627 | 2395 | 0.007 | 1.09 | 0.619 | 3047 |
| C03 | rs13079598 | 29295837 | A | 0.906 | 28315 | 0.0015 | 1.20 | 0.920 | 2395 | 0.0022 | 1.17 | 0.918 | 3046 |
| C03 | rs2853705 | 39306976 | T | 0.209 | 28313 | 0.0043 | 1.12 | 0.228 | 2395 | 0.0065 | 1.10 | 0.226 | 3047 |
| C03 | rs1530733 | 54364155 | A | 0.155 | 28318 | 0.029 | 1.10 | 0.168 | 2395 | 0.033 | 1.09 | 0.167 | 3047 |
| C03 | rs603052 | 61765300 | T | 0.778 | 28301 | 0.0083 | 1.11 | 0.796 | 2394 | 0.041 | 1.08 | 0.791 | 3046 |
| C03 | rs1997366 | 62414626 | C | 0.798 | 28304 | 1.80E-01 | 1.06 | 0.807 | 2394 | 0.21 | 1.05 | 0.805 | 3045 |
| C03 | rs1534725 | 62420416 | T | 0.788 | 28304 | 4.80E-02 | 1.08 | 0.801 | 2394 | 0.041 | 1.08 | 0.800 | 3046 |
| C03 | rs9874646 | 62437858 | T | 0.717 | 28314 | 0.11 | 1.06 | 0.729 | 2394 | 0.16 | 1.05 | 0.726 | 3046 |
| C03 | rs1452075 | 62456103 | T | 0.739 | 28228 | 0.2 | 1.05 | 0.748 | 2386 | 0.15 | 1.05 | 0.748 | 3033 |
| C03 | rs7653410 | 63619805 | C | 0.324 | 28036 | 0.01 | 1.09 | 0.344 | 2383 | 0.051 | 1.06 | 0.338 | 3030 |
| C03 | rs1563545 | 66782776 | G | 0.530 | 28035 | 1.50E-02 | 1.08 | 0.550 | 2369 | 0.015 | 1.07 | 0.548 | 3016 |
| C03 | rs2046867 | 72863765 | T | 0.279 | 28307 | 0.0088 | 1.10 | 0.298 | 2395 | 0.021 | 1.08 | 0.294 | 3047 |
| C03 | rs7632726 | 72895436 | T | 0.310 | 28319 | 0.013 | 1.09 | 0.329 | 2395 | 0.026 | 1.07 | 0.325 | 3047 |
| C03 | rs2208 | 72954463 | A | 0.268 | 28315 | 0.0087 | 1.10 | 0.287 | 2395 | 0.024 | 1.08 | 0.283 | 3047 |
| C03 | rs4234186 | 74348999 | A | 0.945 | 28271 | 0.0037 | 1.25 | 0.956 | 2390 | 0.13 | 1.11 | 0.950 | 3040 |
| C03 | rs2055707 | 78706269 | A | 0.418 | 28182 | 0.00039 | 1.12 | 0.447 | 2392 | 0.0016 | 1.10 | 0.441 | 3044 |
| C03 | rs13075335 | 83006051 | T | 0.904 | 28256 | 1.60E-02 | 1.15 | 0.915 | 2393 | 0.047 | 1.11 | 0.913 | 3044 |
| C03 | rs1531377 | 99825932 | T | 0.177 | 28216 | 0.013 | 1.11 | 0.192 | 2391 | 0.2 | 1.05 | 0.184 | 3041 |
| C03 | rs3796139 | 99861391 | G | 0.150 | 28071 | 0.012 | 1.12 | 0.165 | 2375 | 0.2 | 1.05 | 0.157 | 3018 |
| C03 | rs9847700 | 124635632 | G | 0.851 | 28188 | 0.00017 | 1.20 | 0.872 | 2345 | 0.00021 | 1.17 | 0.870 | 2995 |
| C03 | rs6438839 | 125536191 | A | 0.078 | 28316 | 0.0077 | 1.17 | 0.090 | 2395 | 0.0044 | 1.16 | 0.090 | 3047 |
| C03 | rs10935515 | 146038973 | C | 0.576 | 26370 | 0.00024 | 1.13 | 0.606 | 2294 | 0.00051 | 1.11 | 0.602 | 2902 |
| C03 | rs4401402 | 146073240 | T | 0.584 | 28314 | 3.20E-03 | 1.10 | 0.608 | 2394 | 0.0032 | 1.09 | 0.606 | 3046 |
| C03 | rs13061557 | 146128702 | T | 0.698 | 28295 | 0.092 | 1.06 | 0.710 | 2395 | 0.13 | 1.05 | 0.708 | 3047 |
| C03 | rs10935523 | 146138781 | A | 0.740 | 28317 | 0.036 | 1.08 | 0.754 | 2395 | 0.026 | 1.08 | 0.754 | 3047 |
| C03 | rs1430435 | 170561887 | G | 0.501 | 28314 | 0.017 | 1.08 | 0.521 | 2395 | 0.056 | 1.06 | 0.515 | 3047 |
| C03 | rs756644 | 170567521 | A | 0.429 | 28313 | 2.20E-03 | 1.11 | 0.454 | 2394 | 0.023 | 1.07 | 0.446 | 3046 |
| C03 | rs1549100 | 175400363 | T | 0.732 | 28237 | 0.078 | 1.07 | 0.745 | 2386 | 0.28 | 1.04 | 0.739 | 3038 |
| C03 | rs1421420 | 175408319 | A | 0.727 | 28307 | 0.037 | 1.08 | 0.742 | 2395 | 0.15 | 1.05 | 0.737 | 3047 |
| C03 | rs2111956 | 175432500 | G | 0.763 | 28317 | 0.043 | 1.08 | 0.777 | 2395 | 0.099 | 1.06 | 0.773 | 3046 |
| C03 | rs6803944 | 186606110 | C | 0.845 | 28320 | 0.0084 | 1.13 | 0.860 | 2395 | 0.012 | 1.11 | 0.858 | 3047 |
| C03 | rs9864293 | 191695400 | C | 0.387 | 28308 | 0.00012 | 1.14 | 0.418 | 2395 | 0.00013 | 1.12 | 0.415 | 3046 |
| C03 | rs883279 | 194931805 | C | 0.907 | 28311 | 0.061 | 1.11 | 0.916 | 2394 | 0.03 | 1.12 | 0.916 | 3046 |
| C03 | rs4497978 | 195990481 | A | 0.666 | 28228 | 0.00031 | 1.14 | 0.694 | 2375 | 0.0019 | 1.10 | 0.688 | 3023 |
| C04 | rs6822427 | 3749870 | A | 0.488 | 28256 | 0.022 | 1.08 | 0.506 | 2391 | 0.016 | 1.07 | 0.506 | 3040 |
| C04 | rs3756193 | 8284241 | G | 0.461 | 28262 | 0.0072 | 1.09 | 0.483 | 2393 | 0.004 | 1.09 | 0.482 | 3043 |
| C04 | rs714086 | 9922140 | G | 0.761 | 28317 | 0.013 | 1.10 | 0.778 | 2395 | 0.11 | 1.06 | 0.771 | 3047 |
| C04 | rs4697653 | 10337857 | G | 0.524 | 28161 | 0.0021 | 1.11 | 0.549 | 2392 | 0.0081 | 1.08 | 0.543 | 3040 |
| C04 | rs2170752 | 10493178 | T | 0.576 | 28317 | 0.011 | 1.09 | 0.597 | 2395 | 0.077 | 1.05 | 0.589 | 3047 |
| C04 | rs959233 | 10511257 | G | 0.446 | 28291 | 2.60E-02 | 1.08 | 0.464 | 2394 | 0.02 | 1.07 | 0.463 | 3044 |
| C04 | rs10011464 | 10515465 | T | 0.462 | 28191 | 0.031 | 1.07 | 0.480 | 2375 | 0.038 | 1.06 | 0.478 | 3025 |
| C04 | rs7442366 | 16944686 | T | 0.824 | 28321 | 0.024 | 1.10 | 0.838 | 2395 | 0.066 | 1.08 | 0.835 | 3047 |
| C04 | rs2697684 | 17045056 | G | 0.774 | 28318 | 0.033 | 1.09 | 0.788 | 2395 | 0.018 | 1.09 | 0.788 | 3047 |
| C04 | rs1827591 | 21341284 | G | 0.538 | 28319 | 0.0079 | 1.09 | 0.560 | 2395 | 0.012 | 1.08 | 0.557 | 3047 |
| C04 | rs6448072 | 21352329 | C | 0.659 | 28318 | 0.017 | 1.09 | 0.677 | 2395 | 0.009 | 1.09 | 0.677 | 3047 |
| C04 | rs10516402 | 21407661 | C | 0.505 | 28313 | 0.0023 | 1.10 | 0.530 | 2394 | 0.0024 | 1.09 | 0.527 | 3046 |
| C04 | rs4282162 | 21426819 | T | 0.530 | 28305 | 0.002 | 1.11 | 0.555 | 2395 | 0.0031 | 1.09 | 0.552 | 3047 |
| C04 | rs4377576 | 21434824 | C | 0.541 | 28316 | 0.0034 | 1.10 | 0.565 | 2395 | 0.0044 | 1.09 | 0.562 | 3047 |
| C04 | r2060515 | 29994854 | T | 0.114 | 28189 | 0.059 | 1.10 | 0.124 | 2379 | 0.064 | 1.09 | 0.123 | 3028 |
| C04 | rs4132132 | 30610399 | G | 0.451 | 28229 | 0.12 | 1.05 | 0.464 | 2390 | 0.17 | 1.04 | 0.461 | 3040 |
| C04 | rs10517277 | 33143198 | C | 0.102 | 28290 | 0.0086 | 1.15 | 0.115 | 2394 | 0.041 | 1.10 | 0.111 | 3046 |
| C04 | rs3849018 | 37325240 | T | 0.280 | 28319 | 0.003 | 1.11 | 0.301 | 2395 | 0.0016 | 1.11 | 0.301 | 3046 |
| C04 | rs4447895 | 40092010 | C | 0.887 | 28318 | 0.015 | 1.14 | 0.899 | 2395 | 0.0087 | 1.13 | 0.899 | 3047 |
| C04 | rs6447404 | 44714926 | G | 0.620 | 11414 | 0.015 | 1.10 | 0.643 | 1775 | 0.048 | 1.08 | 0.638 | 2094 |
| C04 | rs7661204 | 45728007 | T | 0.449 | 28320 | 0.0055 | 0.91 | 0.427 | 2395 | 0.00074 | 0.91 | 0.425 | 3047 |
| C04 | rs10026644 | 82020315 | G | 0.924 | 27964 | 0.0003 | 1.27 | 0.939 | 2382 | 0.01 | 1.16 | 0.933 | 3028 |
| C04 | rs10026120 | 82037699 | T | 0.959 | 28199 | 0.006 | 1.28 | 0.968 | 2393 | 0.03 | 1.19 | 0.965 | 3040 |
| C04 | rs6830629 | 82083395 | A | 0.975 | 28273 | 0.0036 | 1.39 | 0.982 | 2393 | 0.034 | 1.24 | 0.979 | 3043 |
| C04 | rs2589506 | 87158716 | G | 0.456 | 28313 | 0.018 | 1.08 | 0.475 | 2395 | 0.0053 | 1.09 | 0.476 | 3047 |
| C04 | rs2199309 | 87318595 | C | 0.219 | 26388 | 0.022 | 1.10 | 0.235 | 2276 | 0.0032 | 1.11 | 0.238 | 2883 |
| C04 | rs10516770 | 87391122 | T | 0.056 | 28310 | 0.083 | 1.13 | 0.062 | 2395 | 0.014 | 1.16 | 0.064 | 3047 |
| C04 | rs2924936 | 89860537 | G | 0.871 | 28318 | 0.11 | 1.08 | 0.879 | 2395 | 0.019 | 1.11 | 0.882 | 3047 |
| C04 | rs13130 | 89867868 | T | 0.872 | 28227 | 0.11 | 1.08 | 0.880 | 2388 | 0.019 | 1.11 | 0.883 | 3039 |
| C04 | rs6850673 | 96366883 | C | 0.142 | 28314 | 0.014 | 1.12 | 0.156 | 2394 | 0.0082 | 1.12 | 0.156 | 3046 |
| C04 | rs10516987 | 97050762 | G | 0.798 | 28265 | 0.0073 | 1.12 | 0.815 | 2385 | 0.019 | 1.09 | 0.812 | 3035 |
| C04 | rs1982346 | 106798203 | C | 0.058 | 27647 | 0.00038 | 1.26 | 0.072 | 2352 | 0.0016 | 1.21 | 0.069 | 2991 |
| C04 | rs6820367 | 119312058 | A | 0.353 | 28319 | 0.036 | 1.07 | 0.369 | 2395 | 0.078 | 1.06 | 0.365 | 3047 |
| C04 | rs6534683 | 129601758 | T | 0.656 | 28310 | 0.076 | 1.06 | 0.670 | 2395 | 0.034 | 1.07 | 0.671 | 3047 |
| C04 | rs7690289 | 129628963 | T | 0.637 | 27978 | 0.028 | 1.08 | 0.654 | 2381 | 0.0076 | 1.09 | 0.655 | 3023 |
| C04 | rs4549394 | 129650748 | T | 0.102 | 28313 | 0.094 | 1.09 | 0.110 | 2395 | 0.14 | 1.07 | 0.108 | 3047 |
| C04 | rs4383635 | 131414021 | A | 0.491 | 28225 | 0.031 | 1.07 | 0.508 | 2394 | 0.0099 | 1.08 | 0.510 | 3045 |
| C04 | rs1027884 | 131431891 | T | 0.497 | 28311 | 0.02 | 1.08 | 0.516 | 2395 | 0.0072 | 1.08 | 0.517 | 3047 |
| C04 | rs4864193 | 134641403 | C | 0.961 | 28307 | 0.16 | 1.13 | 0.965 | 2395 | 0.15 | 1.12 | 0.965 | 3047 |
| C04 | rs10028163 | 138689635 | G | 0.207 | 28316 | 0.0012 | 1.14 | 0.228 | 2395 | 0.00075 | 1.13 | 0.227 | 3047 |
| C04 | rs7669668 | 139472904 | T | 0.123 | 28319 | 0.02 | 1.12 | 0.136 | 2395 | 0.16 | 1.06 | 0.130 | 3047 |
| C04 | rs723794 | 147095001 | T | 0.773 | 28318 | 0.011 | 1.11 | 0.791 | 2395 | 0.037 | 1.08 | 0.786 | 3047 |
| C04 | rs2241521 | 147218561 | A | 0.239 | 28318 | 0.03 | 1.09 | 0.254 | 2395 | 0.017 | 1.09 | 0.255 | 3047 |
| C04 | rs17326199 | 161219141 | G | 0.214 | 28307 | 0.0099 | 1.11 | 0.231 | 2393 | 0.0041 | 1.11 | 0.231 | 3045 |
| C04 | rs17359034 | 161797877 | T | 0.390 | 28227 | 0.011 | 1.09 | 0.410 | 2394 | 0.044 | 1.06 | 0.404 | 3046 |
| C04 | rs12503470 | 162109433 | T | 0.383 | 28267 | 0.049 | 1.07 | 0.399 | 2394 | 0.019 | 1.07 | 0.400 | 3046 |
| C04 | rs9942159 | 162382460 | T | 0.380 | 28203 | 0.005 | 1.10 | 0.402 | 2383 | 0.0029 | 1.09 | 0.401 | 3034 |
| C04 | rs958247 | 162412247 | A | 0.289 | 28271 | 0.0036 | 1.11 | 0.310 | 2393 | 0.0014 | 1.11 | 0.310 | 3043 |
| C04 | rs9991428 | 163627494 | G | 0.961 | 28297 | 0.0014 | 1.34 | 0.971 | 2394 | 0.0013 | 1.30 | 0.970 | 3046 |
| C04 | rs11723570 | 165962251 | C | 0.509 | 28320 | 0.002 | 1.11 | 0.534 | 2395 | 0.0019 | 1.10 | 0.532 | 3047 |
| C04 | rs4695894 | 175054285 | G | 0.500 | 28257 | 0.0079 | 1.09 | 0.522 | 2393 | 0.017 | 1.07 | 0.518 | 3043 |
| C04 | rs309770 | 177690442 | G | 0.891 | 28321 | 0.027 | 1.13 | 0.902 | 2395 | 0.073 | 1.09 | 0.899 | 3047 |
| C04 | rs309800 | 177704874 | C | 0.799 | 28259 | 0.019 | 1.10 | 0.814 | 2391 | 0.01 | 1.10 | 0.814 | 3043 |
| C04 | rs1983130 | 178505657 | G | 0.415 | 28301 | 0.022 | 1.08 | 0.434 | 2394 | 0.073 | 1.06 | 0.428 | 3045 |
| C04 | rs17090640 | 180904641 | G | 0.952 | 28319 | 6.70E-03 | 1.24 | 0.961 | 2395 | 0.029 | 1.17 | 0.958 | 3047 |
| C05 | rs1697952 | 667963 | C | 0.374 | 28278 | 0.035 | 1.07 | 0.391 | 2392 | 0.052 | 1.06 | 0.388 | 3043 |
| C05 | rs1697997 | 3595548 | A | 0.918 | 28300 | 0.0099 | 1.17 | 0.929 | 2395 | 0.014 | 1.15 | 0.928 | 3046 |
| C05 | rs710998 | 3644916 | T | 0.903 | 28254 | 0.0014 | 1.20 | 0.918 | 2390 | 0.0045 | 1.16 | 0.915 | 3040 |
| C05 | rs11956711 | 3978083 | C | 0.066 | 28288 | 0.0047 | 1.19 | 0.077 | 2393 | 0.021 | 1.14 | 0.074 | 3044 |
| C05 | rs634344 | 3997125 | G | 0.066 | 28294 | 0.0042 | 1.20 | 0.078 | 2392 | 0.019 | 1.14 | 0.074 | 3042 |
| C05 | rs864267 | 5893433 | A | 0.451 | 28310 | 0.0023 | 1.10 | 0.476 | 2394 | 0.0083 | 1.08 | 0.470 | 3046 |
| C05 | rs2617549 | 5904873 | A | 0.453 | 28283 | 0.0023 | 1.11 | 0.477 | 2393 | 0.0072 | 1.08 | 0.472 | 3045 |
| C05 | rs1457122 | 6326004 | T | 0.338 | 28304 | 0.03 | 1.08 | 0.354 | 2395 | 0.081 | 1.06 | 0.350 | 3047 |
| C05 | rs870347 | 6898035 | T | 0.926 | 28313 | 0.0052 | 1.20 | 0.937 | 2395 | 0.0088 | 1.17 | 0.936 | 3047 |
| C05 | rs3213627 | 9694628 | T | 0.172 | 28296 | 0.0025 | 1.14 | 0.191 | 2393 | 0.03 | 1.09 | 0.184 | 3045 |
| C05 | rs2106687 | 9700012 | G | 0.130 | 28310 | 0.0055 | 1.14 | 0.146 | 2395 | 0.074 | 1.08 | 0.139 | 3046 |
| C05 | rs16883905 | 10009338 | T | 0.112 | 28320 | 0.00056 | 1.19 | 0.130 | 2395 | 0.00012 | 1.19 | 0.130 | 3047 |
| C05 | rs30616 | 14320173 | T | 0.852 | 28316 | 0.0016 | 1.16 | 0.870 | 2395 | 0.0018 | 1.14 | 0.868 | 3047 |
| C05 | rs7715223 | 15953124 | C | 0.819 | 28263 | 0.0012 | 1.15 | 0.839 | 2384 | 0.021 | 1.09 | 0.832 | 3036 |
| C05 | rs11743379 | 23752107 | T | 0.514 | 28310 | 0.093 | 1.06 | 0.528 | 2395 | 0.096 | 1.05 | 0.527 | 3047 |
| C05 | rs1382932 | 26922341 | C | 0.683 | 28294 | 0.0083 | 1.10 | 0.703 | 2393 | 0.0093 | 1.09 | 0.701 | 3045 |
| C05 | rs6875512 | 31367532 | A | 0.743 | 28305 | 0.011 | 1.10 | 0.760 | 2395 | 0.021 | 1.08 | 0.757 | 3047 |
| C05 | rs525735 | 38917385 | C | 0.777 | 28312 | 0.064 | 1.08 | 0.789 | 2394 | 0.18 | 1.05 | 0.785 | 3046 |
| C05 | rs357291 | 38959536 | C | 0.403 | 28138 | 0.048 | 1.07 | 0.418 | 2377 | 0.18 | 1.04 | 0.412 | 3025 |
| C05 | rs9292729 | 39061343 | C | 0.345 | 28300 | 0.044 | 1.07 | 0.361 | 2395 | 0.14 | 1.05 | 0.356 | 3047 |
| C05 | rs6880882 | 39112718 | A | 0.360 | 28252 | 0.051 | 1.07 | 0.375 | 2389 | 0.13 | 1.05 | 0.370 | 3039 |
| C05 | rs444556 | 39149695 | C | 0.310 | 28223 | 0.024 | 1.08 | 0.327 | 2391 | 0.085 | 1.06 | 0.322 | 3040 |
| C05 | rs13165359 | 40271560 | G | 0.881 | 28320 | 0.031 | 1.12 | 0.892 | 2395 | 0.16 | 1.07 | 0.888 | 3047 |
| C05 | rs13175020 | 40646263 | T | 0.817 | 28309 | 0.0022 | 1.14 | 0.836 | 2393 | 0.01 | 1.11 | 0.831 | 3045 |
| C05 | rs10038238 | 50797350 | A | 0.569 | 28268 | 0.00027 | 1.13 | 0.598 | 2393 | 0.0007 | 1.11 | 0.594 | 3043 |
| C05 | rs1423611 | 50806871 | C | 0.443 | 28301 | 0.00032 | 1.12 | 0.472 | 2395 | 0.0051 | 1.09 | 0.464 | 3047 |
| C05 | rs6449708 | 50887332 | T | 0.456 | 27678 | 0.00067 | 1.12 | 0.484 | 2369 | 0.0026 | 1.09 | 0.479 | 3009 |
| C05 | rs7708362 | 50924592 | T | 0.348 | 28252 | 0.0041 | 1.10 | 0.370 | 2381 | 0.02 | 1.07 | 0.364 | 3033 |
| C05 | rs4865673 | 50964567 | G | 0.647 | 28318 | 0.0086 | 1.09 | 0.668 | 2395 | 0.0038 | 1.09 | 0.668 | 3046 |
| C05 | rs38055 | 52596401 | T | 0.387 | 28311 | 0.052 | 1.07 | 0.403 | 2395 | 0.091 | 1.05 | 0.399 | 3047 |
| C05 | rs32496 | 55677396 | C | 0.576 | 28296 | 0.0066 | 1.09 | 0.598 | 2395 | 0.029 | 1.07 | 0.592 | 3046 |
| C05 | rs30365 | 55864542 | C | 0.900 | 28303 | 0.0018 | 1.19 | 0.914 | 2395 | 0.019 | 1.13 | 0.910 | 3046 |
| C05 | rs7720499 | 56057578 | T | 0.112 | 28276 | 0.0075 | 1.14 | 0.126 | 2394 | 0.0037 | 1.14 | 0.125 | 3045 |
| C05 | rs4700485 | 56069964 | A | 0.326 | 28316 | 0.013 | 1.09 | 0.345 | 2395 | 0.039 | 1.07 | 0.340 | 3047 |
| C05 | rs12657615 | 56099890 | C | 0.116 | 28319 | 0.012 | 1.13 | 0.129 | 2395 | 0.0046 | 1.14 | 0.129 | 3047 |
| C05 | rs832540 | 56234959 | C | 0.422 | 28307 | 0.0061 | 1.09 | 0.444 | 2395 | 0.0043 | 1.09 | 0.443 | 3047 |
| C05 | rs252890 | 56262167 | A | 0.430 | 27978 | 0.015 | 1.08 | 0.450 | 2373 | 0.0087 | 1.08 | 0.450 | 3012 |
| C05 | rs1445998 | 56303962 | A | 0.406 | 28292 | 0.015 | 1.08 | 0.425 | 2393 | 0.004 | 1.09 | 0.427 | 3045 |
| C05 | rs2408654 | 56318303 | C | 0.274 | 28293 | 0.0045 | 1.11 | 0.295 | 2395 | 0.0025 | 1.10 | 0.294 | 3047 |
| C05 | rs2962014 | 57506028 | G | 0.504 | 27810 | 0.051 | 1.07 | 0.520 | 2365 | 0.069 | 1.06 | 0.518 | 3011 |
| C05 | rs2112905 | 57520064 | A | 0.405 | 28312 | 0.015 | 1.08 | 0.424 | 2395 | 0.012 | 1.08 | 0.423 | 3047 |
| C05 | rs2441109 | 66174994 | G | 0.164 | 28313 | 0.017 | 1.11 | 0.178 | 2395 | 0.042 | 1.08 | 0.175 | 3047 |
| C05 | rs39700 | 66197800 | G | 0.375 | 28315 | 0.034 | 1.07 | 0.391 | 2394 | 0.05 | 1.06 | 0.389 | 3046 |
| C05 | rs974280 | 76535005 | G | 0.358 | 28315 | 0.0013 | 1.11 | 0.383 | 2395 | 0.0011 | 1.10 | 0.381 | 3047 |
| C05 | rs4704400 | 76586766 | T | 0.498 | 28306 | 0.0057 | 1.09 | 0.520 | 2394 | 0.023 | 1.07 | 0.514 | 3046 |
| C05 | rs251421 | 76608726 | C | 0.262 | 28278 | 0.02 | 1.09 | 0.278 | 2383 | 0.063 | 1.06 | 0.274 | 3033 |
| C05 | rs3816609 | 76757694 | A | 0.359 | 28316 | 0.03 | 1.08 | 0.376 | 2395 | 0.049 | 1.06 | 0.373 | 3047 |
| C05 | rs690280 | 76794989 | T | 0.429 | 28241 | 0.027 | 1.08 | 0.447 | 2388 | 0.1 | 1.05 | 0.441 | 3040 |
| C05 | rs251470 | 76814241 | T | 0.445 | 28294 | 0.02 | 1.08 | 0.464 | 2395 | 0.081 | 1.05 | 0.458 | 3047 |
| C05 | rs6452479 | 82055874 | C | 0.936 | 28169 | 0.014 | 1.19 | 0.945 | 2384 | 0.0067 | 1.19 | 0.945 | 3032 |
| C05 | rs6556861 | 94554256 | G | 0.570 | 28317 | 0.0082 | 1.09 | 0.591 | 2395 | 0.006 | 1.09 | 0.590 | 3047 |
| C05 | rs464906 | 94633980 | C | 0.619 | 28287 | 0.049 | 1.07 | 0.635 | 2394 | 0.051 | 1.06 | 0.633 | 3045 |
| C05 | rs950195 | 97466690 | G | 0.633 | 28228 | 0.0033 | 1.11 | 0.656 | 2395 | 0.002 | 1.10 | 0.654 | 3046 |
| C05 | rs168654 | 97924372 | T | 0.641 | 28309 | 0.053 | 1.07 | 0.656 | 2394 | 0.07 | 1.06 | 0.654 | 3046 |
| C05 | rs3776859 | 102379933 | T | 0.709 | 28311 | 0.001 | 1.13 | 0.733 | 2395 | 0.0067 | 1.09 | 0.727 | 3046 |
| C05 | rs26434 | 102391301 | T | 0.705 | 28286 | 0.0012 | 1.12 | 0.729 | 2391 | 0.0098 | 1.09 | 0.722 | 3042 |
| C05 | rs9327877 | 102409540 | T | 0.194 | 28309 | 0.00071 | 1.15 | 0.216 | 2395 | 0.0026 | 1.12 | 0.212 | 3046 |
| C05 | rs34374 | 102431920 | A | 0.673 | 28279 | 2.70E-03 | 1.11 | 0.696 | 2394 | 0.016 | 1.08 | 0.690 | 3046 |
| C05 | rs34813 | 102461308 | G | 0.710 | 28316 | 0.0015 | 1.12 | 0.733 | 2395 | 0.011 | 1.09 | 0.727 | 3047 |
| C05 | rs183752 | 102535711 | T | 0.717 | 28280 | 0.0027 | 1.12 | 0.739 | 2394 | 0.021 | 1.08 | 0.733 | 3046 |
| C05 | rs26821 | 102548299 | A | 0.710 | 28196 | 0.0014 | 1.12 | 0.733 | 2389 | 0.011 | 1.09 | 0.727 | 3041 |
| C05 | rs4957775 | 108007478 | G | 0.837 | 28311 | 0.0013 | 1.16 | 0.856 | 2395 | 0.0008 | 1.15 | 0.854 | 3047 |
| C05 | rs9326981 | 115348507 | C | 0.950 | 28315 | 0.076 | 1.15 | 0.956 | 2395 | 0.16 | 1.10 | 0.954 | 3047 |
| C05 | rs17138668 | 115379140 | C | 0.948 | 28317 | 0.0041 | 1.25 | 0.958 | 2395 | 0.014 | 1.19 | 0.956 | 3047 |
| C05 | rs728676 | 118087433 | T | 0.444 | 28310 | 0.0023 | 1.10 | 0.468 | 2394 | 0.001 | 1.10 | 0.468 | 3046 |
| C05 | rs7721883 | 119467718 | T | 0.466 | 28215 | 0.0048 | 1.10 | 0.489 | 2390 | 0.04 | 1.06 | 0.482 | 3041 |
| C05 | rs9327287 | 122547512 | G | 0.263 | 28313 | 0.012 | 1.10 | 0.281 | 2395 | 0.091 | 1.06 | 0.274 | 3047 |
| C05 | rs1476097 | 133864960 | T | 0.658 | 28313 | 0.0025 | 1.11 | 0.681 | 2394 | 0.03 | 1.07 | 0.673 | 3045 |
| C05 | rs4141140 | 135980112 | G | 0.701 | 28318 | 0.0043 | 1.11 | 0.723 | 2395 | 0.0031 | 1.10 | 0.721 | 3046 |
| C05 | rs1870739 | 153183640 | C | 0.853 | 28308 | 0.015 | 1.12 | 0.866 | 2392 | 0.037 | 1.09 | 0.863 | 3044 |
| C05 | rs1461236 | 153221364 | T | 0.854 | 28176 | 0.015 | 1.12 | 0.868 | 2381 | 0.039 | 1.09 | 0.865 | 3031 |
| C05 | rs716376 | 157479655 | G | 0.189 | 28314 | 0.034 | 1.09 | 0.202 | 2395 | 0.13 | 1.06 | 0.197 | 3046 |
| C05 | rs2964513 | 157878493 | A | 0.696 | 28222 | 0.025 | 1.08 | 0.713 | 2389 | 0.35 | 1.03 | 0.702 | 3040 |
| C05 | rs7442701 | 158456100 | T | 0.181 | 28293 | 0.00051 | 1.15 | 0.203 | 2394 | 0.005 | 1.11 | 0.197 | 3046 |
| C05 | rs7447732 | 158461713 | C | 0.320 | 28280 | 0.0033 | 1.11 | 0.343 | 2393 | 0.015 | 1.08 | 0.337 | 3045 |
| C05 | rs270661 | 158492732 | A | 0.210 | 28319 | 0.011 | 1.11 | 0.227 | 2394 | 0.025 | 1.08 | 0.223 | 3046 |
| C05 | rs270654 | 158497687 | G | 0.137 | 28318 | 0.012 | 1.12 | 0.151 | 2395 | 0.021 | 1.10 | 0.149 | 3047 |
| C05 | rs254850 | 158599309 | T | 0.220 | 28316 | 0.0011 | 1.13 | 0.242 | 2395 | 0.0082 | 1.10 | 0.236 | 3047 |
| C05 | rs4921437 | 158623529 | T | 0.216 | 28259 | 0.00049 | 1.1.4 | 0.240 | 2386 | 0.0022 | 1.11 | 0.235 | 3037 |
| C05 | rs1433048 | 158688423 | G | 0.193 | 27899 | 0.0074 | 1.12 | 0.211 | 2344 | 0.021 | 1.09 | 0.207 | 2989 |
| C05 | rs7709212 | 158696755 | T | 0.680 | 28305 | 0.004 | 1.11 | 0.702 | 2394 | 0.0061 | 1.09 | 0.699 | 3046 |
| C05 | rs953861 | 158705160 | G | 0.161 | 28318 | 0.0027 | 1.14 | 0.180 | 2395 | 0.052 | 1.08 | 0.172 | 3047 |
| C05 | rs10045431 | 158747111 | A | 0.266 | 28238 | 0.011 | 1.10 | 0.285 | 2364 | 0.095 | 1.06 | 0.277 | 3014 |
| C05 | rs889053 | 159028256 | T | 0.159 | 28319 | 0.012 | 1.12 | 0.174 | 2395 | 0.016 | 1.10 | 0.172 | 3047 |
| C05 | rs7705176 | 167424970 | G | 0.786 | 28317 | 0.15 | 1.06 | 0.796 | 2395 | 0.37 | 1.03 | 0.791 | 3047 |
| C05 | rs7736530 | 167453404 | G | 0.802 | 28315 | 0.093 | 1.07 | 0.813 | 2395 | 0.14 | 1.06 | 0.811 | 3047 |
| C05 | rs2617972 | 167476408 | C | 0.813 | 28316 | 0.095 | 1.07 | 0.824 | 2393 | 0.29 | 1.04 | 0.819 | 3045 |
| C05 | rs1077046 | 167478603 | C | 0.554 | 28303 | 0.0099 | 1.09 | 0.575 | 2395 | 0.064 | 1.06 | 0.567 | 3047 |
| C05 | rs7724098 | 176592176 | G | 0.289 | 28310 | 0.007 | 1.10 | 0.309 | 2392 | 0.034 | 1.07 | 0.304 | 3044 |
| C05 | rs3756566 | 178509237 | G | 0.404 | 28315 | 0.0033 | 1.10 | 0.427 | 2395 | 0.008 | 1.08 | 0.423 | 3047 |
| C06 | rs2113691 | 5382731 | C | 0.485 | 28112 | 0.016 | 1.08 | 0.505 | 2339 | 0.044 | 1.06 | 0.500 . | 2987 |
| C06 | rs6459613 | 10170597 | A | 0.549 | 28236 | 0.039 | 1.07 | 0.565 | 2393 | 0.044 | 1.06 | 0.563 | 3043 |
| C06 | rs9357002 | 10958298 | T | 0.637 | 27588 | 0.002 | 1.11 | 0.661 | 2346 | 0.0021 | 1.10 | 0.659 | 2986 |
| C06 | rs1028716 | 12338790 | T | 0.061 | 28313 | 0.025 | 1.16 | 0.070 | 2395 | 0.0065 | 1.17 | 0.071 | 3047 |
| C06 | rs6940891 | 17345030 | C | 0.172 | 28319 | 0.013 | 1.11 | 0.187 | 2395 | 0.012 | 1.10 | 0.186 | 3047 |
| C06 | rs6905466 | 19958854 | T | 0.332 | 28315 | 0.0027 | 1.11 | 0.355 | 2395 | 0.0058 | 1.09 | 0.351 | 3047 |
| C06 | rs2294809 | 20707867 | G | 0.800 | 28290 | 0.027 | 1.10 | 0.814 | 2394 | 0.053 | 1.08 | 0.811 | 3046 |
| C06 | rs9393558 | 24562175 | T | 0.347 | 28292 | 0.0028 | 1.11 | 0.370 | 2395 | 0.046 | 1.06 | 0.361 | 3046 |
| C06 | rs722086 | 25536933 | T | 0.860 | 27686 | 0.0019 | 1.16 | 0.877 | 2373 | 0.0099 | 1.12 | 0.873 | 3018 |
| C06 | rs9461366 | 27418512 | G | 0.614 | 28267 | 0.011 | 1.09 | 0.634 | 2392 | 0.1 | 1.05 | 0.626 | 3043 |
| C06 | rs149951 | 28141066 | T | 0.726 | 28316 | 0.006 | 1.11 | 0.745 | 2395 | 0.13 | 1.05 | 0.735 | 3047 |
| C06 | rs203877 | 28156603 | T | 0.729 | 27802 | 0.0058 | 1.11 | 0.749 | 2347 | 0.1 | 1.06 | 0.740 | 2978 |
| C06 | rs1635 | 28335583 | G | 0.940 | 28201 | 0.00018 | 1.32 | 0.954 | 2382 | 0.00032 | 1.27 | 0.952 | 3031 |
| C06 | rs2074464 | 29516507 | T | 0.446 | 28296 | 0.0015 | 1.11 | 0.471 | 2392 | 0.006 | 1.08 | 0.466 | 3044 |
| C06 | rs2107191 | 29542274 | G | 0.439 | 28311 | 0.00083 | 1.12 | 0.466 | 2394 | 0.0033 | 1.09 | 0.460 | 3046 |
| C06 | rs1345227 | 29574935 | G | 0.376 | 28036 | 0.00079 | 1.12 | 0.402 | 2385 | 0.0011 | 1.10 | 0.399 | 3032 |
| C06 | rs3094572 | 29588972 | A | 0.385 | 28320 | 0.0012 | 1.11 | 0.410 | 2395 | 0.0021 | 1.10 | 0.407 | 3047 |
| C06 | rs3094564 | 29594324 | G | 0.384 | 28318 | 0.00099 | 1.12 | 0.410 | 2395 | 0.0017 | 1.10 | 0.407 | 3047 |
| C06 | rs166327 | 30110860 | A | 0.601 | 28296 | 0.00011 | 1.14 | 0.631 | 2395 | 0.0024 | 1.10 | 0.622 | 3047 |
| C06 | rs1864750 | 36812785 | G | 0.253 | 28236 | 0.0024 | 1.12 | 0.274 | 2391 | 0.00097 | 1.12 | 0.274 | 3042 |
| C06 | rs236444 | 36831682 | T | 0.257 | 28318 | 0.0042 | 1.11 | 0.277 | 2394 | 0.0019 | 1.11 | 0.277 | 3046 |
| C06 | rs9296225 | 37622439 | C | 0.478 | 28316 | 0.078 | 1.06 | 0.492 | 2395 | 0.13 | 1.05 | 0.489 | 3047 |
| C06 | rs9380681 | 37638433 | C | 0.311 | 28318 | 0.038 | 1.08 | 0.327 | 2395 | 0.25 | 1.04 | 0.319 | 3047 |
| C06 | rs9296304 | 39854857 | A | 0.500 | 28315 | 0.022 | 1.08 | 0.519 | 2395 | 0.03 | 1.07 | 0.516 | 3047 |
| C06 | rs9471170 | 39888226 | G | 0.887 | 28320 | 0.0047 | 1.16 | 0.901 | 2395 | 0.061 | 1.09 | 0.896 | 3047 |
| C06 | rs713383 | 42348347 | G | 0.684 | 28317 | 0.00018 | 1.14 | 0.712 | 2395 | 0.0011 | 1.11 | 0.706 | 3047 |
| C06 | rs7756421 | 57141968 | A | 0.851 | 28291 | 0.011 | 1.13 | 0.865 | 2394 | 0.017 | 1.11 | 0.863 | 3046 |
| C06 | rs1565487 | 69237128 | A | 0.860 | 28266 | 0.0024 | 1.16 | 0.877 | 2395 | 0.0025 | 1.14 | 0.875 | 3045 |
| C06 | rs9454539 | 69273869 | A | 0.880 | 28293 | 0.0025 | 1.17 | 0.895 | 2394 | 0.0022 | 1.16 | 0.894 | 3046 |
| C06 | rs10806602 | 69291821 | T | 0.877 | 11393 | 0.0019 | 1.21 | 0.896 | 1771 | 0.0011 | 1.21 | 0.896 | 2090 |
| C06 | rs7753765 | 71732495 | A | 0.144 | 28312 | 0.00014 | 1.19 | 0.166 | 2395 | 0.000047 | 1.18 | 0.165 | 3047 |
| C06 | rs9360453 | 72211053 | G | 0.710 | 11391 | 0.00044 | 1.17 | 0.741 | 1772 | 0.00034 | 1.16 | 0.739 | 2090 |
| C06 | rs182106 | 72270027 | T | 0.521 | 28315 | 0.000019 | 1.15 | 0.556 | 2394 | 0.000015 | 1.14 | 0.553 | 3046 |
| C06 | rs199623 | 72270621 | A | 0.521 | 28319 | 0.000024 | 1.15 | 0.555 | 2394 | 0.000019 | 1.13 | 0.552 | 3046 |
| C06 | rs12207816 | 72336597 | A | 0.915 | 28321 | 0.0014 | 1.22 | 0.929 | 2395 | 0.0013 | 1.20 | 0.928 | 3047 |
| C06 | rs10943606 | 79718496 | G | 0.748 | 28313 | 0.00034 | 1.15 | 0.773 | 2395 | 0.00041 | 1.13 | 0.770 | 3046 |
| C06 | rs1338023 | 79785047 | T | 0.622 | 28072 | 0.00013 | 1.14 | 0.653 | 2337 | 0.00019 | 1.12 | 0.649 | 2984 |
| C06 | rs9352688 | 79832882 | A | 0.623 | 27921 | 0.000091 | 1.14 | 0.654 | 2347 | 0.000086 | 1.13 | 0.651 | 2991 |
| C06 | rs6454860 | 92261323 | G | 0.327 | 27828 | 0.00016 | 1.14 | 0.357 | 2331 | 0.00098 | 1.11 | 0.350 | 2979 |
| C06 | rs9373729 | 104320935 | G | 0.356 | 28116 | 0.00046 | 1.13 | 0.383 | 2392 | 0.00082 | 1.11 | 0.380 | 3041 |
| C06 | rs1149305 | 105852507 | T | 0.062 | 28237 | 0.092 | 1.12 | 0.068 | 2388 | 0.18 | 1.08 | 0.066 | 3038 |
| C06 | rs845851 | 106254239 | A | 0.769 | 28278 | 0.0014 | 1.14 | 0.791 | 2395 | 0.0067 | 1.10 | 0.786 | 3047 |
| C06 | rs4946680 | 106265726 | T | 0.638 | 28315 | 0.00093 | 1.12 | 0.663 | 2395 | 0.007 | 1.09 | 0.657 | 3047 |
| C06 | rs4440505 | 106293118 | C | 0.786 | 28320 | 0.0094 | 1.11 | 0.803 | 2394 | 0.047 | 1.08 | 0.797 | 3046 |
| C06 | rs4945955 | 113999876 | T | 0.096 | 28209 | 0.0016 | 1.18 | 0.112 | 2388 | 0.00014 | 1.20 | 0.113 | 3039 |
| C06 | rs1360782 | 119475713 | A | 0.351 | 27923 | 0.028 | 1.08 | 0.368 | 2381 | 0.0084 | 1.09 | 0.369 | 3029 |
| C06 | rs9388117 | 123058137 | A | 0.676 | 28260 | 0.015 | 1.09 | 0.695 | 2394 | 0.029 | 1.07 | 0.691 | 3045 |
| C06 | rs11154416 | 127903550 | G | 0.759 | 28194 | 0.00041 | 1.15 | 0.783 | 2379 | 0.0043 | 1.11 | 0.776 | 3028 |
| C06 | rs376632 | 130100763 | G | 0.891 | 28306 | 0.019 | 1.13 | 0.902 | 2394 | 0.14 | 1.07 | 0.898 | 3046 |
| C06 | rs9376173 | 136401139 | A | 0.636 | 28291 | 0.063 | 1.07 | 0.651 | 2386 | 0.15 | 1.05 | 0.646 | 3038 |
| C06 | rs478884 | 143072921 | A | 0.878 | 28308 | 0.0091 | 1.14 | 0.892 | 2395 | 0.0037 | 1.14 | 0.892 | 3046 |
| C06 | rs6570956 | 149604532 | T | 0.736 | 28313 | 0.0054 | 1.11 | 0.756 | 2395 | 0.006 | 1.10 | 0.754 | 3047 |
| C06 | rs4512236 | 156341799 | T | 0.421 | 28311 | 0.027 | 1.08 | 0.439 | 2395 | 0.11 | 1.05 | 0.433 | 3047 |
| C06 | rs16900254 | 157876267 | A | 0.408 | 11415 | 0.0076 | 1.11 | 0.434 | 1775 | 0.013 | 1.10 | 0.430 | 2094 |
| C06 | rs2758317 | 159987277 | C | 0.427 | 28017 | 0.00011 | 1.14 | 0.458 | 2380 | 0.002 | 1.10 | 0.450 | 3028 |
| C06 | rs11751605 | 160883220 | C | 0.111 | 28085 | 0.00098 | 1.18 | 0.128 | 2387 | 0.00039 | 1.17 | 0.128 | 3033 |
| C06 | rs7739802 | 162154323 | T | 0.493 | 28266 | 0.027 | 1.08 | 0.511 | 2393 | 0.011 | 1.08 | 0.512 | 3043 |
| C06 | rs3016536 | 162157939 | G | 0.519 | 28320 | 0.02 | 1.08 | 0.538 | 2395 | 0.013 | 1.08 | 0.537 | 3047 |
| C06 | rs875785 | 162250622 | C | 0.917 | 28294 | 0.0013 | 1.22 | 0.931 | 2391 | 0.00067 | 1.21 | 0.931 | 3043 |
| C06 | rs1744503 | 165554293 | T | 0.487 | 28235 | 0.053 | 1.07 | 0.503 | 2391 | 0.02 | 1.07 | 0.504 | 3040 |
| C06 | rs9348022 | 165948264 | T | 0.659 | 28165 | 0.022 | 1.08 | 0.677 | 2385 | 0.064 | 1.06 | 0.672 | 3032 |
| C07 | rs10267593 | 1903787 | G | 0.837 | 28151 | 0.0063 | 1.13 | 0.853 | 2387 | 0.00087 | 1.15 | 0.854 | 3036 |
| C07 | rs12234501 | 8738675 | T | 0.137 | 28284 | 0.012 | 1.12 | 0.151 | 2394 | 0.002 | 1.14 | 0.153 | 3046 |
| C07 | rs917038 | 8839252 | C | 0.787 | 28193 | 0.029 | 1.09 | 0.801 | 2380 | 0.045 | 1.08 | 0.799 | 3030 |
| C07 | rs1526543 | 11723301 | T | 0.831 | 28317 | 0.0026 | 1.14 | 0.849 | 2395 | 0.0031 | 1.13 | 0.847 | 3047 |
| C07 | rs6460851 | 11868337 | G | 0.290 | 28289 | 0.0013 | 1.12 | 0.314 | 2393 | 0.0013 | 1.11 | 0.312 | 3043 |
| C07 | rs10215881 | 11880480 | C | 0.500 | 28305 | 0.0087 | 1.09 | 0.521 | 2394 | 0.02 | 1.07 | 0.517 | 3045 |
| C07 | rs2355129 | 11892064 | A | 0.366 | 28317 | 0.0049 | 1.10 | 0.388 | 2394 | 0.004 | 1.09 | 0.386 | 3046 |
| C07 | rs3735344 | 13901213 | G | 0.422 | 28199 | 0.007 | 1.09 | 0.443 | 2379 | 0.007 | 1.08 | 0.441 | 3030 |
| C07 | rs706076 | 16813149 | G | 0.145 | 28302 | 0.0057 | 1.13 | 0.161 | 2394 | 0.073 | 1.08 | 0.154 | 3046 |
| C07 | rs1029577 | 17032434 | T | 0.318 | 28274 | 0.00028 | 1.13 | 0.346 | 2391 | 0.0015 | 1.10 | 0.340 | 3043 |
| C07 | rs11766489 | 17970195 | T | 0.427 | 28308 | 0.013 | 1.08 | 0.447 | 2394 | 0.015 | 1.08 | 0.445 | 3045 |
| C07 | rs4470902 | 17974835 | C | 0.423 | 28315 | 0.008 | 1.09 | 0.444 | 2395 | 0.011 | 1.08 | 0.441 | 3047 |
| C07 | rs2269752 | 18665810 | C | 0.930 | 28307 | 0.0068 | 1.20 | 0.941 | 2395 | 0.011 | 1.17 | 0.939 | 3047 |
| C07 | rs10486378 | 19952661 | G | 0.279 | 28001 | 0.048 | 1.07 | 0.294 | 2365 | 0.15 | 1.05 | 0.289 | 3011 |
| C07 | rs2110007 | 21159866 | C | 0.942 | 28252 | 0.00095 | 1.28 | 0.954 | 2390 | 0.0036 | 1.21 | 0.952 | 3038 |
| C07 | rs4722O54 | 21773017 | G | 0.088 | 28234 | 0.012 | 1.15 | 0.100 | 2373 | 0.052 | 1.10 | 0.097 | 3024 |
| C07 | rs1174943 | 21973643 | G | 0.159 | 28309 | 0.0049 | 1.13 | 0.176 | 2394 | 0.025 | 1.09 | 0.171 | 3046 |
| C07 | rs17146355 | 22181251 | A | 0.050 | 28301 | 0.039 | 1.16 | 0.057 | 2392 | 0.02 | 1.16 | 0.058 | 3044 |
| C07 | rs1542608 | 23521138 | G | 0.195 | 28319 | 0.003 | 1.13 | 0.214 | 2395 | 0.013 | 1.10 | 0.210 | 3047 |
| C07 | rs740127 | 28004900 | G | 0.598 | 28316 | 0.092 | 1.06 | 0.612 | 2394 | 0.48 | 1.02 | 0.603 | 3046 |
| C07 | rs10225945 | 28216608 | A | 0.849 | 28145 | 0.026 | 1.11 | 0.862 | 2391 | 0.096 | 1.07 | 0.858 | 3042 |
| C07 | rs10486588 | 28561447 | G | 0.230 | 28252 | 0.0091 | 1.10 | 0.248 | 2388 | 0.016 | 1.09 | 0.245 | 3039 |
| C07 | rs1976489 | 28576321 | G | 0.248 | 28319 | 0.011 | 1.10 | 0.266 | 2395 | 0.027 | 1.08 | 0.263 | 3047 |
| C07 | rs1357648 | 38150571 | T | 0.322 | 28233 | 0.0023 | 1.11 | 0.346 | 2386 | 0.014 | 1.08 | 0.339 | 3035 |
| C07 | rs1525798 | 39079105 | G | 0.306 | 28287 | 0.011 | 1.09 | 0.325 | 2395 | 0.011 | 1.08 | 0.324 | 3046 |
| C07 | rs11763728 | 39112387 | T | 0.335 | 28309 | 0.016 | 1.09 | 0.353 | 2394 | 0.063 | 1.06 | 0.348 | 3046 |
| C07 | rs4478468 | 39208006 | G | 0.312 | 28300 | 0.013 | 1.09 | 0.331 | 2395 | 0.037 | 1.07 | 0.326 | 3047 |
| C07 | rs3757564 | 43169187 | C | 0.072 | 28304 | 0.06 | 1.12 | 0.080 | 2391 | 0.065 | 1.11 | 0.079 | 3042 |
| C07 | rs10243723 | 46499109 | G | 0.972 | 28311 | 0.018 | 1.29 | 0.978 | 2394 | 0.045 | 1.21 | 0.977 | 3046 |
| C07 | rs956010 | 46744540 | A | 0.883 | 28321 | 0.0085 | 1.15 | 0.896 | 2395 | 0.00084 | 1.17 | 0.898 | 3047 |
| C07 | rs6956126 | 46756987 | G | 0.888 | 28318 | 0.019 | 1.13 | 0.900 | 2395 | 0.0026 | 1.16 | 0.902 | 3047 |
| C07 | rs13240380 | 49733144 | T | 0.436 | 28313 | 0.14 | 1.05 | 0.448 | 2395 | 0.1 | 1.05 | 0.448 | 3046 |
| C07 | rs12669163 | 50249131 | C | 0.300 | 27988 | 0.0062 | 1.10 | 0.320 | 2367 | 0.028 | 1.07 | 0.315 | 3015 |
| C07 | rs17134122 | 51042598 | A | 0.967 | 28320 | 0.093 | 1.17 | 0.972 | 2395 | 0.11 | 1.15 | 0.971 | 3047 |
| C07 | rs4527771 | 67522223 | A | 0.406 | 28212 | 0.00063 | 1.12 | 0.433 | 2385 | 0.016 | 1.07 | 0.423 | 3034 |
| C07 | rs12540706 | 67550019 | G | 0.313 | 28310 | 0.000047 | 1.15 | 0.344 | 2394 | 0.0011 | 1.11 | 0.335 | 3046 |
| C07 | rs7787362 | 73030539 | C | 0.562 | 28182 | 0.0075 | 1.09 | 0.583 | 2389 | 0.012 | 1.08 | 0.580 | 3038 |
| C07 | rs2192658 | 78023891 | T | 0.078 | 28313 | 0.032 | 1.13 | 0.088 | 2394 | 0.02 | 1.13 | 0.087 | 3045 |
| C07 | rs6467882 | 81513097 | C | 0.168 | 28308 | 0.024 | 1.10 | 0.182 | 2395 | 0.03 | 1.09 | 0.180 | 3045 |
| C07 | rs2373018 | 85426196 | G | 0.371 | 28312 | 0.015 | 1.09 | 0.391 | 2395 | 0.0087 | 1.08 | 0.390 | 3045 |
| C07 | rs10276025 | 85428427 | T | 0.371 | 28319 | 0.014 | 1.09 | 0.391 | 2395 | 0.0084 | 1.08 | 0.390 | 3047 |
| C07 | rs1012995 | 85495735 | C | 0.218 | 28145 | 0.029 | 1.09 | 0.233 | 2375 | 0.024 | 1.08 | 0.232 | 3022 |
| C07 | rs2373593 | 86874878 | A | 0.864 | 28311 | 0.0018 | 1.17 | 0.881 | 2395 | 0.0026 | 1.14 | 0.879 | 3047 |
| C07 | rs31662 | 86883520 | G | 0.864 | 28315 | 0.0018 | 1.17 | 0.881 | 2395 | 0.0021 | 1.15 | 0.879 | 3047 |
| C07 | rs999885 | 99539112 | T | 0.505 | 27962 | 0.02 | 1.08 | 0.524 | 2351 | 0.033 | 1.07 | 0.521 | 2997 |
| C07 | rs7804867 | 102820993 | A | 0.208 | 28315 | 0.056 | 1.08 | 0.221 | 2395 | 0.16 | 1.05 | 0.216 | 3046 |
| C07 | rs2270019 | 102844244 | G | 0.168 | 28300 | 0.0047 | 1.13 | 0.186 | 2392 | 0.012 | 1.10 | 0.182 | 3044 |
| C07 | rs17152161 | 102928939 | C | 0.158 | 28304 | 0.002 | 1.14 | 0.177 | 2395 | 0.00069 | 1.14 | 0.177 | 3047 |
| C07 | rs10282605 | 102949606 | C | 0.188 | 28303 | 0.00052 | 1.15 | 0.210 | 2395 | 0.00081 | 1.13 | 0.208 | 3047 |
| C07 | rs1029396 | 116812056 | G | 0.071 | 28315 | 0.0018 | 1.21 | 0.084 | 2395 | 0.000061 | 1.24 | 0.086 | 3047 |
| C07 | rs2283054 | 116913637 | A | 0.070 | 28319 | 0.0022 | 1.20 | 0.083 | 2394 | 0.000086 | 1.24 | 0.086 | 3046 |
| C07 | rs2237721 | 116920956 | C | 0.070 | 28314 | 0.0022 | 1.20 | 0.083 | 2395 | 0.000088 | 1.24 | 0.085 | 3047 |
| C07 | rs10487368 | 116927331 | T | 0.194 | 28319 | 0.034 | 1.09 | 0.208 | 2395 | 0.0033 | 1.11 | 0.212 | 3047 |
| C07 | rs2237723 | 116934783 | T | 0.197 | 28304 | 0.032 | 1.09 | 0.211 | 2395 | 0.0031 | 1.11 | 0.215 | 3047 |
| C07 | rs2237724 | 116941395 | A | 0.194 | 28320 | 0.034 | 1.09 | 0.208 | 2395 | 0.0033 | 1.11 | 0.212 | 3047 |
| C07 | rs2027945 | 116975058 | A | 0.194 | 28316 | 0.032 | 1.09 | 0.208 | 2395 | 0.0031 | 1.11 | 0.212 | 3047 |
| C07 | rs213977 | 117025681 | G | 0.412 | 28316 | 0.002 | 1.11 | 0.437 | 2395 | 0.00051 | 1.11 | 0.438 | 3047 |
| C07 | rs2299445 | 117033551 | C | 0.080 | 28315 | 0.00061 | 1.21 | 0.096 | 2394 | 0.000033 | 1.24 | 0.098 | 3046 |
| C07 | rs213987 | 117048529 | T | 0.328 | 28300 | 0.0036 | 1.11 | 0.351 | 2395 | 0.0017 | 1.10 | 0.350 | 3046 |
| C07 | rs12534186 | 117079572 | T | 0.081 | 28310 | 0.00056 | 1.22 | 0.096 | 2394 | 0.00003 | 1.24 | 0.098 | 3046 |
| C07 | rs975722 | 117120150 | G | 0.392 | 28318 | 0.0044 | 1.10 | 0.414 | 2395 | 0.0009 | 1.11 | 0.415 | 3047 |
| C07 | rs324594 | 136305863 | C | 0.266 | 28310 | 0.0023 | 1.12 | 0.289 | 2395 | 0.0018 | 1.11 | 0.287 | 3047 |
| C07 | rs11761344 | 136433359 | T | 0.119 | 28295 | 0.0042 | 1.15 | 0.134 | 2393 | 0.0046 | 1.13 | 0.132 | 3045 |
| C07 | rs7791708 | 144537777 | A | 0.677 | 28231 | 0.0046 | 1.11 | 0.698 | 2387 | 0.013 | 1.08 | 0.694 | 3035 |
| C07 | rs10271435 | 147385206 | A | 0.063 | 28313 | 0.05 | 1.14 | 0.071 | 2395 | 0.0065 | 1.17 | 0.073 | 3047 |
| C07 | rs10277774 | 150219733 | C | 0.390 | 28309 | 0.11 | 1.06 | 0.402 | 2394 | 0.11 | 1.05 | 0.401 | 3046 |
| C07 | rs11762978 | 150241033 | A | 0.654 | 28261 | 0.0057 | 1.10 | 0.676 | 2391 | 0.0029 | 1.10 | 0.675 | 3043 |
| C07 | rs2373885 | 150309013 | A | 0.749 | 28303 | 0.014 | 1.10 | 0.766 | 2395 | 0.013 | 1.09 | 0.765 | 3046 |
| C07 | rs11769878 | 157343230 | C | 0.741 | 27759 | 0.0011 | 1.13 | 0.764 | 2355 | 0.0054 | 1.10 | 0.759 | 2996 |
| C08 | rs722782 | 506479 | C | 0.892 | 28261 | 0.011 | 1.15 | 0.904 | 2363 | 0.026 | 1.12 | 0.902 | 3014 |
| C08 | rs7386449 | 1315247 | A | 0.681 | 28312 | 0.0066 | 1.10 | 0.701 | 2395 | 0.0044 | 1.10 | 0.700 | 3046 |
| C08 | rs10503316 | 5498753 | C | 0.776 | 28318 | 0.022 | 1.10 | 0.792 | 2395 | 0.058 | 1.07 | 0.788 | 3047 |
| C08 | rs6991017 | 5508780 | T | 0.793 | 28273 | 0.013 | 1.11 | 0.809 | 2393 | 0.044 | 1.08 | 0.805 | 3044 |
| C08 | rs890014 | 5835084 | C | 0.762 | 28320 | 0.15 | 1.06 | 0.772 | 2395 | 0.25 | 1.04 | 0.769 | 3047 |
| C08 | rs7006687 | 18277862 | T | 0.579 | 28306 | 0.0018 | 1.11 | 0.604 | 2392 | 0.00068 | 1.11 | 0.604 | 3044 |
| C08 | rs334198 | 18933089 | C | 0.922 | 28309 | 0.037 | 0.89 | 0.913 | 2393 | 0.082 | 0.91 | 0.915 | 3045 |
| C08 | rs11775256 | 23107430 | T | 0.274 | 28317 | 0.048 | 1.07 | 0.288 | 2395 | 0.029 | 1.07 | 0.288 | 3047 |
| C08 | rs4732849 | 28249765 | T | 0.275 | 28321 | 0.0069 | 1.10 | 0.295 | 2395 | 0.13 | 1.05 | 0.285 | 3047 |
| C08 | rs904053 | 28258855 | G | 0.224 | 28313 | 0.035 | 1.08 | 0.238 | 2395 | 0.43 | 1.03 | 0.229 | 3047 |
| C08 | rs12547858 | 32606595 | T | 0.193 | 28139 | 0.0023 | 1.13 | 0.213 | 2382 | 0.0065 | 1.11 | 0.209 | 3025 |
| C08 | rs10503927 | 32662772 | T | 0.476 | 28318 | 0.0011 | 1.11 | 0.503 | 2395 | 0.00025 | 1.11 | 0.503 | 3047 |
| C08 | rs9656744 | 32836025 | T | 0.509 | 28311 | 0.0033 | 1.10 | 0.533 | 2394 | 0.01 | 1.08 | 0.528 | 3044 |
| C08 | rs16880128 | 32852935 | G | 0.441 | 27756 | 0.0014 | 1.11 | 0.467 | 2362 | 0.014 | 1.08 | 0.459 | 3002 |
| C08 | rs7017907 | 37527288 | C | 0.088 | 28266 | 0.15 | 1.08 | 0.095 | 2385 | 0.86 | 1.01 | 0.089 | 3037 |
| C08 | rs7830101 | 39527585 | T | 0.278 | 28099 | 0.071 | 1.07 | 0.291 | 2372 | 0.027 | 1.08 | 0.292 | 3021 |
| C08 | rs7819949 | 41507078 | T | 0.274 | 28315 | 0.038 | 1.08 | 0.289 | 2395 | 0.32 | 1.03 | 0.281 | 3047 |
| C08 | rs7010934 | 59139573 | C | 0.843 | 28316 | 0.0004 | 1.18 | 0.864 | 2395 | 0.01 | 1.11 | 0.857 | 3047 |
| C08 | rs4738034 | 70924464 | C | 0.703 | 28321 | 0.0094 | 1.10 | 0.723 | 2395 | 0.016 | 1.08 | 0.720 | 3047 |
| C08 | rs3829049 | 70925162 | G | 0.710 | 28294 | 0.006 | 1.11 | 0.730 | 2393 | 0.011 | 1.09 | 0.727 | 3045 |
| C08 | rs7015359 | 70957101 | G | 0.186 | 28320 | 0.0028 | 1.13 | 0.205 | 2395 | 0.0083 | 1.10 | 0.201 | 3047 |
| C08 | rs10504537 | 73402234 | A | 0.881 | 28213 | 0.035 | 1.12 | 0.891 | 2387 | 0.027 | 1.11 | 0.891 | 3035 |
| C08 | rs1837544 | 73839615 | G | 0.243 | 28305 | 0.03 | 1.09 | 0.259 | 2395 | 0.15 | 1.05 | 0.253 | 3046 |
| C08 | rs10504576 | 75429234 | A | 0.482 | 28062 | 0.0036 | 1.10 | 0.506 | 2370 | 0.0083 | 1.08 | 0.502 | 3018 |
| C08 | rs4410896 | 75433049 | A | 0.484 | 28320 | 0.0045 | 1.10 | 0.507 | 2395 | 0.011 | 1.08 | 0.503 | 3047 |
| C08 | rs6996971 | 75455047 | C | 0.481 | 28218 | 0.0056 | 1.09 | 0.504 | 2388 | 0.012 | 1.08 | 0.500 | 3037 |
| C08 | rs10283076 | 75470386 | G | 0.482 | 28290 | 0.0045 | 1.10 | 0.505 | 2392 | 0.0087 | 1.08 | 0.501 | 3043 |
| C08 | rs2369121 | 79361197 | T | 0.720 | 28319 | 0.004 | 1.11 | 0.741 | 2395 | 0.0061 | 1.10 | 0.738 | 3047 |
| C08 | rs2607102 | 92586353 | T | 0.926 | 28218 | 0.0015 | 1.23 | 0.939 | 2380 | 0.0088 | 1.17 | 0.936 | 3028 |
| C08 | rs3102526 | 96858538 | G | 0.647 | 27995 | 0.013 | 1.09 | 0.666 | 2382 | 0.015 | 1.08 | 0.664 | 3019 |
| C08 | rs3134517 | 96859237 | G | 0.647 | 28308 | 0.016 | 1.09 | 0.666 | 2395 | 0.018 | 1.08 | 0.664 | 3047 |
| C08 | rs2440213 | 97275495 | A | 0.360 | 28316 | 0.0016 | 1.11 | 0.385 | 2395 | 0.0078 | 1.09 | 0.379 | 3047 |
| C08 | rs1992087 | 97552943 | G | 0.261 | 28309 | 0.019 | 1.09 | 0.278 | 2394 | 0.028 | 1.08 | 0.276 | 3046 |
| C08 | rs1075479 | 97849402 | A | 0.792 | 28280 | 0.017 | 1.10 | 0.808 | 2390 | 0.075 | 1.07 | 0.803 | 3039 |
| C08 | rs4620270 | 125506992 | T | 0.133 | 28307 | 0.082 | 1.09 | 0.143 | 2394 | 0.041 | 1.09 | 0.143 | 3046 |
| C08 | rs2648896 | 129169615 | C | 0.149 | 28304 | 0.0004 | 1.17 | 0.170 | 2395 | 0.00052 | 1.15 | 0.167 | 3047 |
| C08 | rs2302793 | 129177879 | G | 0.434 | 28273 | 0.0018 | 1.11 | 0.459 | 2382 | 0.0075 | 1.08 | 0.454 | 3033 |
| C08 | rs3931282 | 129179963 | T | 0.568 | 28319 | 0.0016 | 0.90 | 0.543 | 2395 | 0.007 | 0.92 | 0.548 | 3047 |
| C08 | rs10505507 | 129311983 | A | 0.923 | 28320 | 0.000057 | 1.30 | 0.939 | 2395 | 0.0019 | 1.20 | 0.935 | 3047 |
| C08 | rs4451334 | 138344030 | A | 0.064 | 28282 | 0.1 | 1.11 | 0.071 | 2390 | 0.13 | 1.09 | 0.070 | 3042 |
| C08 | rs4909759 | 139293683 | T | 0.263 | 28309 | 0.051 | 1.07 | 0.277 | 2395 | 0.013 | 1.09 | 0.279 | 3047 |
| C08 | rs4909761 | 139294744 | T | 0.264 | 28318 | 0.037 | 1.08 | 0.279 | 2395 | 0.0088 | 1.09 | 0.281 | 3047 |
| C08 | rs747551 | 143209084 | T | 0.433 | 28310 | 0.0092 | 1.09 | 0.454 | 2393 | 0.034 | 1.07 | 0.449 | 3045 |
| C08 | rs7834060 | 143220109 | G | 0.431 | 28316 | 0.015 | 1.08 | 0.450 | 2395 | 0.046 | 1.06 | 0.445 | 3047 |
| C08 | rs3934930 | 143226261 | T | 0.430 | 28320 | 0.014 | 1.08 | 0.450 | 2395 | 0.043 | 1.06 | 0.445 | 3047 |
| C08 | rs11167132 | 143245585 | T | 0.216 | 28136 | 0.0076 | 1.11 | 0.234 | 2383 | 0.031 | 1.08 | 0.230 | 3028 |
| C08 | rs9644545 | 143276606 | T | 0.475 | 28309 | 0.0002 | 1.13 | 0.505 | 2394 | 0.0004 | 1.11 | 0.501 | 3046 |
| C08 | rs5297 | 143952659 | C | 0.091 | 28314 | 0.003 | 1.18 | 0.105 | 2394 | 0.0072 | 1.14 | 0.102 | 3046 |
| C08 | rs7844961 | 143999186 | T | 0.092 | 28314 | 0.00094 | 1.19 | 0.108 | 2395 | 0.0035 | 1.15 | 0.105 | 3047 |
| C08 | rs4977114 | 144250835 | C | 0.028 | 28320 | 0.014 | 1.26 | 0.035 | 2395 | 0.039 | 1.19 | 0.033 | 3047 |
| C09 | rs2890795 | 8437732 | T | 0.579 | 28315 | 0.011 | 1.09 | 0.599 | 2395 | 0.0031 | 1.09 | 0.600 | 3046 |
| C09 | rs10119236 | 8506194 | A | 0.297 | 28304 | 0.042 | 1.07 | 0.313 | 2395 | 0.017 | 1.08 | 0.314 | 3047 |
| C09 | rs10960174 | 11743695 | G | 0.614 | 28312 | 0.00026 | 1.13 | 0.643 | 2392 | 0.000093 | 1.13 | 0.642 | 3043 |
| C09 | rs1343705 | 14600866 | G | 0.475 | 28306 | 0.0019 | 1.11 | 0.500 | 2394 | 0.0021 | 1.10 | 0.498 | 3046 |
| C09 | rs4961695 | 16318137 | C | 0.801 | 28297 | 0.00029 | 1.16 | 0.824 | 2392 | 0.00012 | 1.16 | 0.823 | 3042 |
| C09 | rs1888955 | 16319537 | A | 0.740 | 28315 | 0.00025 | 1.15 | 0.766 | 2395 | 0.0003 | 1.13 | 0.763 | 3047 |
| C09 | rs9407756 | 16321304 | G | 0.674 | 28318 | 0.0021 | 1.11 | 0.697 | 2395 | 0.013 | 1.08 | 0.691 | 3047 |
| C09 | rs12683609 | 16324761 | A | 0.572 | 28317 | 0.00017 | 1.13 | 0.602 | 2394 | 0.00038 | 1.11 | 0.598 | 3046 |
| C09 | rs1329044 | 23737791 | C | 0.156 | 28317 | 0.0092 | 1.12 | 0.172 | 2395 | 0.011 | 1.11 | 0.170 | 3047 |
| C09 | rs7038525 | 23762277 | T | 0.155 | 28251 | 0.0084 | 1.12 | 0.171 | 2393 | 0.0082 | 1.11 | 0.169 | 3045 |
| C09 | rs1452335 | 28113402 | G | 0.720 | 28196 | 0.018 | 1.09 | 0.737 | 2385 | 0.022 | 1.08 | 0.735 | 3036 |
| C09 | rs1452336 | 28114180 | A | 0.500 | 28241 | 0.013 | 1.09 | 0.520 | 2389 | 0.0021 | 1.10 | 0.523 | 3040 |
| C09 | rs1452337 | 28116482 | G | 0.800 | 28150 | 0.047 | 1.09 | 0.813 | 2377 | 0.078 | 1.07 | 0.810 | 3026 |
| C09 | rs2044961 | 28154645 | G | 0.573 | 28307 | 0.008 | 1.09 | 0.594 | 2395 | 0.00083 | 1.11 | 0.597 | 3046 |
| C09 | rs10758186 | 33091838 | G | 0.385 | 28316 | 0.052 | 1.07 | 0.400 | 2395 | 0.096 | 1.05 | 0.397 | 3047 |
| C09 | rs943535 | 72064166 | A | 0.920 | 28254 | 0.01 | 1.18 | 0.931 | 2385 | 0.0061 | 1.17 | 0.931 | 3034 |
| C09 | rs10867740 | 83153344 | C | 0.237 | 28282 | 0.0067 | 1.11 | 0.256 | 2394 | 0.028 | 1.08 | 0.251 | 3046 |
| C09 | rs3211650 | 91115047 | T | 0.091 | 28320 | 0.00074 | 1.20 | 0.107 | 2394 | 0.0018 | 1.17 | 0.104 | 3046 |
| C09 | rs3211683 | 91119689 | A | 0.090 | 28319 | 0.00071 | 1.20 | 0.107 | 2395 | 0.0019 | 1.17 | 0.104 | 3047 |
| C09 | rs7854755 | 106902871 | A | 0.202 | 28257 | 0.0036 | 1.12 | 0.221 | 2389 | 0.008 | 1.10 | 0.218 | 3038 |
| C09 | rs4979085 | 113970927 | T | 0.296 | 28305 | 0.048 | 1.07 | 0.311 | 2392 | 0.048 | 1.07 | 0.309 | 3044 |
| C09 | rs2236388 | 116209121 | G | 0.901 | 28319 | 0.048 | 1.12 | 0.910 | 2395 | 0.01 | 1.14 | 0.912 | 3047 |
| C09 | rs3780680 | 125819315 | T | 0.456 | 28295 | 0.021 | 1.08 | 0.475 | 2393 | 0.037 | 1.06 | 0.471 | 3045 |
| C09 | rs7026302 | 132818296 | G | 0.909 | 28320 | 0.001 | 1.22 | 0.924 | 2395 | 0.018 | 1.13 | 0.919 | 3047 |
| C09 | rs4917341 | 136289075 | C | 0.479 | 28257 | 0.00013 | 1.13 | 0.510 | 2392 | 0.000018 | 1.14 | 0.510 | 3041 |
| C09 | rs1930788 | 136303539 | C | 0.250 | 28321 | 0.0022 | 1.12 | 0.272 | 2395 | 0.0049 | 1.10 | 0.268 | 3047 |
| C09 | rs2297538 | 138684489 | G | 0.730 | 28226 | 0.0012 | 1.13 | 0.753 | 2395 | 0.00079 | 1.12 | 0.752 | 3047 |
| C09 | rs2385891 | 138716241 | A | 0.547 | 28259 | 0.0014 | 1.11 | 0.572 | 2387 | 0.011 | 1.08 | 0.565 | 3037 |
| C10 | rs12776254 | 2144862 | G | 0.171 | 28314 | 0.0054 | 1.12 | 0.189 | 2395 | 0.0048 | 1.11 | 0.187 | 3047 |
| C10 | rs4880711 | 5217871 | C | 0.351 | 27953 | 0.042 | 1.07 | 0.367 | 2379 | 0.26 | 1.04 | 0.359 | 3015 |
| C10 | rs7912210 | 5984421 | C | 0.963 | 28273 | 0.00083 | 1.37 | 0.973 | 2374 | 0.0009 | 1.33 | 0.972 | 3023 |
| C10 | rs541045 | 8232155 | C | 0.429 | 27990 | 0.00024 | 1.13 | 0.459 | 2351 | 0.00032 | 1.11 | 0.455 | 3000 |
| C10 | rs4747760 | 8313669 | G | 0.874 | 28308 | 0.0098 | 1.14 | 0.888 | 2395 | 0.019 | 1.11 | 0.885 | 3047 |
| C10 | rs7092384 | 8340796 | C | 0.861 | 28315 | 0.0091 | 1.13 | 0.875 | 2395 | 0.011 | 1.12 | 0.873 | 3047 |
| C10 | rs1419294 | 8347160 | T | 0.710 | 28320 | 0.0036 | 1.11 | 0.731 | 2395 | 0.0045 | 1.10 | 0.728 | 3047 |
| C10 | rs10508403 | 10448081 | T | 0.091 | 28314 | 0.0017 | 1.19 | 0.106 | 2395 | 0.01 | 1.14 | 0.102 | 3047 |
| C10 | rs2138930 | 15423774 | T | 0.110 | 28304 | 0.0065 | 1.15 | 0.124 | 2395 | 0.021 | 1.11 | 0.120 | 3046 |
| C10 | rs11596590 | 15455285 | T | 0.560 | 28303 | 0.02 | 1.08 | 0.579 | 2393 | 0.072 | 1.06 | 0.573 | 3044 |
| C10 | rs12269612 | 15475877 | T | 0.401 | 28314 | 0.0078 | 1.09 | 0.423 | 2395 | 0.024 | 1.07 | 0.418 | 3047 |
| C10 | rs1925819 | 15492765 | T | 0.560 | 28319 | 0.026 | 1.08 | 0.578 | 2395 | 0.087 | 1.05 | 0.572 | 3047 |
| C10 | rs1013406 | 15501576 | C | 0.562 | 28307 | 0.024 | 1.08 | 0.580 | 2392 | 0.087 | 1.05 | 0.575 | 3044 |
| C10 | rs2184383 | 16416276 | G | 0.807 | 28313 | 0.0019 | 1.14 | 0.827 | 2395 | 0.016 | 1.10 | 0.821 | 3047 |
| C10 | rs10828393 | 23329068 | C | 0.892 | 28259 | 0.0039 | 1.17 | 0.906 | 2392 | 0.012 | 1.13 | 0.903 | 3043 |
| C10 | rs993352 | 23347212 | G | 0.885 | 28271 | 0.011 | 1.14 | 0.898 | 2384 | 0.009 | 1.13 | 0.897 | 3032 |
| C10 | rs7086207 | 26227683 | G | 0.408 | 28303 | 0.00068 | 1.12 | 0.435 | 2394 | 0.0019 | 1.10 | 0.430 | 3046 |
| C10 | rs788053 | 29363574 | T | 0.747 | 28203 | 0.0021 | 1.12 | 0.768 | 2391 | 0.0029 | 1.11 | 0.766 | 3043 |
| C10 | rs7086224 | 30857283 | A | 0.419 | 28287 | 0.028 | 1.08 | 0.437 | 2393 | 0.04 | 1.06 | 0.434 | 3045 |
| C10 | rs2804453 | 33708138 | G | 0.110 | 28256 | 0.021 | 1.12 | 0.122 | 2392 | 0.051 | 1.09 | 0.119 | 3043 |
| C10 | rs2279434 | 45275070 | T | 0.067 | 28318 | 0.0029 | 1.20 | 0.080 | 2395 | 0.014 | 1.15 | 0.076 | 3047 |
| C10 | rs3793786 | 50413094 | C | 0.247 | 28319 | 0.3 | 1.04 | 0.255 | 2395 | 0.79 | 1.01 | 0.249 | 3047 |
| C10 | rs4948410 | 61786053 | A | 0.788 | 28175 | 0.15 | 1.06 | 0.797 | 2391 | 0.4 | 1.03 | 0.793 | 3041 |
| C10 | rs990828 | 66253744 | G | 0.417 | 28283 | 0.014 | 1.08 | 0.436 | 2392 | 0.021 | 1.07 | 0.434 | 3042 |
| C10 | rs17456902 | 69554012 | G | 0.874 | 28317 | 0.0021 | 1.17 | 0.890 | 2395 | 0.00092 | 1.16 | 0.889 | 3047 |
| C10 | rs1865017 | 78364450 | C | 0.737 | 28304 | 0.0091 | 1.10 | 0.755 | 2395 | 0.047 | 1.07 | 0.749 | 3047 |
| C10 | rs697171 | 78886007 | G | 0.912 | 28288 | 0.0084 | 1.17 | 0.923 | 2391 | 0.023 | 1.13 | 0.921 | 3042 |
| C10 | rs4617529 | 85064616 | G | 0.856 | 28321 | 0.0034 | 1.15 | 0.872 | 2395 | 0.0021 | 1.14 | 0.872 | 3047 |
| C10 | rs7921473 | 85156395 | G | 0.820 | 28314 | 0.0011 | 1.15 | 0.840 | 2395 | 0.0013 | 1.13 | 0.838 | 3047 |
| C10 | rs10787854 | 85162315 | A | 0.835 | 28318 | 0.0052 | 1.13 | 0.851 | 2395 | 0.0028 | 1.13 | 0.851 | 3047 |
| C10 | rs2349157 | 85169216 | G | 0.860 | 28317 | 0.0089 | 1.13 | 0.875 | 2395 | 0.014 | 1.11 | 0.873 | 3047 |
| C10 | rs4411234 | 85211638 | C | 0.648 | 28293 | 0.074 | 1.06 | 0.662 | 2395 | 0.027 | 1.07 | 0.663 | 3047 |
| C10 | rs10886432 | 85217844 | G | 0.648 | 28271 | 0.064 | 1.07 | 0.662 | 2394 | 0.023 | 1.07 | 0.663 | 3046 |
| C10 | rs11198993 | 85244450 | A | 0.697 | 28239 | 0.022 | 1.09 | 0.714 | 2387 | 0.0037 | 1.10 | 0.717 | 3038 |
| C10 | rs11199158 | 85297471 | G | 0.618 | 28319 | 0.024 | 1.08 | 0.635 | 2395 | 0.0062 | 1.09 | 0.637 | 3047 |
| C10 | rs11199316 | 85333276 | C | 0.724 | 27673 | 0.037 | 1.08 | 0.739 | 2358 | 0.03 | 1.08 | 0.738 | 3002 |
| C10 | rs10749535 | 88094506 | T | 0.478 | 28319 | 0.026 | 1.08 | 0.496 | 2395 | 0.048 | 1.06 | 0.492 | 3047 |
| C10 | rs4933391 | 88111974 | A | 0.393 | 28298 | 0.011 | 1.09 | 0.413 | 2395 | 0.021 | 1.07 | 0.409 | 3047 |
| C10 | rs3814614 | 88118261 | T | 0.508 | 28312 | 0.013 | 1.08 | 0.528 | 2394 | 0.013 | 1.08 | 0.526 | 3046 |
| C10 | rs1412444 | 90992907 | A | 0.361 | 28293 | 0.00037 | 1.13 | 0.389 | 2394 | 0.0014 | 1.10 | 0.383 | 3045 |
| C10 | rs2243548 | 91000293 | A | 0.512 | 28294 | 0.0007 | 1.12 | 0.540 | 2392 | 0.0087 | 1.08 | 0.532 | 3043 |
| C10 | rs2243547 | 91000459 | G | 0.331 | 28033 | 0.000027 | 1.15 | 0.363 | 2386 | 0.00066 | 1.11 | 0.355 | 3033 |
| C10 | rs1028935 | 93339072 | A | 0.189 | 28316 | 0.15 | 1.06 | 0.199 | 2395 | 0.25 | 1.04 | 0.196 | 3047 |
| C10 | rs10509657 | 95166923 | C | 0.103 | 28305 | 0.024 | 1.12 | 0.115 | 2395 | 0.078 | 1.09 | 0.111 | 3047 |
| C10 | rs10509680 | 96724329 | T | 0.052 | 28256 | 0.064 | 1.14 | 0.059 | 2389 | 0.17 | 1.09 | 0.057 | 3039 |
| C10 | rs1539089 | 102211414 | C | 0.224 | 28318 | 0.027 | 1.09 | 0.239 | 2395 | 0.0086 | 1.10 | 0.240 | 3047 |
| C10 | rs11190578 | 102228943 | T | 0.224 | 28317 | 0.027 | 1.09 | 0.239 | 2395 | 0.0085 | 1.10 | 0.240 | 3047 |
| C10 | rs9420802 | 102341775 | G | 0.210 | 11411 | 0.0064 | 1.14 | 0.232 | 1773 | 0.0074 | 1.13 | 0.230 | 2092 |
| C10 | rs4630219 | 102472174 | C | 0.099 | 11332 | 0.008 | 1.18 | 0.115 | 1768 | 0.012 | 1.16 | 0.113 | 2083 |
| C10 | rs11192557 | 107442673 | T | 0.878 | 28251 | 0.017 | 1.13 | 0.890 | 2390 | 0.019 | 1.11 | 0.889 | 3040 |
| C10 | rs1337430 | 108996443 | A | 0.796 | 28310 | 0.2 | 1.05 | 0.804 | 2392 | 0.25 | 1.04 | 0.803 | 3044 |
| C10 | rs1557227 | 119643657 | C | 0.859 | 28316 | 0.0093 | 1.13 | 0.873 | 2394 | 0.0018 | 1.15 | 0.874 | 3046 |
| C10 | rs7922488 | 120006858 | G | 0.536 | 28291 | 0.022 | 1.08 | 0.554 | 2385 | 0.051 | 1.06 | 0.550 | 3037 |
| C10 | rs2935689 | 123476616 | C | 0.839 | 28314 | 0.00098 | 1.16 | 0.858 | 2395 | 0.013 | 1.11 | 0.852 | 3047 |
| C10 | rs2936875 | 123481633 | T | 0.782 | 28304 | 0.00031 | 1.16 | 0.806 | 2394 | 0.0031 | 1.11 | 0.800 | 3046 |
| C10 | rs2239586 | 124239225 | T | 0.119 | 28306 | 0.0046 | 1.15 | 0.134 | 2395 | 0.011 | 1.12 | 0.131 | 3047 |
| C10 | rs639079 | 132085590 | G | 0.192 | 27862 | 0.00062 | 1.15 | 0.214 | 2354 | 0.0009 | 1.13 | 0.211 | 2998 |
| C10 | rs11597160 | 132163778 | A | 0.907 | 28320 | 0.0034 | 1.19 | 0.920 | 2395 | 0.015 | 1.14 | 0.917 | 3047 |
| C10 | rs1484652 | 132164140 | T | 0.906 | 28319 | 0.002 | 1.20 | 0.920 | 2395 | 0.0092 | 1.15 | 0.917 | 3047 |
| C10 | rs893504 | 132859084 | C | 0.073 | 28315 | 0.021 | 1.15 | 0.082 | 2395 | 0.0064 | 1.16 | 0.083 | 3047 |
| C11 | rs7122936 | 1331032 | A | 0.576 | 28230 | 0.00078 | 1.12 | 0.603 | 2395 | 0.0013 | 1.10 | 0.600 | 3046 |
| C11 | rs7944761 | 1361414 | T | 0.532 | 28309 | 0.0053 | 1.10 | 0.555 | 2395 | 0.0027 | 1.09 | 0.554 | 3047 |
| C11 | rs4963076 | 1379752 | T | 0.481 | 28301 | 0.011 | 1.09 | 0.502 | 2395 | 0.04 | 1.06 | 0.496 | 3047 |
| C11 | rs11041390 | 7432565 | A | 0.730 | 28304 | 0.0048 | 1.11 | 0.750 | 2394 | 0.013 | 1.09 | 0.746 | 3046 |
| C11 | rs7113222 | 7437034 | G | 0.740 | 28315 | 0.0037 | 1.12 | 0.760 | 2395 | 0.015 | 1.09 | 0.755 | 3047 |
| C11 | rs12292613 | 7580592 | T | 0.890 | 28149 | 0.0001 | 1.24 | 0.910 | 2362 | 0.00018 | 1.20 | 0.907 | 3011 |
| C11 | rs10500734 | 10898494 | A | 0.771 | 28271 | 0.006 | 1.12 | 0.789 | 2385 | 0.025 | 1.08 | 0.785 | 3035 |
| C11 | rs3935878 | 12963973 | T | 0.740 | 28267 | 0.000093 | 1.16 | 0.767 | 2392 | 0.00024 | 1.13 | 0.763 | 3039 |
| C11 | rs10832223 | 14142269 | T | 0.841 | 28293 | 0.083 | 1.08 | 0.851 | 2395 | 0.046 | 1.09 | 0.851 | 3047 |
| C11 | rs7946133 | 17748398 | T | 0.914 | 28127 | 0.02 | 1.15 | 0.925 | 2332 | 0.12 | 1.09 | 0.920 | 2977 |
| C11 | rs2237921 | 17894230 | C | 0.159 | 28318 | 0.0049 | 1.13 | 0.176 | 2395 | 0.014 | 1.10 | 0.172 | 3046 |
| C11 | rs3993323 | 17910658 | A | 0.143 | 28305 | 0.004 | 1.14 | 0.160 | 2392 | 0.0089 | 1.11 | 0.157 | 3043 |
| C11 | rs920671 | 20006628 | A | 0.187 | 28318 | 0.11 | 1.07 | 0.197 | 2395 | 0.2 | 1.05 | 0.195 | 3047 |
| C11 | rs920675 | 20006846 | A | 0.363 | 28319 | 0.0039 | 1.10 | 0.386 | 2394 | 0.0041 | 1.09 | 0.383 | 3046 |
| C11 | rs10836066 | 33455044 | T | 0.303 | 28316 | 0.00026 | 1.14 | 0.330 | 2395 | 0.042 | 1.07 | 0.317 | 3046 |
| C11 | rs1532096 | 42869929 | T | 0.860 | 28317 | 0.0012 | 1.17 | 0.878 | 2395 | 0.0024 | 1.14 | 0.875 | 3047 |
| C11 | rs7939657 | 42934015 | T | 0.557 | 28307 | 0.021 | 1.08 | 0.575 | 2395 | 0.018 | 1.07 | 0.574 | 3047 |
| C11 | rs10838271 | 44341504 | C | 0.216 | 28257 | 0.019 | 1.10 | 0.232 | 2392 | 0.034 | 1.08 | 0.229 | 3042 |
| C11 | rs717117 | 56651181 | G | 0.077 | 28317 | 0.037 | 1.13 | 0.086 | 2395 | 0.048 | 1.11 | 0.085 | 3047 |
| C11 | rs1005858 | 68613492 | G | 0.471 | 28318 | 0.0019 | 1.11 | 0.496 | 2395 | 0.015 | 1.07 | 0.489 | 3047 |
| C11 | rs7111999 | 68617234 | G | 0.471 | 27937 | 0.0018 | 1.11 | 0.496 | 2354 | 0.013 | 1.08 | 0.489 | 2996 |
| C11 | rs2924536 | 68626681 | A | 0.448 | 28308 | 0.0024 | 1.10 | 0.473 | 2394 | 0.012 | 1.08 | 0.467 | 3046 |
| C11 | rs4623925 | 82740177 | A | 0.270 | 28306 | 0.0021 | 1.12 | 0.293 | 2395 | 0.013 | 1.09 | 0.287 | 3047 |
| C11 | rs2163612 | 82856844 | G | 0.479 | 28294 | 0.000001 | 1.17 | 0.519 | 2393 | 0.0000036 | 1.15 | 0.513 | 3045 |
| C11 | rs7952084 | 83059967 | A | 0.358 | 28300 | 0.0018 | 1.11 | 0.382 | 2395 | 0.0061 | 1.09 | 0.377 | 3046 |
| C11 | rs1436622 | 90698546 | A | 0.686 | 28303 | 0.00056 | 1.13 | 0.712 | 2395 | 0.00023 | 1.13 | 0.711 | 3047 |
| C11 | rs2568712 | 91046953 | T | 0.389 | 28264 | 0.00072 | 1.12 | 0.416 | 2392 | 0.014 | 1.08 | 0.407 | 3043 |
| C11 | rs808003 | 91127230 | T | 0.554 | 28317 | 0.0003 | 1.13 | 0.583 | 2395 | 0.0053 | 1.09 | 0.574 | 3047 |
| C11 | rs9971402 | 92396199 | C | 0.872 | 28196 | 0.0066 | 1.15 | 0.887 | 2388 | 0.034 | 1.10 | 0.882 | 3038 |
| C11 | rs2658801 | 92856399 | A | 0.273 | 28317 | 0.023 | 1.09 | 0.290 | 2395 | 0.004 | 1.10 | 0.292 | 3047 |
| C11 | rs1039953 | 97062996 | C | 0.680 | 28302 | 0.011 | 1.09 | 0.700 | 2395 | 0.0066 | 1.09 | 0.699 | 3047 |
| C11 | rs7120353 | 98546758 | T | 0.903 | 28289 | 0.036 | 1.13 | 0.913 | 2395 | 0.017 | 1.13 | 0.914 | 3047 |
| C11 | rs1461684 | 98601689 | T | 0.854 | 28312 | 0.072 | 1.09 | 0.864 | 2395 | 0.045 | 1.09 | 0.864 | 3047 |
| C11 | rs11225090 | 101337818 | T | 0.966 | 28172 | 0.00053 | 1.42 | 0.976 | 2340 | 0.0024 | 1.31 | 0.974 | 2989 |
| C11 | rs1276252 | 102039767 | G | 0.255 | 28318 | 0.031 | 1.08 | 0.270 | 2395 | 0.09 | 1.06 | 0.266 | 3047 |
| C11 | rs719527 | 102945919 | C | 0.236 | 28278 | 0.019 | 1.09 | 0.252 | 2395 | 0.045 | 1.07 | 0.248 | 3047 |
| C11 | rs10502020 | 103220691 | G | 0.449 | 28319 | 0.0071 | 1.09 | 0.471 | 2395 | 0.0056 | 1.09 | 0.469 | 3046 |
| C11 | rs1509729 | 108432693 | G | 0.691 | 28318 | 0.0051 | 1.11 | 0.712 | 2395 | 0.0074 | 1.09 | 0.709 | 3047 |
| C11 | rs11604632 | 110013250 | A | 0.173 | 28295 | 0.001 | 1.15 | 0.194 | 2395 | 0.000026 | 1.17 | 0.197 | 3045 |
| C11 | rs531743 | 121625352 | A | 0.955 | 28306 | 0.00062 | 1.34 | 0.966 | 2393 | 0.0032 | 1.25 | 0.964 | 3045 |
| C11 | rs4936757 | 122257924 | C | 0.567 | 28307 | 0.011 | 1.09 | 0.588 | 2395 | 0.0084 | 1.08 | 0.587 | 3047 |
| C11 | rs7933433 | 127699660 | G | 0.648 | 28312 | 0.0055 | 1.10 | 0.670 | 2394 | 0.006 | 1.09 | 0.668 | 3046 |
| C11 | rs1395504 | 132234377 | A | 0.497 | 28319 | 0.033 | 1.07 | 0.514 | 2395 | 0.031 | 1.07 | 0.513 | 3047 |
| C11 | rs1289455 | 134148357 | T | 0.150 | 28259 | 0.0013 | 1.15 | 0.169 | 2391 | 0.00055 | 1.15 | 0.169 | 3042 |
| C11 | rs1289450 | 134151851 | A | 0.198 | 28099 | 0.00044 | 1.15 | 0.222 | 2390 | 0.00015 | 1.15 | 0.221 | 3039 |
| C12 | rs797773 | 6177827 | T | 0.401 | 28283 | 0.00077 | 1.12 | 0.428 | 2393 | 0.0082 | 1.08 | 0.420 | 3044 |
| C12 | rs2160424 | 9180220 | G | 0.556 | 28256 | 0.00048 | 1.12 | 0.585 | 2384 | 0.0016 | 1.10 | 0.579 | 3035 |
| C12 | rs3213831 | 9208040 | T | 0.547 | 28320 | 0.019 | 1.08 | 0.566 | 2395 | 0.012 | 1.08 | 0.566 | 3047 |
| C12 | rs3782677 | 9208670 | C | 0.548 | 28320 | 0.017 | 1.08 | 0.567 | 2395 | 0.011 | 1.08 | 0.566 | 3047 |
| C12 | rs9805106 | 19263624 | G | 0.822 | 28153 | 0.021 | 1.11 | 0.836 | 2380 | 0.0052 | 1.12 | 0.838 | 3028 |
| C12 | rs10743315 | 19360345 | G | 0.882 | 28166 | 0.023 | 1.13 | 0.894 | 2384 | 0.017 | 1.12 | 0.893 | 3035 |
| C12 | rs2417821 | 20081535 | T | 0.754 | 28317 | 0.014 | 1.10 | 0.771 | 2395 | 0.0042 | 1.11 | 0.772 | 3047 |
| C12 | rs11045018 | 20118148 | G | 0.752 | 28298 | 0.01 | 1.10 | 0.770 | 2394 | 0.0071 | 1.10 | 0.769 | 3046 |
| C12 | rs7299937 | 20196656 | T | 0.060 | 28243 | 0.0014 | 1.23 | 0.073 | 2394 | 0.0079 | 1.17 | 0.069 | 3045 |
| C12 | rs3764006 | 20945636 | C | 0.104 | 28304 | 0.022 | 1.13 | 0.116 | 2395 | 0.022 | 1.11 | 0.115 | 3047 |
| C12 | rs1910186 | 21065184 | C | 0.111 | 28301 | 0.01 | 1.14 | 0.124 | 2395 | 0.02 | 1.11 | 0.122 | 3047 |
| C12 | rs11045689 | 21092930 | G | 0.301 | 28210 | 0.017 | 1.09 | 0.319 | 2386 | 0.015 | 1.08 | 0.317 | 3037 |
| C12 | rs2955503 | 22090132 | A | 0.655 | 28289 | 0.00054 | 1.13 | 0.681 | 2394 | 0.000033 | 1.14 | 0.684 | 3045 |
| C12 | rs2171392 | 22797592 | A | 0.196 | 28289 | 0.0068 | 1.12 | 0.213 | 2394 | 0.011 | 1.10 | 0.211 | 3045 |
| C12 | rs16925384 | 22807863 | A | 0.195 | 28302 | 0.0069 | 1.12 | 0.213 | 2394 | 0.0098 | 1.10 | 0.210 | 3046 |
| C12 | rs12425518 | 47206273 | A | 0.046 | 28295 | 0.031 | 1.17 | 0.054 | 2392 | 0.15 | 1.10 | 0.051 | 3042 |
| C12 | rs12581026 | 47223943 | C | 0.048 | 28310 | 0.0073 | 1.21 | 0.058 | 2394 | 0.059 | 1.13 | 0.054 | 3046 |
| C12 | rs784563 | 52152886 | G | 0.445 | 28308 | 0.049 | 1.07 | 0.461 | 2395 | 0.12 | 1.05 | 0.457 | 3047 |
| C12 | rs6580960 | 52445647 | A | 0.544 | 28320 | 0.068 | 1.06 | 0.558 | 2395 | 0.06 | 1.06 | 0.557 | 3047 |
| C12 | rs10878126 | 62930978 | G | 0.495 | 28299 | 0.016 | 1.08 | 0.515 | 2395 | 0.017 | 1.07 | 0.513 | 3046 |
| C12 | rs1465026 | 65789635 | G | 0.837 | 28309 | 0.011 | 1.12 | 0.852 | 2395 | 0.024 | 1.10 | 0.849 | 3047 |
| C12 | rs1526839 | 65801361 | G | 0.837 | 28240 | 0.019 | 1.11 | 0.851 | 2392 | 0.037 | 1.09 | 0.848 | 3042 |
| C12 | r11835794 | 65812323 | T | 0.858 | 28321 | 0.056 | 1.10 | 0.868 | 2395 | 0.095 | 1.07 | 0.866 | 3047 |
| C12 | rs1470383 | 67493429 | C | 0.144 | 28320 | 0.0068 | 1.13 | 0.160 | 2395 | 0.0065 | 1.12 | 0.159 | 3047 |
| C12 | rs2584019 | 69318052 | T | 0.889 | 11415 | 0.00091 | 1.24 | 0.909 | 1775 | 0.0015 | 1.21 | 0.907 | 2094 |
| C12 | rs7978668 | 69325443 | T | 0.917 | 28319 | 0.0043 | 1.19 | 0.929 | 2395 | 0.0082 | 1.16 | 0.927 | 3047 |
| C12 | rs767760 | 69326403 | A | 0.914 | 28320 | 0.0041 | 1.19 | 0.927 | 2395 | 0.0068 | 1.16 | 0.925 | 3047 |
| C12 | rs7309888 | 69340682 | C | 0.794 | 28317 | 0.0034 | 1.13 | 0.813 | 2395 | 0.00095 | 1.13 | 0.814 | 3047 |
| C12 | rs7976576 | 69347673 | T | 0.795 | 28318 | 0.0049 | 1.12 | 0.813 | 2395 | 0.0015 | 1.13 | 0.813 | 3046 |
| C12 | rs949664 | 69368222 | A | 0.860 | 28228 | 0.0019 | 1.16 | 0.878 | 2393 | 0.0015 | 1.15 | 0.876 | 3043 |
| C12 | rs7135414 | 75996159 | A | 0.917 | 28317 | 0.0074 | 1.18 | 0.928 | 2395 | 0.0029 | 1.18 | 0.928 | 3047 |
| C12 | rs1401875 | 87986337 | T | 0.058 | 28291 | 0.095 | 1.12 | 0.064 | 2392 | 0.056 | 1.12 | 0.065 | 3041 |
| C12 | rs1568383 | 88017285 | T | 0.098 | 28316 | 0.008 | 1.15 | 0.111 | 2395 | 0.0049 | 1.14 | 0.110 | 3045 |
| C12 | rs11108788 | 95989532 | G | 0.036 | 27597 | 0.0085 | 1.24 | 0.044 | 2388 | 0.035 | 1.17 | 0.041 | 3040 |
| C12 | rs989264 | 96044433 | A | 0.044 | 28316 | 0.14 | 1.12 | 0.049 | 2395 | 0.18 | 1.10 | 0.049 | 3047 |
| C12 | rs7135604 | 96910191 | A | 0.657 | 28303 | 0.0022 | 1.11 | 0.680 | 2394 | 0.0076 | 1.09 | 0.676 | 3045 |
| C12 | rs864302 | 100490144 | T | 0.483 | 28309 | 0.033 | 1.07 | 0.501 | 2395 | 0.035 | 1.06 | 0.499 | 3047 |
| C12 | rs12311520 | 101693527 | C | 0.870 | 28247 | 0.09 | 1.09 | 0.879 | 2395 | 0.23 | 1.06 | 0.876 | 3046 |
| C12 | rs2247919 | 101699389 | T | 0.742 | 28307 | 0.071 | 1.07 | 0.755 | 2395 | 0.28 | 1.04 | 0.749 | 3046 |
| C12 | rs1015249 | 111609694 | C | 0.692 | 28316 | 0.0002 | 1.14 | 0.720 | 2395 | 0.00038 | 1.12 | 0.716 | 3046 |
| C12 | rs1293758 | 111917970 | A | 0.408 | 28316 | 0.0027 | 1.10 | 0.432 | 2394 | 0.0041 | 1.09 | 0.429 | 3046 |
| C12 | rs249047 | 112562237 | A | 0.614 | 28289 | 0.000091 | 1.14 | 0.645 | 2392 | 0.0015 | 1.10 | 0.637 | 3044 |
| C12 | rs1920947 | 114285453 | C | 0.810 | 28301 | 0.012 | 1.11 | 0.826 | 2394 | 0.014 | 1.10 | 0.824 | 3046 |
| C12 | rs816204 | 116492124 | A | 0.855 | 28175 | 0.0023 | 1.16 | 0.872 | 2384 | 0.0033 | 1.14 | 0.870 | 3034 |
| C12 | rs4767629 | 116739460 | T | 0.140 | 28316 | 0.025 | 1.11 | 0.153 | 2395 | 0.06 | 1.08 | 0.150 | 3047 |
| C12 | rs2592289 | 116759379 | A | 0.156 | 28311 | 0.0039 | 1.13 | 0.173 | 2395 | 0.0013 | 1.14 | 0.173 | 3047 |
| C12 | rs719450 | 119438416 | C | 0.918 | 28320 | 0.024 | 1.15 | 0.928 | 2394 | 0.019 | 1.14 | 0.928 | 3046 |
| C12 | rs3742049 | 119438873 | C | 0.919 | 28300 | 0.029 | 1.14 | 0.928 | 2394 | 0.023 | 1.13 | 0.928 | 3046 |
| C12 | rs1169081 | 120890295 | C | 0.693 | 28306 | 0.0032 | 1.11 | 0.715 | 2394 | 0.06 | 1.06 | 0.706 | 3045 |
| C12 | rs7979863 | 121629420 | A | 0.380 | 2828309 | 0.022 | 1.08 | 0.399 | 2395 | 0.0025 | 1.10 | 0.402 | 3047 |
| C12 | rs10847227 | 121632638 | T | 0.279 | 28258 | 0.012 | 1.09 | 0.297 | 2395 | 0.00028 | 1.12 | 0.303 | 3047 |
| C12 | rs4765477 | 123287223 | T | 0.045 | 28300 | 0.016 | 1.20 | 0.053 | 2395 | 0.023 | 1.17 | 0.052 | 3047 |
| C12 | rs3759107 | 123548064 | T | 0.127 | 28320 | 0.023 | 1.11 | 0.139 | 2395 | 0.014 | 1.11 | 0.139 | 3047 |
| C12 | rs12304836 | 124654446 | C | 0.143 | 28205 | 0.00054 | 1.17 | 0.163 | 2385 | 0.00095 | 1.14 | 0.160 | 3034 |
| C12 | rs7310042 | 124675580 | G | 0.192 | 28317 | 0.018 | 1.10 | 0.207 | 2395 | 0.0043 | 1.11 | 0.209 | 3047 |
| C12 | rs470477 | 128010541 | T | 0.311 | 28306 | 0.0061 | 1.10 | 0.332 | 2395 | 0.013 | 1.08 | 0.328 | 3046 |
| C13 | rs4770252 | 21699475 | A | 0.806 | 28293 | 0.0088 | 1.12 | 0.822 | 2394 | 0.016 | 1.10 | 0.819 | 3044 |
| C13 | rs9285207 | 21722774 | C | 0.200 | 28256 | 0.0015 | 1.13 | 0.220 | 2387 | 0.0061 | 1.10 | 0.216 | 3037 |
| C13 | rs1887263 | 22381716 | A | 0.204 | 28175 | 0.067 | 1.08 | 0.216 | 2393 | 0.037 | 1.08 | 0.217 | 3042 |
| C13 | rs4769613 | 28036609 | T | 0.465 | 28313 | 0.00041 | 1.12 | 0.494 | 2394 | 0.00013 | 1.12 | 0.493 | 3046 |
| C13 | rs9566343 | 38000184 | T | 0.814 | 28309 | 0.011 | 1.11 | 0.830 | 2395 | 0.013 | 1.10 | 0.828 | 3046 |
| C13 | rs9315759 | 39616680 | C | 0.132 | 28308 | 0.0045 | 1.14 | 0.147 | 2394 | 0.0045 | 1.13 | 0.146 | 3046 |
| C13 | rs9525923 | 43681715 | A | 0.210 | 28320 | 0.00015 | 1.16 | 0.236 | 2395 | 0.0019 | 1.12 | 0.229 | 3047 |
| C13 | rs963569 | 44140379 | A | 0.753 | 28315 | 0.0074 | 1.11 | 0.771 | 2395 | 0.037 | 1.08 | 0.766 | 3047 |
| C13 | rs7322927 | 46552894 | A | 0.129 | 28318 | 0.021 | 1.12 | 0.142 | 2395 | 0.059 | 1.09 | 0.139 | 3047 |
| C13 | rs9534568 | 46693565 | A | 0.769 | 11414 | 0.086 | 1.08 | 0.783 | 1775 | 0.094 | 1.08 | 0.782 | 2094 |
| C13 | rs9526329 | 46866462 | G | 0.922 | 28290 | 0.00038 | 1.26 | 0.937 | 2395 | 0.0067 | 1.17 | 0.933 | 3047 |
| C13 | rs2050972 | 54308642 | C | 0.881 | 28309 | 0.01 | 1.14 | 0.894 | 2395 | 0.011 | 1.13 | 0.893 | 3047 |
| C13 | rs1993776 | 54406486 | C | 0.872 | 28309 | 0.011 | 1.14 | 0.885 | 2395 | 0.011 | 1.12 | 0.884 | 3047 |
| C13 | rs17070671 | 67943127 | T | 0.830 | 28318 | 0.00076 | 1.16 | 0.850 | 2395 | 0.017 | 1.10 | 0.843 | 3047 |
| C13 | rs1375719 | 102208783 | C | 0.499 | 28316 | 0.032 | 1.07 | 0.517 | 2394 | 0.12 | 1.05 | 0.511 | 3046 |
| C13 | rs2038709 | 102370990 | C | 0.084 | 28237 | 0.0084 | 1.16 | 0.096 | 2393 | 0.038 | 1.11 | 0.093 | 3045 |
| C13 | rs1347329 | 104032541 | C | 0.592 | 11392 | 0.005 | 1.12 | 0.619 | 1775 | 0.031 | 1.09 | 0.612 | 2093 |
| C13 | rs354438 | 105737486 | T | 0.355 | 28306 | 0.12 | 1.05 | 0.367 | 2395 | 0.33 | 1.03 | 0.362 | 3047 |
| C13 | rs1927771 | 106592839 | A | 0.416 | 28317 | 0.05 | 1.07 | 0.432 | 2395 | 0.078 | 1.05 | 0.429 | 3046 |
| C13 | rs1163852 | 110962872 | C | 0.713 | 28319 | 0.0031 | 1.11 | 0.735 | 2395 | 0.0056 | 1.10 | 0.732 | 3046 |
| C14 | rs10872856 | 20356515 | C | 0.914 | 28314 | 0.04 | 1.13 | 0.923 | 2395 | 0.022 | 1.13 | 0.923 | 3047 |
| C14 | rs991387 | 32384515 | G | 0.803 | 28319 | 0.026 | 1.10 | 0.818 | 2394 | 0.047 | 1.08 | 0.815 | 3046 |
| C14 | rs1950216 | 32385175 | T | 0.803 | 28318 | 0.027 | 1.10 | 0.818 | 2395 | 0.048 | 1.08 | 0.815 | 3047 |
| C14 | rs1742655 | 45622604 | C | 0.885 | 28321 | 0.0015 | 1.19 | 0.901 | 2395 | 0.0049 | 1.14 | 0.898 | 3047 |
| C14 | rs8007161 | 49042790 | T | 0.886 | 28278 | 0.027 | 1.12 | 0.898 | 2393 | 0.085 | 1.09 | 0.894 | 3044 |
| C14 | rs12436454 | 49076076 | T | 0.717 | 28226 | 0.0028 | 1.12 | 0.738 | 2393 | 0.012 | 1.09 | 0.733 | 3044 |
| C14 | rs4898630 | 49269010 | A | 0.878 | 28293 | 0.0079 | 1.15 | 0.892 | 2394 | 0.022 | 1.11 | 0.889 | 3046 |
| C14 | rs1458114 | 52093597 | T | 0.706 | 27829 | 0.0005 | 1.14 | 0.732 | 2338 | 0.0043 | 1.10 | 0.725 | 2979 |
| C14 | rs4444235 | 53480669 | T | 0.536 | 28305 | 0.37 | 1.03 | 0.543 | 2394 | 0.32 | 1.03 | 0.543 | 3046 |
| C14 | rs1957860 | 53499105 | T | 0.448 | 28313 | 0.27 | 0.97 | 0.439 | 2395 | 0.55 | 0.98 | 0.444 | 3047 |
| C14 | rs392030 | 58368609 | G | 0.636 | 28319 | 0.015 | 1.09 | 0.654 | 2395 | 0.029 | 1.07 | 0.651 | 3047 |
| C14 | rs2069334 | 58368941 | A | 0.188 | 28314 | 0.043 | 1.09 | 0.201 | 2395 | 0.099 | 1.06 | 0.197 | 3047 |
| C14 | rs17095237 | 58383406 | G | 0.164 | 28314 | 0.056 | 1.09 | 0.176 | 2395 | 0.17 | 1.06 | 0.172 | 3046 |
| C14 | rs10483707 | 58477558 | A | 0.141 | 28319 | 0.054 | 1.09 | 0.152 | 2395 | 0.14 | 1.06 | 0.148 | 3047 |
| C14 | rs11158238 | 58517378 | T | 0.142 | 28317 | 0.06 | 1.09 | 0.153 | 2395 | 0.11 | 1.07 | 0.150 | 3047 |
| C14 | rs7147624 | 64935378 | G | 0.801 | 28311 | 0.011 | 1.11 | 0.817 | 2395 | 0.034 | 1.08 | 0.813 | 3046 |
| C14 | rs2411822 | 64948148 | C | 0.486 | 28314 | 0.024 | 1.08 | 0.505 | 2395 | 0.11 | 1.05 | 0.498 | 3047 |
| C14 | rs1953416 | 64948560 | T | 0.483 | 28267 | 0.016 | 1.08 | 0.503 | 2391 | 0.073 | 1.05 | 0.497 | 3042 |
| C14 | rs7145500 | 64953965 | A | 0.663 | 28319 | 0.031 | 1.08 | 0.679 | 2395 | 0.05 | 1.06 | 0.676 | 3047 |
| C14 | rs12433694 | 65380138 | C | 0.483 | 28319 | 0.031 | 1.07 | 0.500 | 2395 | 0.1 | 1.05 | 0.494 | 3047 |
| C14 | rs1147443 | 65538514 | T | 0.251 | 28316 | 0.000014 | 1.17 | 0.282 | 2395 | 0.0000074 | 1.16 | 0.280 | 3047 |
| C14 | rs1147437 | 65544105 | G | 0.380 | 28316 | 0.000065 | 1.14 | 0.412 | 2395 | 0.000058 | 1.13 | 0.409 | 3046 |
| C14 | rs894921 | 65546005 | G | 0.376 | 28318 | 0.000056 | 1.14 | 0.408 | 2395 | 0.000054 | 1.13 | 0.405 | 3047 |
| C14 | rs6573648 | 65617378 | G | 0.272 | 28316 | 0.000041 | 1.16 | 0.302 | 2395 | 0.000028 | 1.15 | 0.300 | 3047 |
| C14 | rs17754686 | 65667738 | A | 0.307 | 28239 | 0.00015 | 1.14 | 0.335 | 2392 | 0.00007 | 1.13 | 0.334 | 3044 |
| C14 | rs8003722 | 68388015 | A | 0.106 | 28317 | 0.0085 | 1.14 | 0.119 | 2394 | 0.024 | 1.11 | 0.116 | 3046 |
| C14 | rs1291302 | 69449823 | C | 0.265 | 28043 | 0.13 | 1.06 | 0.276 | 2363 | 0.22 | 1.04 | 0.273 | 3015 |
| C14 | rs10498639 | 93845279 | A | 0.428 | 28316 | 0.0088 | 1.09 | 0.449 | 2395 | 0.018 | 1.07 | 0.445 | 3047 |
| C14 | rs2281519 | 93846385 | T | 0.260 | 28281 | 0.0023 | 1.12 | 0.282 | 2395 | 0.014 | 1.09 | 0.276 | 3045 |
| C14 | rs2895811 | 99203695 | C | 0.449 | 28199 | 0.0018 | 1.11 | 0.474 | 2385 | 0.0015 | 1.10 | 0.472 | 3033 |
| C14 | rs7158073 | 99211442 | T | 0.430 | 28102 | 0.0005 | 1.12 | 0.458 | 2379 | 0.0016 | 1.10 | 0.453 | 3027 |
| C14 | rs12435918 | 99240648 | G | 0.333 | 28318 | 0.0006 | 1.12 | 0.359 | 2395 | 0.0016 | 1.10 | 0.355 | 3047 |
| C14 | rs2273840 | 99257897 | A | 0.931 | 28124 | 0.0031 | 1.22 | 0.943 | 2369 | 0.0035 | 1.20 | 0.941 | 3019 |
| C14 | rs17776453 | 100774793 | G | 0.955 | 28313 | 0.095 | 1.15 | 0.961 | 2394 | 0.033 | 1.17 | 0.962 | 3045 |
| C14 | rs4708 | 102469201 | A | 0.549 | 28293 | 0.007 | 1.09 | 0.571 | 2393 | 0.019 | 1.07 | 0.566 | 3043 |
| C14 | rs10129841 | 102473912 | A | 0.578 | 28316 | 0.0014 | 1.11 | 0.604 | 2395 | 0.012 | 1.08 | 0.596 | 3047 |
| C14 | rs8016595 | 103791279 | C | 0.756 | 28304 | 0.016 | 1.10 | 0.773 | 2395 | 0.04 | 1.07 | 0.769 | 3047 |
| C15 | rs4572353 | 24287601 | T | 0.498 | 28314 | 0.0003 | 1.13 | 0.528 | 2393 | 0.00013 | 1.12 | 0.526 | 3043 |
| C15 | rs2110209 | 24946433 | A | 0.048 | 28318 | 0.00051 | 1.28 | 0.061 | 2395 | 0.0061 | 1.20 | 0.057 | 3047 |
| C15 | rs4779984 | 30302218 | G | 0.100 | 28236 | 0.0015 | 1.18 | 0.116 | 2392 | 0.003 | 1.15 | 0.114 | 3043 |
| C15 | rs7183966 | 34836718 | G | 0.129 | 28307 | 0.00037 | 1.18 | 0.149 | 2393 | 0.0032 | 1.13 | 0.144 | 3044 |
| C15 | rs1901725 | 34837201 | T | 0.129 | 28318 | 0.00038 | 1.18 | 0.149 | 2395 | 0.0034 | 1.13 | 0.144 | 3047 |
| C15 | rs1194620 | 34902036 | T | 0.233 | 28268 | 0.0084 | 1.11 | 0.251 | 2394 | 0.042 | 1.07 | 0.246 | 3044 |
| C15 | rs1841441 | 37167818 | A | 0.471 | 28308 | 0.021 | 1.08 | 0.489 | 2393 | 0.0055 | 1.09 | 0.491 | 3045 |
| C15 | rs7172161 | 43778072 | G | 0.517 | 28267 | 0.067 | 1.06 | 0.532 | 2393 | 0.045 | 1.06 | 0.532 | 3045 |
| C15 | rs11630033 | 43850557 | C | 0.902 | 28204 | 0.065 | 1.11 | 0.911 | 2389 | 0.1 | 1.09 | 0.909 | 3040 |
| C15 | rs1156234 | 51156742 | G | 0.795 | 28314 | 0.0028 | 1.13 | 0.815 | 2395 | 0.0077 | 1.10 | 0.811 | 3047 |
| C15 | rs2553223 | 52657225 | G | 0.344 | 28301 | 0.000086 | 1.14 | 0.375 | 2395 | 0.00015 | 1.12 | 0.371 | 3047 |
| C15 | rs2414325 | 52675829 | T | 0.489 | 28318 | 0.00012 | 1.13 | 0.520 | 2395 | 0.00022 | 1.12 | 0.516 | 3047 |
| C15 | rs8041044 | 53452605 | C | 0.915 | 28200 | 0.015 | 1.16 | 0.925 | 2389 | 0.044 | 1.12 | 0.923 | 3039 |
| C15 | rs4775234 | 58117938 | C | 0.259 | 27735 | 0.0047 | 1.11 | 0.280 | 2354 | 0.031 | 1.08 | 0.273 | 2995 |
| C15 | rs11072793 | 76793497 | G | 0.231 | 28268 | 0.00025 | 1.15 | 0.257 | 2388 | 0.0013 | 1.12 | 0.251 | 3040 |
| C15 | rs4255747 | 77512872 | G | 0.677 | 28311 | 0.07 | 1.07 | 0.691 | 2395 | 0.24 | 1.04 | 0.685 | 3047 |
| C15 | rs4887480 | 84366589 | G | 0.771 | 28306 | 0.0014 | 1.13 | 0.793 | 2395 | 0.0051 | 1.11 | 0.788 | 3045 |
| C15 | rs12595195 | 88001739 | C | 0.280 | 28292 | 0.0093 | 1.10 | 0.299 | 2395 | 0.056 | 1.06 | 0.292 | 3046 |
| C15 | rs3743369 | 90508573 | T | 0.616 | 28313 | 0.032 | 1.07 | 0.633 | 2394 | 0.029 | 1.07 | 0.632 | 3046 |
| C15 | rs6496899 | 90511768 | C | 0.766 | 28152 | 0.02 | 1.10 | 0.781 | 2375 | 0.014 | 1.09 | 0.781 | 3024 |
| C15 | rs10152962 | 92112175 | G | 0.153 | 28319 | 0.014 | 1.11 | 0.168 | 2393 | 0.0078 | 1.11 | 0.167 | 3045 |
| C15 | rs176656 | 93537286 | A | 0.093 | 28317 | 0.054 | 1.11 | 0.103 | 2395 | 0.055 | 1.10 | 0.102 | 3047 |
| C15 | rs7177625 | 95764563 | G | 0.687 | 28300 | 0.029 | 1.08 | 0.703 | 2395 | 0.16 | 1.05 | 0.696 | 3046 |
| C15 | rs2684790 | 97309063 | T | 0.146 | 28314 | 0.0036 | 1.14 | 0.163 | 2395 | 0.011 | 1.11 | 0.160 | 3047 |
| C16 | rs8057913 | 2009168 | C | 0.266 | 28244 | 0.0066 | 1.10 | 0.286 | 2391 | 0.0057 | 1.10 | 0.285 | 3042 |
| C16 | rs2286472 | 2010569 | C | 0.271 | 28249 | 0.011 | 1.10 | 0.290 | 2385 | 0.007 | 1.09 | 0.289 | 3036 |
| C16 | rs8045288 | 2013121 | T | 0.269 | 28279 | 0.01 | 1.10 | 0.287 | 2394 | 0.0082 | 1.09 | 0.286 | 3045 |
| C16 | rs3785284 | 2014037 | C | 0.267 | 28138 | 0.012 | 1.10 | 0.285 | 2384 | 0.009 | 1.09 | 0.284 | 3032 |
| C16 | rs8053779 | 18979415 | G | 0.738 | 28314 | 0.12 | 1.06 | 0.749 | 2395 | 0.13 | 1.05 | 0.748 | 3047 |
| C16 | rs7500550 | 18985697 | G | 0.782 | 28319 | 0.065 | 1.08 | 0.794 | 2394 | 0.045 | 1.08 | 0.794 | 3046 |
| C16 | rs1634675 | 19801122 | G | 0.789 | 28308 | 0.023 | 1.10 | 0.804 | 2395 | 0.054 | 1.07 | 0.800 | 3047 |
| C16 | rs8050136 | 52373776 | A | 0.404 | 28316 | 0.0046 | 1.10 | 0.426 | 2394 | 0.00029 | 1.11 | 0.430 | 3046 |
| C16 | rs3751812 | 52375961 | T | 0.403 | 28246 | 0.0041 | 1.10 | 0.426 | 2392 | 0.00024 | 1.12 | 0.430 | 3044 |
| C16 | rs1033043 | 52774858 | T | 0.577 | 28311 | 0.027 | 1.08 | 0.594 | 2394 | 0.052 | 1.06 | 0.591 | 3046 |
| C16 | rs2665282 | 52779886 | A | 0.577 | 28267 | 0.029 | 1.07 | 0.594 | 2391 | 0.059 | 1.06 | 0.591 | 3041 |
| C16 | rs12924635 | 60072235 | G | 0.397 | 27765 | 0.0043 | 1.10 | 0.420 | 2306 | 0.023 | 1.07 | 0.414 | 2944 |
| C16 | rs2962080 | 61410292 | G | 0.609 | 28310 | 0.0056 | 1.10 | 0.631 | 2394 | 0.06 | 1.06 | 0.623 | 3046 |
| C16 | rs7192142 | 62724486 | T | 0.810 | 28254 | 0.00063 | 1.16 | 0.832 | 2385 | 0.0059 | 1.11 | 0.826 | 3036 |
| C16 | rs12927636 | 72310379 | G | 0.108 | 28296 | 0.0028 | 1.16 | 0.123 | 2393 | 0.0065 | 1.13 | 0.121 | 3045 |
| C16 | rs1038864 | 72320058 | C | 0.185 | 28319 | 0.012 | 1.11 | 0.201 | 2395 | 0.019 | 1.09 | 0.198 | 3047 |
| C16 | rs4243110 | 73810027 | T | 0.462 | 28299 | 0.01 | 1.09 | 0.482 | 2394 | 0.0043 | 1.09 | 0.482 | 3046 |
| C16 | rs1862737 | 73839465 | C | 0.484 | 28309 | 0.0017 | 1.11 | 0.509 | 2395 | 0.0013 | 1.10 | 0.508 | 3046 |
| C16 | rs7188879 | 78364890 | T | 0.966 | 28303 | 0.048 | 1.21 | 0.972 | 2394 | 0.11 | 1.14 | 0.970 | 3046 |
| C16 | rs1870843 | 81316815 | T | 0.602 | 28313 | 0.0081 | 1.09 | 0.623 | 2395 | 0.0098 | 1.08 | 0.621 | 3047 |
| C16 | rs10514560 | 81328576 | C | 0.734 | 28315 | 0.011 | 1.10 | 0.752 | 2395 | 0.022 | 1.08 | 0.749 | 3047 |
| C16 | rs4075964 | 86985334 | A | 0.283 | 28229 | 0.0063 | 1.10 | 0.304 | 2392 | 0.016 | 1.08 | 0.299 | 3042 |
| C16 | rs904800 | 87044441 | G | 0.383 | 28296 | 0.0057 | 1.10 | 0.405 | 2395 | 0.0062 | 1.09 | 0.403 | 3045 |
| C17 | rs4130140 | 257873 | A | 0.174 | 28319 | 0.00063 | 1.15 | 0.195 | 2395 | 0.0027 | 1.12 | 0.191 | 3047 |
| C17 | rs4791759 | 7618652 | A | 0.041 | 28305 | 0.065 | 1.16 | 0.047 | 2394 | 0.32 | 1.07 | 0.044 | 3044 |
| C17 | rs3891720 | 9331114 | C | 0.872 | 28298 | 0.0034 | 1.16 | 0.888 | 2392 | 0.026 | 1.11 | 0.883 | 3044 |
| C17 | rs9903582 | 10175559 | C | 0.749 | 28317 | 0.01 | 1.10 | 0.767 | 2395 | 0.022 | 1.08 | 0.764 | 3047 |
| C17 | rs12449610 | 13998813 | T | 0.669 | 28319 | 0.0025 | 1.11 | 0.692 | 2395 | 0.0087 | 1.09 | 0.687 | 3047 |
| C17 | rs2323095 | 14000644 | A | 0.664 | 28312 | 0.0027 | 1.11 | 0.687 | 2395 | 0.013 | 1.08 | 0.681 | 3047 |
| C17 | rs2856177 | 14041754 | A | 0.622 | 28308 | 0.0085 | 1.09 | 0.642 | 2395 | 0.016 | 1.08 | 0.639 | 3047 |
| C17 | rs4401079 | 16755421 | T | 0.211 | 27570 | 0.028 | 1.09 | 0.226 | 2340 | 0.015 | 1.09 | 0.226 | 2975 |
| C17 | rs4925108 | 17590148 | C | 0.394 | 28297 | 0.0015 | 1.11 | 0.420 | 2394 | 0.014 | 1.08 | 0.412 | 3046 |
| C17 | rs752579 | 17601072 | G | 0.390 | 28286 | 0.0051 | 1.10 | 0.412 | 2394 | 0.043 | 1.06 | 0.404 | 3046 |
| C17 | rs4925109 | 17602527 | A | 0.318 | 28316 | 0.00032 | 1.13 | 0.345 | 2395 | 0.0045 | 1.09 | 0.337 | 3047 |
| C17 | rs11649804 | 17637480 | A | 0.292 | 28148 | 0.00029 | 1.14 | 0.319 | 2341 | 0.0041 | 1.10 | 0.312 | 2987 |
| C17 | rs4925114 | 17651995 | A | 0.357 | 28266 | 0.000045 | 1.15 | 0.389 | 2391 | 0.0017 | 1.10 | 0.379 | 3042 |
| C17 | rs11868035 | 17655826 | A | 0.294 | 28278 | 0.000068 | 1.15 | 0.324 | 2393 | 0.0016 | 1.11 | 0.315 | 3043 |
| C17 | rs9902941 | 17674485 | C | 0.347 | 28319 | 0.0000029 | 1.17 | 0.384 | 2395 | 0.00095 | 1.11 | 0.371 | 3047 |
| C17 | rs4925119 | 17683629 | A | 0.345 | 28218 | 0.0000077 | 1.16 | 0.380 | 2387 | 0.0021 | 1.10 | 0.367 | 3036 |
| C17 | rs3183702 | 17688014 | T | 0.348 | 28108 | 0.0000041 | 1.17 | 0.384 | 2388 | 0.0013 | 1.10 | 0.371 | 3038 |
| C17 | rs4925125 | 17735169 | T | 0.353 | 28060 | 0.0000082 | 1.16 | 0.388 | 2374 | 0.0016 | 1.10 | 0.376 | 3021 |
| C17 | rs6502622 | 17775416 | G | 0.358 | 28303 | 0.0000065 | 1.16 | 0.393 | 2395 | 0.0019 | 1.10 | 0.380 | 3047 |
| C17 | rs2955355 | 17889200 | C | 0.285 | 28312 | 0.00000087 | 1.19 | 0.321 | 2395 | 0.00033 | 1.12 | 0.309 | 3047 |
| C17 | rs854813 | 17944570 | C | 0.385 | 28182 | 0.000011 | 1.16 | 0.420 | 2393 | 0.0031 | 1.09 | 0.406 | 3042 |
| C17 | rs854793 | 17971249 | A | 0.202 | 28313 | 0.000033 | 1.18 | 0.229 | 2395 | 0.0018 | 1.12 | 0.220 | 3047 |
| C17 | rs854787 | 17975744 | C | 0.400 | 28104 | 0.000046 | 1.14 | 0.432 | 2381 | 0.0046 | 1.09 | 0.420 | 3028 |
| C17 | rs854771 | 18000917 | C | 0.554 | 28297 | 0.00061 | 1.12 | 0.581 | 2395 | 0.00086 | 1.10 | 0.578 | 3046 |
| C17 | rs2746025 | 18092336 | G | 0.640 | 28315 | 0.0001 | 1.14 | 0.670 | 2394 | 0.0013 | 1.11 | 0.663 | 3046 |
| C17 | rs9911448 | 21030674 | C | 0.961 | 28319 | 0.023 | 1.23 | 0.968 | 2395 | 0.083 | 1.15 | 0.966 | 3047 |
| C17 | rs7210156 | 34250987 | G | 0.929 | 28256 | 0.0024 | 1.23 | 0.941 | 2392 | 0.019 | 1.15 | 0.937 | 3043 |
| C17 | rs1122326 | 37528399 | C | 0.242 | 28309 | 0.00086 | 1.13 | 0.265 | 2395 | 0.02 | 1.08 | 0.257 | 3047 |
| C17 | rs12603327 | 37544078 | C | 0.242 | 28318 | 0.00093 | 1.13 | 0.265 | 2395 | 0.021 | 1.08 | 0.256 | 3047 |
| C17 | rs7214921 | 37547551 | C | 0.242 | 28291 | 0.00095 | 1.13 | 0.265 | 2394 | 0.021 | 1.08 | 0.256 | 3045 |
| C17 | rs4794128 | 45727464 | A | 0.276 | 28309 | 0.0061 | 1.10 | 0.296 | 2395 | 0.0081 | 1.09 | 0.293 | 3047 |
| C17 | rs11656018 | 48969375 | C | 0.707 | 28240 | 0.0012 | 1.13 | 0.731 | 2394 | 0.002 | 1.11 | 0.728 | 3045 |
| C17 | rs9895713 | 50752149 | G | 0.178 | 28297 | 0.013 | 1.11 | 0.194 | 2395 | 0.03 | 1.09 | 0.191 | 3047 |
| C17 | rs1985961 | 59470994 | c | 0.413 | 28311 | 0.014 | 1.09 | 0.433 | 2395 | 0.0086 | 1.08 | 0.432 | 3047 |
| C17 | rs312750 | 65855134 | C | 0.548 | 28314 | 0.0018 | 1.11 | 0.573 | 2395 | 0.019 | 1.07 | 0.565 | 3047 |
| C17 | rs9911654 | 67172007 | T | 0.899 | 28314 | 0.0099 | 1.16 | 0.912 | 2395 | 0.037 | 1.11 | 0.908 | 3047 |
| C17 | rs2041088 | 67702720 | A | 0.722 | 28312 | 0.0041 | 1.11 | 0.743 | 2395 | 0.0028 | 1.11 | 0.741 | 3047 |
| C17 | rs2159010 | 67708572 | A | 0.730 | 28262 | 0.0097 | 1.10 | 0.748 | 2395 | 0.0053 | 1.10 | 0.748 | 3045 |
| C17 | rs191227 | 69202771 | A | 0.579 | 28300 | 0.022 | 1.08 | 0.597 | 2394 | 0.043 | 1.06 | 0.594 | 3046 |
| C17 | rs1000299 | 70660148 | A | 0.055 | 28315 | 0.0044 | 1.21 | 0.066 | 2394 | 0.015 | 1.16 | 0.063 | 3046 |
| C17 | rs17674253 | 74732189 | A | 0.583 | 28311 | 0.032 | 1.07 | 0.600 | 2393 | 0.071 | 1.06 | 0.596 | 3045 |
| C18 | rs1791098 | 3294629 | C | 0.763 | 28318 | 0.022 | 1.09 | 0.779 | 2395 | 0.24 | 1.04 | 0.770 | 3047 |
| C18 | rs12964045 | 13442635 | C | 0.131 | 28312 | 0.015 | 1.12 | 0.145 | 2394 | 0.031 | 1.10 | 0.142 | 3046 |
| C18 | rs4271662 | 26051291 | C | 0.587 | 28256 | 0.061 | 1.06 | 0.602 | 2390 | 0.092 | 1.05 | 0.599 | 3039 |
| C18 | rs12964741 | 35159625 | A | 0.668 | 28119 | 0.0049 | 1.10 | 0.689 | 2359 | 0.0013 | 1.11 | 0.690 | 3008 |
| C18 | rs1015735 | 39877321 | C | 0.267 | 28220 | 0.035 | 1.08 | 0.283 | 2387 | 0.15 | 1.05 | 0.277 | 3037 |
| C18 | rs4340393 | 39884052 | T | 0.266 | 28320 | 0.033 | 1.08 | 0.282 | 2395 | 0.13 | 1.05 | 0.276 | 3047 |
| C18 | rs17734724 | 43268749 | G | 0.276 | 28318 | 0.00071 | 1.13 | 0.300 | 2395 | 0.0024 | 1.10 | 0.296 | 3047 |
| C18 | rs1790778 | 45715039 | G | 0.512 | 28314 | 0.00027 | 1.13 | 0.541 | 2394 | 0.000024 | 1.13 | 0.543 | 3046 |
| C18 | rs1557359 | 45734896 | G | 0.408 | 28317 | 0.00063 | 1.12 | 0.435 | 2395 | 0.00016 | 1.12 | 0.435 | 3047 |
| C18 | rs634285 | 53273824 | G | 0.913 | 11415 | 0.00075 | 1.28 | 0.930 | 1775 | 0.0036 | 1.22 | 0.927 | 2094 |
| C18 | rs580647 | 53284831 | A | 0.886 | 28267 | 0.034 | 1.12 | 0.897 | 2394 | 0.057 | 1.09 | 0.895 | 3045 |
| C18 | rs4940582 | 53743264 | G | 0.851 | 28314 | 0.0016 | 1.16 | 0.869 | 2394 | 0.0016 | 1.14 | 0.867 | 3046 |
| C18 | rs657773 | 55097571 | C | 0.556 | 28318 | 0.00062 | 1.12 | 0.583 | 2395 | 0.012 | 1.08 | 0.574 | 3047 |
| C18 | rs4806 | 55146376 | C | 0.285 | 28175 | 0.016 | 1.09 | 0.303 | 2376 | 0.049 | 1.07 | 0.299 | 3025 |
| C18 | rs9319948 | 55193376 | A | 0.285 | 28174 | 0.025 | 1.08 | 0.302 | 2387 | 0.074 | 1.06 | 0.297 | 3033 |
| C18 | rs8088514 | 55213078 | C | 0.461 | 28276 | 0.0093 | 1.09 | 0.482 | 2386 | 0.037 | 1.06 | 0.476 | 3038 |
| C18 | rs8082768 | 55792708 | T | 0.182 | 28281 | 0.007 | 1.12 | 0.200 | 2390 | 0.01 | 1.10 | 0.197 | 3040 |
| C18 | rs9948303 | 55911746 | G | 0.102 | 28316 | 0.084 | 1.10 | 0.110 | 2395 | 0.0079 | 1.13 | 0.114 | 3047 |
| C18 | rs603119 | 56407631 | T | 0.391 | 28278 | 0.072 | 1.06 | 0.406 | 2394 | 0.021 | 1.07 | 0.408 | 3045 |
| C18 | rs2332026 | 56572162 | A | 0.505 | 28318 | 0.059 | 1.06 | 0.520 | 2395 | 0.051 | 1.06 | 0.519 | 3047 |
| C18 | rs3744927 | 58396227 | G | 0.772 | 28317 | 0.0024 | 1.13 | 0.793 | 2395 | 0.017 | 1.09 | 0.787 | 3047 |
| C18 | rs2332575 | 58449327 | A | 0.054 | 28302 | 0.014 | 1.18 | 0.064 | 2392 | 0.028 | 1.15 | 0.062 | 3044 |
| C18 | rs7241455 | 58476248 | T | 0.063 | 28318 | 0.0045 | 1.20 | 0.075 | 2395 | 0.0039 | 1.18 | 0.074 | 3047 |
| C18 | rs12458349 | 58505190 | G | 0.061 | 28155 | 0.0014 | 1.23 | 0.074 | 2389 | 0.0013 | 1.21 | 0.073 | 3036 |
| C18 | rs1365254 | 70238283 | T | 0.196 | 28319 | 0.066 | 1.08 | 0.208 | 2395 | 0.036 | 1.08 | 0.209 | 3047 |
| C18 | rs4892210 | 70241069 | A | 0.196 | 28316 | 0.057 | 1.08 | 0.209 | 2395 | 0.031 | 1.08 | 0.209 | 3047 |
| C18 | rs2850889 | 73087595 | C | 0.409 | 28316 | 0.00013 | 1.13 | 0.440 | 2395 | 0.00015 | 1.12 | 0.437 | 3046 |
| C18 | rs2156643 | 73088668 | G | 0.409 | 28304 | 0.00013 | 1.13 | 0.440 | 2395 | 0.00015 | 1.12 | 0.436 | 3047 |
| C18 | rs2850892 | 73088829 | C | 0.409 | 28292 | 0.00014 | 1.13 | 0.440 | 2393 | 0.00016 | 1.12 | 0.436 | 3043 |
| C19 | rs10414677 | 330003 | T | 0.285 | 27883 | 0.048 | 1.07 | 0.299 | 2322 | 0.27 | 1.04 | 0.292 | 2956 |
| C19 | rs758503 | 446722 | T | 0.302 | 27981 | 0.012 | 1.09 | 0.321 | 2371 | 0.011 | 1.08 | 0.319 | 3018 |
| C19 | rs4740 | 4187996 | A | 0.274 | 28312 | 0.017 | 1.09 | 0.291 | 2395 | 0.054 | 1.07 | 0.286 | 3047 |
| C19 | rs1045750 | 4202069 | C | 0.269 | 28318 | 0.021 | 1.09 | 0.286 | 2394 | 0.054 | 1.07 | 0.281 | 3046 |
| C19 | rs4807567 | 4229502 | T | 0.427 | 28263 | 0.07 | 1.06 | 0.442 | 2394 | 0.07 | 1.06 | 0.441 | 3045 |
| C19 | rs10419363 | 4231186 | T | 0.306 | 28279 | 0.076 | 1.06 | 0.319 | 2390 | 0.069 | 1.06 | 0.318 | 3040 |
| C19 | rs10406508 | 8795907 | T | 0.764 | 1111415 | 0.0031 | 1.15 | 0.788 | 1775 | 0.0092 | 1.12 | 0.784 | 2094 |
| C19 | rs7248277 | 14236895 | C | 0.570 | 28306 | 0.0065 | 1.09 | 0.592 | 2394 | 0.0034 | 1.09 | 0.591 | 3045 |
| C19 | rs2305758 | 17024661 | G | 0.691 | 28308 | 0.0044 | 1.11 | 0.712 | 2394 | 0.0026 | 1.10 | 0.711 | 3046 |
| C19 | rs2278997 | 17027981 | T | 0.229 | 28315 | 0.0000018 | 1.20 | 0.262 | 2395 | 0.0000088 | 1.16 | 0.257 | 3047 |
| C19 | rs12459084 | 17031885 | T | 0.350 | 27962 | 0.00013 | 1.14 | 0.380 | 2387 | 0.002 | 1.10 | 0.372 | 3037 |
| C19 | rs7254154 | 17039119 | C | 0.352 | 28271 | 0.00019 | 1.13 | 0.381 | 2389 | 0.0028 | 1.10 | 0.373 | 3041 |
| C19 | rs7246865 | 17080105 | A | 0.205 | 28321 | 0.00016 | 1.16 | 0.230 | 2395 | 0.00025 | 1.14 | 0.227 | 3047 |
| C19 | rs4933027 | 22695812 | T | 0.083 | 28318 | 0.00019 | 1.23 | 0.101 | 2395 | 0.0011 | 1.18 | 0.097 | 3047 |
| C19 | rs4805463 | 34828875 | G | 0.909 | 28316 | 0.0018 | 1.20 | 0.923 | 2395 | 0.0073 | 1.15 | 0.920 | 3047 |
| C19 | rs346535 | 48943265 | T | 0.234 | 28317 | 0.0031 | 1.12 | 0.255 | 2395 | 0.00078 | 1.12 | 0.255 | 3047 |
| C19 | rs377702 | 50054507 | T | 0.391 | 28304 | 0.007 | 1.09 | 0.412 | 2395 | 0.014 | 1.08 | 0.409 | 3047 |
| C19 | rs3760802 | 53768499 | A | 0.505 | 28309 | 0.073 | 1.06 | 0.519 | 2392 | 0.13 | 1.05 | 0.516 | 3044 |
| C19 | rs11668495 | 58638423 | C | 0.940 | 28270 | 0.065 | 1.14 | 0.947 | 2392 | 0.12 | 1.11 | 0.945 | 3044 |
| C19 | rs8113561 | 62070079 | G | 0.954 | 28311 | 0.0055 | 1.26 | 0.963 | 2395 | 0.025 | 1.18 | 0.960 | 3047 |
| C20 | rs723477 | 237362 | T | 0.742 | 28284 | 0.004 | 1.12 | 0.762 | 2389 | 0.0029 | 1.11 | 0.761 | 3040 |
| C20 | rs1741285 | 4064436 | G | 0.560 | 28300 | 0.00075 | 1.12 | 0.588 | 2392 | 0.00072 | 1.11 | 0.585 | 3043 |
| C20 | rs6084644 | 4069799 | G | 0.437 | 28319 | 0.00096 | 1.11 | 0.464 | 2395 | 0.00062 | 1.11 | 0.462 | 3047 |
| C20 | rs6076623 | 4084671 | T | 0.367 | 28302 | 0.000045 | 1.15 | 0.399 | 2395 | 0.0000036 | 1.15 | 0.400 | 3047 |
| C20 | rs1741315 | 4103948 | A | 0.484 | 28317 | 0.0014 | 1.11 | 0.510 | 2395 | 0.0012 | 1.10 | 0.508 | 3047 |
| C20 | rs1741318 | 4105600 | A | 0.450 | 28315 | 0.0025 | 1.10 | 0.474 | 2394 | 0.0013 | 1.10 | 0.473 | 3046 |
| C20 | rs236110 | 5881108 | G | 0.871 | 28299 | 0.00079 | 1.19 | 0.889 | 2393 | 0.0036 | 1.14 | 0.885 | 3044 |
| C20 | rs236113 | 5882566 | T | 0.883 | 28321 | 0.001 | 1.19 | 0.900 | 2395 | 0.0063 | 1.14 | 0.896 | 3047 |
| C20 | rs2026058 | 13639900 | T | 0.768 | 28319 | 0.048 | 1.08 | 0.781 | 2395 | 0.084 | 1.06 | 0.779 | 3047 |
| C20 | rs6079235 | 13886789 | C | 0.736 | 28308 | 0.096 | 1.06 | 0.748 | 2394 | 0.12 | 1.05 | 0.746 | 3046 |
| C20 | rs411944 | 15006882 | G | 0.651 | 28275 | 0.0033 | 1.11 | 0.673 | 2392 | 0.035 | 1.07 | 0.665 | 3044 |
| C20 | rs6034134 | 15182479 | G | 0.596 | 28318 | 0.026 | 1.08 | 0.614 | 2395 | 0.065 | 1.06 | 0.610 | 3047 |
| C20 | rs199812 | 22264552 | C | 0.888 | 28288 | 0.0074 | 1.15 | 0.902 | 2394 | 0.0026 | 1.16 | 0.902 | 3046 |
| C20 | rs734484 | 31943651 | C | 0.713 | 28316 | 0.079 | 1.07 | 0.726 | 2394 | 0.1 | 1.06 | 0.724 | 3045 |
| C20 | rs981303 | 45947245 | A | 0.931 | 28301 | 0.057 | 1.14 | 0.939 | 2391 | 0.011 | 1.17 | 0.941 | 3043 |
| C20 | rs4811008 | 48277796 | G | 0.463 | 27959 | 0.00032 | 1.13 | 0.493 | 2348 | 0.0065 | 1.09 | 0.483 | 2993 |
| C20 | rs913670 | 48339923 | C | 0.634 | 28276 | 0.00081 | 1.12 | 0.660 | 2395 | 0.0051 | 1.09 | 0.654 | 3046 |
| C20 | rs913673 | 48341447 | C | 0.541 | 28304 | 0.00017 | 1.13 | 0.571 | 2395 | 0.0029 | 1.09 | 0.563 | 3047 |
| C20 | rs6068930 | 52700213 | G | 0.040 | 28199 | 0.013 | 1.22 | 0.048 | 2389 | 0.021 | 1.18 | 0.046 | 3039 |
| C20 | rs486921 | 56349782 | C | 0.854 | 28319 | 0.0042 | 1.15 | 0.870 | 2394 | 0.013 | 1.11 | 0.867 | 3046 |
| C20 | rs6128327 | 56375963 | A | 0.647 | 27522 | 0.029 | 1.08 | 0.664 | 2355 | 0.079 | 1.06 | 0.660 | 2992 |
| C20 | rs6128397 | 56686538 | G | 0.690 | 28315 | 0.012 | 1.09 | 0.708 | 2395 | 0.031 | 1.07 | 0.704 | 3047 |
| C20 | rs6128843 | 58519280 | T | 0.183 | 28319 | 0.00057 | 1.15 | 0.205 | 2395 | 0.0027 | 1.12 | 0.201 | 3047 |
| C21 | rs2822391 | 14334270 | A | 0.553 | 28310 | 0.021 | 1.08 | 0.572 | 2394 | 0.0088 | 1.08 | 0.572 | 3045 |
| C21 | rs2822744 | 14821123 | A | 0.158 | 28318 | 0.026 | 1.10 | 0.171 | 2395 | 0.045 | 1.08 | 0.169 | 3047 |
| C21 | rs2822753 | 14824849 | G | 0.356 | 28297 | 0.057 | 1.07 | 0.371 | 2394 | 0.13 | 1.05 | 0.367 | 3046 |
| C21 | rs1349227 | 16738666 | T | 0.242 | 28301 | 0.018 | 1.09 | 0.258 | 2393 | 0.021 | 1.08 | 0.256 | 3045 |
| C21 | rs2017705 | 21803122 | C | 0.074 | 28287 | 0.0091 | 1.17 | 0.085 | 2390 | 0.058 | 1.11 | 0.081 | 3041 |
| C21 | rs2828495 | 24038081 | A | 0.212 | 28316 | 0.001 | 1.14 | 0.234 | 2395 | 0.00089 | 1.12 | 0.232 | 3047 |
| C21 | rs2828515 | 24056599 | G | 0.124 | 28296 | 0.0093 | 1.13 | 0.138 | 2394 | 0.0024 | 1.14 | 0.139 | 3046 |
| C21 | rs2829040 | 24693776 | A | 0.452 | 28251 | 0.047 | 1.07 | 0.468 | 2392 | 0.23 | 1.04 | 0.460 | 3044 |
| C21 | rs2830357 | 26972748 | G | 0.575 | 28313 | 0.0019 | 1.11 | 0.600 | 2395 | 0.0025 | 1.10 | 0.597 | 3046 |
| C21 | rs2251540 | 26986173 | G | 0.651 | 28262 | 0.0027 | 1.11 | 0.674 | 2391 | 0.0089 | 1.09 | 0.669 | 3042 |
| C21 | rs1882757 | 40711890 | A | 0.507 | 28320 | 0.079 | 1.06 | 0.522 | 2393 | 0.068 | 1.06 | 0.521 | 3045 |
| C21 | rs3788014 | 42722104 | C | 0.925 | 28314 | 0.0012 | 1.24 | 0.938 | 2395 | 0.0019 | 1.20 | 0.937 | 3046 |
| C22 | rs2019061 | 17343340 | T | 0.689 | 28294 | 0.01 | 1.10 | 0.708 | 2388 | 0.0051 | 1.09 | 0.708 | 3038 |
| C22 | rs1978058 | 18288219 | T | 0.372 | 28137 | 0.000015 | 1.16 | 0.406 | 2388 | 0.00038 | 1.11 | 0.397 | 3034 |
| C22 | rs3804044 | 18432442 | T | 0.858 | 28284 | 0.0037 | 1.15 | 0.875 | 2395 | 0.0039 | 1.13 | 0.873 | 3046 |
| C22 | rs5992507 | 18437458 | A | 0.859 | 28309 | 0.004 | 1.15 | 0.875 | 2395 | 0.0042 | 1.13 | 0.873 | 3047 |
| C22 | rs2073778 | 18454575 | C | 0.858 | 28316 | 0.0036 | 1.15 | 0.875 | 2395 | 0.0046 | 1.13 | 0.873 | 3047 |
| C22 | rs2238798 | 18487729 | G | 0.859 | 28289 | 0.0045 | 1.15 | 0.875 | 2394 | 0.0057 | 1.13 | 0.873 | 3044 |
| C22 | rs178260 | 19654308 | T | 0.142 | 28310 | 0.029 | 1.10 | 0.154 | 2395 | 0.039 | 1.09 | 0.152 | 3047 |
| C22 | rs430305 | 19688522 | T | 0.829 | 28319 | 0.0096 | 1.12 | 0.844 | 2395 | 0.038 | 1.09 | 0.840 | 3047 |
| C22 | rs8135273 | 32527802 | C | 0.157 | 28046 | 0.025 | 1.10 | 0.170 | 2369 | 0.057 | 1.08 | 0.167 | 3012 |
| C22 | rs2267296 | 32549939 | G | 0.444 | 28313 | 0.0015 | 1.11 | 0.470 | 2394 | 0.00076 | 1.10 | 0.469 | 3046 |
| C22 | rs2071744 | 34147553 | C | 0.782 | 28278 | 0.012 | 1.11 | 0.798 | 2394 | 0.038 | 1.08 | 0.794 | 3044 |
| C22 | rs132717 | 34926598 | C | 0.618 | 28303 | 0.069 | 1.06 | 0.633 | 2395 | 0.56 | 1.02 | 0.622 | 3047 |
| C22 | rs7364143 | 34932129 | T | 0.279 | 28278 | 0.00042 | 1.13 | 0.305 | 2391 | 0.0024 | 1.10 | 0.299 | 3043 |
| C22 | rs5995259 | 34941809 | A | 0.338 | 28320 | 0.00058 | 1.12 | 0.365 | 2395 | 0.004 | 1.09 | 0.358 | 3047 |
| C22 | rs763086 | 34949003 | A | 0.301 | 28254 | 0.0059 | 1.10 | 0.322 | 2391 | 0.014 | 1.08 | 0.318 | 3038 |
| C22 | rs738407 | 42655288 | A | 0.640 | 28301 | 0.0014 | 1.12 | 0.664 | 2394 | 0.0018 | 1.10 | 0.662 | 3046 |
| C22 | rs739164 | 45063222 | A | 0.892 | 27998 | 0.0056 | 1.17 | 0.906 | 2330 | 0.018 | 1.12 | 0.903 | 2978 |
| CX | rs6638525 | 5464517 | C | 0.110 | 22852 | 0.016 | 1.16 | 0.126 | 1545 | 0.017 | 1.14 | 0.124 | 1962 |
| CX | rs1919026 | 5471853 | C | 0.195 | 22929 | 0.04 | 1.11 | 0.212 | 1550 | 0.22 | 1.06 | 0.204 | 1967 |
| CX | rs7892324 | 6679297 | C | 0.167 | 22905 | 0.0011 | 1.19 | 0.192 | 1542 | 0.005 | 1.14 | 0.186 | 1956 |
| CX | rs3013140 | 41283447 | G | 0.933 | 22939 | 0.042 | 1.19 | 0.943 | 1551 | 0.037 | 1.17 | 0.943 | 1968 |
| CX | rs5914778 | 56774956 | G | 0.804 | 22928 | 0.002 | 1.18 | 0.828 | 1550 | 0.0032 | 1.15 | 0.825 | 1966 |
| CX | rs2031751 | 67165559 | C | 0.941 | 22937 | 0.08 | 1.17 | 0.949 | 1550 | 0.051 | 1.17 | 0.949 | 1967 |
| CX | rs1576059 | 67171306 | T | 0.941 | 22937 | 0.076 | 1.17 | 0.949 | 1551 | 0.056 | 1.17 | 0.949 | 1968 |
| CX | rs7050085 | 67278825 | G | 0.908 | 22941 | 0.015 | 1.19 | 0.922 | 1550 | 0.0059 | 1.20 | 0.922 | 1967 |
| CX | rs2341629 | 70367614 | C | 0.470 | 22923 | 0.093 | 1.07 | 0.487 | 1550 | 0.16 | 1.05 | 0.483 | 1967 |
| CX | rs2213468 | 86099449 | G | 0.668 | 22877 | 0.047 | 1.09 | 0.687 | 1541 | 0.15 | 1.06 | 0.680 | 1958 |
| CX | rs6615536 | 92662125 | G | 0.988 | 22935 | 0.0094 | 1.77 | 0.993 | 1551 | 0.00098 | 1.97 | 0.994 | 1968 |
| CX | rs2227165 | 92880063 | C | 0.899 | 8732 | 0.00000074 | 1.56 | 0.933 | 1121 | 0.00013 | 1.37 | 0.924 | 1314 |
| CX | rs3788769 | 109423718 | C | 0.441 | 22401 | 0.0016 | 1.14 | 0.473 | 1534 | 0.0018 | 1.12 | 0.470 | 1944 |
| CX | rs2526763 | 116328390 | G | 0.365 | 22923 | 0.006 | 1.12 | 0.392 | 1549 | 0.0083 | 1.10 | 0.388 | 1965 |
| CX | rs1948488 | 139835516 | A | 0.222 | 22466 | 0.043 | 1.10 | 0.239 | 1527 | 0.02 | 1.11 | 0.240 | 1937 |
| CX | rs11094472 | 144273211 | G | 0.787 | 22928 | 0.00017 | 1.21 | 0.818 | 1545 | 0.004 | 1.14 | 0.808 | 1962 |

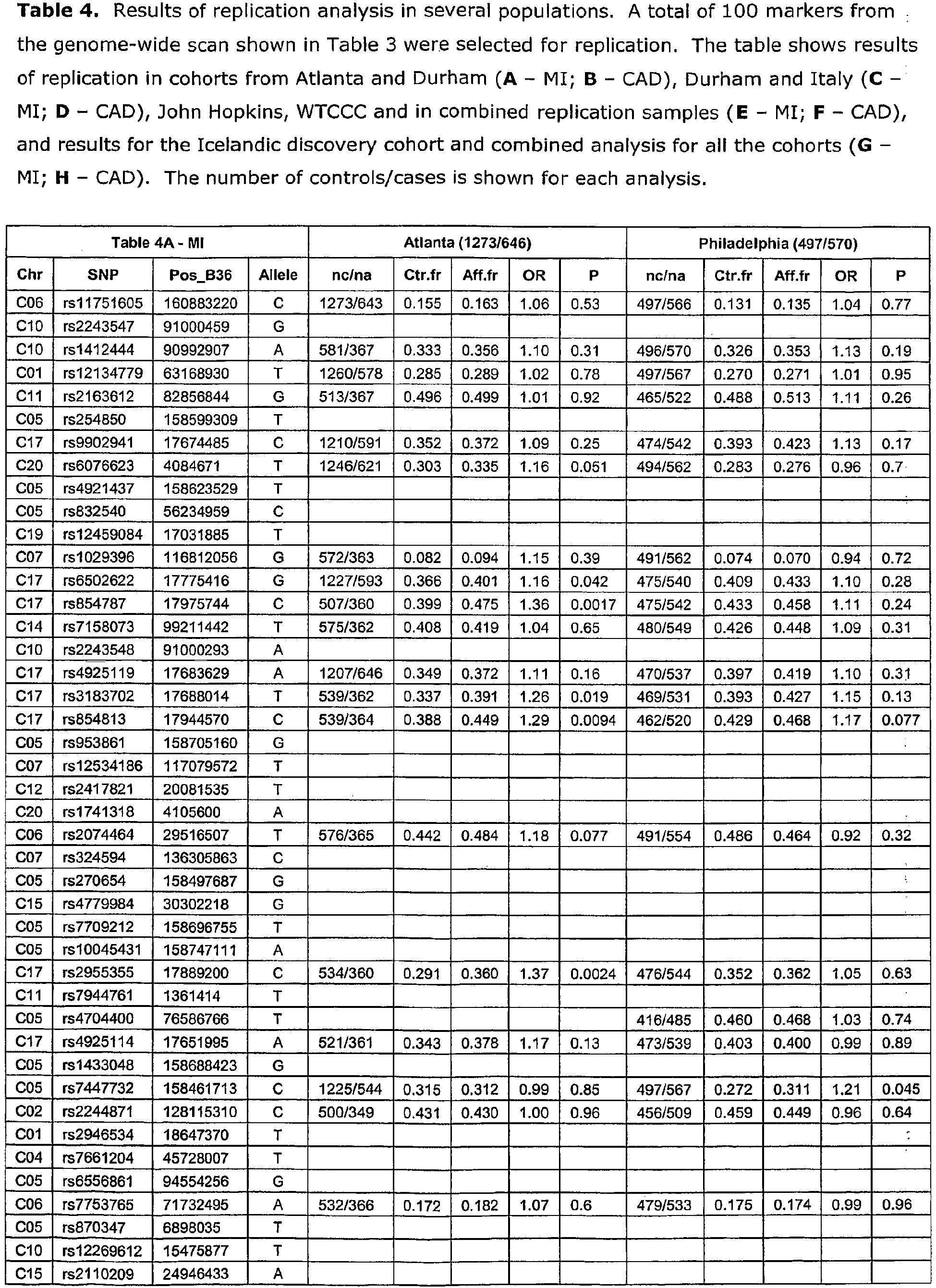

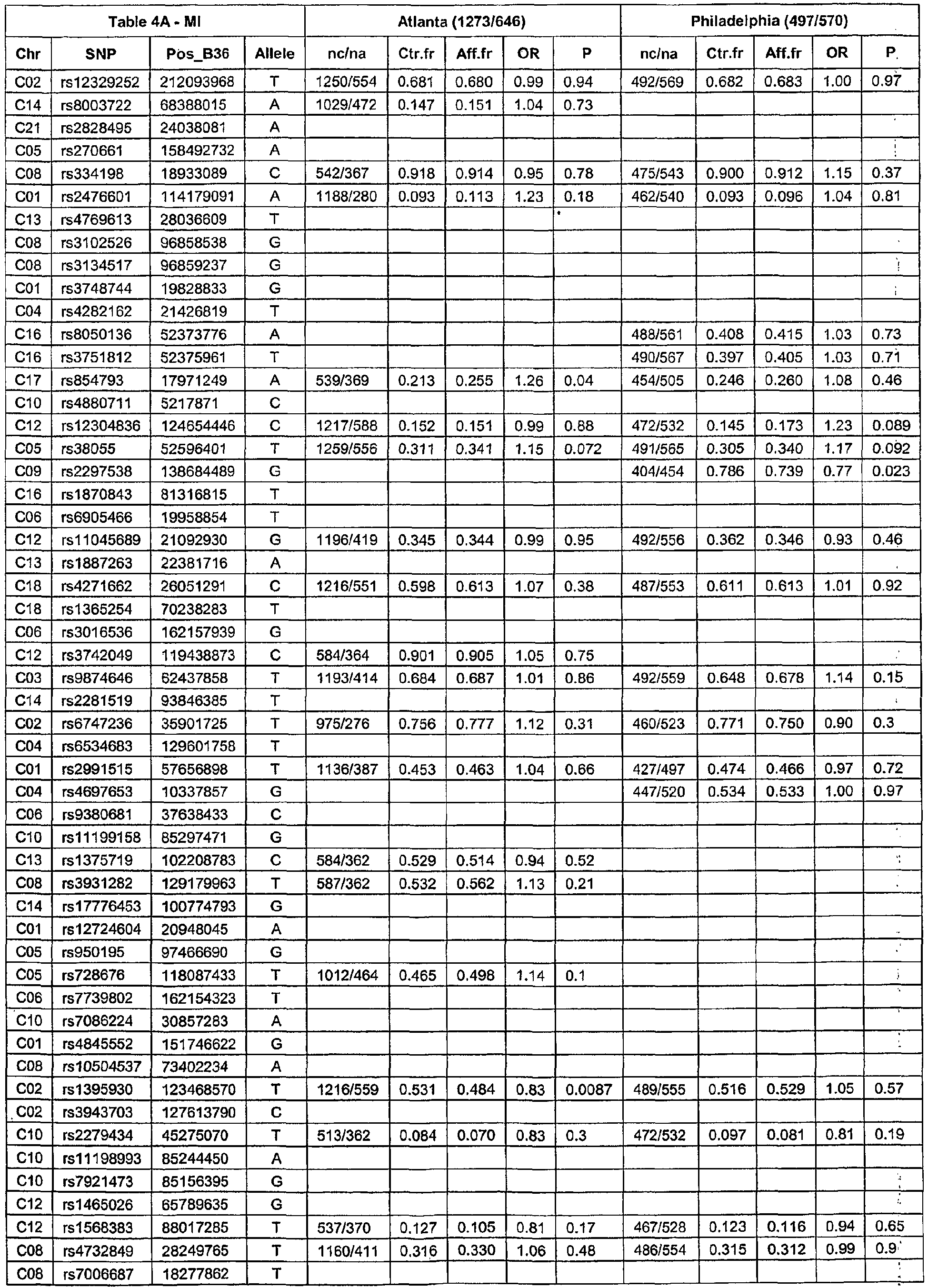

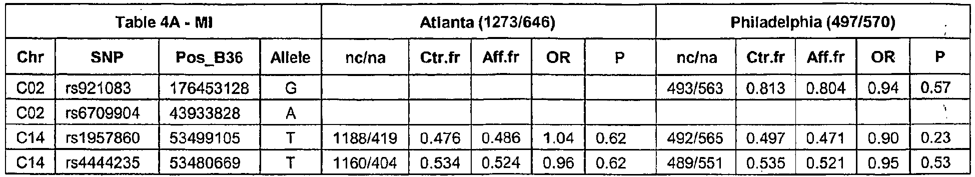

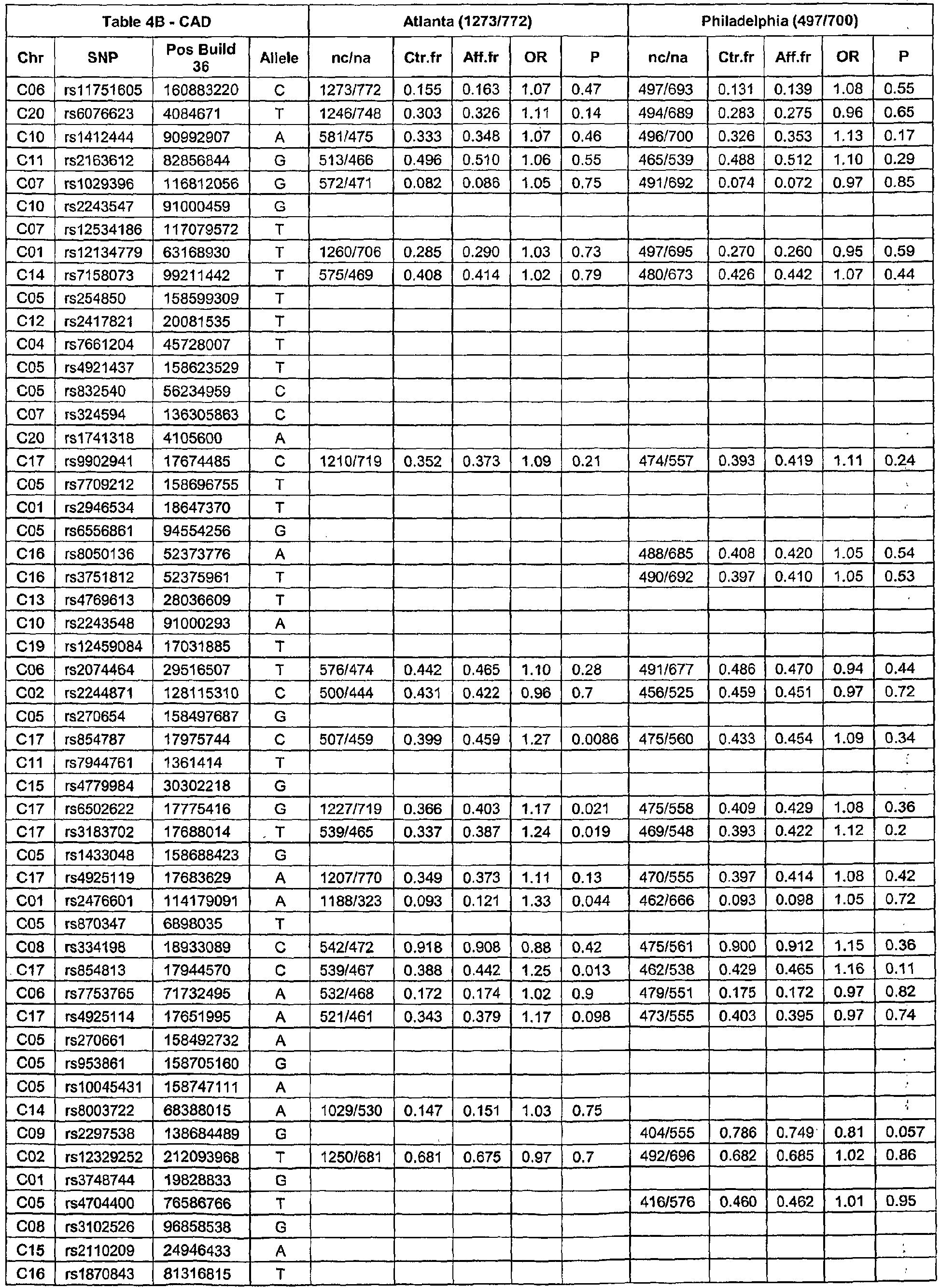

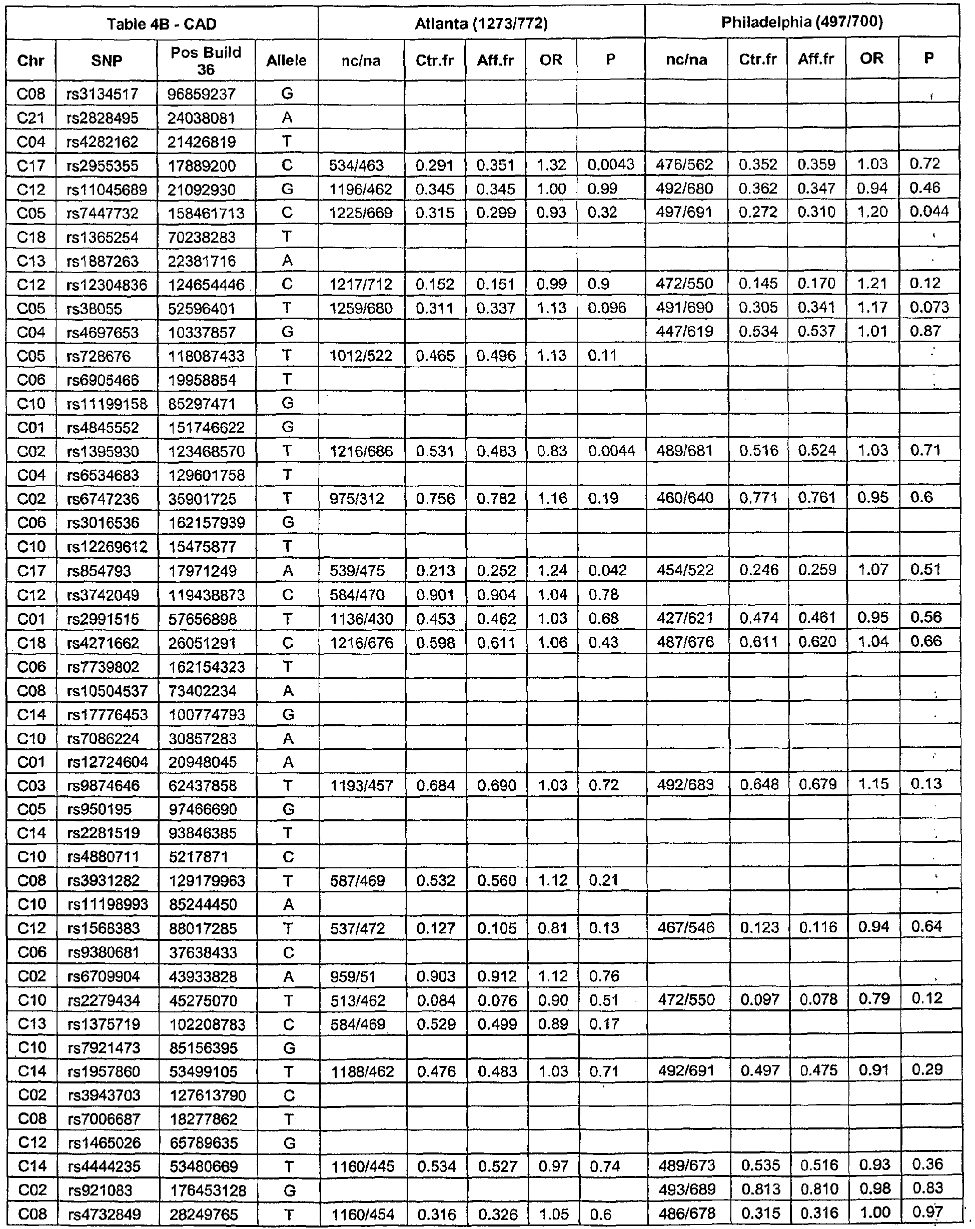

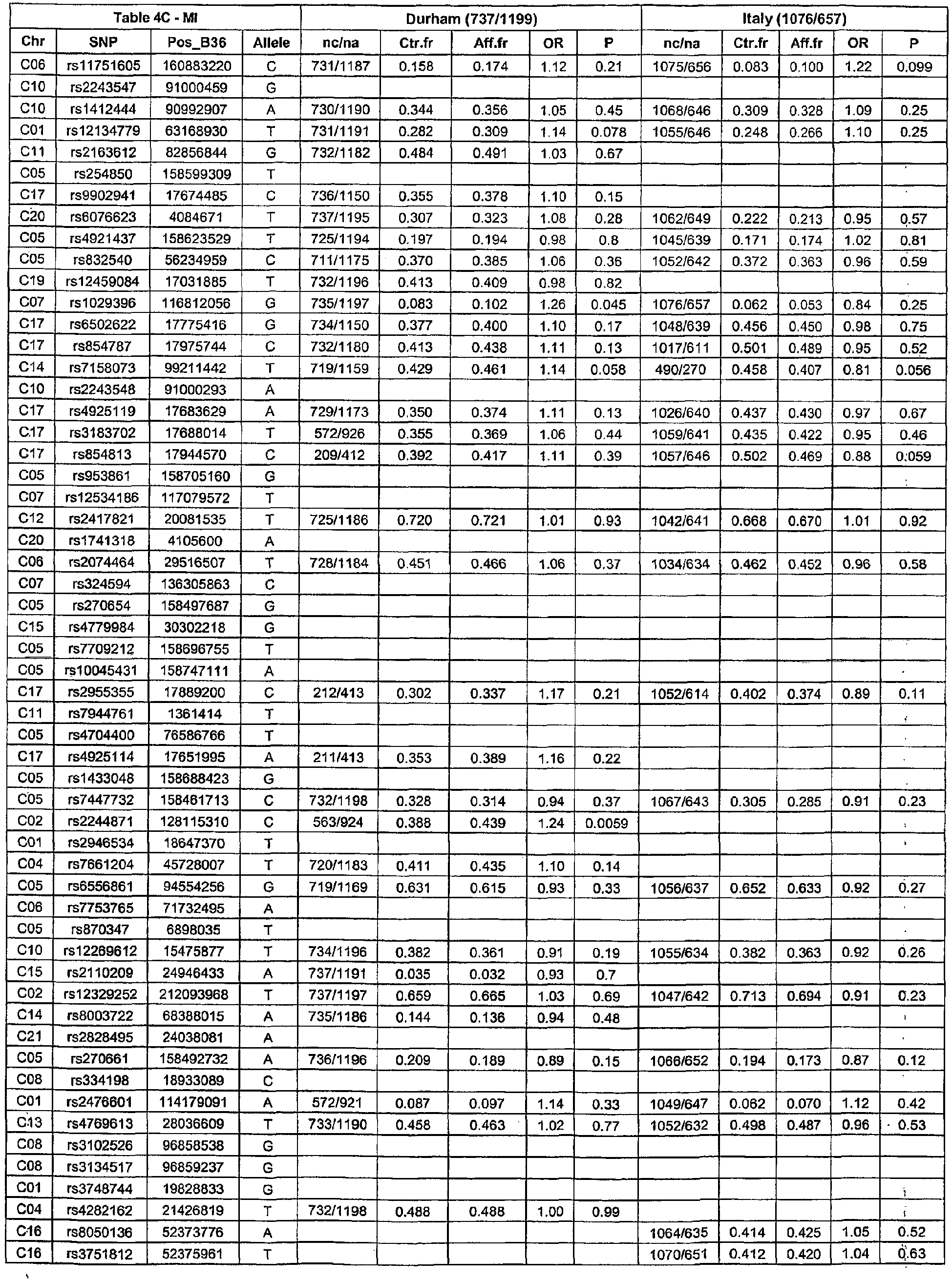

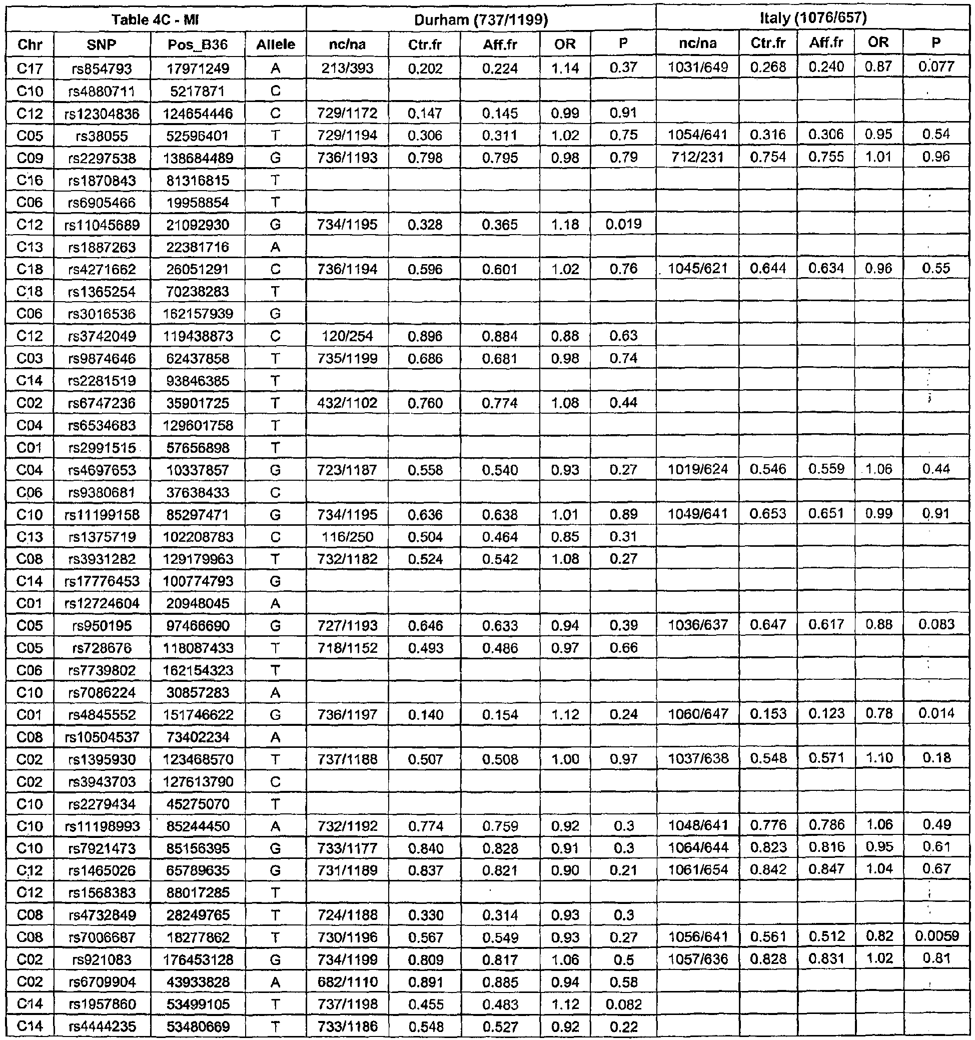

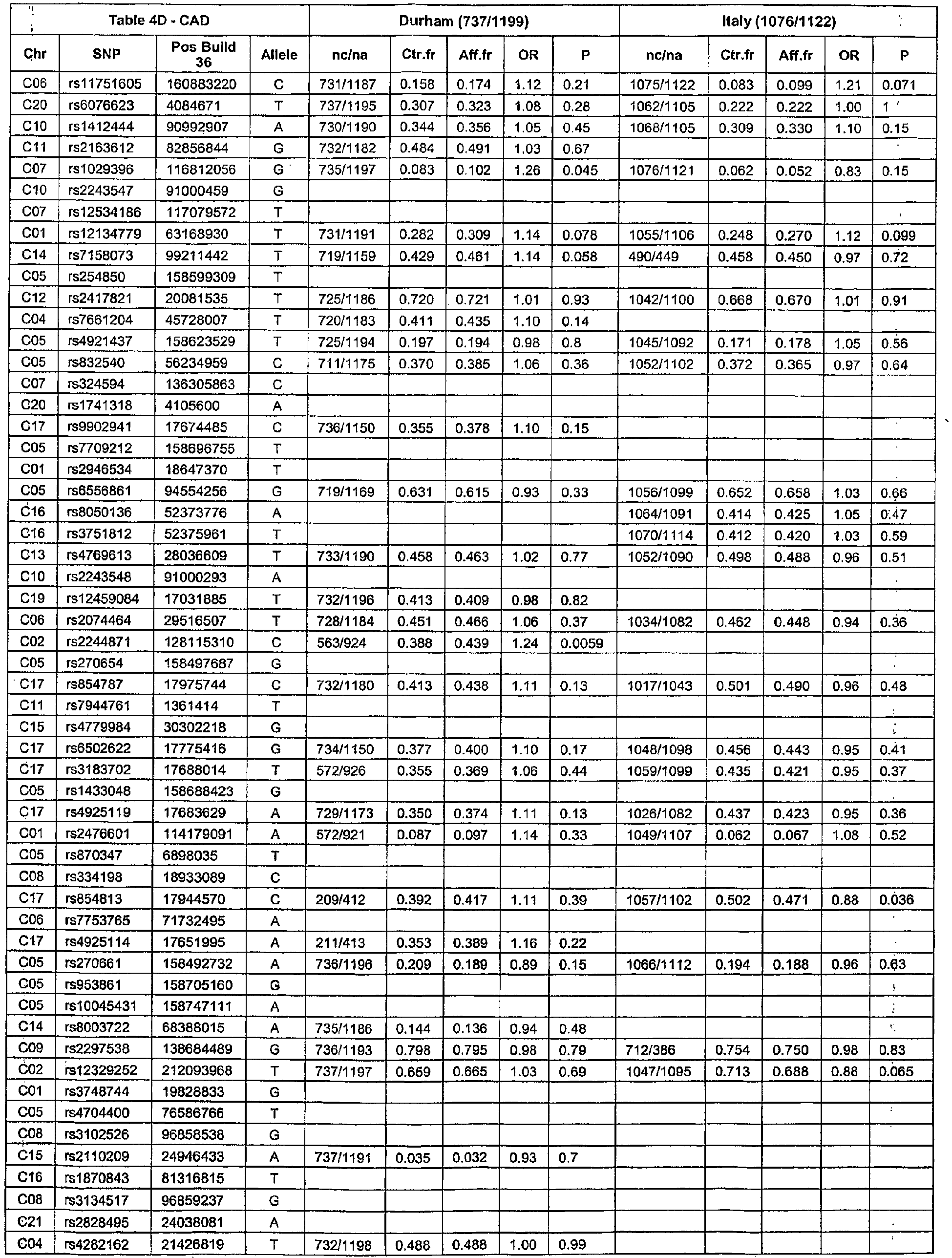

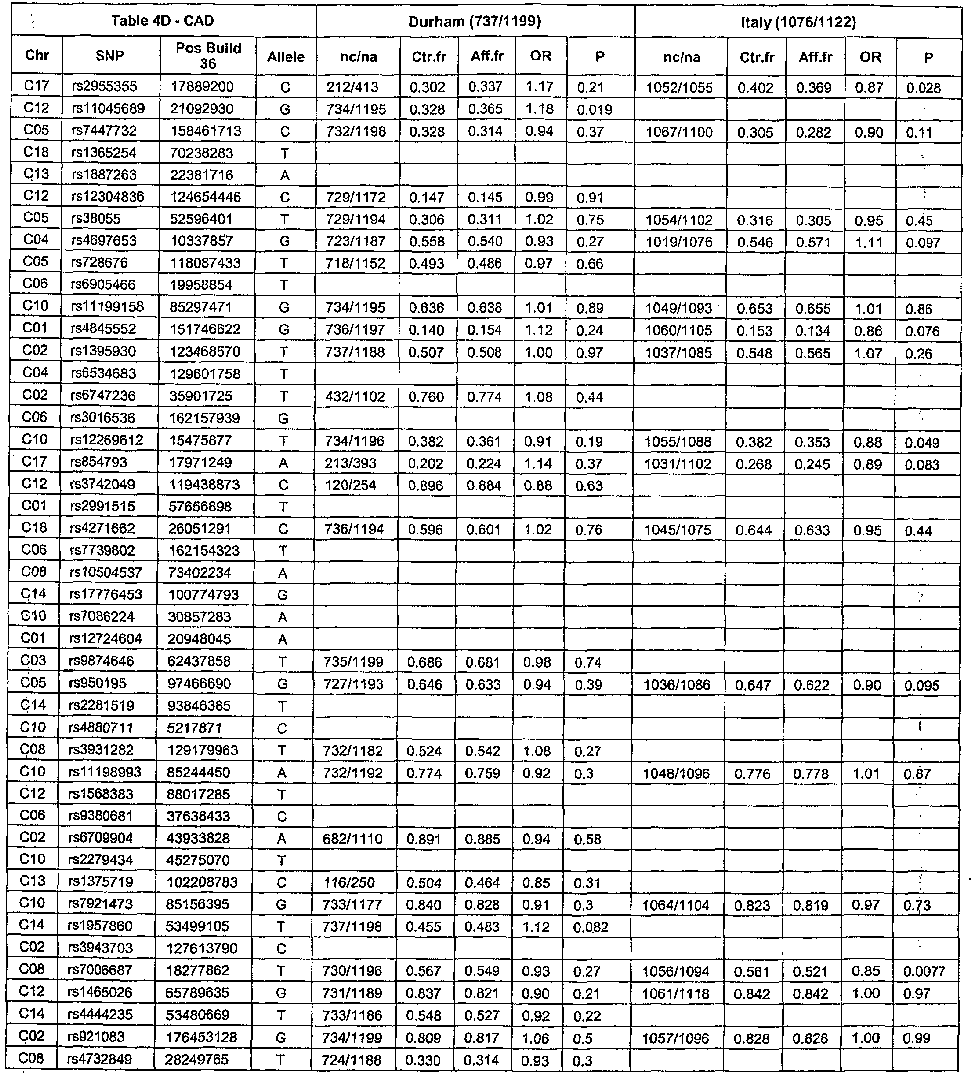

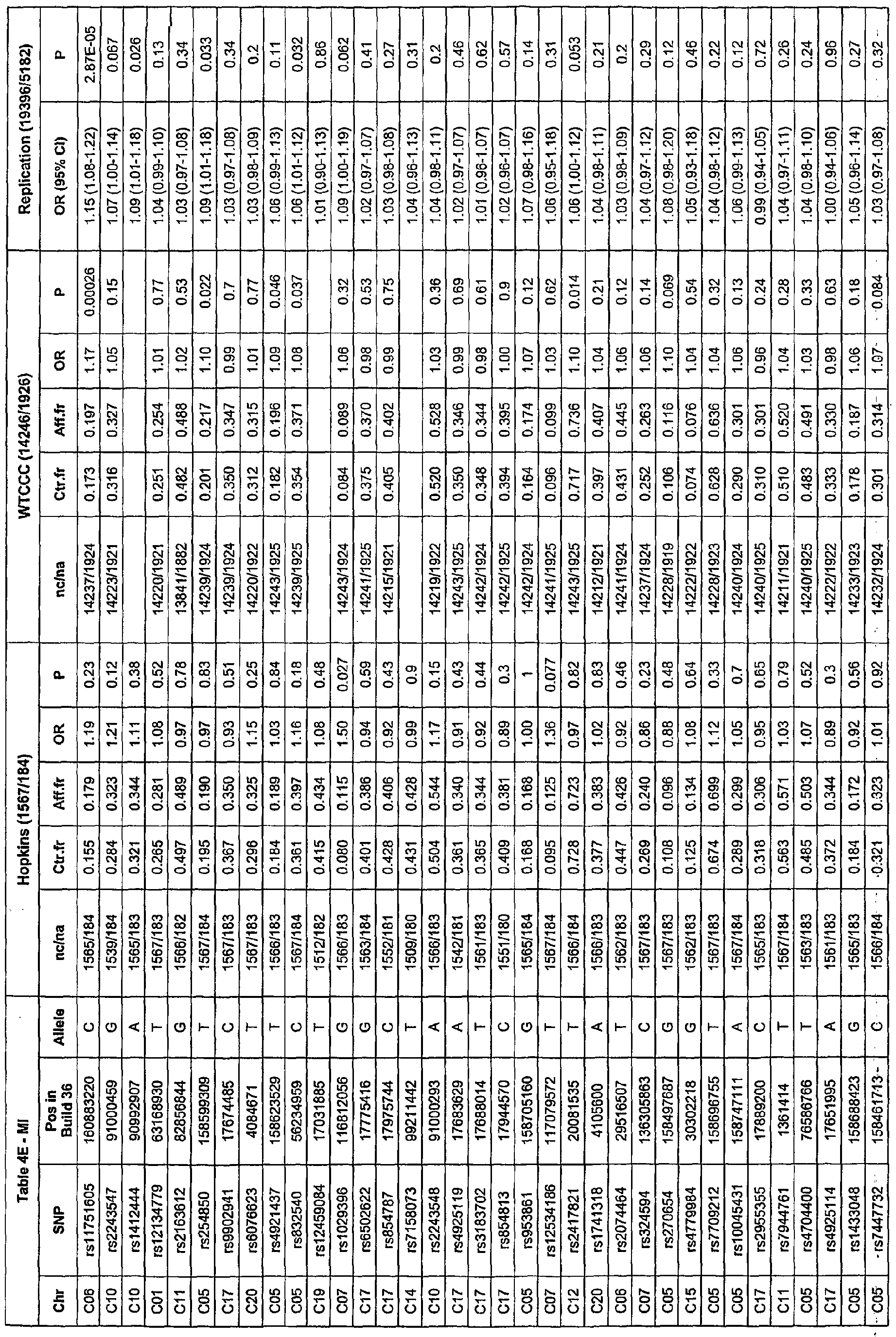

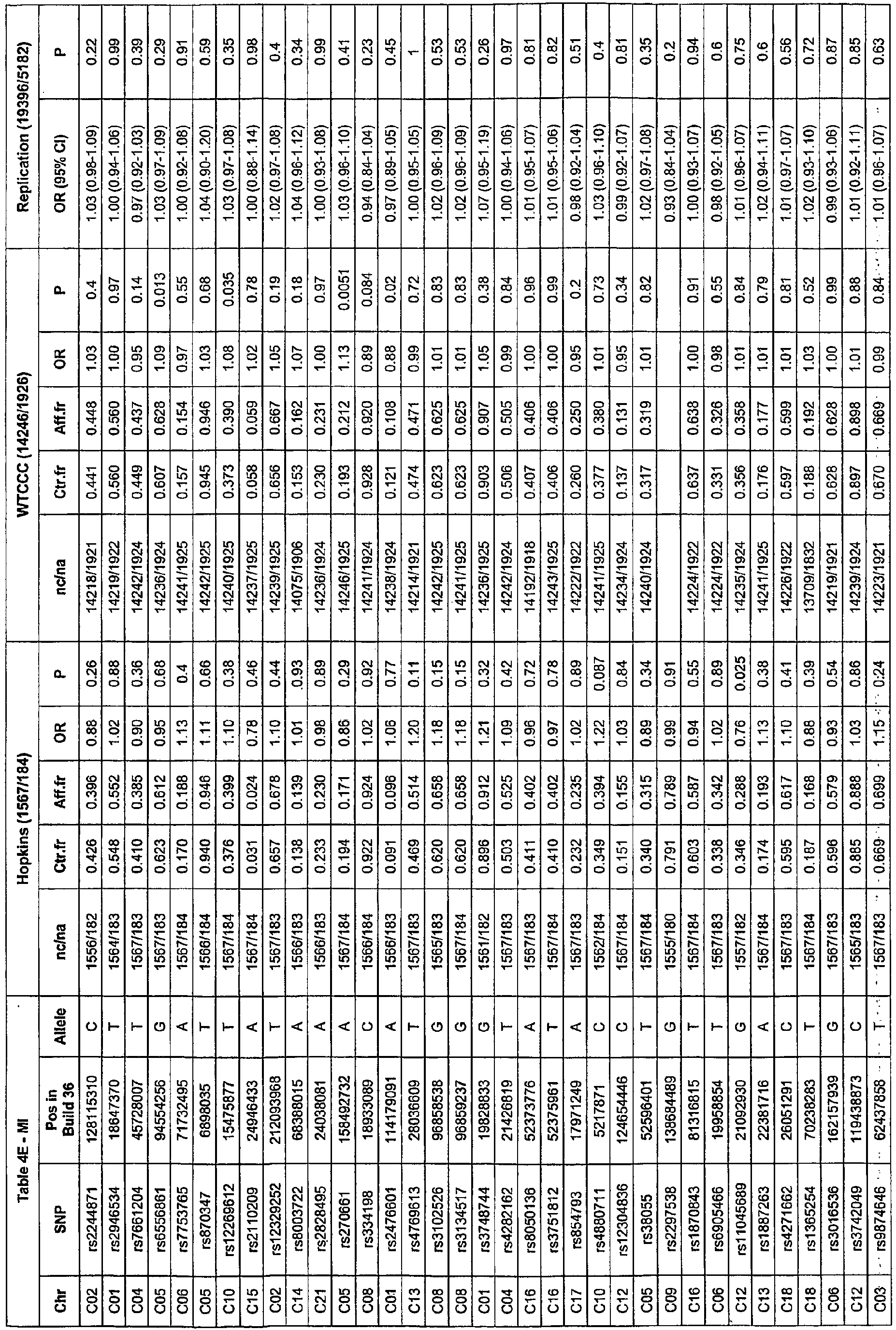

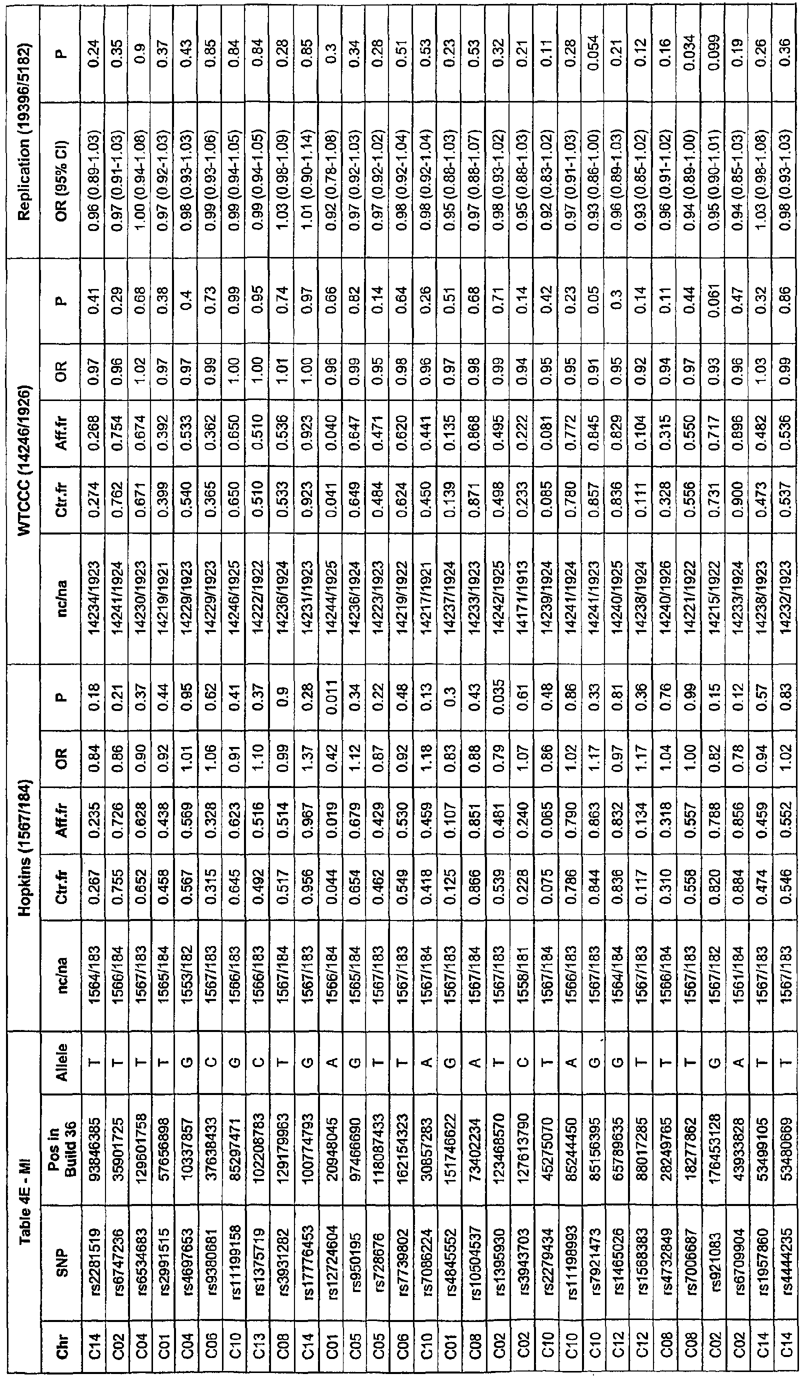

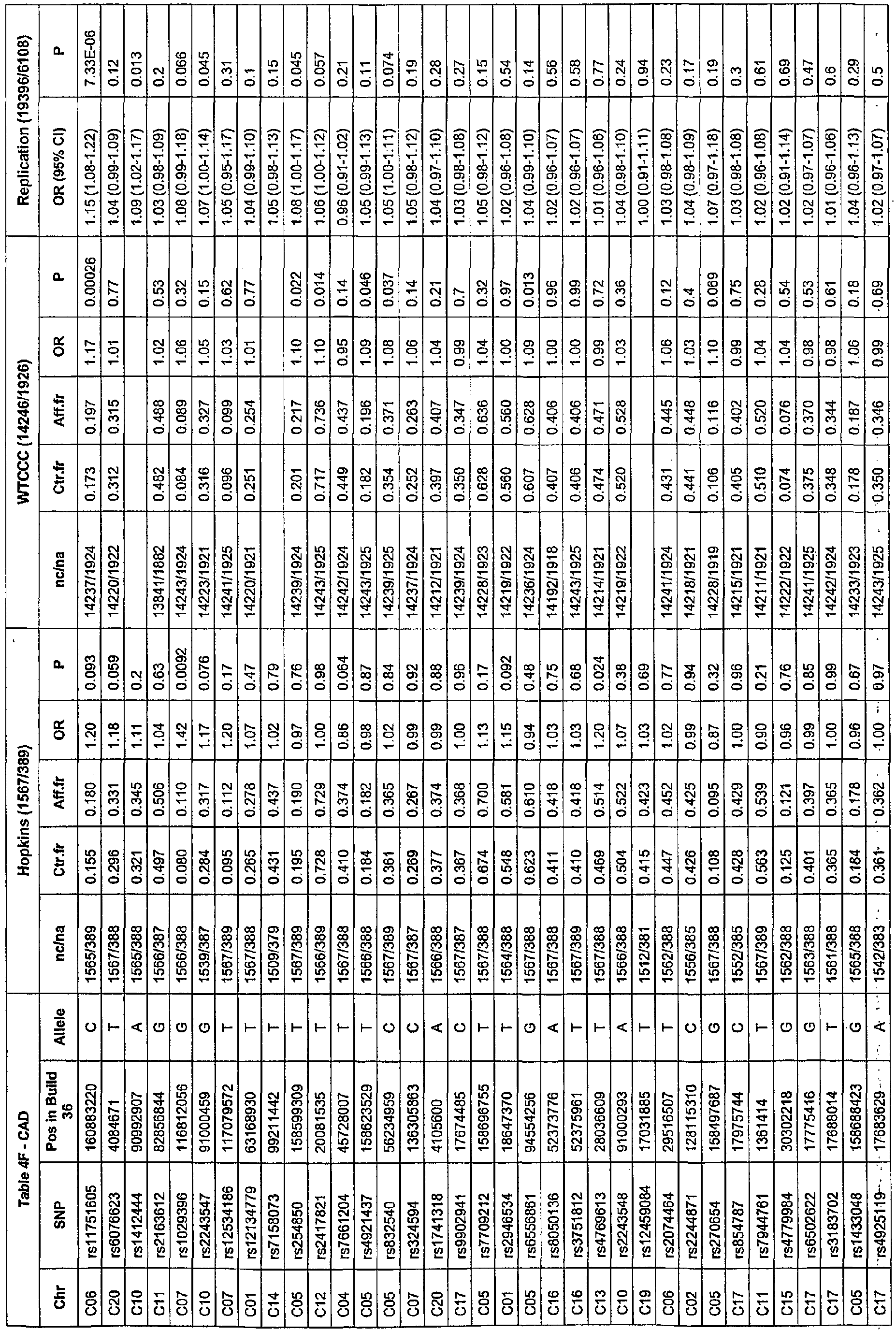

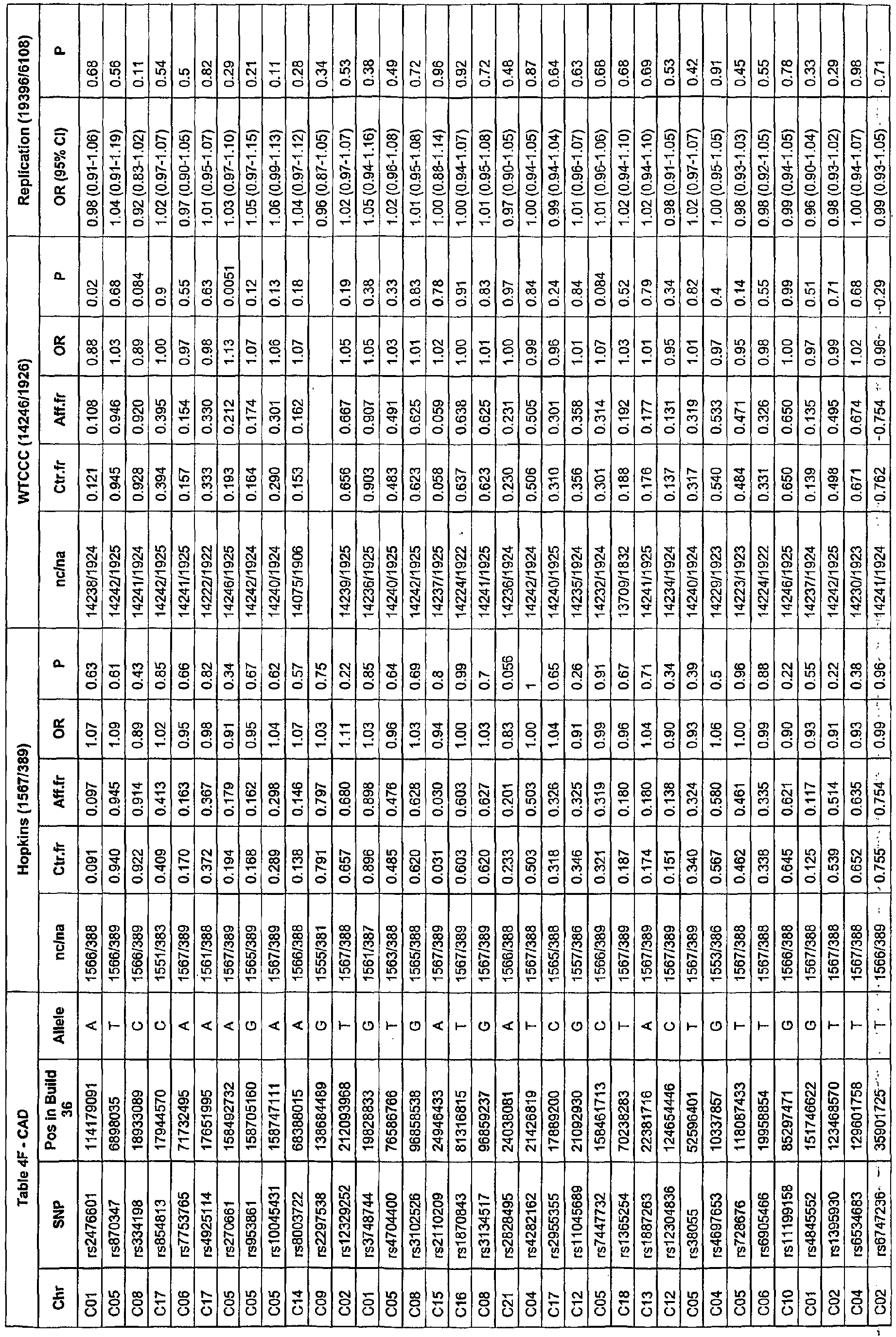

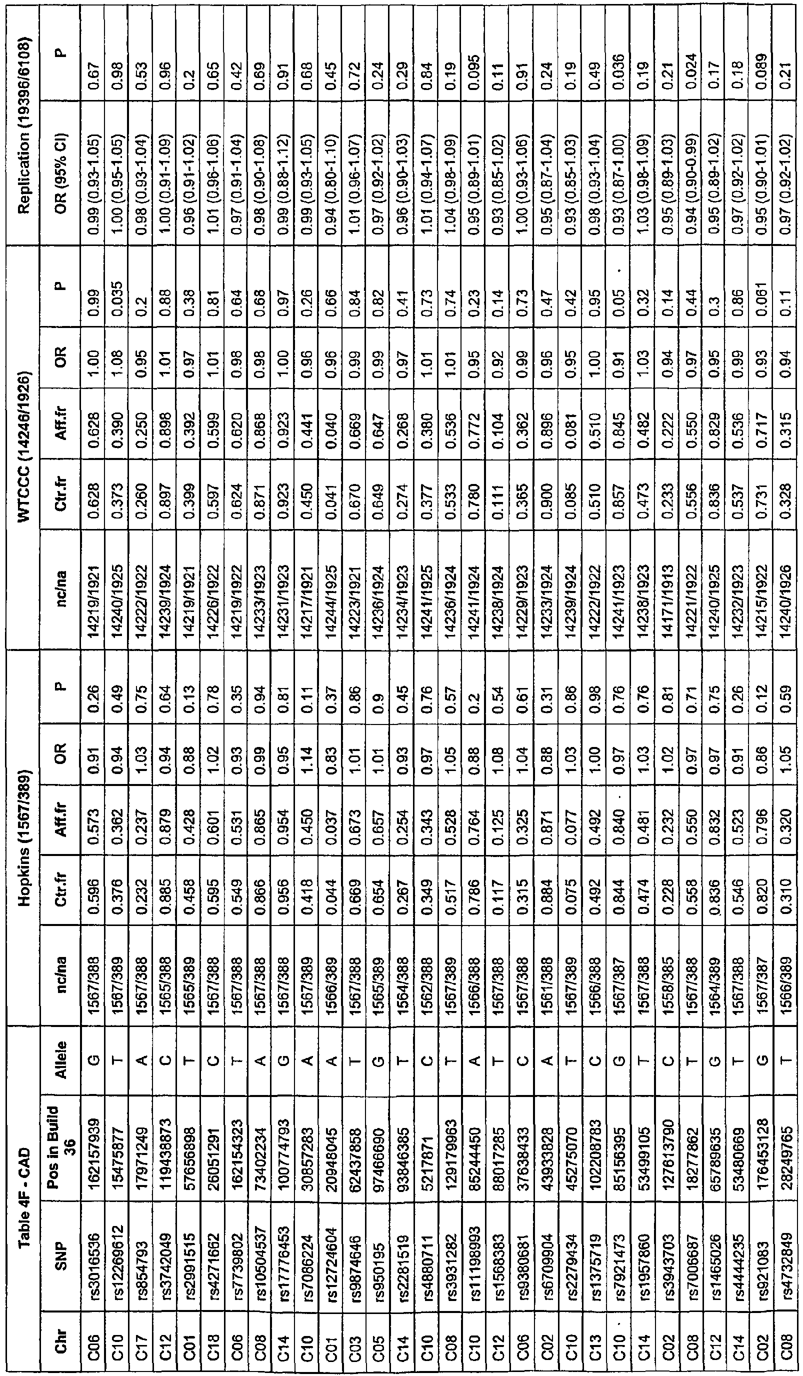

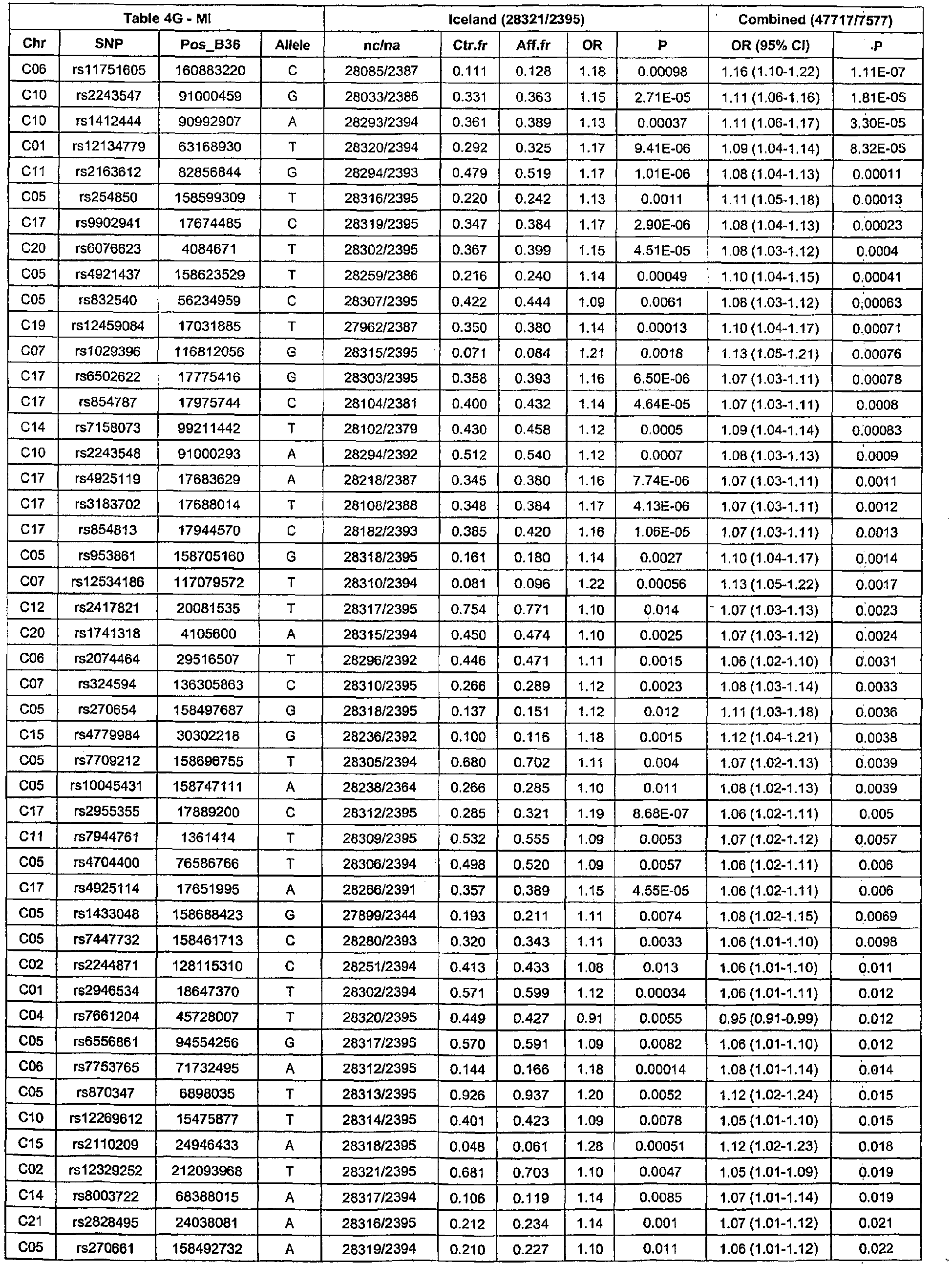

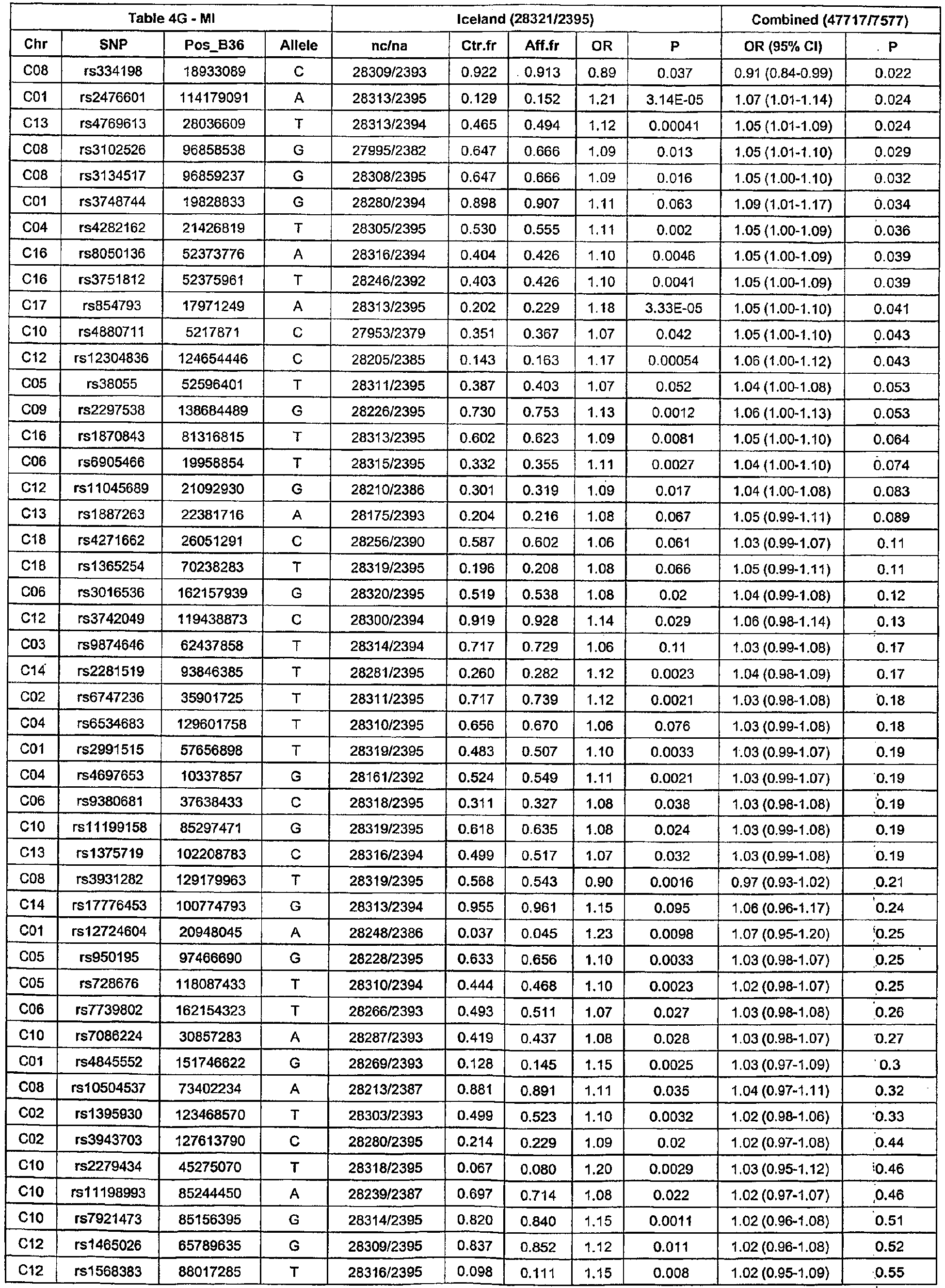

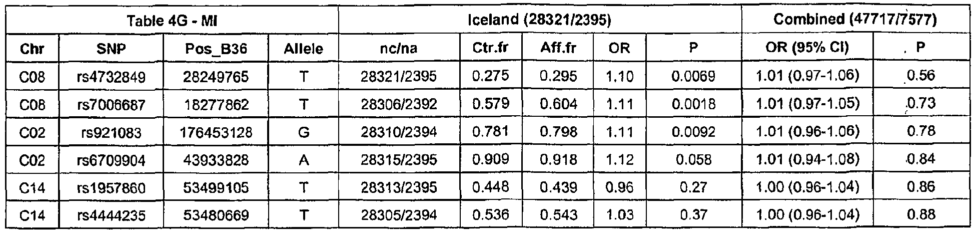

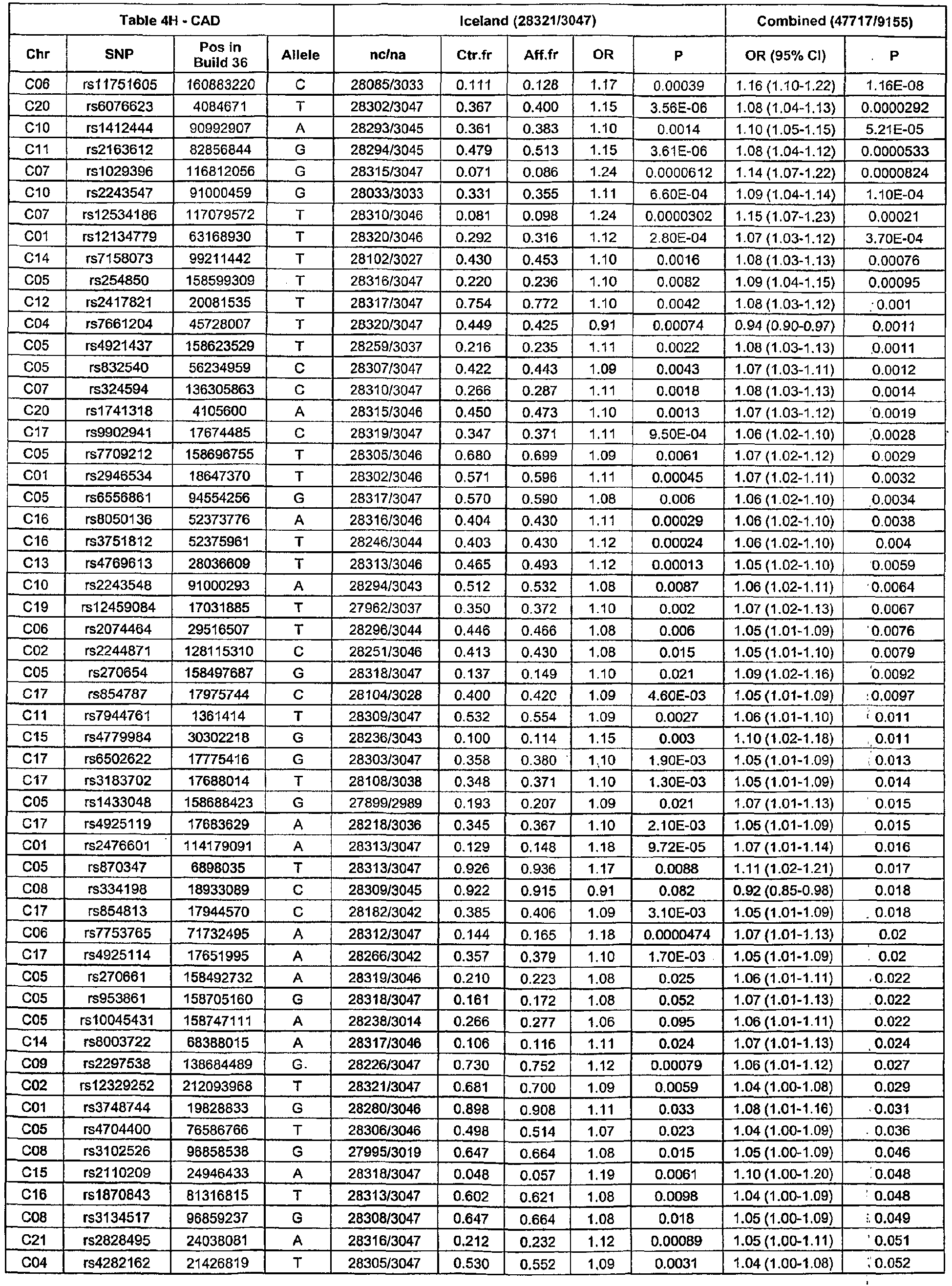

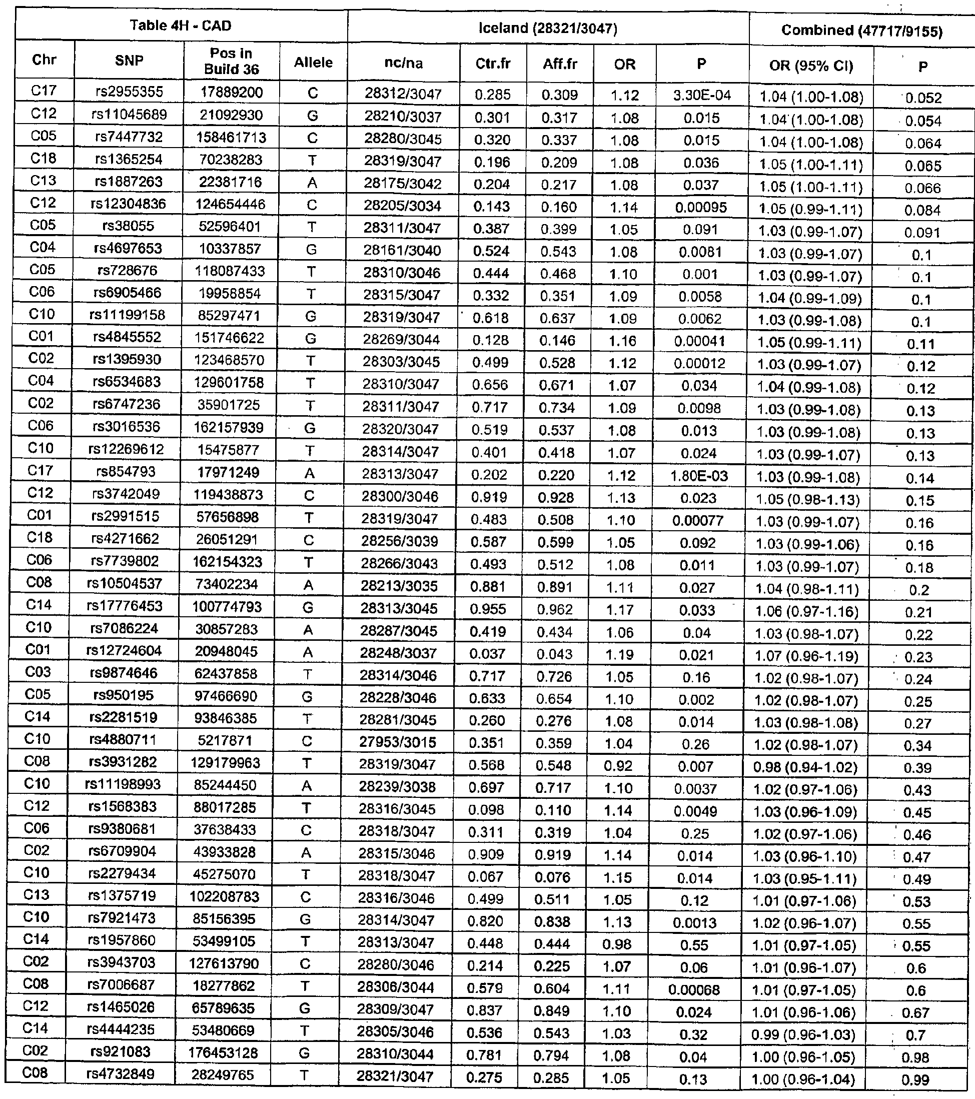

**Table 5. Surrogate markers for marker rs11751605. The markers were selected from the Caucasian data set of the HapMap sample (CEU; see http://www.hapmap.org), using a cutoff of r² of 0.2. The search was done in a 2Mb interval flanking the rs11751605 marker. The table shows (1) chromosome; (2) SNP rs name; (3) position in NCBI build 36; (4) value for D' to rs11751605; (5) value for r² to rs11751605; (6) P-value of the association with rs11751605.**

| **Surrogates of rs11751605 based on HapMap CEU v22** | | | | | |
|---|---|---|---|---|---|
| **Chr** | **SNP** | **Pos in Build 36** | **D'** | **R2** | **P** |
| C06 | rs3798156 | 160596206 | 0.55 | 0.22 | 5.8E-05 |
| C06 | rs3127573 | 160601383 | 0.80 | 0.43 | 4.9E-08 |
| C06 | rs3127572 | 160602208 | 0.71 | 0.36 | 4.7E-07 |
| C06 | rs3119309 | 160605062 | 0.80 | 0.44 | 2.4E-08 |
| C06 | rs7756836 | 160609247 | 0.80 | 0.44 | 2.4E-08 |
| C06 | rs7757336 | 160609548 | 0.56 | 0.29 | 3.6E-06 |
| C06 | rs3119310 | 160612365 | 0.80 | 0.44 | 2.4E-08 |
| C06 | rs3119311 | 160613097 | 0.80 | 0.44 | 2.4E-08 |
| C06 | rs3127578 | 160618145 | 0.80 | 0.44 | 2.4E-08 |
| C06 | rs3127586 | 160624902 | 0.85 | 0.67 | 9.9E-13 |
| C06 | rs12206585 | 160633514 | 0.92 | 0.72 | 1.1E-13 |
| C06 | rs12203303 | 160633686 | 0.83 | 0.55 | 4.6E-09 |
| C06 | rs2183470 | 160635083 | 0.85 | 0.68 | 1.5E-13 |
| C06 | rs3120151 | 160637462 | 0.85 | 0.68 | 1.5E-13 |
| C06 | rs3103350 | 160642995 | 0.85 | 0.68 | 1.5E-13 |
| C06 | rs3127583 | 160647121 | 0.85 | 0.68 | 1.5E-13 |
| C06 | rs3106172 | 160647965 | 0.85 | 0.64 | 1.2E-10 |
| C06 | rs3106170 | 160649914 | 0.86 | 0.72 | 6.6E-13 |
| C06 | rs3125057 | 160651662 | 0.84 | 0.60 | 2.7E-11 |
| C06 | rs3125056 | 160655271 | 0.87 | 0.45 | 4.8E-06 |
| C06 | rs3125055 | 160656777 | 0.86 | 0.37 | 2.2E-06 |
| C06 | rs3106167 | 160657380 | 0.85 | 0.68 | 1.5E-13 |
| C06 | rs3127591 | 160657792 | 0.92 | 0.71 | 3.2E-12 |
| C06 | rs3125052 | 160658363 | 0.85 | 0.68 | 1.5E-13 |
| C06 | rs3120140 | 160658821 | 0.90 | 0.58 | 1.1E-08 |
| C06 | rs3103349 | 160660711 | 0.85 | 0.68 | 1.5E-13 |
| C06 | rs3125050 | 160660826 | 0.85 | 0.68 | 1.5E-13 |
| C06 | rs3120139 | 160661612 | 0.85 | 0.68 | 1.5E-13 |
| C06 | rs3125049 | 160674702 | 0.86 | 0.74 | 4.0E-15 |
| C06 | rs12209517 | 160676726 | 0.72 | 0.46 | 2.4E-07 |
| C06 | rs3120137 | 160691182 | 0.93 | 0.87 | 1.2E-17 |
| C06 | rs3127574 | 160711360 | 1.00 | 0.20 | 3.1E-08 |
| C06 | rs6905958 | 160717615 | 1.00 | 0.20 | 3.1E-08 |
| C06 | rs3123633 | 160719595 | 1.00 | 0.20 | 3.1E-08 |
| C06 | rs3123634 | 160722229 | 1.00 | 0.20 | 3.1E-08 |
| C06 | rs1317652 | 160727151 | 1.00 | 0.20 | 2.9E-08 |
| C06 | rs884742 | 160727422 | 1.00 | 0.20 | 3.1E-08 |

| **Surrogates of ras11751605 based on HapMap CEU v22** | | | | | |
|---|---|---|---|---|---|
| **Chr** | **SNP** | **Pos in Build 36** | **D'** | **R2** | **P** |
| C06 | rs7740824 | 160728891 | 0.91 | 0.45 | 1.1E-09 |
| C06 | rs3105748 | 160730710 | 1.00 | 0.20 | 3.1E-08 |
| C06 | rs3105749 | 160740968 | 1.00 | 0.20 | 3.1E-08 |
| C06 | rs3105752 | 160742915 | 1.00 | 0.20 | 2.9E-08 |
| C06 | rs10945670 | 160747660 | 1.00 | 0.20 | 3.1E-08 |
| C06 | rs10806731 | 160747825 | 1.00 | 0.20 | 3.1E-08 |
| C06 | rs9364554 | 160753654 | 0.92 | 0.46 | 5.2E-11 |
| C06 | rs12194182 | 160754505 | 0.92 | 0.46 | 5.2E-11 |
| C06 | rs3106166 | 160758351 | 1.00 | 0.22 | 2.2E-08 |
| C06 | rs10455782 | 160759340 | 1.00 | 0.39 | 4.6E-12 |
| C06 | rs7758229 | 160760242 | 1.00 | 0.38 | 2.2E-11 |
| C06 | rs3123636 | 160762527 | 1.00 | 0.37 | 1.9E-11 |
| C06 | rs3106164 | 160770263 | 1.00 | 0.37 | 6.9E-10 |
| C06 | rs388170 | 160773276 | 1.00 | 0.36 | 1.0E-11 |
| C06 | rs9365164 | 160776833 | 0.91 | 0.33 | 3.9E-08 |
| C06 | rs2292334 | 160778178 | 1.00 | 0.33 | 2.8E-10 |
| C06 | rs2048327 | 160783522 | 1.00 | 0.33 | 4.5E-11 |
| C06 | rs7769879 | 160785635 | 1.00 | 0.33 | 4.5E-11 |
| C06 | rs3918285 | 160788644 | 1.00 | 0.33 | 4.5E-11 |
| C06 | rs3918286 | 160788658 | 1.00 | 0.33 | 1.1E-10 |
| C06 | rs1810126 | 160792141 | 1.00 | 0.34 | 3.0E-11 |
| C06 | rs3088442 | 160792642 | 1.00 | 0.33 | 4.5E-11 |
| C06 | rs2063347 | 160802019 | 1.00 | 0.33 | 4.5E-11 |
| C06 | rs3106162 | 160803284 | 1.00 | 0.33 | 4.5E-11 |
| C06 | rs2063346 | 160804265 | 1.00 | 0.34 | 3.5E-11 |
| C06 | rs1112073 | 160810832 | 1.00 | 0.38 | 8.0E-12 |
| C06 | rs3127602 | 160812231 | 1.00 | 0.37 | 8.9E-12 |
| C06 | rs3123629 | 160826076 | 1.00 | 0.37 | 7.6E-12 |
| C06 | rs3127599 | 160827124 | 1.00 | 0.37 | 7.6E-12 |
| C06 | rs3127569 | 160837549 | 1.00 | 0.39 | 4.0E-12 |
| C06 | rs3127596 | 160873025 | 1.00 | 0.38 | 4.7E-12 |
| C06 | rs3124785 | 160874495 | 1.00 | 0.38 | 7.6E-12 |
| **C06** | **rs11751605** | **160883220** | **1.00** | **1.00** | **0.0E+00** |
| C06 | rs10455872 | 160930108 | 1.00 | 0.55 | 1.3E-11 |
| C06 | rs2315065 | 161028134 | 0.66 | 0.35 | 4.6E-07 |
| C06 | rs9458266 | 161798149 | 0.58 | 0.22 | 1.7E-04 |

**Table 6. Key to the sequence listing provided herein.**

| **Seq ID No:** | **SNP** |
|---|---|
| 1 | rs11751605 |
| 2 | rs6076623 |
| 3 | rs1412444 |
| 4 | rs2163612 |
| 5 | rs1029396 |
| 6 | rs2243547 |
| 7 | rs12534186 |
| 8 | rs12134779 |
| 9 | rs7158073 |
| 10 | rs254850 |
| 11 | rs2417821 |
| 12 | rs7661204 |
| 13 | rs4921437 |
| 14 | rs832540 |
| 15 | rs324594 |
| 16 | rs1741318 |
| 17 | rs9902941 |
| 18 | rs7709212 |
| 19 | rs2946534 |
| 20 | rs6556861 |
| 21 | rs8050136 |
| 22 | rs3751812 |
| 23 | rs4769613 |
| 24 | rs2243548 |
| 25 | rs12459084 |
| 26 | rs2074464 |
| 27 | rs2244871 |
| 28 | rs270654 |
| 29 | rs854787 |
| 30 | rs7944761 |
| 31 | rs4779984 |
| 32 | rs6502622 |
| 33 | rs3183702 |
| 34 | rs1433048 |
| 35 | rs4925119 |
| 36 | rs2476601 |
| 37 | rs870347 |
| 38 | rs334198 |
| 39 | rs854813 |
| 40 | rs7753765 |
| 41 | rs4925114 |
| 42 | rs270661 |
| 43 | rs953861 |
| 44 | rs10045431 |
| 45 | rs8003722 |
| 46 | rs2297538 |
| 47 | rs12329252 |
| 48 | rs3748744 |
| 49 | rs4704400 |
| 50 | rs3102526 |
| 51 | rs2110209 |
| 52 | rs1870843 |
| 53 | rs3134517 |
| 54 | rs2828495 |
| 55 | rs4282162 |
| 56 | rs2955355 |
| 57 | rs11045689 |
| 58 | rs7447732 |
| 59 | rs1365254 |
| 60 | r1887263 |
| 61 | rs12304836 |
| 62 | rs38055 |
| 63 | rs4697653 |
| 64 | rs728676 |
| 65 | rs6905466 |
| 66 | rs11199158 |
| 67 | rs4845552 |
| 68 | rs1395930 |
| 69 | rs6534683 |
| 70 | rs6747236 |
| 71 | rs3016536 |
| 72 | rs12269612 |
| 73 | rs854793 |
| 74 | rs3742049 |
| 75 | rs2991515 |
| 76 | rs4271662 |
| 77 | rs7739802 |
| 78 | rs10504537 |
| 79 | rs17776453 |
| 80 | rs7086224 |
| 81 | rs12724604 |
| 82 | rs9874646 |
| 83 | rs950195 |
| 84 | rs2281519 |
| 85 | rs488071 |
| 86 | rs3931282 |
| 87 | rs11198993 |
| 88 | rs1568383 |
| 89 | rs9380681 |
| 90 | rs6709904 |
| 91 | rs2279434 |
| 92 | rs1375719 |
| 93 | rs7921473 |
| 94 | rs1957860 |
| 95 | rs3943703 |
| 96 | rs7006687 |
| 97 | rs1465026 |
| 98 | rs4444235 |
| 99 | rs921083 |
| 100 | rs4732849 |
| 101 | rs3798156 |
| 102 | rs3127573 |
| 103 | rs3127572 |
| 104 | rs3119309 |
| 105 | rs7756836 |
| 106 | rs7757336 |
| 107 | rs3119310 |
| 108 | rs3119311 |
| 109 | rs3127578 |
| 110 | rs3127586 |
| 111 | rs12206585 |
| 112 | rs12203303 |
| 113 | rs2183470 |
| 114 | rs3120151 |
| 115 | rs3103350 |
| 116 | rs3127583 |
| 117 | rs3106172 |
| 118 | rs3106170 |
| 119 | rs3125057 |
| 120 | rs3125056 |
| 121 | rs3125055 |
| 122 | rs3106167 |
| 123 | rs3127591 |
| 124 | rs3125052 |
| 125 | rs3120140 |
| 126 | rs3103349 |
| 127 | rs3125050 |
| 128 | rs3120139 |
| 129 | rs3125049 |
| 130 | rs12209517 |
| 131 | rs3120137 |
| 132 | rs3127574 |
| 133 | rs6905958 |
| 134 | rs3123633 |
| 135 | rs3123634 |
| 136 | rs1317652 |
| 137 | rs884742 |
| 138 | rs7740824 |
| 139 | rs3105748 |
| 140 | rs3105749 |
| 141 | rs3105752 |
| 142 | rs10945670 |
| 143 | rs10806731 |
| 144 | rs9364554 |
| 145 | rs12194182 |
| 146 | rs3106166 |
| 147 | rs10455782 |
| 148 | rs7758229 |
| 149 | rs3123636 |
| 150 | rs3106164 |
| 151 | rs388170 |
| 152 | rs9365164 |
| 153 | rs2292334 |
| 154 | rs2048327 |
| 155 | rs7769879 |
| 156 | rs3918285 |
| 157 | rs3918286 |
| 158 | rs1810126 |
| 159 | rs3088442 |
| 160 | rs2063347 |
| 161 | rs3106162 |
| 162 | rs2063346 |
| 163 | rs1112073 |
| 164 | rs3127602 |
| 165 | rs3123629 |
| 166 | rs3127599 |
| 167 | rs3127569 |
| 168 | rs3127596 |
| 169 | rs3124785 |
| 170 | rs10455872 |
| 171 | rs2315065 |
| 172 | rs9458266 |

## Claims

1. A method for determining a susceptibility to myocardial infarction or coronary artery disease in a human individual from a Caucasian population, comprising determining the presence or absence of at least one allele of at least one polymorphic marker in a nucleic acid sample obtained from the individual wherein the at least one polymorphic marker is selected from the marker rs11751605, and markers in linkage disequilibrium therewith selected from the group consisting of the markers set forth in table 5, and wherein the presence of the at least one allele is indicative of a susceptibility to myocardial infarction or coronary artery disease in individuals from a Caucasian population.

2. The method according to Claim 1, wherein the susceptibility conferred by the presence of the at least one allele is increased susceptibility.

3. The method according to Claim 1, wherein the presence of allele C in rs11751605 is indicative of increased susceptibility to myocardial infarction or coronary artery disease.

4. The method according to any of the Claims 1-3, wherein the presence of the marker or haplotype is indicative of a different response rate of the subject to a particular treatment modality for myocardial infarction or coronary artery disease.

5. The method of any of the preceding Claims, further comprising assessing at least one biomarker in a sample from the individual, wherein the biomarker is a cardiac marker or an inflammatory marker selected from creatin kinase, troponin, glycogen phosphorylase, C-reactive protein (CRP), serum amyloid A, fibrinogen, interleukin-6, tissue necrosis factor-alpha, soluble vascular cell adhesion molecules (sVCAM), soluble intervascular adhesion molecules (sICAM), E-selectin, matrix metalloprotease type-1, matrix metalloprotease type-2, matrix metalloprotease type-3, matrix metalloprotease type-9, serum sCD40L, leukotrienes, leukotriene metabolites, interleukin-6, tissue necrosis factor-alpha, myeloperoxidase (MPO), and N-tyrosine.

6. The method according to Claim 5, wherein the leukotriene is selected from LTB4, LTC4, LTD4 and LTE4.

7. The method of any of the preceding Claims, further comprising analyzing non-genetic information to make risk assessment, diagnosis, or prognosis of the individual, wherein the non-genetic information is selected from age, gender, socioeconomic status, previous disease diagnosis, medical history of subject, family history of coronary artery disease or myocardial infarction, biochemical measurements, and clinical measurements.

8. An apparatus for determining a genetic indicator for coronary artery disease or myocardial infarction in a human individual from a Caucasian population, comprising:
a computer readable memory; and
a routine stored on the computer readable memory;
wherein the routine is adapted to be executed on a processor to analyze marker and/or
haplotype information for at least one human individual with respect to at least one polymorphic marker selected from the marker rs11751605, and markers in linkage disequilibrium therewith selected from the group consisting of the markers set forth in table 5, and generate an output based on the marker or haplotype information, wherein
the output comprises a risk measure of the at least one marker or haplotype as a genetic indicator of coronary artery disease or myocardial infarction for the human individual.

## Patentansprüche

1. Verfahren zur Bestimmung einer Anfälligkeit für Myokardinfarkt oder koronarer Herzkrankheit bei einem menschlichen Individuum aus einer kaukasischen Population, wobei man das Vorliegen oder Fehlen wenigstens eines Allels wenigstens eines polymorphen Markers in einer von dem Individuum erhaltenen Nukleinsäureprobe bestimmt, wobei der wenigstens eine polymorphe Marker aus dem Marker rs11751605 und im Kopplungsungleichgewicht damit stehenden Markern, die aus der aus den in Tabelle 5 aufgeführten Markern bestehenden Gruppe ausgewählt sind, ausgewählt ist und wobei das Vorliegen des wenigstens einen Allels auf eine Anfälligkeit für Myokardinfarkt oder koronare Herzkrankheit bei Individuen aus einer kaukasischen Population hindeutet.

2. Verfahren nach Anspruch 1, wobei es sich bei der durch das Vorliegen des wenigstens einen Allels verliehenen Anfälligkeit um erhöhte Anfälligkeit handelt.

3. Verfahren nach Anspruch 1, wobei das Vorliegen von Allel C in rs11751605 auf erhöhte Anfälligkeit für Myokardinfarkt oder koronare Herzkrankheit hindeutet.

4. Verfahren nach einem der Ansprüche 1-3, wobei das Vorliegen des Markers oder Haplotyps auf eine unterschiedliche Ansprechrate des Patienten auf eine bestimmte Behandlungsmodalität für Myokardinfarkt oder koronare Herzkrankheit hindeutet.

5. Verfahren nach einem der vorhergehenden Ansprüche, wobei man ferner wenigstens einen Biomarker in einer Probe von dem Individuum beurteilt, wobei es sich bei dem Biomarker um einen Herzmarker oder einen Entzündungsmarker handelt, der aus Creatinkinase, Troponin, Glykogen-Phosphorylase, C-reaktivem Protein (CRP), Serumamyloid A, Fibrinogen, Interleukin-6, Gewebenekrosefaktor-alpha, löslichen vaskulären Zelladhäsionsmolekülen (sVCAM), löslichen intervaskulären Adhäsionsmolekülen (sICAM), E-Selectin, Matrix-Metalloprotease Typ-1, Matrix-Metalloprotease Typ-2, Matrix-Metalloprotease Typ-3, Matrix-Metalloprotease Typ-9, Serum-sCD40L, Leukotrienen, Leukotrien-Metaboliten, Interleukin-6, Gewebenekrosefaktor-alpha, Myeloperoxidase (MPO) und N-Tyrosin ausgewählt ist.

6. Verfahren nach Anspruch 5, wobei das Leukotrien aus LTB4, LTC4, LTD4 und LTE4 ausgewählt ist.

7. Verfahren nach einem der vorhergehenden Ansprüche, wobei man ferner zur Erstellung einer Risikobeurteilung, Diagnose oder Prognose für das Individuum nichtgenetische Informationen analysiert, wobei die nichtgenetischen Informationen aus Alter, Geschlecht, sozioökonomischem Status, einer früheren Krankheitsdiagnose, Anamnese des Patienten, Familienanamnese von koronarer Herzkrankheit oder Myokardinfarkt, biochemischen Messdaten und klinischen Messdaten ausgewählt sind.

8. Vorrichtung zur Bestimmung eines genetischen Indikators für koronare Herzkrankheit oder Myokardinfarkt bei einem menschlichen Individuum aus einer kaukasischen Population, umfassend:
einen computerlesbaren Speicher; und
eine auf dem computerlesbaren Speicher gespeicherte Routine;
wobei die Routine zur Ausführung auf einem Prozessor adaptiert ist, um Marker- und/oder Haplotyp-Informationen für wenigstens ein menschliches Individuum in Bezug auf wenigstens einen polymorphen Marker, der aus dem Marker rs11751605 und im Kopplungsungleichgewicht damit stehenden Markern, die aus der aus den in Tabelle 5 aufgeführten Markern bestehenden Gruppe ausgewählt sind, ausgewählt ist, zu analysieren und einen auf den Marker- bzw. Haplotyp-Informationen basierenden Output zu erzeugen, wobei der Output ein Risikomaß des wenigstens einen Markers bzw. Haplotyps als genetischen Indikator von koronarer Herzkrankheit oder Myokardinfarkt für das menschliche Individuum umfasst.

## Revendications

1. Procédé de détermination d'une susceptibilité à un infarctus du myocarde ou une maladie coronarienne chez un individu humain d'une population caucasienne, comprenant la détermination de la présence ou l'absence d'au moins un allèle d'au moins un marqueur polymorphe dans un échantillon d'acide nucléique obtenu à partir de l'individu dans lequel l'au moins un marqueur polymorphe est choisi parmi le marqueur rs11751605, et des marqueurs en déséquilibre de liaison avec celui-ci choisis dans le groupe constitué des marqueurs décrits dans le tableau 5, et dans lequel la présence de l'au moins un allèle est indicatif d'une susceptibilité à un infarctus du myocarde ou une maladie coronarienne chez des individus d'une population caucasienne.

2. Procédé selon la revendication 1, dans lequel la susceptibilité conférée par la présence de l'au moins un allèle est une susceptibilité augmentée.

3. Procédé selon la revendication 1, dans lequel la présence de l'allèle C dans rs11751605 est indicatif de la susceptibilité augmentée à un infarctus du myocarde ou une maladie coronarienne.

4. Procédé selon l'une quelconque des revendications 1 à 3, dans lequel la présence du marqueur ou l'haplotype est indicatif d'un taux de réponse différent du sujet à une modalité de traitement particulière pour un infarctus du myocarde ou une maladie coronarienne.

5. Procédé de l'une quelconque des revendications précédentes, comprenant en outre l'évaluation d'au moins un biomarqueur dans un échantillon de l'individu, dans lequel le biomarqueur est un marqueur cardiaque ou un marqueur inflammatoire choisi parmi la créatine kinase, la troponine, la glycogène phosphorylase, la protéine C-réactive (CRP), l'amyloïde A sérique, le fibrinogène, l'interleukine-6, le facteur de nécrose tissulaire alpha, des molécules d'adhésion cellulaire vasculaire solubles (sVCAM), des molécules d'adhésion intervasculaires solubles (sICAM), la E-sélectine, la métalloprotéase de matrice de type 1, la métalloprotéase de matrice de type 2, la métalloprotéase de matrice de type 3, la métalloprotéase de matrice de type 9, sCD40L sérique, des leucotriènes, des métabolites de leucotriène, l'interleukine-6, le facteur de nécrose tissulaire alpha, la myéloperoxydase (MPO), et la N-tyrosine.

6. Procédé selon la revendication 5, dans lequel le leucotriène est choisi parmi LTB4, LTC4, LTD4 et LTE4.

7. Procédé de l'une quelconque des revendications précédentes, comprenant en outre l'analyse d'informations non génétiques pour effectuer une évaluation de risque, un diagnostic, ou un pronostic de l'individu, dans lequel les informations non génétiques sont choisies parmi l'âge, le sexe, le statut socio-économique, le diagnostic de maladie antérieure, les antécédents médicaux du sujet, les antécédents familiaux de maladie coronarienne ou d'infarctus du myocarde, les mesures biochimiques, et les mesures cliniques.

8. Appareil pour déterminer un indicateur génétique pour une maladie coronarienne ou un infarctus du myocarde chez un individu humain d'une population caucasienne, comprenant :
une mémoire lisible par ordinateur ; et
un sous-programme stocké sur la mémoire lisible par ordinateur ;
dans lequel le sous-programme est adapté pour être exécuté sur un processeur pour analyser des informations de marqueur et/ou d'haplotype pour au moins un individu humain en ce qui concerne au moins un marqueur polymorphe choisi parmi le marqueur rs11751605, et des marqueurs en déséquilibre de liaison avec celui-ci choisis dans le groupe constitué des marqueurs décrits dans le tableau 5, et générer une sortie basée sur les informations de marqueur ou d'haplotype, dans lequel la sortie comprend une mesure de risque de l'au moins un marqueur ou haplotype en tant qu'indicateur génétique de maladie coronarienne ou d'infarctus du myocarde pour l'individu humain.


### SEQUENCE LISTING

<110> deCODE Genetics ehf
<120> GENETIC VARIANTS USEFUL FOR RISK ASSESSMENT OF CORONARY ARTERY DISEASE
   AND MYOCARDIAL INFARCTION
<130> 2511-PC00
<160> 172
<210> 1
   <211> 599
   <212> DNA
   <213> Homo sapiens
<400> 1
   caactcaaca gcagagtgaa atgcgatgcg atcaggtttg tcgatggcat ttcacaaact      60
   tacagaagga agtctcttta gaggagaaca gcaattgagc acaggtgccc tggtgtagaa     120
   aggagatgag gaagcaaaac atgcaggcag atgggtctgt gtcctatgtg ttcttcctaa     180
   gagacaccca agcaaactga acttacgaat tgcatgttcc actgaaacga tacccaaaat     240
   gtccaggttc tgtgggttcg gactgattac tcttcattta tggggatcac aacccataay     300
   ttgttcatgt gtttgtgaat agatgaatag cctcggcatt gaattcaatg attcacaaaa     360
   gtgtaaaaaa aaatctgtgc ctgtgataga ctgtaggtaa gtttgctctt gtcctccctc     420
   actgcaaagt gtcacatttc taaaaacttc cctttctttt catctgacag tgagacaatg     480
   gatatccata gaaaccctca gagcatcttc aatattttcc aaaagtattg gaacttggtg     540
   caaatcactg tgcctacaga ttttgatgga actgcccaga tcaagaaaaa gatttgtat      599
<210> 2
   <211> 599
   <212> DNA
   <213> Homo sapiens
<400> 2
   ctcaggctct acatggcctg gggtgcgtat ctgaggacac tggtgggttg agtcccccca      60
   gttgctggaa gcggggtagc agtcactaca cccaaaagaa ggtacagagg gcagacacaa     120
   agggcaaagg ttccactgcc ctgggcagtg ccatgagggg ctggcagggt aggggttgac     180
   tggctctcag tctgagctcc ccagatatcc cacacacacc tcctcaaagg agggcaaggc     240
   caagccaatc acaccccact ttgatccctg cccctgggcc aggccgtcct gcgggatggk     300
   aggggataac atttctcaaa gactgttctg cgcaatctca agcgttcagg ggacaaataa     360
   gggtagaaac tggcaaattc actccactac cctacgggag attcagaata catttaagag     420
   aattaaaggt cctgacaagt cctgtagaaa agaaactgtt tagtgtttcc cagcctgatc     480
   tgaccgtgga aatctttttt ttaaatgact gccaataatg tcccacccac caggaaaaca     540
   cctgctggga aaagctgatg tggaaagatg ctgctcactg gaaggccacg aacatggta      599
<210> 3
   <211> 599
   <212> DNA
   <213> Homo sapiens
<400> 3
   ctaaatctgt tcatagcatt tggtcctcat gttctttctc tattctgaga ggccctccct      60
   gtaggggatc actgatgtgt atgtcatacc tgctatgagc taacctcagt ggggagtctt     120
   caaggaagtc tacagtgaac aactcagttc tctaaaggaa ttgtgatttt cctaagaact     180
   gcataggcgc tttcctgatg gtttgcattt ccacattcta aaacagagca tggacagagg     240
   ctctttactg tccttcactc ctattcaaga gcaaggttgt tgccactggt gttatttccr     300
   gtgtttaaag gcaaatagat aaaaagaatg gagaaggcct ttcacaccca aagtgaaacg     360
   cgtcctcttc tcatgcatcc cgcttcccct cctgacttcc tcctctatta ttaatagcac     420
   cagcatcctc ctaatgacct agcctggaat ctgcagaatc attcttgacc acgggtcctc     480
   tctaggccac ccacgttaaa ttgcttcagt tcatcttttt cttctgtttc cctgaccacc     540
   agcctacatt aggctcttgt taccacttaa ctgggcgcct gaaataagct cagcagctg      599
<210> 4
   <211> 599
   <212> DNA
   <213> Homo sapiens
<400> 4
   tatcaactaa atgaatactg gttctagtca aactgtcctg gataattgca cctattttcc      60
   tctttgttca tgttgttaac ttgaaatgtt ctgactggtg aataatcaac cagcatttat     120
   acatgttatg tggctgctgc acttcacaga tagaaaactt gaaatctatt acctaagtaa     180
   taacggcttg aaccatctta gtgggtttct cagataagaa gacttcaagt attcactgga     240
   ctgtattcat tttcatgtga acattataga tgagatttta gcccttatta ttgatttagk     300
   attgttccta tctgcataaa gtgccttttt tctcttcttg cataaagtta ttctagcctc     360
   agggtaatga ggacatttct ctccaggagg tagaatatga tgtggcaaat gtatctgcat     420
   atccagaaat gctttataaa gcagctgctt gctctgagca ctcattgtgg tggaagccaa     480
   gttaaatgag gtgtgctata atgtaatagc atttttattt gttaacaaac aagaaattta     540
   catacctcat gcaagccttt ctctcttggc atttaacccg catttaattt atatatata      599
<210> 5
   <211> 599
   <212> DNA
   <213> Homo sapiens
<400> 5
   ctggggccag aataagattt tattactctt ttatagagct tttaaattac tataagtgct      60
   tcttgtttca tagaagatga atactctagc aaaataaagt tctcattacc tggctggagg     120
   aaagatacat atttaaacat ttaataattg ccagatacac tataattaca cttttaattc     180
   taacagaata atacaaaagt tctacaaatg ataatgtgat atgccacaga aaataagttt     240
   aatatgtaca gaaggatacc tcatgatgtt tgtttctttt tgcaatctca cttggcatck     300
   ttccatcttt ggtttgtagc attttattag ctccaagttc aagcaacttc aaaactatat     360
   ttttatgacc ctgacgtgct gcccacgtta aagcctgtaa gtagggggaa aagaagattg     420
   tttaaagaga aggagtgtat atgagacaga caggaaaagt tttcaacagt atttatgaca     480
   caataaaaaa gagaacatag ttaattaatg gaatgatgca atctcttcat tttacaatat     540
   aatgtaaaca tatttccaaa gttgtaattg caaagctgac tgcaaagctc attgatttt      599
<210> 6
   <211> 599
   <212> DNA
   <213> Homo sapiens
<400> 6
   atcttttttc agaatctgta ctatgcatct aatatatacg tggcactttg cttggtcctg       60
   ggggtacaaa gctgagtcag attgttacaa agtctaaata actgagataa aggtgttaat     120
   acagcccaga aaagcacact taattcagcc tggaaggttt tttttccccc ctccttcgag     180
   attttcagag tatctccttc ttagtaagaa ttgcttggct tagtatgttg agaaaaacat     240
   actaagtatg tagtgttgtg ttggaaggaa aacttaactg ggtgagattt tcagctgagk     300
   agctttagct gttactctca tggaccttcc tctgggcggt tgatacaggt tctgtaggaa     360
   aaccgctgaa tgaaagcagc tcctgcagat gctattctgt tttcaattgt ggtaattcct     420
   ttaatttttt ccctttcaat ttactcctta ttctctagta ataatggact tttgggaggt     480
   tgcatttatg ataaaaaata gcctggggct gtacaatcct tcctatcatg aagaagtaca     540
   aagttgtaag tagaagcagt ggccttgctt tgccaatgca cactgaaggg gataacgtt      599
<210> 7
   <211> 599
   <212> DNA
   <213> Homo sapiens
<400> 7
   tattctaaat gttaatatca aaaatattat tttcaattca ccccaggcac agagaactaa      60
   gtattattat tgctattgca ccggcattcc ccaatgagac agtgattttc ttttaagaca     120
   tttttaaata atataggcag aattaagtag acggtgatct ggtaagtaga tgtttcaggg     180
   taacagctgt gcaatgctcc atgcagggaa ttagattgtc attttattcc ttaccaggaa     240
   catacattca gttaaacaat tatttgactt ctgctcttcc actgatttct aagttgaggy     300
   tctctcttgt gcctgtctga tcagataagt agagttgtgc cttggtttat agatgagata     360
   aatgtgtatt tgaataagca taagttaaag aaattttaaa atcccttagg aagctaggct     420
   tatcagagaa atccaaggaa atacattaac aaactaggaa tttgttctaa caggttaatt     480
   ataactcata aacttattgg gtttttttac cttttaattt tatattacat ttgcttataa     540
   taaggaatat tgctaggaat aaaatttttt aatattctac aattaacaat tatctcaat      599
<210> 8
   <211> 599
   <212> DNA
   <213> Homo sapiens
<400> 8
   atcctgaaca actgaataga agtcagggcc taccttgaac cagtcagaaa atgcattcca      60
   gaggagatgc ttttgaatca cacgtacaag tcccagaaaa cttctctaca acctttacct     120
   ttgtccttta ggtggaacaa agtactatat aaaactgtgt ggggattatt ttgatgaggg     180
   ttgaaaaatg aaggctctta tccgttattt aaattcctgt gtgcaccgcc cccttcacaa     240
   agtgtgggag taggtcttct acatcaagat gcctggagtt tggttcagtg aaaaccttty     300
   ggagggcctg gggatcttga ttgtccctca gtgccctgat gaaatgtttt tatttatcct     360
   gcttgctgcc tggagttgac actggtaata tcaatcattc cctgccagcg tattaacatg     420
   gctgcacctg cttaaaccat ccacatgttg taactcacaa gaagttggga atcaaatgca     480
   gtattattta tgtactccct taacttgtcc catactacaa aaatttccac tctgacacca     540
   tctgctaggg aaagggattt atctatcgat gtgaaacacc cactgaaccg gtatcacat      599
<210> 9
   <211> 599
   <212> DNA
   <213> Homo sapiens
<400> 9
   cctgggtccc tctgacggca tactcttcct ctgcgcagag ttcatcccga agacacggag      60
   caccccgcgg cctacagcgc gggcgcccac gcgggcgccc cgccgagggc gccccacggc     120
   cgctcccccc gcgccaaccc cgcggccagc gcggcccacc cagcagccag ggagccggag     180
   gggcggcggg cggcggcggg ggcggctgaa ctcggcgagc cgggcgttcc gggatggcga     240
   ggtgcgcctg gtgcggcccg cgggcctgag ctctggcagc gggcgcgtgg aggtgttcgy     300
   gggcggacgc tggggcaccg tgtgcgacga ctcctggaac atcagcggcg ccgccgtcgt     360
   gtgtcgccag ctggggtttg cctacgccgt gcgcgccgtc aagagagccg agttcggcca     420
   gggcggctcg ctgcccattc tgctggacga tgtgcgctgc gcgggctggg agcggaacct     480
   gctggagtgc cagcacaacg gcgtgggcac ccacaactgc gagcacgacg aggatgcggg     540
   cgtcgtgtgc agccaccaga accccgacct gtaggcaaca cgccgctgcc ccaggccat      599
<210> 10
   <211> 599
   <212> DNA
   <213> Homo sapiens
<400> 10
   gaggtgagag gatcatttga gcacaggagt tcaaagctgc agtgagctaa gaaggtgcca 60
   ctgcatgggt gatagagcaa gaccccatct cttataaaca aacaaacaaa caattttttt 120
   tatcccctag ttctttaata gatcatttat gtttcttttt ctcctgtcct ttcccctttt 180
   ttcctattct ttcttcctcc ttcccgaggt atggaaggat tgtgaatcta ctcagtcaat 240
   cttaaaaggg gcaatgggga gagggaaaac aaggaaccct cacaggctaa tttttaaacy 300
   acatctctta agaaaatatg gagaagcaaa gagaggaaaa tgtaaattac ctgaaattct 360
   accactgtaa atatgttgat atactttcag actttttcct atgcatttat aacacacaca 420
   cacacacaca catatataag ttgcaaaaaa tggccaacag tatatatata catactcttt 480
   tgtagtcccc tttaagcaac ttattgtgaa tgtctttccc tctcaagctt tatactgaca 540
   acatcatttt gaaaagctac atgactttcc attgtgtgaa tgtactatca tttatgaac 599
<210> 11
   <211> 599
   <212> DNA
   <213> Homo sapiens
<400> 11
   ctgtggacaa atattgattg tggcacacat aaagacagtt ttgtaagtgt gagtggtgag 60
   aaaacatttt aaaatatcaa tcgtccagag aagattagaa aatttgcaca ctttatgaat 120
   ggttccaata acccgcagat tgtctcatat tagacttcct cctgagctga tctccagtca 180
   tataagagtg catattgcta tcaccgcttg acaaactggt gtaaaaatta aggaaacata 240
   acttttcctt gtagcatctt aagaataaca agttgtcctt gacagtaata aagaaataay 300
   gcagggaatt cagaagaaaa ctatttcaag ggagtcatag aataaaagcc agagtaatga 360
   gtttggtgac aacaacactg agacaagaga aagaatagaa actgctttaa aaaatttgca 420
   aaatctactg atcgcagtga tccttctctt ttttgttgac atacttaaga ataaatttta 480
   gaataaaaaa attctggaag atatctctgg tgacttaaat gttctagata caaagctggt 540
   cgctcatggt agttggatga tgtgttcatt tctacttttc atttgaatgt gacttagag 599
<210> 12
   <211> 599
   <212> DNA
   <213> Homo sapiens
<400> 12
   acataatatt ttactctata aaaattaatg attcttctct cactttagtg tctgtgtgcc 60
   gatgcctccg taggaaaaat tgaataaata cgcaccaaag tgagaaatga gttgtattaa 120
   atttatactg aagtgcgtgg tcatcttagt aattctagaa ctttccataa actattttat 180
   gaaatgcatc aacgtttcta ttttagtgtc aagacaccag aaaaaattaa aaatgattct 240
   actgtattta gtcctacgat agatcacacg actattcatc gaagtgaaaa tactggtgcy 300
   aacaaaagcc aacctttcaa gaaataatct gcctacagtg tttgatccag tatctataca 360
   cataaaacca gctgcaagat aagcaataca atttaaaaca gttgttacaa tttcaagtga 420
   aattggttgc ttccaataag acttcctttt atatagaggg gaaactcaca gtgtggcaat 480
   aaattcatat tcatttaaat tagtgcctaa atataatgtg ggtggtctcc caagcactcc 540
   ttggtgaatt attggctaag aggtgtttag aaagtctggg agggtgggta gtgatcatg 599
<210> 13
   <211> 599
   <212> DNA
   <213> Homo sapiens
<400> 13
   ctcaacctcc ttgttgaagg catcccggcc tttttggttc tggagcctcc acctgcctcc 60
   taatgctccc ctaagtcagt tagcacagcc cccgaccctg caccacctcg cagttgttgc 120
   tggtgtgcgg ttctcgccgg agttcttagt gcttcacggt tatcttttgc tgggattgtt 180
   gaaagttaac ctcccgttaa ttcccaaagt gttactttta ctgctatact ttaaaatttc 240
   ttcatttgcc actttttagg tttgtggtcc cgggcaagtt taatttcttt tcaggattcy 300
   catttgtata atgaggacaa cagctgttgg ttcaggattg tgaagcttca atgagataat 360
   tgtttagcat gtttttattc catcatgaac accatttgta ttgtgtttta cagtttacaa 420
   agtgctgtca caggtgttac ttcatacctt aacactactc ctgtccagtg aatattagta 480
   gcctcatctt gttgagaaga ctgaggccaa gaggtggatt gactaggcaa agtcaacaca 540
   gtatgttagg tagaactcca acctaaacag ctattcttga ccctaggttg accatatgg 599
<210> 14
   <211> 599
   <212> DNA
   <213> Homo sapiens
<400> 14
   ctggtgatag agtctttagc cagagccctg gatgagatca cctaggaaga aaagggggat 60
   ggcaaagaac atagattgag cagtgaccat gaagtcacct gtcaataaat gcagaaccct 120
   cataacttgg tcaaaagcag ttttggtgat aggtttgagt gtgtttgagt gcgttgagaa 180
   gcaaatgtga ggtgaggaat gggaaacaag tgtgtgtgtc tctttcagta aattttatgg 240
   aaagagaaga agagaaatgg agccaaaggc agagaataat tcagtaagat aaaagatcty 300
   gtagcatatt tgtgtgttga tgggaatgac ccaacacaga gaaggaaaat gtggtgtgtg 360
   tgtgtgtgtg tttattgggt gtcaggggga cagaggaagt agcaggcaca actttgattt 420
   cacaattctg tgctggaaga gagataatag ttagagatag tggatattct gtaggctatt 480
   tgaatagcta tgtagtctaa tgtgggtaac ataactggca aatatttatt cattgaaaac 540
   tgtatgccta ggtacaactt taaaagatta cagtctactt gggaaaacat ataatttct 599
<210> 15
   <211> 599
   <212> DNA
   <213> Homo sapiens
<400> 15
   tctctttatc ttttatctcc cttacctaat ccttcttctt caacaaaatc tgtttattca 60
   cactttattt atgatagtct ttttgccttc atgacttcct tccaagatat acccgtccaa 120
   tttattcaaa tactacccat ctttagctta ttacatttct tctattattc tttgaaactg 180
   ttgtaatccc atattttact tgagttttca caggagtgca tgaagtggtg gtggctgtgt 240
   taattctttt acaaccacat tgtaattttt ctgaatgtag ggaccatgtc ttggacttcy 300
   ttatactgag cacgtaggtc tataaaagat aattgcttgc ttgaatcacc ttcagttcat 360
   tcatgcaaca acatttaatg agcaattact ttgtgctagc aggttaaata taatacttct 420
   gaaaaaaaag tttttcaaaa ttatttactc ttcctaaaac ttacatgtca ataccttaga 480
   gaaaaaaggc catttctctt ttcacctctc tttttgtaac tttgcacagt taaactctgt 540
   ctcactcacc tggctcacta gtgactcaga gattttgctt gggtcccaaa tctaagatc 599
<210> 16
   <211> 599
   <212> DNA
   <213> Homo sapiens
<400> 16
   cacagaggca acttgcctca gagtatatga tgccttggct ggggatgggg tagtagacct 60
   agttcccagg ccatcccaac ttggacccac gagatgtccc ctgccccagt ccggctctca 120
   gggttttttg tactgtgtca aaggatgtag agggcttcac tttccctgga aatgtgcttt 180
   agattgcttt ttgggggaat cttgcaaact gggatgtttg gacttctctc ctgccctatg 240
   ctgaatttgg agggagccat tttctgtctg ggaaatgctt tagatcttgg agccactggr 300
   gatgttgaag aattgaaaaa ggccaggtgg ggaccctcag gtacccacag tgcttctccc 360
   cggtgaggag gcaccagtga ggaagtgggg tctgatatct ggtgttacct gagaaaaagg 420
   tgggacggaa aaagaagagc cttctaactg gtccagtaag gccactctgg gacaaatcac 480
   agagtcatcc aaggtggcag ttatcttagc tataaggtgg ttttcagtgg gaacttttca 540
   ctgaggacct tcgttggttg ccagcagtgg aagttcaagc tctttcctgc cctgttgta 599
<210> 17
   <211> 599
   <212> DNA
   <213> Homo sapiens
<400> 17
   taaacctatc gcgtgccagg tactggttag gtgctctaaa tggtgatttg tttgttttct 60
   ctaaaacaaa cagggccttg ctatgttgcc caggctggtc ttgaactcct ggtctcaagc 120
   aatcctccaa cctcagcctc taaagtcaat aagattatag gcgtgagcca ctgcatctgg 180
   ctaaatggtg ataataataa cactaactgg cattggttca gggcctacta tgtgccatgc 240
   actgcttaag ccttcactca tcggatcccc acaattccaa gaagctgctt ttagtactgy 300
   tccctttaca ttgttaagaa cctgaaggtt cttggacttg tccaaggtca tacagtaggg 360
   ctgcttgtta agacccctga tcccagcctt ctagctgcag ggagcacagg gaccccagag 420
   cctggcaggg gcagcatgca gaggcaaggc ctgctgggag acaggtgcag ggacggggag 480
   tcagttagga aaggagctca aacagggttc ggggtaggcc acaaattccc cctaggggga 540
   tggaccccca gccccgtccc acctcccaca agccccaacc cagcagggga cccacacct 599
<210> 18
   <211> 599
   <212> DNA
   <213> Homo sapiens
<400> 18
   gtgtatatat gagtggtatg agggcgaatt atttttgttt tctttttaat gatctcttaa 60
   attttccaga atgtctacaa tgaccttgta ttacttttct aatcagaaaa aaagaaaaaa 120
   gttaccgaag aagagataag tgaagcacac acaaaaggca gaatatatag tgcatcaaga 180
   atgtgtgttt tataatctga cagacctagt tagaccatag tccatatttc aaatataatt 240
   acatgtgctc atagctgaga accttctcct gggatggatg catttcacca ggtcactgcy 300
   gaaatgttgt acttttatgg atggtgatga ggaagcatct gttttaggtg tggtatttcc 360
   tggaggcaga aaactgcttg agttagctca ttcagttttt cagtggcctt tctaatgcat 420
   gttttgtttt gttttaatgc tttgcaaaag attcctagag tggaaattgc ctttgcaaaa 480
   attataacag tgagaaaatt atgacagtga aagggatctg ccctaactgt ccccatcttg 540
   cttctgggta tctttgctca ttcctgggtg tgggccaagc taactttggg agaaattta 599
<210> 19
   <211> 599
   <212> DNA
   <213> Homo sapiens
<400> 19
   agcctggctg ttttcatttc caaagacagg gctggtttcc ttaggagcag atgggtccta      60
   tccatctctt catccctgca tccccactac actgtgggcc tgtggacatg gctgtatctc     120
   actgtggtcc aggactcggc aagggttgga atcctggctc tggaggacct gaggctgctc     180
   agtaagtgtt ctaagcgttc atgtctgcat ttgtagaaag agagtagaat aataatactt     240
   gtgttgtaaa gttgttgaag cattcagtaa gctaatctac tcaaaactct gtgaatgggk     300
   cctggagtta agtaagcact cagctaacat catctgccat catggtggtg ggcgactaag     360
   acggcatccc caggtgactc catattcaat gccattgtct acacttcccc acaggcacct     420
   gatcaggtaa cacgccaggc attagcattc attcaaacag cactgagact ctgaaaggtt     480
   acatggcgtg ctccaggtga gccgcttgac aggggatgag cttttacctc caaacccacc     540
   ccttctaccc agaaagaggg tgcgttccca gtggccggga ggatgggagg gtggaggtg      599
<210> 20
   <211> 599
   <212> DNA
   <213> Homo sapiens
<400> 20
   gttttatggt gcatatgtta cacatcataa cctttttcaa gtctctttta gataacaggt      60
   aggatacaat aaaagggcag acatatttat ttaagatcaa ggaggtaagt tgttatttta     120
   ctaaaactta acagaaattg acaaatactt ttatatgtct tgaaaaagtg cttaaagcca     180
   catttcatca aataaaatag attttatttt agcttaccta ttggtaattt tcagggctct     240
   gaaaaggctt taattaaaga gtatcagagg atttaagagc aggatggctg tttgtgtcar     300
   tattaagcca agatttacaa aggcctatta cggttccagt gacatttcct tcctattttt     360
   ccatatcagc aggaacattt cttgagggaa tagattgcta cttgctgata agatctctga     420
   cagtaaaggt aggctaagca ggatgccagg tatgaaacta gagaataaaa tcttatcaaa     480
   tgtaaaactt gtaacattgc acctgccatg tagttggttt acctcgaatg gtcattgctt     540
   ttcctcaaaa gcatataaag ttgtatccta ataagtgttg gtatgcaaca aaattatgt      599
<210> 21
   <211> 599
   <212> DNA
   <213> Homo sapiens
<400> 21
   ccccagacaa gtgcccgtat acccagaact ttgtatttaa tttcatgggg ttcatgaagc      60
   ctctgaaact cagcttaaga gtccatacca accaaggtcc ttataggaag agcttgtgtt     120
   tttgttttgt tttggctttc tgcagtctct taataatgtt tattgaatga gagaatttaa     180
   ctaatttccg gtttccataa tcactttaaa ctcggtattt gatttccttt tccctgggac     240
   ctgtgacagt gccagcttca tagcctagtc taggcatgcc agttgcccac tgtggcaatm     300
   aatatctgag cctgtggttt ttgccttagg taaactgtag agatggactc atggaatgct     360
   tggaaaattt ttcagtttat gataatgtgt aaatgtcgag agccaattat tgaggaatgg     420
   cacctcaaag tatttgggta ctctagatca gacatgacca tcttggtgtg tgaaattttg     480
   ctaatgcatc tttcctaata gaatatacaa tctcagggct agaaagtctt agagatcatt     540
   taatttaatt cctccagatt gattaagtgg tttgtccaag gtcattcaac tggtatagt      599
<210> 22
   <211> 599
   <212> DNA
   <213> Homo sapiens
<400> 22
   ccactgtgaa aactccttga aatcatgtag acaaatgaaa gctaaacatc aaataaataa      60
   cctaaactgg taatactccc atttggctct ccttgtgtct tttcatgtgg gttagctctt     120
   caaccagatt cagcatataa attgagtatg tatggttata tggtgacttt caaaagtaaa     180
   tactttttct gtggaacaaa agagatggat ttgtggtttt ccaggattcc ttttccctga     240
   cttggtagaa agatagccag gcatagctca gcagacctga aaataggtga gctgtcaagk     300
   tgttggcagg gagaggctcc tctgggtggg acttggggca tcctaccagc gaaaaagaga     360
   ctaagtttaa gtgatttcac atttgggctt tacttactat aattgctgaa gtgctaaatt     420
   gagtgtgaca aatctaacag gtaattgcca agtaggtttc attaccagtc attgcataga     480
   taaatgatgt tccttatgtt aaaaaataaa accaaactaa agaaaaaaaa ttggactata     540
   tttggaggta gaattcagat attgcagata tgttcatata catcaaagct gtttcaatg      599
<210> 23
   <211> 599
   <212> DNA
   <213> Homo sapiens
<400> 23
   cactgaggct ctaaggcgcg gttctgggct cctttccagg gaaactctag gcctctcctg      60
   tgctcatgct gcctctcaag tggctcctgc atcctccaca ctaaccggtt tcacaagttt     120
   ttttttgagc attcttctcc ctcctccaca gccagtttag agctttcttg atcaaatcgc     180
   ctaacctccg ggcgaacacg ttcttccctt tcctcgggca gggcacccca tcccagccaa     240
   ggagccacca tcccactatc ttcagtcact cccagctggg ttacccgtct catgtgggcy     300
   tgtttgtagt cagttctgcc tagggttggc agatttcgat taaacctccc actaggtcca     360
   ctctcttctg agatctggcc ttccagaagg cacacttggg tctttgaaca ggaatggaag     420
   gtgattaggg ttgtcccggg gttggcagaa gcgcttgggg ctacaccagt gtggggccga     480
   aacagctcta agcacctccc actgccccac cacagatcca agtttgagca agaaagactt     540
   ctgtgtcctc cctgaactcc atctcctccg ctctgcccac agagactgga cctggcttg      599
<210> 24
   <211> 599
   <212> DNA
   <213> Homo sapiens
<400> 24
   ccccctcctt cgagattttc agagtatctc cttcttagta agaattgctt ggcttagtat      60
   gttgagaaaa acatactaag tatgtagtgt tgtgttggaa ggaaaactta actgggtgag     120
   attttcagct gagtagcttt agctgttact ctcatggacc ttcctctggg cggttgatac     180
   aggttctgta ggaaaaccgc tgaatgaaag cagctcctgc agatgctatt ctgttttcaa     240
   ttgtggtaat tcctttaatt ttttcccttt caatttactc cttattctct agtaataatr     300
   gacttttggg aggttgcatt tatgataaaa aatagcctgg ggctgtacaa tccttcctat     360
   catgaagaag tacaaagttg taagtagaag cagtggcctt gctttgccaa tgcacactga     420
   aggggataac gtttgctcct gttcagagtt cacggtcggg cgcggtggct cacgcctgta     480
   atcctagcac ttttggaggc cgaggccggc ggatcacgag gtcaggagat cgagaccatc     540
   ctggctaaca cggtgaaacc ctgtttctac taaaaataca aaaacaaaat tagccaggc      599
<210> 25
   <211> 599
   <212> DNA
   <213> Homo sapiens
<400> 25
   cttataacag tccagttaaa gttgttcaac tttgcaaggg gcaaaaggca agtttaatac 60
   cctttaccaa ggtattaagt atatttttga tgtacaatat tttcaactta caatgggttt 120
   gttggatgtg accccactgt aggccaattg tactttggaa gcccactgag acctactgca 180
   acccccagtg agtgacagaa ggggtgaaac aaatgattta ccattaatag cagagagatc 240
   caggtcaggt ggagctgtgc catgcccttc cagcaacgtg gactgcaggt caccctttcy 300
   gaccccactg ctatctgctg ttaagaaaag aatccccgaa agtcagcaca gtcaagagac 360
   actctccatt caagccagga aacgcaggcc gtgtttttga acaataccac ttgcttctaa 420
   aagcccaatt tcgtccaagg tggaaaggct aaaaaaaaaa aaaaaaaaaa aacctgtgga 480
   aattcccagc catcaccttg actctatttt taaagtacca ctagaagaca gagacattct 540
   gtataaccaa agagacgcaa ataggccttt ctgctagttc acaagatgct acaggtggg 599
<210> 26
   <211> 599
   <212> DNA
   <213> Homo sapiens
<400> 26
   ccctaatgtg aagaaagacc ttaagggaga ctctttgatc cttaggtatt tgggactgac 60
   tcagagcact gcacagaagt cactatcgcc cccttttcaa atctccatag gcaccgtttt 120
   ctggatggtt ctctttaggg cagctttgac ctctgtgttc cgcaggctgt agatgatggg 180
   gttgaggatg ggggtgacca cagcatagaa gagggacacc agagggtcag tggccggatc 240
   gtagctggcc ttagggcgaa tatagataaa gagtgcggtg ccatagaaga gggagaccah 300
   gatcaggtgg gaggagcagg tggagaaggc cttgcggcgg cccgcaacag atgggatccg 360
   gaagatggta acgaggatac gcccgtagga gcccaggatg aggccaaagg ggcagaggat 420
   gaggagggct gttgccagga taatctgcag ttcattaagc gaggtgtctc cacataccag 480
   ctgcaggaca ggctggatct cacagaagaa ctgcgggatg gtattggggc cgcagaaggg 540
   caaagagaag atgaaagggg tgtggcccag ccccaccagc accccacagg cccacgccg 599
<210> 27
   <211> 599
   <212> DNA
   <213> Homo sapiens
<400> 27
   tcacacttgg cacagacaaa gtgctgcaag gacaaagggc gggccgggtg gcatcagggc 60
   tagagcggtg accccgaggg acagtggtga cccggggaca acagtgaccc caggagccag 120
   tgactccagg ggaccacagt ggccccagga ggcagcagtg acagaagggg acagaaagca 180
   gagtctgggc aggtgcaggc ctttcaggat tcacactggg cagaggggac tgacacctct 240
   gtgaatgccc tggggtcagc agaccctacc cctcctctaa ggcactgatg gtaaccttgy 300
   gagtcaccat cccctcccag tctggcctcc ccctcggcag cctcagcccc aggagtcagg 360
   tgccagcctg gtgctgcggc atggtgggcc ctcataggca gaggcggccc tgcctgtcca 420
   cgtggggcct cctgcaggca ccccctgcaa gcctgggatc actgagcagc accttgcatt 480
   cccccaggga tctgcatgtg aggatgaggg ctgggggctg gggcctgcat cagggcagga 540
   ccgcatggcc tcccacccaa gccgccaccc aaggcacagc ttccggacac agcgctggc 599
<210> 28
   <211> 599
   <212> DNA
   <213> Homo sapiens
<400> 28
   ctttgggagg ctgaggcagg tggatcactt gagatcagga gtttgagacc agcctggcca 60
   acatggtgaa accccatctc tactaaaaat acaaaaattg gctgggtgtg gtggtgggca 120
   cctgtaatcc ctgctactca ggagactaag gcaggagaat cacttaaacc caggaggtgg 180
   aggtggcagt gagctgagat cacgccactg tactccagcc tgggtgacag aatgagactc 240
   tgtctccaaa aataaataat taaaaatgct atgaaaacca agcaaaacac attgatgggr 300
   ggaatcataa ctgtgaccat ccattggtgt cccctaccat ggattaatga acaaacaagg 360
   gcagagggaa aaataccctg cacatctgtt ggtagaaagc aaaaggtaca attaatgggt 420
   tatttccctg aggcacagcc ttacttgcac caatgaatgt ttactggttg aaaatttgtc 480
   agtggcagag cttatataca tctcatccta tcatatgaac gcatatagca tgtgacatgt 540
   aggctgagac ataaaggatg aagaagtatt tacatgagtg agaacagtga cgcaaggac 599
<210> 29
   <211> 599
   <212> DNA
   <213> Homo sapiens
<400> 29
   gctagggaga cagacactca gcaaataact gcttaaaaca taaaatatat gagacaggga 60
   aaagtacact ggagagaaat aaagcagtgg acggggtagg ggctctgatg ggatggagag 120
   gggctgcgtt tttaaatggg gagcttcctg agtgggtgaa attgtagggg agacttggag 180
   gaggtgaggg gtttggccaa gcaggtgcct gaaggataag tgctcctgag ggaaggaaca 240
   gaaggtgcaa aggccctggc aggagcgtgc ctgtgtgctg cggggcaggg aggagccacy 300
   gtggctggag gagggtcggg gaggggaggt cctgggctca catgtacaca tgtacgcatg 360
   tattcgttca ttaatttgac gtctacctag ccctaccagg ggccaggctc tgagtatctg 420
   tacctcccac cccgaccctg cccaacacag aggcctggga gggggtggcc cacctgaaac 480
   acgatcctgg atttctcaag caggtactgg gaggttatgg caccagagat cacgcccctg 540
   gggtggggag cacagtcagc tctcagtgag ggtaggaggg ctggtgttgg ctccgaggg 599
<210> 30
   <211> 599
   <212> DNA
   <213> Homo sapiens
<400> 30
   acagcgaagg gatggcgccg tgagccctca gcgcggggag acacacgccg ggccggggcg 60
   ccaggagcgg gggtggcggg gaggggaggg agggaaggct ggtgacgtca gtggttccag 120
   ttccgcagca gcggaggccg cggccgggga cgtgagacac cagctgacgc ccggagcctc 180
   acaacctctg cacacacatt ctcctgaggg ccggtgcctg gaaacaaagg ccacccccgc 240
   cggccggaaa cttctaggct ggcaatggag atgggacatc tggtgacccc ctctgtatcy 300
   tcctctccct tggggtcctc caggaagggc tgcgtgatca atgacccact tttttgtttt 360
   gtttttgaga tggcctcttg ttctgtctcc caggctgggg tgcagtggtg tgacctaggt 420
   tcactgcaac ctccacctcc cgggttcaag cgattctcct gccccagcct ccctcgtagc 480
   tgggactgca ggtgcgtgcc accgcgcccg gctaattttt tttttttttt ttgagacggg 540
   gcctccctct gtcgcccagg ctggagtgca gtggcgctat ctcagctcac tgtaaactc 599
<210> 31
   <211> 599
   <212> DNA
   <213> Homo sapiens
<400> 31
   taacataaaa tttactggct ttctcatttt tgatttctct aaagtcttac tatttaaagc 60
   aaaaataata acagattttg tgtctaaagc atatgtaaaa ttgtagtgta taaaaatagt 120
   acataggtca gagggaggat tggagatata tatagatata tagagagaga tatatacaca 180
   cacatacaca aacacattct tataatatgt aattttaatt ttatttttcc ctttattctt     240
   atcattgtat atgtaatgtt tttgcagccc attttttgcc acttactatg tgttgaaggr     300
   catgaaccaa tcacaatatt tgtaatgact tcatgataat ccctctgtgt gtcagtgaaa     360
   atgtattcac agataaaagg ctcctgacta actaatttaa gcagagaagg actttgcaca     420
   aggcattaaa ttgcttaatc catgacagga gtgagacagc tggatttaat gtctagaaat     480
   gacacccaaa gacagcctgc accactaaaa gcccaggaga gttgcttctt tggacttagc     540
   attaggccac ttgtattagt cacagttctc ctgagagtat tacacacatc tctctgtct      599
<210> 32
   <211> 599
   <212> DNA
   <213> Homo sapiens
<400> 32
   gtcccagtta cttttgcagc ctctcttttc ctattttgcc ccatcccatc cccacaaaga      60
   actacacatt caaccaaacc agactcctct ttccaagaac acatccccac cttttcgtta     120
   ctacctattt ttacccatcc tatcctcttt aaatccttcc tcttctacag atttccccaa     180
   ttctccaatc aaaagaggtc tcattctacc acgcagccca tgcctctgca ctgccaagca     240
   aggcagagca ccaacaaggc aaagtcatgc agaagtggca gcaagatctt tggagtcagr     300
   ccaattgagc tcaaatctga ttcagctgct tatatgctgt gtgatcctaa ccaaataact     360
   aaatgtctct gagcctcagt ttccacatct atagaacaga aacagggcct ggcacacagg     420
   agtcctcagg agaactgggg gagcaatagc tggcacaata gagacttggg tatctgtctt     480
   ctctccccct cccccgtaca aaatgagctc cttgaaaggc aatacaatga agcatctccc     540
   agaagacctt acacatgagg ggacctcaaa aatggagttg cagagttatc taaaagcct      599
<210> 33
   <211> 599
   <212> DNA
   <213> Homo sapiens
<400> 33
   ttcccgctgc cacaccccac cctgggcccc cgcagacacg catctctaac tcagttgggc      60
   ccagccttct ggatggcttg gggtaggcca tgggcccacc tggggccagg ccagcccctg     120
   gggcagctct ggaagagcag tgtggaggag cacttgcttg cagcctggct tcagcctctg     180
   gcactgctgg agtggtccct gggagcttct gcactgtcgg ctttggggac gtctcaccca     240
   cttgggttac agtaggcctt ccccacccag agagaagtgt ttccacccca gagacattgy     300
   ctgtcagccc ctgaagtgct cgcctccccc agtgcccgtc accagccctt cctatctgtg     360
   gggtccaagt caggcttccc ctgcggccac cagccatagg gagcagccat cagcccccga     420
   gtcagaactg cttctgtctg tccatacctc caggctctcc cggagagggg gacggatatt     480
   tatttcctaa agtttgcact taattgtgag gattctcagg attgttgggg gctactgaaa     540
   agaggaatgt gttgaatgtc gcgtttgctg tccactcgtc ctagaagttt agtgttttt      599
<210> 34
   <211> 599
   <212> DNA
   <213> Homo sapiens
<400> 34
   gaagaggtaa gggaacaatt cggtatctgt gttttatata tcatggatag tcaagaaagt      60
   cattctaggc agtagggaaa tgtgcacaga ggtactgcaa ttacatgcgc ctgtaaaacc     120
   cagaggctgg cacactggaa gggaaatagt ggagattcct ggcatctcct ggccacttac     180
   gctgaggtat tcccactagc tgtgtgccca gcacttcctc tgcatgcctc agatgcattt     240
   gacaatctca ggtgaactgc acttcagggt caagggaacc ccggccatgg ttctaagaar     300
   caactcccat tttagtatca cctacatttg aaaccacaga gcactgtcca ggagaggtga     360
   tggtggtggg tctcctcctt tggctctctg gcccatcagc tgatactagg gagcgctatc     420
   cctcagccca catttctcag catggtaagt ctgagagtct tgatgattaa aacacatttt     480
   tgtgtgtgca atttcatagg ttttctaaac tcttgaaacc cttgcatgaa ggacaaaggg     540
   accccacgtt acacactcct acttcctgga gtatgctaag ggcttcccaa cactggtgc      599
<210> 35
   <211> 599
   <212> DNA
   <213> Homo sapiens
<400> 35
   ttttagtaga gatggggttt tgtcatgttg gtcagactgg tttcgaactc ctagcctcca      60
   gtaatctacc cacttcggct tcccaaagtg ctcggattac agacgtgagc tactgcaccc     120
   aacccgcctc accgttttta aatgggttac cacatatgta aatagaatag ctcaataaat     180
   attcactcca taactgcagg agttagggga aagggaaaga aggtaagaca aggtcaacag     240
   aagacctgca ccccagaaaa tccaaccccc atggggcaca gtccaacgtg ggggtgggar    300
   gaatttacat ctttgccacc aaagaaacaa atctttgccc cacagcctgg gattaccagg     360
   gcaaccctca acgtgagggt aggggaaaac agaagagggg atccattttt cagggggaac    420
   atgcttgtct tggcagtttc tctcctcaaa gatcctgaga cctccaaaca agaccctcgg     480
   gccccggttg gctggggagc gttgagcacg cccaaggaac gtggacacaa gcccctctgt     540
   aatggtgtgc ctatcagggg tcagggatcc agcctggtcc acggtgaccc cggcgggca      599
<210> 36
   <211> 599
   <212> DNA
   <213> Homo sapiens
<400> 36
   ctcctgggct caagtgatcc tctcacctcc accatccaaa tagttgggac tacacatgca      60
   tgctgctatt gctctgctaa attaaaaaaa aaaaattaga gatgaggtct cactatgttg     120
   cgcaggctag tcttgaactc ctggcctcaa tgaactcctc aaactcaagg ctcacacatc     180
   agcttcccaa agtgctggaa ttaaaggcat gagccaccat gcccatccca cactttattt     240
   tatacttact gaactgtact caccagcttc ctcaaccaca ataaatgatt caggtgtccr     300
   tacaggaagt ggagggggga tttcatcatc tatccttgga gcagttgcta tccaaaatgt     360
   caaaaatatt gtaacaattg ttaattagaa caatccaaag gaaattctta tattctaata     420
   ttaaatataa atttaccata atttatattt aaattccgtt gaagcaacat tatcagtaaa     480
   gttgacactt gttcattcaa ggaaaaagca aaataaattc tcttaaggta caaacccagg     540
   aggtttttgc ttattcttta acatttattg ttttaaaagc tcaaaagtag ggctgggcg      599
<210> 37
   <211> 599
   <212> DNA
   <213> Homo sapiens
<400> 37
   aataaaatat aaagaggtga cattgacaga gttgcaaata atagaaactg ttgtttccca      60
   aaggcaaacc cacagttgtc caaagagcac cataagcatg tattttacag catcagataa     120
   gtgtctggct tcctcctttc agctgactga ggcctctctc ctatgaggaa gaagctttga     180
   gtcctggcca ttcagcatct gccttgtaat gagggaaaaa ccaagaacca aaaggaatta     240
   agagactagg gggaaaggaa aacctttttc agcctgactc ccttgctaag tccctcactk     300
   acagtatttt agctacctgg atgtcgcatg atatgtgatc tgacagatct ataatgaaaa     360
   cagagacact agggaacaat gtgattggag gaacgtaatt acggaatgtg gaacagcttt     420
   ccatgggccc tggtgtgtct gcaaggcagt gtctgccatt cctctgggtc attgtcaagg     480
   acagtgatca gccttggaga actgaggtca tgtctccctc ctcagcaaag acaggcattg     540
   ttagaagatt tggcttccct gtgttcaggg ttggtctcct gtaacaaaac ctgctgcat      599
<210> 38
   <211> 599
   <212> DNA
   <213> Homo sapiens
<400> 38
   ggaagactac atttcccagt ctgattgtat ctatgtgggg ctatgctacc agttctggca      60
   aatggactat gtaccagcag cacgatatac cacttcatgc ctagcaccta caatctgcaa     120
   gacagcatct gcattctcct ctctgtctac tgtaggatta tcagtgtcca gcaaaaccag     180
   gacattcacc aacatatttt gtcaagtaag caagaaataa atttctattg tgttcatgta     240
   ctgaaactta agggtttcta ttaaagtggg atgattactt ccgttgacta atacctagcm     300
   caaactacac tcaatcaagt atcaataata agcatattat ttattatacg tttgaaagct     360
   ggaagagaat tattttgttc aaagaaatgt ggatcaagta tcttgaaagc atttatcagt     420
   ttaataacat ttggcatact actttgtgga gaaaaatcac atagggaaca gagtactata     480
   cggtaaatgc aaagtccttc tataatattt tctgttgagc tgacctctgc tttttgatat     540
   gacaaagtca agaacaatct gaaaaaagta tcaaacacat atgtaggtga gggtgttcc      599
<210> 39
   <211> 599
   <212> DNA
   <213> Homo sapiens
<400> 39
   ctgggttgct ggggaagggg gtggaggaga gagtcagtct ctgggtgggc agtgacatcc      60
   tgcgtaacag ggaagctgtg catctggctc tgtggacagc ctgtgagggg cttaaggggt     120
   actcttccta ggtctctgct ggagaaggag ctggagtctg tgggcatccg cctcaacaag     180
   cacaagccta acatctactt caaggtgagg cacctctctg gccttcaggc caggggtgtg     240
   gcagttttga gactgcattg gctggcggct gaggctgtgg gaccattgct gtgaccccty    300
   ggtcagcagt tctccccatc cctttctcct ctttcagccc aagaaaggtg gtggcatctc     360
   ctttaactcg acagtcacgc tgacccagtg ctcggaaaag ctggtgcagc tcatcctgca     420
   cgaatacagt atccttccct gaaagacacg tgaaggaggg cagccaccac cgtcagcgca     480
   gcggggggac tgactaagac aggaggcctc atggagcagg gtggcagcag gtcaccccca    540
   ctgcccctgc tgtccattca agtagcctct gggcaagctc cagactgcca ggtctgcag      599
<210> 40
   <211> 599
   <212> DNA
   <213> Homo sapiens
<400> 40
   ctcataacaa taagaacttg tggtggtagt tcatagaatt aatttctata gtgtttgagt      60
   gaaattctat ctgtaatagg gatagctcta taaaaatttt agtcagtatt tagagaaggt     120
   aatatttttt gagactaagg atttattggc actccatgct tcctctttgt tgctgccagc     180
   atcagacttg ctataactgg aggaagaagg gggtatttac tggatcatct gtaattgatg     240
   taccgtggga tacaaactgt gggttcaaag gaatgacttt ctttttcttt ctttttaacr     300
   agggcaattg actatcaaga gggggaaaaa agtctctaat aatataaatt tttagaaaat     360
   taatttagtg tattatttga tatatatgga gttcttcaaa tagtgcttgc acttgctgag     420
   ggctacttgt gttatctggt attattactg gtatttttag agaaattgac taggagtggc     480
   aaactgtgct agccagctgt gcctacttga aagggttgtt atgaggctca tacttgttaa     540
   tgtacagaca aaggctctct ccttgaccaa actttagaca ggctcctttg agccctctt      599
<210> 41
   <211> 599
   <212> DNA
   <213> Homo sapiens
<400> 41
   gggtggtgct ggggcagtgc tgagggagcc agtttggaag tggcgacctc ctaccctcaa      60
   cttcccttgg tgggagtgcc cgcagctacc caggcgtgcc aggggcaggc ctgggatttt     120
   agcctagtgt ctttggaata tctagttcag ccccagaacc acatctggct tgcctgagcc     180
   aagggaaggg gaacagccgt ggcacatctc tagctgctgg ccctgaatgg cagtttccag     240
   gtccgtggca ctccagcttc tggagacaag ggcagatcgc ctcagagagg cctgctaccr     300
   cccagccctg ccctcagggc tgcagagctg tccctgccct gccttctggc caccaccctg     360
   tcatgatgcc attgcacaga gccacctcca gtgtgaacag gaagattgtg ttggagagag     420
   caccagcctc cagctacttt tgggtctggg gtttgttcag aacagtggct ggtgaggctt     480
   tgggggaggg gagccgagaa ggggactaga gactgggggg tccctggggg aaaagccagc     540
   ttctccctag gtggcagtag gcaggggagt ggagactccc tgagatagca acacctcct      599
<210> 42
   <211> 599
   <212> DNA
   <213> Homo sapiens
<400> 42
   gcctagactt cactactaag catgtaagaa acctgcgctt gtaccctcta aatatgtaaa      60
   aatattttta aagttgcatt ttcaaggaat accactagat gtcactgtgg ttttgcaggt     120
   tgcacaaatt ttatcagtaa agacataaat tccttggtgg cctgcctctt gactttgact     180
   catttctaga ctgggcacat ttagccaatt tcaacacata tagcctttgt gaagcctaca     240
   aattaaataa tggtaaaagc aacgaggaag gaggcagccg gcttaaagct tgtttctgcr     300
   attacaagcc gtgggagccg ggcagttact gctccgtctc tggacttggt ttccacatgt     360
   ggaggagact tggattgcct gctcttagtc tcttcaagct ctcgtagtct atgactttaa     420
   actgatgttt tttcaactaa aattatttca gtgcccctta caagattttt gtccaacgac     480
   attgaaccca ctactgattt ttctgttttg ttttgttttt aaactcagag cttttattta     540
   tatagtactt gtttttaagt tgactcagct ttttaaaatg taaataaaga aaaaaatgt      599
<210> 43
   <211> 599
   <212> DNA
   <213> Homo sapiens
<400> 43
   tccattactg ttattatttg accaggggaa tcatcttagg ctttatggag gagttgacat     60
   tttcaatcag ctttgagggt gggtaggctt tggacagaca gggatggggc agaagaacat    120
   tctggactat gggcacaaca agagctaagg ctaggaggtg ggactgtgta actttgagat    180
   ggagaatagg gaggagacct caaagcagaa ccttaggctc taagggaaac aagacagaag    240
   gattctgctg acaagacagt aaagtagcct gctcatctgg tggtaggcac tgtgtcagcr    300
   ttctaggttg taaatgtagg aagtaagcag atcagaggtt tgctcaacaa cctgcctagt    360
   gagccaaact gcttgctctt gaggccatgt agtccttctg gggcagctct tcaaacccta     420
   gtccacagag gcaggtaccc agttctttca gtgatgcccc aagccagata agaaaatgat    480
   gttatggaga tctggaatcc tcacccaaaa gtccagcttt cattctaaat gcatccgtcc    540
   atcaggacag tcttgtgtgt gttcagaggt gagggatgaa agagaaactt cacagcaga     599
<210> 44
   <211> 599
   <212> DNA
   <213> Homo sapiens
<400> 44
   agtgggctgg gtgtgtaagc aggttctcag acccctgggc agcttgtgtg gtgtgcttta     60
   tggcagtagc agtggtggag caaccaaccc acttagaccc agttagtcca tgttggtgtt    120
   gctggtggct gccatggatt ggttgaaata gttctcagtc ccccagctgc ctgtagcagg    180
   gtagtgggta ttgtcctaag tatgcttagg agagcttggt ccatcttgtt cctcttctgg    240
   ctgggtggca gctgcagctg tgtcaccttc aacttggcct gagggcaggc acagcccagm    300
   attaaactct caaattgatg ccttggcctt gagactagag aggttggggc ccctcccagg    360
   caagcagcat gaacaagaag ctgtggcggg tgcagtctgt tcatgtctca atctcaacag    420
   caggctgcac agagtggtag gcattttctc atggggtacg tgggagtgcc tgatctcgcc     480
   tctccctcct tgaagaagtg caacagcagc agccatgtct gtagatctgt ggtatctagc    540
   ttctcaaaat ggtgtccagc tgaagctgct ctaggcttgg atgtctgtgg aactctgtg     599
<210> 45
   <211> 599
   <212> DNA
   <213> Homo sapiens
<400> 45
   aggcgacttc cggagtgggg ccgcctcatc agtcattgta ccctgctatc ttccagagac     60
   agaagcccca gaagcacaca aaccaccaat cagaagaaaa agaactactg catttcatcg    120
   attgtaagat acacattttt ttatattaca ttttgttatc tctgaaatct gggtgcatct    180
   tacaactgat ggcaggtcag tttatttggc agctgttttc ctttcttaga ggagctaaag    240
   taaaggtgcc tcttcaaatg agtggcatgc cagattcaat gaaatatagg atgacttacr    300
   gagccctaat tatgctgcag gcatcatttt ggccagttag cacacaccat catacccctc   360
   caagagatct gcattacaaa ccacaaaagc ggctcagagg ggttcagaaa acttcccaaa    420
   gtcacacagc ctgtaaacag cagacgcagc attcaaaccc aagtgtgctg gggcccaatg     480
   catctatccc tctcactata ttggcctcat ctcctttcct tctaggtgaa gtgatggggg    540
   acaaggccaa attcagtgtg tcatttcggt cctcaggtct gtgggtcctg gataattct     599
<210> 46
   <211> 599
   <212> DNA
   <213> Homo sapiens
<400> 46
   tatgccagcc gccatgccgg aacggaggga gctgtgtcca gcctggccgc tgccgctgcc      60
   ctgcaggatg gcggggtgac acttgccagt caggtgaggc tggctctacc ctggggggcc     120
   ctggaagggt cctgggcacc tccttgcatg tcctggggtt actgctggcc catgagtggg     180
   tggttgtgaa gggagctgtg tgggcaggac ccctcccgca ggcatggccg cctgacaccc     240
   cgtttcctgc agatgtggat gaatgcagtg ctaggagggg cggctgtccc cagcgctgcr     300
   tcaacaccgc cggcagttac tggtgccagt gttgggaggg gcacagcctg tctgcagacg     360
   gtacactctg tgtgcccaag ggagggcccc ccagggtggc ccccaacccg acaggtaaac     420
   agccctggct gtgcctggcc tggggaggcg ggcaggcagt ggacattgcc gtgtggctgt     480
   taggcatggt ggggggcact ggaatctggg cggaaggcgg tggggactcc ctctccaggg      540
   agggaggatg gggagggagg ataggtgggt tcccgagaac tgggggcagg ttgcccgga      599
<210> 47
   <211> 599
   <212> DNA
   <213> Homo sapiens
<400> 47
   acattgcatt ttttacagat tgaaggtttg tggcaaccct gcattgaaca agtttatttt      60
   tctaatagca tgtactcact ccacatctct gtttctcatt ttggcaactc ttgtaatatt     120
   ttaaactttg ttataattat tatatctatt atggttatct gtgatcagtg gtctttgttg     180
   tcactatttt agttgttttg gagcatcatg aatcatgccc atataagaca atgaacttaa     240
   tggataaatg taggttctaa ctgctctacc aatcagccat tacccaatct tttcccatck     300
   gctggggcct tcctatcccc tgacatacag aaatgttaaa attatgccaa ttaataaact     360
   acaatggccc ctaagtgttt aagtaaatgg aagagttgca agtctctcat tttaaatcaa     420
   aagctagaaa tgattaagca tagtgaggaa ggcatgttga aacccaaggt agactgaaag     480
   ctagtcccct tgcaccaaac agatagccaa gttgtgaatg cagacaaaga gttacttaga     540
   caaactaaaa gtgctactcc agtgaacaca aatgataaga aagcaaaaca gccttattg      599
<210> 48
   <211> 599
   <212> DNA
   <213> Homo sapiens
<400> 48
   ggcattccat gtacccacta ggggaagctt tatcaggctg catttgcctt atgcagaagt      60
   atgcctttgt tctgagaaat ggcctcagaa cacactgttg agctgtctcc tttagtgagt     120
   gtcatcctct catccatttc aaaagattgc agatgccgtc ttgaagtcct gcttgtaaga     180
   aatgcagggg ttgggaatgg aggagatgac gatgtagttt tgcttttagt tttcccaaac     240
   ctgtttacat tttttaaaaa tgtaaccaac ccataattgc attttacttg tcgtggttcr     300
   atctgattgt attgtcgaag gactattttt gaacctgtcc aaataaactc tgcaaagtat     360
   ttccacaagg atgtgactcc atggcgcttg gacagccttt atcctgaatg gtactgagac     420
   acaaatgttt tcctgttgcg tgctttctaa ttacctaaat gcaaacgggg tggaagggag     480
   aaagtggaaa ttctaagtac caaaaggaat gttaaattta gaataataag ctcatctgga     540
   agctatcacc acttttttct taaagtgcgc tgcaagcatc gcttcccctt ggacaccaa      599
<210> 49
   <211> 599
   <212> DNA
   <213> Homo sapiens
<400> 49
   cggttgtttt aaataaatgg aaccttttgg ggagacaggg agggtggatg tgtcttctga      60
   ttttatgagt ctcttttgtt tctagagaat ctttcgtttc tgttgcttgc tttttctttt     120
   cctcctttca ccatcaagca tccaggggag gtgaccctcc aagttacctc cctccctccg     180
   aagtggtgcc ctcccaagac tttggtcctg gcaacttttc agccccttct ttcatttcct     240
   ctgtagccaa ggatcagact tgttctcagt atttccacac ttagggtggt acactcttty     300
   tgggggtgat tttcatctgt tccccaacca ggctctgctg cctgactctc ttttctggaa     360
   aggtttctgc tgctttcaga cgttcatttt tctccctatg cattaattga agtttagagt     420
   atatttcgtg tcctggctat gctacaggca gggctggctt cataggcatg ccaccaacgc     480
   ggctgcacag ggccctattc tcagaagggc ctctcacctg gggtttaatg cttggcagtc     540
   actgtcttaa aatccttaat aatttagtat ttgaatttgt gttttgtaag tgaagtctg      599
<210> 50
   <211> 599
   <212> DNA
   <213> Homo sapiens
<400> 50
   gaggatttca catttagctg atgaatatgt tagtgaattg aagaaatcct caaaaacttt      60
   agcaagtgga acattcctga cgtagtgcgt taactggagt aacctaatat aataaccata     120
   aggaaaaaac aagtcatgct ccccagagac tatctatagt catagaaaga agatgtggaa     180
   ctttaagtaa ataactaatt ggtatgatcc aaagtgttgt cttgactact agtggggtct     240
   ctatttggaa atatgaactg tttttgcttt gaggtttttc acacaaagaa taatcctacr     300
   ccagtgaaat ttccgagtgt ttagtaaatg acagagtctt ggaaattggg attagaacca     360
   agcaatttca aggcccatct gcaagacaaa gacaagcagg taataaaacg ccctgctctg     420
   tttcctcttt ggcctctttt agtttgtgtg aacttttaac ccaagaacat taagtttaaa     480
   atcaaattca atttgaagca attttgagcc acacaactgg gtgaggataa atggctctat     540
   attaaaatga tcttttccat tagaaatgac ttcttggata tcatttggtc tcattggtt      599
<210> 51
   <211> 599
   <212> DNA
   <213> Homo sapiens
<400> 51
   ccaaaattat aaaactctct gggccaagac tccaaagcat ctaaagaaaa cagaagaacc      60
   cagaaagtcc taataataca tgaagggtaa atatagcatg aagggtatgg tttttaaatt     120
   tcatatcctc attagtgaat cagcagaatg actgagacat aaccctagat tttcctatga     180
   cttttggtaa aattttatgt taccaatttt attcagactg caccagtgtg gtctacatgg     240
   taagacaaac agcttcagag tggaactctt aaggcactgg gtgattcaca aattgaaccr     300
   agtcagatgc acagtagttg tatattaagt acatgcacaa atgacactaa tggacattgc     360
   tgccatggca cgtctcagtc atctgccctg gtgctgttct ttttgcaata tcagaattag     420
   taacataaaa cagagggcct ggataccaaa ttcatgagtg acatgttgtt cggagggaaa     480
   gcaagtaggt tgaaatgcca tgttagtcaa tgccatcatc cttatctcgt gtaccatata     540
   atacgtacac ataagccatg ctgccagtag tcccttccct ctctgttcca ggccttgga      599
<210> 52
   <211> 599
   <212> DNA
   <213> Homo sapiens
<400> 52
   tgaatggcac ggccaccctc tgtgcaccaa aggtttcaca ccttcacctg agagctttaa    60
   taattctccc atcttagcca agaaaactct gccacataac ctcaggcctt attaagtcta   120
   tgaacagaaa ggtaactgga gaactgcaat aattatctgg ttaatgagtt aaaaatcact   180
   cttgcctgaa ataacctggt aataagtaag tcttgctatc atattcatta aaaaagaaca   240
   tttctcagag agccaaaagg aatgaggatg gtgttcaaat cagtttacaa gtcagcatty   300
   ggggcactgc atacaacttt ccaaagtaaa aataaaggta caatgtatag agaaaagcca   360
   ccaagtaaag ttgaaggcac ctcttaattt caaaatgagg atatctagca ttcattaaga   420
   ggtcaccatg tgccaggaat gaggcttata gttttatgca gatcatttta ccattcctta   480
   aaacacctaa atgtggcagt tactattttc atccccattt ttcagatgag aaagctgggg   540
   ctctgaggag ttgcatgact tcctcaaggg tatcctgctg ctaagtggtg gagctagct    599
<210> 53
   <211> 599
   <212> DNA
   <213> Homo sapiens
<400> 53
   tgtaactttg ccagagaggg gctatctgag ggcttatgta gttcagctgg acttctgaat    60
   atctgcacgg ctcacaaagg aaccattctg agacagcttg cccatctgtg tgcattttct   120
   ctgaaaacaa aaggatcaag agatgaaaga ggacaggatg gaggaaaaga gaaggctcag   180
   aggtgagaaa gaaagacatg gagacaggac agacagagta tcccacactc tggaactcct   240
   cctggtatga tattgaatgg aattgctcct ggctttacct ggtgaatgat atccttcccr   300
   agcccatgaa taatctgaca gggatcaaaa ttgcctctga gtagcagagg ccccagatta   360
   ctcacacaac attcaggtat gcaaaggaaa ttagctactt tatttcttta tacttgatct   420
   aattttttaa tgagccttcc acctgtttgg gtaggaagaa aaaaaaacaa ctatatttag   480
   tccaatttag tttaaagtta gtttggccca attatttatg cagaaactct gtattaacta   540
   gtttttctgc tcagttcttc acagctatct tcttcctttc ttgcttctct ctgggtccc    599
<210> 54
   <211> 599
   <212> DNA
   <213> Homo sapiens
<400> 54
   ggtgagatga cctataggag caagtggagg taggctagga ggctaagggc tttctctcca    60
   gtggaaaggc ttttagttga atgttttagt ttttcagttc tcagtcacat cttctgtttt   120
   tatggtatct gagctcacca ctggaatcac tgcttcagtt tccgctgaac agtggccttt   180
   ggtctgaagg tagaggaaag catacaagaa ggaacagtgg ttttgctgca tgggatggag   240
   gaggtctaac tgctttgaat agacttttac tatctttctc ctttaagctc cacaattcar   300
   tgctctcata tccaacttct gtgtattgta ttatcccagt cttgagcctg tgcattctct   360
   tgcagcaaat taaattgttc ctcctgtttc ccttggagcc ccaccttttc tgccagtctg   420
   tgtgttgcat aatccctcct gtgctggtta ctagttacat aactcatgtg actcaactcg   480
   cagccaatta gcataaacct aggtcttatg ttcaaattga tagtcaaatt caagggacac   540
   ataagcttta tttggtatga catttgttca gctttaagga aaatataata gaagcgata    599
<210> 55
   <211> 599
   <212> DNA
   <213> Homo sapiens
<400> 55
   agccagcata ttatcttctc cccattaaag agctttaggc catctgtccc atctagacta 60
   ccagctggta tgatctccag aaatattcct caactgtagg aactaattgg gtttttccat 120
   atgtataaat tgtagattaa ctgtatttac caataaaaaa agttcaattt catctttgtg 180
   gcaggtgacc tgatcttaat aatgagggtg aatctcacag gcaggtcctt tgggatggtt 240
   gtaatgtttc tactatcaaa gctgttgctt gtgacaactc tgggacattt tttcttatay 300
   aaaatcaaag ccagagttat gaactcattt aaaagagcaa ctgtaacaga agaaaatgta 360
   ggtttagaag aagtgataat gacatcacca gaagaaggga cactgcaaaa atgtacccta 420
   acatagtgtt cataatgctg ttatacaaaa catgatggct ctccaagata aaattaacca 480
   tttccagata ggattaaaca taactctgaa tatctatgga catgtgatta aacattatga 540
   attcaacaac caagctataa tataactcat ctttgtaagg gagacactta aaaagaaga 599
<210> 56
   <211> 599
   <212> DNA
   <213> Homo sapiens
<400> 56
   taaaatcaat agtgttggtt gtgacaatta tgatgtactt ataaactttt ctttaaaaca 60
   tttgtgaatc tgtacacttc tcaacaggac acactgaaac tgcctgtttt atgttcccta 120
   tacagtagca atgctcagga gtagtctgct ttaattaggc ctcaaggagt tgagaccgcc 180
   tgccctgaac cccaatgagc ctgtggagca caccacagac tttctagtgc tggtttgtgt 240
   gagcaagtgc ttacctcagt aaggccttta atcttcaagt acccacaaag gtaagagtty 300
   cccgtgtcca cgtgctgaaa agaaaggata cacagcctgt cacaaataga taaacttact 360
   aggggacatg caggagggta ctctgcaaat cctgtgaggc cagactggtg gtcagtgaac 420
   acccagggca gtccaaggct cacccagtct ctggaatctt gataagttct ccccacacac 480
   atgcctgttc tgccaaagca aactgatccc aagaccctct caactttctt cagctgaata 540
   tcatgagact gaagtgtctt tctgaaatgg ggtgcccact caaattcatt ttctgtact 599
<210> 57
   <211> 599
   <212> DNA
   <213> Homo sapiens
<400> 57
   accttagtct gccaccctcc ttttaaagaa gtgataagat gggatgaatt tggttcaccc 60
   agcctcatac tgtgtttcat ataaaaacac atatccaaaa aaatatatat atatttttat 120
   atatatattt atttaaatat atatttaaat atgcatatat atttttatat atttaaatgt 180
   gtatatatat ttaaatatac atatttaaat gtgtgtgtgt gtgtgtgtgt gtgtgtgtgt 240
   gtgtgtgtgt atatatttac agcttttttc tcttatttct aggagtcctc gccctacctr 300
   ctgttgcaat tggcatgttt tcaggaggat atatcattaa aaaattcaaa ttgtctttag 360
   ttggacttgc caaattggca ttttgttctg caacagtgca tctcttatct caagttttat 420
   atttctttct aatctgtgaa agcaaatcag ttgccggcct aaccttgacc tatgatgggt 480
   ttgtatatat aattggataa tatgttaacc atccaatcaa aagactatgt gtaaggaaaa 540
   tagggtagaa aacaattgta attaaatttt cttttcactg agagaaattt catttttga 599
<210> 58
   <211> 599
   <212> DNA
   <213> Homo sapiens
<400> 58
   gtacacatgc ccatgttgaa tagagacagg caagagttat gctttgtgct cttggcagag     60
   cagtggagcc acttcctgat tcctaaactg gatgaaagag tgtggtcctg gcctgcagtg    120
   catggccact gtcctcaaac actgctttat ctcctgtgca cgaggcctgc ccagctctca    180
   cacatgtaat ctagacttct tgtgtgctga gcactttccc ctggtaccat aattaatgcc    240
   atctcttcca ggacttccct gaagggattg gtctgatctt ttgggatccc catagcatty    300
   acaggggttc ctaacatagc gttcatgtcg aagaagagta taatggtgga gagacttgaa    360
   gggctgtagg tttttgggga taggtatgtg aacattttca gtctctatag tatttaaatg    420
   cacaattgaa acttggcctg ctaaaaactt tctaaagctc caaacttaac cttatgctcc    480
   aggtccaaag gggtccccag agtcaatccc tccccctgcc tgggccatat tctccagctc    540
   agagaggatc cttgaaggta aggagatgag gaagcaaagc ccacccatcc tatcaatgg     599
<210> 59
   <211> 599
   <212> DNA
   <213> Homo sapiens
<400> 59
   aatattaacc atcacattcc ccttggacta tggaggccat cctacactac acagttgaaa     60
   atcttcctat tcaaccactt gtttcccttt ggttgttcta aacccttcaa gaaaatctta    120
   ttcaacttca cgtggcatag agaatgaagg gctggggctg agaaggcagg gcgctcaggc    180
   cactgtcatc ctctgctctg ctggggtacc caagtgagga catggattct ggagaggcgg    240
   aggaggaagg ggtagccaca ggggtgtggg ggcagcccct ggagggtgac tggatttcay    300
   gtttggagtg gaacccactt aagatgtctt cagtcgactc ataagggatc ccaacaagtc    360
   aattagcagc tgtaaataat ttacaatgct gagcaagctg ctgaaatggg cagaggagtc    420
   atcagtttga aatcttggag agggagacgt gctgatacat ggttggcaga aaagtcactg    480
   aagtgcatct taacaaatgg acctcacttc tggcgatgtg ataatgagct tagtggataa    540
   caggagtgaa gtgaactagg gtacctttct gtctctttta agcacttaag cctctccat     599
<210> 60
   <211> 599
   <212> DNA
   <213> Homo sapiens
<400> 60
   cacacatcac accagggact tctactcata cgtgaaaagc tagagaatca tgtttaggta     60
   ggcaagggga gagaggtcct ggacaggtta ttgaaggact gattttaaat tacaaaatac    120
   ctctttatgt ttagtttgca aacaaaagga gtcaattagt gaaggcttgg ctgagctgat    180
   ttcagaaaca gtagggaccc actttttgca gtagttagga agcctcagga tctttttaaa    240
   tgtatcctaa ttactagcct aggaaagtct taaaagagct atagtttgtt gggagaaagr    300
   gatttgcctt ctttagaaaa atctatcttt ggtaatgcac ttggttttga caaaaccaag    360
   tgcattggtt ttgttacttt cctttaaaaa ttctatcttt ttaaaacatt cctggaactc    420
   atctgttctt tttttttttt tttttttttt tgagatgaag ttttgctctt gttgcccagg    480
   atggagtgca atggtgcaat ctcagctcac tgcaacctcc gcctcctggg ttcaagtgat    540
   tctcctgcct catccttcct agtagctggg attacaggtg cccaccacca cacccagct     599
<210> 61
   <211> 599
   <212> DNA
   <213> Homo sapiens
<400> 61
   atcagagtga acaggcaacc tacagaatgg gataaacttt ttgcaatcta cccatctgac 60
   aaagatctaa tatccagaat cttcagaact taatcaaatt tacaagaaaa aaacaaccct 120
   attgaaaagt gggcaaagga tatgaacaga cacttctcga aagaggacat ttatgcagcc 180
   aacaaatata tgaagaaaag ctcaacatca ctgatcatta gagaaatgca aatcaaaacc 240
   acaatgagat accatctcat gctagtcaga atggcaattc ttaaaaagtc aagaaacaay 300
   ggagactggc gaggctgtga agaattaaga acaattttac actgttggtg ggaatgtaaa 360
   ttagttcaat cattgtggaa gacagtgctg tgattcctca aggatctaga accagaaata 420
   ccatttgacc cagcaatccc attactgggt atatacctaa aggaatgtaa atcattctac 480
   tataaagaca catgcacaca tatgtttatt gcagcactat ttacaatagc aaagagatgg 540
   aaccaaccaa aatgcccatc aatgatagac tggataaaga aaatgtggca catatacac 599
<210> 62
   <211> 599
   <212> DNA
   <213> Homo sapiens
<400> 62
   cttagtttta ctaaggcctt tactgcttct tttactccct ttctcctgaa agtaatatct 60
   caataaatca ttcattgaag aatttccatc tcaggccctg cttctaggac actggtactg 120
   ggagcagtcc taagaaagaa accctatagc tggaattctg gagttgaatt atttactggc 180
   cagacggtag tgcatcctgg gttggtggag ccatgatagt tcctggctaa gatgttcacc 240
   catggtgaac tggcaggggg tgcacatgaa aggagggaca ctgactgatc aaatatctcy 300
   ggcatttcaa aggcatatgg aaaagggtaa ctctatgggc catggaattg ttttgctttt 360
   gctaaatgtt gtagatctag agatcatgat aatctcagat ctattaattc acagtttgat 420
   gtaaatatga gtcagagggc ctccttagga gcatttaaag aaatcctaat ttctgtaaat 480
   ggagggcaga gaaaactgat ggcttgaaac agaacttaat tggaagaggc agaaaatttt 540
   ggagaaactt gaattctcaa cctagggaag tttcctaagc caaagtcaga gttctgata 599
<210> 63
   <211> 599
   <212> DNA
   <213> Homo sapiens
<400> 63
   gcctgcttgc tgttacctag caggaaggtt agagatcaaa tggaactgtt gctatgaaaa 60
   tgtgttgtta cctgtaacag acatgtatgt gtaatacgtg tttctttagg gccagatagc 120
   aagtgacggg ccaaaatgca ataggtatct tttgagcatc atcacatcaa gtctggagtt 180
   tgttcctcag acacagcagt atcccaggca ttcctttatg tagtggtcag tgcagaagcc 240
   tgagactcac aagcaggcac aggggacatg tggcaacaac tcattctcaa accgtaaaar 300
   tattgaaatt tgacccagga aatccaagtc aattatgtgg ggaactcata tagagcaaga 360
   gccattctct tttcacttgc aaaattctca gcaaaataac tgatctttct gcccacttaa 420
   ggacatgagt aggaggttaa acaagcacag tttgcaggtg gggctcatac tgaaggtcag 480
   cttgagagtc gtggaaaatt ccgtggtgga acacaaggcc actgaaagat catttcgtca 540
   gttaccagca gggtggagtg agactggggt ttcaccaaga cattcactca ctagctgtg 599
<210> 64
   <211> 599
   <212> DNA
   <213> Homo sapiens
<400> 64
   aaaattttca tcttataggt gattaaactg aggttcagag agctgaagtg gcttgcccaa      60
   gatcacttgg ctattaagtg gcagagctgg atcttgatga catgtcttct gatggctagt     120
   attattctct ctaactgata aaaacgtgtt tctgagttga ccaccctgac catatgctct     180
   agtaactctg cgttctgctt tcaacttcta aagggcattg gggaaaggca gaaatgccac     240
   gtgaactgat acttctctag attattaata agatgctcca tacacttttt ggcatcatay     300
   aataagtgac aaaactttca gaatttctgc aagtacctaa tgtagaacca tggagaattt     360
   tagtttatgg aagttagtct gcttgtcctc tcttttatgg tatcattctg cagggccagt     420
   taaagtggat ttgggcctca gtggaaaaaa ttgagattct ctcaacaaga cagagacttt     480
   gtgctgaaat gcaaattgtc aatcaatacc actttatcag gtttctgggt ttcccccaaa     540
   gtgtgagttc cgtaccaata tacaacatca acatgcaggt atcatcaagt cctgataat      599
<210> 65
   <211> 599
   <212> DNA
   <213> Homo sapiens
<400> 65
   gttattcctg agagggctat gttcctgaga gaacaaaatt attgtttttg aaactctaga      60
   gagaactgct ctggcaaaag aaatgtatct tttcatctac agtcattctg aggtgaaaga     120
   tctcatgatc actctggact atacaaccca caagcagact tcaaggatac ctacaggaac     180
   cccagaagtc ctgattgatc acacaggccc taaagaccct atcaacttgt ttatcaactt     240
   gcaattctaa tttcctgaga gtaaaacacc agtaaaaaat aatctcctcc ctcaatatty     300
   tagagaatgc aaagaaatac acagtcactg tagacctgtg agcttttgcc aagtttggtt     360
   ttcattatta tacatatgaa gagaatcatc ttcgccaaaa tctataaagg ggctgattaa     420
   ctcagctctg tagtaattgt ttcatattaa atcagtccaa aagtttaggg catgtttctc     480
   aggaactctt tatctagcat caggtatgta tctaaacagt atagtatctg ttaattttga     540
   agttatataa aaaaagaaaa tgtgcatttt cacacgtctt agtagaagct ctttacagt      599
<210> 66
   <211> 599
   <212> DNA
   <213> Homo sapiens
<400> 66
   aagctcctcc tcctgggttc gtgccattct cctgcctcag cttcccaagt agctgggact      60
   acaggcgccc accaccacgc ctggctaatt tttgtatttt taatagagac ggggtttcac     120
   tgtgttagcc aggatgatct cgatctcctg acctcgtgtt cggcccacct cggcctccca     180
   aagtgctggg attacaggcg tgagccaccg cgcccagcct gaataaaggc tttttgacaa     240
   tggtccaggc agccctaggt tttagatatg tttttctaca gttatagaag ggcactcttr     300
   tcctggcatt gatgagaaca gtatcacatg tgttggcctt atgtatttaa gttttaggta     360
   agattgtatt tgaaaagtga gctctagtgc aaataaaaac tttgaaaact attaaatgaa     420
   atgagcctta aggagtcttt taacttttta agaaatatta cgttttacag cacctcttct     480
   ttagtttcac ccatggaagc accagatgaa gaaccttccc agagaacaat gtgtgtgaca     540
   tgtccatcca ttctgccttc tcctaagagt ttggacagct aatccgttca ccgtctaaa      599
<210> 67
   <211> 599
   <212> DNA
   <213> Homo sapiens
<400> 67
   cacggctttc tcccctaaca gccactccac ccaggggcca gaaactgact tcacctctgc      60
   actgaattcc tccccccatg tctccctcct cccacccatc ttcctcctgg tgcttccagc     120
   agagccaaga aggcttcgcc ccacctactc ccctcctgct gacacggacc ccccacacac     180
   acacccaagg aacaggaata ccaaaaatga tactaccaga gtgaagcgtg tcctgcagac     240
   attatctctt taatcctaac tataatagtc cctatttcag agatgaggca aatgtgagar     300
   aacaagtaac tttcccaaga acacacaacc aagaaaaaag aaagccaaga ttcaagtgca     360
   agtctgcctg ggggtctgga gccggctccc ctgctgcccc ctgccatccc tcccttccac     420
   tcccacagcc ccattttcca cccagaagcc ctcctggaag caaccctcac ccccgggccc     480
   cgttattcta agtccagttg ttcacaattc attctacaag ccctcatgta atggaatgct     540
   aaaatagaac tttgtcacca gctccttttt tatgttggtc tcaaagctgg ggacaaagg      599
<210> 68
   <211> 599
   <212> DNA
   <213> Homo sapiens
<400> 68
   tatttttcta ttattaacat aatgaactta tttaagttaa agtcttacat agcataatac      60
   agttgaatca tatttttaaa tcaaaacttg caatctctgc ttttctgatt ggattattta     120
   atctattcac ttttaatttt cttattttgg gagttagatt tatgtcttgg actttacttt     180
   taaattttta tgtctcatat gttatatttt ttgttcttct atttctactt tactgctttc     240
   atttgcacta ggagaatata ttttaatgtg gcgttttaat tcctttaatg tgtttaaagk     300
   tatatttctt atgacttctt ttctggttgt tatagagctt accatagaag tcctagactc     360
   atactcatct aacttaattc ttgtctgata tagatatatt attcatataa agttatattt     420
   tactatcctt ccatttgttc cattattttt atacacatta catctttaaa tgttattaag     480
   ccaaaaacgt attattataa taattacttc atagaagttt gtcttttaaa gaagctgaca     540
   ttttttaaaa aatttaaata agtatgtatt tatagctttt gtaatattaa ctttcttac       599
<210> 69
   <211> 599
   <212> DNA
   <213> Homo sapiens
<400> 69
   agaagcatta ctccaagagt ctagtgttgg ctctcttctt cttgtggtcc ccatgacctc      60
   agcactctat gcagacaaat atatgaatat atctacagct ctagttagga aatgggcaga     120
   agtaactcaa ccatgtcccc aggaccaaga gcagtctctg gcatgtagtg ggtactcagt     180
   aaatagtagg gatttcagat gaagagaaca ttgtgaacag aggctgtgca tgtatgtatt     240
   gatttatttg gaggataccc ctttcaggct gctgaactcc tcaaactcag gaactgtgcy     300
   ttttcctcat ttttaatttc ctgatccagc ataatacata gtagctgttc aatacatgtt     360
   tgctgaatga atgagtgagt ccaaggtcga tgataaattg atgcaaaact gtggcagtcc     420
   ttatatagga atttaggctt tactgtatgt gcagtaggct tgcagccatg caaggctttt     480
   tctcatttga gtaactcaat tgaagctgag ctccaggatg actcatgtgg ctgccgtcag     540
   cattgaaacc ttgtgccgtc ttcctccctt gcccttgctg ttccatctct aatcagtag      599
<210> 70
   <211> 599
   <212> DNA
   <213> Homo sapiens
<400> 70
   aaataaccag ttagccctgg ctgaatgtcc gttgtctaca gggccacctg gagccagcag     60
   tagccagtcg ccttgccaac ttttgttctc tctcagcagg aagaaaagag acaaagcagc     120
   tactccttga cttttcttgg ttcaaaaaga atacttgaat tataacagaa atgggagagt     180
   gagagtgaat ttcaatattt tcactgtgaa agacaaaata cacacacaat taaagagatt     240
   attttgttca gctcttgcat tagggggaga atgttcatta atgaggaacg tgtcaaagay     300
   gggagagaga gaacctagag ttttataaaa caagagagcg gttgaagaag ggtagaaata     360
   ggtcacacct gggaatatac tagatcatcg caggggtctt agttatcaac ctagccgttt     420
   ctcagaacgg aaaagcaggg cgtgaatttc tcgaccatcc ttgctttcta ggagcacagg     480
   actcagataa agtttaacat ttctattgaa ggacatcaat atatttaact ccaaagtata     540
   ttttttgaca tattttaaga tggctgccgc agggccagca gaccaaaatg gtcctgcaa      599
<210> 71
   <211> 599
   <212> DNA
   <213> Homo sapiens
<400> 71
   acattgattt ttcttcctat tcttttcctt cagaagctat agtgatgggg gctccaggag     60
   tttttaatgt gattccagat taattctcta gcagtggacc gggaaggtag aattgatcaa     120
   attctaggac ccagtctcct gggcaaatcg aagcacaaat tagaataaca aaacaaaaac     180
   tttatgtgtg tgtgagtacg tgtgcatgtg cgtgtgtgtg tatgaaagta atgaaagccc     240
   aaagccagga cctcattttt tcagttccct tttgcaggga gaacttacct atgggtaccr     300
   ttttggagga caggaatcaa ggcatatact gaaccctgtt ctctttgtga gtgaccctgt     360
   tgccatttgg gacatctgaa acagggcatg ctttcaaatg cagcatagtg acaatccttt     420
   tacacgttgc tcaatagcaa aattggggtt aagaaagtga ctgcgcatga acaatttttt     480
   tttttttttg agatggagtc tcactctgtc acccaagctg gagtgcagtg gcgtgatctc     540
   ggctcactgc aacctccaac tcctggggtg tgaacacatt cttcttgtgg cctccctcc      599
<210> 72
   <211> 599
   <212> DNA
   <213> Homo sapiens
<400> 72
   gtggtctgag tgggcttctt actgtttact tcatacattg agcaaactcg ctggatccca     60
   ggcagggttt gggatgactg caggctgctc ccacagggca ggtctactcg gatgtgcatt     120
   ctgcacggac aatgcagggg aagaaggtgg attcatgccc ggctctcctc ccccatgctc     180
   tgccttttct tctattcata gtacagtcta ttgctttctg gctcttataa ttgtcagtta     240
   tttcatgcag cttgaagggc agcagtgcct aagacagagc caaagagatg tgctgctgay     300
   gagcataact tttagtgaat ttaccagtcc tgaagagaaa catggaacaa tgctttagac     360
   tgtcattgcc tcaccaaaca tgtttgcatg tttgcaaata gcatcaaact tagagaagag     420
   ctagtgggta gactttctct ttttaaaaaa aatttttttg agacagtctc gctctttcac     480
   ccaggcttga gtgcagtagc acaatcttgg ttcactgcaa gttctacctc ctgggctcaa     540
   accaccctcc tacctaagcc tcccagggag ctgggactac agacacgtgc caccatgcc      599
<210> 73
   <211> 599
   <212> DNA
   <213> Homo sapiens
<400> 73
   tagcaggcct gcatctggcc agaactgctg tgggaccttg gaagattgcc tggtacctct      60
   gggttggccc accgtacata tcctcttgct tcatgatggg agtcagggtg ccccagaaga     120
   ggcagcgaaa ggttggagct cactctgccc ctttgctcgg tcagtggaga gagcggctct     180
   ggcaaaactg aggccaccaa gctgattctg cgctacctgg ccgccatgaa ccagaaacgg     240
   gaggtcatgc agcaggtgag tctacctgtc tccccaggac cctaggctga acaccctttr     300
   ataagcacac ctcatgtact cccgaagagg caaggctcct tggcccacca ggaagatgag     360
   gaggctgagt tccagaaggg ggccttgccc agggctgctt tttctgccac ccttcccagg     420
   ggtgtgcctg aggggtgaga gttcctgcct gtctgtgttt ctgtggcagc tcctgcttcc     480
   agccctccgg gtccagttca gagtggagac cacataggga aggggagtcc acatagggaa     540
   ggggagtcat cccatctctc ccacctggcc tcaccttcct ggtcatggcc ctggggcca      599
<210> 74
   <211> 599
   <212> DNA
   <213> Homo sapiens
<400> 74
   tgcatacata atgcatataa cagcacacaa actagattca gccatgcaaa tcatcttcaa      60
   cctgcccatc tataagtttc tgaaagccaa aagataaagc atttcaatat tttacatatt     120
   ccaccttatt ctataaagat acaaatactt cccctgtgtc tgccttctgc aggactcacc     180
   agctctgtat ccttgagcca aggctttctg ctttccaacc tttagcatct ccttgttgat     240
   gtcacacacc acgacacgag acccgcccaa ggaatcttct tcattctggt actctttggy     300
   aatttcttcc caggataaat tttgttgggc ccttaactgc ctcttctgtt ttctctgatg     360
   ctgggactga acataattaa ggaaccggaa tgcaatgtca cctgtgggaa gccaacatga     420
   ggaaagatgc agactaaaac aaaaataaaa aaggaagaaa cagcatttcc taaaacccaa     480
   aggggtaaca gaggcacagg agtacctgtg ttagctaact ggatgcagtg tggctgaaag     540
   ccaagtcctt tcttcttttt gttagagaca gggactcact ccatcaccct agctggagt      599
<210> 75
   <211> 599
   <212> DNA
   <213> Homo sapiens
<400> 75
   ggagagcaaa tgttcagatt tctttttctt tgacatgttc tttttatttt tcttccattt      60
   agttttgaga tgtgggaagg ttttggttga ggaatcataa aactcctctg gcatctattt     120
   tatgaaatat gtagcatatt ctattggaaa gaaattgcaa gcttgctttt attattatta     180
   ttttatttta tttgaagctg acatataacc aatctgcaat gccagtgggg gagtctcata     240
   aacagaaagg aaaatataaa taaaattctg ctgaatagaa gagatgacag ctaaaatgay     300
   gaaaattttg tggtggtgaa ggcgggctgt ttaacttatg gtataaaaat aaggggatgg     360
   gtcctcccaa atagttgtaa gtagttcagc atgaacccaa atcggccctc ttgaccacct     420
   gtagtgagtc ttgcaaatta ccaaccccat gcatctgctc cgagcttcat attgatttac     480
   aattttcttt gttggtcagt ctctgcatct cttgaatgga ctcatgattt gtttacttgg     540
   tatccatcaa ttcaggtatc tctttctttg ctctttctct gtctctgtct ttcctcgtt      599
<210> 76
   <211> 599
   <212> DNA
   <213> Homo sapiens
<400> 76
   gggaatagac gatataaggt cctgattaca gtctaaaatt ctcttggtga tcccagattc      60
   agtttccttg ctgatgggtt tatacctgga ctaatcagtc agccagcctg gcatggctaa     120
   agtccagagg ggcatacaac ccccaggtct ctgtggcagt catgggaaaa tgccacggag     180
   attgtgctta aagggaggaa ctgctgggag aatttgataa cctggaattc tccagtggct     240
   acaccttcaa ggtgtgctgt agagtttaca ctgtcctcac atatatgttg ggctgctctm     300
   agccaatggc taagcaagca gggaaattgg agctggacca ttcctgacaa atgaaagact     360
   tagtcaatgg gcagtctttg ctttgagggt ccccattagc ctggttgaaa ttctctgaga     420
   actgcactct aatcagaggc tctttctatg cagccctcat tacacctctc tctactttca     480
   aaggggtcaa ccttgcattg tggtctgaag tctccacacc tacctctgct ttctatcgtt     540
   tgttcacttg catttcctcc aacaactgtc ctatatatct tgttccttct tgataactg      599
<210> 77
   <211> 599
   <212> DNA
   <213> Homo sapiens
<400> 77
   gaataggaca tggaaccaga agttttcagg caggaagtaa caaagtgagg cttgtttatt      60
   caaaagataa atcagacatg gaagttggac tgaagcaggt gagagactcg tagcaggaag     120
   accagatgct taacctcctt ggatggattc tgttttaact gacaggaaga cagttggatt     180
   tcctaaagga gaggcttagc ttgtgggagg aagtgcagtg gaaaggatat gaacttctca     240
   tcatcctggt tcagcatccc aggtgcgcac ctgagcatgt gagtccccct ttgcccaacy     300
   tccagtctca ggcgcctcac ccaccacagg aggggatgtc cccagcctca ccgggctgcg     360
   gcgaggcggt gcagtatctg tggacagctt ctggctgatg cctgccacat gggtgggact     420
   cggggtagct gagtttcctt ctccttctct tctaacttct tatccaagga aaaggaaaag     480
   acctgtctgg ccttccataa tcagtaaggt aaattcatcc atccacagag aaaataaccg     540
   ggaagcctac agtgcacatt ttctctctgc ccccaccccc tatgcccatg ccgcatcac      599
<210> 78
   <211> 599
   <212> DNA
   <213> Homo sapiens
<400> 78
   agtgcccctc atcccacagg gtcatgaaga agagccctag ctgaaactct tctccccagt      60
   gtccctctcc ttctaatcct tctccattct tctcctgagt ctagccttct gctgaagagc     120
   ccttaacaga gatcactacc ctatgatcct gcacatccac atctgcgact ttaatgttcc     180
   ttgaaaaggg tttacatagg attgaatgga gggagggtta ctattgtttt tacttcttct     240
   ctaagccatg tctcctctct cacacaccag attttagtgg cttgacttct gtgaacacar     300
   ttttctgtca gacacttatt ttgttgattt agttctgttg gtaaacattg gagcacagtc     360
   ttacagtcat ccccccgcag ctagaactgt gagctactag gagctaaaaa taagagggtc     420
   tcctaggcat ttaaacaaat attgccgttc aacagagtct ctttctttat tctaagaatt     480
   aaaaaataag gtccggacat tttttttcac atagttaagt ataatctagc taaaaatagt      540
   gccatgtttt aatatctgta acctagaatt cttatccaag caaaaacatt ttcacttct      599
<210> 79
   <211> 599
   <212> DNA
   <213> Homo sapiens
<400> 79
   gatgggtctc acgctgggct ctgacctttc ttcttcccaa accacccacg agctgttgga      60
   agttggggct gtgtctctgt catgcctgta ctcctctggt gcctactggg tgcccagtga     120
   gtgatcaatg gatgcaagaa gcagcaaata ctgttggctg catctgatat gtggttatca     180
   aatataaggt ttcaaatgaa ccttttaggc ctaagagtct tttctcaaca aatgattctt     240
   gaggagctat tttcacattg ttcaaaaggt ttatgtctgc tcaccactca acaggcaacr     300
   tctgacattt taactaactg cttcttgtgg actcatccct ggtgagcacc caggtaccta     360
   ccacataaac agcttgcaag aggtacttga tgaatgaatg aatccatcaa taaacaaagg     420
   ggctacattg taacaggtgg cctgccagta tgtctctttc cttagactgt gagttccagg     480
   agggcaggaa gattttcgca tttatttttt attccctgga gcaggcagga aggtccccgc     540
   atatttcttg aatgaacaaa gggatgaatg aagacacact cgtacaaata gatgcattg      599
<210> 80
   <211> 599
   <212> DNA
   <213> Homo sapiens
<400> 80
   ttgacttctg ttcaaatgga gctcaaagct tatctcccgt gtccataaac aaggctgttc      60
   aaacaggtta ggccacctgg ggtcggaagg tattttaaag tattttaggc atgttcaaga     120
   cattatacgg ggcccctgac agccatcaga tatgcagaaa cttatgtaca aacacatcaa     180
   gtgagacttc actggataaa aagatttcca aagccatctg gagttggaag gtattttaaa     240
   gtcggccaca gcatcattgc aggctggccc cctgagcttg tatttctgct ttgttccacr     300
   cgagcttcct acaaccttat ccattcattt caaaatattg aactttgtat tttttctaaa     360
   attttaaggc tttataacag agtttcagga cgtctagtct gccatattcc tagaagcaga      420
   actctcactt tattttataa tacactggag tcttgcaacg tgtctttcgc agtggaggcc     480
   ctcacatgtg tgctggatga atgtgtgacg gctgcttttc acatgttaat tccatctctc     540
   ctgtcagata ctcttgcatt tctttcatgt ctgccctagc gtctagctta gtgctgtgc      599
<210> 81
   <211> 599
   <212> DNA
   <213> Homo sapiens
<400> 81
   aggagaatgg cgtgaaccca ggggaggcgg agcttgcagt gagccaagat cgcaccactg      60
   cgccccccag cctgggcaac acagcgagac tccgtctaaa aaaaaaaaaa gacttcaaaa     120
   ttacctttta attacaataa tatattctgg attgtcttct tctgttactt aatttccata     180
   tccctcaccc tacaacattg tgcctcttct aatagttcat tgagaagtca cccagtctca     240
   ccaatccaca cgataatgct gataaggcag ggtgtctcat ctcaaatcac cacctcctar     300
   agtaaatata ctttactgaa ataaagtatt gtctgttgtg gggcttaggt taataagaaa     360
   taatataagt agtaagtact ctaaaatcct ggctttgttt caaagtaaaa ttagatgttt     420
   ttggtgaaca ttcaaaagta ggaaaaaata cactataatt tagagttgct ttgaacctag     480
   agatttatct aaattacaat aaatatcaga aaaatcttta cagtaaaagc aacttcttat     540
   aagcagatgc tttaaattaa gaattcagtt ttgattattt gtttttcttt ttgtaaata      599
<210> 82
   <211> 599
   <212> DNA
   <213> Homo sapiens
<400> 82
   tggaatggtt tggccattgg aaaagcattg cttttcctaa aattgatata gactatctag     60
   gagatcgtgg aattcaagac cctaaggaaa tattttgatc ttgagtttga gaagtaggcc    120
   tgccttcagt tctaagagag agaataaaag agcaatacta aaaaatctgg atcactacaa    180
   ctttaagctt ctggaagtgg cctcaaacac ctgcccagat tctccatagt gcatagttcc    240
   tttctcaatt cctgctgaga tgctgttcac ctacctgaga ttgcattgtt cttttgctty    300
   tttgctttcc agttctgatg atattacttc acctttcctc ttccaagata tgaaataata    360
   aattcaaaga acttccagac aaacccggcc cccatcaccc acaattgatc catcaaccaa    420
   gctacacaac atctggagat ttggagtagc ctgtgctgct tgcaatctgg gttttttttt    480
   ttttttgaat gaggaaaaat aatgcaacag ttatgaacta gaaaaggaac ttggatgtgt    540
   atctgggggt agacagagtg atgatgatga tgtcccctca gtggaatttt atttctata     599
<210> 83
   <211> 599
   <212> DNA
   <213> Homo sapiens
<400> 83
   caaactctgg gttatttttc aatcccctgg gttaagtcct gggtgattac cacagcatcc     60
   ctttcctcta gatcttgctg ctctacaaag ccatacagag cccttcagat gttcaaacac    120
   agaaatggtt tcataataat ttatttgtaa cagaactctg ttgtttgctt ttctgagatc    180
   tgctcactca ggaagcaaga ttccttaggg caaaatctct gtgcaaggca caaggtgaga    240
   ggctcagaac atattgcacc gatttaaata aaaaaaacct ctccttggcc tgtgtgtctr    300
   gctcctccgc tgacagagtc tttctcatag gagaaagaag agaaccagga aaggtgatct    360
   ttcctaaaca cactctacca tcttagatat ctttacaaca ggctttcaga actcttgtgg    420
   gggacatatt gcactgccca atttaattgc cagtgcttac tggcagtatc ttaccagggc    480
   agctatggtt ctgcccactg tcaataaaat gcgcagagtt tcagtgaagc aaaataaaca    540
   ggctctagag atctgctgta accactgtca acaataatat actgtacact taaaaatgt     599
<210> 84
   <211> 599
   <212> DNA
   <213> Homo sapiens
<400> 84
   ctggatggtg gcaccctgga acatggtaga tgggacttga gggcctgctg ggggtgggag     60
   tctgtgaccc tgaaggagtc ttctcaccct gactcttcat taggacaacg tggcctagaa    120
   ggaaggacca ttcagcaggg tcaggagcct cagctgggcc ggccaggctg gcaggggtgg    180
   agatggcccc ctcgcttccc accagggact ctgagtttga gacttgaggt ctagcaccac    240
   ttccagagcc ctcagaactg ggacccactc taggcatggg gagccaaggc acagtctgay    300
   ctacccccaa atccaaggac cagtaccctc ttggtctctc cctacttcct tgtttagaag    360
   ccagtctcag aaacagagct catcctcaac ccattccaag aagtagagga tgaaacagga    420
   tttaaatttc tgggaatttt tatatcttac ctaaaaaccc ttgaggcttg aatggggaca    480
   ttccaagcat gcagaaggcc actgtgtcac ttggatagta ggcccgtgag atcacctgga    540
   actgtgtttt cctctctgcc ctacagacct gtctcctcct cttcctaggc acatggaca     599
<210> 85
   <211> 599
   <212> DNA
   <213> Homo sapiens
<400> 85
   ccacccctat tcccagtata tttccctcct gtgtcaagcc acactccatt ttctctgact      60
   tgtttccttg aatgttttgc agaaaaaact tgtctgagat catggataca tttctatgct     120
   cttgcctttg ttccagccat tgtctctttt tagaacagcc tgcttaactc cctcctggtc     180
   tccagcgggt ttcttatctt ttacttatcc taaaagcacc tcctacagtc taacatcctt     240
   tatgactcct tctttttatt gttgtttttt gtttgtttgt tttgaacaaa gataggagay     300
   agcaaagcag cctgggtctt aacagaacag cttggaattc cagggcctaa attgcttttg     360
   caaacataaa cacacacaca gcaaccatga gaaaaatgaa aattaaaaat ggcttccaat     420
   tcagatgcca agaggctcag cctaaatggc cttctgcata attcactttg gacactttca     480
   tcctctgtag gtgaggacaa caggtctgga ccaaggttaa taccttaaat ggttcgaaat     540
   aactgacttt gggcagggtg cagtgcctca tgcctgtaat cccagcactt tgggaggct      599
<210> 86
   <211> 599
   <212> DNA
   <213> Homo sapiens
<400> 86
   cctgggggtc ccgaggcctg ggcagaaggc actagggtgc actaacagga atttaggtgc      60
   tgccagcttg ctcagcatgg aggaaacctc tgcagaggag gggtggactg atgaagagga     120
   gcagcagaag acaatttcac accgagtgct tacaaagtgc caggaaccat attatctcta     180
   atataagaca cgctgccagg tagttatcat cttcatcatt ttaaaaacaa gtaaagtgag     240
   gcacagaatg ttatgtaacc tgtgcaaggt cacacaacca gaacatggcc tctctaccty     300
   cactctacat gtactaatct ccatccccag ggccgagagg ccgcaaccgg atggtgaggt     360
   ttagtcttag ccctgatggt ggggtgggga ggggacaagg gtgacttgaa aagctctccg     420
   ttccccttgg cagctgtctt tccattcgca ccccctcctg cacccctctg ccaggcctgt     480
   tgatcacaga ggcaccatcc catctgctcc gcctagaagt tcagaatctc ccgagatttc     540
   tgaagcctcc agcactgttc tttgtgacac tctaaacaca gcagcttctc ctcctgggg      599
<210> 87
   <211> 599
   <212> DNA
   <213> Homo sapiens
<400> 87
   aatagggaga gagactaagt tcaactaagt agaagcaaaa ggtgagaagg ttttgaggca      60
   cttggttgat ccagtgaaaa agaagttatg gtgaagatta atcaatggga tgttttgagc     120
   atattgagtc gttcctgagt ttgcaaatgc ttttccttgt gattaggcca tctgtgttag     180
   caaatcagca tacccacaga aattaagcta ctactctcct atagaggatg gaggatagaa     240
   gtgttatttt cttcatgttt acatttcaaa gagatggttc ccagcccttt gagacagacr     300
   ttttctgggt tgtaaactgg aaagaggcta tttacagcca tttcaaagga acagaaaaag     360
   agtatacaat tggcaagttt tctacagtaa atgctgtaag taaaggggga tcaggagcct     420
   aaaggtagaa gaagcctgtt taaaatgtgt aaagctgagg aaaacattga ttccatgttg     480
   gttagtagtc attaatcttt aatatattct tggaactgtg ttatttaata atctcattaa     540
   atatttagaa caattttgta aaaaaaagat atttatctcc attttacaga aaaataaaa      599
<210> 88
   <211> 599
   <212> DNA
   <213> Homo sapiens
<400> 88
   cagtggcagc cagaaggctc ttttctcaca gtggaactgc agggggtggg gggaggggaa      60
   atgataaaag aattttcttt tttaattttt gcgatttttc atccatctca atgcatcagc     120
   tgtttggcag cagcacaagt cagagacata tgtccatcgg atttactcct ttttcactgc     180
   ttccattgaa ggccaagaaa ttcttccact gtgacttcct cccccattcc ggaaaggaag     240
   tccttagaac ttttgcctgt aggaccccaa atgtcgcaag cccttagaac agtggtcccy     300
   aaccttttta gcaccaggga caggtttcat ggaagacaat ttttccacta ccgtggggag     360
   gggggtatgc tccacctcag atcatcaggc attagttaga ctctcataag gaggacaacc     420
   ttgatccctc acatgcacag ttcacaatag ggttcgcgct cctatgagaa tctaatgccg     480
   ctactgatct gacaggaggc ggagctcagg agagcagcct gggagttggg gagccctgcc     540
   ttagaacttg cctataggac cccagatgga gaacaggcca tggattgttc ctcttcacc      599
<210> 89
   <211> 599
   <212> DNA
   <213> Homo sapiens
<400> 89
   ctgggttggg gctttctggg acttctatta ggtgagaatg tcccaggcca gctctcccct      60
   acccctccca acctggggtg gttctaggag ggtgctgggg gtcaggcagg cctgggtggg     120
   aatcctggca cttctgactg gctgtgggac cccatgtaaa catacaatct tatagagcct     180
   cagttttcta atctataaaa tgggactaat aataataata gctgctccat gagatttttc     240
   atgacattga agttaatctg tataaagctt tcgtcactgg cacatactga agttctccay     300
   agatgctgtg gttgctaaat catcctggta agagactcct ctagtacgtg ctttagaaag     360
   cagctcttct ggataggcgt ggtggctcac gcctgtaatc ccagcacttt gggagtccga     420
   agtaggcaga tcacttgagc tcaggagttc gagactagcc tggccaacat ggtgaaaccc     480
   tgtctctact aaaaatacaa aaagtttagc caggtgtggt ggcgtgtgcc tgtagtccca     540
   gctactctag aggctgaggc aggagaatca cttgaaccca ggaggtggag gttgcagtg      599
<210> 90
   <211> 599
   <212> DNA
   <213> Homo sapiens
<400> 90
   gcagccaggg ccctggcaca cagggcctcc ccgatgctta aaccctgccc aagcttgctg      60
   gaaagattca ggggagaaac tcccagaggt ttcaggagag gaggagcaat gatggggaag     120
   aacatggggt ggtttgccag gtatcaaatg ctagaaatga gggcctggaa atagaagctt     180
   ctgacaaaga gctacccttc tgtgcatgat agattagaaa cttgcaaact ccaaagatgg     240
   gagggcatgg aattcaagag ggtggggatc aataaatatc agagtcttta ttcagactcr     300
   gtagggaaac aacctttaag gtcaagaagt caccagatct taaaacatgc cctggtgttc     360
   ccacagtggc agggagcctg aaggtgcagg gtggcagagg ggggcccagg gtggtggcct     420
   tggccaggga atctctagat gtgaaaattg ctctggcagc cccaggcttg ccctttgctt     480
   gggtcctgaa cactcagcat attacactca gggagcatga gccgcttcct tcattctgca     540
   ggggacagtt gccttcccag gtacctggcc tgaaatcctg agtgctgagg aagggcatg      599
<210> 91
   <211> 599
   <212> DNA
   <213> Homo sapiens
<400> 91
   cggtggggtg agagcattgt aggtaggtaa gattggcttt ccaccccact tctcccttcc     60
   caaactagat gcattccccc aagtattgtt ctcctaaaac cacttggctt caaccaggta    120
   ccaggtgcag ccaactttct atgcaatgaa aagaagccag gatcaagatg aatgcagaac    180
   taccatcaaa aatccccttg tctgcttatt cttgcaagca gacataagat ggcctgataa     240
   agaattctat gatttcagtc ctaatgaaaa cagtatgttg gcccaggacc caggtactay    300
   gttttaagtc tccctgatgt gctgactggc atggacgagc ggaagaagag ggttgatgat    360
   gccacaacac actagcagat gggtcacact acattgtgaa ggtgaatggc agaagacgct    420
   cactctttgg tccttgctac tttggcagca acagctcgaa atcgacgcta agagttcctg    480
   gtacaagcag actctgctag tcccatctcc tctaggagct cattaattta ctccaaatag    540
   ccagactttc tttttcattt actggtttcc gaatcaggag aaaacacata aaggtagag     599
<210> 92
   <211> 599
   <212> DNA
   <213> Homo sapiens
<400> 92
   agttagagta ggaaagcaag tcacttatcc tcttcaagct ttattttcct catctgtaag     60
   aaactacctg cctcgaagga taatttcaat aatatacgaa aagtccttaa cacagcttgg    120
   agcatagcaa caatttaaag taaatgttag attgaatcat gatattgaac agctaggaaa    180
   acccttctct gaaattcaga agtaaaggca attttgaata gaacacaacc aacctatttc    240
   atttagcttc aaaatatttt tgtgatatat cacatacctt tctcctgaag atgaattaay    300
   aactgaccaa ttctctggag ctgaatagga ataaaaaagt cacagtatca atgagaaaag    360
   ctcaggatga acaagtttta tattaaaatg tgaaattgta taatacttac caagtttcct    420
   gcttttgggg caactgtctt ccgtctattt taatggaaaa aaaattgtta gaggagtaat    480
   cggagtatca aaattcaaaa attttacaaa ttattcttat gtctgatttt gaaaactagg    540
   aaaaggggaa atcaaccatt tattcttacc tcctggacat ctgaactacc cttttcagg     599
<210> 93
   <211> 599
   <212> DNA
   <213> Homo sapiens
<400> 93
   aaaatttatg aagtatattt attttttctt gctgctataa caaattacca caaagctagt     60
   ggttttgtat agcacgaatt tattatctta ctgatctgta actcagaagt tccaagttct    120
   gtaactttcc aatgcaggtt tcactgagct aaaatgaagg cactggcagt gctgcatttc    180
   cttgtggagg ctttagggaa gagtcagttt ccttgcctct tctagcttct agaggctacc    240
   tgcattcttt gactcacggc cctttcctcc attttcaaaa tcagaaacat tgcatctctr    300
   tgaccattct tccatactaa aagatccttc tgggcatcac agcatggaaa ggttatttga    360
   ttttaaatac ccttgtaatt agattggact ctccccaggt agcctatgac aatctcccat    420
   ctgtcaggtc ccttttgcca ttcaaagtaa catacacaag tttcagggat taagacatgg    480
   acatctttgg ccatcgcatt ctgcctccca caagtaaagt tgacatcaaa agcacgttca    540
   ataaaaagaa acattgaaat aaaattaaat cttttgctct gtcaaagaca ctagtaaga     599
<210> 94
   <211> 599
   <212> DNA
   <213> Homo sapiens
<400> 94
   taaatactta tagaaactat gcttttgaaa tgattttttt aaagttattt tctttgacca     60
   ttagttgaag cctactagca acacacaatg tttggagcca aatggttagg cagggcaaaa    120
   ccagggggag cctaatcttt ggtcctctgg tggccacagc acctgagaaa ctgaactgtg   180
   gcttcaggaa atccactctg gggccacaga agaaagaaca gttgtaccaa ccaaggcctg    240
   gcctctgggg accagtacag cccatagcag aaggcaagat gtctgacaca atcctggcay    300
   gttccttaaa ctcatgggag ggctggtaac cactggacca gaacagaagc tcctggaggc    360
   atctgagact tgtgctcagc tctttccttc cttggcttct cttgctaaga agctcagact    420
   ggcactgcaa accatcaatc tctggggcga aatggcaagg tcatcagata tgcatttctt    480
   aggatagaaa tgcaggaagg taaaacttca tccaaaacag ataacagttt aagagaaaga    540
   tatgggctcc acccccaaat tctgggagcc ctgggcttag gagatgggct ggggaaagg     599
<210> 95
   <211> 599
   <212> DNA
   <213> Homo sapiens
<400> 95
   tttttctgtc attatttctt tgtatctcag aaatagcatg tagctgcatt ttagggattt     60
   cttttcctcc tgatttgttg tttttcatga gacaattcaa accattccca tttattgtga    120
   ttactgacat gtcaaagctt gtttctgcca tattgtttta tatttattaa tatatgtacc    180
   atgctttcta cttttcccca cggttttcct tttttatcaa ttgaccagct cttcttcatt    240
   taatttctga ttaagtgctt acctttaaat tttaactcca acatctgtcc attattttay    300
   tttcccttat agcctgtgta agtgtagtgc cccttgtcat tatgctcggc aaagcacaaa    360
   ccttagccca cttggacttc cttcctctcc ttctcctccc cacctgcaat gttaatacca    420
   cttgagcttt tagttccagt tccatttaga cttagcagca aattctaatt cctttactca    480
   ccactgcttc ttgaatctgt agctttctct tggatttatt ttcattgtgt tgaagtacct    540
   cctcctacag gtaggatgga aggtgactca ttttctcatc tgtggatgtc tgaaattgt     599
<210> 96
   <211> 599
   <212> DNA
   <213> Homo sapiens
<400> 96
   cagaaaggag taagatgata tattcaagac ctagtagggg gaagccaact aagaatacta     60
   tacccagaaa tcatgtcttt caaaaatgaa ggattgaaaa aacttcgtca gacaagcaaa    120
   agttgagagt ttatcaccac tagacttgca ttgtaaaaat ttcaaaggta gttcttcaag    180
   atggagaaaa atggtaatta gtaacatgaa aatataaatc ttactgataa aagtaagact    240
   gttttaaaat ccagtacact ctaacactgt aatgacaatg ggtaagtcaa tcatgtctty    300
   agtataaagg ttaaaggtca aaacgattaa aaatgactaa agctacaata atttcttaag    360
   ggatacaaat gctgaaaaga tgtaaattgt gacatcaaaa acataaaaca taggaggaag    420
   aggattaaaa ctgtagagct tatatatgtg attaaaatca cattgttatc agtattattc    480
   acaataccca agatatgaaa tcagcctaaa tgtccatcaa cagatgaatg ggtaagaaag    540
   gatgctgtat atattcacaa tggaatatta ttcagtcata aaatataatg aaatccagt     599
<210> 97
   <211> 599
   <212> DNA
   <213> Homo sapiens
<400> 97
<210> 98
   <211> 599
   <212> DNA
   <213> Homo sapiens
<400> 98
   gagtgcatga gacagagacc ttttaagcat gtccttcctt ccccaggttg caactgactt     60
   tctccaggga aggaatatat tcccaagttg atcaaatcgt gtggatgcaa gtcttccaat     120
   gtgctcagtc aacaaaaatg tctgagcctg ggatgtcagg taacccactt ttcagaatac     180
   ttaaatcaca caaaggagca ttctgtttag tactgggacc acaatgctta agctgagcct     240
   taagaagtca gtaagaatct taataagatt tggacatgat gcccacagcc ctgatactay     300
   gtccaggcag cttaaaattg cttgaggtta aacatttaag ttttataccc agccaacttt     360
   ctcacaaagg agtggaggtg atccacactg acatatgtat aaaccataca tttaggattt     420
   aacatatgtt aaggttataa tattaatccg aaattaagaa aaacagaaca caaacgctat     480
   aggaaaacat aaacatatga acaaagacta gaagaaaaca aaaacctaag tagttgtttt     540
   aggaggatgg gattgtaagt aaactcgctt tctcaaaaag gaatctgaat gtcatttta       599
<210> 99
   <211> 599
   <212> DNA
   <213> Homo sapiens
<400> 99
   ggcagacggg gagtagcagc tgcccctctc tggatcaggc ctgagttctc tggttctcta     60
   taatctctac cactccctaa tgtctcatac actggcctgc ttgtctattt gttacctccc     120
   tggatcctgc agccccttta gtcagcctgt ttaatccatt ctgcttggca actgaggaga      180
   agaaggaaat tatagacagg ttgtatgtgg gtgtccatgt gttaaaatcc ttctgaaaac     240
   tcctgatctg gctgctctcc tttccctatg accaactttc cacccagcct tgccacatcr     300
   gacctagtag actcatcacc aagatgtgat gagcctagca tcctactccc aaatgaaatt     360
   cctggttatc cacacagggc cctcactttt gagtcccata tgctgaagac caggattaca     420
   gtggatgcca ggtcagtttc atgtactgtg cccctctcca ttccttcctt attcagcaca     480
   tatatctgag tggttactgt gtgcctggca ctgctctagg cattgcactt gtcctcatgg     540
   attttacact ctatggggga aagtcagaaa acaaataata tgtaatacat aagatgctg       599
<210> 100
   <211> 599
   <212> DNA
   <213> Homo sapiens
<400> 100
   gcatgctaag cactaggggc gtcccagtcc tggttgcaac tagctgcgac ccctgttctt      60
   ccagggactc tttctcacct cttttcattc ccttctttta tactcttagc taaatataga     120
   gtgacttctt gattaattca tctcagttca actaacctct actggaaccc tactctttac     180
   tggaaggaga agattcctgc aagggattca cacatgagta agatgtggtc cctaacttgt     240
   catattccgc cttagaaggt attgcaattt aatgacataa agcattaaaa tttccacaak     300
   aggaatacaa agttctgtgg gaagacagag gcaggagaga gtgattcttg ccagggaact     360
   agaagttatc agagaggggc agcactggag cttgtctggg tcccaaagga tggatggaag     420
   gatgcaagga ggtcattgca tccaagggac agagagaaag gcatgcaggt gcgaggagct     480
   ttgcggtgtt taaggcacta ggaacagcct cataggcctc aaggggtgtg tgatgaagaa     540
   tcggaaataa gacctagatg gtgggctgga ccaggtggta gagggatttg aatgctttg      599
<210> 101
   <211> 599
   <212> DNA
   <213> Homo sapiens
<400> 101
   tacatctatg atataatcca aacctgtgag tataatgtta aatgcaagta gaacattttc      60
   tctgattata ttattttagg ctgaattaaa acttgcaaat gtgttaagga tgaataatga     120
   tggcttaata tatcacaccc ccaaggcata tgcacttcac aggggcctgg ggcccaactg     180
   gcagcagaag ggaagaggga taaaagtgga ggcacaggcc acgtgcacct catccatcac     240
   ccatggttat taattgtctc ctcagctgcc ctgacaaggc tcttcctggg gctttctgcr     300
   ggaggcttgc aaggtgaaag ggaaggtttt tatttttatt gctgcagaat tgaggatgat     360
   tctttaaaga tgacgcatta aatcaggaga gacacattaa caaggggagg tccggagtcc     420
   accttgcacc cctgggcagc tctggccctg caggcattca ctctctctgc actccctcct     480
   ccgccaccag gtagaggtgc taacactcac tccctgacag gctgtgagta aacaggtgac     540
   tcaatcttta acccttgctc agtgggttaa gaaattagtt aattattcac atcccactc      599
<210> 102
   <211> 599
   <212> DNA
   <213> Homo sapiens
<400> 102
   gagcagcctt ggcttccggc tcatgcactc acgatgctgg ctgcgaatgg gaagtcccta      60
   aacagaactg agccccctca ttcagctcag agcaaacggg gctggcagca gcccagggca     120
   ccacgaagca gagtgcctct gtgtgcccaa cacagccgca gtggaggtca atggatgtcc     180
   ttgccggcgc cctgcttttt gcttttttaa gaaaataaga agcctgcagc tgcggcaagg     240
   agaaagaccc cgtgggtcgc tccttttaga ctacgctatt acacctgctg actatccaay     300
   agaaaaaaga aggagaagga aaatcgggtt aactttctgg tatttagttt gcagatatgt     360
   cagcaggcca ccttctctac ttggcaggat cacgatgtat taagaatcct tttgtccacc     420
   aggaaagaca aatttcctgt ttgtcttgca tttagatgca acaacatcat cctaaagaga     480
   tgcccctgat gtgtgagagc agaaaggggt gatccctttc ttccttatcc taaggctcac     540
   ggccaacacc cctataacaa aagacaggtt aacaagagaa aagcatgaca aatttattt      599
<210> 103
   <211> 599
   <212> DNA
   <213> Homo sapiens
<400> 103
   aagtacctga tgtctggctt gccatgggat aggtaggatt catccatcct aacttcccac 60
   cctaactgcc cccgtgtctt tttgtgcagg gggcccagaa ggttagtctg ggcacaactg 120
   gccttcctca gtgccgtaga aagggtggta cactggggca atatttattt acattgcaga 180
   gaaacctgat aagaggttga gaaatacttc tgacattaat tataatatat taccaggtta 240
   tatggattaa atgatcagat tacactccag tcatgcttct gaatatgtaa gcacccccay 300
   aaaagaagtg acaatttccc aagatacctg gactcaaacc gtagggtgtc tacccctcct 360
   ctctgtggtt tctttccctg agttgctcac tgcccatgtg gcttctctgt agggtctgtc 420
   accttatggg attgatttta tggcagctgt gctgtgactc gtgcatccac agaacaatgt 480
   tgaatgactg tgtaaacgca ttgctgtaag caacctgagg tctgaacttc tccttatctg 540
   attgttccct ctaagtaatt tgtagcaaat ttcaacattg tttcccattg gccagcaaa 599
<210> 104
   <211> 599
   <212> DNA
   <213> Homo sapiens
<400> 104
   ggaggccaag gtgggaggat cccttgagcc cagtagttca agaccagcct gggcaacaca 60
   gtgagaccct gttcctaaaa aaatttaaaa aattaaaaaa ataaaaacat gccacaggac 120
   cactgctcag agctctttgg tctggcaacc ctggaccttc acatggtttt gtttgccaaa 180
   gcaaagtcct tattaatatc caaatacagt ttgagttttc ttgggcttta tcctctccag 240
   aaacccagga gtgtgtcaac ttcttggttg cattgctttc ttcaaagaga gagtgaatcy 300
   ggtagagata gaaaggatgt cctttacgta tcccacagcg gcctcacgga gaacgataag 360
   agaacaatca ttaaaacttt cattgccata attttctgct ttcgtttttt aaaagtttgg 420
   gttagcaacc aatttcctcc ttgaccaggt gattttttcc ttagactgtt tatccagagc 480
   attaaggaaa acagagcata ccaccctccc accatagctg ataaggggat tcaacacagg 540
   tgcgtgtgct agagttaagg gaacatatta tgctaacatg aatctagact gacttaaaa 599
<210> 105
   <211> 599
   <212> DNA
   <213> Homo sapiens
<400> 105
   ccatacactc agcaagaggg ccatggaaac ctggctatta aaaggactcc cccatggagc 60
   acagccctgt ggaaatctga gggtgcatta aagtaaaaca agtcaatgct catcagaact 120
   cccttccaga ttccaaaggt gactggagtc aacaagcagt tatccctgtg tcctcccctg 180
   atgtgggcag ctgggtccat gaaataagtg ggtagctgca gcagtgcaga gatgggctga 240
   atctagacat gttctgtata gacgctgact ggtctgggct ttgcttaccc ggtggaggay 300
   gcaaatgctc actgtgccat tcaaaagcca gaacagaaga tattgcacca atttggatgg 360
   cccagtcaca tttctttaga ccaaggaaca cactttacag cctgtaatat ccaacaatgg 420
   gcagagctag tctcctccgg gtaatagttt gatagaaaag tagaacaggc aattcaaaca 480
   ttagttgtcc taaacggggg gagatgaaga cacgaagggc tggcttgcac atcttcacaa 540
   gtgtagtgca cactcaccat gagtggggct aaaggcgtgt tcccctaaca ctttcctcc 599
<210> 106
   <211> 599
   <212> DNA
   <213> Homo sapiens
<400> 106
   caaatgctca ctgtgccatt caaaagccag aacagaagat attgcaccaa tttggatggc     60
   ccagtcacat ttctttagac caaggaacac actttacagc ctgtaatatc caacaatggg    120
   cagagctagt ctcctccggg taatagtttg atagaaaagt agaacaggca attcaaacat    180
   tagttgtcct aaacgggggg agatgaagac acgaagggct ggcttgcaca tcttcacaag    240
   tgtagtgcac actcaccatg agtggggcta aaggcgtgtt cccctaacac tttcctccgk    300
   ttcctggtgg atctggggta gaggggtggg aggatgctgg tatgactatg cagttcttgc    360
   caagggagaa gtacccaggc ataatgactg caattttttt tccttccctc tccacatcac    420
   gttaactttt tttcccctac ctgatgcagg ggtcataggc ccagggctgc aattataaat    480
   gctgacagca gggatgattc ctaagcagga aattgtcact atgtttttaa aacttgtgtc    540
   agaattccca agggcctgat ggggatgggt tgtgccttca ccccatctgg caaactggg     599
<210> 107
   <211> 599
   <212> DNA
   <213> Homo sapiens
<400> 107
   gcccttataa agagacttga gtgagcttcc tcgcccttgc tgccatgtga gaaggcagca     60
   acaggacacc atctatgaaa cctcacaaga cccttaatct gctggcatct tgaccttgga    120
   cttttgagcc ttctgaactg cgagcaatac atttctgttg tttatgaatt ggtcagttta    180
   aggtattttg ttacagcagc tcaaacggat taagacagag cccaaccaaa cacccgctcc    240
   ttggatcgct cccctccttc cttttgttgc ctctcccctc attaaggaac gggaagggar    300
   agatgaggga taaacaagcg tggtgacgga ttggtcctag actgatgcgg atgacgtatg    360
   catcagagat ttcctgggcc ttgctctgac tttggtcttc cagcagtgcg caggcctgct    420
   ctgccccgct ctgccccgct ctgctggcct cagccttcct cattcatcat tgttcacctc    480
   actcccatcc accttcttcc ctttttccca aacctatcct gatgagaagt ttgaattctg    540
   ttttaaggaa agaccttcca atgcagtgtg gagtgagggc cattcccaca tacatacac      599
<210> 108
   <211> 599
   <212> DNA
   <213> Homo sapiens
<400> 108
   tgtgctgaca tttttgaaag ccctaaaata atttggtgcc catagtggga accttgtcat     60
   acttatttac atagtatgga aggctatttg acaaaaaaat caaatgaggt ataggaatag    120
   taactcatta taaattgggg acaataaagc caaattccac tggattttaa agttatacaa    180
   atgattctct agtccctaaa ttagggatat aaaagtccgt aggcagcttt ggggctgaaa    240
   ggggttctgt aagcatcttg ctgtataatg gagcaagtcc tgacccagaa aagaatctgy    300
   actttgtccc agatgggcta taacttgctc tgtgactgcc atcatgtgtg ttctggcaga    360
   gattcatagg caggcagagg agtggggaag cttcatagca gaaaagggaa ggtgtcaggt    420
   gtgtgctgac tggaggctgc tggccgggga agctggaacg aagtgggcta tccagaggtg    480
   gtgtatccat cctctgtagt tggttagggc acttagttca ctctctccag ttggggttga    540
   ttgtagagtt ctaacttttt acgtggtctg gccattgtcc atgtgtatat tcagtctct     599
<210> 109
   <211> 599
   <212> DNA
   <213> Homo sapiens
<400> 109
   aacgggtggg tgagttatgt gctggcaaag gcaggtgtgt ccacatgcgc gagcagacac      60
   tgagactgca gcccacacac ccagaggaac aagcaggaca tcttaggtta ggaatgtgcc     120
   tgcagctggc agtgcccagg agagggactt ttctaactgt gttgtccagt gccttagtcg     180
   ctaaccacat gtggctgcat aaactttaaa ccagttagaa tgacataaaa ctgcaaatgc     240
   agtttcccac ttgcactagc cccattctaa gtctccatag ccacctgtgc ccactcgccr     300
   ctgctccagg agtacagatt ccagacattt cagccactgc agaactgtct cttgggtggt     360
   gtctgcaggt gcctgctgca gctctgccag gcaaagtaga gtggctgctt ggctgcctat     420
   ggatttagat gatcctgcca acatctgatc agttgctctc agcacctgag cttcatcatt     480
   aggatggatc tggggcatgc agggagaatc ccagactgtc agagaggtgg cctggcctgg     540
   ggtgaccagt caggcctctt gtccttatgg tcacattctc agaattgggg ccagggatc      599
<210> 110
   <211> 599
   <212> DNA
   <213> Homo sapiens
<400> 110
   taatttcttt ttaacaacaa agtcttcatt tttaagacat tttttaaaag taagtcacta      60
   tggattttat agatttctgt ggtttaagta aaatgtgttt caacatagca taaaacctga     120
   agaagaaaaa tacagagttc aactattata catacttgta ctatatgaaa gtcatatacc     180
   atttgaagta cgtaactatc agccctaaag aagccactaa aatagcaaaa gcctctatct     240
   aataagtcaa caaagcaaat taaatgaggt attaaaaaaa ttaattcaaa agaagaaagr     300
   acataaggaa aaaggaaaaa aaacaggcaa aacaaataga aaacaaatat caaaatggta     360
   gactttaatc taactatata attacattaa atctaattaa atctaaataa agctaaataa     420
   atctaaataa aaaatctaat agatctaaat aaagctaata aagtaaaaag gcaaacattg     480
   tcagagtgta taaataaagc aaaactcatt taaatgccgt ttataagaaa tgtactttaa     540
   atataaagaa acaagcaggt taaaatgaaa aggatggaaa aagatatgac atgctaaca      599
<210> 111
   <211> 599
   <212> DNA
   <213> Homo sapiens
<400> 111
   caggccattt tcacttcttt tgtggtggaa tgtcaccagt taaggcggga accggtcatc      60
   tggatgtgta catgcaggtc acaggggata tgatggctta gcttgggctc agaggcctga     120
   cataaagtaa taaaacttct aaaaaaaaat acttagcacg atgaggtcct gaggcacgga     180
   aatatttaga cattttaaat agatattcaa caatgctgta agtaactggt agaatttcca     240
   taccaggtct cttcctatgt cactagccca tgggcacaag aggtcatgaa agaggaacar     300
   aaataggaaa acctgcatca acacagaaac agcctgttct ttgccagaat ggagaacaag     360
   gcaaagctgg tacttttaga aagctgaaaa tttgtgccaa tgattttttt cctagcttat     420
   ttttggcttt ttgattctaa ggagttttga cctacaaaga tgctcaatat ttaaatagac     480
   agatattttt gttttatttc ttccattgct tttatacttc ggcatcctcc ttactgagat     540
   gagataaagt tttcaaaaac attgtatgga attattttct atgtatcatt atttaattc      599
<210> 112
   <211> 599
   <212> DNA
   <213> Homo sapiens
<400> 112
   ggcacggaaa tatttagaca ttttaaatag atattcaaca atgctgtaag taactggtag 60
   aatttccata ccaggtctct tcctatgtca ctagcccatg ggcacaagag gtcatgaaag 120
   aggaacagaa ataggaaaac ctgcatcaac acagaaacag cctgttcttt gccagaatgg 180
   agaacaaggc aaagctggta cttttagaaa gctgaaaatt tgtgccaatg atttttttcc 240
   tagcttattt ttggcttttt gattctaagg agttttgacc tacaaagatg ctcaatatty 300
   aaatagacag atatttttgt tttatttctt ccattgcttt tatacttcgg catcctcctt 360
   actgagatga gataaagttt tcaaaaacat tgtatggaat tattttctat gtatcattat 420
   ttaattcata tggaatttat taagggcatg agataaaaaa taatatttct tccaaaagga 480
   aagacaactc tctcagacca ttacttaaaa aaatccccat tgatttgtga agccactttt 540
   ataaaacatg tccttttggt gtatactatg atttgttgat gggttctcag ctctgttct 599
<210> 113
   <211> 599
   <212> DNA
   <213> Homo sapiens
<400> 113
   agggacagag ttatttccga ctggttgcag atggggagaa aggtctatgg tatctgttgc 60
   tgatctctgg gctcacatca gacactttgc aaatctggct ctctccaggg gatttgggta 120
   acagccctgc ctttcagagc aaataacagt atacactcgc agagctcgtt atagcagttt 180
   tggtgggttt agggacgcaa gttcccagcc gggactattc ctgtggagtt ccctgagtta 240
   caccctccag gccacgcccc ctggccaaac gtgagtctag agcagagagt ttccatgtar 300
   aggcttcatc tgtttgtatt ggctcattac agacactcct tgagatttga ccaatgcaac 360
   atgtggcccc atgctagcgt gctatgtgct gagacaacag cttagagcaa cagctgggga 420
   accttccctt ctgcctctgt tctgaaataa agcacgtcca tttaagacaa agggggctat 480
   gctgaaatga cttatgggga aaattacagg tcacaaaatg taatgtgact ataacatatt 540
   ggaatgttga tgactgctcg ccataaagtg gtcaccaatg tttctttcac cccccaaca 599
<210> 114
   <211> 599
   <212> DNA
   <213> Homo sapiens
<400> 114
   taataaaaaa aagtaaggtg attgatatgt taattagctt gattttagta atcatgtcac 60
   gatatatatg tgtctcatca tgttgtgcac tttaaatata tacaactttt gtcatttcta 120
   tcttaataga gctgtaaaaa ataaaaatat gaagcaaggg acagtgttca ccagctcata 180
   atggatagaa agtatacctt atatttattt ttatgctaga ggtttattca gttattcact 240
   catgccttta ctgaaaacta ccctcttcta ggcatagtgc tgggaactga gcacacaccr 300
   atgaatatgt cacagcccct gtccttggag accttcacct catggtctag caacaggtga 360
   acaaaaaaaa aagcaatatg ggcattcagt aaatccctga ctggtatgca aacatgaggc 420
   cagtgccccc actggatacc cctccctagt aatgtagaat agtgaccttt tcaccacacc 480
   ctggctaaag cttctagaaa cacacacaca cacacacaca cacacattta gaggtacaga 540
   ctgtgactat ggaacaattc agtgtaacgg ccacatgagg aaccgatgtt tgaccttaa 599
<210> 115
   <211> 599
   <212> DNA
   <213> Homo sapiens
<400> 115
   tctacctaat actctgtaac tatctccaaa aagttaaata aatcagatca ttaactagag     60
   aggcctttga ggctaagtga cttgcccgag tttgcaaatc ttgttcagag aagaacaggg     120
   accagttccc agcacctggc tcttcctgcc aggattgatg atattgaccc cactgccctc     180
   ttgtcatggt tcctgaaaaa aactcctctg attaatgcac caccttaaac cctactagat     240
   gatttcagaa tccaaccaga tgacacctct tagataaagg gctctcttag agatcggctr     300
   tcggggcaat cgttttctag aaatagcctc tgcccagaat agatggaagt ctttcaagct     360
   ggctattttc taaggtagac cctttctgag gcctttgaag ttactttgca gctgcaaatg     420
   gctgccctat ggacagaata ggactagatg tctaataatt ccagagcagg cctgcacagt     480
   tatctcaaag cacagtgccc caagtgaaca ttttcttagc ataaacatcc tattccttaa     540
   tatgttatta cttccagcaa acatagattt cttaagttct ttcaatttga gataaagtg      599
<210> 116
   <211> 599
   <212> DNA
   <213> Homo sapiens
<400> 116
   ctgagcccag ttagaaagtc agcacggcac acaagcattt gggaagttta ctttagattc     60
   ttccacagat atcagtgtgt gagttaacca attttaaatt atcctctatt taacatgttc     120
   ttattacaaa tatttgtatc aaaaattcca atcagatgta ctttgcttca ctacctttag     180
   tctacaatta ttgatcttaa tcactttgat gccagtcttc aaccttggtg gtcagcacat     240
   gacttctggg ttttatgaat ggccataaaa ccagacacag cgtgtccagc tctgtgtgty     300
   aggaaccaag ataaatcatg atcttcgcag ggttcagttg gctcttctgt taaatgaggg     360
   gacagaccag attcgtttgc cttcttagtt tcataattct acaattttaa ttctattcaa     420
   gttttaaagc ttctagagct tatagtataa attcaggatc taaagctaat aacaaagtat    480
   aaaataaaga tctcttgtta ccacttccct atcttcagtg accccttgca aagaaaagac    540
   acatcttttt attttcagag atctcactaa agtgcactgt aaactaaaga gcccatgaa      599
<210> 117
   <211> 599
   <212> DNA
   <213> Homo sapiens
<400> 117
   tgatgtttgt gagggaaagc atatgttaat cagcttgatt tagccattcc acaatgtgta     60
   catatgttaa aacagcatgt tgtacatgat aattatatac agtttgtatt tgccaattaa     120
   aataagattt ttttaaaaag tgtaactcaa atgtacctgt tagcaaagag ctgagaggcc     180
   ctggaggggt cctgggaagc aagagacctg ggccaggagc ccaaagcccc ccacccccat    240
   acacattcct gccatcaatc ccttgtgtta cattggacaa atcacttttt ccctgagccy     300
   gttttctcct gtctaaaaga gagatgccac ctgtaccctc acttccctcc cccgccttcc     360
   ttccatcctt tctttcttca tccaagcatt agttgagctc ctggcaaata ctggcctggt     420
   ggcccagaca tgagttactc tgtcttctgg atagcttcca cacccccacc aactttattg     480
   gatcattgtg ggggaataaa tgataagaca gaggtgatgc tgttcttttt aagaaggtac    540
   ttcccaggca tgaggcatga ttgatctttt tacagcattc ctgatctaaa ggcaggatt      599
<210> 118
   <211> 599
   <212> DNA
   <213> Homo sapiens
<400> 118
   aagacaacaa tcctatttta aatggctacc aaataattgg actaaattta accaaggagg      60
   taaaagatct gtatgctgaa aaccataaaa cgttgattaa agaagttgag gaagacataa     120
   ataaatggaa agatatctca tgtttatgga ttagaataat taatattaat atcaatttat     180
   gaataaataa aggtttgtca cctttattgc actgaggtac attcatttat actaaatttg     240
   ttaagaatgt ttatactgaa agttaaaatg tttatactac ccaaagccgt ctattgacty     300
   gatgcaattc ctatcaaaat tccagtaaaa tttttcacag aaatagaaaa aaaatcctaa     360
   aatccatatg gaaagcaata gaccccaaat agccaaagta atattgaggg gaaaaaagcc     420
   agagacatta tatactacgt gacttcaaaa tatactataa agctataata gtcaagacag     480
   tattacactg gcataaaaag agacaatgac cagtgaaaga gaatagcaag cacagaaata     540
   aatccatgaa gttagtcagt tgtttttttt tttttttttt tttttttttt gagacagag      599
<210> 119
   <211> 599
   <212> DNA
   <213> Homo sapiens
<400> 119
   tcacctgagg taaggagttc aagaccaacc tagccaacgt ggtgaaactg catttccact      60
   aaaaatacaa aaattagcca ggcatggtgg caggcacctg taatcccagc tacttgggag     120
   gctgaggcag gagaattgct tgaacctgag aggcagaggt tgtagtgagc cagtgcactc     180
   cagcctgggt gacagagtga gactcatctc aaaaagcaaa caaacaaaaa gtgaaaaata     240
   aaaataaata tttattttaa aaaagaagag agatctcaaa taaacaagct aacatttatw     300
   tctcaaaaaa ctagaaaaag gagaacagac taacccaaaa gttagtagag aaaaggaaat     360
   aataaagatc acagcagaaa cagaagaaca agaaactaga aaaatgataa aaaagagaaa     420
   caaaactaag aaataatttt taaaaagata attaaaatgg acaaacttta tctaaactaa     480
   gaaaaacaga atactcaaac aaaatcataa atgaaagaga aaacactata atagatactg     540
   aagaagtaca aatgatcata agagactact ttgaacaatt atataccaac aaattgtat      599
<210> 120
   <211> 599
   <212> DNA
   <213> Homo sapiens
<400> 120
   tggctgagtg gaactcttgt ctctcagtat tatactacct tcatcttctc tgttaagtgc      60
   acagcttaac agaactccta cttagacagg cctcatagaa cctcattctg ttcagaagca     120
   agtgtttctc agccaaagat tccatgatct atgaagattt ttacaggcct atgccagtag     180
   atcccagtgc tgggccagcc ccccagacct catcccttgt ggacctaagt ttcaaaccag     240
   cctctacagc ccgaggatct aggttagccc tcatgaaccc accctccagg ctggccccar     300
   tggaccaata caccaggcac accccaatgt atccaagccc caggaccatc tcagtgccca     360
   gaaagcccct gcagactaaa gcttaaggcc cactccagtg ccaggtcagc tcttgtggac     420
   ccaggcttca ggccattccc tccagggctg cccttctgga cccaggctcc aggcccaccc     480
   catagaccca gtcaacttgt ccccccagtg gatccaggat gtagacccac cacatgggcc     540
   caggcaccag gtcagcctgc ctgaagattc cagcagtaaa gtttcttaca aactatacc      599
<210> 121
   <211> 599
   <212> DNA
   <213> Homo sapiens
<400> 121
   gtctgtcatt attcttctag ttgttcctgt agggattgca atataaatcc ttatcactgt 60
   ctacttagaa ttaaagttgt gccacttctc ataaaatata aggactttga agtattatag 120
   ttccacttat taccatcctt agttatatta ttgtcatatt tatcatatct aaatacatta 180
   taaatcccca atacagtatt gtctttttaa aaattaagag aaataaaaaa ctgtgtaatc 240
   ttcatatgtg ccatatactt actatttctg atgctccttg cttgttcctg tacttctacw 300
   attacatttg gagtcgattt ttttcagcct gaataacttt ttttgctatt ccttataatg 360
   tgaatctgct ggtgactgat tctattttca ttcacctgaa aaagtctgta tttcatcatc 420
   atttttgaat gaaatttttg ctaggtacag aattctgggt tgaaggttgt ttttattccc 480
   ctgaacttta aagaagtttt tttcaaagta ttctccctcc attaattctg atgtgaatca 540
   ttctgatgtg agtcattatt catgttgttg cttccctgta gttaatgtgg tttctcact 599
<210> 122
   <211> 599
   <212> DNA
   <213> Homo sapiens
<400> 122
   atctccatct gccatctatt acctccatct accaactcca cctaccacca gtgatgaggt 60
   cctcattgcc agctgaataa gtgagtgtgc cagctccaaa actctattgt ttcacctgct 120
   ttcttagtac attcctgcta ttaactgtta cagattgagt tctctgggaa agtctctgag 180
   aaagaatttt gcataagtgc ttgttcaaga gtgtccttga gattaatgcc tgaaggaggg 240
   agaaaggaaa aggaaggaga atggggcaga gagagaagtt gagctgtaac aggggcccam 300
   cagtaacacc tgaagaccca cagagacctc tggagctaca gtggcttttc agagttgacc 360
   tcagttgggc caaaatggtc agacattata ttttttttat tgttctctca tttaatgtgg 420
   gctgcccaag gaaggagtgg gcaagacgcc tttctgaagc taacataatt actgaagggt 480
   ctgacagtag aagacatcag ccaacaacag tccctgctgc taggtcaaca agtctttcct 540
   tagagggatc tagaaggcac aactcagtgt tcatcagttc cttcactgga ttttcagct 599
<210> 123
   <211> 599
   <212> DNA
   <213> Homo sapiens
<400> 123
   ttgtaatggt gaattttatc tatgttaaac aacaaattta aaaaaaatat ttggaggaac 60
   attgacccgc ttgccccact ccatgcgtcc cattgagcac cctgactttg gtataacaga 120
   cctgccttga ttgggcattt agcattcttt aaagcttggc tactctgaat taagtctgag 180
   gtcttatcct cagctctgtc ttttctcatg ctgcagatgg gaaagtcttt gtatgctacc 240
   aaggtttcca cttgcttgtt aatcaatctc aagtctttgt tatacttttc tttgaaatgm 300
   ctgatgtatc atgtcatcca catttcagca catctcttca ttatactatc tcagtttccc 360
   actagagaag tgaagccatt agacttcact tcttagtctt atactagaag ctatctccat 420
   ctgccatcta ttacctccat ctaccaactc cacctaccac cagtgatgag gtcctcattg 480
   ccagctgaat aagtgagtgt gccagctcca aaactctatt gtttcacctg ctttcttagt 540
   acattcctgc tattaactgt tacagattga gttctctggg aaagtctctg agaaagaat 599
<210> 124
   <211> 599
   <212> DNA
   <213> Homo sapiens
<400> 124
   actggcatat ggataaatgt gtagaacaat ggactagcac cgaaagtcca gaaataagtt      60
   tatatgccta tggtcaattg atttttgtca agggttccaa gaccatttga agatggaaat     120
   aataatcctt tcaacgaatg atgctgaata tctacatgca aaagaataaa tgtggatccc     180
   tgctttacac cgtatacaaa aatcaatgca aagtgggtca aagacccaaa tattagagct     240
   gaaactataa attcttagaa gaaaacatag gtacaagttt tagtgacctc acacatgcar     300
   caacatgcat gaacctataa aacattatgc taagtgatag aagccagaca caaaatgcca     360
   cattttgtat gacttcattt ctataggata tccataatag aaaaatctgt agagacagaa     420
   agtaaatagg caatttccag aacgtgggag aaggagggaa tggggaggta catggtttct     480
   ttggggtatg atggaaatat tctggaatta gataatgata tggttgcaca ttttgtatat     540
   aataaaaacc actaaattga agaatttgac attgtaatgg tgaattttat ctatgttaa      599
<210> 125
   <211> 599
   <212> DNA
   <213> Homo sapiens
<400> 125
   atacaagatc agcatgcaaa tatcagttgt atttctatac agtagcgatg aacaatccaa      60
   agataaagtt aataaaacca ttcagtttat aatagcaaca aaaagaatat ttgggaataa     120
   atttgaccaa ggaagtgcaa gtgttgtaca ctgaagacta caaaacatta ttgaaaaata     180
   ataaagacct aaataaatgg aaagatgtgt aatttcatgg attggaaaac ttaataatat     240
   caagatggca atatatgcca aaccgatcaa cagattccat gcaattcctg ccaaaattcy     300
   agtggctttt tttttttcag aaatggacaa attgattcta aaatgaacaa gtgacaccaa     360
   atagccaaga caatgtttaa aaaggaagaa aattggagga ctcacacttt ccagtttcaa     420
   aacttactac aaatttacag tatttgaaac aatgtgatac tggcatatgg ataaatgtgt     480
   agaacaatgg actagcaccg aaagtccaga aataagttta tatgcctatg gtcaattgat     540
   ttttgtcaag ggttccaaga ccatttgaag atggaaataa taatcctttc aacgaatga      599
<210> 126
   <211> 599
   <212> DNA
   <213> Homo sapiens
<400> 126
   ttattgtgtg aacttgctgt gatgttgcag gattcttcct cctggagccc agatactttt      60
   caaaagatga atgtcatttc tggtaaaaat aaaataaaat aaaaatggca ttgattcagt     120
   aactgctcaa ggaatcataa cactgtacga gggcaattgc tgtcaacctc ctgatcatct     180
   atccttgttt tgttctttgt tttgttttgt tttttgtaca gattgagtag taaccttgtt     240
   ggcagctcat ttattttgcc cctaaacatg ccataatcta ttaggggtct aactctcagr     300
   taatcaggtc cccgactgtc cctacaacct tgccgtttac tgtggcaact gcatgtacca     360
   atgcattcct tgtggcctgg aaatagtgtt ctcttgaagt ttcccatgaa acacaggcat     420
   ttggtgggtt tcccagtctt acaaagtcta tgccctcccg caggctcact tctctgagcc     480
   ttttaatcct ttttcccact ggcaaccatg gtaattctgg aatttcaacc tcacttggca     540
   tggaccatta ctgtttctag gctcctagga gccatcccag tagtgagttc acactgtat      599
<210> 127
   <211> 599
   <212> DNA
   <213> Homo sapiens
<400> 127
   caacttcgac ccagtgtcca acaatccttg acatgtttgg tattctcctt tccgtagtgt      60
   acaattaccc aagtaactgg ccataaatcc ctttgcagaa agactggggt aattattatt     120
   gtgtgaactt gctgtgatgt tgcaggattc ttcctcctgg agcccagata cttttcaaaa     180
   gatgaatgtc atttctggta aaaataaaat aaaataaaaa tggcattgat tcagtaactg     240
   ctcaaggaat cataacactg tacgagggca attgctgtca acctcctgat catctatcck     300
   tgttttgttc tttgttttgt tttgtttttt gtacagattg agtagtaacc ttgttggcag     360
   ctcatttatt ttgcccctaa acatgccata atctattagg ggtctaactc tcaggtaatc     420
   aggtccccga ctgtccctac aaccttgccg tttactgtgg caactgcatg taccaatgca     480
   ttccttgtgg cctggaaata gtgttctctt gaagtttccc atgaaacaca ggcatttggt     540
   gggtttccca gtcttacaaa gtctatgccc tcccgcaggc tcacttctct gagcctttt     599
<210> 128
   <211> 599
   <212> DNA
   <213> Homo sapiens
<400> 128
   atttcttgtc atccagcccc attctcacga taccactgat aaaatgaatc aaaggccagg      60
   acattcaaac agtccagatc tctttggact atattgtaac agagggtggg taggacaaag     120
   aactaacagg tctgggacac actgcaaatt tacactgtta accatttcag ttaaaattta     180
   cactattaac catttcatgt gagtgcaaat ggtttctcat cctcttttct gcaaaggatg     240
   tgaaaaaacg tatttgccaa atcaatagcc acatgccatg cccctaaggc tgtgttgacy     300
   tcttctaaat atatcatatt ctgcagggtg agggcaaggc ttgctactac ttgcttgagt     360
   tggtggtagt ctactgtcat cctccagggt tcacctgggt tttgcagagc ccagcctggt     420
   gagttaaata aagatatgat gagaaccact gtactgcact ttttagatct ttaaggaggg     480
   gattaatttc catcatcctc ctcaggggat acaatattga tttttatata ctatctctgc     540
   tgaggcaagg gtgggaggga accatttcag gagctttcct ttgggctcct acccaatag      599
<210> 129
   <211> 599
   <212> DNA
   <213> Homo sapiens
<400> 129
   agaaaaccac ctggtatgag acaaccacct ggacatgagg cagccacctg gacatgagat      60
   agccacctgg acacaagaca cctggaagga gacagccacc tggacagtag atgagagttt     120
   tggaaattat ttgcctgtat ctttaatttc ccttttttca ttagaattta aacatggcct     180
   ctgctcaaag aagaaaaaga aggaggaatg aaggaaagaa ctgaaaagaa agaaaagaag     240
   aaggaagaaa gcacatcttc ctgcaactac cacttctccc cttgccctca ttaccccacy     300
   gtgtccactt accctgtcct caccatgcca ctatcttgac acgattgttc aagacctcct     360
   tctggcaagg tcattacttg tatccctggc agtgtttcat gccattgaac cttcctcctt     420
   aaagaatgtg caaccactct gtgggccact ttcttccctt ggcttctgaa cggccccaaa     480
   gcccactcct cagacatctt agtgggttct ttttccctct gtccactcct ctcactcggg     540
   cattccatgg ggttctgtcc aaattgctct cctacaaact ccccttgggc aatctccac      599
<210> 130
   <211> 599
   <212> DNA
   <213> Homo sapiens
<400> 130
   tatattttga gtttatgaca tgttttataa attgattatt tttttctcaa ttaggaacat 60
   ctatgcttat ctaagagatt ccctttattt tatgaggttt atattctttt tggtagaaaa 120
   cattttgttc caccttaact gtttgaggat taataaagta agatgtataa atgttattat 180
   tttgatttct taattgaaat gtaatttaaa gttgggtctt gctgcctttt ggggcttcgt 240
   gccgggcacc tgaaggaaag acaaattaaa ttattctcca acgtgctctg gatggtgags 300
   ctcaaacctg aatattttgt gttttgaaat ttgagttttc agagtcagaa atttagagta 360
   tatccataca gatatcctag aataaaacat atacttctta tttaaaagta aagtttaaag 420
   atactttatc attgaaatca tccatccatc tagttgattt ctacaggtga gctagcataa 480
   ttaaatcatt ttcctttcat ttccattaat attggctttt ctgaaaggat aacaggaatc 540
   tcaaattctg ttatagagct gatatttaaa agttcaattt cgccattttt ccaaaccca 599
<210> 131
   <211> 599
   <212> DNA
   <213> Homo sapiens
<400> 131
   cctttcaact gtgaaatctc taaccagacc atcaccaatt ataaagcagt ccccagcctt 60
   accatggatt aagtagaaat taaagatccc aacacacata tgtattgcct ggctgtcttg 120
   ccaggggggt ttatcctgag atcactcagc atagagccac tcagtgtgcg aacacacttg 180
   catgtggcat tatagaaaca ccctgaacac acatacacat tgcctggctg tcctgccagg 240
   gggttttatc cctggagcac ttagcataga gccatccact gtgcgaacac agcagcacay 300
   ggcgttacag aaagatccca aacacataca cacatcgcct ggctgtcctg ccatgggttt 360
   tatccctgga gcacatagca tagagccatc cactgtgcga acacaccagc acatggcgta 420
   taaaacatgt acaattgcaa ccaccgacac tcccaactgc agcgagataa gctcaccaga 480
   ccaacagcaa cagcggcaga aagggcgctc ctacctcctc atccagtgtt ttccccactc 540
   ttcaagtagg tgatttttta aagtttatca caaaaaaaag acaaaccttt tttcctaat 599
<210> 132
   <211> 599
   <212> DNA
   <213> Homo sapiens
<400> 132
   gtagcttggg cttcctccta gcatggtggc tgcagacgtc ttacaaagtg gttcagagcc 60
   ccacaggtgt gttctataat agctcacaag gcagaagctg cattgccttt caggacctag 120
   cctcagaagt cacactgcct catttctgct gtattctgtc agttccagga gagtcacaag 180
   cccttcctgg attcaaggga aagggaatta gatgccattt cctgatgaag gtggggcaaa 240
   gttttgaaag agcatgtgtg ggggactcat gccatctttg gatagtccca gtctccctcs 300
   tcaaggacat caactcttgt agtttgtctc cagtccaaac atgtgtgggc actttccctg 360
   catatgtggg tatccacgtg aatgtctctt cattgcttct ccatctttct aaaaagtaat 420
   cacagagagt catgaaatcc agtgttagaa ccaggcattg ttaatgctct tatgttatta 480
   ttattcaagc tgggctaggg gaatgtggca ttaataaaac aatatgtaca ttggatttag 540
   ttatactata ctctctattc taatactttg taatcattag taataaatag tagcatatt 599
<210> 133
   <211> 599
   <212> DNA
   <213> Homo sapiens
<400> 133
   agaacacatg gactcaggca ggggaacaac acacaccggg gcctggttgg cgtgggggca 60
   ggggggtggt gcggagaggg gtgaggggag ggcgaacacc aggacaaaga gctaatgctc 120
   gtgggtctta aaatatagat catgggttga taggtgcagc taaccaccat ggcacacgta 180
   tacctatgta acaaacctgc acatgtatcc cagaacttaa actaaactaa aatttttttt 240
   taagtgaatt ttagccacta gattttagag ttgaagcatg ccatggtact tgtgattagk 300
   catctacctg tgtatttatt cacctgaaca cctggatgtg tagaataaaa attgctcaat 360
   tgttgaaggc tgctgcatcg ctctatgcta cttgttctgt taaagtatgt tcagggagcc 420
   agcccagaga ggcacctcct tttagctctg tgaggcctta aagctgggtc accatgaagt 480
   ccctgtatat atattttttg ttttaataac tccaagtaac caaatgaaaa caattattta 540
   taaaatgtat taagaagtaa atgttatcaa tttgggactt tgcacaacta ttattaatg 599
<210> 134
   <211> 599
   <212> DNA
   <213> Homo sapiens
<400> 134
   atatttaaat tctgtaggct agttttcata ttctggaaag gtggattttc atatggcact 60
   ggtgctggtg tttcatcagg tcagctgtct atgtcataag agggtgttct tattagaaaa 120
   tgtaggcttt ggcaagcttt aaaaggtaca cgtgttactg catgttctga cgagattttg 180
   aaaatctttg gcaactgtgc tttccaagtg gagaaaaagg gctaccataa tgttgaaaga 240
   cggaaattaa aatctaatca gatatggtgg cccttatcct ccaccttaga atttctttty 300
   gtggaatgtc tggtggacag taatatatag aggctcctcg acttaggatg ggatcacatc 360
   cagatacacc cattgttaaa ttgaaaatat cataagttga aaaatgcatt taatatacct 420
   aatctaccag acatcatagc ttagcccagc ctactttaaa ttgctcagaa tgcttacact 480
   agcctgcagt cgggcaaaat tatctaacac aaagcctatt ttgtaataaa gggttaaata 540
   gctcatgtaa tttattggat atagtatcag ttgtttcccc ttgtgatcgc atcactgac 599
<210> 135
   <211> 599
   <212> DNA
   <213> Homo sapiens
<400> 135
   ctagagcgaa ctgaaccaga tatctgctaa ggctctgata gagaacagct ggaagctcag 60
   aaaggtaaac ataattcaaa tattggtgaa tatgccagtg cagccgagaa ggtgacagga 120
   tttgaaaaat gtgccaggac acaatgagaa aggccttgga ataaatacaa aaagcccaaa 180
   ctttattctg taaaagtggg gagctactga aggttttaat caaagcaggg ataatctcat 240
   ttccaaggca aaaaaacaaa aagaggcatt ccttcctaat accacatgta ggagctgtty 300
   ttattctatg gtggattatt actttagtag tatcttataa agacagtgcc tgaaggtctt 360
   ttatctgtac attgatgtaa cacttttcaa aagcatatca gcagacacat ttttggaagc 420
   atgcattctg aaacttgaac ttcaaatagg agttatggct gatatcatgg agtaaggtaa 480
   aacagaagct gactcctctc cagtattcaa ggctgactgt tgtctctgga actgagaata 540
   ctgggaaaag tgtgagttgg acactgtgca caactctaat taacactgtt ctaaaggga 599
<210> 136
   <211> 599
   <212> DNA
   <213> Homo sapiens
<400> 136
   tgggcatgac cattctctag tttctatctg tggcttggaa tttgtgttaa tgtattggtt 60
   gggtcctttc aattggaatg gggagagaga ggaatccata aatctgtgac tcagtgacct 120
   ttaggaattg gaagccaagg tctatttctt atgtcttatt ttgttattgt gcatagcagt 180
   gtctctcaac tgtttgaatc caggcccctg gagatgaatg tggactgtca gtggtgtggc 240
   tttttccacc tgcaccttct gcagagcagg gtgcaccttg agcaccttga gcacgggcay 300
   cttctgcctg tgcacctgtg cacatgaagg agcctcttgg ggtcttgctg cctttggggc 360
   ttttgctttc gcctccagct ccttatactt ctgcccatat atctcactct ctgttttcca 420
   gcctgtcttt tctgttttct gaagtctgcc ctttcccctt catggttagg tgaaggaagg 480
   ggaaatagaa ggaaatattt gaagaggaaa ggttgtggca ctgcagggta tttgtgactt 540
   tgtccccaat tgcattcttc aagtgcagtt cttggttttt agggtatctc tttgagtga 599
<210> 137
   <211> 599
   <212> DNA
   <213> Homo sapiens
<400> 137
   ggaagatgtc tgtgctgctg gtggcagcag ctgctctatg catgggatct ggagggctct 60
   gtggggaccc acatgtgtcc actttcctgc ccactctgcc ccactcccct tccctcactc 120
   tgcccgcctg ggcctatact tccccttccc acagcaacct agcagaagct ttgcttctcc 180
   atgtgttttc tgagaaatcc aggctatgaa aagatatagg gaaactttat aagattttct 240
   cagaataagg acaaaagact taaagataat ctcactcaaa gagataccct aaaaaccaak 300
   aactgcactt gaagaatgca attggggaca aagtcacaaa taccctgcag tgccacaacc 360
   tttcctcttc aaatatttcc ttctatttcc ccttccttca cctaaccatg aaggggaaag 420
   ggcagacttc agaaaacaga aaagacaggc tggaaaacag agagtgagat atatgggcag 480
   aagtataagg agctggaggc gaaagcaaaa gccccaaagg cagcaagacc ccaagaggct 540
   ccttcatgtg cacaggtgca caggcagaag gtgcccgtgc tcaaggtgct caaggtgca 599
<210> 138
   <211> 599
   <212> DNA
   <213> Homo sapiens
<400> 138
   tccgagacat tatactaatg tatgatggtc tccactcatg tttttggagg cctcatggga 60
   gtagtgttta taggcgtcac cttaggaaat gctgccctag acttttctga ctgtttgcca 120
   gccccatcag gctttggatc tcgtatcttc agaaatgctg tcagtatttt ccaaggtaaa 180
   aactagtaaa gaaaaccaaa caaaaatcca gaaatgtgct cagttaatag ttctcctagt 240
   atcttcccag aatagcatgt gcatttcacc cttgtcctgg agataatgtc tattacctgk 300
   attataatct ggaaatgaat tctaactcct tttattatgg gcatgacctt gtttctgtac 360
   tcccagcctc caaccagcaa gaaaatgggg gtctcagtcc cacagccaca aggactggga 420
   ttctgtaaat gccccagatg agctgggagg cagattcctc cccagagcct gcagatggag 480
   ttcagcctgc ccctgacacc ttgcgtggga gacactaaca gggagaagag ctgagcctcc 540
   cccagccttc tgacctacag caccatgtga tcatgaatat gtgttattct aagccacta 599
<210> 139
   <211> 599
   <212> DNA
   <213> Homo sapiens
<400> 139
   cactgcattg atttaatgca attccatcca aaaggatgag acccacacag aatcccaccc      60
   aaggccaacc acgttagact tcactataca gactctgccc catccctgct ggagccagtg     120
   ctgaccaggt tcaacacagt ctccaaggct gtcagcccaa ccagcaggtc ctgctgcctg     180
   gatgggtttt gttgctctca gccctgcagc cataaacctg gcactaccag ggtttccctt     240
   ccactaagag agaagagtgc taagccgggg catgagttaa gagctgaaat tgcagcctcr     300
   ctctgaccat ctgggtgtag tatcatcaac actgggagta caaatggtca agaagggacc     360
   aatctctgac ctgctctacc agtgggctag gaggagagac gtgtcacaga gtcaagtgga     420
   catgccacgc tgcacatcag ggcacagtga cagttgcctg acacccctta cttgctccag    480
   tggcagtggc aggactgaat tttggagaat taccctcccc actgtgaaca agaacagaac     540
   cctgtcagtt aaggtgcttg gccatccttt gccgtggcct cccacatggc cacgttgga      599
<210> 140
   <211> 599
   <212> DNA
   <213> Homo sapiens
<400> 140
   aagaacaaga aataacttaa taggccattt gtcaatgaga gtacagtgta ggaagggtgg      60
   aaagtgaata taaaatctag attggggctt ctggtttcct gttcagccta taaggagctt     120
   agaatttgcc actcagtctt gacaacaagt aaaatgctga acaaactgaa aaatcaataa     180
   ttcttctcag atccataaga gaagtgagat tacagggcaa actactacct tcagcatcac     240
   cccgcacccc caaccccact aaatagaaag acaggagaat acagagaatc ataacacagr     300
   cgcagaaacc tccttgagag agccagggta gataaacatg aactgtaatt gatgaattcc     360
   tggaggatca ctgtggatga cctgaaggat taaaaactct agagggactc actcaaagga     420
   gggcccaagc ttttgtgatt tttttttttt tttttttttt ttttgccacc tgaagctcta     480
   caaggttcca aaggtgaata ttaaggaaaa tccctcatgc tctggcagcc agaggggaaa     540
   aggaacaatt ttgaaatatg ccagaatatc gttcttaaca atgtctgccc tcaggagaa      599
<210> 141
   <211> 599
   <212> DNA
   <213> Homo sapiens
<400> 141
   aaaggcattg gagaggaata agcaattgag aatatgtttc ttattctcaa ttgatctaac      60
   agataatagt ttattcaaaa taatagcaac aatgtgtttg gttgtgtatg tttatatata     120
   taagcataca taagcatata tagagtattt tttgttataa ggtactcata ctactcatga     180
   agtgatacag tgttatttga aagtggattt gaattagtta taaatgtata ttgtaaactc     240
   taaagcaact actaaaaaaa gatttttttt aaaaaagcat gactgatatg ctaaaatgar     300
   gaaaatgtgg aatcctataa actgctgttc aatgaaacca caaaagccag gaaaaagtgt     360
   ggaatacaaa cataagaata aaaaataagg gaaacaaata gaagatgata acaaatacgg     420
   tagacattaa tccaaccatg ccaatactca ctttaaatgt aagatggtct aaatgagcca     480
   actaaaagac agagattttc acagtggatc aacaaacaag acccaaccat atatgttgtc     540
   tacaagaaat ccactttcaa tatacagaca catatacact aatagtaaat ggatggaga      599
<210> 142
   <211> 599
   <212> DNA
   <213> Homo sapiens
<400> 142
   accagacaat aatttgacac cactatgctg aaccaattca caccagtctc tcagaatgtt      60
   gtgcatgtta ccttgttcct tttcctaaac tatttaataa cacatatgcc aatggtgaaa     120
   ataaaataca aaatttaaga acaactgaca ttggctgtga actttgtagt ttataaaaca     180
   ctttcacatt aacatctcat ttgatcctct ctacaacctc cctctagaaa aaggtgggaa     240
   taaacaaaaa caagcaagca aaaccttcgt tttgaagacc ttaggaatta gcccaaggty     300
   ttatgactgg tagatggttg aggtgagatt caaggcaagg ccttctggcc ataacacacc     360
   attccttctt ctttctgcct ctctgtcctt atgacttgac acataaaaaa tgctagaaag     420
   tgtcacatct gtaccaccgg ggagactctg tgtgaaacaa tagtttcaag gagggtcatc     480
   ttttcagaaa ctgcaatcac aaaggatgca ttctgtactt taaattttgt aattccagcc     540
   taggcaacat agggacaccc tgtctctaca aaacatcttt aaaataaaat gatataatg      599
<210> 143
   <211> 599
   <212> DNA
   <213> Homo sapiens
<400> 143
   gtagtttata aaacactttc acattaacat ctcatttgat cctctctaca acctccctct      60
   agaaaaaggt gggaataaac aaaaacaagc aagcaaaacc ttcgttttga agaccttagg     120
   aattagccca aggtcttatg actggtagat ggttgaggtg agattcaagg caaggccttc     180
   tggccataac acaccattcc ttcttctttc tgcctctctg tccttatgac ttgacacata     240
   aaaaatgcta gaaagtgtca catctgtacc accggggaga ctctgtgtga aacaatagtk     300
   tcaaggaggg tcatcttttc agaaactgca atcacaaagg atgcattctg tactttaaat     360
   tttgtaattc cagcctaggc aacataggga caccctgtct ctacaaaaca tctttaaaat     420
   aaaatgatat aatgtaatac agtataatat ggtatgatat aatattttat gttatatttt     480
   cccattgtgt tgaagaaaat gtttaaggtg agctcttttc ctgtctttct caaaaaggta     540
   tggcaggatc gtcatttact tgctatcctg ccttggtgtt ggcgtcactg gggttgtgg      599
<210> 144
   <211> 599
   <212> DNA
   <213> Homo sapiens
<400> 144
   agcctgagca aaaagagtga aactctgtct aaaaaagaac tgggttttgt tggttctatt      60
   gtcccctccc cagcccttga agcttggtca tgtatatttg gatttctgcc ttcttcctct     120
   ctcttatatt aagtggcaag cagtaggctc ttttccaaag gtttcaactg aacagatgtc     180
   actggtgcaa tggtgcaagt acattaatga tgtaccttga ggtttacaga tcataaaatg     240
   tgtggtaaag ataaagcatg atctttttaa accactatga ttagtccatc cttgtcacty     300
   tttgagtgaa cacatttgag ctgtgtgttc cttttaggga aaaataatat acccctgttt     360
   cccagcttgc actcatccat tgtctatagt cctcatagcc aggtcaaaga tctgatcatt     420
   tgtattcaat tttaatcatc cagtgaataa tctctgctct tttgcacaca tagtccagag     480
   gtaagtccaa ggttttgatc ttgatttaaa ggtctaaata tagaaatata tttaggaata     540
   tagaatatag aagaaacaaa tatagaaggg aatatagaaa aataggatca tagaaaata      599
<210> 145
   <211> 599
   <212> DNA
   <213> Homo sapiens
<400> 145
   ccgaagcagc tgctgttaca catcccatga gtgtacagtt tagcactgct cccacagacc 60
   cactgaccca gctttattca tgagcaccgt agagatcatg acaacaggta agtagttcaa 120
   tgttccacat ggctgtgctt atgagagatc atgaactatg ggatccagtg gcttccaagt 180
   cattttaatt ttaatatgga aatttccaaa catactcaaa aatacaaaaa tagttgaatg 240
   aatctctatg catatccctc agctccaaca gttaccaact cctgatcaat cttagtttty 300
   ctatactctg cctagtttcc ctatccactg agtaggaatc aatccagcca tcatagcatt 360
   tcatcttgta atattttcct ctaaagtagg aacacatagg tatctctgga actatgtgtg 420
   cactatcttc aggacactgc tgaaggagga gcaagaacta ttcctgtggt tcttaaaatt 480
   caatacaagt aatcacacct ttgatccacc tgtggggact gatccctctt gtgggagcaa 540
   agatggggcc atggtatata aaatataaat ataaaactaa tatcaatgtc attgtccta 599
<210> 146
   <211> 599
   <212> DNA
   <213> Homo sapiens
<400> 146
   caggaagtat ttccatccct tggaaatgag ggaaaatgtg aatctctcca aacagtaatt 60
   taatctacca caatcagata cccctgcatc accaaagaat gtgttgaacc ctactctggc 120
   agttgaacta gtaatatgac caattgcatt agctagattt ggctgcaaga acagataccc 180
   ccaaatctct gtagtacatg cagacacatt gagcagtagt ttactgggga aacagctttt 240
   tcttggccat gtcccataca gcagctgtag gtcagctgct gcagctctgc tccaggctgy 300
   agctctcctt ctgagaccca ccctgaagga acagcactga tttgggatct gctgttcttg 360
   tggcagacag aaggagcaag agggctgtca ggaattcatg atgcctctca aagcttctgc 420
   cctgaattga tattctgcca cttctgctcc ttgggcgcaa tcaagtcagg tggccaagcc 480
   tgacactggg tggggaagta tattgcctac agagagatac tgctgtcaat catctggcaa 540
   tgggtgggaa tgggatgtaa aatcctctta cagagaggaa gtgaatagct gagaggatt 599
<210> 147
   <211> 599
   <212> DNA
   <213> Homo sapiens
<400> 147
   ctctgatggt aaagtgtcaa cttgattgga ttgaaggatg cctagatagc tgggaaagta 60
   ttgtttctgg gcgtgtctag gagggtgttg ccagaggaga ttcacatttg agtcagtgga 120
   ctgggagagg aagcctcccc tcaatgtggg tgagcaccat ccagttggct gccagtgtgg 180
   cttgaacaaa gcaggcagaa aaaggtggga taggatgtct tgctgagtct tctagctttc 240
   atcattctcc tgggctggat gcttcctgcc cctggacatc agactccagg ttcttcttgy 300
   acttacatga gtggtttgcc aggggctctc gggcctttgg ccacatactg aaggctgcac 360
   tgttggtttc cctacttttg aggcttttgg actcagactg agccactcct ggcttcctac 420
   ctcctcagct tgcagacaac ctatcgtggg acttcacctt gtggccgtgt gagccaattc 480
   tcctaataaa ctccctttca tagatatgta gatcctatca gttttgtccc tctagagaac 540
   cttgaccaat ataatagctg actgtgtcaa cactgagtga gcagccactg tcactgtac 599
<210> 148
   <211> 599
   <212> DNA
   <213> Homo sapiens
<400> 148
   tgtattgttt atagtgaatc tactgctttt aaaattaaac attagatgtt atatcaataa 60
   tgttcacaca taaacatgct tgaggcagtt cccatggtgg tatgtttatg tgacatcagg 120
   aacggaattt gagctttcca attccaaagg gattccttta ggcccatcat caatataaat 180
   gataccattg tttgaaatat ttgcaaaata gaaaaaatta aaacctcatt agaaagtgtc 240
   ttactgattt tttttattac cttagttgtc agggagactt ctagacctta atagctacck 300
   tcttttctat aatttaatga gcagtcacac aaacaccaat attgctacag aaagggactt 360
   tccagctttg ggaatatctg tttctgacat ttcctttcaa agggggattt ttataaacat 420
   aatttttctt cttccaaaag aggaaccaca ctatgattcc aacttccatg gagcccatcg 480
   gggatggtgg tatttcagtt caattagggt gctgtctttg ttaactgttg tgtttagttg 540
   tgatctcaga cttcagattt gaaatctctc ttcggggaat tcatttcaaa agatccaga 599
<210> 149
   <211> 599
   <212> DNA
   <213> Homo sapiens
<400> 149
   cagggccagc ccacggaaaa cacaaagccc ttttttccct agctagcctc tccatgtgga 60
   ggaacaagtc ctattgcttg gtggagagcc aaagcccttt cctttctcac ttactcttct 120
   ctcactgcta ctgggatcct ggcaaaccac acaggcaggt ccatgtggct tatgacacag 180
   gagttccgag ctttgcgaca tgacattggc aactgctcag ttatccgtga ttagttatga 240
   atctaaatgc gtgggcttcc cccttttcct tctcaaaggc tgagcttccc cttgccctty 300
   aggagcaaaa gtcttcccct ttctactcca gcactccttg gtaggcagag aagcatttac 360
   ttcttccctg cctaccctga gccccggcag tattacaaac aaaagtaaaa agtaaaacac 420
   aaggcaaaac tcatcagcca cacgggcttg ttttattcca ttaatattgg ttgttctctt 480
   ttctgggagt tgtgtaggca tctaatttga ttattttagt tatgtgtccc tctctaatag 540
   aaaagaggag tgagtttgcc tgtccttttt ccgtttcaag cgtctgctgg ggaagggcc 599
<210> 150
   <211> 599
   <212> DNA
   <213> Homo sapiens
<400> 150
   gctcactccc attgtttact tccacctggg gctctgggtg cagaaacgtg agtcctccca 60
   ggtttaggag ggagaaatac atttgccagc agggtttacc atgggcaggg ggaaggggat 120
   ggtgttagta gctgcttgtc tagccttgtg atttcgtctg gttattctca ataggagact 180
   tcttcatcag ttgccgtatt tcacaaggaa agatgacacc aataagaaag aaaacgtatt 240
   tgctcttgag tataatactg tgatttattc agtgaatgca tcatcactag ttccctaaar 300
   gctctgctct ctagaaactt ttcatctcta agaagtctgt gtgaataaaa tgttatttga 360
   ggtctttggg ctcaccgatt gggggaacca gaaaccacct tcaaaagtta aagtacccat 420
   tggtcttaca gacaagcatc aggaaccaga gggcctggtg gggctgggag gaagctcggc 480
   agtgacagct gaggtgctca tgtccttcca tcccactgcc cagtggataa tgagctcatt 540
   agtcagacga ggaccagccc agaatagcca ggagtaagca tgtcacatta cagagctgt 599
<210> 151
   <211> 599
   <212> DNA
   <213> Homo sapiens
<400> 151
   ttcttctcct ctctccactc tgttgacagg atagcttcat cttaagactt gttgtctcat 60
   cagtgtaaga tgactgctaa gttgtatgtg agcccacaat ttcctcattc attccttgaa 120
   ggaagaaaaa agatttcttc aggaacttct gacttaaaag caatgaagga ctttcccaga 180
   agtccatttc tcattggccc gaattgggtt gtatgcccat ttctcattgg ttcaaattgg 240
   gtcacgtgcc cagtcactga catggaggat acgatttccc taaactaatc aggcctcccr 300
   ctacaactag aaggagactc agctcccaca aacatatgaa ttgatggtgc gggagagtgt 360
   agtgggaatt ctgttaggaa agagaaggaa gtcaacaatc gggtacgctg tgtctaattt 420
   aagtccatga ctttaaaagt gagctttttt ttttgttaca aaaataatgt aggcttaatt 480
   tttttttttt ttttgagacg gagtctcccc gacacccagg ctggagtgca atggcacgat 540
   ctcggctcac tgcaactccg cctcccaggt tcaaacgatt ctcctgcctc agcctcccg 599
<210> 152
   <211> 599
   <212> DNA
   <213> Homo sapiens
<400> 152
   tgtgtgactt tccttggagc aaagataggc ataagatatc aagctttggt tgatctattg 60
   aatttactgt ttagaatctg tgattgacaa ggtactatca gtgtgtgtat gctctaagtc 120
   aaattcctgt gattactggt agagagactt tagctcaaag ctttggggaa aatgtgccta 180
   gattttcatt caatctgggc caaatctgga gttgtacaaa tagatagcat cggtcttgtc 240
   cttagtagcc agcatttttg tttaatagga ggagagaaac ccaggacgac actgaggaay 300
   agatatgacc agggcacttg acttcttgtt tcagcactca gtcattacac aaacagttgt 360
   tgaacaccag gcactttgct tggtgtgaca atgcactccc gccttccaag agctccagac 420
   ccacagggga gtgctgttgt acaggtgcta ggatagaggt ccggggagta gaggcagaat 480
   ttgggagcca tcagttctat ctgaggctag gaaaggcttc ccaggggagg taaaatcagg 540
   actgaggagg tcaaagagcc ctccatgcag aggtaagaga ctgacaagca caggaaact 599
<210> 153
   <211> 599
   <212> DNA
   <213> Homo sapiens
<400> 153
   ctagattggg cacaatatct atttcattca taaatgatcc tagcaattga gaaaagagaa 60
   tcatctacag aaccaatctc ttacttcgaa gtttttatgc tgattaagca gaaaatggag 120
   tgacatcacg aaagactgtg attccattgc acatttccta agaacaagtg gctccctata 180
   cttgattgtg gatttaggtg ttttcaagtc ctttggctgc caaacagatg tggggtaaga 240
   ttaccttctg gtaagaacgc agtgacaagg catgccaccc ctgccactat attgcttgcy 300
   gcaaagggga ggcgtcgtcc aaggcgctca atggttagta agatcaagag agctcctggc 360
   agttccacca cgcccgagat gaaaaagtct atatagaggt tgccccctat aattcccagg 420
   cgcatgacaa gtccttgata caccactgcg cttgtgaacc tgtagcaaca ttgaaagaca 480
   ccagagaaca agtaagagat aacttcaagc tgatttgaag gaagggatgt attgtaaatt 540
   taacgcaagc ttttaaatcc atcacaagtc aaacttacca agcaaacata agaataagt 599
<210> 154
   <211> 599
   <212> DNA
   <213> Homo sapiens
<400> 154
   ttcccaatgt agccactgtg gtcctcaacc atgctattcc tgcaaacaca gaaagtttga 60
   aagggaggag ttaggggtct gatttcatta acattttaca gaacaacaaa aagtatttgc 120
   ttagtggcct ccagtctgaa gtctttgcca attttaagca caaatgatag aactttctaa 180
   taatgtaacc aaatttcaac aacaatgaaa ctagatgata gctaatattt tagagcgcta 240
   gatgcctttc tgagcaggtt ggattatagg aagtacaatt caagaaccgt ttgttgagcr 300
   tgagcagcca ctacattgga acattgttcc aggcaatgac ctcctagaga gccaaagcac 360
   actggctttt tgtccaggtg gtagtttcca gtcatgcctt tgagccacca cgttcccaca 420
   gctggtgccc agctgtcctt tctgtgcata ccttgcggtg gtgacacaag gaatagccgt 480
   tcccagcaca cctggggagt tcctcacgtg agctgacctt ggtttctaga gtggagcaag 540
   ctttgaggac caggatcttt ggaaggggac atctattctg tgtgttacca ctaaggcaa 599
<210> 155
   <211> 599
   <212> DNA
   <213> Homo sapiens
<400> 155
   ttgcactcca gcctgggcaa cagggtaaga acctatctct aaaataaata aataataaaa 60
   aataacaaaa ataagcctaa tgtctaattg tatttctggt gttctctctc tatggatatc 120
   atatcttcat tggcccgatg ctaaaaatcc atgtggaggg agcaggtgac acaggatccc 180
   gggcacacat tccatcacct cctccaggtc cctcgagtgc acctctcctc ctacctccag 240
   cttccatagg acttgtgaag gaacagaatc atagtctagg agggaccttg gggaattcas 300
   tgtcccactt ctccagggct gctccaacgg tcctgcctgg gtttcacaca cttcccctgt 360
   gccctcttct gcagtaaagg atacgggttg gcccctttct cacgcaaccg tttctctatc 420
   tccacttcct tacttctggt agagaatgtt tttcatttat ggttcagcag ttgaaatttg 480
   aatgaaaata ctgagcccat gaggacagac cttctgcttc atgcaaaagt cctttgacag 540
   tgtgagggac aaagagtcgg cttctaccac ttgacaccag tgacctcaat acaggatca 599
<210> 156
   <211> 599
   <212> DNA
   <213> Homo sapiens
<400> 156
   ggaggaatcg gagggagagg ggcaggtaag tgagagtgtc agtgagcggg cgcagagggg 60
   actcccctcc agcacttctg tttccctgtg atgcaggaga ggaggccttg ggtgttggac 120
   cctaagaagg aactcagtca atcactgtga gatgtgtgag tgaacacatg gctgccaggt 180
   atacaggggc cgtggtgcta ctcagggttt gaggaaggga gagaaccttt gaagctgtgg 240
   taagggagag ctggggcatt gatctgggat gcagaggttg ctgtggttga gagctactcy 300
   agtgagcaac atgatggctt cagagtgagc aggccccatg ggagagggcc cagctgtgtc 360
   ttcctggagc ggtaacacct ttcccctatt ccataggtat cctggcatcc atctgtggtg 420
   gccttgtgat gcttttgcct gaaaccaagg gtattgcctt gccagagaca gtggatgatg 480
   tagaaaaact tggcaggtac tgtacaaaat tcaatgcacc ctaaataaaa gcaatattta 540
   aaaccacggg ggaaagaatg acattgagaa aattgaatat ccacagggaa aaaaataag 599
<210> 157
   <211> 599
   <212> DNA
   <213> Homo sapiens
<400> 157
   gagaggggca ggtaagtgag agtgtcagtg agcgggcgca gaggggactc ccctccagca      60
   cttctgtttc cctgtgatgc aggagaggag gccttgggtg ttggacccta agaaggaact     120
   cagtcaatca ctgtgagatg tgtgagtgaa cacatggctg ccaggtatac aggggccgtg     180
   gtgctactca gggtttgagg aagggagaga acctttgaag ctgtggtaag ggagagctgg     240
   ggcattgatc tgggatgcag aggttgctgt ggttgagagc tactccagtg agcaacatgr     300
   tggcttcaga gtgagcaggc cccatgggag agggcccagc tgtgtcttcc tggagcggta     360
   acacctttcc cctattccat aggtatcctg gcatccatct gtggtggcct tgtgatgctt     420
   ttgcctgaaa ccaagggtat tgccttgcca gagacagtgg atgatgtaga aaaacttggc     480
   aggtactgta caaaattcaa tgcaccctaa ataaaagcaa tatttaaaac cacgggggaa     540
   agaatgacat tgagaaaatt gaatatccac agggaaaaaa ataagaataa atcctcatc      599
<210> 158
   <211> 599
   <212> DNA
   <213> Homo sapiens
<400> 158
   tcccacattc ttgatggagc tgtcatgaga atcaggttgg gggcttctct tgtctctctt      60
   tagcctaatg ggtcagagac cacagggaac acaagaaaaa aacccaggaa gggctgtgca     120
   cctgacagct tctttgttaa actggctaca tattatgtgt tcccagaaac ttcaacagca     180
   ttacaaatcc taactcatcc aacattatat aaaaatactc tggatagatt gaggtcttta     240
   tacagtatta caacagcgcg gcatgatgta gctgaaatgc acacgtgcat ctctgcaagr     300
   cacagctttg caaggaggat cagctcctgg atagctcctt ctttctgtct ttgtcggggg     360
   cctcaaaggt gagagcggga aactggggtt ttcttattcc tgccacattt acaggaatgt     420
   ggactgaaaa aacaaaggag aaagtcttta ggaaagatta tgtaactagt tagaagaggg     480
   tgttctgtta ttatttaata gtatagctgg ttttgatcaa atacattcat gaaaacccaa     540
   ctctaaggta acaacaatat ctgtctccag gatcattttt tgtctgtgtt cagacctta      599
<210> 159
   <211> 599
   <212> DNA
   <213> Homo sapiens
<400> 159
   tttcttgtgt tccctgtggt ctctgaccca ttaggctaaa gagagacaag agaagccccc      60
   aacctgattc tcatgacagc tccatcaaga atgtgggatg tgccgaccaa ggatttgaga     120
   aagttgtaca gaaatgtgtt catcaaatct ggtcaaggga ctaagctcct agctgaccat     180
   tcattctgaa gattgcatgg aggatgaaca tctgggaatc ctgttaatga gaaggctgaa     240
   tcacaggcac ctgggccaaa gggtgtgagc attcatgttc tctgctcacc ttggtttccr     300
   cacaccttcg caatgtgaac aggtcaggag tccctcccgt ccacctcctc tgtaacagct     360
   ggggttccag gcatggttta ggccctgttc cagcaataag aaccaatctg ctgtacaatc     420
   tgaggacttg gctctgttat ttacaaaatg atgctgtggt tctgagatta tttgggacat     480
   ttttggctct cctttagtgg acacctagag ccacagattc ccttctttac taaacaaatc     540
   ccatggattc tgatttctgg gtcttaggat tttaaaagtg aagggatatt tttcttata      599
<210> 160
   <211> 599
   <212> DNA
   <213> Homo sapiens
<400> 160
   ccacccacat tcctggccct ggcaagtcag tcttacaaca aggtaagaac aggcccagaa 60
   accaactata ttgtccttcc acacaagccc cacaaaatcc ttctacattt acacagaacc 120
   cacacagctg aggcatcagt acctgcctct atgttttctg aaggaggaaa aaatatagaa 180
   aaataaaaat aattatatta ttttaggttc tctactttga aaatggccaa aattggaatc 240
   tttttcttga tctaagtagc tacatcaaaa tctgtaggcg cagtgatttg caccaagtty 300
   cgatattgct ggaaaatatt gaagatgctc tgagggtttc tatggatatc cattgtctca 360
   ctgtcagatg aaaagagagg gaagttttta gaaatgtgac actttgcagt gagggaggac 420
   aagagcaaac ttacctacag tctgtcacag gcacgatttt ttttttacac ttttgtgaat 480
   cattgaattc aatgccgagg ctactcatcc attcacaaac acaggaaaaa attataggtc 540
   atgatcccca taaatgaaga gtaatcagtc caatcccaca gaacctggac cttttgggt 599
<210> 161
   <211> 599
   <212> DNA
   <213> Homo sapiens
<400> 161
   tttaatagct agttcatttc cactaagaca ttcagtataa ataataagta aaaataaata 60
   gaaacattca gttttatttt aaatagtagg agcaggtacc ataatttctg tagttactct 120
   tttagtacaa tgatgcatgt atactgtgtg taaggcatat tagcagaaat tgagttcagc 180
   actagggaag atgatttcat tccatgcaat gtttctttaa aaaaaaaaaa gacttatata 240
   gatagattct cagaacagcc catggcaaat ggacagttat ttggaaaact caggttccar 300
   attcactgga gtgtagaatc tctggttggt tggggaggaa attcctcttg cagttgttat 360
   taataattat gtgaatatta ttaactatat taatgtttat aattttgaag accttgaagg 420
   actggagaca gagaagcatt tttgaacacc ctctgtagcc tatgcactgt tgtaggcatt 480
   gatgggtggt accaaagatg ggacactgtt cctacctcca gagatcttgc aggctggctg 540
   cagagagaag gcagagagga ggtaaaagaa aaataaaggc acatgtgtgt aagtcaccc 599
<210> 162
   <211> 599
   <212> DNA
   <213> Homo sapiens
<400> 162
   tatttctgaa aaaacaaaaa gccacttgga cttgtcccct attttacttt ctgctgacca 60
   atcaacttgt ctgtcctagt tacagaaagc tgtcctggac ttttcatttg tataatttgg 120
   ttgcttgtgg ttgggtctgc catgtggaag aaccttgagc tgggggaagc agcttggcct 180
   ccaagtccac tgaagaccac cattccaaga tttcctgggg aggcggtaca gggcagtgat 240
   gaagattatg ggagccacac tgcccagctt cgcatttggg acactcctgg ggagagcaar 300
   agggaagaat gagaggttag ggtgatagta aagattctac ttggccaata ttattgtatt 360
   gtggcattgt ggtctctgga tttagtgtga gttgatagct gaccttttcc ttgagtgggt 420
   gtttttgttt gttctgcaga agcatcttgg tggctcctct acctgaaagt ccctcgatgg 480
   ggtcagttcc agttctccac ttctggcccc gtctggtaca tgccactgcc tctcactgcc 540
   tgggctctct atccttgaca ggctgccttg aagttgagcc cacactgact ttcttgcct 599
<210> 163
   <211> 599
   <212> DNA
   <213> Homo sapiens
<400> 163
   acacatgtga cccagtctca agcccagact cccagctaca ggaggctggt tctccaagca 60
   actggatatg ttagcaagtg tggtattcga aggagatttt tttatctatt ttcaactgag 120
   gtacactttc atgtatagtc aaatgcacag atcttagatg tagtttgatg aattttgatg 180
   aacatgtaca cttgtgtaac tcgcaagtta cgatcgtgac acagaacatt tacatcaccc 240
   cagaaacaac ttttgtttta cggcaaaatc aatctctgct aatttcttcc catggccagw 300
   ggcaaatgat actggtttcc ttcaagagaa attatttttg cctttctgga accttatatc 360
   aatggactca tatataatgt tcatttgtgt tatctgacat ctttccttaa tgtaatattt 420
   ttgagcttca tccatgctat ttgagagtat cattaatgtg ctccctttta ttgctgaata 480
   gtatgccatt gtatgaatag atcgcaaatt atttattcat tttcttattg attgacactg 540
   tagttgccta cagtttgggg tataaaaaat aaagctgcta tgaatattct tgtagatct 599
<210> 164
   <211> 599
   <212> DNA
   <213> Homo sapiens
<400> 164
   aagaattaaa agatctaaat aaagatacac catgcttata gattgaaagg ctcaatatca 60
   cttctcatca aattgatctt tggattaaac tctcacccaa acactattgc tgcagtcctt 120
   ttttggaaaa aatgtggagg acttacatac cttaatataa agacttataa aggtacaaga 180
   atcaagacgt ggtattggcc aggacccttg gctcatgctt gtaaccccaa cactttggga 240
   ggctgaggca ggaggatggc ttgagcccag gtgttcaaga ccagcctcag caacagagcr 300
   agaccctctc tccacaaaaa aaaatgaacc attagatggg tgtgatgagt cacacctgta 360
   gttccagcca ctcaggacac tgaggggaga ggatcacttg acccaggagg tctaggcatg 420
   agtgaacctt gatcatgcca ctacattata gcctggatga cagaatgata ccctatctca 480
   aaaaataaaa agatatgcgt tattggcaaa aaagtatgca aaactaaaaa gggatggtcc 540
   agcaccagac ccacatacat atggtcaatt gattttctat atagatgaca aagcaatta 599
<210> 165
   <211> 599
   <212> DNA
   <213> Homo sapiens
<400> 165
   actctttgtg ctcaaaatgt agcaaaaagg ctctatgtta ttatcatgga aaaccatcat 60
   gcaaatagtg ttactgtctg tcgataccgt atcaggtggg tcagaaaggt ttgtttctgc 120
   aagacttatt aaagcttccg tgcaaaacta gcttccaggc agaatgcagt atctctagga 180
   tgggtcccag tacatgacct cctctcctgc tctcagtcca tcttctactg gccgcagcca 240
   ctccgggact gagggaatgg gggatgagtt gaaggaacag tgggtcccaa ggcataaacr 300
   gagatggatg aatagactat gagatctgaa gaggttaggc agctatggag aagttagtag 360
   ttggtcattt gtggtcatga ccattttttt catgtctttg gtagccactt atggcagaga 420
   gacttgccat tggatggtgg gaccccagct tctgtgcatc tatctaaccc cctcgcttac 480
   ttttcagtct ctgtctctct cctgagtctc atctgtcttt ttgcctttgt tcgtctcttc 540
   ttggaaacct ggacctattc agaccactgg tgctcaggac ccagtgtgtt tctgctgat 599
<210> 166
   <211> 599
   <212> DNA
   <213> Homo sapiens
<400> 166
   aggaaacaca caaagtttcc atgtctgtcc ccacctctga cctcaacagc ctatcgccac 60
   ctaaagcctg cactgccaaa tccatcacac tttgtactct catgccccat tgtctccatg 120
   aacattcagt agagatccct agagatgagc ttgcacgtga gagcagtttc ccatgaaaag 180
   gactctgaac tgttgttttt ctctaagctt ttatatgaaa tatttagaag aacttactac 240
   atttccagaa tggtaggaat ctgttgcttt tgtgtttgtt tgttttctca tgtccatctr 300
   cttacaaata aaaaaaagag aatgattgta attcttgaag actcatttct ggtttgtggc 360
   attgatagct tcatctgttt ccctgtccta ggaaatactc accttgaatt tttctgtttt 420
   aattcagcac tgattgatcc aaggccttca atgtaggagt gctaccatgg aaatggacag 480
   agttatcgag gcacatactt caccaccgtc acaggaagca cttgccaagc ttggtcatct 540
   atgacaccac accagcacag taggacctca gaaaagtacc caaatgcgta cgtctttgt 599
<210> 167
   <211> 599
   <212> DNA
   <213> Homo sapiens
<400> 167
   ttttgtagct atttagagca gagagactgg ccatgagttg gtcagacccg gcttctgtcc 60
   atgtacccac cccttttatt tttcagcatc tgtctctttg ctgtctctca tcagccttcc 120
   tgcctttgcc cttttctttc tggcacctca tatcttttca gaccattgat gcttgggatc 180
   gagtgtgttt ctacccattg gaatagatgt agaaatagaa agagattcga ctctccatag 240
   atatgcacat actccttcct cgtgagccaa gacagaaaag gccaacacaa cttttactty 300
   gggaatcaga ggttagaatg atagaattcc aaggtggaaa ggctgcattg actcttactg 360
   tgtgttgtag aaaacactct aagtgtttgg tcatttgtgg tcctgacagt tttcgtaatg 420
   tgatcacctc gccttcagat agcctcctga tgcataaagc tatgaggaag gagagccaga 480
   ttaacatttc ttatctctga gaccctttgc ttcaaggcaa tattcctgag acattttgct 540
   atgccctcta catctgacac aatttgattc ctgagcactt agctagaagc acgtctctt 599
<210> 168
   <211> 599
   <212> DNA
   <213> Homo sapiens
<400> 168
   atgcttcact ctgtctcccg tccaattaat tatttctcat cattccctca atccaagtaa 60
   caaaccttga aacacgagca tagacaccag gcttattggg gcgtgcacag ccaagacccc 120
   aagaagtgac tccttgtaaa atgtatttgt ccttctcgaa gcaaaccaga ggccctccac 180
   tgtcacccta aacagaggta ggggaaaatt catgtgagct ttaagctgcc aaccttttaa 240
   tatgggatgc catccttctc ttatccatat gtacattttc ccagtaattc aaatcagaar 300
   ggaacttatg agcatattac tcaagcatct gcagtgctta agtacttagc aaagtctatt 360
   caacaaaacc acatcactta gttttttcct gggaaaaaaa ataatccctt gtgaatcctg 420
   catgcattgc ttcctaactt ggtgtttcct ttagactggg ctagcctgga catcattgtt 480
   aagtgtgtgt aatttgtctc ggtcctgtac cctctacctt cctctctact ggaaaagcca 540
   ccacttcagt cactggtgac cagcacacac caggaaggtg agtggcctga ggtagttat 599
<210> 169
   <211> 599
   <212> DNA
   <213> Homo sapiens
<400> 169
   tcctggaaat atggtgacgg ttgcacacca ttgtgaatgt actgaatgcc actcagtggt 60
   acctgcaaaa atggttaaaa tagtaactct atgttgtata aattttattg caataaaaaa 120
   gaatagtcat catttactaa gaatcaacct ttccaggcac aagctcataa tgtggtatat 180
   ctcacttaat cctcacaacg gtcctatgaa acaggcactt gtcattcctg ttcttcagtt 240
   gaggaaactg aggcgtaagg aggttgtata gctcagcttg agtctgggtc tgcggagtcr 300
   ctgcccaaat gcttcctccc catgcgtgtt ggccccactc ttgcttccaa ccttccacat 360
   gtttgttcat aaatttcatt cttattcctt attactttct gaagtctcag ttcccataat 420
   tatatagaca tatttctatc tcacagtaac attgccaaat tattctctaa aagggtgtga 480
   cagtggaaaa tgtcactgga aaacaattac tttgcctcac ccttgtgata ttcaattttt 540
   aaaatattag ctaacatagt aggtggaaat tgtcctgatt cattttctaa tttacactt 599
<210> 170
   <211> 599
   <212> DNA
   <213> Homo sapiens
<400> 170
   aacactgtgt ccaggaagga agacaggtta atgatgaagt tgatctcctt tgattccctt 60
   tctcagttct ctcaagccta ccatatctgt tgtccaacct gtgaaaactg ttttctcatg 120
   tatttgatag tccttcatgg cagaaaggct ggcatagcca gacatgggtt tctgtccatc 180
   ttcctgaatg tgtaactctt cagcatctct atacttcctg actctcagct gccttcctcc 240
   ttttgcccct catgggctgg caacacatag cttttcagac accttgttct cagaacccar 300
   tgtgtttata caggttagag gagaaagtga tcggaagatc tcgaagtcaa tatagatatc 360
   cttcacttgt tacccaagac aaacatggca aacacaacct tcactccagg gcttggagat 420
   tagaatgaca gaattccctg gttgaactat ttcactggtc tttagtgggc attgtggaat 480
   ggtgctattt ctaagcacat ggtcatatgt tcttctgagc tttctattca tgagacctca 540
   ccacctccaa gtatcctcct acttctatca actatgagaa aagagagctg gcctgacat 599
<210> 171
   <211> 599
   <212> DNA
   <213> Homo sapiens
<400> 171
   tcttctcagc ctctagtacc ttctgtccta ctttttactt cctgtgttca actcttttag 60
   ctgacacata tgagtgagaa catgcagtgc taacctttct gttcctggct tatttaacat 120
   aatgtcctct aacgtcactc atgttactgt gaatgatagg attccattct ttttatggct 180
   gaatagtatt ccatgatatg tatataccac attttcttta tccattcatg tgtggtcaga 240
   caactaggtt gattccatgt cttggttatt gtgaagagag ctgcaataaa cctggggctk 300
   cagatgactc tttgatataa tgatttccat tctgttagat aaagcccagt agtgagatag 360
   ctggatcata tggtacttct atttgtagtt ttttaaggaa cctgcatact gttctccaga 420
   gtgattgcgc agtttaaatt cctactaact atgtacaaaa gttccctttt tttctgcatc 480
   ctcaccagca tgctattttt gtctttttga taatagccat ccttactggg gtgagaaaat 540
   agctcattgt ggtttaggtt tgcatttctc tgaggattag tgatgttgag cacattttc 599
<210> 172
   <211> 599
   <212> DNA
   <213> Homo sapiens
<400> 172
   gggcttcctc aggagaagag atggtctcat tgctcaggga accttttctc ctaggcaaac     60
   agaggtagtg tggcagctgg aagaggcctg gagtggtact tggactttga agccaaacac    120
   ttgccctctc agagcctttg cctccactgt ggtcctacca gagagctgag ttcaggacct    180
   gggtttgggc gtggaagggc ggtgtgagag caaggctgaa tagtccaact tcctccttta    240
   acaaatatcc actgagcgtc ttcgctacgc aaggctctct tccagaccgc ttggttcttw    300
   ggagtccagg agttctccac tcggtacacc cagttttacc aaagttaaac ccatgagatt    360
   ttgtaataaa gccaaggcta ataataatgc attccattac ttttcctaca catgtgtctc    420
   tgttaagcac tttgcaagga ggatgggcag ctgggccaat gcaggatcca actattcaag    480
   attcctccac ttacacaata tattgacact aactctgaat gcatccctac tgaatagaga    540
   actctagtaa tttctactga aaacatcaaa taaaaaagct actactttaa tagaaatta     599